# EUROPEAN PATENT SPECIFICATION

(11) **EP 4 399 212 B1**
(45) Date of publication and mention of the grant of the patent: **02.07.2025**
(21) Application number: 22773192.4
(22) Date of filing: 02.09.2022
(51) Int. Cl.: C07D 513/14, A01N 43/78, A01N 43/90, A01N 55/08, A61P 13/00, A61P 21/00

(54) **SUBSTITUTED 2,3-DIHYDRO[1,3]THIAZOLO[4,5-B]PYRIDINES, SALTS THEREOF AND THEIR USE AS HERBICIDALLY ACTIVE SUBSTANCES**
SUBSTITUIERTE 2,3-DIHYDRO[1,3]THIAZOLO[4,5-B]PYRIDINE, SALZE DAVON UND DEREN VERWENDUNG ALS HERBIZID WIRKSAME SUBSTANZEN
2,3-DIHYDRO[1,3]THIAZOLO[4,5-B]PYRIDINES SUBSTITUÉS, LEURS SELS ET LEUR UTILISATION EN TANT QUE SUBSTANCES À ACTION HERBICIDE

(30) Priority: 07.09.2021 EP 21195216
(43) Date of publication of application: 17.07.2024
(73) Proprietor: Bayer Aktiengesellschaft, 51373 Leverkusen (DE)
(72) Inventor: FRACKENPOHL, Jens, 51373 Leverkusen (DE); BARBER, David Michael, 51373 Leverkusen (DE); BRAUN, Ralf, 51373 Leverkusen (DE); BROWN, Ronald Wilson, 51373 Leverkusen (DE); HEINEMANN, Ines, 51373 Leverkusen (DE); KALLUS, Christopher, 51373 Leverkusen (DE); DITTGEN, Jan, 51373 Leverkusen (DE); REINGRUBER, Anna Maria, 51373 Leverkusen (DE); BOLLENBACH-WAHL, Birgit, 51373 Leverkusen (DE); GATZWEILER, Elmar, 51373 Leverkusen (DE)
(74) Representative: BIP Patents
(86) International application number: PCT/EP2022/074486
(87) International publication number: WO 2023/036707

(56) References cited:
- WO-A1-2016/087373
- JP-A- S5 931 778

## Description

The invention relates to the technical field of crop protection agents, in particular that of herbicides for the selective control of broad-leaved weeds and weed grasses in crops of useful plants. Specifically, the present invention relates to substituted 2,3-dihydro[1,3]thiazolo[4,5-b]pyridines and salts thereof, to processes for their preparation and to their use as herbicides.

In their application, crop protection agents known to date for the selective control of harmful plants in crops of useful plants or active compounds for controlling unwanted vegetation sometimes have disadvantages, be it (a) that they have no or insufficient herbicidal activity against particular harmful plants, (b) that the spectrum of harmful plants which can be controlled with an active compound is not broad enough, (c) that their selectivity in crops of useful plants is too low and/or (d) that they have a toxicologically unfavourable profile.

Furthermore, some active compounds which can be used as plant growth regulators for a number of useful plants cause unwanted reduced harvest yields in other useful plants or are not compatible with the crop plant, or only within a narrow application rate range. Some of the known active compounds cannot be produced economically on an industrial scale owing to precursors and reagents which are difficult to obtain, or they have only insufficient chemical stabilities. In the case of other active compounds, the activity is too highly dependent on environmental conditions, such as weather and soil conditions. The herbicidal activity of these known compounds, in particular at low application rates, and/or their compatibility with crop plants remain deserving of improvement.

Various documents describe substituted thiazolopyridines as having useful biological properties and uses. WO2017/009806 and WO2015/104688 demonstrate that substituted thiazolopyridines can inhibit interleukin-1 receptor associated kinases (IRAK), particularly that of IRAK4 and are therefore useful in the treatment of diseases and disorders induced by IRAK4.

In addition, WO2019/089580 discloses that substituted thiazolopyridines or pharmaceutically acceptable salts thereof can be used as a method for treating haematological disorders and solid malignant tumours via inhibition of IRAK4 and BCL-2 kinases.

WO2017/069275 reports that certain substituted thiazolopyridines can inhibit kynurenine aminotransferase II (KAT-II). This modification of KAT-II could be useful for the treatment of KAT-II related diseases such as schizophrenia.

WO2018/178947 concerns the preparation of substituted thiazolopyridines and their use for the treatment of acute myeloid leukaemia.

WO2017/153601 relates to substituted thiazolopyridines and their use as a treatment for diseases that involve the build-up of amyloid-like proteins, such as Parkinson's disease.

Furthermore, WO2010/135524 discloses substituted thiazolopyridines inhibitors of phosphatidylinositol 3-kinase (PI3Kα) that can be used against proliferative diseases.

Several documents (WO2016/087373, WO2014/125651, WO2013/018928, EP 2000/1000946, WO2012/086848 and JP 2019/112369) describe that substituted thiazolopyridines and acceptable salts thereof can be used as effective pest control agents.

WO2012/136751 discloses that substituted oxo- or thionothiazolopyridinones can also be used as effective pest control agents.

In addition, WO2003/006470 reports that substituted thiazolopyridines can be potent fungicidal agents.

WO2010/016846 describes that substituted thiazolopyridines and related compounds being able to modulate TGR5, whilst WO2015/091584 describes that substituted [1,3]thiazolo[5,4-c]pyridines and substituted dihydro[1,3]thiazolo[5,4-c]pyridines can be used as TYK2 inhibitors, useful in the treatment of inflammation. The modulation of TGR5 could represent a new opportunity to treat patients suffering from metabolic syndrome (Syndrome X).

The publication entitled "Synthesis of Thiazolo[4,5-d]pyridines" (Synthesis, 2008, 15, 2337-2346) shows various methods to prepare compounds containing a thiazolopyridine core.

WO2009/010260 describes that substituted phenylthiazolinyl carboxamides can be used as insecticides.

However, 2,3-dihydro[1,3]thiazolo[4,5-b]pyridines as outlined below have not been described in the literature, so far.

Thus, the use of substituted 2,3-dihydro[1,3]thiazolo[4,5-b]pyridines or salts thereof as herbicidally active compounds has not been previously described. Surprisingly, it has now been found that substituted 2,3-dihydro[1,3]thiazolo[4,5-b]pyridines or salts thereof are particularly suitable as herbicides.

In their application, herbicides that are known to date for controlling harmful plants in crops of useful plants or herbicides for controlling unwanted vegetation sometimes have disadvantages, be it (a) that they have no or insufficient herbicidal activity against particularly harmful plants, (b) that the spectrum of harmful plants which can be controlled with an active compound is not broad enough, and/or (c) that

the selectivity of the herbicides in and their compatibility with crop plants is too low, thereby causing unwanted damage and/or unwanted reduced harvest yields of the crops.

Thus, there is still a need for alternative herbicides, in particular highly active herbicides that are useful at low application rates and/or having good compatibility with crop plants, for the selective application in plant crops or use on non-crop land. It is also desirable to provide alternative chemical active compounds which may be used in an advantageous manner as herbicides or plant growth regulators.

It is therefore an objective of the present invention to provide compounds having herbicidal activity which are highly effective against economically important harmful plants even at relatively low application rates and that can be used selectively in crop plants.

It has now been surprisingly found that the compounds following general formula (I) or the salts thereof meet the said objectives. Accordingly, the present invention provides substituted 2,3-dihydro[1,3]thiazolo[4,5-b]pyridines of the general formula (I) or salts thereof 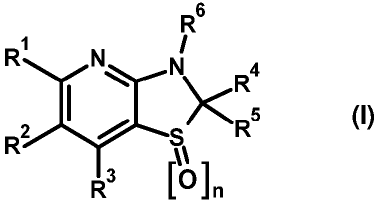 in which
- R¹: represents (C₃-C₈)-cycloalkyl, (C₃-C₈)-cycloalkenyl, (C₃-C₈)-cycloalkoxy, aryl, heteroaryl, heterocyclyl, a bicyclic or a heterobicyclic residue, wherein each of the previously mentioned eight residues is unsubstituted or is independently substituted by one or more residues selected from the group R⁷,
- R²: represents hydrogen, halogen, formyl, hydroxy, hydrothio, hydroxycarbonyl, aminocarbonyl, aminothiocarbonyl, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈) haloalkoxy, (C₁-C₈)-alkoxy-(C₁-C₈)-alkyl, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl, (C₁-C₈)-alkylcarbonyl, (C₂-C₈)-alkenylcarbonyl, (C₂-C₈)-alkynylcarbonyl, (C₁-C₈)-haloalkylcarbonyl, (C₂-C₈)-haloalkenylcarbonyl, (C₂-C₈)-haloalkynylcarbonyl, (C₁-C₈)-alkoxycarbonyl, (C₃-C₈)-cycloalkyl, (C₃-C₈)-cycloalkoxy, (C₃-C₈)-cycloalkylthio, (C₃-C₈)-cycloalkylsulfinyl, (C₃-C₈)-cycloalkylsulfonyl, (C₃-C₈)-halocycloalkyl, (C₃-C₈)-halocycloalkoxy, (C₃-C₈)-halocycloalkylthio, (C₃-C₈)-halocycloalkylsulfinyl, (C₃-C₈)-halocycloalkylsulfonyl, (C₃-C₈)-cycloalkyl-(C₁-C₈)-alkyl, (C₁-C₈)-alkyl-(C₃-C₈)-cycloalkyl, (C₃-C₈)-cycloalkylcarbonyl, (C₃-C₈)-cycloalkyl-(C₁-C₈)-alkylcarbonyl, (C₁-C₈)-alkylaminocarbonyl, (C₂-C₁₂)-dialkylaminocarbonyl, (C₁-C₈)-alkylaminosulfonyl, (C₂-C₁₂)-dialkylaminosulfonyl, tris[(C₁-C₈)alkyl]silyl, or aryl,
- R³: represents hydrogen, halogen, cyano, (C₁-C₈)-alkyl, (C₁-C₈)-haloalkyl, (C₂-C₈)-alkenyl, (C₂-C₈)-haloalkenyl, (C₁-C₈)-alkoxy, (C₁-C₈) haloalkoxy, (C₁-C₈)-alkylthio, (C₁-C₈)-haloalkylthio, (C₃-C₈)-cycloalkyl, (C₃-C₈)-halocycloalkyl, (C₃-C₈)-cycloalkoxy or (C₃-C₈)-halocycloalkoxy,
- R⁴ and R⁵: independently of one another represent hydrogen, (C₁-C₈)-alkyl, (C₁-C₈)-haloalkyl, (C₃-C₈)-cycloalkyl, (C₃-C₈)-halocycloalkyl, or R¹⁰O-(C₁-C₈)-alkyl, or
- R⁴ and R⁵: together with the carbon atom to which they are bonded form a fully saturated, or partly saturated 3- to 10-membered monocyclic or bicyclic ring optionally interrupted by heteroatoms and optionally having further substitution,
- R⁶: represents hydrogen, (C₁-C₈)-alkyl, (C₂-C₈)-alkenyl, (C₂-C₈)-alkynyl, cyano-(C₁-C₈)-alkyl, R¹⁰O-(C₁-C₈)-alkyl, R⁸R⁹N-(C₁-C₈)-alkyl, NR⁸R⁹, (C₃-C₁₀)-cycloalkyl-(C₁-C₈)-alkyl, C(=O)R¹⁰, C(=O)OR¹⁰, C(=O)NR⁸R⁹, R¹⁰O(O)C-(C₁-C₈)-alkyl, R¹⁰(O=)C-(C₁-C₈)-Alkyl, R⁸R⁹N(O)C-(C₁-C₈)-alkyl, SO₂R¹¹, R¹¹S(O)ₘ-(C₁-C₈)-alkyl, BR¹²R¹³, heterocyclyl, heteroaryl, aryl-(C₁-C₈)-alkyl, heteroaryl-(C₁-C₈)-alkyl, heterocyclyl-(C₁-C₈)-alkyl, tris[(C₁-C₈)alkyl]silyl, bis-[(C₁-C₈)-alkyl](aryl)silyl, (C₁-C₈)-alkyl-[bis-(aryl)]silyl, tris-[(C₁-C₈)-alkyl]silyl-(C₁-C₈)-alkyl, bis-[(C₁-C₈)-alkyl](aryl)silyl(C₁-C₈)-alkyl, or (C₁-C₈)-alkyl-[bis-(aryl)]silyl-(C₁-C₈)-alkyl,
- m: is 0, 1, 2,
- n: is 0, 1, 2,
- R⁷: represents hydrogen, halogen, formyl, hydroxy, hydroxycarbonyl, nitro, cyano, amino, aminocarbonyl, aminothiocarbonyl, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl, (C₁-C₈)-alkylcarbonyl, (C₁-C₈)-haloalkylcarbonyl, (C₂-C₈)-alkenylcarbonyl, (C₂-C₈)-haloalkenylcarbonyl, (C₂-C₈)-alkynylcarbonyl, (C₂-C₈)-haloalkynylcarbonyl, (C₁-C₈)-alkoxycarbonyl, (C₁-C₈)-haloalkoxycarbonyl, (C₃-C₈)-cycloalkyl, (C₃-C₈)-halocycloalkyl, (C₃-C₈)-cycloalkoxy, (C₃-C₈)-halocycloalkoxy, (C₃-C₈)-cycloalkylthio, (C₃-C₈)-halocycloalkylthio, (C₃-C₈)-cycloalkylsulfinyl, (C₃-C₈)-halocycloalkylsulfinyl, (C₃-C₈)-cycloalkylsulfonyl, (C₃-C₈)-halocycloalkylsulfonyl, (C₃-C₈)-cycloalkyl-(C₁-C₈)-alkyl, (C₁-C₈)-alkyl-(C₃-C₈)-cycloalkyl, (C₁-C₈)-alkoxycarbonyl-(C₁-C₈)-alkyl, hydroxycarbonyl-(C₁-C₈)-alkyl, (C₃-C₈)-cycloalkylcarbonyl, (C₃-C₈)-cycloalkyl-(C₁-C₈)-alkylcarbonyl, (C₁-C₈)-alkylamino, (C₂-C₁₂)-dialkylamino, (C₁-C₈)-alkylaminocarbonyl, (C₂-C₁₂)-dialkylaminocarbonyl, (C₁-C₈)-alkylaminosulfonyl, (C₂-C₁₂)-dialkylaminosulfonyl or tris[(C₁-C₈)alkyl]silyl.
- R⁸ and R⁹: independently of one another represent hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-haloalkoxy-(C₁-C₈)-alkyl, (C₁-C₈)-alkylthio-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, COR¹⁰, SO₂R¹¹, heterocyclyl, (C₁-C₈)-alkoxycarbonyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkyl-aminocarbonyl-(C₁-C₈)-alkyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl, aryl-(C₁-C₈)-alkoxycarbonyl, heteroaryl-(C₁-C₈)-alkoxycarbonyl, (C₂-C₈)-alkenyloxycarbonyl, (C₂-C₈)-alkynyloxycarbonyl, heterocyclyl-(C₁-C₈)-alkyl, or
- R⁸ and R⁹: together with the carbon atom to which they are bonded form a fully saturated or partly saturated 3- to 10-membered monocyclic or bicyclic ring optionally interrupted by heteroatoms and optionally having further substitution,
- R¹⁰: represents hydrogen, (C₁-C₈)-alkyl, (C₂-C₈)-alkenyl, (C₂-C₈)-alkinyl, (C₁-C₈)-cyanoalkyl, (C₁-C₁₀)-haloalkyl, (C₂-C₈)-haloalkenyl, (C₃-C₈)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-haloalkoxy-(C₁-C₈)-alkyl, (C₁-C₈)-alkoxy-(C₁-C₈)-haloalkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkoxy-(C₁-C₈)-alkyl, aryl, aryl-(C₁-C₈)-alkyl, aryl-(C₁-C₈)-alkoxy-(C₁-C₈)-alkyl, aryloxy-(C₁-C₈)-alkyl, heteroaryl-(C₁-C₈)-alkoxy-(C₁-C₈)-alkyl, heteroaryloxy-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkyl-aminocarbonyl-(C₁-C₈)- alkyl, bis-[(C₁-C₈)-alkyl]amino-(C₂-C₆)-alkyl, (C₁-C₈)-alkyl-amino-(C₂-C₆)-alkyl, aryl-(C₁-C₈)-alkylamino-(C₂-C₆)-alkyl, R¹¹(O)ₘS-(C₁-C₈)-alkyl, hydroxycarbonyl-(C₁-C₈)-alkyl, heterocyclyl, heterocyclyl-(C₁-C₈)-alkyl, heterocyclyl-(C₁-C₈)-alkoxy-(C₁-C₈)-alkyl, heterocyclyloxy-(C₁-C₈)-alkyl, tris-[(C₁-C₈)-alkyl]silyl-(C₁-C₈)-alkyl, bis-[(C₁-C₈)-alkyl](aryl)silyl(C₁-C₈)-alkyl, [(C₁-C₈)-alkyl]-bis-(aryl)silyl-(C₁-C₈)-alkyl, (C₁-C₈)-alkylcarbonyloxy-(C₁-C₈)-alkyl, (C₃-C₈)-cycloalkylcarbonyloxy-(C₁-C₈)-alkyl, arylcarbonyloxy-(C₁-C₈)-alkyl, heteroarylcarbonyloxy-(C₁-C₈)-alkyl, Heterocyclylcarbonyloxy-(C₁-C₈)-alkyl, (C₁-C₈)-alkoxycarbonyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl, hydroxy-(C₁-C₈)-alkyl, hydroxy-(C₁-C₈)-alkoxy-(C₁-C₈)-alkyl, hydroxy-(C₁-C₈)-alkoxy-(C₁-C₈)-alkoxy-(C₁-C₈)-alkyl, hydroxy-(C₁-C₈)-alkoxy-(C₁-C₈)-alkoxy-(C₁-C₈)-alkoxy-(C₁-C₈)-alkyl, (C₁-C₈)-alkylcarbonyloxy-(C₁-C₈)-alkoxy-(C₁-C₈)-alkyl, (C₁-C₈)-alkylcarbonyloxy-(C₁-C₈)-alkoxy-(C₁-C₈)-alkoxy-(C₁-C₈)-alkyl, (C₁-C₈)-alkylcarbonyloxy-(C₁-C₈)-alkoxy-(C₁-C₈)-alkoxy-(C₁-C₈)-alkoxy-(C₁-C₈)-alkyl, (C₁-C₈)-alkoxycarbonyl-(C₁-C₈)-alkoxy-(C₁-C₈)-alkyl, (C₁-C₈)-alkoxycarbonyl-(C₁-C₈)-alkoxy-(C₁-C₈)-alkoxy-(C₁-C₈)-alkyl, (C₁-C₈)-alkoxycarbonyl-(C₁-C₈)-alkoxy-(C₁-C₈)-alkoxy-(C₁-C₈)-alkoxy-(C₁-C₈)-alkyl, heteroarylcarbonyloxy-(C₁-C₈)-alkoxy-(C₁-C₈)-alkyl, heteroarylcarbonyloxy-(C₁-C₈)-alkoxy-(C₁-C₈)-alkoxy-(C₁-C₈)-alkyl, heteroarylcarbonyloxy-(C₁-C₈)-alkoxy-(C₁-C₈)-alkoxy-(C₁-C₈)-alkoxy-(C₁-C₈)-alkyl, heterocyclylcarbonyloxy-(C₁-C₈)-alkoxy-(C₁-C₈)-alkyl, heterocyclylcarbonyloxy-(C₁-C₈)-alkoxy-(C₁-C₈)-alkoxy-(C₁-C₈)-alkyl, heterocyclylcarbonyloxy-(C₁-C₈)-alkoxy-(C₁-C₈)-alkoxy-(C₁-C₈)-alkoxy-(C₁-C₈)-alkyl, hydroxycarbonyl-(C₁-C₈)-alkoxy-(C₁-C₈)-alkyl, hydroxycarbonyl-(C₁-C₈)-alkoxy-(C₁-C₈)-alkoxy-(C₁-C₈)-alkyl, or hydroxycarbonyl-(C₁-C₈)-alkoxy-(C₁-C₈)-alkoxy-(C₁-C₈)-alkoxy-(C₁-C₈)-alkyl,
- R¹¹: represents hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, heterocyclyl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]amino, (C₁-C₈)-alkyl-amino, aryl-(C₁-C₈)-amino, aryl-(C₁-C₆)-alkylamino, aryl-[(C₁-C₈)-alkyl]amino, heteroaryl-(C₁-C₈)-amino, heteroaryl-(C₁-C₆)-alkyl-amino, heteroaryl-[(C₁-C₈)-alkyl]amino; Hetercyclyl-(C₁-C₈)-amino, heterocyclyl-(C₁-C₆)-alkyl-amino, heterocyclyl-[(C₁-C₈)-alkyl]amino; (C₃-C₈)-cycloalkyl-amino, (C₃-C₈)-cycloalkyl-[(C₁-C₈)-alkyl]amino, or N-azetidinyl, N-pyrrolidinyl, N-piperidinyl, N-morpholinyl, and
- R¹² and R¹³: independently of one another represent hydrogen, NR⁸R⁹, OR¹⁰, or
- R¹² and R¹³: together with the boron atom to which they are bonded form a fully saturated or partly saturated 3- to 10-membered monocyclic or bicyclic ring optionally interrupted by heteroatoms and optionally having further substitution.

The compounds of the general formula (I) can form salts by addition of a suitable inorganic or organic acid, for example mineral acids, for example HCl, HBr, H₂SO₄, H₃PO₄ or HNO₃, or organic acids, for example carboxylic acids such as formic acid, acetic acid, propionic acid, oxalic acid, lactic acid or salicylic acid or sulfonic acids, for example p-toluenesulfonic acid, onto a basic group, for example amino, alkylamino, dialkylamino, piperidino, morpholino or pyridino. In such a case, these salts will comprise the conjugated base of the acid as the anion. Suitable substituents in deprotonated form, for example sulfonic acids, particular sulfonamides or carboxylic acids, are capable of forming internal salts with groups, such as amino groups, which are themselves protonatable. Salts may also be formed by action of a base on compounds of the general formula (I). Suitable bases are, for example, organic amines such as trialkylamines, morpholine, piperidine and pyridine, and the hydroxides, carbonates and bicarbonates of ammonium, alkali metals or alkaline earth metals, especially sodium hydroxide, potassium hydroxide, sodium carbonate, potassium carbonate, sodium bicarbonate and potassium bicarbonate. These salts are compounds in which the acidic hydrogen is replaced by an agriculturally suitable cation, for example metal salts, especially alkali metal salts or alkaline earth metal salts, in particular sodium and potassium salts, or else ammonium salts, salts with organic amines or quaternary ammonium salts, for example with cations of the formula [NR^{a}R^{b}R^{c}R^{d}]⁺ in which R^{a} to R^{d} are each independently an organic radical, especially alkyl, aryl, arylalkyl or alkylaryl. Also suitable are alkylsulfonium and alkylsulfoxonium salts, such as (C₁-C₄)-trialkylsulfonium and (C₁-C₄)-trialkylsulfoxonium salts.

The substituted 2,3-dihydro[1,3]thiazolo[4,5-b]pyridines of the general formula (I) according to the invention can, depending on external conditions such as pH, solvent and temperature, be present in various tautomeric structures, all of which are embraced by the general formula (I).

The compounds of the formula (I) used in accordance with the invention and salts thereof are also referred to hereinafter as "compounds of the general formula (I)".

The invention preferably provides compounds of the general formula (I) in which
- R¹: represents (C₃-C₇)-cycloalkyl, (C₃-C₇)-cycloalkenyl, (C₃-C₇)-cycloalkoxy, aryl, heteroaryl, heterocyclyl, a bicyclic or a heterobicyclic residue, wherein each of the previously mentioned eight residues is unsubstituted or is independently substituted by one or more residues selected from the group R⁷,
- R²: represents hydrogen, halogen, formyl, hydroxy, hydrothio, hydroxycarbonyl, aminocarbonyl, aminothiocarbonyl, (C₁-C₇)-alkyl, (C₁-C₇)-haloalkyl, (C₂-C₇)-alkenyl, (C₂-C₇)-haloalkenyl, (C₂-C₇)-alkynyl, (C₂-C₇)-haloalkynyl, (C₁-C₇)-alkoxy, (C₁-C₇) haloalkoxy, (C₁-C₇)-alkoxy-(C₁-C₇)-alkyl, (C₁-C₇)-alkylthio, (C₁-C₇)-haloalkylthio, (C₁-C₇)-alkylsulfinyl, (C₁-C₇)-haloalkylsulfinyl, (C₁-C₇)-alkylsulfonyl, (C₁-C₇)-haloalkylsulfonyl, (C₁-C₇)-alkylcarbonyl, (C₂-C₇)-alkenylcarbonyl, (C₂-C₇)-alkynylcarbonyl, (C₁-C₇)-haloalkylcarbonyl, (C₂-C₇)-haloalkenylcarbonyl, (C₂-C₇)-haloalkynylcarbonyl, (C₁-C₇)-alkoxycarbonyl, (C₃-C₇)-cycloalkyl, (C₃-C₇)-cycloalkoxy, (C₃-C₇)-cycloalkylthio, (C₃-C₇)-cycloalkylsulfinyl, (C₃-C₇)-cycloalkylsulfonyl, (C₃-C₇)-halocycloalkyl, (C₃-C₇)-halocycloalkoxy, (C₃-C₇)-halocycloalkylthio, (C₃-C₇)-halocycloalkylsulfinyl, (C₃-C₇)-halocycloalkylsulfonyl, (C₃-C₇)-cycloalkyl-(C₁-C₇)-alkyl, (C₁-C₇)-alkyl-(C₃-C₇)-cycloalkyl, (C₃-C₇)-cycloalkylcarbonyl, (C₃-C₇)-cycloalkyl-(C₁-C₇)-alkylcarbonyl, (C₁-C₇)-alkylaminocarbonyl, (C₂-C₁₂)-dialkylaminocarbonyl, (C₁-C₇)-alkylaminosulfonyl, (C₂-C₁₂)-dialkylaminosulfonyl or tris[(C₁-C₇)alkyl]silyl, or aryl,
- R³: represents hydrogen, halogen, cyano, (C₁-C₇)-alkyl, (C₁-C₇)-haloalkyl, (C₂-C₇)-alkenyl, (C₂-C₇)-haloalkenyl, (C₁-C₇)-alkoxy, (C₁-C₇) haloalkoxy, (C₁-C₇)-alkylthio, (C₁-C₇)-haloalkylthio, (C₃-C₇)-cycloalkyl, (C₃-C₇)-halocycloalkyl, (C₃-C₇)-cycloalkoxy or (C₃-C₇)-halocycloalkoxy,
- R⁴ and R⁵: independently of one another represent hydrogen, (C₁-C₇)-alkyl, (C₁-C₇)-haloalkyl, (C₃-C₇)-cycloalkyl, (C₃-C₇)-halocycloalkyl, or R¹⁰O-(C₁-C₇)-alkyl, or
- R⁴ and R⁵: together with the carbon atom to which they are bonded form a fully saturated, or partly saturated 3- to 10-membered monocyclic or bicyclic ring optionally interrupted by heteroatoms and optionally having further substitution,
- R⁶: represents hydrogen, (C₁-C₇)-alkyl, (C₂-C₇)-alkenyl, (C₂-C₇)-alkynyl, cyano-(C₁-C₇)-alkyl, R¹⁰O-(C₁-C₇)-alkyl, R⁸R⁹N-(C₁-C₇)-alkyl, NR⁸R⁹, (C₃-C₁₀)-cycloalkyl-(C₁-C₇)-alkyl, C(=O)R¹⁰, C(=O)OR¹⁰, C(=O)NR⁸R⁹, R¹⁰O(O)C-(C₁-C₇)-alkyl, R¹⁰(O=)C-(C₁-C₇)-Alkyl, R⁸R⁹N(O)C-(C₁-C₇)-alkyl, SO₂R¹¹, R¹¹S(O)ₘ-(C₁-C₇)-alkyl, BR¹²R¹³, heterocyclyl, heteroaryl, aryl-(C₁-C₇)-alkyl, heteroaryl-(C₁-C₇)-alkyl, heterocyclyl-(C₁-C₇)-alkyl, tris[(C₁-C₇)alkyl]silyl, bis-[(C₁-C₇)-alkyl](aryl)silyl, (C₁-C₇)-alkyl-[bis-(aryl)]silyl, tris-[(C₁-C₇)-alkyl]silyl-(C₁-C₇)-alkyl, bis-[(C₁-C₇)-alkyl](aryl)silyl(C₁-C₇)-alkyl, or (C₁-C₇)-alkyl-[bis-(aryl)]silyl-(C₁-C₇)-alkyl,
- m: is 0, 1, 2,
- n: is 0, 1, 2,
- R⁷: represents hydrogen, halogen, formyl, hydroxy, hydroxycarbonyl, nitro, cyano, amino, aminocarbonyl, aminothiocarbonyl, (C₁-C₇)-alkyl, (C₁-C₇)-haloalkyl, (C₂-C₇)-alkenyl, (C₂-C₇)-haloalkenyl, (C₂-C₇)-alkynyl, (C₂-C₇)-haloalkynyl, (C₁-C₇)-alkoxy, (C₁-C₇)-haloalkoxy, (C₁-C₇)-alkylthio, (C₁-C₇)-haloalkylthio, (C₁-C₇)-alkylsulfinyl, (C₁-C₇)-haloalkylsulfinyl, (C₁-C₇)-alkylsulfonyl, (C₁-C₇)-haloalkylsulfonyl, (C₁-C₇)-alkylcarbonyl, (C₁-C₇)-haloalkylcarbonyl, (C₂-C₇)-alkenylcarbonyl, (C₂-C₇)-haloalkenylcarbonyl, (C₂-C₇)-alkynylcarbonyl, (C₂-C₇)-haloalkynylcarbonyl, (C₁-C₇)-alkoxycarbonyl, (C₁-C₇)-haloalkoxycarbonyl, (C₃-C₇)-cycloalkyl, (C₃-C₇)-halocycloalkyl, (C₃-C₇)-cycloalkoxy, (C₃-C₇)-halocycloalkoxy, (C₃-C₇)-cycloalkylthio, (C₃-C₇)-halocycloalkylthio, (C₃-C₇)-cycloalkylsulfinyl, (C₃-C₇)-halocycloalkylsulfinyl, (C₃-C₇)-cycloalkylsulfonyl, (C₃-C₇)-halocycloalkylsulfonyl, (C₃-C₇)-cycloalkyl-(C₁-C₇)-alkyl, (C₁-C₇)-alkyl-(C₃-C₇)-cycloalkyl, (C₁-C₇)-alkoxycarbonyl-(C₁-C₇)-alkyl, hydroxycarbonyl-(C₁-C₇)-alkyl, (C₃-C₇)-cycloalkylcarbonyl, (C₃-C₇)-cycloalkyl-(C₁-C₇)-alkylcarbonyl, (C₁-C₇)-alkylamino, (C₂-C₁₂)-dialkylamino, (C₁-C₇)-alkylaminocarbonyl, (C₂-C₁₂)-dialkylaminocarbonyl, (C₁-C₇)-alkylaminosulfonyl, (C₂-C₁₂)-dialkylaminosulfonyl or tris[(C₁-C₇)alkyl]silyl,
- R⁸ and R⁹: independently of one another represent hydrogen, (C₁-C₇)-alkyl, (C₂-C₇)-alkenyl, (C₂-C₇)-alkynyl, (C₁-C₇)-cyanoalkyl, (C₁-C₇)-haloalkyl, (C₂-C₇)-haloalkenyl, (C₃-C₇)-haloalkynyl, (C₃-C₇)-cycloalkyl, (C₃-C₇)-halocycloalkyl, (C₄-C₇)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-haloalkoxy-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, (C₃-C₇)-cycloalkyl-(C₁-C₇)-alkyl, (C₄-C₇)-cycloalkenyl-(C₁-C₇)-alkyl, COR¹⁰, SO₂R¹¹, heterocyclyl, (C₁-C₇)-alkoxycarbonyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkyl-aminocarbonyl-(C₁-C₇)-alkyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl, aryl-(C₁-C₇)-alkoxycarbonyl, heteroaryl-(C₁-C₇)-alkoxycarbonyl, (C₂-C₇)-alkenyloxycarbonyl, (C₂-C₇)-alkynyloxycarbonyl, heterocyclyl-(C₁-C₇)-alkyl, or
- R⁸ and R⁹: together with the carbon atom to which they are bonded form a fully saturated or partly saturated 3- to 10-membered monocyclic or bicyclic ring optionally interrupted by heteroatoms and optionally having further substitution,
- R¹⁰: represents hydrogen, (C₁-C₇)-alkyl, (C₂-C₇)-alkenyl, (C₂-C₇)-alkinyl, (C₁-C₇)-cyanoalkyl, (C₁-C₇)-haloalkyl, (C₂-C₇)-haloalkenyl, (C₃-C₇)-haloalkinyl, (C₃-C₇)-cycloalkyl, (C₃-C₇)-halocycloalkyl, (C₄-C₇)-cycloalkenyl, (C₄-C₇)-halocycloalkenyl, (C₁-C₇)-alkoxy-(C₁-C₇)-alkyl, (C₁-C₇)-haloalkoxy-(C₁-C₇)-alkyl, (C₁-C₇)-alkoxy-(C₁-C₇)-haloalkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkoxy-(C₁-C₇)-alkyl, aryl, aryl-(C₁-C₇)-alkyl, aryl-(C₁-C₇)-alkoxy-(C₁-C₇)-alkyl, aryloxy-(C₁-C₇)-alkyl, heteroaryl-(C₁-C₇)-alkoxy-(C₁-C₇)-alkyl, heteroaryloxy-(C₁-C₇)-alkyl, heteroaryl, heteroaryl-(C₁-C₇)-alkyl, (C₃-C₇)-cycloalkyl-(C₁-C₇)-alkyl, (C₄-C₇)-cycloalkenyl-(C₁-C₇)-alkyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkyl-aminocarbonyl-(C₁-C₇)-alkyl, bis-[(C₁-C₇)-alkyl]amino-(C₂-C₆)-alkyl, (C₁-C₇)-alkyl-amino-(C₂-C₆)-alkyl, aryl-(C₁-C₇)-alkylamino-(C₂-C₆)-alkyl, R¹¹(O)ₘS-(C₁-C₇)-alkyl, hydroxycarbonyl-(C₁-C₇)-alkyl, heterocyclyl, heterocyclyl-(C₁-C₇)-alkyl, heterocyclyl-(C₁-C₇)-alkoxy-(C₁-C₇)-alkyl, heterocyclyloxy-(C₁-C₇)-alkyl, tris-[(C₁-C₇)-alkyl]silyl-(C₁-C₇)-alkyl, bis-[(C₁-C₇)-alkyl](aryl)silyl(C₁-C₇)-alkyl, [(C₁-C₇)-alkyl]-bis-(aryl)silyl-(C₁-C₇)-alkyl, (C₁-C₇)-alkylcarbonyloxy-(C₁-C₇)-alkyl, (C₃-C₇)-cycloalkylcarbonyloxy-(C₁-C₇)-alkyl, arylcarbonyloxy-(C₁-C₇)-alkyl, heteroarylcarbonyloxy-(C₁-C₇)-alkyl, Heterocyclylcarbonyloxy-(C₁-C₇)-alkyl, (C₁-C₇)-alkoxycarbonyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl, hydroxy-(C₁-C₇)-alkyl, hydroxy-(C₁-C₇)-alkoxy-(C₁-C₇)-alkyl, hydroxy-(C₁-C₇)-alkoxy-(C₁-C₇)-alkoxy-(C₁-C₇)-alkyl, hydroxy-(C₁-C₇)-alkoxy-(C₁-C₇)-alkoxy-(C₁-C₇)-alkoxy-(C₁-C₇)-alkyl, (C₁-C₇)-alkylcarbonyloxy-(C₁-C₇)-alkoxy-(C₁-C₇)-alkyl, (C₁-C₇)-alkylcarbonyloxy-(C₁-C₇)-alkoxy-(C₁-C₇)-alkoxy-(C₁-C₇)-alkyl, (C₁-C₇)-alkylcarbonyloxy-(C₁-C₇)-alkoxy-(C₁-C₇)-alkoxy-(C₁-C₇)-alkoxy-(C₁-C₇)-alkyl, (C₁-C₇)-alkoxycarbonyl-(C₁-C₇)-alkoxy-(C₁-C₇)-alkyl, (C₁-C₇)-alkoxycarbonyl-(C₁-C₇)-alkoxy-(C₁-C₇)-alkoxy-(C₁-C₇)-alkyl, (C₁-C₇)-alkoxycarbonyl-(C₁-C₇)-alkoxy-(C₁-C₇)-alkoxy-(C₁-C₇)-alkoxy-(C₁-C₇)-alkyl, heteroarylcarbonyloxy-(C₁-C₇)-alkoxy-(C₁-C₇)-alkyl, heteroarylcarbonyloxy-(C₁-C₇)-alkoxy-(C₁-C₇)-alkoxy-(C₁-C₇)-alkyl, heteroarylcarbonyloxy-(C₁-C₇)-alkoxy-(C₁-C₇)-alkoxy-(C₁-C₇)-alkoxy-(C₁-C₇)-alkyl, heterocyclylcarbonyloxy-(C₁-C₇)-alkoxy-(C₁-C₇)-alkyl, heterocyclylcarbonyloxy-(C₁-C₇)-alkoxy-(C₁-C₇)-alkoxy-(C₁-C₇)-alkyl, heterocyclylcarbonyloxy-(C₁-C₇)-alkoxy-(C₁-C₇)-alkoxy-(C₁-C₇)-alkoxy-(C₁-C₇)-alkyl, hydroxycarbonyl-(C₁-C₇)-alkoxy-(C₁-C₇)-alkyl, hydroxycarbonyl-(C₁-C₇)-alkoxy-(C₁-C₇)-alkoxy-(C₁-C₇)-alkyl, or hydroxycarbonyl-(C₁-C₇)-alkoxy-(C₁-C₇)-alkoxy-(C₁-C₇)-alkoxy-(C₁-C₇)-alkyl,
- R¹¹: represents hydrogen, (C₁-C₇)-alkyl, (C₂-C₇)-alkenyl, (C₂-C₇)-alkynyl, (C₁-C₇)-cyanoalkyl, (C₁-C₇)-haloalkyl, (C₂-C₇)-haloalkenyl, (C₃-C₇)-haloalkynyl, (C₃-C₇)-cycloalkyl, (C₃-C₇)-halocycloalkyl, (C₄-C₇)-cycloalkenyl, (C₄-C₇)-halocycloalkenyl, (C₁-C₇)-alkoxy-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, heterocyclyl-(C₁-C₇)-alkyl, (C₃-C₇)-cycloalkyl-(C₁-C₇)-alkyl, (C₄-C₁₀)-cycloalkenyl-(C₁-C₇)-alkyl, bis-[(C₁-C₇)-alkyl]amino, (C₁-C₇)-alkyl-amino, aryl-(C₁-C₇)-amino, aryl-(C₁-C₆)-alkylamino, aryl-[(C₁-C₇)-alkyl]amino; heteroaryl-(C₁-C₇)-amino, heteroaryl-(C₁-C₆)-alkyl-amino, heteroaryl-[(C₁-C₇)-alkyl]amino; Hetercyclyl-(C₁-C₇)-amino, heterocyclyl-(C₁-C₆)-alkyl-amino, heterocyclyl-[(C₁-C₇)-alkyl]amino; (C₃-C₇)-cycloalkyl-amino, (C₃-C₇)-cycloalkyl-[(C₁-C₇)-alkyl]amino; N-azetidinyl, N-pyrrolidinyl, N-piperidinyl, or N-morpholinyl,
- R¹² and R¹³: independently of one another represent hydrogen,NR⁸R⁹, OR¹⁰, or
- R¹² and R¹³: together with the boron atom to which they are bonded form a fully saturated or partly saturated 3- to 10-membered monocyclic or bicyclic ring optionally interrupted by heteroatoms and optionally having further substitution.

The invention more preferably provides compounds of the general formula (I) in which
- R¹: represents (C₃-C₆)-cycloalkyl, (C₃-C₆)-cycloalkenyl, (C₃-C₆)-cycloalkoxy, aryl, heteroaryl, heterocyclyl, a bicyclic or a heterobicyclic residue, wherein each of the previously mentioned eight residues is unsubstituted or is independently substituted by one or more residues selected from the group R⁷,
- R²: represents hydrogen, halogen, formyl, hydroxy, hydrothio, hydroxycarbonyl, aminocarbonyl, aminothiocarbonyl, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₁-C₆) haloalkoxy, (C₁-C₆)-alkoxy-(C₁-C₆)-alkyl, (C₁-C₆)-alkylthio, (C₁-C₆)-haloalkylthio, (C₁-C₆)-alkylsulfinyl, (C₁-C₆)-haloalkylsulfinyl, (C₁-C₆)-alkylsulfonyl, (C₁-C₆)-haloalkylsulfonyl, (C₁-C₆)-alkylcarbonyl, (C₂-C₆)-alkenylcarbonyl, (C₂-C₆)-alkynylcarbonyl, (C₁-C₆)-haloalkylcarbonyl, (C₂-C₆)-haloalkenylcarbonyl, (C₂-C₆)-haloalkynylcarbonyl, (C₁-C₆)-alkoxycarbonyl, (C₃-C₆)-cycloalkyl, (C₃-C₆)-cycloalkoxy, (C₃-C₆)-cycloalkylthio, (C₃-C₆)-cycloalkylsulfinyl, (C₃-C₆)-cycloalkylsulfonyl, (C₃-C₆)-halocycloalkyl, (C₃-C₆)-halocycloalkoxy, (C₃-C₆)-halocycloalkylthio, (C₃-C₆)-halocycloalkylsulfinyl, (C₃-C₆)-halocycloalkylsulfonyl, (C₃-C₆)-cycloalkyl-(C₁-C₆)-alkyl, (C₁-C₆)-alkyl-(C₃-C₆)-cycloalkyl, (C₃-C₆)-cycloalkylcarbonyl, (C₃-C₆)-cycloalkyl-(C₁-C₆)-alkylcarbonyl, (C₁-C₆)-alkylaminocarbonyl, (C₂-C₁₂)-dialkylaminocarbonyl, (C₁-C₆)-alkylaminosulfonyl, (C₂-C₁₂)-dialkylaminosulfonyl or tris[(C₁-C₆)alkyl]silyl, or 2,3,4,5,6-pentafluorophenyl,
- R³: represents hydrogen, halogen, cyano, (C₁-C₆)-alkyl, (C₁-C₆)-haloalkyl, (C₂-C₆)-alkenyl, (C₂-C₆)-haloalkenyl, (C₁-C₆)-alkoxy, (C₁-C₆) haloalkoxy, (C₁-C₆)-alkylthio, (C₁-C₆)-haloalkylthio, (C₃-C₆)-cycloalkyl, (C₃-C₆)-halocycloalkyl, (C₃-C₆)-cycloalkoxy or (C₃-C₆)-halocycloalkoxy,
- R⁴ and R⁵: independently of one another represent hydrogen, (C₁-C₆)-alkyl, (C₁-C₆)-haloalkyl, (C₃-C₆)-cycloalkyl, (C₃-C₆)-halocycloalkyl, or R¹⁰O-(C₁-C₆)-alkyl, or
- R⁴ and R⁵: together with the carbon atom to which they are bonded form a fully saturated, or partly saturated 3- to 10-membered monocyclic or bicyclic ring optionally interrupted by heteroatoms and optionally having further substitution,
- R⁶: represents hydrogen, (C₁-C₆)-alkyl, (C₂-C₆)-alkenyl, (C₂-C₆)-alkynyl, cyano-(C₁-C₆)-alkyl, R¹⁰O-(C₁-C₆)-alkyl, R⁸R⁹N-(C₁-C₆)-alkyl, NR⁸R⁹, (C₃-C₁₀)-cycloalkyl-(C₁-C₆)-alkyl, C(=O)R¹⁰, C(=O)OR¹⁰, C(=O)NR⁸R⁹, R¹⁰O(O)C-(C₁-C₆)-alkyl, R¹⁰(O=)C-(C₁-C₆)-Alkyl, R⁸R⁹N(O)C-(C₁-C₆)-alkyl, SO₂R¹¹, R¹¹S(O)ₘ-(C₁-C₆)-alkyl, BR¹²R¹³, heterocyclyl, heteroaryl, aryl-(C₁-C₆)-alkyl, heteroaryl-(C₁-C₆)-alkyl, heterocyclyl-(C₁-C₆)-alkyl, tris[(C₁-C₆)alkyl]silyl, bis-[(C₁-C₆)-alkyl](aryl)silyl, (C₁-C₆)-alkyl-[bis-(aryl)]silyl, tris-[(C₁-C₆)-alkyl]silyl-(C₁-C₆)-alkyl, bis-[(C₁-C₆)-alkyl](aryl)silyl(C₁-C₆)-alkyl, or (C₁-C₆)-alkyl-[bis-(aryl)]silyl-(C₁-C₆)-alkyl,
- m: is 0, 1, 2,
- n: is 0, 1, 2,
- R⁷: represents hydrogen, halogen, formyl, hydroxy, hydroxycarbonyl, nitro, cyano, amino, aminocarbonyl, aminothiocarbonyl, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₁-C₆)-haloalkoxy, (C₁-C₆)-alkylthio, (C₁-C₆)-haloalkylthio, (C₁-C₆)-alkylsulfinyl, (C₁-C₆)-haloalkylsulfinyl, (C₁-C₆)-alkylsulfonyl, (C₁-C₆)-haloalkylsulfonyl, (C₁-C₆)-alkylcarbonyl, (C₁-C₆)-haloalkylcarbonyl, (C₂-C₆)-alkenylcarbonyl, (C₂-C₆)-haloalkenylcarbonyl, (C₂-C₆)-alkynylcarbonyl, (C₂-C₆)-haloalkynylcarbonyl, (C₁-C₆)-alkoxycarbonyl, (C₁-C₆)-haloalkoxycarbonyl, (C₃-C₆)-cycloalkyl, (C₃-C₆)-halocycloalkyl, (C₃-C₆)-cycloalkoxy, (C₃-C₆)-halocycloalkoxy, (C₃-C₆)-cycloalkylthio, (C₃-C₆)-halocycloalkylthio, (C₃-C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyl-(C₁-C₆)-alkyl, (C₁-C₆)-alkyl-(C₃-C₆)-cycloalkyl, (C₁-C₆)-alkoxycarbonyl-(C₁-C₆)-alkyl, hydroxycarbonyl-(C₁-C₆)-alkyl, (C₃-C₆)-cycloalkylcarbonyl, (C₃-C₆)-cycloalkyl-(C₁-C₆)-alkylcarbonyl, (C₁-C₆)-alkylamino, (C₂-C₁₂)-dialkylamino, (C₁-C₆)-alkylaminocarbonyl, (C₂-C₁₂)-dialkylaminocarbonyl, (C₁-C₆)-alkylaminosulfonyl, (C₂-C₁₂)-dialkylaminosulfonyl or tris[(C₁-C₆)alkyl]silyl,
- R⁸ and R⁹: independently of one another represent hydrogen, (C₁-C₆)-alkyl, (C₂-C₆)-alkenyl, (C₂-C₆)-alkynyl, (C₁-C₆)-cyanoalkyl, (C₁-C₆)-haloalkyl, (C₂-C₆)-haloalkenyl, (C₃-C₆)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₆)-alkoxy-(C₁-C₆)-alkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-haloalkoxy-(C₁-C₆)-alkyl, (C₁-C₆)-alkylthio-(C₁-C₆)-alkyl, (C₁-C₆)-haloalkylthio-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₄-C₆)-cycloalkenyl-(C₁-C₆)-alkyl, COR¹⁰, SO₂R¹¹, heterocyclyl, (C₁-C₆)-alkoxycarbonyl, bis-[(C₁-C₆)-alkyl]aminocarbonyl-(C₁-C₆)-alkyl, (C₁-C₆)-alkyl-aminocarbonyl-(C₁-C₆)-alkyl, aryl-(C₁-C₆)-alkyl-aminocarbonyl-(C₁-C₆)-alkyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl, aryl-(C₁-C₆)-alkoxycarbonyl, heteroaryl-(C₁-C₆)-alkoxycarbonyl, (C₂-C₆)-alkenyloxycarbonyl, (C₂-C₆)-alkynyloxycarbonyl, heterocyclyl-(C₁-C₆)-alkyl, or
- R⁸ and R⁹: together with the carbon atom to which they are bonded form a fully saturated or partly saturated 3- to 10-membered monocyclic or bicyclic ring optionally interrupted by heteroatoms and optionally having further substitution,
- R¹⁰: represents hydrogen, (C₁-C₆)-alkyl, (C₂-C₆)-alkenyl, (C₂-C₆)-alkinyl, (C₁-C₆)-cyanoalkyl, (C₁-C₆)-haloalkyl, (C₂-C₆)-haloalkenyl, (C₃-C₆)-haloalkinyl, (C₃-C₆)-cycloalkyl, (C₃-C₆)-halocycloalkyl, (C₄-C₆)-cycloalkenyl, (C₄-C₆)-halocycloalkenyl, (C₁-C₆)-alkoxy-(C₁-C₆)-alkyl, (C₁-C₆)-haloalkoxy-(C₁-C₆)-alkyl, (C₁-C₆)-alkoxy-(C₁-C₆)-haloalkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkoxy-(C₁-C₆)-alkyl, aryl, aryl-(C₁-C₆)-alkyl, aryl-(C₁-C₆)-alkoxy-(C₁-C₆)-alkyl, aryloxy-(C₁-C₆)-alkyl, heteroaryl-(C₁-C₆)-alkoxy-(C₁-C₆)-alkyl, heteroaryloxy-(C₁-C₆)-alkyl, heteroaryl, heteroaryl-(C₁-C₆)-alkyl, (C₃-C₆)-cycloalkyl-(C₁-C₆)-alkyl, (C₄-C₆)-cycloalkenyl-(C₁-C₆)-alkyl, bis-[(C₁-C₆)-alkyl]aminocarbonyl-(C₁-C₆)-alkyl, (C₁-C₈)-alkyl-aminocarbonyl-(C₁-C₈)-alkyl, aryl-(C₁-C₆)-alkyl-aminocarbonyl-(C₁-C₆)-alkyl, bis-[(C₁-C₆)-alkyl]amino-(C₂-C₆)-alkyl, (C₁-C₆)-alkyl-amino-(C₂-C₆)-alkyl, aryl-(C₁-C₆)-alkylamino-(C₂-C₆)-alkyl, R¹¹(O)ₘS-(C₁-C₆)-alkyl, hydroxycarbonyl-(C₁-C₆)-alkyl, heterocyclyl, heterocyclyl-(C₁-C₆)-alkyl, heterocyclyl-(C₁-C₆)-alkoxy-(C₁-C₆)-alkyl, heterocyclyloxy-(C₁-C₆)- alkyl, tris-[(C₁-C₆)-alkyl]silyl-(C₁-C₆)-alkyl, bis-[(C₁-C₆)-alkyl](aryl)silyl(C₁-C₆)-alkyl, [(C₁-C₆)-alkyl]-bis-(aryl)silyl-(C₁-C₆)-alkyl, (C₁-C₆)-alkylcarbonyloxy-(C₁-C₆)-alkyl, (C₃-C₆)-cycloalkylcarbonyloxy-(C₁-C₆)-alkyl, arylcarbonyloxy-(C₁-C₆)-alkyl, heteroarylcarbonyloxy-(C₁-C₆)-alkyl, Heterocyclylcarbonyloxy-(C₁-C₆)-alkyl, (C₁-C₆)-alkoxycarbonyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl, hydroxy-(C₁-C₆)-alkyl, hydroxy-(C₁-C₆)-alkoxy-(C₁-C₆)-alkyl, hydroxy-(C₁-C₆)-alkoxy-(C₁-C₆)-alkoxy-(C₁-C₆)-alkyl, hydroxy-(C₁-C₆)-alkoxy-(C₁-C₆)-alkoxy-(C₁-C₆)-alkoxy-(C₁-C₆)-alkyl, (C₁-C₆)-alkylcarbonyloxy-(C₁-C₆)-alkoxy-(C₁-C₆)-alkyl, (C₁-C₆)-alkylcarbonyloxy-(C₁-C₆)-alkoxy-(C₁-C₆)-alkoxy-(C₁-C₆)-alkyl, (C₁-C₆)-alkylcarbonyloxy-(C₁-C₆)-alkoxy-(C₁-C₆)-alkoxy-(C₁-C₆)-alkoxy-(C₁-C₆)-alkyl, (C₁-C₆)-alkoxycarbonyl-(C₁-C₆)-alkoxy-(C₁-C₆)-alkyl, (C₁-C₆)-alkoxycarbonyl-(C₁-C₆)-alkoxy-(C₁-C₆)-alkoxy-(C₁-C₆)-alkyl, (C₁-C₆)-alkoxycarbonyl-(C₁-C₆)-alkoxy-(C₁-C₆)-alkoxy-(C₁-C₆)-alkoxy-(C₁-C₆)-alkyl, heteroarylcarbonyloxy-(C₁-C₆)-alkoxy-(C₁-C₆)-alkyl, heteroarylcarbonyloxy-(C₁-C₆)-alkoxy-(C₁-C₆)-alkoxy-(C₁-C₆)-alkyl, heteroarylcarbonyloxy-(C₁-C₆)-alkoxy-(C₁-C₆)-alkoxy-(C₁-C₆)-alkoxy-(C₁-C₆)-alkyl, heterocyclylcarbonyloxy-(C₁-C₆)-alkoxy-(C₁-C₆)-alkyl, heterocyclylcarbonyloxy-(C₁-C₆)-alkoxy-(C₁-C₆)-alkoxy-(C₁-C₆)-alkyl, heterocyclylcarbonyloxy-(C₁-C₆)-alkoxy-(C₁-C₆)-alkoxy-(C₁-C₆)-alkoxy-(C₁-C₆)-alkyl, hydroxycarbonyl-(C₁-C₆)-alkoxy-(C₁-C₆)-alkyl, or hydroxycarbonyl-(C₁-C₆)-alkoxy-(C₁-C₆)-alkoxy-(C₁-C₆)-alkyl, hydroxycarbonyl-(C₁-C₆)-alkoxy-(C₁-C₆)-alkoxy-(C₁-C₆)-alkoxy-(C₁-C₆)-alkyl,
- R¹¹: represents hydrogen, (C₁-C₆)-alkyl, (C₂-C₆)-alkenyl, (C₂-C₆)-alkynyl, (C₁-C₆)-cyanoalkyl, (C₁-C₆)-haloalkyl, (C₂-C₆)-haloalkenyl, (C₃-C₆)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₆)-alkoxy-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, heterocyclyl-(C₁-C₆)-alkyl, (C₃-C₆)-cycloalkyl-(C₁-C₆)-alkyl, (C₄-C₁₀)-cycloalkenyl-(C₁-C₆)-alkyl, bis-[(C₁-C₆)-alkyl]amino, (C₁-C₆)-alkyl-amino, aryl-(C₁-C₆)-amino, aryl-(C₁-C₆)-alkylamino, aryl-[(C₁-C₆)-alkyl]amino; heteroaryl-(C₁-C₆)-amino, heteroaryl-(C₁-C₆)-alkyl-amino, heteroaryl-[(C₁-C₆)-alkyl]amino; Hetercyclyl-(C₁-C₆)-amino, heterocyclyl-(C₁-C₆)-alkyl-amino, heterocyclyl-[(C₁-C₆)-alkyl]amino; (C₃-C₆)-cycloalkyl-amino, (C₃-C₆)-cycloalkyl-[(C₁-C₆)-alkyl]amino; N-azetidinyl, N-pyrrolidinyl, N-piperidinyl, or N-morpholinyl,
- R¹² and R¹³: independently of one another represent hydrogen, NR⁸R⁹, OR¹⁰, or
- R¹² and R¹³: together with the boron atom to which they are bonded form a fully saturated or partly saturated 3- to 10-membered monocyclic or bicyclic ring optionally interrupted by heteroatoms and optionally having further substitution.

The invention particularly provides compounds of the general formula (I) in which
- R¹: represents phenyl, furyl, pyrrolyl, thienyl, pyridyl, pyrimidinyl, pyridazinyl, pyrazinyl, thiazolyl, isothiazolyl, thiadiazolyl, oxazolyl, isoxazolyl, pyrazolyl, imidazolyl, triazolyl, tetrazolyl, cyclopentenyl, cyclohexenyl or an oxabicycloheptanyl residue, wherein each of the previously mentioned 20 residues is unsubstituted or is independently substituted by one or more residues selected from the group R⁷,
- R²: represents hydrogen, fluoro, chloro, bromo, iodo, formyl, hydroxy, hydrothio, hydroxycarbonyl, aminocarbonyl, aminothiocarbonyl, (C₁-C₅)-alkyl, (C₁-C₅)-haloalkyl, (C₂-C₅)-alkenyl, (C₂-C₅)-haloalkenyl, (C₂-C₅)-alkynyl, (C₂-C₅)-haloalkynyl, (C₁-C₅)-alkoxy, (C₁-C₅)-haloalkoxy, (C₁-C₅)-alkoxy-(C₁-C₅)-alkyl, (C₁-C₅)-alkylthio, (C₁-C₅)-haloalkylthio, (C₁-C₅)-alkylsulfinyl, (C₁-C₅)-haloalkylsulfinyl, (C₁-C₅)-alkylsulfonyl, (C₁-C₅)-haloalkylsulfonyl, (C₁-C₅)-alkylcarbonyl, (C₂-Cs)-alkenylcarbonyl, (C₂-C₅)-alkynylcarbonyl, (C₁-C₅)-haloalkylcarbonyl, (C₂-C₅)-haloalkenylcarbonyl, (C₂-C₅)-haloalkynylcarbonyl, (C₁-C₅)-alkoxycarbonyl, (C₃-C₆)-cycloalkyl, (C₃-C₆)-cycloalkoxy, (C₃-C₆)-cycloalkylthio, (C₃-C₆)-cycloalkylsulfinyl, (C₃-C₆)-cycloalkylsulfonyl, (C₃-C₅)-halocycloalkyl, (C₃-C₅)-halocycloalkoxy, (C₃-C₅)-halocycloalkylthio, (C₃-C₅)-halocycloalkylsulfinyl, (C₃-C₅)-halocycloalkylsulfonyl, (C₃-C₆)-cycloalkyl-(C₁-C₅)-alkyl, (C₁-C₅)-alkyl-(C₃-C₆)-cycloalkyl, (C₃-C₆)-cycloalkylcarbonyl, (C₃-C₆)-cycloalkyl-(C₁-C₅)-alkylcarbonyl, (C₁-C₅)-alkylaminocarbonyl, (C₂-C₁₀)-dialkylaminocarbonyl, (C₁-C₅)-alkylaminosulfonyl, (C₂-C₁₀)-dialkylaminosulfonyl or tris[(C₁-C₅)alkyl]silyl, 2,3,4,5,6-pentafluorophenyl,
- R³: represents hydrogen, fluoro, chloro, bromo, iodo, cyano, (C₁-C₅)-alkyl, (C₁-C₅)-haloalkyl, (C₂-C₅)-alkenyl, (C₂-C₅)-haloalkenyl, (C₁-C₅)-alkoxy, (C₁-C₅) haloalkoxy, (C₁-C₅)-alkylthio, (C₁-C₅)-haloalkylthio, (C₃-C₆)-cycloalkyl, (C₃-C₅)-halocycloalkyl, (C₃-C₆)-cycloalkoxy or (C₃-C₆)-halocycloalkoxy,
- R⁴ and R⁵: independently of one another represent hydrogen, (C₁-C₅)-alkyl, (C₁-C₅)-haloalkyl, (C₃-C₆)-cycloalkyl, (C₃-C₆)-halocycloalkyl, or R¹⁰O-(C₁-C₅)-alkyl, or
- R⁴ and R⁵: together with the carbon atom to which they are bonded form a fully saturated, or partly saturated 3- to 10-membered monocyclic or bicyclic ring optionally interrupted by heteroatoms and optionally having further substitution,
- R⁶: represents hydrogen, (C₁-C₅)-alkyl, (C₂-C₅)-alkenyl, (C₂-C₅)-alkynyl, cyano-(C₁-C₅)-alkyl, R¹⁰O-(C₁-C₅)-alkyl, R⁸R⁹N-(C₁-C₅)-alkyl, NR⁸R⁹, (C₃-C₆)-cycloalkyl-(C₁-C₅)-alkyl, C(=O)¹⁰, C(=O)OR¹⁰, C(=O)NR⁸R⁹, R¹⁰O(O)C-(C₁-C₅)-alkyl, R¹⁰(O=)C-(C₁-C₅)-Alkyl, R⁸R⁹N(O)C-(C₁-C₅)-alkyl, SO₂R¹¹, R¹¹S(O)ₘ-(C₁-C₅)-alkyl, BR¹²R¹³, heterocyclyl, heteroaryl, aryl-(C₁-C₅)-alkyl, heteroaryl-(C₁-C₅)-alkyl, heterocyclyl-(C₁-C₅)-alkyl, tris[(C₁-C₅)alkyl]silyl, bis-[(C₁-C₅)-alkyl](aryl)silyl, (C₁-C₅)-alkyl-[bis-(aryl)]silyl, tris-[(C₁-C₅)-alkyl]silyl-(C₁-C₅)-alkyl, bis-[(C₁-C₅)-alkyl](aryl)silyl(C₁-C₅)-alkyl, or (C₁-C₅)-alkyl-[bis-(aryl)]silyl-(C₁-C₅)-alkyl,
- m: is 0, 1, 2,
- n: is 0, 1, 2,
- R⁷: represents hydrogen, fluoro, chloro, bromo, iodo, formyl, hydroxy, hydroxycarbonyl, nitro, cyano, amino, aminocarbonyl, aminothiocarbonyl, (C₁-C₅)-alkyl, (C₁-C₅)-haloalkyl, (C₂-C₅)-alkenyl, (C₂-C₅)-haloalkenyl, (C₂-C₅)-alkynyl, (C₂-C₅)-haloalkynyl, (C₁-C₅)-alkoxy, (C₁-C₅)-haloalkoxy, (C₁-C₅)-alkylthio, (C₁-C₅)-haloalkylthio, (C₁-C₅)-alkylsulfinyl, (C₁-C₅)-haloalkylsulfinyl, (C₁-C₅)-alkylsulfonyl, (C₁-C₅)-haloalkylsulfonyl, (C₁-C₅)-alkylcarbonyl, (C₁-C₅)-haloalkylcarbonyl, (C₂-C₅)-alkenylcarbonyl, (C₂-C₅)-haloalkenylcarbonyl, (C₂-C₅)-alkynylcarbonyl, (C₂-C₅)-haloalkynylcarbonyl, (C₁-C₅)-alkoxycarbonyl, (C₁-C₅)-haloalkoxycarbonyl, (C₃-C₆)-cycloalkyl, (C₃-C₆)-halocycloalkyl, (C₃-C₆)-cycloalkoxy, (C₃-C₆)-halocycloalkoxy, (C₃-C₆)-cycloalkylthio, (C₃-C₆)-halocycloalkylthio, (C₃-C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyl-(C₁-C₅)-alkyl, (C₁-C₅)-alkyl-(C₃-C₆)-cycloalkyl, (C₁-C₅)-alkoxycarbonyl-(C₁-C₅)-alkyl, hydroxycarbonyl-(C₁-C₅)-alkyl, (C₃-C₆)-cycloalkylcarbonyl, (C₃-C₆)-cycloalkyl-(C₁-C₅)-alkylcarbonyl, (C₁-C₅)-alkylamino, (C₂-C₁₀)-dialkylamino, (C₁-C₅)-alkylaminocarbonyl, (C₂-C₁₀)-dialkylaminocarbonyl, (C₁-C₅)-alkylaminosulfonyl, (C₂-C₁₀)-dialkylaminosulfonyl or tris[(C₁-C₅)alkyl]silyl,
- R⁸ and R⁹: independently of one another represent hydrogen, (C₁-C₅)-alkyl, (C₂-C₅)-alkenyl, (C₂-C₅)-alkynyl, (C₁-C₅)-cyanoalkyl, (C₁-C₅)-haloalkyl, (C₂-C₅)-haloalkenyl, (C₃-C₅)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₅)-alkoxy-(C₁-C₅)-alkyl, (C₁-C₅)-alkoxy-(C₁-C₅)-alkoxy-(C₁-C₅)-alkyl, (C₁-C₅)-alkoxy-(C₁-C₅)-alkoxy-(C₁-C₅)-alkoxy-(C₁-C₅)-alkyl, (C₁-C₅)-haloalkoxy-(C₁-C₅)-alkyl, (C₁-C₅)- alkylthio-(C₁-C₅)-alkyl, (C₁-C₅)-haloalkylthio-(C₁-C₅)-alkyl, (C₁-C₅)-alkoxy-(C₁-C₅)-haloalkyl, aryl, aryl-(C₁-C₅)-alkyl, heteroaryl, heteroaryl-(C₁-C₅)-alkyl, (C₃-C₆)-cycloalkyl-(C₁-C₅)-alkyl, (C₄-C₆)-cycloalkenyl-(C₁-C₅)-alkyl, COR¹⁰, SO₂R¹¹, heterocyclyl, (C₁-C₅)-alkoxycarbonyl, bis-[(C₁-C₅)-alkyl]aminocarbonyl-(C₁-C₅)-alkyl, (C₁-C₅)-alkyl-aminocarbonyl-(C₁-C₅)-alkyl, aryl-(C₁-C₅)-alkyl-aminocarbonyl-(C₁-C₅)-alkyl, (C₁-C₅)-alkoxycarbonyl-(C₁-C₅)-alkyl, (C₂-C₅)-alkenyloxycarbonyl-(C₁-C₅)-alkyl, (C₂-C₅)-alkynyloxycarbonyl-(C₁-C₅)-alkyl, aryl-(C₁-C₅)-alkoxycarbonyl-(C₁-C₅)-alkyl, heteroaryl-(C₁-C₅)-alkoxycarbonyl-(C₁-C₅)-alkyl, heterocyclyl-(C₁-C₅)-alkoxycarbonyl-(C₁-C₅)-alkyl, aryl-(C₁-C₅)-alkoxycarbonyl, heteroaryl-(C₁-C₅)-alkoxycarbonyl, (C₂-C₅)-alkenyloxycarbonyl, (C₂-C₅)-alkynyloxycarbonyl, heterocyclyl-(C₁-C₅)-alkyl, or
- R⁸ and R⁹: together with the carbon atom to which they are bonded form a fully saturated or partly saturated 3- to 10-membered monocyclic or bicyclic ring optionally interrupted by heteroatoms and optionally having further substitution,
- R¹⁰: represents hydrogen, (C₁-C₅)-alkyl, (C₂-C₅)-alkenyl, (C₂-C₅)-alkinyl, (C₁-C₅)-cyanoalkyl, (C₁-C₅)-haloalkyl, (C₂-C₅)-haloalkenyl, (C₃-C₅)-haloalkinyl, (C₃-C₆)-cycloalkyl, (C₃-C₆)-halocycloalkyl, (C₄-C₆)-cycloalkenyl, (C₄-C₆)-halocycloalkenyl, (C₁-C₅)-alkoxy-(C₁-C₅)-alkyl, (C₁-C₅)-haloalkoxy-(C₁-C₅)-alkyl, (C₁-C₅)-alkoxy-(C₁-C₅)-haloalkyl, (C₁-C₅)-alkoxy-(C₁-C₅)-alkoxy-(C₁-C₅)-alkyl, (C₁-C₅)-alkoxy-(C₁-C₅)-alkoxy-(C₁-C₅)-alkoxy-(C₁-C₅)-alkyl, (C₁-C₅)-alkoxy-(C₁-C₅)-alkoxy-(C₁-C₅)-alkoxy-(C₁-C₅)-alkoxy-(C₁-C₅)-alkyl, aryl, aryl-(C₁-C₅)-alkyl, aryl-(C₁-C₅)-alkoxy-(C₁-C₅)-alkyl, aryloxy-(C₁-C₅)-alkyl, heteroaryl-(C₁-C₅)-alkoxy-(C₁-C₅)-alkyl, heteroaryloxy-(C₁-C₅)-alkyl, heteroaryl, heteroaryl-(C₁-C₅)-alkyl, (C₃-C₆)-cycloalkyl-(C₁-C₅)-alkyl, (C₄-C₆)-cycloalkenyl-(C₁-C₅)-alkyl, bis-[(C₁-C₅)-alkyl]aminocarbonyl-(C₁-C₅)-alkyl, (C₁-C₅)-alkyl-aminocarbonyl-(C₁-C₅)-alkyl, aryl-(C₁-C₅)-alkyl-aminocarbonyl-(C₁-C₅)-alkyl, bis-[(C₁-C₅)-alkyl]amino-(C₂-C₅)-alkyl, (C₁-C₅)-alkyl-amino-(C₂-C₅)-alkyl, aryl-(C₁-C₅)-alkylamino-(C₂-C₅)-alkyl, R¹¹(O)ₘS-(C₁-C₅)-alkyl, hydroxycarbonyl-(C₁-C₅)-alkyl, heterocyclyl, heterocyclyl-(C₁-C₅)-alkyl, heterocyclyl-(C₁-C₅)-alkoxy-(C₁-C₅)-alkyl, heterocyclyloxy-(C₁-C₅)-alkyl, tris-[(C₁-C₅)-alkyl]silyl-(C₁-C₅)-alkyl, bis-[(C₁-C₅)-alkyl](aryl)silyl(C₁-C₅)-alkyl, [(C₁-C₅)-alkyl]-bis-(aryl)silyl-(C₁-C₅)-alkyl, (C₁-C₅)-alkylcarbonyloxy-(C₁-C₅)-alkyl, (C₃-C₆)-cycloalkylcarbonyloxy-(C₁-C₅)-alkyl, arylcarbonyloxy-(C₁-C₅)-alkyl, heteroarylcarbonyloxy-(C₁-C₅)-alkyl, Heterocyclylcarbonyloxy-(C₁-C₅)-alkyl, (C₁-C₅)-alkoxycarbonyl, (C₁-C₅)-alkoxycarbonyl-(C₁-C₅)-alkyl, (C₂-C₅)-alkenyloxycarbonyl-(C₁-C₅)-alkyl, (C₂-C₅)-alkynyloxycarbonyl-(C₁-C₅)-alkyl, aryl-(C₁-C₅)-alkoxycarbonyl-(C₁-C₅)-alkyl, heteroaryl-(C₁-C₅)-alkoxycarbonyl-(C₁-C₅)-alkyl, heterocyclyl-(C₁-C₅)-alkoxycarbonyl-(C₁-C₅)-alkyl, hydroxy-(C₁-C₅)-alkyl, hydroxy-(C₁-C₅)-alkoxy-(C₁-C₅)-alkyl, hydroxy-(C₁-C₅)-alkoxy-(C₁-C₅)-alkoxy-(C₁-C₅)-alkyl, hydroxy-(C₁-C₅)-alkoxy-(C₁-C₅)-alkoxy-(C₁-C₅)-alkoxy-(C₁-C₅)-alkyl, (C₁-C₅)-alkylcarbonyloxy-(C₁-C₅)-alkoxy-(C₁-C₅)-alkyl, (C₁-C₅)-alkylcarbonyloxy-(C₁-C₅)-alkoxy-(C₁-C₅)-alkoxy-(C₁-C₅)-alkyl, (C₁-C₅)-alkylcarbonyloxy-(C₁-C₅)-alkoxy-(C₁-C₅)-alkoxy-(C₁-C₅)-alkoxy-(C₁-C₅)-alkyl,(C₁-C₅)-alkoxycarbonyl-(C₁-C₅)-alkoxy-(C₁-C₅)-alkyl, (C₁-C₅)-alkoxycarbonyl-(C₁-C₅)-alkoxy-(C₁-C₅)-alkoxy-(C₁-C₅)-alkyl, (C₁-C₅)-alkoxycarbonyl-(C₁-C₅)-alkoxy-(C₁-C₅)-alkoxy-(C₁-C₅)-alkoxy-(C₁-C₅)-alkyl, heteroarylcarbonyloxy-(C₁-C₅)-alkoxy-(C₁-C₅)-alkyl, heteroarylcarbonyloxy-(C₁-C₅)-alkoxy-(C₁-C₅)-alkoxy-(C₁-C₅)-alkyl, heteroarylcarbonyloxy-(C₁-C₅)-alkoxy-(C₁-C₅)-alkoxy-(C₁-C₅)-alkoxy-(C₁-C₅)-alkyl, heterocyclylcarbonyloxy-(C₁-C₅)-alkoxy-(C₁-C₅)-alkyl, heterocyclylcarbonyloxy-(C₁-C₅)-alkoxy-(C₁-C₅)-alkoxy-(C₁-C₅)-alkyl, heterocyclylcarbonyloxy-(C₁-C₅)-alkoxy-(C₁-C₅)-alkoxy-(C₁-C₅)-alkoxy-(C₁-C₅)-alkyl, hydroxycarbonyl-(C₁-C₅)-alkoxy-(C₁-C₅)-alkyl, hydroxycarbonyl-(C₁-C₅)-alkoxy-(C₁-C₅)-alkoxy-(C₁-C₅)-alkyl, or hydroxycarbonyl-(C₁-C₅)-alkoxy-(C₁-C₅)-alkoxy-(C₁-C₅)-alkoxy-(C₁-C₅)-alkyl,
- R¹¹: represents hydrogen, (C₁-C₅)-alkyl, (C₂-C₅)-alkenyl, (C₂-C₅)-alkynyl, (C₁-C₅)-cyanoalkyl, (C₁-C₅)-haloalkyl, (C₂-C₅)-haloalkenyl, (C₃-C₅)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₅)-alkoxy-(C₁-C₅)-alkyl, (C₁-C₅)-alkoxy-(C₁-C₅)-haloalkyl, aryl, aryl-(C₁-C₅)-alkyl, heteroaryl, heteroaryl-(C₁-C₅)-alkyl, heterocyclyl-(C₁-C₅)-alkyl, (C₃-C₆)-cycloalkyl-(C₁-C₅)-alkyl, (C₄-C₁₀)-cycloalkenyl-(C₁-C₅)-alkyl, bis-[(C₁-C₅)-alkyl]amino, (C₁-C₅)-alkyl-amino, aryl-(C₁-C₅)-amino, aryl-(C₁-C₅)-alkylamino, aryl-[(C₁-C₅)-alkyl]amino; heteroaryl-(C₁-C₅)-amino, heteroaryl-(C₁-C₅)-alkyl-amino, heteroaryl-[(C₁-C₅)-alkyl]amino; Hetercyclyl-(C₁-C₅)-amino, heterocyclyl-(C₁-C₅)-alkyl-amino, heterocyclyl-[(C₁-C₅)-alkyl]amino; (C₃-C₆)-cycloalkyl-amino, (C₃-C₆)-cycloalkyl-[(C₁-C₅)-alkyl]amino; N-azetidinyl, N-pyrrolidinyl, N-piperidinyl, N-morpholinyl.
- R¹² and R¹³: independently of one another represent hydrogen, NR⁸R⁹, OR¹⁰, or
- R¹² and R¹³: together with the boron atom to which they are bonded form a fully saturated or partly saturated 3- to 10-membered monocyclic or bicyclic ring optionally interrupted by heteroatoms and optionally having further substitution.

The invention more particularly provides compounds of the general formula (I) in which
- R¹: represents phenyl, 2-fluorophenyl, 3-fluorophenyl, 4-fluorophenyl, 2-chlorophenyl, 3-chlorophenyl, 4-chlorophenyl, 2-bromophenyl, 2-methylphenyl, 2-ethylphenyl, 2-methoxyphenyl, 2-trifluoromethylphenyl, 2,3-difluorophenyl, 2,4-difluorophenyl, 2,5-difluorophenyl, 2,6-difluorophenyl, 2,3,4-trifluorophenyl, 2,4,5-trifluorophenyl, 2,4,6-trifluorophenyl, 2-fluoro-6-methylphenyl, 2,4-dichlorophenyl, 2,5-dichlorophenyl, 2,6-dichlorophenyl, 3,5-dichlorophenyl, 2,6-dimethylphenyl, 2-chloro-6-methylphenyl, 2-bromo-6-fluorophenyl, 2-bromo-6-chlorophenyl, 2-bromo-6-methylphenyl, 2-bromo-6-methoxyphenyl, 2-fluoro-3-chlorophenyl, 2-fluoro-3-methylphenyl, 2-chloro-3-fluorophenyl, 2-fluoro-4-chlorophenyl, 2-fluoro-6-chlorophenyl, 2,3-dimethylphenyl, 2,4-dimethylphenyl, 2-methyl-3-fluorophenyl, 2-thienyl, 3-fluoro-2-thienyl, 3-chloro-2-thienyl, 3-bromo-2-thienyl, 3-methyl-2-thienyl, 3-methoxy-2-thienyl, 3-thienyl, 2-fluoro-3-thienyl, 2-chloro-3-thienyl, 2-bromo-3-thienyl, 2-methyl-3-thienyl, 2-methoxy-3-thienyl, 4-fluoro-3-thienyl, 4-chloro-3-thienyl, 4-bromo-3-thienyl, 4-methyl-3-thienyl, 4-methoxy-3-thienyl, 3,5-dimethyl-2-thienyl, 5-bromo-3-methyl-2-thienyl, 2,5-dimethyl-3-thienyl, 4,5-dimethyl-3-thienyl, 5-bromo-2-methyl-3-thienyl, 5-bromo-4-methyl-3-thienyl, 2,4,5-trimethyl-3-thienyl, 2,5-dibromo-4-methyl-3-thienyl, pyridin-2-yl, 3-fluoropyridin-2-yl, 3-chloropyridin-2-yl, 3-bromopyridin-2-yl, 3-methylpyridin-2-yl, 3-methoxypyridin-2-yl, 4-fluoropyridin-2-yl, 5-fluoropyridin-2-yl, 6-fluoropyridin-2-yl, pyridin-3-yl, 2-methylpyridin-3-yl, 2-fluoropyridin-3-yl, 2,4-difluoropyridin-3-yl, pyridin-4-yl, 3-fluoropyridin-4-yl, 2-fluoropyridin-4-yl, 2,3-difluoropyridin-4-yl, 4-methylthiazol-5-yl, 4-methylisothiazol-5-yl, cyclopenten-1-yl, cyclohexen-1-yl or 7-oxabicyclo[4.1.0]heptan-1-yl,
- R²: represents hydrogen, fluorine, chlorine, bromine, iodine, methyl, ethyl, n-propyl, iso-propyl, cyclopropyl, cyclobutyl, vinyl, prop-2-en-2-yl, ethynyl, prop-1-yn-1-yl, methoxy, ethoxy, methylthio, ethoxycarbonyl, 2,3,4,5,6-pentafluorophenyl, difluoromethyl or trifluoromethyl,
- n: is 0, 1, 2,
- R³: represents hydrogen, fluorine, chlorine, bromine or methyl, preferably hydrogen,
- R⁴ and R⁵: independently of one another represent hydrogen, methyl, ethyl, prop-1-yl, prop-2-yl, but-1-yl, but-2-yl, 1,1-dimethyleth-1-yl, 2-methylprop-1-yl, hydroxymethyl, hydroxyethyl, hydroxyprop-1-yl, methoxymethyl, methoxyethyl, methoxyprop-1-yl, ethoxymethyl, ethoxyethyl, ethoxyprop-1-yl, difluoromethyl, trifluoromethyl, 2,2-difluoroethyl, 3,3,3-trifluoroethyl, pentafluoroethyl, cyclopropyl, cyclobutyl, cyclopentyl, or cyclohexyl, or
- R⁴ and R⁵: together with the carbon atom to which they are bonded form a fully saturated, or partly saturated 3- to 10-membered monocyclic or bicyclic ring optionally interrupted by heteroatoms and optionally having further substitution
- R⁶: represents hydrogen, BH₂, B(NH₂)₂,4-amino-1,3,2,4-diazadiboretidin-2-yl, 4-amino-1,3,2,4-dioxadiboretan-2-yl, amino, tris(methyl)silyl, tris(ethyl)silyl, tris(prop-1-yl)silyl, tris(prop-2-yl)silyl, (1,1-dimethyleth-1-yl)(dimethyl)silyl, cyanomethyl, 2-cyanoeth-1-yl, 3-cyanoprop-1-yl, 2-cyanoprop-1-yl, methoxymethyl, 2-methoxyeth-1-yl, 2-methoxyprop-1-yl, 3-methoxyprop-1-yl, ethoxymethyl, 2-(ethoxy)eth-1-yl, 2-(ethoxy)prop-1-yl, 3-ethoxyprop-1-yl, 4-methoxybut-1-yl, 2-phenoxyeth-1-yl, 2-benzyloxyeth-1-yl, 2-(methylcarbonyloxy)eth-1-yl, 2-(ethylcarbonyloxy)eth-1-yl, 2-(iso-propylcarbonyloxy)eth-1-yl, 2-(tert-butylcarbonyloxy)eth-1-yl, 2-(phenylcarbonyloxy)eth-1-yl, 2-(2-trifluormethylphenylcarbonyl)oxyeth-1-yl, 2-(2-chlorophenylcarbonyl)oxyeth-1-yl, 2-(4-trifluormethylpyridin-3-ylcarbonyl)oxyeth-1-yl, 2-(methylsulfonyloxy)eth-1-yl, 3-(methylcarbonyloxy)prop-1-yl, 3-(ethylcarbonyloxy)prop-1-yl, 3-(iso-propylcarbonyloxy)prop-1-yl, 3-(tert-butylcarbonyloxy)prop-1-yl, 2-trifluoromethoxyeth-1-yl, methyl, ethyl, prop-1-yl, 1-methylethyl, but-1-yl, 1-methylprop-1-yl, 2-methylprop-1-yl, 1,1-dimethylethyl, pent-1-yl, 1-methylbut-1-yl, 2-methylbut-1-yl, 3-methylbut-1-yl, 1,1-dimethylprop-1-yl, 1,2-dimethylprop-1-yl, 2,2-dimethylprop-1-yl, 1-ethylprop-1-yl, prop-2-en-1-yl, but-2-en-1-yl, but-3-en-1-yl, pent-2-en-1-yl, pent-3-en-1-yl, pent-4-en-1-yl, 1-methylprop-2-en-1-yl, 2-methylprop-2-en-1-yl, 1-methylbut-2-en-1-yl, 2-methylbut-2-en-1-yl, 3-methylbut-2-en-1-yl, 1-methylbut-3-en-1-yl, 2-methylbut-3-en-1-yl, 3-methylbut-3-en-1-yl, 1,1-dimethylprop-2-en-1-yl, 1,2-dimethylprop-2-en-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methylbuty-2-n-1-yl, 1-methylbut-3-yn-1-yl, 2-methylbut-3-yn-1-yl, cyclobutyl, cyclopentyl, cyclohexyl, cyclopropylmethyl, cyclobutylmethyl, cyclopentylmethyl, cyclohexylmethyl, 2-(dimethylamino)eth-1-yl, 2-(diethylamino)eth-1-yl, 3-(dimethylamino)prop-1-yl, 3-(diethylamino)prop-1-yl, 2-(N-pyrrolidin-1-yl)eth-1-yl, 2-(N-piperidin-1-yl)eth-1-yl, tetrahydrofuran-2-ylmethyl, tetrahydrofuran-3-ylmethyl, tetrahydropyran-2-ylmethyl, tetrahydropyran-3-ylmethyl, tetrahydropyran-4-ylmethyl, oxetan-3-ylmethyl, methoxyethoxyeth-1-yl, ethoxyethoxyeth-1-yl, benzyl, 2-phenyleth-1-yl, (2-fluorophenyl)methyl, (3-fluorophenyl)methyl, (4-fluorophenyl)methyl, (2,6-difluorophenyl)methyl, (2,5-difluorophenyl)methyl, (2,4-difluorophenyl)methyl, (2,3-difluorophenyl)methyl, (2-fluoro-6-chlorophenyl)methyl, (2-chlorophenyl)methyl, (3-chlorophenyl)methyl, (4-chlorophenyl)methyl, (2,6-dichlorophenyl)methyl, (2,5-dichlorophenyl)methyl, (2,4-dichlorophenyl)methyl, (2,3-dichlorophenyl)methyl, (2-bromophenyl)methyl, (3-bromophenyl)methyl, (4-bromophenyl)methyl, (2-methylphenyl)methyl, (2-trifluoromethylphenyl)methyl, (2-methoxyphenyl)methyl, (2-nitrophenyl)methyl, (3-methylphenyl)methyl, (3-trifluoromethylphenyl)methyl, (3-methoxyphenyl)methyl, (3-nitrophenyl)methyl, (4-methylphenyl)methyl, (4-trifluoromethylphenyl)methyl, (4-methoxyphenyl)methyl, (4-nitrophenyl)methyl, pyridin-2-ylmethyl, pyridin-3-ylmethyl, pyridin-4-ylmethyl, (6-fluoropyridin-2-yl)methyl, (6-chloro-4-trifluoromethylpyridin-2-yl)methyl, (4,6-dichloropyridin-2-yl)methyl, (4-fluoropyridin-3-yl)methyl, (4-chloropyridin-3-yl)methyl, thiophen-2-ylmethyl, methoxycarbonyl, ethoxycarbonyl, 1,1-dimethyleth-1-yloxycarbonyl, benzyloxycarbonyl, (prop-2-en-1-yl)oxycarbonyl, methylaminocarbonyl, ethylaminocarbonyl, prop-1-ylaminocarbonyl, prop-2-ylaminocarbonyl, cyclopentylaminocarbonyl, cyclohexylaminocarbonyl, phenylaminocarbonyl, dimethylamincarbonyl, diethylaminocarbonyl, ethyl(methyl)aminocarbonyl, (2-fluorophenyl)aminocarbonyl, (3-fluorophenyl)aminocarbonyl, (4-fluorophenyl)aminocarbonyl, (2,6-difluorophenyl)aminocarbonyl, (2,5-difluorophenyl)aminocarbonyl, (2,4-difluorophenyl)aminocarbonyl, (2,3-difluorophenyl)aminocarbonyl, (2-fluoro-6-chlorophenyl)aminocarbonyl, (2-chlorophenyl)aminocarbonyl, (3-chlorophenyl)aminocarbonyl, (4-chlorophenyl)aminocarbonyl, (2,6-dichlorophenyl)aminocarbonyl, (2,5-dichlorophenyl)aminocarbonyl, (2,4-dichlorophenyl)aminocarbonyl, (2,3-dichlorophenyl)aminocarbonyl, (2-bromophenyl)aminocarbonyl, (3-bromophenyl)aminocarbonyl, (4-bromophenyl)aminocarbonyl, methoxycarbonylmethyl, 2-(methoxycarbonyl)eth-1-yl, 1-(methoxycarbonyl)eth-1-yl, 3-(methoxycarbonyl)prop-1-yl, 2-(methoxycarbonyl)prop-1-yl, 4-(methoxycarbonyl)but-1-yl, 5-(methoxycarbonyl)pent-1-yl, ethoxycarbonylmethyl, 2-(ethoxycarbonyl)eth-1-yl, 1-(ethoxycarbonyl)eth-1-yl, 3-(ethoxycarbonyl)prop-1-yl, 2-(ethoxycarbonyl)prop-1-yl, 4-(ethoxycarbonyl)but-1-yl, 5-(ethoxycarbonyl)pent-1-yl, benzyloxycarbonylmethyl, 2-(benzyloxycarbonyl)eth-1-yl, hydroxycarbonylmethyl, 2-(hydroxycarbonyl)eth-1-yl, 1-(hydroxycarbonyl)eth-1-yl, 3-(hydroxycarbonyl)prop-1-yl, 2-(hydroxycarbonyl)prop-1-yl, 4-(hydroxycarbonyl)but-1-yl, 5-(hydroxycarbonyl)pent-1-yl, aminocarbonylmethyl, methylaminocarbonylmethyl, ethylaminocarbonylmethyl, prop-1-ylaminocarbonylmethyl, prop-2-ylaminobarbonylmethyl, dimethylaminocarbonylmethyl, diethylaminocarbonylmethyl, benzylaminocarbonylmethyl, cyclopropylaminocarbonylmethyl, cyclobutylaminocarbonylmethyl, cyclopentylaminocarbonylmethyl, cyclohexylaminocarbonylmethyl, 2-(aminocarbonyl)eth-1-yl, 2-(methylaminocarbonyl)eth-1-yl, 2-(ethylaminocarbonyl)eth-1-yl, 2-(prop-1-ylaminocarbonyl)eth-1-yl, 2-(prop-2-ylaminobarbonyl)eth-1-yl, 2-(dimethylaminocarbonyl)eth-1-yl, 2-(diethylaminocarbonyl)eth-1-yl, 2-(benzylaminocarbonyl)eth-1-yl, 2-(cyclopropylaminocarbonyl)eth-1-yl, 2-(cyclobutylaminocarbonyl)eth-1-yl, 2-(cyclopentylaminocarbonyl)eth-1-yl, 2-(cyclohexylaminocarbonyl)eth-1-yl, 2-(methylsulfanyl)eth-1-yl, 2-(methylsulfinyl)eth-1-yl, 2-(methylsulfonyl)eth-1-yl, 2-(ethylsulfanyl)eth-1-yl, 2-(ethylsulfinyl)eth-1-yl, 2-(ethylsulfonyl)eth-1-yl, 2-(methylsulfanyl)prop-1-yl, 2-(methylsulfinyl)prop-1-yl, 2-(methylsulfonyl)prop-1-yl, methylsulfonyl, ethylsulfonyl, prop-1-ylsulfonyl, 1-methyleth-1-ylsulfonyl, but-1-ylsulfonyl, 1-methylprop-1-ylsulfonyl, 2-methylprop-1-ylsulfonyl, 1,1-dimethylethylsulfonyl, pent-1-ylsulfonyl, 1-methylbut-1-ylsulfonyl, 2-methylbut-1-ylsulfonyl, 3-methylbut-1-ylsulfonyl, 1,1-dimethylprop-1-ylsulfonyl, 1,2-dimethylprop-1-ylsulfonyl, 2,2-dimethylprop-1-ylsulfonyl, 1-ethylprop-1-ylsulfonyl, cyanomethylsulfonyl, 3-cyanoprop-1-ylsulfonyl, 1-methylcycloprop-1-ylsulfonyl, tetrahydrofuran-3-ylsulfonyl, methoxycarbonylmethylsulfonyl, ethoxycarbonylmethylsulfonyl, cyclopropylmethylsulfonyl, cyclobutylmethylsulfonyl, cyclopentylmethylsulfonyl, cyclohexylmethylsulfonyl, cyclopropylsulfonyl, cyclobutylsulfonyl, cyclopentylsulfonyl, cyclohexylsulfonyl, prop-2-en-1-ylsulfonyl, prop-2-en-2-ylsulfonyl, but-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-methylprop-2-en-1-ylsulfonyl, 2-methylprop-2-en-1-ylsulfonyl, trifluormethylsulfonyl, benzylsulfonyl, 2-phenyleth-1-ylsulfonyl, (2-fluorophenyl)methylsulfonyl, (3-fluorophenyl)methylsulfonyl, (4-fluorophenyl)methylsulfonyl, (2,6-difluorophenyl)methylsulfonyl, (2,5-difluorophenyl)methylsulfonyl, (2,4-difluorophenyl)methylsulfonyl, (2,3-difluorophenyl)methylsulfonyl, (2-fluoro-6-chlorophenyl)methylsulfonyl, (2-chlorophenyl)methylsulfonyl, (3-chlorophenyl)methylsulfonyl, (4-chlorophenyl)methylsulfonyl, (2,6-dichlorophenyl)methylsulfonyl, (2,5-dichlorophenyl)methylsulfonyl, (2,4-dichlorophenyl)methylsulfonyl, (2,3-dichlorophenyl)methylsulfonyl, (2-bromophenyl)-methylsulfonyl, (3-bromophenyl)methylsulfonyl, (4-bromophenyl)methylsulfonyl, (2-methylphenyl)methylsulfonyl, (2-trifluoromethylphenyl)methylsulfonyl, (2-methoxyphenyl)methylsulfonyl, (2-nitrophenyl)methylsulfonyl, (3-methylphenyl)methylsulfonyl, (3-trifluoromethylphenyl)methylsulfonyl, (3-methoxyphenyl)methylsulfonyl, (3-nitrophenyl)methylsulfonyl, (4-methylphenyl)methylsulfonyl, (4-trifluoromethylphenyl)methylsulfonyl, (4-methoxyphenyl)methylsulfonyl, (4-nitrophenyl)methylsulfonyl, pyridin-2-ylmethylsulfonyl, pyridin-3-ylmethylsulfonyl, pyridin-4-ylmethylsulfonyl, (6-fluoropyridin-2-yl)methylsulfonyl, (6-chloro-4-trifluoromethylpyridin-2-yl)methylsulfonyl, (4,6-dichloropyridin-2-yl)methylsulfonyl, (4-fluoropyridin-3-yl)methylsulfonyl, (4-chloropyridin-3-yl)methylsulfonyl, thiophen-2-ylmethylsulfonyl, phenylsulfonyl, (2-fluorophenyl)sulfonyl, (3-fluorophenyl)sulfonyl, (4-fluorophenyl)sulfonyl, (2,6-difluorophenyl)sulfonyl, (2,5-difluorophenyl)sulfonyl, (2,4-difluorophenyl)sulfonyl, (2,3-difluorophenyl)sulfonyl, (2-fluoro-6-chlorophenyl)sulfonyl, (2-chlorophenyl)sulfonyl, (3-chlorophenyl)sulfonyl, (4-chlorophenyl)sulfonyl, (2,6-dichlorophenyl)sulfonyl, (2,5-dichlorophenyl)sulfonyl, (2,4-dichlorophenyl)sulfonyl, (2,3-dichlorophenyl)sulfonyl, (2-bromophenyl)sulfonyl, (3-bromophenyl)sulfonyl, (4-bromophenyl)sulfonyl, (2-methylphenyl)sulfonyl, (2-trifluoromethylphenyl)sulfonyl, (2-methoxyphenyl)sulfonyl, (2-nitrophenyl)sulfonyl, (3-methylphenyl)sulfonyl, (3-trifluoromethylphenyl)sulfonyl, (3-methoxyphenyl)sulfonyl, (3-nitrophenyl)sulfonyl, (4-methylphenyl)sulfonyl, (4-trifluoromethylphenyl)sulfonyl, (4-methoxyphenyl)sulfonyl, (4-nitrophenyl)sulfonyl, pyridin-2-ylsulfonyl, pyridin-3-ylsulfonyl, pyridin-4-ylsulfonyl, (6-fluoropyridin-2-yl)sulfonyl, (6-chloro-4-trifluorosulfonylpyridin-2-yl)sulfonyl, (4,6-dichloropyridin-2-yl)sulfonyl, (4-fluoropyridin-3-yl)sulfonyl, (4-chloropyridin-3-yl)sulfonyl, thiophen-2-ylsulfonyl, methoxymethylsulfonyl, 2-(methoxy)eth-1-ylsulfonyl, 2-(methoxy)eth-1-ylsulfonyl, methylaminosulfonyl, ethylaminosulfonyl, dimethylaminosulfonyl, diethylaminosulfonyl, methyl(ethyl)aminosulfonyl, azetidin-1-ylsulfonyl, pyrrolidin-1-ylsulfonyl, piperidin-1-ylsulfonyl, N-morpholinylsulfonyl, 2-(methoxycarbonyl)eth-1-ylsulfonyl, 2-(ethoxycarbonyl)eth-1-ylsulfonyl, 3-(methoxycarbonyl)prop-1-ylsulfonyl, 3-(ethoxycarbonyl)prop-1-ylsulfonyl, 1-(methoxycarbonyl)eth-1-ylsulfonyl, 2-ethoxyeth-1-ylsulfonyl, 1-(prop-2-en-1-yl)cycloprop-1-ylsulfonyl, 3,3,3-trifluoroethylsulfonyl, tetrahydropyran-4-ylsulfonyl, tetrahydrofuran-3-ylmethylsulfonyl, formyl, methylcarbonyl, ethylcarbonyl, prop-1-ylcarbonyl, 1-methyleth-1-ylcarbonyl, but-1-ylcarbonyl, 1-methylprop-1-ylcarbonyl, 2-methylprop-1-ylcarbonyl, 1,1-dimethylethylcarbonyl, pent-1-ylcarbonyl, 1-methylbut-1-ylcarbonyl, 2-methylbut-1-ylcarbonyl, 3-methylbut-1-ylcarbonyl, 1,1-dimethylprop-1-ylcarbonyl, 1,2-dimethylprop-1-ylcarbonyl, 2,2-dimethylprop-1-ylcarbonyl, 1-ethylprop-1-ylcarbonyl, cyclopropylmethylcarbonyl, cyclobutylmethylcarbonyl, cyclopentylmethylcarbonyl, cyclohexylmethylcarbonyl, cyclopropylcarbonyl, cyclobutylcarbonyl, cyclopentylcarbonyl, cyclohexylcarbonyl, methoxymethylcarbonyl, 2-methoxyeth-1-ylcarbonyl, 2-ethoxyeth-1-ylcarbonyl, 3-methoxyprop-1-ylcarbonyl, 3-ethoxyprop-1-ylcarbonyl, methoxycarbonylmethylcarbonyl, 2-(methoxycarbonyl)eth-1-ylcarbonyl, 3-(methoxycarbonyl)prop-1-ylcarbonyl, 4-(methoxycarbonyl)but-1-ylcarbonyl, ethoxycarbonylmethylcarbonyl, 2-(ethoxycarbonyl)eth-1-ylcarbonyl, 3-(ethoxycarbonyl)prop-1-ylcarbonyl, 4-(ethoxycarbonyl)but-1-ylcarbonyl, benzylcarbonyl, 2-phenyleth-1-ylcarbonyl, (2-fluorophenyl)methylcarbonyl, (3-fluorophenyl)methylcarbonyl, (4-fluorophenyl)methylcarbonyl, (2,6-difluorophenyl)methylcarbonyl, (2,5-difluorophenyl)methylcarbonyl, (2,4-difluorophenyl)methylcarbonyl, (2,3-difluorophenyl)methylcarbonyl, (2-fluoro-6-chlorophenyl)methylcarbonyl, (2-chlorophenyl)methylcarbonyl, (3-chlorophenyl)methylcarbonyl, (4-chlorophenyl)methylcarbonyl, (2,6-dichlorophenyl)methylcarbonyl, (2,5-dichlorophenyl)methylcarbonyl, (2,4-dichlorophenyl)methylcarbonyl, (2,3-dichlorophenyl)methylcarbonyl, (2-bromophenyl)methylcarbonyl, (3-bromophenyl)methylcarbonyl, (4-bromophenyl)methylcarbonyl, (2-methylphenyl)methylcarbonyl, (2-trifluoromethylphenyl)methylcarbonyl, (2-methoxyphenyl)methylcarbonyl, (2-nitrophenyl)methylcarbonyl, (3-methylphenyl)methylcarbonyl, (3-trifluoromethylphenyl)methylcarbonyl, (3-methoxyphenyl)methylcarbonyl, (3-nitrophenyl)methylcarbonyl, (4-methylphenyl)methylcarbonyl, (4-trifluoromethylphenyl)methylcarbonyl, (4-methoxyphenyl)methylcarbonyl, (4-nitrophenyl)methylcarbonyl, pyridin-2-ylmethylcarbonyl, pyridin-3-ylmethylcarbonyl, pyridin-4-ylmethylcarbonyl, (6-fluoropyridin-2-yl)methylcarbonyl, (6-chloro-4-trifluoromethylpyridin-2-yl)methylcarbonyl, (4,6-dichloropyridin-2-yl)methylcarbonyl, (4-fluoropyridin-3-yl)methylcarbonyl, (4-chloropyridin-3-yl)methylcarbonyl, thiophen-2-ylmethylcarbonyl, phenylcarbonyl, (2-fluorophenyl)carbonyl, (3-fluorophenyl)carbonyl, (4-fluorophenyl)carbonyl, (2,6-difluorophenyl)carbonyl, (2,5-difluorophenyl)carbonyl, (2,4-difluorophenyl)carbonyl, (2,3-difluorophenyl)carbonyl, (2-fluoro-6-chlorophenyl)carbonyl, (2-chlorophenyl)carbonyl, (3-chlorophenyl)carbonyl, (4-chlorophenyl)carbonyl, (2,6-dichlorophenyl)carbonyl, (2,5-dichlorophenyl)carbonyl, (2,4-dichlorophenyl)carbonyl, (2,3-dichlorophenyl)carbonyl, (2-bromophenyl)carbonyl, (3-bromophenyl)carbonyl, (4-bromophenyl)carbonyl, (2-methylphenyl)carbonyl, (2-trifluoromethylphenyl)carbonyl, (2-methoxyphenyl)carbonyl, (2-nitrophenyl)carbonyl, (3-methylphenyl)carbonyl, (3-trifluoromethylphenyl)carbonyl, (3-methoxyphenyl)carbonyl, (3-nitrophenyl)carbonyl, (4-methylphenyl)carbonyl, (4-trifluoromethylphenyl)carbonyl, (4-methoxyphenyl)carbonyl, (4-nitrophenyl)carbonyl, (2-chloro-4-methylsulfonylphenyl)carbonyl, pyridin-2-ylcarbonyl, pyridin-3-ylcarbonyl, pyridin-4-ylcarbonyl, (6-fluoropyridin-2-yl)carbonyl, (6-chloro-4-trifluorocarbonylpyridin-2-yl)carbonyl, (4,6-dichloropyridin-2-yl)carbonyl, (4-fluoropyridin-3-yl)carbonyl, (4-chloropyridin-3-yl)carbonyl, thiophen-2-ylcarbonyl, 1,3-oxazol-2-ylcarbonyl, 1,2-oxazol-3-ylcarbonyl, 3-chloro-5-methyl-4,5-dihydro-1,2-oxazol-5-ylcarbonyl, 3-bromo-5-methyl-4,5-dihydro-1,2-oxazol-5-ylcarbonyl, 3-chloro-5-methoxymethyl-4,5-dihydro-1,2-oxazol-5-ylcarbonyl, 3-chloro-5-ethyl-4,5-dihydro-1,2-oxazol-5-ylcarbonyl, 3-chloro-3a,4,5,6-tetrahydro-6aH-cyclopenta[d][1,2]oxazol-6a-ylcarbonyl, (4-trifluoromethylpyridin-3-yl)carbonyl, 3,5-dimethyl-4,5-dihydro-1,2-oxazol-5-ylcarbonyl, 5-methyl-3-trifluoromethyl-4,5-dihydro-1,2-oxazol-5-ylcarbonyl, 3-chloro-5-hydroxymethyl-4,5-dihydro-1,2-oxazol-5-ylcarbonyl, 3-chloro-5-tert-butyldimethylsilyloxymethyl-4,5-dihydro-1,2-oxazol-5-ylcarbonyl, 3-chloro-5-(1-hydroxyeth-1-yl)-4,5-dihydro-1,2-oxazol-5-ylcarbonyl, 3-chloro-5-(1-tert-butyldimethylsilyloxyeth-1-yl-4,5-dihydro-1,2-oxazol-5-ylcarbonyl, methoxyethoxymethylcarbonyl, ethoxyethoxymethylcarbonyl, hydroxyethoxymethylcarbonyl, methoxyethoxyethoxymethylcarbonyl, ethoxyethoxyethoxymethylcarbonyl, hydroxyethoxyethoxymethylcarbonyl, 4-trifluoromethylpyridin-3-ylcarbonyloxyethoxyethoxymethylcarbonyl, methoxyethoxyethoxyethoxymethylcarbonyl, ethoxyethoxyethoxyethoxymethylcarbonyl, hydroxyethoxyethoxyethoxymethylcarbonyl, methoxycarbonylmethoxyethoxymethylcarbonyl, hydroxycarbonylmethoxyethoxymethylcarbonyl, methoxycarbonylmethoxyethoxyethoxymethylcarbonyl, hydroxycarbonylmethoxyethoxyethoxymethylcarbonyl, methoxycarbonylmethoxyethoxyethoxyethoxymethylcarbonyl, or hydroxycarbonylmethoxyethoxyethoxyethoxymethylcarbonyl,

The invention especially provides compounds of the general formula (I) in which
- R¹: represents phenyl, 2-fluorophenyl, 3-fluorophenyl, 4-fluorophenyl, 2-chlorophenyl, 3-chlorophenyl, 4-chlorophenyl, 2-bromophenyl, 2-methylphenyl, 2-ethylphenyl, 2-methoxyphenyl, 2-trifluoromethylphenyl, 2,3-difluorophenyl, 2,4-difluorophenyl, 2,5-difluorophenyl, 2,6-difluorophenyl, 2,3,4-trifluorophenyl, 2,4,5-trifluorophenyl, 2,4,6-trifluorophenyl, 2-fluoro-6-methylphenyl, 2-fluoro-3-methylphenyl 2,4-dichlorophenyl, 2,5-dichlorophenyl, 2,6-dichlorophenyl, 3,5-dichlorophenyl, 2,6-dimethylphenyl, 2-chloro-6-methylphenyl, 2-bromo-6-fluorophenyl, 2-bromo-6-chlorophenyl, 2-bromo-6-methylphenyl, 2-bromo-6-methoxyphenyl, 2-fluoro-3-chlorophenyl, 2-chloro-3-fluorophenyl, 2-fluoro-4-chlorophenyl, 2-fluoro-6-chlorophenyl, 2,3-dimethylphenyl, 2,4-dimethylphenyl, 2-methyl-3-fluorophenyl, 2-thienyl, 3-fluoro-2-thienyl, 3-chloro-2-thienyl, 3-bromo-2-thienyl, 3-methyl-2-thienyl, 3-methoxy-2-thienyl, 3-thienyl, 2-fluoro-3-thienyl, 2-chloro-3-thienyl, 2-bromo-3-thienyl, 2-methyl-3-thienyl, 2-methoxy-3-thienyl, 4-fluoro-3-thienyl, 4-chloro-3-thienyl, 4-bromo-3-thienyl, 4-methyl-3-thienyl, 4-methoxy-3-thienyl, 3,5-dimethyl-2-thienyl, 5-bromo-3-methyl-2-thienyl, 2,5-dimethyl-3-thienyl, 4,5-dimethyl-3-thienyl, 5-bromo-2-methyl-3-thienyl, 5-bromo-4-methyl-3-thienyl, 2,4,5-trimethyl-3-thienyl, 2,5-dibromo-4-methyl-3-thienyl, pyridin-2-yl, 3-fluoropyridin-2-yl, 3-chloropyridin-2-yl, 3-bromopyridin-2-yl, 3-methylpyridin-2-yl, 3-methoxypyridin-2-yl, 4-fluoropyridin-2-yl, 5-fluoropyridin-2-yl, 6-fluoropyridin-2-yl, pyridin-3-yl, 2-methylpyridin-3-yl, 2-fluoropyridin-3-yl, 2,4-difluoropyridin-3-yl, pyridin-4-yl, 3-fluoropyridin-4-yl, 2-fluoropyridin-4-yl, 2,3-difluoropyridin-4-yl, 4-methylthiazol-5-yl, 4-methylisothiazol-5-yl, cyclopenten-1-yl, cyclohexen-1-yl or 7-oxabicyclo[4.1.0]heptan-1-yl,
- R²: represents hydrogen, fluorine, chlorine, bromine, iodine, methyl, ethyl, n-propyl, iso-propyl, cyclopropyl, cyclobutyl, vinyl, prop-2-en-2-yl, ethynyl, prop-1-yn-1-yl, methoxy, ethoxy, methylthio, ethoxycarbonyl, 2,3,4,5,6-pentafluorophenyl, difluoromethyl or trifluoromethyl,
- R³: represents hydrogen,
- n: is 0, 1, 2,
- R⁴ and R⁵: independently of one another represent hydrogen, methyl, ethyl, prop-1-yl, prop-2-yl, but-1-yl, but-2-yl, 1,1-dimethyleth-1-yl, methoxymethyl, methoxyethyl, methoxyprop-1-yl, ethoxymethyl, ethoxyethyl, difluoromethyl, trifluoromethyl, cyclopropyl, cyclobutyl, or
- R⁴ and R⁵: together with the carbon atom to which they are bonded form a fully saturated, or partly saturated 3- to 10-membered monocyclic or bicyclic ring optionally interrupted by heteroatoms and optionally having further substitution, and
- R⁶: represents hydrogen, BH₂, B(NH₂)₂, 4-amino-1,3,2,4-diazadiboretidin-2-yl, 4-amino-1,3,2,4-dioxadiboretan-2-yl, amino, tris(methyl)silyl, tris(ethyl)silyl, tris(prop-1-yl)silyl, tris(prop-2-yl)silyl, (1,1-dimethyleth-1-yl)(dimethyl)silyl, methyl, ethyl, prop-1-yl, methoxycarbonylmethyl, 2-(methoxy-carbonyl)eth-1-yl, 1-(methoxycarbonyl)eth-1-yl, 3-(methoxycarbonyl)prop-1-yl, 2-(methoxycarbonyl)prop-1-yl, 4-(methoxycarbonyl)but-1-yl, 5-(methoxycarbonyl)pent-1-yl, ethoxycarbonylmethyl, 2-(ethoxycarbonyl)eth-1-yl, 1-(ethoxycarbonyl)eth-1-yl, 3-(ethoxycarbonyl)prop-1-yl, 2-(ethoxycarbonyl)prop-1-yl, 4-(ethoxycarbonyl)but-1-yl, 5-(ethoxycarbonyl)pent-1-yl, benzyloxycarbonylmethyl, 2-(benzyloxycarbonyl)eth-1-yl, hydroxycarbonylmethyl, 2-(hydroxycarbonyl)eth-1-yl, 1-(hydroxycarbonyl)eth-1-yl, 3-(hydroxycarbonyl)prop-1-yl, 2-(hydroxycarbonyl)prop-1-yl, 4-(hydroxycarbonyl)but-1-yl, 5-(hydroxycarbonyl)pent-1-yl, methylsulfonyl, ethylsulfonyl, prop-1-ylsulfonyl, 1-methyleth-1-ylsulfonyl, but-1-ylsulfonyl, 1-methylprop-1-ylsulfonyl, 2-methylprop-1-ylsulfonyl, 1,1-dimethylethylsulfonyl, pent-1-ylsulfonyl, 1-methylbut-1-ylsulfonyl, 2-methylbut-1-ylsulfonyl, 3-methylbut-1-ylsulfonyl, 1,1-dimethylprop-1-ylsulfonyl, 1,2-dimethylprop-1-ylsulfonyl, 2,2-dimethylprop-1-ylsulfonyl, 1-ethylprop-1-ylsulfonyl, cyanomethylsulfonyl, 3-cyanoprop-1-ylsulfonyl, 1-methylcycloprop-1-ylsulfonyl, tetrahydrofuran-3-ylsulfonyl, methoxycarbonyl-methylsulfonyl, ethoxycarbonylmethylsulfonyl, cyclopropylmethylsulfonyl, cyclobutylmethylsulfonyl, cyclopentylmethylsulfonyl, cyclohexylmethylsulfonyl, cyclopropylsulfonyl, cyclobutylsulfonyl, cyclopentylsulfonyl, cyclohexylsulfonyl, prop-2-en-1-ylsulfonyl, prop-2-en-2-ylsulfonyl, but-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-methylprop-2-en-1-ylsulfonyl, 2-methylprop-2-en-1-ylsulfonyl, trifluormethylsulfonyl, benzylsulfonyl, 2-phenyleth-1-ylsulfonyl, (2-fluorophenyl)methylsulfonyl, (3-fluorophenyl)methylsulfonyl, (4-fluorophenyl)methylsulfonyl, (2,6-difluorophenyl)-methylsulfonyl, (2,5-difluorophenyl)-methylsulfonyl, (2,4-difluorophenyl)methylsulfonyl, (2,3-difluorophenyl)methylsulfonyl, (2-fluoro-6-chlorophenyl)methylsulfonyl, (2-chlorophenyl)methylsulfonyl, (3-chlorophenyl)methylsulfonyl, (4-chlorophenyl)-methylsulfonyl, (2,6-dichlorophenyl)-methylsulfonyl, (2,5-dichlorophenyl)methylsulfonyl, (2,4-dichlorophenyl)methylsulfonyl, (2,3-dichlorophenyl)methylsulfonyl, (2-bromophenyl)-methylsulfonyl, (3-bromophenyl)-methylsulfonyl, (4-bromophenyl)methylsulfonyl, (2-methylphenyl)methylsulfonyl, (2-trifluoromethylphenyl)methylsulfonyl, (2-methoxyphenyl)-methylsulfonyl, (2-nitrophenyl)-methylsulfonyl, (3-methylphenyl)methylsulfonyl, (3-trifluoromethylphenyl)methylsulfonyl, (3-methoxyphenyl)methylsulfonyl, (3-nitrophenyl)methylsulfonyl, (4-methylphenyl)methyl-sulfonyl, (4-trifluoromethylphenyl)-methylsulfonyl, (4-methoxyphenyl)methylsulfonyl, (4-nitrophenyl)methylsulfonyl, pyridin-2-ylmethylsulfonyl, pyridin-3-ylmethylsulfonyl, pyridin-4-ylmethylsulfonyl, (6-fluoropyridin-2-yl)methylsulfonyl, (6-chloro-4-trifluoromethylpyridin-2-yl)methylsulfonyl, (4,6-dichloro-pyridin-2-yl)methylsulfonyl, (4-fluoropyridin-3-yl)methylsulfonyl, (4-chloropyridin-3-yl)methylsulfonyl, thiophen-2-ylmethylsulfonyl, phenylsulfonyl, (2-fluorophenyl)sulfonyl, (3-fluorophenyl)sulfonyl, (4-fluorophenyl)sulfonyl, (2,6-difluorophenyl)sulfonyl, (2,5-difluorophenyl)sulfonyl, (2,4-difluorophenyl)sulfonyl, (2,3-difluorophenyl)sulfonyl, (2-fluoro-6-chlorophenyl)sulfonyl, (2-chlorophenyl)sulfonyl, (3-chlorophenyl)sulfonyl, (4-chlorophenyl)sulfonyl, (2,6-dichlorophenyl)sulfonyl, (2,5-dichlorophenyl)sulfonyl, (2,4-dichlorophenyl)sulfonyl, (2,3-dichlorophenyl)sulfonyl, (2-bromophenyl)sulfonyl, (3-bromophenyl)sulfonyl, (4-bromophenyl)sulfonyl, (2-methylphenyl)sulfonyl, (2-trifluoromethylphenyl)sulfonyl, (2-methoxyphenyl)sulfonyl, (2-nitrophenyl)sulfonyl, (3-methylphenyl)sulfonyl, (3-trifluoromethylphenyl)sulfonyl, (3-methoxyphenyl)sulfonyl, (3-nitrophenyl)sulfonyl, (4-methylphenyl)sulfonyl, (4-trifluoromethylphenyl)sulfonyl, (4-methoxyphenyl)sulfonyl, (4-nitrophenyl)sulfonyl, pyridin-2-ylsulfonyl, pyridin-3-ylsulfonyl, pyridin-4-ylsulfonyl, (6-fluoropyridin-2-yl)sulfonyl, (6-chloro-4-trifluorosulfonylpyridin-2-yl)sulfonyl, (4,6-dichloropyridin-2-yl)sulfonyl, (4-fluoropyridin-3-yl)sulfonyl, (4-chloropyridin-3-yl)sulfonyl, thiophen-2-ylsulfonyl, methoxymethylsulfonyl, 2-(methoxy)eth-1-ylsulfonyl, 2-(methoxy)eth-1-ylsulfonyl, methylaminosulfonyl, ethylaminosulfonyl, dimethylaminosulfonyl, diethylaminosulfonyl, methyl(ethyl)aminosulfonyl, azetidin-1-ylsulfonyl, pyrrolidin-1-ylsulfonyl, piperidin-1-ylsulfonyl, N-morpholinylsulfonyl, 2-(methoxycarbonyl)eth-1-ylsulfonyl, 2-(ethoxycarbonyl)eth-1-ylsulfonyl, 3-(methoxycarbonyl)prop-1-ylsulfonyl, 3-(ethoxycarbonyl)prop-1-ylsulfonyl, 1-(methoxycarbonyl)eth-1-ylsulfonyl, 2-ethoxyeth-1-ylsulfonyl, 1-(prop-2-en-1-yl)cycloprop-1-ylsulfonyl, 3,3,3-trifluoroethylsulfonyl, tetrahydropyran-4-ylsulfonyl, tetrahydrofuran-3-ylmethylsulfonyl, formyl, methylcarbonyl, ethylcarbonyl, prop-1-ylcarbonyl, 1-methyleth-1-ylcarbonyl, but-1-ylcarbonyl, 1-methylprop-1-ylcarbonyl, 2-methylprop-1-ylcarbonyl, 1,1-dimethylethylcarbonyl, pent-1-ylcarbonyl, 1-methylbut-1-ylcarbonyl, 2-methylbut-1-ylcarbonyl, 3-methylbut-1-ylcarbonyl, 1,1-dimethylprop-1-ylcarbonyl, 1,2-dimethylprop-1-ylcarbonyl, 2,2-dimethylprop-1-ylcarbonyl, 1-ethylprop-1-ylcarbonyl, cyclopropylmethylcarbonyl, cyclobutylmethylcarbonyl, cyclopentylmethylcarbonyl, cyclohexylmethylcarbonyl, cyclopropylcarbonyl, cyclobutylcarbonyl, cyclopentylcarbonyl, cyclohexylcarbonyl, methoxymethylcarbonyl, 2-methoxyeth-1-ylcarbonyl, 2-ethoxyeth-1-ylcarbonyl, 3-methoxyprop-1-ylcarbonyl, 3-ethoxyprop-1-ylcarbonyl, methoxycarbonylmethylcarbonyl, 2-(methoxycarbonyl)eth-1-ylcarbonyl, 3-(methoxycarbonyl)prop-1-ylcarbonyl, 4-(methoxycarbonyl)but-1-ylcarbonyl, ethoxycarbonylmethylcarbonyl, 2-(ethoxycarbonyl)eth-1-ylcarbonyl, 3-(ethoxycarbonyl)prop-1-ylcarbonyl, 4-(ethoxycarbonyl)but-1-ylcarbonyl, benzylcarbonyl, 2-phenyleth-1-ylcarbonyl, (2-fluorophenyl)methylcarbonyl, (3-fluorophenyl)methylcarbonyl, (4-fluorophenyl)methylcarbonyl, (2,6-difluorophenyl)-methylcarbonyl, (2,5-difluorophenyl)methylcarbonyl, (2,4-difluorophenyl)methylcarbonyl, (2,3-difluorophenyl)methylcarbonyl, (2-fluoro-6-chlorophenyl)methylcarbonyl, (2-chlorophenyl)methylcarbonyl, (3-chlorophenyl)methylcarbonyl, (4-chlorophenyl)methylcarbonyl, (2,6-dichlorophenyl)methylcarbonyl, (2,5-dichlorophenyl)methylcarbonyl, (2,4-dichlorophenyl)-methylcarbonyl, (2,3-dichlorophenyl)methylcarbonyl, (2-bromophenyl)methylcarbonyl, (3-bromophenyl)methylcarbonyl, (4-bromophenyl)methylcarbonyl, (2-methylphenyl)-methylcarbonyl, (2-trifluoromethylphenyl)methylcarbonyl, (2-methoxyphenyl)methylcarbonyl, (2-nitrophenyl)methylcarbonyl, (3-methylphenyl)methylcarbonyl, (3-trifluoromethylphenyl)-methylcarbonyl, (3-methoxyphenyl)methylcarbonyl, (3-nitrophenyl)methylcarbonyl, (4-methylphenyl)methylcarbonyl, (4-trifluoromethylphenyl)methylcarbonyl, (4-methoxyphenyl)methylcarbonyl, (4-nitrophenyl)methylcarbonyl, pyridin-2-ylmethyl-carbonyl, pyridin-3-ylmethylcarbonyl, pyridin-4-ylmethylcarbonyl, (6-fluoropyridin-2-yl)methylcarbonyl, (6-chloro-4-trifluoromethylpyridin-2-yl)methylcarbonyl, (4,6-dichloropyridin-2-yl)methylcarbonyl, (4-fluoropyridin-3-yl)methylcarbonyl, (4-chloropyridin-3-yl)methylcarbonyl, thiophen-2-ylmethylcarbonyl, phenylcarbonyl, (2-fluorophenyl)carbonyl, (3-fluorophenyl)carbonyl, (4-fluorophenyl)carbonyl, (2,6-difluorophenyl)carbonyl, (2,5-difluorophenyl)carbonyl, (2,4-difluorophenyl)carbonyl, (2,3-difluorophenyl)carbonyl, (2-fluoro-6-chlorophenyl)carbonyl, (2-chlorophenyl)carbonyl, (3-chlorophenyl)carbonyl, (4-chlorophenyl)carbonyl, (2,6-dichlorophenyl)carbonyl, (2,5-dichlorophenyl)carbonyl, (2,4-dichlorophenyl)carbonyl, (2,3-dichlorophenyl)carbonyl, (2-bromophenyl)carbonyl, (3-bromophenyl)carbonyl, (4-bromophenyl)carbonyl, (2-methylphenyl)carbonyl, (2-trifluoromethylphenyl)carbonyl, (2-methoxyphenyl)carbonyl, (2-nitrophenyl)carbonyl, (3-methylphenyl)carbonyl, (3-trifluoromethylphenyl)carbonyl, (3-methoxyphenyl)carbonyl, (3-nitrophenyl)carbonyl, (4-methylphenyl)carbonyl, (4-trifluoromethylphenyl)carbonyl, (4-methoxyphenyl)carbonyl, (4-nitrophenyl)carbonyl, (2-chloro-4-methylsulfonylphenyl)carbonyl, pyridin-2-ylcarbonyl, pyridin-3-ylcarbonyl, pyridin-4-ylcarbonyl, (6-fluoropyridin-2-yl)carbonyl, (6-chloro-4-trifluorocarbonylpyridin-2-yl)carbonyl, (4,6-dichloropyridin-2-yl)carbonyl, (4-fluoropyridin-3-yl)carbonyl, (4-chloropyridin-3-yl)carbonyl, thiophen-2-ylcarbonyl, 1,3-oxazol-2-ylcarbonyl, 1,2-oxazol-3-ylcarbonyl, 3-chloro-5-methyl-4,5-dihydro-1,2-oxazol-5-ylcarbonyl, 3-bromo-5-methyl-4,5-dihydro-1,2-oxazol-5-ylcarbonyl, 3-chloro-5-methoxymethyl-4,5-dihydro-1,2-oxazol-5-yl-carbonyl, 3-chloro-5-ethyl-4,5-dihydro-1,2-oxazol-5-ylcarbonyl, 3-chloro-3a,4,5,6-tetrahydro-6aH-cyclopenta[d][1,2]oxazol-6a-ylcarbonyl, (4-trifluoromethylpyridin-3-yl)carbonyl, 3,5-dimethyl-4,5-dihydro-1,2-oxazol-5-ylcarbonyl, 5-methyl-3-trifluoromethyl-4,5-dihydro-1,2-oxazol-5-ylcarbonyl, 3-chloro-5-hydroxymethyl-4,5-dihydro-1,2-oxazol-5-ylcarbonyl, 3-chloro-5-tert-butyldimethylsilyloxymethyl-4,5-dihydro-1,2-oxazol-5-ylcarbonyl, 3-chloro-5-(1-hydroxyeth-1-yl)-4,5-dihydro-1,2-oxazol-5-ylcarbonyl, 3-chloro-5-(1-tert-butyldimethyl-silyloxyeth-1-yl-4,5-dihydro-1,2-oxazol-5-ylcarbonyl, methoxyethoxymethylcarbonyl, ethoxyethoxymethylcarbonyl, hydroxyethoxymethylcarbonyl, methoxyethoxyethoxymethylcarbonyl, ethoxyethoxyethoxymethylcarbonyl, hydroxyethoxyethoxymethylcarbonyl, methoxyethoxyethoxyethoxymethylcarbonyl, ethoxyethoxyethoxyethoxymethylcarbonyl, hydroxyethoxyethoxyethoxymethylcarbonyl, methoxycarbonylmethoxyethoxymethylcarbonyl, 4-trifluoromethylpyridin-3-ylcarbonyloxyethoxyethoxymethylcarbonyl, hydroxycarbonylmethoxyethoxymethylcarbonyl, methoxycarbonylmethoxyethoxyethoxymethylcarbonyl, hydroxycarbonylmethoxyethoxyethoxymethylcarbonyl, methoxycarbonylmethoxyethoxyethoxyethoxymethylcarbonyl, or hydroxycarbonylmethoxyethoxyethoxyethoxymethylcarbonyl.

The invention more especially provides compounds of the general formula (I) in which
- R¹: represents phenyl, 2-fluorophenyl, 3-fluorophenyl, 2-chlorophenyl, 2-methylphenyl, 2-ethylphenyl, 2-methoxyphenyl, 2,3-difluorophenyl, 2,4-difluorophenyl, 2,5-difluorophenyl, 2,6-difluorophenyl, 2,4,5-trifluorophenyl, 2,3,4-fluorophenyl, 2-fluoro-6-methylphenyl, 2-fluoro-3-methylphenyl, 2-fluoro-3-chlorophenyl, 2,3-dimethylphenyl, 2-thienyl, 3-chloro-2-thienyl, 3-methyl-2-thienyl, 4-methyl-3-thienyl,
- R²: represents represents hydrogen, fluorine, chlorine, bromine, iodine, methyl, ethyl, 2,3,4,5,6-pentafluorophenyl, ethoxycarbonyl, cyclopropyl,
- R³: represents hydrogen,
- n: is 0, 2,
- R⁴ and R⁵: independently of one another represent hydrogen, methyl, ethyl, prop-2-yl, methoxymethyl, cyclopropyl, cyclobutyl, or
- R⁴ and R⁵: together with the carbon atom to which they are bonded form a fully saturated, or partly saturated 3- to 10-membered monocyclic or bicyclic ring optionally interrupted by heteroatoms and optionally having further substitution, and
- R⁶: represents hydrogen, BH₂, B(NH₂)₂, 4-amino-1,3,2,4-diazadiboretidin-2-yl, 4-amino-1,3,2,4-dioxadiboretan-2-yl, methylsulfonyl, ethylsulfonyl, prop-1-ylsulfonyl, cyanomethylsulfonyl, methoxycarbonylmethylsulfonyl, 2-ethoxyeth-1-ylsulfonyl, prop-2-en-1-ylsulfonyl, prop-2-en-2-ylsulfonyl, (4-fluorophenyl)methylsulfonyl, formyl, methylcarbonyl, ethylcarbonyl, prop-1-ylcarbonyl, 1-methyleth-1-ylcarbonyl, cyclopropylcarbonyl, cyclopentylcarbonyl, 2-(methoxycarbonyl)eth-1-ylcarbonyl, ethoxycarbonylmethylcarbonyl, 3-(ethoxycarbonyl)prop-1-ylcarbonyl, (2-trifluoromethylphenyl)carbonyl, 3-chloro-5-methyl-4,5-dihydro-1,2-oxazol-5-ylcarbonyl, 3-chloro-5-methoxymethyl-4,5-dihydro-1,2-oxazol-5-ylcarbonyl, 3-chloro-3a,4,5,6-tetrahydro-6aH-cyclopenta[d][1,2]oxazol-6a-ylcarbonyl, hydroxyethoxyethoxymethylcarbonyl, methoxyethoxyethoxymethylcarbonyl, 4-trifluoromethylpyridin-3-ylcarbonyloxyethoxy-ethoxymethylcarbonyl, hydroxycarbonylmethoxyethoxymethylcarbonyl.

The invention very especially provides compounds of the general formula (I) in which
- R¹: represents phenyl, 2-fluorophenyl, 3-fluorophenyl, 2-chlorophenyl, 2-methylphenyl, 2-ethylphenyl, 2-methoxyphenyl, 2,3-difluorophenyl, 2,4-difluorophenyl, 2,6-difluorophenyl, 2-fluoro-6-methylphenyl, 2-fluoro-3-methylphenyl, 2-fluoro-3-chlorophenyl, 2,3-dimethylphenyl, 2-thienyl, 3-chloro-2-thienyl, 3-methyl-2-thienyl, or 4-methyl-3-thienyl,
- R²: represents hydrogen, fluorine, chlorine, bromine, methyl, ethyl, 2,3,4,5,6-pentafluorophenyl, or ethoxycarbonyl,
- R³: represents hydrogen,
- n: is 0
- R⁴ and R⁵: independently of one another represent hydrogen, and
- R⁶: represents hydrogen, BH₂, B(NH₂)₂, 4-amino-1,3,2,4-diazadiboretidin-2-yl, 4-amino-1,3,2,4-dioxadiboretan-2-yl, methylsulfonyl, ethylsulfonyl, prop-1-ylsulfonyl, cyanomethylsulfonyl, methoxycarbonylmethylsulfonyl, 2-ethoxyeth-1-ylsulfonyl, prop-2-en-1-ylsulfonyl, prop-2-en-2-ylsulfonyl, (4-fluorophenyl)methylsulfonyl, formyl, methylcarbonyl, ethylcarbonyl, prop-1-ylcarbonyl, 1-methyleth-1-ylcarbonyl, cyclopropylcarbonyl, cyclopentylcarbonyl, 2-(methoxy- carbonyl)eth-1-ylcarbonyl, ethoxycarbonylmethylcarbonyl, 3-(ethoxycarbonyl)prop-1-ylcarbonyl, (2-trifluoromethylphenyl)carbonyl, 3-chloro-5-methyl-4,5-dihydro-1,2-oxazol-5-ylcarbonyl, 3-chloro-5-methoxymethyl-4,5-dihydro-1,2-oxazol-5-ylcarbonyl, or 3-chloro-3a,4,5,6-tetrahydro-6aH-cyclopenta[d] [1,2]oxazol-6a-ylcarbonyl.

The definitions of radicals listed above in general terms or within areas of preference apply both to the end products of the general formula (I) and correspondingly to the starting materials or intermediates required for preparation in each case. These radical definitions can be combined with one another as desired, i.e. including combinations between the given preferred ranges.

Primarily for reasons of higher herbicidal activity, better selectivity and/or better producibility, compounds of the abovementioned general formula (I) according to the invention or their salts or their use according to the invention are of particular interest in which individual radicals have one of the preferred meanings already specified or specified below, or in particular those in which one or more of the preferred meanings already specified or specified below occur in combination.

With regard to the compounds according to the invention, the terms used above and further below will be elucidated. These are familiar to the person skilled in the art and especially have the definitions elucidated hereinafter.

Unless defined differently, names of chemical groups are generally to be understood such that attachment to the skeleton or the remainder of the molecule is via the structural element mentioned last, i.e. for example in the case of (C₂-C₈)-alkenyloxy via the oxygen atom and in the case of (C₁-C₈)-alkoxy-(C₁-C₄)-alkyl or (C₁-C₈)-alkoxycarbonyl-(C₁-C₈)-alkyl, in each case via the carbon atom of the alkyl group. In the case of specified groups, such as OR¹⁰, NR⁸R⁹, S(O)ₘR¹¹, C(=O)R¹⁰, C(=O)OR¹⁰, or C(=O)NR⁸R⁹ the attachment to the skeleton is via the structural element mentioned first, i.e. for example in the case of OR¹⁰ via the oxgyen atom and in the case of C(=O)OR¹⁰ via the carbonyl atom. According to the invention, "alkylsulfonyl" - alone or as part of a chemical group - refers to straight-chain or branched alkylsulfonyl, preferably having 1 to 8 or 1 to 6 carbon atoms, for example (but not limited to) (C₁-C₆)-alkylsulfonyl such as methylsulfonyl, ethylsulfonyl, propylsulfonyl, 1-methylethylsulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethylpropylsulfonyl, 1,2,2-trimethylpropylsulfonyl, 1-ethyl-1-methylpropylsulfonyl and 1-ethyl-2-methylpropylsulfonyl.

According to the invention, "alkylthio" - alone or as part of a chemical group - denotes straight-chain or branched S-alkyl, preferably having 1 to 8 or 1 to 6 carbon atoms, such as (C₁-C₁₀)-, (C₁-C₆)- or (C₁-C₄)-alkylthio, for example (but not limited to) (C₁-C₆)-alkylthio such as methylthio, ethylthio, propylthio, 1-methylethylthio, butylthio, 1-methylpropylthio, 2-methylpropylthio, 1,1-dimethylethylthio, pentylthio, 1-methylbutylthio, 2-methylbutylthio, 3-methylbutylthio, 1,1-dimethylpropylthio, 1,2-dimethylpropylthio, 2,2-dimethylpropylthio, 1-ethylpropylthio, hexylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and 1-ethyl-2-methylpropylthio.

According to the invention, "alkylsulfinyl (alkyl-S(=O)-)", unless defined differently elsewhere, denotes alkyl radicals which are attached to the skeleton via -S(=O)-, such as (C₁-C₁₀)-, (C₁-C₆)- or (C₁-C₄)-alkylsulfinyl, for example (but not limited to) (C₁-C₆)-alkylsulfinyl such as methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 1,1-dimethylpropylsulfinyl, 1,2-dimethylpropylsulfinyl, 2,2-dimethylpropylsulfinyl, 1-ethylpropylsulfinyl, hexylsulfinyl, 1-methylpentylsulfinyl, 2-methylpentylsulfinyl, 3-methylpentylsulfinyl, 4-methylpentylsulfinyl, 1,1-dimethylbutylsulfinyl, 1,2-dimethylbutylsulfinyl, 1,3-dimethylbutylsulfinyl, 2,2-dimethylbutylsulfinyl, 2,3-dimethylbutylsulfinyl, 3,3-dimethylbutylsulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl and 1-ethyl-2-methylpropylsulfinyl.

"Alkoxy" denotes an alkyl radical bonded via an oxygen atom, for example (but not limited to) (C₁-C₆)-alkoxy such as methoxy, ethoxy, propoxy, 1-methylethoxy, butoxy, 1-methylpropoxy, 2-methylpropoxy, 1,1-dimethylethoxy, pentoxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy. Alkenyloxy denotes an alkenyl radical attached via an oxygen atom, and alkynyloxy denotes an alkynyl radical attached via an oxygen atom, such as (C₂-C₁₀)-, (C₂-C₆)- or (C₂-C₄)-alkenoxy and (C₃-C₁₀)-, (C₃-C₆)- or (C₃-C₄)-alkynoxy.

"Cycloalkoxy" denotes a cycloalkyl radical attached via an oxygen atom and cycloalkenyloxy denotes a cycloalkenyl radical attached via an oxygen atom.

According to the invention, "alkylcarbonyl" (alkyl-C(=O)-), unless defined differently elsewhere, represents alkyl radicals attached to the skeleton via -C(=O)-, such as (C₁-C₁₀)-, (C₁-C₆)- or (C₁-C₄)-alkylcarbonyl. Here, the number of the carbon atoms refers to the alkyl radical in the alkylcarbonyl group.

Analogously, "alkenylcarbonyl" and "alkynylcarbonyl", unless defined differently elsewhere, in accordance with the invention, respectively represent alkenyl and alkynyl radicals attached to the skeleton via -C(=O)-, such as (C₂-C₁₀)-, (C₂-C₆)- or (C₂-C₄)-alkenylcarbonyl and (C₂-C₁₀)-, (C₂-C₆)- or (C₂-C₄)-alkynylcarbonyl. Here, the number of the carbon atoms refers to the alkenyl or alkynyl radical in the alkenyl or alkynyl group.

"Alkoxycarbonyl (alkyl-O-C(=O)-)," unless defined differently elsewhere: alkyl radicals attached to the skeleton via -O-C(=O)-, such as (C₁-C₁₀)-, (C₁-C₆)- or (C₁-C₄)-alkoxycarbonyl. Here, the number of the carbon atoms refers to the alkyl radical in the alkoxycarbonyl group. Analogously, "alkenyloxycarbonyl" and "alkynyloxycarbonyl", unless defined differently elsewhere, in accordance with the invention, respectively represent alkenyl and alkynyl radicals attached to the skeleton via -O-C(=O)-, such as (C₂-C₁₀)-, (C₂-C₆)- or (C₂-C₄)-alkenyloxycarbonyl and (C₃-C₁₀)-, (C₃-C₆)- or (C₃-C₄)-alkynyloxycarbonyl. Here, the number of the carbon atoms refers to the alkenyl or alkynyl radical in the alkenyloxycarbonyl or alkynyloxycarbonyl group.

The term "aryl" denotes an optionally substituted mono-, bi- or polycyclic aromatic system having preferably 6 to 14, especially 6 to 10, ring carbon atoms, for example phenyl, naphthyl, anthryl, phenanthrenyl and the like, preferably phenyl.

The term "optionally substituted aryl" also embraces polycyclic systems, such as tetrahydronaphthyl, indenyl, indanyl, fluorenyl, biphenylyl, where the bonding site is on the aromatic system. In systematic terms, "aryl" is generally also encompassed by the term "optionally substituted phenyl". Preferred aryl substituents here are, for example, hydrogen, halogen, alkyl, cycloalkyl, cycloalkylalkyl, cycloalkenyl, halocycloalkyl, alkenyl, alkynyl, aryl, arylalkyl, arylalkenyl, heteroaryl, heteroarylalkyl, heterocyclyl, heterocyclylalkyl, alkoxyalkyl, alkylthio, haloalkylthio, haloalkyl, alkoxy, haloalkoxy, cycloalkoxy, cycloalkylalkoxy, aryloxy, heteroraryloxy, alkoxyalkoxy, alkynylalkoxy, alkenyloxy, dialkylamino-alkoxy, tris-[alkyl]silyl, di-[alkyl]arylsilyl, di-[alkyl]alkylsilyl, tris-[alkyl]silylalkynyl, arylalkynyl, heteroarylalkynyl, alkylalkynyl, cycloalkylalkynyl, haloalkylalkynyl, heterocyclyl-N-alkoxy, nitro, cyano, amino, alkylamino, dialkylamino, alkylcarbonylamino, cycloalkylcarbonylamino, arylcarbonylamino, alkoxycarbonylamino, alkoxycarbonylalkylamino, arylalkoxycarbonylalkylamino, hydroxycarbonyl, alkoxycarbonyl, aminocarbonyl, alkylaminocarbonyl, cycloalkylaminocarbonyl, dialkylaminocarbonyl, heteroarylalkoxy, arylalkoxy.

A heterocyclic radical (heterocyclyl) contains at least one heterocyclic ring (=carbocyclic ring in which at least one carbon atom has been replaced by a heteroatom, preferably by a heteroatom from the group of N, O, S, P) which is saturated, unsaturated, partially saturated or heteroaromatic and may be unsubstituted or substituted, in which case the bonding site is localized on a ring atom. If the heterocyclyl radical or the heterocyclic ring is optionally substituted, it may be fused to other carbocyclic or heterocyclic rings. In the case of optionally substituted heterocyclyl, polycyclic systems are also included, for example 8-azabicyclo[3.2.1]octanyl, 8-azabicyclo[2.2.2]octanyl or 1-azabicyclo[2.2.1]heptyl. Optionally substituted heterocyclyl also includes spirocyclic systems, such as, for example, 1-oxa-5-aza-spiro[2.3]hexyl. Unless defined otherwise, the heterocyclic ring preferably contains 3 to 9 ring atoms, in particular 3 to 6 ring atoms, and one or more, preferably 1 to 4, in particular 1, 2 or 3 heteroatoms in the heterocyclic ring, preferably from the group N, O and S, where, however, two oxygen atoms must not be directly adjacent to one another, for example having one heteroatom from the group consisting of N, O and S 1- or 2- or 3-pyrrolidinyl, 3,4-dihydro-2H-pyrrol-2-or -3-yl, 2,3-dihydro-1H-pyrrol-1- or -2- or -3- or -4- or -5-yl; 2,5-dihydro-1H-pyrrol-1- or -2- or -3-yl, 1- or 2- or 3- or 4-piperidinyl; 2,3,4,5-tetrahydropyridin-2- or -3- or -4- or -5-yl or -6-yl; 1,2,3,6-tetrahydropyridin-1- or -2- or -3- or -4- or -5- or -6-yl; 1,2,3,4-tetrahydropyridin-1- or -2- or -3- or -4- or -5- or -6-yl; 1,4-dihydropyridin-1- or -2- or -3- or -4-yl; 2,3-dihydropyridin-2- or -3- or -4- or -5- or -6-yl; 2,5-dihydropyridin-2- or -3- or -4- or -5- or -6-yl, 1- or 2- or 3- or 4-azepanyl; 2,3,4,5-tetrahydro-1H-azepin-1- or -2- or -3- or -4- or -5- or -6- or -7-yl; 2,3,4,7-tetrahydro-1H-azepin-1- or -2- or -3- or -4- or -5- or -6- or -7-yl; 2,3,6,7-tetrahydro-1H-azepin-1- or -2- or -3- or -4-yl; 3,4,5,6-tetrahydro-2H-azepin-2-or -3- or -4- or -5- or -6- or -7-yl; 4,5-dihydro-1H-azepin-1- or -2- or -3- or -4-yl; 2,5-dihydro-1H-azepin-1- or -2- or -3- or -4- or -5- or -6- or -7-yl; 2,7-dihydro-1H-azepin-1- or -2- or -3- or -4-yl; 2,3-dihydro-1H-azepin-1- or -2- or -3- or -4- or -5- or -6- or -7-yl; 3,4-dihydro-2H-azepin-2- or -3- or -4- or -5- or -6- or -7-yl; 3,6-dihydro-2H-azepin-2- or -3- or -4- or -5- or -6- or -7-yl; 5,6-dihydro-2H-azepin-2-or -3- or -4- or -5- or -6- or -7-yl; 4,5-dihydro-3H-azepin-2- or -3- or -4- or -5- or -6- or -7-yl; 1H-azepin-1- or -2- or -3- or -4- or -5- or -6- or -7-yl; 2H-azepin-2- or -3- or -4- or -5- or -6- or -7-yl; 3H-azepin-2- or -3- or -4- or -5- or -6- or -7-yl; 4H-azepin-2- or -3- or -4- or -5- or -6- or -7-yl, 2- or 3-oxolanyl (= 2- or 3-tetrahydrofuranyl); 2,3-dihydrofuran-2- or -3- or -4- or -5-yl; 2,5-dihydrofuran-2- or -3-yl, 2- or 3- or 4-oxanyl (= 2- or 3- or 4-tetrahydropyranyl); 3,4-dihydro-2H-pyran-2- or -3- or -4- or - 5- or -6-yl; 3,6-dihydro-2H-pyran-2- or -3-or -4- or -5- or -6-yl; 2H-pyran-2- or -3- or -4- or -5- or -6-yl; 4H-pyran-2- or -3- or -4-yl, 2- or -3- or -4-oxepanyl; 2,3,4,5-tetrahydrooxepin-2- or -3- or -4- or -5- or - 6- or -7-yl; 2,3,4,7-tetrahydrooxepin-2- or -3- or -4- or -5- or -6- or -7-yl; 2,3,6,7-tetrahydrooxepin-2- or -3- or -4-yl; 2,3-dihydrooxepin-2- or -3- or -4- or -5- or -6- or -7-yl; 4,5-dihydrooxepin-2- or -3- or -4-yl; 2,5-dihydrooxepin-2- or -3- or -4- or -5- or -6- or -7-yl; oxepin-2- or -3- or -4- or -5- or -6- or -7-yl; 2- or 3-tetrahydrothiophenyl; 2,3-dihydrothiophen-2- or -3- or -4- or -5-yl; 2,5-dihydrothiophen-2- or -3-yl; tetrahydro-2H-thiopyran-2- or -3- or -4-yl; 3,4-dihydro-2H-thiopyran-2- or -3- or -4- or -5- or -6-yl; 3,6-dihydro-2H-thiopyran-2- or -3- or -4- or -5- or -6-yl; 2H-thiopyran-2- or -3- or -4- or -5- or -6-yl; 4H-thiopyran-2- or -3- or -4-yl. Preferred 3-membered and 4-membered heterocycles are, for example, 1- or 2-aziridinyl, oxiranyl, thiiranyl, 1- or 2- or 3-azetidinyl, 2- or 3-oxetanyl, 2- or 3-thietanyl, 1,3-dioxetan-2-yl. Further examples of "heterocyclyl" are a partially or fully hydrogenated heterocyclic radical having two heteroatoms from the group of N, O and S, for example 1- or 2- or 3- or 4-pyrazolidinyl; 4,5-dihydro-3H-pyrazol-3- or -4- or -5-yl; 4,5-dihydro-1H-pyrazol-1- or -3- or -4- or -5-yl; 2,3-dihydro-1H-pyrazol-1- or -2- or -3- or -4- or -5-yl; 1- or -2- or -3- or -4-imidazolidinyl; 2,3-dihydro-1H-imidazol-1-or -2- or -3- or -4-yl; 2,5-dihydro-1H-imidazol-1- or -2- or -4- or -5-yl; 4,5-dihydro-1H-imidazol-1- or - 2- or -4- or -5-yl; hexahydropyridazin-1- or -2- or -3- or -4-yl; 1,2,3,4-tetrahydropyridazin-1- or -2- or - 3- or -4- or -5- or -6-yl; 1,2,3,6-tetrahydropyridazin-1- or -2- or -3- or -4- or -5- or -6-yl; 1,4,5,6-tetrahydropyridazin-1- or -3- or -4- or -5- or -6-yl; 3,4,5,6-tetrahydropyridazin-3- or -4- or -5-yl; 4,5-dihydropyridazin-3- or -4-yl; 3,4-dihydropyridazin-3- or -4- or -5- or -6-yl; 3,6-dihydropyridazin-3- or - 4-yl; 1,6-dihydropyridazin-1- or -3- or -4- or -5- or -6-yl; hexahydropyrimidin-1- or -2- or -3- or -4-yl; 1,4,5,6-tetrahydropyrimidin-1- or -2- or -4- or -5- or -6-yl; 1,2,5,6-tetrahydropyrimidin-1- or -2- or -4- or -5- or -6-yl; 1,2,3,4-tetrahydropyrimidin-1- or -2- or -3- or -4- or -5- or -6-yl; 1,6-dihydropyrimidin-1- or -2- or -4- or -5- or -6-yl; 1,2-dihydropyrimidin-1- or -2- or -4- or -5- or -6-yl; 2,5-dihydropyrimidin-2- or -4- or -5-yl; 4,5-dihydropyrimidin- 4- or -5- or -6-yl; 1,4-dihydropyrimidin-1- or -2- or -4- or -5- or -6-yl; 1- or -2- or -3-piperazinyl; 1,2,3,6-tetrahydropyrazin-1- or -2- or -3- or -5- or -6-yl; 1,2,3,4-tetrahydropyrazin-1- or -2- or -3- or -4- or -5- or -6-yl; 1,2-dihydropyrazin-1- or -2- or -3- or -5- or -6-yl; 1,4-dihydropyrazin-1- or -2- or -3-yl; 2,3-dihydropyrazin-2- or -3- or -5- or -6-yl; 2,5-dihydropyrazin-2-or -3-yl; 1,3-dioxolan-2- or -4- or -5-yl; 1,3-dioxol-2- or -4-yl; 1,3-dioxan-2- or -4- or -5-yl; 4H-1,3-dioxin-2- or -4- or -5- or -6-yl; 1,4-dioxan-2- or -3- or -5- or -6-yl; 2,3-dihydro-1,4-dioxin-2- or -3- or - 5- or -6-yl; 1,4-dioxin-2- or -3-yl; 1,2-dithiolan-3- or -4-yl; 3H-1,2-dithiol-3- or -4- or -5-yl; 1,3-dithiolan-2- or -4-yl; 1,3-dithiol-2- or -4-yl; 1,2-dithian-3- or -4-yl; 3,4-dihydro-1,2-dithiin-3- or -4- or - 5- or -6-yl; 3,6-dihydro-1,2-dithiin-3- or -4-yl; 1,2-dithiin-3- or -4-yl; 1,3-dithian-2- or -4- or -5-yl; 4H-1,3-dithiin-2- or -4- or -5- or -6-yl; isoxazolidin-2- or -3- or -4- or -5-yl; 2,3-dihydroisoxazol-2- or -3- or -4- or -5-yl; 2,5-dihydroisoxazol-2- or -3- or -4- or -5-yl; 4,5-dihydroisoxazol-3- or -4- or -5-yl; 1,3-oxazolidin-2- or -3- or -4- or -5-yl; 2,3-dihydro-1,3-oxazol-2- or -3- or -4- or -5-yl; 2,5-dihydro-1,3-oxazol-2- or -4- or -5-yl; 4,5-dihydro-1,3-oxazol-2- or -4- or -5-yl; 1,2-oxazinan-2- or -3- or -4- or -5- or -6-yl; 3,4-dihydro-2H-1,2-oxazin-2- or -3- or -4- or -5- or -6-yl; 3,6-dihydro-2H-1,2-oxazin-2- or -3- or - 4- or -5- or -6-yl; 5,6-dihydro-2H-1,2-oxazin-2- or -3- or -4- or -5- or -6-yl; 5,6-dihydro-4H-1,2-oxazin-3- or -4- or -5- or -6-yl; 2H-1,2-oxazin-2- or -3- or -4- or -5- or -6-yl; 6H-1,2-oxazin-3- or -4- or -5- or - 6-yl; 4H-1,2-oxazin-3- or -4- or -5- or -6-yl; 1,3-oxazinan-2- or -3- or -4- or -5- or -6-yl; 3,4-dihydro-2H-1,3-oxazin-2- or -3- or -4- or -5- or -6-yl; 3,6-dihydro-2H-1,3-oxazin-2- or -3- or -4- or -5- or -6-yl; 5,6-dihydro-2H-1,3-oxazin-2- or -4- or -5- or -6-yl; 5,6-dihydro-4H-1,3-oxazin-2- or -4- or -5- or -6-yl; 2H-1,3-oxazin-2- or -4- or -5- or -6-yl; 6H-1,3-oxazin-2- or -4- or -5- or -6-yl; 4H-1,3-oxazin-2- or -4-or -5- or -6-yl; morpholin-2- or -3- or -4-yl; 3,4-dihydro-2H-1,4-oxazin-2- or -3- or -4- or -5- or -6-yl; 3,6-dihydro-2H-1,4-oxazin-2- or -3- or -5- or -6-yl; 2H-1,4-oxazin-2- or -3- or -5- or -6-yl; 4H-1,4-oxazin-2- or -3-yl; 1,2-oxazepan-2- or -3- or -4- or -5- or -6- or -7-yl; 2,3,4,5-tetrahydro-1,2-oxazepin-2-or -3- or -4- or -5- or -6- or -7-yl; 2,3,4,7-tetrahydro-1,2-oxazepin-2- or -3- or -4- or -5- or -6- or -7-yl; 2,3,6,7-tetrahydro-1,2-oxazepin-2- or -3- or -4- or -5- or -6- or -7-yl; 2,5,6,7-tetrahydro-1,2-oxazepin-2-or -3- or -4- or -5- or -6- or -7-yl; 4,5,6,7-tetrahydro-1,2-oxazepin-3- or -4- or -5- or -6- or -7-yl; 2,3-dihydro-1,2-oxazepin-2- or -3- or -4- or -5- or -6- or -7-yl; 2,5-dihydro-1,2-oxazepin-2- or -3- or -4- or - 5- or -6- or -7-yl; 2,7-dihydro-1,2-oxazepin-2- or -3- or -4- or -5- or -6- or -7-yl; 4,5-dihydro-1,2-oxazepin-3- or -4- or -5- or -6- or -7-yl; 4,7-dihydro-1,2-oxazepin-3- or -4- or -5- or -6- or -7-yl; 6,7-dihydro-1,2-oxazepin-3- or -4- or -5- or -6- or -7-yl; 1,2-oxazepin-3- or -4- or -5- or -6- or -7-yl; 1,3-oxazepan-2- or -3- or -4- or -5- or -6- or -7-yl; 2,3,4,5-tetrahydro-1,3-oxazepin-2- or -3- or -4- or -5- or - 6- or -7-yl; 2,3,4,7-tetrahydro-1,3-oxazepin-2- or -3- or -4- or -5- or -6- or -7-yl; 2,3,6,7-tetrahydro-1,3-oxazepin-2- or -3- or -4- or -5- or -6- or -7-yl; 2,5,6,7-tetrahydro-1,3-oxazepin-2- or -4- or -5- or -6- or - 7-yl; 4,5,6,7-tetrahydro-1,3-oxazepin-2- or -4- or -5- or -6- or -7-yl; 2,3-dihydro-1,3-oxazepin-2- or -3-or -4- or -5- or -6- or -7-yl; 2,5-dihydro-1,3-oxazepin-2- or -4- or -5- or -6- or -7-yl; 2,7-dihydro-1,3-oxazepin-2- or -4- or -5- or -6- or -7-yl; 4,5-dihydro-1,3-oxazepin-2- or -4- or -5- or -6- or -7-yl; 4,7-dihydro-1,3-oxazepin-2- or -4- or -5- or -6- or -7-yl; 6,7-dihydro-1,3-oxazepin-2- or -4- or -5- or -6- or - 7-yl; 1,3-oxazepin-2- or -4- or -5- or -6- or -7-yl; 1,4-oxazepan-2- or -3- or -5- or -6- or -7-yl; 2,3,4,5-tetrahydro-1,4-oxazepin-2- or -3- or -4- or -5- or -6- or -7-yl; 2,3,4,7-tetrahydro-1,4-oxazepin-2- or -3-or -4- or -5- or -6- or -7-yl; 2,3,6,7-tetrahydro-1,4-oxazepin-2- or -3- or -5- or -6- or -7-yl; 2,5,6,7-tetrahydro-1,4-oxazepin-2- or -3- or -5- or -6- or -7-yl; 4,5,6,7-tetrahydro-1,4-oxazepin-2- or -3- or -4-or -5- or -6- or -7-yl; 2,3-dihydro-1,4-oxazepin-2- or -3- or -5- or -6- or -7-yl; 2,5-dihydro-1,4-oxazepin-2- or -3- or -5- or -6- or -7-yl; 2,7-dihydro-1,4-oxazepin-2- or -3- or -5- or -6- or -7-yl; 4,5-dihydro-1,4-oxazepin-2- or -3- or -4- or -5- or -6- or -7-yl; 4,7-dihydro-1,4-oxazepin-2- or -3- or -4- or -5- or -6- or - 7-yl; 6,7-dihydro-1,4-oxazepin-2- or -3- or -5- or -6- or -7-yl; 1,4-oxazepin-2- or -3- or -5- or -6- or -7-yl; isothiazolidin-2- or -3- or -4- or -5-yl; 2,3-dihydroisothiazol-2- or -3- or -4- or -5-yl; 2,5-dihydroisothiazol-2- or -3- or -4- or -5-yl; 4,5-dihydroisothiazol-3- or -4- or -5-yl; 1,3-thiazolidin-2- or - 3- or -4- or -5-yl; 2,3-dihydro-1,3-thiazol-2- or -3- or -4- or -5-yl; 2,5-dihydro-1,3-thiazol-2- or -4- or -5-yl; 4,5-dihydro-1,3-thiazol-2- or -4- or -5-yl; 1,3-thiazinan-2- or -3- or -4- or -5- or -6-yl; 3,4-dihydro-2H-1,3-thiazin-2- or -3- or -4- or -5- or -6-yl; 3,6-dihydro-2H-1,3-thiazin-2- or -3- or -4- or -5- or -6-yl; 5,6-dihydro-2H-1,3-thiazin-2- or -4- or -5- or -6-yl; 5,6-dihydro-4H-1,3-thiazin-2- or -4- or -5- or -6-yl; 2H-1,3-thiazin-2- or -4- or -5- or -6-yl; 6H-1,3-thiazin-2- or -4- or -5- or -6-yl; 4H-1,3-thiazin-2- or -4-or -5- or -6-yl. Further examples of "heterocyclyl" are a partially or fully hydrogenated heterocyclic radical having 3 heteroatoms from the group of N, O and S, for example 1,4,2-dioxazolidin-2- or -3- or - 5-yl; 1,4,2-dioxazol-3- or -5-yl; 1,4,2-dioxazinan-2- or -3- or -5- or -6-yl; 5,6-dihydro-1,4,2-dioxazin-3-or -5- or -6-yl; 1,4,2-dioxazin-3- or -5- or -6-yl; 1,4,2-dioxazepan-2- or -3- or -5- or -6- or -7-yl; 6,7-dihydro-5H-1,4,2-dioxazepin-3- or -5- or -6- or -7-yl; 2,3-dihydro-7H-1,4,2-dioxazepin-2- or -3- or -5-or -6- or -7-yl; 2,3-dihydro-5H-1,4,2-dioxazepin-2- or -3- or -5- or -6- or -7-yl; 5H-1,4,2-dioxazepin-3-or -5- or -6- or -7-yl; 7H-1,4,2-dioxazepin-3- or -5- or -6- or -7-yl. Structural examples of heterocycles which are optionally substituted further are also listed below:

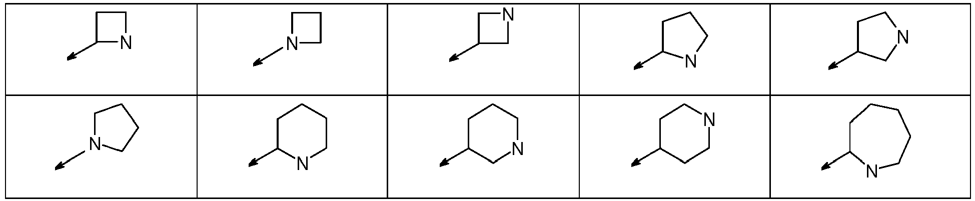

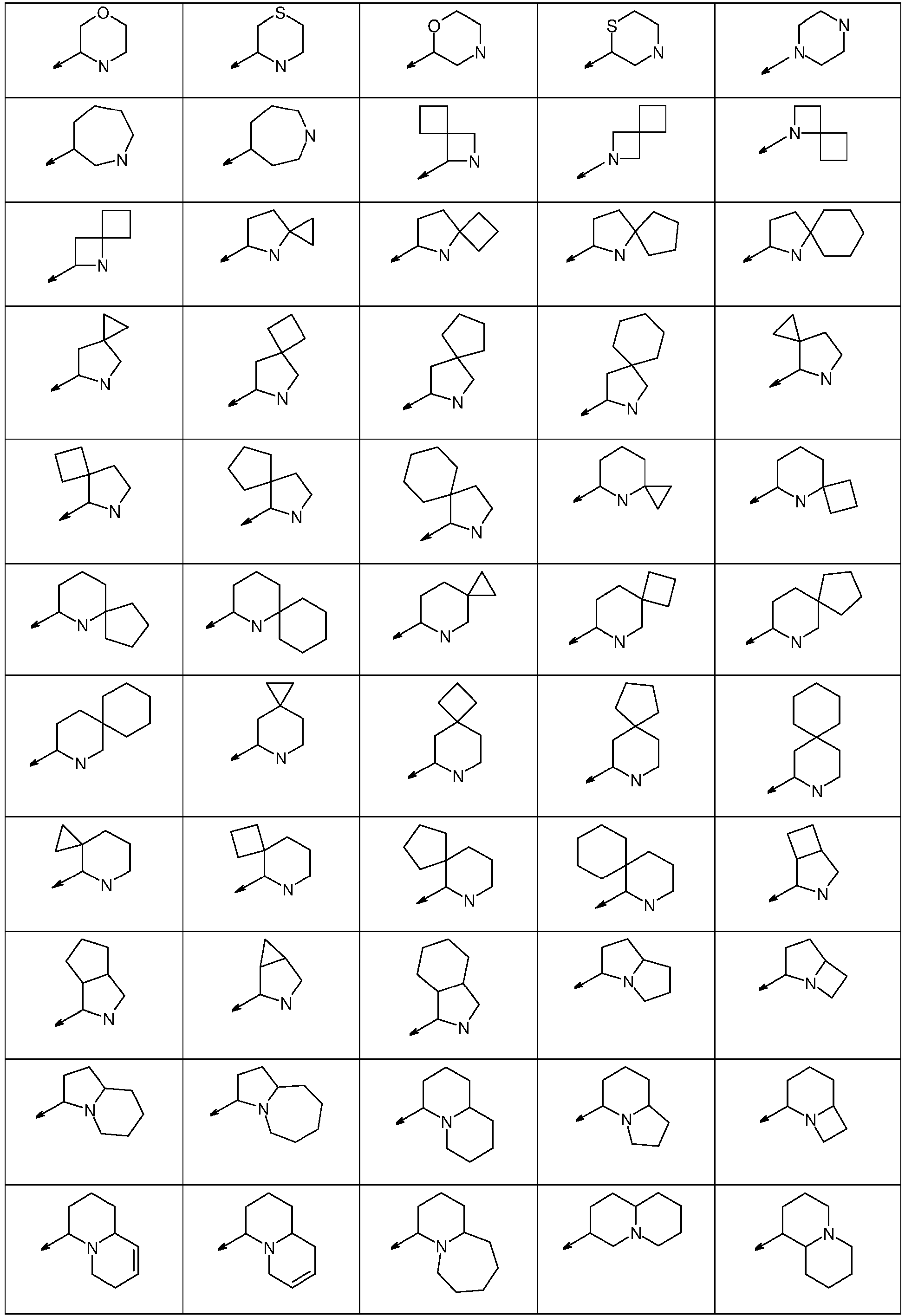

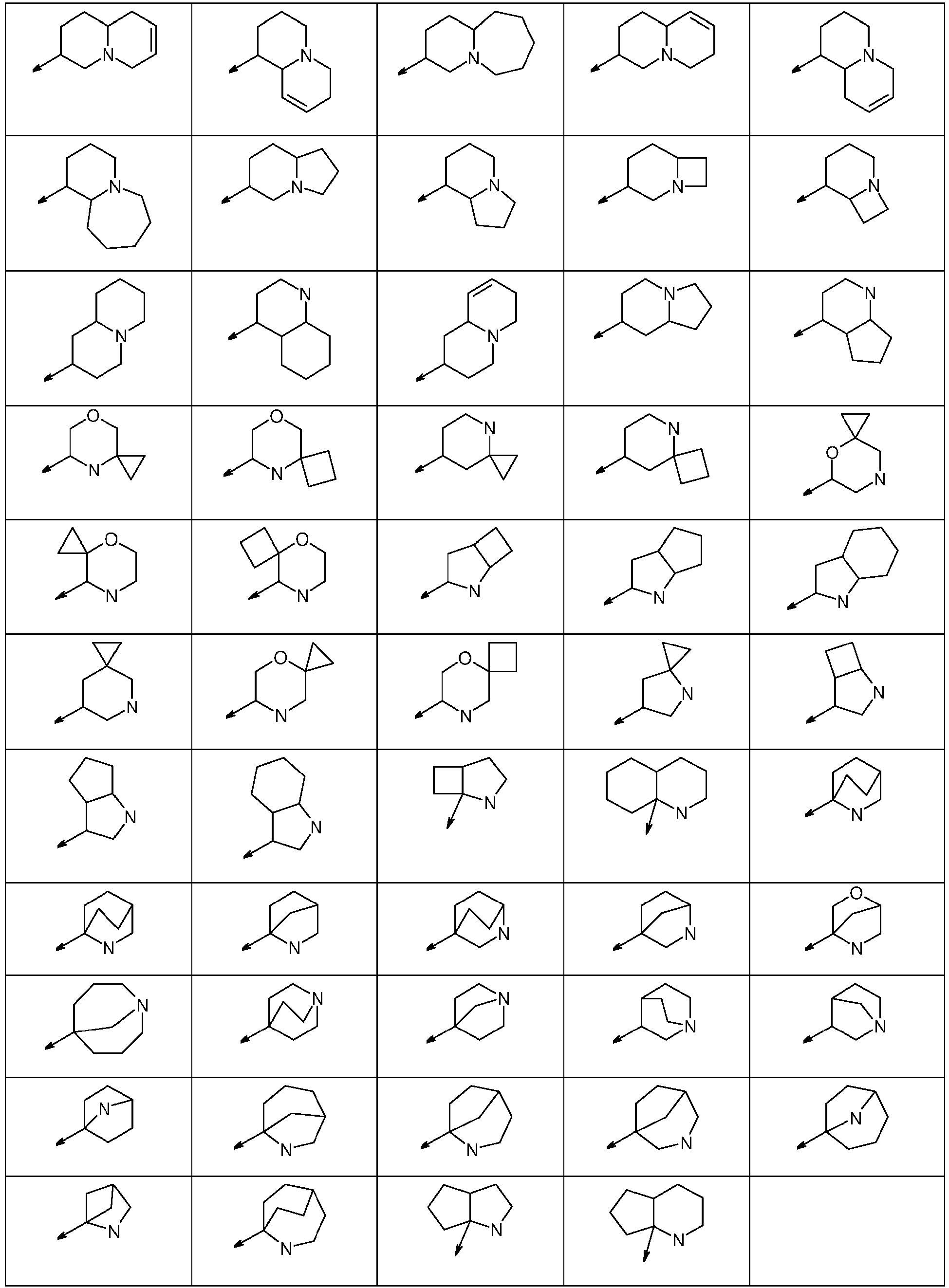

The heterocycles listed above are preferably substituted, for example, by hydrogen, halogen, alkyl, haloalkyl, hydroxyl, alkoxy, cycloalkoxy, aryloxy, alkoxyalkyl, alkoxyalkoxy, cycloalkyl, halocycloalkyl, aryl, arylalkyl, heteroaryl, heterocyclyl, alkenyl, alkylcarbonyl, cycloalkylcarbonyl, arylcarbonyl, heteroarylcarbonyl, alkoxycarbonyl, hydroxycarbonyl, cycloalkoxycarbonyl, cycloalkylalkoxycarbonyl, alkoxycarbonylalkyl, arylalkoxycarbonyl, arylalkoxycarbonylalkyl, alkynyl, alkynylalkyl, alkylalkynyl, trisalkylsilylalkynyl, nitro, amino, cyano, haloalkoxy, haloalkylthio, alkylthio, hydrothio, hydroxyalkyl, oxo, heteroarylalkoxy, arylalkoxy, heterocyclylalkoxy, heterocyclylalkylthio, heterocyclyloxy, heterocyclylthio, heteroaryloxy, dialkylamino, alkylamino, cycloalkylamino, hydroxycarbonylalkylamino, alkoxycarbonylalkylamino, arylalkoxycarbonylalkylamino, alkoxycarbonylalkyl(alkyl)amino, aminocarbonyl, alkylaminocarbonyl, dialkylaminocarbonyl, cycloalkylaminocarbonyl, hydroxycarbonylalkylaminocarbonyl, alkoxycarbonylalkylaminocarbonyl, arylalkoxycarbonylalkylaminocarbonyl.

When a base structure is substituted "by one or more substituents" from a list of radicals (= group) or a generically defined group of radicals, this in each case includes simultaneous substitution by a plurality of identical and/or structurally different radicals. In the case of a partially or fully saturated nitrogen heterocycle, this may be joined to the remainder of the molecule either via carbon or via the nitrogen. Suitable substituents for a substituted heterocyclic radical are the substituents specified further down, and additionally also oxo and thioxo. The oxo group as a substituent on a ring carbon atom is then, for example, a carbonyl group in the heterocyclic ring. As a result, lactones and lactams are preferably also included. The oxo group may also occur on the ring heteroatoms, which may exist in different oxidation states, for example in the case of N and S, and in that case form, for example, the divalent -N(O)-, -S(O)- (also SO for short) and -S(O)₂- (also SO₂ for short) groups in the heterocyclic ring. In the case of -N(O)- and -S(O)- groups, both enantiomers in each case are included.

The naming of bicyclic heterocylic moieties is carried out herein following the IUPAC rules, in particular the rules outlined in the Hantzsch-Widman nomenclature. For example, in the name 'thiazolo[2,3-b]pyridine' (cf. structure A), the numbers signify the positions of the first named heterocycle, numbered as if it were a separate entity, which are the points of ring fusion; the letter, 'b' in this case, designates the side of the second named heterocycle to which the other ring is fused, the lettering deriving from the numbering of that heterocycle as a separate entity, that is, side a is between atoms 1 and 2, side b is that between atoms 2 and 3, accordingly. Similarly, an exemplified related heterobicyclic system with a shifted nitrogen is systematically called 'thiazolo[5,4-c]pyridine' (cf. structure B) - note that the order of the numbers '5,4-' arises because the first atom of the thiazole encountered in counting round from the pyridine nitrogen to determine the side of fusion, and thus the label 'c', is C-5 of the thiazole unit. Likewise, in the name "2,3-dihydro[1,3]thiazolo[4,5-b]pyridine" (cf. structure C), the identifier "[1,3]" signifies the positions of S (sulfur) and N (nitrogen) in the thiazolo moiety, whilst "2,3-dihydro" indicates a saturated bond. Thus, the following three structures correspond to the names explained above

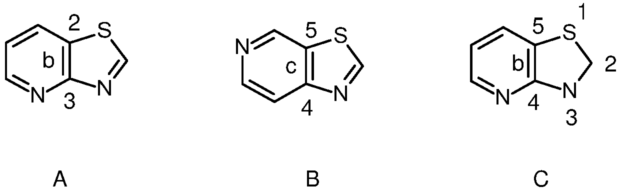

Furthermore, bridgehead atoms in partially saturated heterobicyclic systems can be named with the suffix "a", for example in 4,5,6,6a-tetrahydro-3aH-cyclopenta[d][1,2]oxazole (cf. below).

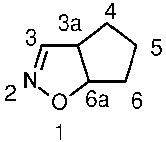

The numbering of a bi- or polycyclic system as a whole is generated from a series of rules concerned with the orientation of the rings and the positions of the nitrogen atom or atoms. These rules are outlined, for example, in "Heterocyclic Chemistry at a Glance, Second Edition. John A. Joule and Keith Mills, 2013 John Wiley & Sons, Ltd.", "A. D. McNaught, Advances in Heterocyclic Chemistry, Volume 20, 1976, Pages 175-319", or in "Brief Guide to the Nomenclature of Organic Chemistry, K.-H. Hellwich, R. M. Hartshorn, A. Yerin, T. Damhus, A. T. Hutton; IUPAC Division of Chemical Nomenclature and Structure Representation"

According to the invention, the expression "heteroaryl" refers to heteroaromatic compounds, i.e. fully unsaturated aromatic heterocyclic compounds, preferably 5- to 7-membered rings having 1 to 4, preferably 1 or 2, identical or different heteroatoms, preferably O, S or N. Inventive heteroaryls are, for example, 1H-pyrrol-1-yl; 1H-pyrrol-2-yl; 1H-pyrrol-3-yl; furan-2-yl; furan-3-yl; thien-2-yl; thien-3-yl, 1H-imidazol-1-yl; 1H-imidazol-2-yl; 1H-imidazol-4-yl; 1H-imidazol-5-yl; 1H-pyrazol-1-yl; 1H-pyrazol-3-yl; 1H-pyrazol-4-yl; 1H-pyrazol-5-yl, 1H-1,2,3-triazol-1-yl, 1H-1,2,3-triazol-4-yl, 1H-1,2,3-triazol-5-yl, 2H-1,2,3-triazol-2-yl, 2H-1,2,3-triazol-4-yl, 1H-1,2,4-triazol-1-yl, 1H-1,2,4-triazol-3-yl, 4H-1,2,4-triazol-4-yl, 1,2,4-oxadiazol-3-yl, 1,2,4-oxadiazol-5-yl, 1,3,4-oxadiazol-2-yl, 1,2,3-oxadiazol-4-yl, 1,2,3-oxadiazol-5-yl, 1,2,5-oxadiazol-3-yl, azepinyl, pyridin-2-yl, pyridin-3-yl, pyridin-4-yl, pyrazin-2-yl, pyrazin-3-yl, pyrimidin-2-yl, pyrimidin-4-yl, pyrimidin-5-yl, pyridazin-3-yl, pyridazin-4-yl, 1,3,5-triazin-2-yl, 1,2,4-triazin-3-yl, 1,2,4-triazin-5-yl, 1,2,4-triazin-6-yl, 1,2,3-triazin-4-yl, 1,2,3-triazin-5-yl, 1,2,4-, 1,3,2-, 1,3,6- and 1,2,6-oxazinyl, isoxazol-3-yl, isoxazol-4-yl, isoxazol-5-yl, 1,3-oxazol-2-yl, 1,3-oxazol-4-yl, 1,3-oxazol-5-yl, isothiazol-3-yl, isothiazol-4-yl, isothiazol-5-yl, 1,3-thiazol-2-yl, 1,3-thiazol-4-yl, 1,3-thiazol-5-yl, oxepinyl, thiepinyl, 1,2,4-triazolonyl and 1,2,4-diazepinyl, 2H-1,2,3,4-tetrazol-5-yl, 1H-1,2,3,4-tetrazol-5-yl, 1,2,3,4-oxatriazol-5-yl, 1,2,3,4-thiatriazol-5-yl, 1,2,3,5-oxatriazol-4-yl, 1,2,3,5-thiatriazol-4-yl. The heteroaryl groups according to the invention may also be substituted by one or more identical or different radicals. If two adjacent carbon atoms are part of a further aromatic ring, the systems are fused heteroaromatic systems, such as benzofused or polyannealed heteroaromatics. Preferred examples are quinolines (e.g. quinolin-2-yl, quinolin-3-yl, quinolin-4-yl, quinolin-5-yl, quinolin-6-yl, quinolin-7-yl, quinolin-8-yl); isoquinolines (e.g. isoquinolin-1-yl, isoquinolin-3-yl, isoquinolin-4-yl, isoquinolin-5-yl, isoquinolin-6-yl, isoquinolin-7-yl, isoquinolin-8-yl); quinoxaline; quinazoline; cinnoline; 1,5-naphthyridine; 1,6-naphthyridine; 1,7-naphthyridine; 1,8-naphthyridine; 2,6-naphthyridine; 2,7-naphthyridine; phthalazine; pyridopyrazines; pyridopyrimidines; pyridopyridazines; pteridines; pyrimidopyrimidines. Examples of heteroaryl are also 5- or 6-membered benzofused rings from the group of 1H-indol-1-yl, 1H-indol-2-yl, 1H-indol-3-yl, 1H-indol-4-yl, 1H-indol-5-yl, 1H-indol-6-yl, 1H-indol-7-yl, 1-benzofuran-2-yl, 1-benzofuran-3-yl, 1-benzofuran-4-yl, 1-benzofuran-5-yl, 1-benzofuran-6-yl, 1-benzofuran-7-yl, 1-benzothiophen-2-yl, 1-benzothiophen-3-yl, 1-benzothiophen-4-yl, 1-benzothiophen-5-yl, 1-benzothiophen-6-yl, 1-benzothiophen-7-yl, 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 2H-indazol-3-yl, 2H-indazol-4-yl, 2H-indazol-5-yl, 2H-indazol-6-yl, 2H-indazol-7-yl, 2H-isoindol-2-yl, 2H-isoindol-1-yl, 2H-isoindol-3-yl, 2H-isoindol-4-yl, 2H-isoindol-5-yl, 2H-isoindol-6-yl; 2H-isoindol-7-yl, 1H-benzimidazol-1-yl, 1H-benzimidazol-2-yl, 1H-benzimidazol-4-yl, 1H-benzimidazol-5-yl, 1H-benzimidazol-6-yl, 1H-benzimidazol-7-yl, 1,3-benzoxazol-2-yl, 1,3-benzoxazol-4-yl, 1,3-benzoxazol-5-yl, 1,3-benzoxazol-6-yl, 1,3-benzoxazol-7-yl, 1,3-benzothiazol-2-yl, 1,3-benzothiazol-4-yl, 1,3-benzothiazol-5-yl, 1,3-benzothiazol-6-yl, 1,3-benzothiazol-7-yl, 1,2-benzisoxazol-3-yl, 1,2-benzisoxazol-4-yl, 1,2-benzisoxazol-5-yl, 1,2-benzisoxazol-6-yl, 1,2-benzisoxazol-7-yl, 1,2-benzisothiazol-3-yl, 1,2-benzisothiazol-4-yl, 1,2-benzisothiazol-5-yl, 1,2-benzisothiazol-6-yl, 1,2-benzisothiazol-7-yl.

The term "halogen" denotes, for example, fluorine, chlorine, bromine or iodine. If the term is used for a radical, "halogen" denotes, for example, a fluorine, chlorine, bromine or iodine atom.

According to the invention, "alkyl" denotes a straight-chain or branched open-chain, saturated hydrocarbon radical which is optionally mono- or polysubstituted, and in the latter case is referred to as "substituted alkyl". Preferred substituents are halogen atoms, alkoxy, haloalkoxy, cyano, alkylthio, haloalkylthio, amino or nitro groups, particular preference being given to methoxy, methyl, fluoroalkyl, cyano, nitro, fluorine, chlorine, bromine or iodine.

The prefix "di" includes the combination of equal or different alkyl radicals, e.g. dimethyl or methyl(ethyl) or ethyl(methyl).

"Haloalkyl", "-alkenyl" and "-alkynyl" respectively denote alkyl, alkenyl and alkynyl partially or fully substituted by identical or different halogen atoms, for example monohaloalkyl such as CH₂CH₂Cl, CH₂CH₂Br, CHClCH₃, CH₂Cl, CH₂F; perhaloalkyl such as CCl₃, CClF₂, CFCl₂, CF₂CClF₂, CF₂CClFCF₃; polyhaloalkyl such as CH₂CHFCl, CF₂CClFH, CF₂CBrFH, CH₂CF₃; the term perhaloalkyl also encompasses the term perfluoroalkyl.

"Haloalkoxy" is, for example, OCF₃, OCHF₂, OCH₂F, OCF₂CF₃, OCH₂CF₃ and OCH₂CH₂Cl; this applies correspondingly to haloalkenyl and other halogen-substituted radicals.

The expression "(C₁-C₄)-alkyl" mentioned here by way of example is a brief notation for straight-chain or branched alkyl having one to 4 carbon atoms according to the range stated for carbon atoms, i.e. encompasses the methyl, ethyl, 1-propyl, 2-propyl, 1-butyl, 2-butyl, 2-methylpropyl or tert-butyl radicals. General alkyl radicals with a larger specified range of carbon atoms, e.g. "(C₁-C₆)-alkyl", correspondingly also encompass straight-chain or branched alkyl radicals with a greater number of carbon atoms, i.e. according to the example also the alkyl radicals having 5 and 6 carbon atoms.

Unless stated specifically, preference is given to the lower carbon skeletons, for example having from 1 to 6 carbon atoms, or having from 2 to 6 carbon atoms in the case of unsaturated groups, in the case of the hydrocarbyl radicals such as alkyl, alkenyl and alkynyl radicals, including in composite radicals. Alkyl radicals, including in composite radicals such as alkoxy, haloalkyl, etc., are, for example, methyl, ethyl, n-propyl or i-propyl, n-, i-, t- or 2-butyl, pentyls, hexyls such as n-hexyl, i-hexyl and 1,3-dimethylbutyl, heptyls such as n-heptyl, 1-methylhexyl and 1,4-dimethylpentyl; alkenyl and alkynyl radicals are defined as the possible unsaturated radicals corresponding to the alkyl radicals, where at least one double bond or triple bond is present. Preference is given to radicals having one double bond or triple bond.

The term "alkenyl" also includes, in particular, straight-chain or branched open-chain hydrocarbon radicals having more than one double bond, such as 1,3-butadienyl and 1,4-pentadienyl, but also allenyl or cumulenyl radicals having one or more cumulated double bonds, for example allenyl (1,2-propadienyl), 1,2-butadienyl and 1,2,3-pentatrienyl. Alkenyl denotes, for example, vinyl which may optionally be substituted by further alkyl radicals, for example (but not limited thereto) (C₂-C₆)-alkenyl such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl.

The term "alkynyl" also includes, in particular, straight-chain or branched open-chain hydrocarbon radicals having more than one triple bond, or else having one or more triple bonds and one or more double bonds, for example 1,3-butatrienyl or 3-penten-1-yn-1-yl. (C₂-C₆)-Alkynyl denotes, for example, ethynyl, 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl.

The term "cycloalkyl" denotes a carbocyclic saturated ring system having preferably 3-8 ring carbon atoms, for example cyclopropyl, cyclobutyl, cyclopentyl or cyclohexyl, which optionally has further substitution, preferably by hydrogen, alkyl, alkoxy, cyano, nitro, alkylthio, haloalkylthio, halogen, alkenyl, alkynyl, haloalkyl, amino, alkylamino, dialkylamino, alkoxycarbonyl, hydroxycarbonyl, arylalkoxycarbonyl, aminocarbonyl, alkylaminocarbonyl, cycloalkylaminocarbonyl. In the case of optionally substituted cycloalkyl, cyclic systems with substituents are included, also including substituents with a double bond on the cycloalkyl radical, for example an alkylidene group such as methylidene. In the case of optionally substituted cycloalkyl, polycyclic aliphatic systems are also included, for example bicyclo[1.1.0]butan-1-yl, bicyclo[1.1.0]butan-2-yl, bicyclo[2.1.0]pentan-1-yl, bicyclo[1.1.1]pentan-1-yl, bicyclo[2.1.0]pentan-2-yl, bicyclo[2.1.0]pentan-5-yl, bicyclo[2.1.1]hexyl, bicyclo[2.2.1]hept-2-yl, bicyclo[2.2.2]octan-2-yl, bicyclo[3.2.1]octan-2-yl, bicyclo[3.2.2]nonan-2-yl, adamantan-1-yl and adamantan-2-yl, but also systems such as 1,1'-bi(cyclopropyl)-1-yl, 1,1'-bi(cyclopropyl)-2-yl, for example. The term "(C₃-C₇)-cycloalkyl" is a brief notation for cycloalkyl having three to 7 carbon atoms, corresponding to the range specified for carbon atoms.

In the case of substituted cycloalkyl, spirocyclic aliphatic systems are also included, for example spiro[2.2]pent-1-yl, spiro[2.3]hex-1-yl, spiro[2.3]hex-4-yl, 3-spiro[2.3]hex-5-yl, spiro[3.3]hept-1-yl, spiro[3.3]hept-2-yl.

"Cycloalkenyl" denotes a carbocyclic, nonaromatic, partially unsaturated ring system having preferably 4-8 carbon atoms, e.g. 1-cyclobutenyl, 2-cyclobutenyl, 1-cyclopentenyl, 2-cyclopentenyl, 3-cyclopentenyl, or 1-cyclohexenyl, 2-cyclohexenyl, 3-cyclohexenyl, 1,3-cyclohexadienyl or 1,4-cyclohexadienyl, also including substituents with a double bond on the cycloalkenyl radical, for example an alkylidene group such as methylidene. In the case of optionally substituted cycloalkenyl, the elucidations for substituted cycloalkyl apply correspondingly.

According to the invention, "haloalkylthio" - on its own or as constituent part of a chemical group - represents straight-chain or branched S-haloalkyl, preferably having 1 to 8, or having 1 to 6 carbon atoms, such as (C₁-C₈)-, (C₁-C₆)- or (C₁-C₄)-haloalkylthio, for example (but not limited thereto) trifluoromethylthio, pentafluoroethylthio, difluoromethyl, 2,2-difluoroeth-1-ylthio, 2,2,2-difluoroeth-1-ylthio, 3,3,3-prop-1-ylthio.

"Halocycloalkyl" and "halocycloalkenyl" denote cycloalkyl and cycloalkenyl, respectively, which are partially or fully substituted by identical or different halogen atoms, such as F, Cl and Br, or by haloalkyl, such as trifluoromethyl or difluoromethyl, for example 1-fluorocycloprop-1-yl, 2-fluoro-cycloprop-1-yl, 2,2-difluorocycloprop-1-yl, 1-fluorocyclobut-1-yl, 1-trifluoromethylcycloprop-1-yl, 2-trifluoromethylcycloprop-1-yl, 1-chlorocycloprop-1-yl, 2-chlorocycloprop-1-yl, 2,2-dichlorocyclo-prop-1-yl, 3,3-difluorocyclobutyl.

According to the invention, "trialkylsilyl" - on its own or as constituent part of a chemical group - represents straight-chain or branched Si-alkyl, preferably having 1 to 8, or having 1 to 6 carbon atoms, such as tri[(C₁-C₈)-, (C₁-C₆)- or (C₁-C₄)-alkyl]silyl, for example (but not limited thereto) trimethylsilyl, triethylsilyl, tri(n-propyl)silyl, tri(isopropyl)silyl, tri(n-butyl)silyl, tri(1-methylprop-1-yl)silyl, tri(2-methylprop-1-yl)silyl, tri(1,1-dimethyleth-1-yl)silyl, tri(2,2-dimethyleth-1-yl)silyl.

If the compounds can form, through a hydrogen shift, tautomers whose structure is not formally covered by the general formula (I), these tautomers are nevertheless covered by the definition of the inventive compounds of the general formula (I), unless a particular tautomer is under consideration. For example, many carbonyl compounds may be present both in the keto form and in the enol form, both forms being encompassed by the definition of the compound of the general formula (I).

Depending on the nature of the substituents and the manner in which they are attached, the compounds of the general formula (I) may be present as stereoisomers. The general formula (I) embraces all possible stereoisomers defined by the specific three-dimensional form thereof, such as enantiomers, diastereomers, Z and E isomers. If, for example, one or more alkenyl groups are present, diastereomers (Z and E isomers) may occur. If, for example, one or more asymmetric carbon atoms are present, enantiomers and diastereomers may occur. Stereoisomers can be obtained from the mixtures obtained in the preparation by customary separation methods. The chromatographic separation can be affected either on the analytical scale to find the enantiomeric excess or the diastereomeric excess, or else on the preparative scale to produce test specimens for biological testing. It is likewise possible to selectively prepare stereoisomers by using stereoselective reactions with use of optically active starting materials and/or auxiliaries. The invention thus also relates to all stereoisomers which are embraced by the general formula (I) but are not shown in their specific stereomeric form, and to mixtures thereof.

If the compounds are obtained as solids, the purification can also be carried out by recrystallization or digestion. If individual compounds of general formula (I) cannot be obtained in a satisfactory manner by the routes described below, they can be prepared by derivatization of other compounds of general formula (I).

Suitable isolation methods, purification methods and methods for separating stereoisomers of compounds of the general formula (I) are methods generally known to the person skilled in the art from analogous cases, for example by physical processes such as crystallization, chromatographic methods, in particular column chromatography and HPLC (high pressure liquid chromatography), distillation, optionally under reduced pressure, extraction and other methods, any mixtures that remain can generally be separated by chromatographic separation, for example on chiral solid phases. Suitable for preparative amounts or on an industrial scale are processes such as crystallization, for example of diastereomeric salts which can be obtained from the diastereomer mixtures using optically active acids and, if appropriate, provided that acidic groups are present, using optically active bases.

Synthesis of substituted 2,3-dihydro[1,3]thiazolo[4,5-b]pyridines of the general formula (I).

The substituted 2,3-dihydro[1,3]thiazolo[4,5-b]pyridines of the general formula (I) according to the invention can be prepared using known processes from key thiazolopyridine intermediates (II). The synthesis routes used and examined for the synthesis of the key thiazolopyridines (II) proceed from commercially available or easily synthesised substituted thiazoles or substituted pyridines. In the following schemes, the groups R¹, R², R³, R⁴, R⁵ and R⁶ of the general formula (I) have the meanings defined above, unless exemplary, but not limiting definitions are given. The synthesis route for substituted 2,3-dihydro[1,3]thiazolo[4,5-b]pyridines of the general formula (I) proceeds via the reaction of optionally substituted thiazolopyridines (II) with a suitable nucleophilic reagent (e.g. ammonia-borane complex) and ligand system (e.g. B(C₆F₅)₃ = tris(pentafluorophenyl)borane) in an appropriate solvent (e.g. toluene) (cf. Adv. Synth. Catal., 2019, 361, 2301-2308) (Scheme 1). The afforded 2,3-dihydro[1,3]thiazolo[4,5-b]pyridines of the general formula (I), where R⁶ = hydrogen, can then be further transformed into substituted 2,3-dihydro[1,3]thiazolo[4,5-b]pyridines (I) by installation of the R⁶ moiety via either alkylation, acylation or sulfonylation reactions using a suitable base (e.g. triethylamine) and solvent (e.g. dichloromethane) system. the furnishes compounds of the general formula (I). In Scheme 1 below, R¹, R², R³ and R⁶ have the meanings defined above. In thiazolopyridine (II) R⁴ represents hydrogen, halogen or sulfone groups. In pyridothiazoline (I) R⁴ by way of example, but not by limitation represents hydrogen. R⁵ by way of example, but not by limitation represent hydrogen and X = halogen.

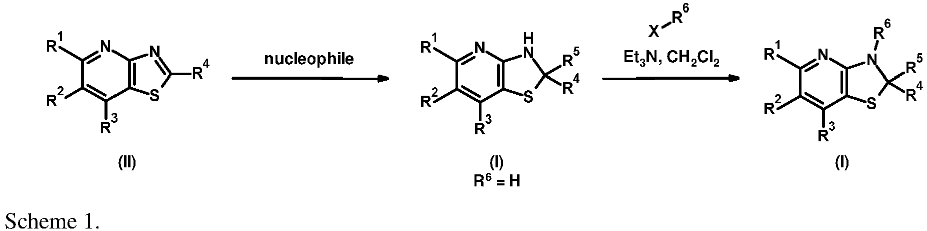

The key thiazolopyridine intermediates (II) can be prepared using a variety of synthetic methods. The first synthesis route for substituted thiazolopyridines (II) proceeds via an optionally substituted Boc-protected aminothiazole (III) (Scheme 2). To this end, a substituted methylcarboxylate aminothiazole is protected (e.g. with Boc₂O and DMAP = 4-dimethylaminopyridine, where Boc = tert-butyloxycarbonyl) and then reduced to the corresponding alcohol (V) with a suitable nucleophilic reagent (e.g. lithium aluminium hydride or methylmagnesium chloride). Subsequent oxidation of the alcohol to the corresponding carbonyl group using a suitable oxidizing agent (e.g. manganese dioxide) afforded the optionally substituted Boc-protected aminothiazole (VI). To complete the synthesis of thiazolopyridines (II), the optionally substituted Boc-protected aminothiazole (VI) is further reacted with an optionally substituted phosphonium salt (cf. Org. Lett., 1999, 1, 1579-1581) in the presence of a base (e.g. potassium tert-butoxide) followed by Boc-deprotection and cyclisation under acidic conditions (e.g. TFA = trifluoroacetic acid) in a suitable polar-aprotic solvent (e.g. dichloromethane) (cf Org. Lett., 2016, 18, 1562-1565). During the synthesis of compounds (VIII) the corresponding *trans* and *cis-*isomers could be isolated, as well as isomeric mixtures of differing ratios. In Scheme 2 below, R¹ has the meanings defined above. R², R³ and R⁴, by way of example, but not by limitation represent hydrogen.

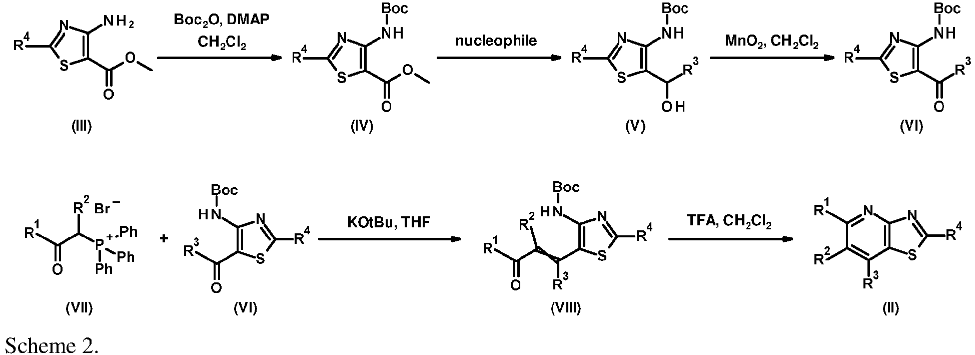

The synthesis of the thiazolopyridines (II) can, in addition, be completed via a Friedländer style reaction of substituted aminothiazoles (IX) with substituted ketones (Scheme 2). Following this method, Boc-protected thiazole (VI) is deprotected in the presence of a mild acid in a suitable solvent (e.g. silica gel and EtOAc, where Boc = tert-butyloxycarbonyl) to a substituted aminothiazole (IX). This compound then undergoes a cyclisation reaction with a substituted ketone (X) in the presence of a suitable base (e.g. potassium hydroxide) in an appropriate solvent (e.g. methanol) to afford thiazolopyridines (II) (cf. US1998/5723413). In Scheme 3 below, R¹ and R² have the meanings defined above. R³ and R⁴, by way of example, but not by limitation represent hydrogen.

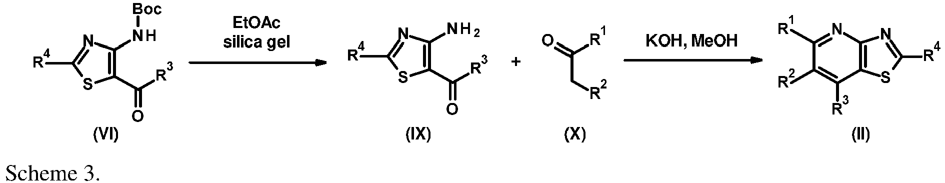

The synthesis of substituted thiazolopyridines (II) can also be accomplished starting from substituted hydroxypyridines (Scheme 4). Using this method, a substituted hydroxypyridine (XI) is nitrated (e.g. with sulfuric acid and nitric acid) and then coupled to a boronic acid in a palladium catalysed cross coupling reaction using a suitable palladium complex (e.g. Pd(dppf)Cl₂), an appropriate base (e.g. potassium carbonate) and a suitable aprotic solvent (e.g. toluene, 1,4-dioxane or DME = dimethoxyethane) (cf. WO2015/123133). The free hydroxy group of compound (XIV) is then reacted with N,N-dimethylcarbamothioyl chloride in the presence of a suitable base (e.g. DBU = 1,8-diazabicycloundec-7-ene) followed by a Newmann-quart rearrangement facilitated by heating in an appropriate solvent (e.g. xylene) to afford pyridine (XVI) (cf. Bioorg. Med. Chem., 2013, 21, 6804-6820). Reduction of the nitro group to an amino group is accomplished in the presence of a suitable reagent system (e.g. iron powder and ammonium chloride) followed by removal of the formamide group to afford disulphide (XVIII). Reagents that can accomplish the formamide deprotection include a mixture of potassium hydroxide, lithium hydroxide and lithium aluminium hydride (cf. Chem. Eur. J., 2018, 24, 4864-487; WO2016/120403). Lastly, cyclisation using an appropriate reaction partner (e.g. triethyl orthoformate) in the presence of a suitable acid catalyst (e.g. p-TsOH = *para*-toluenesulphonic acid) completed the synthesis route to the key thiazolopyridine intermediates (II) (cf. WO2018/203235). In Scheme 4 below, R¹, R² and R³ have the meanings defined above and X = halogen. R⁴, by way of example, but not by limitation represents hydrogen.

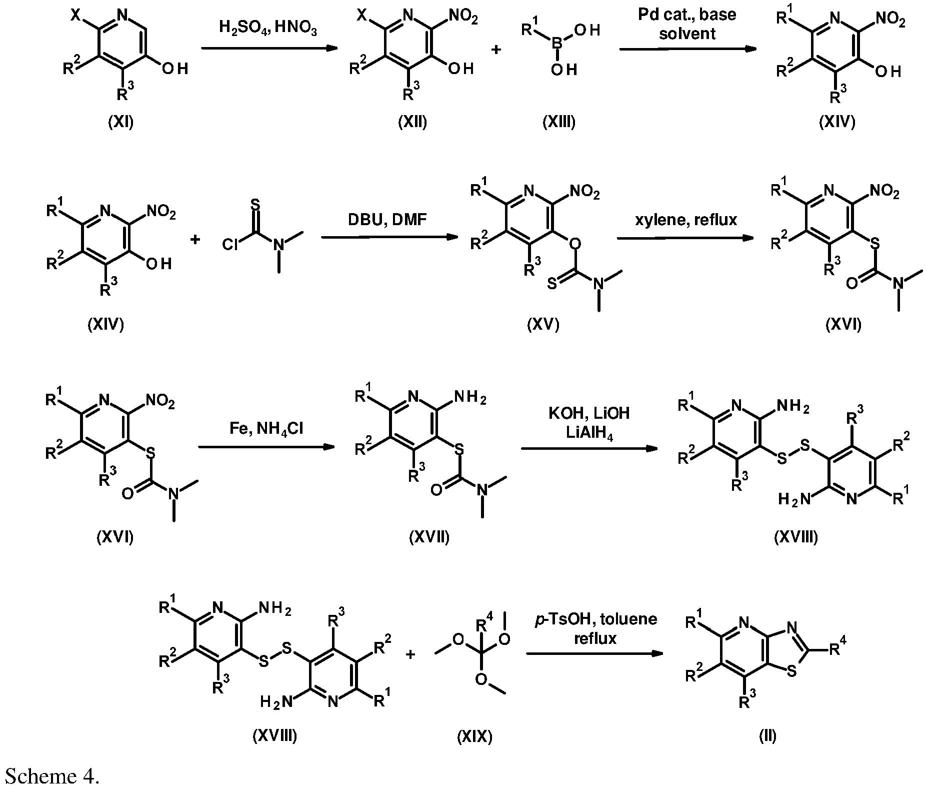

The synthesis of substituted thiazolopyridines (II) is furthermore described starting from substituted aminopyridines (Scheme 5). To this end, a halogenated aminopyridine (XX) is coupled to a boronic acid in a palladium catalysed cross coupling reaction using a suitable palladium complex (e.g. Pd(dppf)Cl₂), an appropriate base (e.g. potassium carbonate) and a suitable aprotic solvent (e.g. toluene, 1,4-dioxane or DME = dimethoxyethane). The resulting product (XXI) is then dihalogenated using an appropriate reagent (e.g. NBS = *N*-bromosuccinimide or NCS = *N*-chlorosuccinimide) in a polar solvent (e.g. acetonitrile) to afford compound (XXII). A cyclization reaction using potassium ethyl xanthate at high temperature in a suitable solvent (e.g. DMF = *N,N*-dimethylformamide) then enables the synthesis of bicycle (IIa, R² = Br; R⁴ = SH). Reduction of this compound with a metal (e.g. Zn or Fe) in a protic solvent or in a mixture containing protic reagents (e.g an organic acid such as acetic acid) produces the unsubstituted thiazolopyridine (IId, R² = Br; R⁴ = H). On the other hand, alkylation of the sulfur atom with an alkylating agent (e.g. methyl iodide) in the presence of an appropriate base (e.g. potassium carbonate) followed by subsequent oxidation using a suitable oxidizing reagent (e.g. *m*-CPBA = *meta-*chloroperbenzoic acid) affords sulfone (IIc, R² = Br; R⁴ = SO₂Me) (cf. WO2017/9806; WO2006/53166). Displacement of the sulfone group using a suitable nucleophile (e.g. sodium borohydride) followed by a palladium cross coupling reaction of a boronic acid coupling using an appropriate palladium complex (e.g. Pd(dppf)Cl₂), a suitable base (e.g. potassium carbonate) and a suitable aprotic solvent (e.g. toluene, 1,4-dioxane or DME = dimethoxyethane) affords thiazolopyridines (II). In Scheme 5 below, R¹ and R² and have the meanings defined above. R³ and R⁴, by way of example, but not by limitation represent hydrogen.

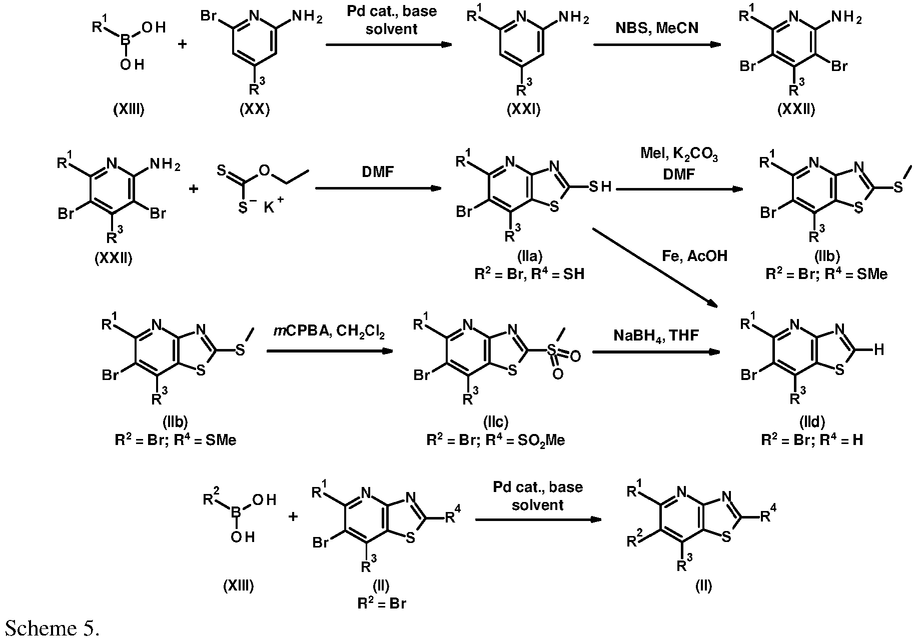

The synthesis of substituted thiazolopyridines (II) is also possible via the cyclisation of substituted ketoenamines and substituted aminothiazoles (Scheme 6). Following this synthesis route, ketone (X) is treated with a suitable reagent (e.g. N,N-dimethylformamide dimethyl acetal) at reflux to afford substituted ketoenamine (XXIII). A subsequent cyclisation reaction between ketoenamine (XXIII) and substituted aminothiazole (XXIV) under acidic conditions with a suitable co-solvent (e.g. HCl in 1,4-dioxane) furnishes thiazolopyridines (II). In Scheme 6 below, R¹ and R² and have the meanings defined above. R³ and R⁴, by way of example, but not by limitation represent hydrogen.

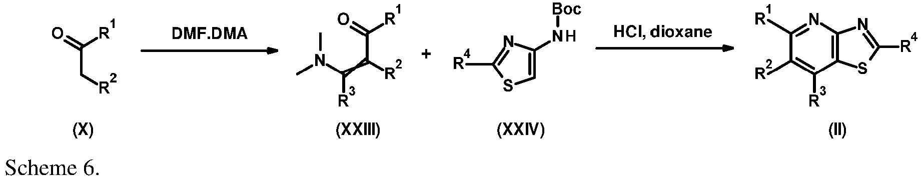

The synthesis of substituted 2,3-dihydro[1,3]thiazolo[4,5-b]pyridines of the general formula (I) can also be accomplished starting from pyridine intermediate (XXV) (Scheme 7). Following this reaction pathway substituted pyridine (XXV) is reacted with the sulfur nucleophile (XXVI) in a palladium cross coupling reaction under appropriate conditions (e.g. tris(dibenzylideneacetone)dipalladium, xantphos and DIPEA = N,N-diisopropylethylamine in toluene). Substituted pyridine (XXVII) is then deprotected in the presence of a suitable base (e.g. sodium methoxide) to afford intermediate (XXVIII) which is subsequently cyclized with an optionally substituted carbonyl compound (XXIX) in the presence of a suitable reagent (e.g. TFA = trifluoroacetic acid or alumina) to furnish the substituted 2,3-dihydro[1,3]thiazolo[4,5-b]pyridines of the general formula (I) (cf. Adv. Synth. Catal., 2020, 362, 3824-3829 and Synth. Commun., 2007, 37, 3329-3335). In Scheme 7 below, R¹, R³, R⁴, R⁵ and R⁶ have the meanings defined above. R² by way of example, but not by limitation has the meanings defined above except that it is not bromine or iodine and X = bromine or iodine.

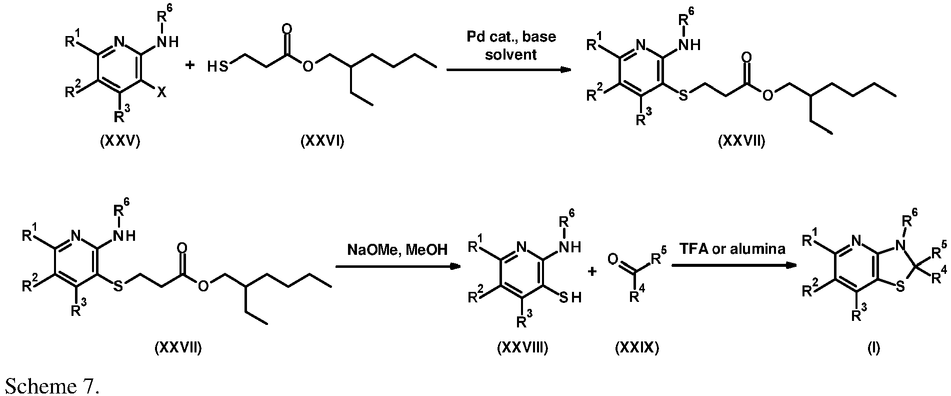

Selected detailed synthesis examples for the compounds of the general formula (I) according to the invention are given below. The example numbers mentioned correspond to the numbering scheme in Schemes 1 to 7 and Table 1. The ¹H NMR spectroscopy data reported for the chemical examples described in the sections that follow were obtained on Bruker instruments at 600 MHz, 400 MHz or 300 MHz using CDCl₃ or d₆-DMSO as the solvent with tetramethylsilane (δ = 0.00 ppm) as the internal standard. The signals listed have the meanings given below: br = broad; s = singlet, d = doublet, t = triplet, dd = doublet of doublets, ddd = doublet of a doublet of doublets, m = multiplet, q = quartet, qu = quintet, sext = sextet, sept = septet, dq = doublet of quartets, dt = doublet of triplets.

### No. I-001: 6-bromo-5-(2-fluorophenyl)-2,3-dihydro[1,3]thiazolo[4,5-b]pyridine

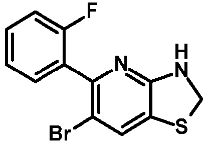

To a stirred mixture of 6-bromopyridin-2-amine (10.00 g, 56.88 mmol, 1.00 equiv.), 2-fluorophenylboronic acid (9.52 g, 65.98 mmol, 1.16 equiv.) and Na₂CO₃ (12.06 g, 113.8 mmol, 2.00 eq.) in a mixture of 1,4-dioxane (80 mL) and water (80 mL) at room temperature was added Pd(dppf)Cl₂ (1.67 g, 2.28 mmol, 0.04 equiv.) and the mixture was stirred at 80 °C for 3 h. Thereafter, the reaction mixture was cooled to room temperature, diluted with water and extracted thoroughly with ethyl acetate. The combined organic layer was washed with brine, dried over Na₂SO₄, filtered and concentrated under reduced pressure. The remaining residue was purified via column chromatography (gradient ethyl acetate/hexane) to afford 6-(2-fluorophenyl)pyridin-2-amine (9.98 g, 93 % of theory). ¹H NMR (400 MHz, CDCl₃): δ_{H} 7.92-7.88 (m, 1H), 7.53-7.49 (m, 1H), 7.36-7.31 (m, 1H), 7.25-7.20 (m, 1H), 7.16-7.10 (m, 1H), 6.50-6.48 (d, 1H), 4.53-4.44 (br. s, 2H, NH₂). 6-(2-Fluorophenyl)pyridin-2-amine (9.98 g, 52.49 mmol, 1.0 equiv.) was dissolved in acetonitrile (140 mL) and cooled to 0 °C. Thereafter, N-bromosuccinimide (20.56 g, 115.49 mmol, 2.2 equiv.) was added carefully. The reaction mixture was warmed to room temperature and stirred for 4 h. Subsequently, the reaction mixture was diluted with water and the resulting solid was filtered off. The solid was washed thoroughly with water and dried to afford compound 3,5-dibromo-6-(2-fluorophenyl)pyridin-2-amine (17.62 g, 97% yield of theory) as an orange solid. ¹H NMR (400 MHz, CDCl₃): δ_{H} 7.92 (s, 1H), 7.44-7.33 (m, 2H), 7.25-7.20 (m, 1H), 7.17-7.12 (m, 1H), 5.07-4.98 (br. s, 2H, NH₂). To a stirred solution of 3,5-dibromo-6-(2-fluorophenyl)-pyridin-2-amine (17.62 g, 50.93 mmol, 1.0 equiv.) in N,N-dimethylformamide (120 mL) at room temperature was added potassium O-ethyl dithiocarbonate (18.52 g, 112.04 mmol, 2.2 equiv.). The resulting mixture was heated under reflux conditions for 7 h. Thereafter, the reaction mixture was cooled to room temperature, poured onto ice water and acidified with 2 N HCl. The obtained precipitate was filtered, washed with water, collected and dried under reduced pressure to afford 6-bromo-5-(2-fluorophenyl)[1,3]thiazolo[4,5-b]pyridine-2-thiol (17.20 g, 97% yield of theory). ¹H NMR (400 MHz, CDCl₃): δ_{H} 9.96 (br. s, 1H), 8.01 (s, 1H), 7.50-7.44 (m, 1H), 7.41-7.38 (m, 1H), 7.29-7.25 (m, 1H), 7.19-7.15 (m, 1H). 6-Bromo-5-(2-fluorophenyl)[1,3]thiazolo[4,5-b]pyridine-2-thiol (14.55 g, 42.64 mmol, 1.0 equiv.) was dissolved in acetic acid (200 mL), and iron powder (35.71 g, 639.61 mmol, 15 equiv.) was added carefully portionwise. The resulting reaction mixture was stirred at a temperature of 100 °C for 10 h. After full conversion (indicated by LC/MS), the reaction mixture was cooled to 60 °C, and the iron powder was filtered off. The remaining solution was diluted with water, and the resulting precipitate was filtered, washed with water and dried under reduced pressure. The remaining crude residue was redissolved in dichloromethane, then water was added, followed by thorough extraction. The combined organic layer was dried over magnesium sulfate, filtered and dried under reduced pressure. The remaining residue was purified via column chromatography (gradient ethyl acetate/hexane) to afford 6-bromo-5-(2-fluorophenyl)[1,3]thiazolo[4,5-b]pyridine (8.75 g, 63 % of theory). ¹H NMR (400 MHz, CDCl₃): δ_{H} 9.32 (s, 1H), 8.64 (s, 1H), 7.54-7.45 (m, 2H), 7.31-7.27 (m, 1H), 7.21-7.16 (m, 1H). 6-Bromo-5-(2-fluorophenyl)[1,3]thiazolo[4,5-b]pyridine (1000 mg, 3.07 mmol, 1.0 equiv.) was dissolved in abs. toluene (10 mL) in a baked out roundflask under argon, ammonia-borane complex (285 mg, 9.20 mmol, 3.0 equiv.) and B(C₆F₅)₃ (79 mg, 0.15 mmol, 0.05 equiv.) were added. The resulting reaction mixture was stirred at a temperature of 45 °C for 3.5 h and concentrated thereafter under reduced pressure. The remaining residue was redissolved in acetonitrile, formic acid was added, and the reaction mixture was stirred at room temperature for 2 h. The phases were separated via phase separator and the organic layer was concentrated under reduced pressure. The remaining crude product was purified via column chromatography (gradient ethyl acetate/hexane) to afford 6-bromo-5-(2-fluorophenyl)-2,3-dihydro[1,3]thiazolo[4,5-b]pyridine (500 mg, 30 % yield of theory). ¹H NMR (400 MHz, CDCl₃): δ_{H} 7.41-7.34 (m, 3H), 7.23-7.19 (m, 1H), 7.15-7.11 (m, 1H), 5.77 (br. s, 1H, NH), 4.82 (s, 2H).

### No. I-007: 5-(2-fluorophenyl)-6-methyl-2,3-dihydro[1,3]thiazolo[4,5-b]pyridine

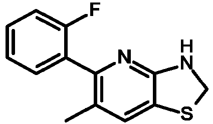

6-Bromo-5-(2-fluorophenyl)[1,3]thiazolo[4,5-b]pyridine (1.88 g, 4.56 mmol, 1.0 equiv.), methyl boronic acid (1.13 g, 18.24 mmol, 4.0 equiv.), potassium phosphate (1.94 g, 9.12 mmol, 2.0 equiv.), palladium(II)acetate (103 mg, 0.46 mmol, 0.1 equiv.) and 2-dicyclohexylphosphino-2',6'-dimethoxy-biphenyl (579 mg, 1.37 mmol, 0.3 equiv.) were dissolved in abs. toluene (40 mL) in a baked out roundflask under argon. The resulting reaction mixture was stirred under reflux conditions for 3.5 h. After cooling to room temperature water (80 mL) and toluene (20 mL) were added, followed by addition of ammonium-pyrrolidine dithiocarbamate (328 mg, 2.00 mmol, 0.44 equiv.). The reaction mixture was stirred for 1 h at room temperature, and the organic layer was washed with saturated sodium hydrogencarbonate solution, dried over magnesium sulfate, filtered and concentrated under reduced pressure. The remaining residue was purified via column chromatography (gradient ethyl acetate/hexane) to afford 5-(2-fluorophenyl)-6-methyl[1,3]thiazolo[4,5-b]pyridine (900 mg, 81 % of theory). ¹H NMR (400 MHz, CDCl₃): δ_{H} 9.25 (s, 1H), 8.23 (s, 1H), 7.55-7.51 (m, 1H), 7.47-7.41 (m, 1H), 7.30-7.27 (m, 1H), 7.19-7.15 (m, 1H), 2.40 (s, 3H). 5-(2-Fluorophenyl)-6-methyl[1,3]thiazolo[4,5-b]pyridine (320 mg, 1.23 mmol, 1.0 equiv.) was dissolved in abs. toluene (38 mL) in a baked out roundflask under argon, ammonia-borane complex (645 mg, 20.26 mmol, 3.0 equiv.) and B(C₆F₅)₃ (173 mg, 0.34 mmol, 0.05 equiv.) were added. The resulting reaction mixture was stirred at a temperature of 45 °C for 3.5 h and concentrated thereafter under reduced pressure. The remaining residue was redissolved in acetonitrile, formic acid was added, and the reaction mixture was stirred at room temperature for further 45 min. The phases were separated via phase separator and the organic layer was concentrated under reduced pressure. The remaining crude product was purified via column chromatography (gradient ethyl acetate/hexane) to afford 5-(2-fluorophenyl)-6-methyl-2,3-dihydro[1,3]thiazolo[4,5-b]pyridine (720 mg, 43 % yield of theory). ¹H NMR (400 MHz, CDCl₃): δ_{H} 7.37-7.31 (m, 2H), 7.22-7.18 (m, 1H), 7.13 (s, 1H), 7.12-7.08 (m, 1H), 5.38 (br. s, 1H, NH), 4.81 (s, 2H), 2.03 (s, 3H).

### No. I-018: cyclopropyl[5-(2-fluorophenyl)-6-methyl[1,3]thiazolo[4,5-b]pyridin-3(2H)-yl]methanone

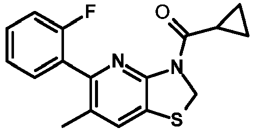

Cyclopropanecarboxylic acid chloride (0.03 mL, 0.31 mmol, 1.1 equiv.) and triethyl amine (0.09 mL, 0.63 mmol, 2.2 equiv.) were added to a stirred solution of 6-methyl-5-(2-fluorophenyl)-2,3-dihydro[1,3]thiazolo[4,5-b]pyridine (70 mg, 0.28 mmol, 1.00 equiv.) in abs. dichloromethane (3 mL). The resulting reaction mixture was stirred at room temperature for 30 min, followed by dilution with dichloromethane and water, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford cyclopropyl[5-(2-fluorophenyl)-6-methyl[1,3]thiazolo[4,5-b]pyridin-3(2H)-yl]methanone (24 mg, 27 % yield of theory). ¹H NMR (600 MHz, CDCl₃): δ_{H} 7.40-7.35 (m, 1H), 7.37 (s, 1H), 7.23-7.20 (m, 1H), 7.15-7.12 (m, 1H), 5.34 (s, 2H), 3.57-3.54 (m, 1H), 2.17 (s, 3H), 1.92-1.78 (m, 4H), 1.14-1.10 (m, 2H), 0.89-0.84 (m, 2H); ¹³C NMR (150 MHz, CDCl₃): δ_{c} 174.3, 160.6, 150.0, 146.8, 132.4, 131.4, 129.9, 127.9, 127.7, 125.7, 124.1, 115.7, 49.8, 18.4, 12.8, 10.6, 10.2.

### No. I-021: [6-bromo-5-(2-fluorophenyl)[1,3]thiazolo[4,5-b]pyridin-3(2H)-yl](cyclopentyl)methanone

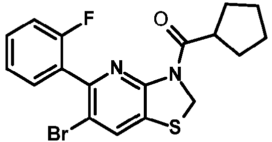

Cyclopentanecarboxylic acid chloride (0.04 mL, 0.36 mmol, 1.1 equiv.) and triethyl amine (0.10 mL, 0.72 mmol, 2.2 equiv.) were added to a stirred solution of 6-bromo-5-(2-fluorophenyl)-2,3-dihydro[1,3]thiazolo[4,5-b]pyridine (120 mg, 0.33 mmol, 1.00 equiv.) in abs. dichloromethane (5 mL). The resulting reaction mixture was stirred at room temperature for 2 h, followed by dilution with dichloromethane and water, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford [6-bromo-5-(2-fluorophenyl)[1,3]thiazolo[4,5-b]pyridin-3(2H)-yl](cyclopentyl)methanone (30 mg, 22 % yield of theory). ¹H NMR (400 MHz, CDCl₃): δ_{H} 7.64 (s, 1H), 7.47-7.37 (m, 2H), 7.25-7.21 (m, 1H), 7.18-7.14 (m, 1H), 5.36 (s, 2H), 4.06-4.02 (quint, 1H), 1.92-1.78 (m, 4H), 1.74-1.66 (m, 2H), 1.58-1.49 (m, 2H).

### No. I-029: 5-(2,3-difluorophenyl)-6-methyl-2,3-dihydro[1,3]thiazolo[4,5-b]pyridine

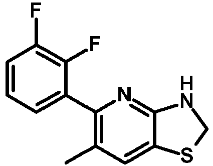

To a stirred mixture of 6-bromopyridin-2-amine (1.00 equiv.), 2,3-difluorophenylboronic acid (1.16 equiv.) and Na₂CO₃ (2.00 eq.) in a mixture of 1,4-dioxane (2 mL/mmol) and water (2 mL/mmol) at r.t. was added Pd(dppf)Cl₂ (0.04 equiv.) and the mixture was stirred at 80 °C for 3 h. Thereafter, the reaction mixture was cooled to r.t, diluted with water and extracted thoroughly with ethyl acetate. The combined organic layer was washed with brine, dried over Na₂SO₄, filtered and concentrated under reduced pressure. The remaining residue was purified via column chromatography (gradient ethyl acetate/hexane) to afford 6-(2,3-difluorophenyl)pyridin-2-amine. 6-(2,3-difluorophenyl)pyridin-2-amine (1.0 equiv.) was dissolved in acetonitrile (4 mL/mmol) and cooled to 0 °C. Thereafter, *N-*bromosuccinimide (2.2 equiv.) was added carefully. The reaction mixture was warmed to room temperature and stirred for 4 h. Subsequently, the reaction mixture was diluted with water and the resulting solid was filtered off. The solid was washed thoroughly with water and dried to afford compound 3,5-dibromo-6-(2,3-difluorophenyl)pyridin-2-amine as a yellowish-orange solid. To a stirred solution of 3,5-dibromo-6-(2,3-difluorophenyl)pyridin-2-amine (1.0 equiv.) in *N,N*-dimethylformamide (3 mL/mmol) at room temperature was added potassium O-ethyl dithiocarbonate (2.2 equiv.). The resulting mixture was heated under reflux conditions for 4 d. Thereafter, the reaction mixture was cooled to room temperature, poured onto ice water and acidified with 2 N HCl. The obtained precipitate was filtered, washed with water, collected and dried under reduced pressure to afford 6-bromo-5-(2,3-difluorophenyl)[1,3]thiazolo[4,5-b]pyridine-2-thiol. 6-Bromo-5-(2,3-difluorophenyl)[1,3]thiazolo[4,5-b]pyridine-2-thiol (1.0 equiv.) was dissolved in acetic acid (5 mL/mmol), and iron powder (15 equiv.) was added carefully portionwise. The resulting reaction mixture was stirred at a temperature of 100 °C for 8 h. After full conversion (indicated by LC/MS), the reaction mixture was cooled to 60 °C, and the iron powder was filtered off. The remaining solution was diluted with water, and the resulting precipitate was filtered, washed with water and dried under reduced pressure. The remaining crude residue was redissolved in dichloromethane, then water was added, followed by thorough extraction. The combined organic layer was dried over magnesium sulfate, filtered and dried under reduced pressure. The remaining residue was purified via column chromatography (gradient ethyl acetate/hexane) to afford 6-bromo-5-(2,3-difluorophenyl)[1,3]thiazolo[4,5-b]pyridine. 6-Bromo-5-(2,3-difluorophenyl)[1,3]thiazolo[4,5-b]pyridine (4.00 g, 11.37 mmol, 1.0 equiv.), methyl boronic acid (2.81 g, 45.48 mmol, 4.0 equiv.), potassium phosphate (4.83 g, 22.74 mmol, 2.0 equiv.), palladium(II)acetate (255 mg, 1.14 mmol, 0.1 equiv.) and 2-dicyclohexylphosphino-2',6'-dimethoxybiphenyl (1444 mg, 3.41 mmol, 0.3 equiv.) were dissolved in abs. toluene (80 mL) in a baked out roundflask under argon. The resulting reaction mixture was stirred under reflux conditions for 3.5 h. After cooling to room temperature water (80 mL) and toluene (20 mL) were added, followed by addition of ammonium-pyrrolidine dithiocarbamate (819 mg, 4.99 mmol, 0.44 equiv.). The reaction mixture was stirred for 1 h at room temperature, and the organic layer was washed with saturated sodium hydrogencarbonate solution, dried over magnesium sulfate, filtered and concentrated under reduced pressure. The remaining residue was purified via column chromatography (gradient ethyl acetate/hexane) to afford 5-(2,3-difluorophenyl)-6-methyl[1,3]thiazolo[4,5-b]pyridine (1440 mg, 51 % of theory). ¹H NMR (400 MHz, CDCl₃): δ_{H} 9.27 (s, 1H), 8.25 (s, 1H), 7.31-7.19 (m, 3H), 2.42 (s, 3H). 5-(2,3-difluorophenyl)-6-methyl[1,3]thiazolo[4,5-b]pyridine (1000 mg, 3.81 mmol, 1.0 equiv.) was dissolved in abs. toluene (14 mL) in a baked out roundflask under argon, ammonia-borane complex (353 mg, 1.44 mmol, 3.0 equiv.) and B(C₆F₅)₃ (98 mg, 0.19 mmol, 0.05 equiv.) were added. The resulting reaction mixture was stirred at a temperature of 45 °C for 3.5 h and concentrated thereafter under reduced pressure. The remaining residue was redissolved in acetonitrile, formic acid was added, and the reaction mixture was stirred at room temperature for further 45 min. The phases were separated via phase separator and the organic layer was concentrated under reduced pressure. The remaining crude product was purified via column chromatography (gradient ethyl acetate/hexane) to afford 5-(2,3-difluorophenyl)-6-methyl-2,3-dihydro[1,3]thiazolo[4,5-b]pyridine (600 mg, 59 % yield of theory). ¹H NMR (400 MHz, CDCl₃): δ_{H} 7.20-7.08 (m, 3H), 5.25 (br. s, 1H, NH), 4.86 (s, 2H), 2.04 (s, 3H).

### No. I-041: 6-bromo-5-(2-chlorophenyl)-2,3-dihydro[1,3]thiazolo[4,5-b]pyridine

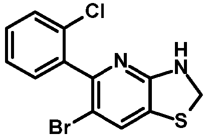

To a stirred mixture of 6-bromopyridin-2-amine (6.00 g, 34.13 mmol, 1.00 equiv.), 2-chlorophenylboronic acid (6.38 g, 39.59 mmol, 1.16 equiv.) and Na₂CO₃ (7.23 g, 68 mmol, 2.00 eq.) in a mixture of 1,4-dioxane (60 mL) and water (60 mL) at RT was added Pd(dppf)Cl₂ (0.99 g, 1.37 mmol, 0.04 equiv.) and the mixture was stirred at 80 °C for 3 h. Thereafter, the reaction mixture was cooled to RT, diluted with water and extracted thoroughly with ethyl acetate. The combined organic layer was washed with brine, dried over Na₂SO₄, filtered and concentrated under reduced pressure. The remaining residue was purified via column chromatography (gradient ethyl acetate/hexane) to afford 6-(2-chlorophenyl)pyridin-2-amine (7.11 g, 98 % of theory). ¹H NMR (400 MHz, CDCl₃): δ_{H} 7.54-7.49 (m, 2H), 7.46-7.43 (m, 1H), 7.35-7.27 (m, 2H), 6.96-6.94 (d, 1H), 6.52-6.50 (d, 1H), 4.57-4.45 (br. s, 2H, NH₂). 6-(2-Chlorophenyl)pyridin-2-amine (7.11 g, 33.69 mmol, 1.0 equiv.) was dissolved in acetonitrile (140 mL) and cooled to 0 °C. Thereafter, N-bromosuccinimide (13.19 g, 74.14 mmol, 2.2 equiv.) was added carefully. The reaction mixture was warmed to room temperature and stirred for 4 h. Subsequently, the reaction mixture was diluted with water and the resulting solid was filtered off. The solid was washed thoroughly with water and dried to afford compound 3,5-dibromo-6-(2-chlorophenyl)pyridin-2-amine (11.42 g, 91 % yield of theory) as an orange solid. ¹H NMR (400 MHz, CDCl₃): δ_{H} 7.92 (s, 1H), 7.48-7.45 (m, 1H), 7.36-7.31 (m, 2H), 7.30-7.27 (m, 1H), 5.10-4.98 (br. s, 2H, NH₂). To a stirred solution of 3,5-dibromo-6-(2-chlorophenyl)pyridin-2-amine (11.42 g, 29.93 mmol, 1.0 equiv.) in N,N-dimethylformamide (90 mL) at room temperature was added potassium O-ethyl dithiocarbonate (10.88 g, 65.85 mmol, 2.2 equiv.). The resulting mixture was heated under reflux conditions for 8 d. Thereafter, the reaction mixture was cooled to room temperature, poured onto ice water and acidified with 2 N HCl. The obtained precipitate was filtered, washed with water, collected and dried under reduced pressure to afford 6-bromo-5-(2-chlorophenyl)[1,3]thiazolo[4,5-b]pyridine-2-thiol (11.42 g, 92% yield of theory). ¹H NMR (400 MHz, CDCl₃): δ_{H} 9.68 (br. s, 1H), 8.01 (s, 1H), 7.53-7.50 (m, 1H), 7.46-7.38 (m, 2H), 7.34-7.31 (m, 1H). 6-Bromo-5-(2-chlorophenyl)[1,3]thiazolo[4,5-b]pyridine-2-thiol (8.00 g, 21.49 mmol, 1.0 equiv.) was dissolved in acetic acid (90 mL), and iron powder (17.79 g, 318.72 mmol, 15 equiv.) was added carefully portionwise. The resulting reaction mixture was stirred at a temperature of 100 °C for 8 h. After full conversion (indicated by LC/MS), the reaction mixture was cooled to 60 °C, and the iron powder was filtered off. The remaining solution was diluted with water, and the resulting precipitate was filtered, washed with water and dried under reduced pressure. The remaining crude residue was redissolved in dichloromethane, then water was added, followed by thorough extraction. The combined organic layer was dried over magnesium sulfate, filtered and dried under reduced pressure. The remaining residue was purified via column chromatography (gradient ethyl acetate/hexane) to afford 6-bromo-5-(2-chlorophenyl)[1,3]thiazolo[4,5-b]pyridine (2.74 g, 31 % of theory). ¹H NMR (400 MHz, CDCl₃): δ_{H} 9.33 (s, 1H), 8.64 (s, 1H), 7.52-7.49 (m, 1H), 7.44-7.38 (m, 3H). 6-Bromo-5-(2-chlorophenyl)[1,3]thiazolo[4,5-b]pyridine (550 mg, 1.69 mmol, 1.0 equiv.) was dissolved in abs. toluene (10 mL) in a baked out roundflask under argon, ammonia-borane complex (156 mg, 5.07 mmol, 3.0 equiv.) and B(C₆F₅)₃ (43 mg, 0.08 mmol, 0.05 equiv.) were added. The resulting reaction mixture was stirred at a temperature of 45 °C for 3.5 h and concentrated thereafter under reduced pressure. The remaining residue was redissolved in acetonitrile, formic acid was added, and the reaction mixture was stirred at room temperature for 45 min. The phases were separated via phase separator and the organic layer was concentrated under reduced pressure. The remaining crude product was purified via column chromatography (gradient ethyl acetate/hexane) to afford 6-bromo-5-(2-chlorophenyl)-2,3-dihydro[1,3]thiazolo[4,5-b]pyridine (260 mg, 46 % yield of theory). ¹H NMR (400 MHz, CDCl₃): δ_{H} 7.48-7.44 (m, 1H), 7.36-7.26 (m, 4H), 6.17 (br. s, 1H, NH), 4.81 (s, 2H).

### No. I-042: 1-[6-bromo-5-(2-chlorophenyl)[1,3]thiazolo[4,5-b]pyridin-3(2H)-yl]butan-1-one

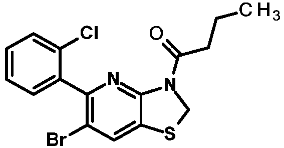

Butyryl chloride (0.03 mL, 0.26 mmol, 1.2 equiv.) and triethyl amine (0.07 mL, 0.47 mmol, 2.2 equiv.) were added to a stirred solution of 6-bromo-5-(2-chlorophenyl)-2,3-dihydro[1,3]thiazolo[4,5-b]pyridine (70 mg, 0.21 mmol, 1.00 equiv.) in abs. dichloromethane (3 mL). The resulting reaction mixture was stirred at room temperature for 30 min, followed by dilution with dichloromethane and water, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford 1-[6-bromo-5-(2-chlorophenyl)[1,3]thiazolo[4,5-b]pyridin-3(2H)-yl]butan-1-one (83 mg, 95 % yield of theory). ¹H NMR (400 MHz, CDCl₃): δ_{H} 7.64 (s, 1H), 7.49-7.47 (m, 1H), 7.39-7.30 (m, 3H), 5.42 (d, 1H), 5.34 (d, 1H), 2.96-2.92 (t, 2H), 1.71-1.60 (m, 2H), 0.87-0.83 (t, 3H).

### I-048: 4-[6-bromo-5-(2-chlorophenyl)-2,3-dihydro[1,3]thiazolo[4,5-b]pyridin-3(2H)-yl]-1,3,2,4-dioxa-diboretan-2-amine

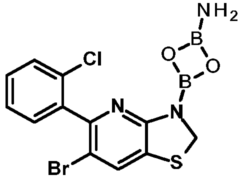

6-Bromo-5-(2-chlorophenyl)[1,3]thiazolo[4,5-b]pyridine (350 mg, 1.08 mmol, 1.0 equiv.) was dissolved in abs. toluene (10 mL) in a baked out roundflask under argon, ammonia-borane complex (99 mg, 3.23 mmol, 3.0 equiv.) and B(C₆F₅)³ (28 mg, 0.05 mmol, 0.05 equiv.) were added. The resulting reaction mixture was stirred at a temperature of 45 °C for 3.5 h and concentrated thereafter under reduced pressure. The remaining crude product was purified via column chromatography (gradient ethyl acetate/hexane) to afford 6-bromo-5-(2-chlorophenyl)-2,3-dihydro[1,3]thiazolo[4,5-b]pyridine (37 mg, 8 % yield of theory). ¹H NMR (600 MHz, CDCl₃): δ_{H} 7.45-7.44 (d, 1H), 7.38-7.32 (m, 2H), 7.19-7.18 (d, 1H), 7.11 (s, 1H), 5.17 (d, 1H), 5.13 (d, 1H), 2.62 (br. s, 1H), 2.48 (br. s, 1H).

### No. I-049: 5-(2-chlorophenyl)-6-methyl-2,3-dihydro[1,3]thiazolo[4,5-b]pyridine

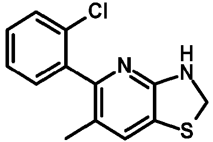

6-Bromo-5-(2-chlorophenyl)[1,3]thiazolo[4,5-b]pyridine (550 mg, 1.69 mmol, 1.0 equiv.), methyl boronic acid (202 mg, 3.38 mmol, 2.0 equiv.), potassium phosphate (1.43 g, 6.76 mmol, 4.0 equiv.), palladium(II)acetate (38 mg, 0.17 mmol, 0.1 equiv.) and 2-dicyclohexylphosphino-2',6'-dimethoxybiphenyl (139 mg, 0.34 mmol, 0.2 equiv.) were dissolved in abs. toluene in a baked out roundflask under argon. The resulting reaction mixture was stirred under reflux conditions for 8 h. After cooling to room temperature water and toluene were added, followed by addition of ammonium-pyrrolidine dithiocarbamate (122 mg, 0.74 mmol, 0.44 equiv.). The reaction mixture was stirred for 1 h at room temperature, and the organic layer was washed with saturated sodium hydrogencarbonate solution, dried over magnesium sulfate, filtered and concentrated under reduced pressure. The remaining residue was purified via column chromatography (gradient ethyl acetate/hexane) to afford 5-(2-chlorophenyl)-6-methyl[1,3]thiazolo[4,5-b]pyridine (320 mg, 70 % of theory). ¹H NMR (400 MHz, CDCl₃): δ_{H} 9.26 (s, 1H), 8.23 (s, 1H), 7.51-7.47 (m, 1H), 7.41-7.37 (m, 3H), 2.33 (s, 3H). 5-(2-Chlorophenyl)-6-methyl[1,3]thiazolo[4,5-b]pyridine (320 mg, 1.23 mmol, 1.0 equiv.) was dissolved in abs. toluene (10 mL) in a baked out roundflask under argon, ammonia-borane complex (114 mg, 3.00 mmol, 3.0 equiv.) and B(C₆F₅)₃ (31 mg, 0.06 mmol, 0.05 equiv.) were added. The resulting reaction mixture was stirred at a temperature of 45 °C for 3.5 h and concentrated thereafter under reduced pressure. The remaining residue was redissolved in acetonitrile, formic acid was added, and the reaction mixture was stirred at room temperature for further 45 min. The phases were separated via phase separator and the organic layer was concentrated under reduced pressure. The remaining crude product was purified via column chromatography (gradient ethyl acetate/hexane) to afford 5-(2-chlorophenyl)-6-methyl-2,3-dihydro[1,3]thiazolo[4,5-b]pyridine (168 mg, 52 % yield of theory). ¹H NMR (400 MHz, CDCl₃): δ_{H} 7.45-7.42 (m, 1H), 7.33-7.27 (m, 3H), 7.12 (s, 1H), 5.32 (br. s, 1H, NH), 4.82 (s, 2H), 1.95 (s, 3H).

### No. I-055: 1-[6-bromo-5-(2-chlorophenyl)[1,3]thiazolo[4,5-b]pyridin-3(2H)-yl]ethanone

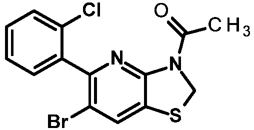

Acetyl chloride (0.02 mL, 0.33 mmol, 1.2 equiv.) and triethyl amine (0.08 mL, 0.60 mmol, 2.2 equiv.) were added to a stirred solution of 6-bromo-5-(2-chlorophenyl)-2,3-dihydro[1,3]thiazolo[4,5-b]pyridine (90 mg, 0.28 mmol, 1.00 equiv.) in abs. dichloromethane (3 mL). The resulting reaction mixture was stirred at room temperature for 30 min, followed by dilution with dichloromethane and water, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford 1-[6-bromo-5-(2-chlorophenyl)[1,3]thiazolo[4,5-b]pyridin-3(2H)-yl]ethanone (93 mg, 83 % yield of theory). ¹H NMR (400 MHz, CDCl₃): δ_{H} 7.65 (s, 1H), 7.49-7.47 (m, 1H), 7.39-7.31 (m, 3H), 5.42 (d, 1H), 5.34 (d, 1H), 2.56 (s, 3H).

### No. I-060: 3-(ethylsulfonyl)-5-(2-fluorophenyl)-6-methyl-2,3-dihydro[1,3]thiazolo[4,5-b]pyridine

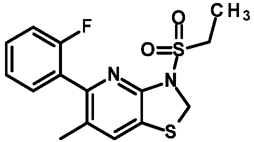

Ethanesulfonyl chloride (52 mg, 0.40 mmol, 1.1 equiv.) and triethyl amine (0.11 mL, 0.80 mmol, 2.2 equiv.) were added to a stirred solution of 5-(2-fluorophenyl)-6-methyl-2,3-dihydro[1,3]thiazolo[4,5-b]pyridine (90 mg, 0.37 mmol, 1.00 equiv.) in abs. dichloromethane (5 mL). The resulting reaction mixture was stirred at room temperature for 30 minutes and concentrated under reduced pressure, followed by addition of dichloromethane, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford 3-(ethylsulfonyl)-5-(2-fluorophenyl)-6-methyl-2,3-dihydro[1,3]thiazolo[4,5-b]pyridine (117 mg, 93 % yield of theory). ¹H-NMR (400 MHz, CD₃OD) δ_{H} 7.55 (s, 1H), 7.48-7.40 (m, 2H), 7.31-7.27 (m, 1H), 7.20-7.17 (m, 1H), 5.35 (s, 2H), 3.68-3.62 (q, 2H), 2.16 (s, 3H), 1.38-1.34 (t, 3H).

### No. I-061: [6-bromo-5-(2-chlorophenyl)[1,3]thiazolo[4,5-b]pyridin-3(2H)-yl][2-(trifluoromethyl)-phenyl]methanone

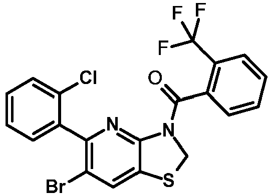

2-(Trifluoromethyl)benzoylchloride (40 mg, 0.18 mmol, 1.2 equiv.) and triethyl amine (0.05 mL, 0.34 mmol, 2.2 equiv.) were added to a stirred solution of 6-bromo-5-(2-chlorophenyl)-2,3-dihydro[1,3]thiazolo[4,5-b]pyridine (50 mg, 0.15 mmol, 1.00 equiv.) in abs. dichloromethane (3 mL).

The resulting reaction mixture was stirred at room temperature for 30 min, followed by dilution with dichloromethane and water, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford [6-bromo-5-(2-chlorophenyl)-[1,3]thiazolo[4,5-b]pyridin-3(2H)-yl][2-(trifluoromethyl)phenyl]methanone (20 mg, 23 % yield of theory). ¹H NMR (400 MHz, CDCl₃): δ_{H} 7.60 (s, 1H), 7.40-7.35 (m, 3H), 7.30-7.20 (m, 3H), 7.17-7.11 (m, 1H), 6.74-6.68 (m, 1H), 5.54 (d, 1H), 5.52 (d, 1H).

### No. I-079: 6-bromo-5-(3-chloro-2-thienyl)-2,3-dihydrothiazolo[4,5-b]pyridine

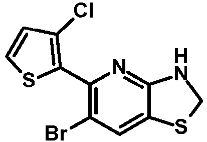

To a stirred mixture of 6-bromopyridin-2-amine (2.00 g, 11.5 mmol, 1.00 equiv.), 3-chlorothiophene-2-boronic acid (2.82 g, 17.3 mmol, 1.5 equiv.) and K₂CO₃ (3.19 g, 23.1 mmol, 2.00 eq.) in a mixture of 1,4-dioxane (15 mL) and water (15 mL) at room temperature was added Pd(dppf)Cl₂ (0.42 g, 0.57 mmol, 0.05 equiv.) and the mixture was stirred at 80 °C for 5 h. Thereafter, the reaction mixture was cooled to room temperature, diluted with water and extracted thoroughly with dichloromethane. The combined organic layer was dried over Na₂SO₄, filtered and concentrated under reduced pressure. The remaining residue was purified via column chromatography (gradient ethyl acetate/heptane) to afford 6-(3-Chloro-2-thienyl)pyridin-2-amine (1.19 g, 49 % yield of theory). ¹H NMR (400 MHz, CDCl₃): δ_{H} 7.59-7.55 (m, 1H), 7.52-7.46 (m, 1H), 7.30-7.23 (m, 1H), 6.98-6.94 (m, 1H), 6.45-6.38 (m, 1H), 4.47 (br. s, 2H, NH₂). 6-(3-Chloro-2-thienyl)pyridin-2-amine (1.10 g, 5.22 mmol, 1.0 equiv.) was dissolved in acetonitrile (22 mL) at room temperature and N-bromosuccinimide (1.95 g, 10.9 mmol, 2.1 equiv.) was added carefully. The reaction mixture was stirred at room temperature for 24 h. Subsequently, the reaction mixture was diluted with water and extracted with dichloromethane. The organic layer was dried over sodium sulfate, filtered and concentrated under reduced pressure. The remaining residue was purified via column chromatography (gradient ethyl acetate/heptane) to afford 3,5-dibromo-6-(3-chloro-2-thienyl)pyridin-2-amine (1.84 g, 95% yield of theory). ¹H NMR (400 MHz, CDCl₃): δ_{H} 7.93 (s, 1H), 7.40-7.37 (m, 1H), 7.00-6.96 (m, 1H), 5.04 (br. s, 2H, NH₂). To a stirred solution of 3,5-dibromo-6-(3-chloro-2-thienyl)pyridin-2-amine (1.83 g, 4.96 mmol, 1.0 equiv.) in *N,N*-dimethylformamide (50 mL) at room temperature was added potassium O-ethyl dithiocarbonate (4.78 g, 29.7 mmol, 6.0 equiv.). The resulting mixture was heated under reflux conditions for 36 h. Thereafter, the reaction mixture was cooled to room temperature, poured onto ice water and acidified with 2 N HCl. The obtained precipitate was filtered, washed with water, collected and dried under reduced pressure to afford 6-bromo-5-(3-chloro-2-thienyl)-3H-thiazolo[4,5-b]pyridine-2-thione (1.80 g, 99 % yield of theory). The product was used directly in the next step without further purification. ¹H NMR (400 MHz, CDCl₅): δ_{H} 8.04 (s, 1H), 8.01 (s, 1H), 7.47-7.43 (m, 1H), 7.04-7.01 (m, 1H). 6-Bromo-5-(3-chloro-2-thienyl)-3H-thiazolo[4,5-b]pyridine-2-thione (1.85 g, 5.08 mmol, 1.0 equiv.) was dissolved in *N,N*-dimethylformamide (40 mL), and iodomethane (0.51 mL, 8.13 mmol, 1.6 equiv.) and potassium carbonate (1.41 g, 10.1 mmol, 2.0 equiv.) were added. The resulting reaction mixture was stirred at room temperature for 2.5 h, followed by dilution with water and extraction with ethyl acetate. The organic layer was washed with brine, dried over sodium sulfate, filtered and concentrated under reduced pressure. The remaining residue was purified via column chromatography (gradient ethyl acetate/heptane) to afford 6-bromo-5-(3-chloro-2-thienyl)-2-methylsulfanyl-thiazolo[4,5-b]pyridine (0.92 g, 47 % yield of theory). ¹H NMR (400 MHz, CDCl₃): δ_{H} 8.37 (s, 1H), 7.44-7.41 (m, 1H), 7.03-7.00 (m, 1H), 2.87 (m, 3H). 6-Bromo-5-(3-chloro-2-thienyl)-2-methylsulfanyl-thiazolo[4,5-b]pyridine (1.23 g, 3.25 mmol, 1.0 equiv.) was dissolved in dichloromethane (18 mL), and meta-chloroperoxybenzoic acid (1.24 g, 7.16 mmol, 2.2 equiv.) was added carefully. The resulting reaction mixture was stirred at room temperature for 48 h, followed by dilution with dichloromethane, washing with saturated sodium carbonate and phase separation. The organic layer was dried over sodium sulfate, filtered and concentrated under reduced pressure. The remaining residue was purified via column chromatography (gradient ethyl acetate/heptane) to afford 6-bromo-5-(3-chloro-2-thienyl)-2-methylsulfonyl-thiazolo[4,5-b]pyridine (1.04 g, 78 % yield of theory). ¹H NMR (400 MHz, CDCl₃): δ_{H} 8.74 (s, 1H), 7.51-7.48 (m, 1H), 7.07-7.04 (m, 1H), 3.51 (m, 3H). 6-Bromo-5-(3-chloro-2-thienyl)-2-methylsulfonyl-thiazolo[4,5-b]pyridine (500 mg, 1.22 mmol, 1.0 equiv.) was dissolved in abs. toluene (14 mL) in a baked out round bottomed flask under argon, ammonia-borane complex (113 mg, 3.66 mmol, 3.0 equiv.) and B(C₆F₅)₃ (31 mg, 0.06 mmol, 0.05 equiv.) were added. The resulting reaction mixture was stirred at a temperature of 45 °C for 24 h and concentrated thereafter under reduced pressure. The remaining residue was redissolved in acetonitrile, formic acid was added, and the reaction mixture was stirred at room temperature for 1 h. The mixture was extracted with dichloromethane and the organic layer was dried over sodium sulfate and concentrated under reduced pressure. The remaining crude product was purified via column chromatography (gradient ethyl acetate/heptane) to afford 6-bromo-5-(3-chloro-2-thienyl)-2,3-dihydrothiazolo[4,5-b]pyridine (245 mg, 59 % yield of theory). ¹H NMR (400 MHz, CDCl₃): δ_{H} 7.37-7.33 (m, 2H), 6.98-6.94 (m, 1H), 5.54 (br. s, 1H, NH), 4.92 (s, 2H).

### No. I-077: methyl 4-[6-bromo-5-(3-chloro-2-thienyl)-2H-thiazolo[4,5-b]pyridin-3-yl]-4-oxo-butanoate

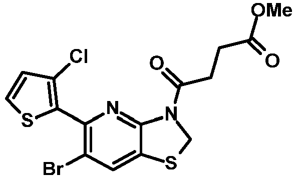

Methyl 4-chloro-4-oxobutanoate (0.11 mL, 0.89 mmol, 3.0 equiv.) and triethyl amine (0.09 mL, 0.65 mmol, 2.2 equiv.) were added to a stirred solution of 6-bromo-5-(3-chloro-2-thienyl)-2,3-dihydrothiazolo[4,5-b]pyridine (100 mg, 0.30 mmol, 1.0 equiv.) in abs. dichloromethane (2 mL). The resulting reaction mixture was stirred at room temperature for 84 h, followed by dilution with dichloromethane and water, extraction and phase separation. The aqueous layer was thoroughly extracted with dichloromethane, and the combined organic layer was dried over sodium sulfate, filtered and concentrated under reduced pressure. The remaining crude product was purified via column chromatography (gradient ethyl acetate/heptane) to afford methyl 4-[6-bromo-5-(3-chloro-2-thienyl)-2H-thiazolo[4,5-b]pyridin-3-yl]-4-oxo-butanoate (48 mg, 35 % yield of theory). ¹H NMR (400 MHz, CDCl₃): δ_{H} 7.64 (s, 1H), 7.41-7.38 (m, 1H), 7.01-6.98 (m, 1H), 5.35 (s, 2H), 3.68 (s, 3H), 3.37-3.32 (m, 2H), 2.71-2.66 (m, 2H).

### No. I-141: [6-bromo-5-(2-chlorophenyl)[1,3]thiazolo[4,5-b]pyridin-3(2H)-yl](3-chloro-5-methyl-4,5-dihydro-1,2-oxazol-5-yl)methanone

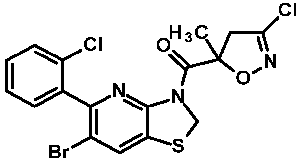

(3-chloro-5-methyl-4H-1,2-oxazol-5-yl)carboxylic acid chloride (76 mg, 0.40 mmol, 1.1 equiv.) and triethyl amine (0.11 mL, 0.81 mmol, 2.2 equiv.) were added to a stirred solution of 6-bromo-5-(2-chlorophenyl)-2,3-dihydro[1,3]thiazolo[4,5-b]pyridine (120 mg, 0.37 mmol, 1.00 equiv.) in abs. tetrahydrofuran (3 mL). The resulting reaction mixture was stirred at room temperature for 1 h and concentrated under reduced pressure, followed by addition of dichloromethane, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford [6-bromo-5-(2-chlorophenyl)[1,3]thiazolo[4,5-b]pyridin-3(2H)-yl](3-chloro-5-methyl-4,5-dihydro-1,2-oxazol-5-yl)methanone (70 mg, 40 % yield of theory).

¹H-NMR(400 MHz, CDCl₃) δ_{H} 7.71 (s, 1H), 7.50-7.47 (m, 1H), 7.40-7.30 (m, 3H), 5.39-5.36 (m, 2H), 3.91-3.86 / 3.86-3.82 (d, 1H), 3.01-2.97 / 2.87-2.83 (d, 1H), 1.79 / 1.70 (s, 3H).

### No. I-142: 5-(2-chlorophenyl)-6-methyl-3-(methylsulfonyl)-2,3-dihydro[1,3]thiazolo[4,5-b]pyridine

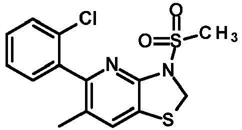

Methanesulfonyl chloride (48 mg, 0.42 mmol, 1.1 equiv.) and triethyl amine (0.12 mL, 0.81 mmol, 2.2 equiv.) were added to a stirred solution of 5-(2-chlorophenyl)-6-methyl-2,3-dihydro[1,3]thiazolo[4,5-b]pyridine (100 mg, 0.38 mmol, 1.00 equiv.) in abs. dichloromethane (5 mL). The resulting reaction mixture was stirred at room temperature for 30 minutes and concentrated under reduced pressure, followed by addition of dichloromethane, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford 5-(2-chlorophenyl)-6-methyl-3-(methylsulfonyl)-2,3-dihydro[1,3]thiazolo[4,5-b]pyridine (65 mg, 49 % yield of theory). ¹H-NMR (400 MHz, CDCl₃) δ_{H} 7.47-7.44 (m, 1H), 7.36-7.29 (m, 4H), 5.39-5.24 (m, 2H), 3.38 (s, 3H), 2.07 (s, 3H).

### No. I-146: 3-(ethylsulfonyl)-5-(2-fluorophenyl)-6-methyl-2,3-dihydro[1,3]thiazolo[4,5-b]pyridine 1,1-dioxide

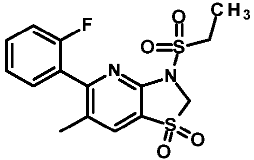

Sodium periodate (98 mg, 0.36 mmol, 2.4 equiv.) and ruthenium(III)chloride (0.46 mg, 0.002 mmol, 0.015 equiv.) were added to a stirred solution of 3-(ethylsulfonyl)-5-(2-fluorophenyl)-6-methyl-2,3-dihydro[1,3]thiazolo[4,5-b]pyridine (50 mg, 0.15 mmol, 1.00 equiv.) in a mixture of abs. dichloromethane (1 mL), acetonitrile (1.5 mL) and water (1.5 mL). The resulting reaction mixture was stirred at room temperature for 16 h and concentrated under reduced pressure, followed by addition of dichloromethane, saturated sodium thiosulfate solution, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford 3-(ethylsulfonyl)-5-(2-fluorophenyl)-6-methyl-2,3-dihydro[1,3]thiazolo[4,5-b]pyridine 1,1-dioxide (30 mg, 52 % yield of theory). ¹H-NMR (400 MHz, CDCl₃) δ_{H} 7.95 (s, 1H), 7.50-7.40 (m, 2H), 7.30-7.27 (m, 1H), 7.20-7.17 (m, 1H), 4.98 (s, 2H), 3.78-3.74 (q, 2H), 2.32 (s, 3H), 1.44-1.40 (t, 3H).

### No. I-147: 5-(2,3-difluorophenyl)-6-methyl-3-(methylsulfonyl)-2,3-dihydro[1,3]thiazolo[4,5-b]pyridine

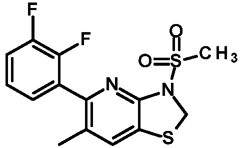

Methanesulfonyl chloride (54 mg, 0.46 mmol, 1.1 equiv.) and triethyl amine (0.13 mL, 0.92 mmol, 2.2 equiv.) were added to a stirred solution of 5-(2,3-difluorophenyl)-6-methyl-2,3-dihydro[1,3]thiazolo-[4,5-b]pyridine (130 mg, 0.42 mmol, 1.00 equiv.) in abs. dichloromethane (5 mL). The resulting reaction mixture was stirred at room temperature for 30 minutes and concentrated under reduced pressure, followed by addition of dichloromethane, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford 5-(2,3-difluorophenyl)-6-methyl-3-(methylsulfonyl)-2,3-dihydro[1,3]thiazolo[4,5-b]pyridine (85 mg, 58 % yield of theory). ¹H-NMR (400 MHz, CDCl₃) δ_{H} 7.36 (s, 1H), 7.25-7.215 (m, 3H), 5.30 (s, 2H), 3.39 (s, 3H), 2.17 (s, 3H).

### No. I-151: 5-(2,3-difluorophenyl)-3-(methylsulfonyl)-6-methyl-2,3-dihydro[1,3]thiazolo[4,5-b]pyridine 1,1-dioxide

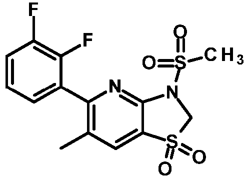

Sodium periodate (107 mg, 0.39 mmol, 2.4 equiv.) and ruthenium(III)chloride (0.67 mg, 0.003 mmol, 0.02 equiv.) were added to a stirred solution of 5-(2,3-difluorophenyl)-3-(methylsulfonyl)-6-methyl-2,3-dihydro[1,3]thiazolo[4,5-b]pyridine (55 mg, 0.16 mmol, 1.00 equiv.) in a mixture of abs. dichloromethane (1 mL), acetonitrile (1.5 mL) and water (1.5 mL). The resulting reaction mixture was stirred at room temperature for 16 h and concentrated under reduced pressure, followed by addition of dichloromethane, saturated sodium thiosulfate solution, extraction and phase separation. The aqueous layer was thoroughly extracted with dichloromethane, and the combined organic layer was dried over magnesium sulfate, filtered and concentrated under reduced pressure. The remaining crude product was purified via column chromatography (gradient ethyl acetate/hexane) to afford 5-(2,3-difluorophenyl)-3-(methylsulfonyl)-6-methyl-2,3-dihydro[1,3]thiazolo[4,5-b]pyridine 1,1-dioxide (34 mg, 55 % yield of theory).

¹H-NMR (400 MHz, CDCl₃) δ_{H} 7.98 (s, 1H), 7.34-7.27 (m, 1H), 7.25-7.17 (m, 2H), 4.99 (s, 2H), 3.52 (s, 2H), 2.34 (s, 3H).

In analogy to the preparation examples cited above and recited at the appropriate point, and taking account of the general details relating to the preparation of substituted 2,3-dihydro[1,3]thiazolo[4,5-b]pyridines of the general formula (I), the compounds cited below are obtained.

**Table 1: Examples of preferred compounds of the general formula (I)**

| 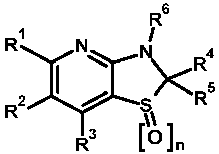 | | | | | | | |
|---|---|---|---|---|---|---|---|
| Ex. No. | R¹ | R² | n | R³ | R⁴ | R⁵ | R⁶ |
| I-001 | 2-fluorophenyl | bromo | 0 | H | H | H | H |
| I-002 | 2-fluorophenyl | chloro | 0 | H | H | H | H |
| I-003 | 2-fluorophenyl | chloro | 0 | H | H | H | 2-(methoxycarbonyl)eth-1-ylcarbonyl |
| I-004 | 2-fluorophenyl | ethoxycarbonyl | 0 | H | H | H | H |
| I-005 | 2-methylphenyl | methyl | 0 | H | H | H | H |
| I-006 | 2-methylphenyl | bromo | 0 | H | H | H | H |
| I-007 | 2-fluorophenyl | methyl | 0 | H | H | H | H |
| I-008 | 2-fluorophenyl | methyl | 0 | H | H | H | methylcarbonyl |
| I-009 | 2-fluorophenyl | bromo | 0 | H | H | H | methylcarbonyl |
| I-010 | 2-fluorophenyl | methyl | 0 | H | H | H | 2-(methoxycarbonyl)eth-1-ylcarbonyl |
| 1-011 | 2-methylphenyl | bromo | 0 | H | H | H | BH₂ |
| I-012 | 2-methylphenyl | 2,3,4,5,6-pentafluorophenyl | 0 | H | H | H | H |
| I-013 | 2-methylphenyl | methyl | 0 | H | H | H | 1-methyleth-1-ylcarbonyl |
| I-014 | 2-methylphenyl | bromo | 0 | H | H | H | methylcarbonyl |
| I-015 | 2-methylphenyl | methyl | 0 | H | H | H | methylcarbonyl |
| I-016 | 2-methylphenyl | bromo | 0 | H | H | H | cyclopentylcarbonyl |
| I-017 | 2-methylphenyl | bromo | 0 | H | H | H | ethylcarbonyl |
| 1-018 | 2-fluorophenyl | methyl | 0 | H | H | H | cyclopropylcarbonyl |
| I-019 | 2-fluorophenyl | bromo | 0 | H | H | H | methylsulfonyl |
| I-020 | 2-fluorophenyl | bromo | 0 | H | H | H | 1-methyleth-1-ylcarbonyl |
| I-021 | 2-fluorophenyl | bromo | 0 | H | H | H | cyclopentylcarbonyl |
| I-022 | 2-fluorophenyl | bromo | 0 | H | H | H | ethylcarbonyl |
| I-023 | 4-methyl-3-thienyl | methyl | 0 | H | H | H | H |
| I-024 | 2,6-difluorophenyl | chloro | 0 | H | H | H | H |
| I-025 | 2,4-difluorophenyl | chloro | 0 | H | H | H | H |
| I-026 | 2,3-difluorophenyl | methyl | 0 | H | H | H | (2-trifluoromethylphenyl)carbonyl |
| I-027 | 2,3-difluorophenyl | methyl | 0 | H | H | H | methylcarbonyl |
| I-028 | 2,3-difluorophenyl | methyl | 0 | H | H | H | 1-methyleth-1-ylcarbonyl |
| I-029 | 2,3-difluorophenyl | methyl | 0 | H | H | H | H |
| I-030 | 2,3-difluorophenyl | bromo | 0 | H | H | H | H |
| I-031 | Phenyl | ethyl | 0 | H | H | H | H |
| I-032 | 2-fluoro-6-methylphenyl | bromo | 0 | H | H | H | H |
| I-033 | 2,3-difluorophenyl | bromo | 0 | H | H | H | ethylcarbonyl |
| I-034 | 2,3-difluorophenyl | bromo | 0 | H | H | H | 1-methyleth-1-ylcarbonyl |
| I-035 | 2,3-difluorophenyl | bromo | 0 | H | H | H | methylsulfonyl |
| I-036 | 2,3-difluorophenyl | bromo | 0 | H | H | H | methylcarbonyl |
| I-037 | 3-chloro-2-thienyl | chloro | 0 | H | H | H | H |
| I-038 | 2,4-difluorophenyl | ethyl | 0 | H | H | H | H |
| I-039 | 2,3-difluorophenyl | chloro | 0 | H | H | H | H |
| I-040 | 2,4-difluorophenyl | bromo | 0 | H | H | H | H |
| I-041 | 2-chlorophenyl | bromo | 0 | H | H | H | H |
| I-042 | 2-chlorophenyl | bromo | 0 | H | H | H | prop-1-ylcarbonyl |
| I-043 | 2,4-difluorophenyl | bromo | 0 | H | H | H | (2-trifluoromethylphenyl)carbonyl |
| I-044 | 2,4-difluorophenyl | methyl | 0 | H | H | H | H |
| I-045 | 2,4-difluorophenyl | bromo | 0 | H | H | H | ethylcarbonyl |
| I-046 | 2,4-difluorophenyl | bromo | 0 | H | H | H | methylcarbonyl |
| I-047 | 2-ethylphenyl | methyl | 0 | H | H | H | H |
| I-048 | 2-chlorophenyl | bromo | 0 | H | H | H | 4-amino-1,3,2,4-dioxadiboretan-2-yl |
| I-049 | 2-chlorophenyl | methyl | 0 | H | H | H | H |
| I-050 | 2,4-difluorophenyl | methyl | 0 | H | H | H | methylcarbonyl |
| I-051 | 2-fluorophenyl | methyl | 0 | H | H | H | 1-methyleth-1-ylcarbonyl |
| I-052 | 2-fluorophenyl | methyl | 0 | H | H | H | ethylcarbonyl |
| I-053 | 2,4-difluorophenyl | methyl | 0 | H | H | H | ethylcarbonyl |
| I-054 | 2,4-difluorophenyl | methyl | 0 | H | H | H | 1-methyleth-1-ylcarbonyl |
| I-055 | 2-chlorophenyl | bromo | 0 | H | H | H | methylcarbonyl |
| I-056 | 2-methylphenyl | bromo | 0 | H | H | H | 4-amino-1,3,2,4-diazadiboretidin-2-yl |
| I-057 | 2-fluorophenyl | methyl | 0 | H | H | H | methylsulfonyl |
| I-058 | Phenyl | bromo | 0 | H | H | H | methylcarbonyl |
| I-059 | 2-fluorophenyl | methyl | 0 | H | H | H | prop-1-ylsulfonyl |
| I-060 | 2-fluorophenyl | methyl | 0 | H | H | H | ethylsulfonyl |
| I-061 | 2-chlorophenyl | bromo | 0 | H | H | H | (2-trifluoromethylphenyl)carbonyl |
| I-062 | Phenyl | bromo | 0 | H | H | H | 1-methyleth-1-ylcarbonyl |
| I-063 | 2-methylphenyl | methyl | 0 | H | H | H | cyclopropylcarbonyl |
| I-064 | Phenyl | bromo | 0 | H | H | H | ethylsulfonyl |
| I-065 | 2-methylphenyl | methyl | 0 | H | H | H | ethylcarbonyl |
| I-066 | Phenyl | bromo | 0 | H | H | H | H |
| I-067 | Phenyl | bromo | 0 | H | H | H | 1-methyleth-1-ylcarbonyl |
| I-068 | Phenyl | bromo | 0 | H | H | H | methylsulfonyl |
| I-069 | Phenyl | methyl | 0 | H | H | H | Methylsulfonyl |
| I-070 | 3-methyl-2-thienyl | bromo | 0 | H | H | H | H |
| I-071 | Phenyl | methyl | 0 | H | H | H | H |
| I-072 | Phenyl | methyl | 0 | H | H | H | methylcarbonyl |
| I-073 | Phenyl | methyl | 0 | H | H | H | 1-methyleth-1-ylcarbonyl |
| I-074 | 2-methylphenyl | methyl | 0 | H | H | H | ethylsulfonyl |
| I-075 | 2-methylphenyl | methyl | 0 | H | H | H | methylsulfonyl |
| I-076 | 3-chloro-2-thienyl | bromo | 0 | H | H | H | 3-(ethoxycarbonyl)prop-1-ylcarbonyl |
| I-077 | 3-chloro-2-thienyl | bromo | 0 | H | H | H | 2-(methoxycarbonyl)eth-1-ylcarbonyl |
| I-078 | 3-chloro-2-thienyl | bromo | 0 | H | H | H | ethoxycarbonylmethyl-carbonyl |
| I-079 | 3-chloro-2-thienyl | bromo | 0 | H | H | H | H |
| I-080 | 2-ethylphenyl | bromo | 0 | H | H | H | B(NH₂)₂ |
| I-081 | 2-ethylphenyl | bromo | 0 | H | H | H | H |
| I-082 | 2-methylphenyl | bromo | 0 | H | H | H | cyclopropylcarbonyl |
| I-083 | 2-fluorophenyl | methyl | 0 | H | H | H | (3-chloro-5-methyl-4,5-dihydro-1,2-oxazol-5-yl)carbonyl |
| I-084 | 2,4-difluorophenyl | methyl | 0 | H | H | H | 2-(methoxycarbonyl)eth-1-ylcarbonyl |
| I-085 | 2,4-difluorophenyl | bromo | 0 | H | H | H | 2-(methoxycarbonyl)eth-1-ylcarbonyl |
| I-086 | 2-methoxyphenyl | bromo | 0 | H | H | H | H |
| I-087 | 2-methoxyphenyl | bromo | 0 | H | H | H | B(NH₂)₂ |
| I-088 | Phenyl | methyl | 0 | H | H | H | (3-chloro-5-methyl-4,5-dihydro-1,2-oxazol-5-yl)carbonyl |
| I-089 | 2,4-difluorophenyl | methyl | 0 | H | H | H | methylsulfonyl |
| I-090 | 2,4-difluorophenyl | bromo | 0 | H | H | H | methylsulfonyl |
| I-091 | 2-methoxyphenyl | methyl | 0 | H | H | H | H |
| I-092 | 2-ethylphenyl | bromo | 0 | H | H | H | methylsulfonyl |
| I-093 | 2-ethylphenyl | bromo | 0 | H | H | H | methylcarbonyl |
| I-094 | 2-ethylphenyl | bromo | 0 | H | H | H | ethylcarbonyl |
| I-095 | 2-ethylphenyl | bromo | 0 | H | H | H | 1-methyleth-1-ylcarbonyl |
| I-096 | 2-thienyl | methyl | 0 | H | H | H | H |
| I-097 | 2-ethylphenyl | methyl | 0 | H | H | H | methylcarbonyl |
| I-098 | 2-ethylphenyl | methyl | 0 | H | H | H | ethylcarbonyl |
| I-099 | 2-methoxyphenyl | methyl | 0 | H | H | H | methylcarbonyl |
| I-100 | 2-ethylphenyl | methyl | 0 | H | H | H | methylsulfonyl |
| I-101 | 2-methoxyphenyl | methyl | 0 | H | H | H | methylsulfonyl |
| I-102 | 2,3-dimethylphenyl | bromo | 0 | H | H | H | H |
| I-103 | 2-fluoro-3-chlorophenyl | bromo | 0 | H | H | H | H |
| I-104 | 2-fluorophenyl | methyl | 0 | H | H | H | 3-chloro-4,5,6,6a-tetrahydro-3aH-cyclopenta[d][1,2]oxa-zol-6a-ylcarbonyl |
| I-105 | 2,3-dimethylphenyl | bromo | 0 | H | H | H | B(NH₂)₂ |
| I-106 | 3-fluorophenyl | bromo | 0 | H | H | H | H |
| I-107 | 2,3-dimethylphenyl | methyl | 0 | H | H | H | H |
| I-108 | 2,3-dimethylphenyl | methyl | 0 | H | H | H | methylsulfonyl |
| I-109 | 2,3-dimethylphenyl | methyl | 0 | H | H | H | prop-1-ylsulfonyl |
| I-110 | 2-fluoro-3-chlorophenyl | methyl | 0 | H | H | H | H |
| I-111 | 2-fluoro-3-chlorophenyl | methyl | 0 | H | H | H | methylsulfonyl |
| I-112 | 2-fluoro-3-chlorophenyl | methyl | 0 | H | H | H | prop-1-ylsulfonyl |
| I-113 | 2-fluoro-3-methylphenyl | methyl | 0 | H | H | H | H |
| I-114 | 2-fluoro-3-chlorophenyl | bromo | 0 | H | H | H | prop-1-ylsulfonyl |
| I-115 | 2-fluoro-3-chlorophenyl | bromo | 0 | H | H | H | methylcarbonyl |
| I-116 | 2-fluoro-3-chlorophenyl | bromo | 0 | H | H | H | methylsulfonyl |
| I-117 | 3-fluorophenyl | methyl | 0 | H | H | H | H |
| I-118 | 3-fluorophenyl | methyl | 0 | H | H | H | methylsulfonyl |
| I-119 | 2-fluorophenyl | methyl | 0 | H | H | H | prop-2-en-1-ylsulfonyl |
| I-120 | 2-fluorophenyl | methyl | 0 | H | H | H | prop-2-en-2-ylsulfonyl |
| I-121 | 2-fluorophenyl | methyl | 0 | H | H | H | methoxycarbonylmethyl-sulfonyl |
| I-122 | 2-fluorophenyl | methyl | 0 | H | H | H | (4-fluorophenyl)methylsulfonyl |
| I-123 | 2-fluorophenyl | methyl | 0 | H | H | H | cyanomethylsulfonyl |
| I-124 | 2-fluorophenyl | methyl | 0 | H | H | H | formyl |
| I-125 | 3-fluorophenyl | methyl | 0 | H | H | H | methylsulfonyl |
| I-126 | 2-fluoro-3-chlorophenyl | bromo | 0 | H | H | H | 3-chloro-4,5,6,6a-tetrahydro-3aH-cyclopenta[d] [1,2]oxazol-6a-ylcarbonyl |
| I-127 | 2-fluorophenyl | methyl | 0 | H | H | H | 2-ethoxyeth-1-ylsulfonyl |
| I-128 | 2-fluorophenyl | methyl | 0 | H | H | H | 3-chloro-5-methoxymethyl-4,5-dihydro-1,2-oxazol-5-ylcarbonyl |
| I-129 | 2-methylphenyl | methyl | 0 | H | H | H | 2-ethoxyeth-1-ylsulfonyl |
| I-130 | 2-methylphenyl | methyl | 0 | H | H | H | cyanomethylsulfonyl |
| I-131 | 2-fluoro-3-chlorophenyl | bromo | 0 | H | H | H | cyanomethylsulfonyl |
| I-132 | 2-fluoro-3-chlorophenyl | bromo | 0 | H | H | H | ethylsulfonyl |
| I-133 | 2-chlorophenyl | bromo | 0 | H | H | H | methylsulfonyl |
| I-134 | 2-chlorophenyl | bromo | 0 | H | H | H | ethylsulfonyl |
| I-135 | 2,4-difluorophenyl | bromo | 0 | H | H | H | ethylsulfonyl |
| I-136 | 2,4-difluorophenyl | bromo | 0 | H | H | H | methoxycarbonylmethyl-sulfonyl |
| I-137 | 2-methylphenyl | methyl | 0 | H | H | H | (3-chloro-5-methyl-4,5-dihydro-1,2-oxazol-5-yl)carbonyl |
| I-138 | 2,3-dimethylphenyl | methyl | 0 | H | H | H | (3-chloro-5-methyl-4,5-dihydro-1,2-oxazol-5-yl)carbonyl |
| I-139 | Phenyl | bromo | 0 | H | H | H | (3-chloro-5-methyl-4,5-dihydro-1,2-oxazol-5-yl)carbonyl |
| I-140 | 2,3-dimethylphenyl | bromo | 0 | H | H | H | (3-chloro-5-methyl-4,5-dihydro-1,2-oxazol-5-yl)carbonyl |
| I-141 | 2-chlorophenyl | bromo | 0 | H | H | H | (3-chloro-5-methyl-4,5-dihydro-1,2-oxazol-5-yl)carbonyl |
| I-142 | 2-chlorophenyl | methyl | 0 | H | H | H | methylsulfonyl |
| I-143 | 2-chlorophenyl | methyl | 0 | H | H | H | methylcarbonyl |
| I-144 | 2-chlorophenyl | methyl | 0 | H | H | H | prop-1-ylsulfonyl |
| I-145 | 2-3,difluorophenyl | methyl | 0 | H | H | H | prop-1-ylsulfonyl |
| I-146 | 2-fluorophenyl | methyl | 2 | H | H | H | ethylsulfonyl |
| I-147 | 2,3-difluorophenyl | methyl | 0 | H | H | H | methylsulfonyl |
| I-148 | 2,3-difluorophenyl | bromo | 0 | H | H | H | methoxyethoxyethoxy-methylcarbonyl |
| I-149 | 2,3-difluorophenyl | methyl | 0 | H | H | H | methoxyethoxyethoxy-methylcarbonyl |
| I-150 | 2-fluorophenyl | methyl | 0 | H | H | H | methoxyethoxyethoxy-methylcarbonyl |
| I-151 | 2,3-difluorophenyl | methyl | 2 | H | H | H | methylsulfonyl |
| I-152 | 2,3-difluorophenyl | methyl | 0 | H | H | H | (3-chloro-5-methyl-4,5-dihydro-1,2-oxazol-5-yl)carbonyl |
| I-153 | 2,3-difluorophenyl | methyl | 0 | H | H | H | 2-(methoxycarbonyl)eth-1-ylcarbonyl |
| I-154 | 2,3-difluorophenyl | methyl | 0 | H | H | H | prop-2-en-2-ylsulfonyl |
| I-155 | 2,3-difluorophenyl | methyl | 0 | H | H | H | 2-ethoxyeth-1-ylsulfonyl |
| I-156 | 2,3-difluorophenyl | methyl | 0 | H | H | H | 3-chloro-4,5,6,6a-tetrahydro-3aH-cyclopenta[d][1,2]oxazol-6a-ylcarbonyl |
| I-157 | 2,3-difluorophenyl | methyl | 0 | H | H | H | prop-2-en-1-ylsulfonyl |
| I-158 | 2,5-difluorophenyl | bromo | 0 | H | H | H | methylcarbonyl |
| I-159 | 2,5-difluorophenyl | bromo | 0 | H | H | H | methylsulfonyl |
| I-160 | 2,5-difluorophenyl | bromo | 0 | H | H | H | prop-1-ylsulfonyl |
| I-161 | 2-fluorophenyl | methyl | 0 | H | H | H | hydroxycarbonylmethoxy-ethoxymethylcarbonyl |
| I-162 | 2,4,5-trifluorophenyl | methyl | 0 | H | H | H | prop-1-ylsulfonyl |
| I-163 | 2,4,5-trifluorophenyl | methyl | 0 | H | H | H | ethylcarbonyl |
| I-164 | 2,4,5-trifluorophenyl | methyl | 0 | H | H | H | methylcarbonyl |
| I-165 | 2,4,5-trifluorophenyl | methyl | 0 | H | H | H | methylsulfonyl |
| I-166 | 2,3,4-trifluorophenyl | bromo | 0 | H | H | H | H |
| I-167 | 2,5-difluorophenyl | methyl | 0 | H | H | H | ethylcarbonyl |
| I-168 | 2,5-difluorophenyl | methyl | 0 | H | H | H | methylcarbonyl |
| I-169 | 2,5-difluorophenyl | methyl | 0 | H | H | H | prop-1-ylsulfonyl |
| I-170 | 2,5-difluorophenyl | methyl | 0 | H | H | H | H |
| I-171 | 2,5-difluorophenyl | methyl | 0 | H | H | H | methylsulfonyl |
| I-172 | 2-fluorophenyl | methyl | 0 | H | H | H | hydroxyethoxy-ethoxymethylcarbonyl |
| I-173 | 2,4,5-trifluorophenyl | bromo | 0 | H | H | H | methylcarbonyl |
| I-174 | 2-chlorophenyl | methyl | 0 | H | H | H | prop-1-ylcarbonyl |
| I-175 | 2-fluorophenyl | methyl | 0 | H | ethyl | H | H |
| I-176 | 2-fluorophenyl | methyl | 0 | H | cyclobutyl | H | H |
| I-177 | 2-fluorophenyl | methyl | 0 | H | ethyl | methyl | H |
| I-178 | 2,4,5-trifluorophenyl | bromo | 0 | H | H | H | formyl |
| I-179 | 2,3,4-trifluorophenyl | methyl | 0 | H | H | H | H |
| I-180 | 2-fluorophenyl | methyl | 0 | H | cyclopropyl | H | H |
| I-181 | 2-fluorophenyl | methyl | 0 | H | methoxymethyl | methyl | H |
| I-182 | 2,3,4-trifluorophenyl | methyl | 0 | H | H | H | methylsulfonyl |
| I-183 | Phenyl | methyl | 0 | H | H | H | ethylsulfonyl |
| I-184 | Phenyl | methyl | 0 | H | H | H | ethylcarbonyl |
| I-185 | 2,3,4-trifluorophenyl | methyl | 0 | H | H | H | ethylcarbonyl |
| I-186 | Phenyl | methyl | 0 | H | H | H | prop-1-ylsulfonyl |
| I-187 | 2-fluorophenyl | methyl | 0 | H | H | H | 4-trifluoromethylpyridin-3-ylcarbonyloxyethoxy-ethoxymethylcarbonyl |
| I-188 | 2-fluorophenyl | methyl | 0 | H | prop-2-yl | methyl | H |
| I-189 | 2-fluorophenyl | methyl | 0 | H | cyclopropyl | methyl | H |
| I-190 | 2-fluorophenyl | methyl | 0 | H | cyclobutyl | methyl | H |
| I-191 | 2-fluorophenyl | methyl | 0 | H | methyl | methyl | H |
| I-192 | 2-fluorophenyl | methyl | 0 | H | -CH₂-CH₂-O-CH₂-CH₂- | | H |

### Spectroscopic data of selected Table examples:

The spectroscopic data listed hereinafter for selected Table examples were evaluated via conventional ¹H-NMR interpretation or via NMR peak list methods.

### a) Conventional ¹H-NMR interpretation

I-004: ¹H-NMR(400 MHz, CDCl₃) δ_{H} 8.15 (s,1H), 7.68 (s,1H), 7.40-7.33 (m,2H), 7.23-7.15 (m,2H), 4.99 (s,2H), 3.97 (q,2H), 0.96 (t,3H).

I-023: ¹H-NMR(400 MHz, CDCl₃) δ_{H} 7.13 (d,1H), 7.10 (s,1H), 6.98 (m,1H), 5.79 (bs,1H), 4.73 (s,2H), 2.10 (d,3H), 1.99 (s,3H).

I-028: ¹H-NMR(400 MHz, CDCl₃) δ_{H} 7.35 (s, 1H), 7.23-7.19 (m, 1H), 7.17-7.12 (m, 2H), 5.33 (s, 2H), 4.08-4.02 (sept, 1H), 2.17 (s, 3H), 1.16 (d, 6H).

I-029: ¹H-NMR(400 MHz, CDCl₃) δ_{H} 7.19-7.09 (m, 4H), 5.49-5.39 (br. s, 1H, NH), 4.83 (s, 2H), 2.04 (s, 3H).

I-030: ¹H-NMR(400 MHz, CDCl₃) δ_{H} 7.39 (s, 1H), 7.24-7.17 (m, 1H), 7.15-7.09 (m, 2H), 5.23 (br. s, 1H, NH), 4.96 (s, 2H).

I-036: ¹H-NMR(400 MHz, CDCl₃) δ_{H} 7.66 (s, 1H), 7.25-7.20 (m, 1H), 7.17-7.13 (m, 2H), 5.36 (s, 2H), 2.58 (s, 3H).

I-040: ¹H-NMR(400 MHz, CDCl₃) δ_{H} 7.37 (s, 1H), 7.36-7.30 (m, 1H), 6.96-6.91 (m, 1H), 6.90-6.85 (m, 1H), 5.39 (br. s, 1H, NH), 4.91 (s, 2H).

I-044: ¹H-NMR(400 MHz, CDCl₃) δ_{H} 7.36-7.29 (m, 1H), 7.11 (s, 1H), 6.97-6.92 (m, 1H), 6.87-6.83 (m, 1H), 5.53 (br. s, 1H, NH), 4.78 (s, 2H), 2.01 (s, 3H).

I-050: ¹H-NMR(400 MHz, CDCl₃) δ_{H} 7.39-7.34 (m, 1H), 7.33 (s, 1H), 7.00-6.95 (m, 1H), 6.93-6.87 (m, 1H), 5.32 (s, 2H), 2.60 (s, 3H), 2.15 (s, 3H).

I-057: ¹H-NMR(400 MHz, CDCl₃) δ_{H} 7.44-7.37 (m, 2H), 7.36 (s, 1H), 7.24-7.20 (m, 1H), 7.15-7.10 (m, 1H), 5.30 (s, 2H), 3.41 (s, 3H), 2.17 (s, 3H).

I-058: ¹H-NMR(400 MHz, CDCl₃) δ_{H} 7.69-7.67 (m, 2H), 7.66 (s, 1H), 7.47-7.41 (m, 3H), 5.35 (s, 2H), 2.65 (s, 3H).

I-059: ¹H-NMR(400 MHz, CDCl₃) δ_{H} 7.42-7.35 (m, 2H), 7.33 (s, 1H), 7.24-7.20 (m, 1H), 7.15-7.10 (m, 1H), 5.30 (s, 2H), 3.65-3.61 (t, 2H), 2.16 (s, 3H), 1.91-1.83 (sext, 2H), 1.05-1.03 (t, 3H).

I-062: ¹H-NMR(400 MHz, CDCl₃) δ_{H} 7.69-7.67 (m, 2H), 7.64 (s, 1H), 7.48-7.42 (m, 3H), 5.36 (s, 2H), 3.10-3.05 (q, 2H), 1.21-1.17 /t, 3H).

I-063: ¹H-NMR(400 MHz, CD₃OD) δ_{H} 7.36 (s, 1H), 7.29-7.21 (m, 3H), 7.15-7.14 (d, 1H), 5.35 (s, 2H), 2.14 (s, 3H), 2.03 (s, 3H), 1.73-1.66 (m, 1H), 1.19-1.15 (m, 2H), 0.84-0.80 (m, 2H).

I-064: ¹H-NMR(400 MHz, CDCl₃) δ_{H} 7.72-7.68 (m, 2H), 7.65 (s, 1H), 7.46-7.39 (m, 3H), 5.35 (s, 2H), 3.70-3.64 (q, 2H), 1.41-1.38 (t, 3H).

I-065: ¹H-NMR(400 MHz, CDCl₃) δ_{H} 7.33 (s, 1H), 7.32-7.22 (m, 3H), 7.14-7.11 (d, 1H), 5.34 (s, 2H), 3.06-2.98 (q, 2H), 2.14 (s, 3H), 2.01 (s, 3H), 1.13-1.10 (t, 3H).

I-066: ¹H-NMR(400 MHz, CDCl₃) δ_{H} 7.55-7.653 (m, 2H), 7.44-7.37 (m, 3H), 7.35 (s, 1H), 6.07-6.00 (br. s, 1H, NH), 4.68 (s, 2H).

I-067: ¹H-NMR(400 MHz, CDCl₃) δ_{H} 7.69-7.67 (m, 2H), 7.65 (s, 1H), 7.47-7.42 (m, 3H), 5.35 (s, 2H), 2.70-2.62 (sept, 1H), 1.25 (d, 3H), 1.20 (d, 3H).

I-068: ¹H-NMR(400 MHz, CDCl₃) δ_{H} 7.72-7.69 (m, 2H), 7.66 (s, 1H), 7.45-7.40 (m, 3H), 5.33 (s, 2H), 3.42 (s, 3H).

I-069: ¹H-NMR(400 MHz, CDCl₃) δ_{H} 7.55-7.53 (m, 2H), 7.45-7.37 (m, 3H), 7.35 (s, 1H), 5.32 (s, 2H), 3.51 (s, 3H), 2.32 (s, 3H).

I-070: ¹H-NMR (400 MHz, CDCl₃): δ_{H} 7.34 (s, 1H), 7.28-7.25 (m, 1H), 6.89-6.86 (m, 1H), 6.05-5.94 (br m, 1H), 4.82-4.80 (m, 2H), 2.16 (s, 3H).

I-071: ¹H-NMR(400 MHz, CDCl₃) δ_{H} 7.44-7.38 (m, 4H), 7.35-7.31 (m, 1H), 7.10 (s, 1H), 6.00-5.88 (br. s, 1H, NH), 4.65 (s, 2H), 2.13 (s, 3H).

I-072: ¹H-NMR(400 MHz, CDCl₃) δ_{H} 7.54-7.51 (m, 2H), 7.46-7.36 (m, 3H), 7.34 (s, 1H), 5.32 (s, 2H), 2.68 (s, 3H), 2.32 (s, 3H).

I-073: ¹H-NMR(400 MHz, CDCl₃) δ_{H} 7.54-7.51 (m, 2H), 7.47-7.38 (m, 3H), 7.33 (s, 1H), 5.32 (s, 2H), 4.21-4.13 (sept, 1H), 2.32 (s, 3H), 1.20 (d, 6H).

I-074: ¹H-NMR(400 MHz, CDCl₃) δ_{H} 7.36 (s, 1H), 7.29-7.21 (m, 3H), 7.15-7.14 (d, 1H), 5.32 (s, 2H), 3.66-3.60 (q, 2H), 2.15 (s, 3H), 2.03 (s, 3H), 1.37-1.34 (t, 3H).

I-075: ¹H-NMR(400 MHz, CDCl₃) δ_{H} 7.38 (s, 1H), 7.30-7.22 (m, 3H), 7.16-7.14 (d, 1H), 5.32 (s, 2H), 3.38 (s, 3H), 2.15 (s, 3H), 2.04 (s, 3H).

I-076: ¹H-NMR (400 MHz, CDCl₃): δ_{H} 7.64 (s, 1H), 7.41-7.38 (m, 1H), 7.01-6.98 (m, 1H), 5.35 (s, 2H), 4.19-4.05 (m, 2H), 3.08 (t, *J =* 8.0 Hz, 2H), 2.51-2.32 (m, 2H), 2.16-1.95 (m, 2H), 1.29-1.19 (m, 3H).

I-084: ¹H-NMR(400 MHz, CDCl₃) δ_{H} 7.40-7.35 (m, 1H), 7.33 (s, 1H), 6.99-6.94 (m, 1H), 6.92-6.87 (m, 1H), 5.32 (s, 2H), 3.67 (s, 3H), 3.36-3.33 (t, 2H), 2.69-2.66 (t, 2H), 2.15 (s, 3H).

I-085: ¹H-NMR(400 MHz, CDCl₃) δ_{H} 7.64 (s, 1H), 7.43-7.37 (m, 1H), 6.97-6.94 (m, 1H), 6.92-6.88 (m, 1H), 5.35 (s, 2H), 3.67 (s, 3H), 3.31-3.28 (t, 2H), 2.69-2.65 (t, 2H).

I-088: ¹H-NMR(400 MHz, CDCl₃) δ_{H} 7.49-7.43 (m, 4H), 7.42-7.39 (m, 1H), 7.38 (s, 1H), 5.37-5.34 (d, 1H), 5.31-5.29 (d, 1H), 3.94-3.90 (d, 1H), 3.08-3.04 (d, 1H), 2.28 (s, 3H), 1.90 (s, 3H).

I-105: ¹H-NMR(400 MHz, CDCl₃) δ_{H} 7.22-7.20 (d, 1H), 7.16-7.12 (dd, 1H), 7.11 (s, 1H), 6.89-6.87 5.14 (d, 1H), 5.12 (d, 1H), 2.61-2.46 (br. m, 4H), 2.31 (s, 3H), 2.03 (s, 3H).

I-106: ¹H-NMR(400 MHz, CDCl₃) δ_{H} 7.40-7.34 (m, 3H), 7.30-7.25 (m, 1H), 7.09-7.04 (m, 1H), 5.42-5.34 (br. m, 1H), 4.89 (s, 2H).

I-107: ¹H-NMR(400 MHz, CDCl₃) δ_{H} 7.14-7.09 (m, 3H), 7.07 (s, 1H), 6.99-6.96 (m, 1H), 6.44-6.39 (br. s, 1H), 4.51 (s, 2H), 2.30 (s, 3H), 2.01 (s, 3H), 1.87 (s, 3H).

I-108: ¹H-NMR(400 MHz, CDCl₃) δ_{H} 7.36 (s, 1H), 7.17 (d, 1H), 7.16-7.12 (m, 1H), 7.01-6.99 (m, 1H), 5.37-5.32 (br. m, 1H), 5.29-5.24 (br. m, 1H), 3.37 (s, 3H), 2.33 (s, 3H), 2.03 (s, 3H), 2.02 (s, 3H).

I-109: ¹H-NMR(400 MHz, CDCl₃) δ_{H} 7.36 (s, 1H), 7.20 (d, 1H), 7.16-7.12 (m, 1H), 7.01-6.99 (m, 1H), 5.40-5.33 (br. m, 1H), 5.31-5.26 (br. m, 1H), 3.62-3.58 (m, 2H), 2.33 (s, 3H), 2.03 (s, 3H), 2.01 (s, 3H), 1.90-1.82 (m, 2H), 1.01 (t, 3H).

I-110: ¹H-NMR(400 MHz, CDCl₃) δ_{H} 7.24-7.04 (m, 4H), 5.42-5.35 (br. s, 1H, NH), 4.81 (s, 2H), 2.31 (s, 3H), 2.02 (s, 3H).

I-111: ¹H-NMR(400 MHz, CDCl₃) δ_{H} 7.46-7.43 (m, 1H), 7.36 (s, 1H), 7.32-7.28 (m, 1H), 7.18-7.15 (m, 1H), 5.30 (s, 2H), 3.39 (s, 3H), 2.16 (s, 3H).

I-112: ¹H-NMR(400 MHz, CDCl₃) δ_{H} 7-46-7.42 (m, 1H), 7.34 (s, 1H), 7.31-7.27 (m, 1H), 7.18-7.14 (m, 1H), 5.31 (s, 2H), 3.62-3.58 (m, 2H), 2.16 (s, 3H), 1.91-1.84 (m, 2H), 1.06-1.02 (t, 3H).

I-113: ¹H-NMR(400 MHz, CDCl₃) δ_{H} 7.40-7.36 (m, 1H), 7.23-7.04 (m, 3H), 5.30-5.26 (br. s, 1H, NH), 4.83 (s, 2H), 2.03 (s, 3H).

I-114: ¹H-NMR(400 MHz, CDCl₃) δ_{H} 7.64 (s, 1H), 7.49-7.45 (m, 1H), 7.34-7.30 (m, 1H), 7.18-7.14 (m, 1H), 5.34 (s, 2H), 3.60-3.56 (m, 2H), 1.90-1.84 (m, 2H), 1.07-1.03 (t, 3H).

I-115: ¹H-NMR(400 MHz, CDCl₃) δ_{H} 7.66 (s, 1H), 7.50-7.46 (m, 1H), 7.32-7.28 (m, 1H), 7.19-7.15 (m, 1H), 5.36 (s, 2H), 2.57 (s, 3H).

I-116: ¹H-NMR(400 MHz, d₆-DMSO) δ_{H} 8.15 (s, 1H), 7.73-7.69 (m, 1H), 7.47-7.43 (m, 1H), 7.38-7.34 (m, 1H), 5.34 (s, 2H), 3.38 (s, 3H).

I-117: ¹H-NMR(400 MHz, CDCl₃) δ_{H} 7.39-7.34 (m, 1H), 7.23-7.20 (m, 1H), 7.17-7.14 (m, 1H), 7.13 (s, 1H), 5.26-5.20 (br. s, 1H), 4.86 (s, 2H), 2.16 (s, 1H).

I-119: ¹H-NMR(400 MHz, CDCl₃) δ_{H} 7.43-7.37 (m, 2H), 7.33 (s, 1H), 7.25-7.20 (m, 1H), 7.16-7.11 (m, 1H), 5.92-5.84 (m, 1H), 5.43-5.40 (d, 1H), 5.36-5.31 (d, 1H), 5.27 (s, 2H), 4.39 (s, 2H), 2.16 (s, 3H).

I-120: ¹H-NMR(400 MHz, CDCl₃) δ_{H} 7.38-7.34 (m, 2H), 7.33 (s, 1H), 7.22-7.18 (m, 1H), 7.12-7.07 (m, 1H), 6.23 (m, 1H), 5.74 (m, 1H), 5.34 (s, 2H), 2.15 (m, 3H), 2.11 (s, 3H).

I-121: ¹H-NMR(400 MHz, CDCl₃) δ_{H} 7.41-7.36 (m, 2H), 7.34 (s, 1H), 7.24-7.20 (m, 1H), 7.15-7.12 (m, 1H), 5.37 (s, 2H), 4.68 (s, 2H), 3.77 (s, 3H), 2.17 (s, 3H).

I-122: ¹H-NMR(400 MHz, CDCl₃) δ_{H} 7.48-7.43 (m, 1H), 7.42-7.38 (m, 1H), 7.34 (s, 1H), 7.29-7.23 (m, 3H), 7.18-7.14 (m, 3H), 4.85 (s, 2H), 4.84 (s, 2H), 2.20 (s, 3H).

I-123: ¹H-NMR(400 MHz, CDCl₃) δ_{H} 7.42-7.35 (m, 3H), 7.25-7.22 (m, 1H), 7.18-7.13 (m, 1H), 5.42 (s, 2H), 4.73 (s, 2H), 2.20 (s, 3H).

I-124: ¹H-NMR(400 MHz, CDCl₃) δ_{H} 9.28 (s, 1H), 7.41-7.37 (m, 2H), 7.36 (s, 1H), 7.25-7.22 (m, 1H), 7.16-7.12 (m, 1H), 5.26 (s, 2H), 2.16 (s, 3H); ¹³C-NMR(150 MHz, CDCl₃) δ_{c} 161.5, 160.1, 149.1, 147.9, 132.1, 131.4, 130.22, 129.3, 127.3, 124.3, 123.7, 115.8, 47.1, 18.6.

I-125: ¹H-NMR(400 MHz, CDCl₃) 7.42-7.36 (m, 1H), 7.35 (s, 1H), 7.34-7.31 (m, 1H), 7.26-7.22 (m, 1H), 7.09-7.05 (m, 1H), 5.30 (s, 2H), 3.44 (s, 3H), 2.32 (s, 3H).

I-126: ¹H-NMR(400 MHz, CDCl₃) δ_{H} 7.71 (s, 1H), 7.48-7.42 (m, 1H), 7.41-7.38 (m, 1H), 7.18-7.14 (m, 1H), 5.39 (d, 1H), 5.31 (d, 1H), 4.36-4.33 (m, 1H), 2.82-2.75 (m, 1H), 2.20-2.10 (m, 1H), 2.08-2.01 (m, 1H), 1.99-1.92 (m, 1H), 1.85-1.78 (m, 1H), 1.69-1.59 (m, 1H).

I-127: ¹H-NMR(400 MHz, CDCl₃) δ_{H} 7.43-7.34 (m, 2H), 7.31 (s, 1H), 7.23-7.19 (m, 1H), 7.14-7.09 (m, 1H), 5.29 (s, 2H), 3.89-3.83 (m, 4H), 3.44-3.39 (q, 2H), 2.15 (s, 3H), 1.11-1.07 (t, 3H).

I-128: ¹H-NMR(400 MHz, CDCl₃) δ_{H} 7.42-7.33 (m, 3H), 7.25-7.21 (m, 1H), 7.19-7.11 (m, 1H), 5.43-5.40 (d, 1H), 5.30-5.28 (d, 1H), 4.17-4.14 (d, 1H), 3.92-3.89 (d, 1H), 3.75-3.71 (d, 1H), 3.44-3.40 (d, 1H), 3.25 (s, 3H), 2.16 (s, 3H).

I-129: ¹H-NMR(400 MHz, CDCl₃) δ_{H} 7.35 (s, 1H), 7.30-7.20 (m, 3H), 7.14-7.10 (m, 1H), 5.36 (s, 2H), 4.72 (m, 2H), 4.62 (br. m, 2H), 3.75 (m, 2H), 2.14 (s, 3H), 2.03 (s, 3H), 1.28-1.25 (t, 3H).

I-130: ¹H-NMR(400 MHz, CDCl₃) δ_{H} 7.43 (s, 1H), 7.32-7.19 (m, 3H), 7.13-7.10 (m, 1H), 5.84 (br. m, 1H), 5.42 (br. m, 1H), 4.67 (s, 2H), 2.14 (s, 3H), 2.06 (s, 3H).

I-131: ¹H-NMR(400 MHz, CDCl₃) δ_{H} 7.75 (s, 1H), 7.52-7.48 (m, 1H), 7.34-7.29 (m, 1H), 7.20-7.16 (m, 1H), 5.48 (s, 2H), 4.65 (s, 2H), 2.20 (s, 3H).

I-132: ¹H-NMR(400 MHz, CDCl₃) δ_{H} 7.65 (s, 1H), 7.49-7.45 (m, 1H), 7.34-7.30 (m, 1H), 7.18-7.14 (m, 1H), 5.36 (s, 2H), 3.65-3.60 (q, 2H), 1.41-1.38 (t, 3H).

I-133: ¹H-NMR(400 MHz, CDCl₃) δ_{H} 7.66 (s, 1H), 7.48-7.44 (m, 1H), 7.38-7.28 (m, 3H), 5.48-5.41 (m, 1H), 5.40-5.29 (m, 1H), 3.38 (s, 3H).

I-134: ¹H-NMR(400 MHz, CDCl₃) δ_{H} 7.65 (s, 1H), 7.48-7.45 (m, 1H), 7.39-7.33 (m, 3H), 5.48-5.39 (m, 1H), 5.37-5.28 (m, 1H), 3.81-3.71 (m, 1H), 3.62-3.49 (m, 1H), 1.39-1.35 (t, 3H).

I-135: ¹H-NMR(400 MHz, CDCl₃) δ_{H} 7.64 (s, 1H), 7.44-7.39 (m, 1H), 6.99-6.93 (m, 1H), 6.92-6.87 (m, 1H), 5.35 (s, 2H), 3.65-3.60 (q, 2H), 1.41-1.37 (t, 3H).

I-136: ¹H-NMR(400 MHz, CDCl₃) δ_{H} 7.65 (s, 1H), 7.45-7.39 (m, 1H), 6.96-6.92 (m, 1H), 6.90-6.86 (m, 1H), 5.42 (s, 2H), 4.62 (s, 2H), 3.78 (s, 3H).

I-137: ¹H-NMR(400 MHz, CDCl₃) δ_{H} 7.38 (s, 1H), 7.30-7.22 (m, 3H), 7.13-7.10 (m, 1H), 5.34 (s, 2H), 3.87-3.82 (d, 1H), 2.95-2.90 (d, 1H), 2.12 (s, 3H), 2.00 (s, 3H), 1.75 (s, 3H).

I-138: ¹H-NMR(400 MHz, CDCl₃) δ_{H} 7.38 (s, 1H), 7.19-7.13 (m, 2H), 6.97-6.93 (m, 1H), 5.34 (s, 2H), 3.88-3.84 / 3.82-3.78 (d, 1H), 3.00-2.95 / 2.92-2.88 (d, 1H), 2.33 (s, 3H), 2.01 (s, 3H), 1.99 (s, 3H), 1.74 (s, 3H).

I-139: ¹H-NMR(400 MHz, CDCl₃) δ_{H} 7.72 (s, 1H), 7.65-7.62 (m, 2H), 7.46-7.40 (m, 3H), 5.39-5.37 (d, 1H), 5.34-5.32 (d, 1H), 3.98-3.93 (d, 1H), 3.06-3.02 (d, 1H), 1.88 (s, 3H).

I-140: ¹H-NMR(400 MHz, CDCl₃) δ_{H} 7.71 (s, 1H), 7.22-7.12 (m, 2H), 7.00-6.97 (m, 1H), 5.37 (s, 2H), 3.91-3.86 / 3.85-3.80 (d, 1H), 2.99-2.94 / 2.90-2.85 (d, 1H), 2.34 (s, 3H), 2.07 (s, 3H), 1.75 (s, 3H).

I-143: ¹H-NMR(400 MHz, CDCl₃) δ_{H} 7.48-7.44 (m, 1H), 7.37-7.32 (m, 3H), 7.29-7.27 (m, 1H), 5.42-5.30 (br. m, 2H), 2.58 (s, 3H), 2.08 (s, 3H).

I-144: ¹H-NMR(400 MHz, CDCl₃) δ_{H} 7.47-7.43 (m, 1H), 7.37-7.27 (m, 1H), 5.38-5.21 (br. m, 2H), 3.38 (s, 3H), 2.07 (s, 3H).

### b) NMR peak list method

¹H-NMR data of selected examples are written in form of ¹H-NMR-peak lists. To each signal peak are listed the δ-value in ppm and the signal intensity in round brackets. Between the δ-value - signal intensity pairs are semicolons as delimiters. The peak list of an example has therefore the form:
δ₁ (intensity₁); δ₂ (intensity,);........; δᵢ (intensity_{y});...... ; δₙ (intensityₙ)

Intensity of sharp signals correlates with the height of the signals in a printed example of a NMR spectrum in cm and shows the real relations of signal intensities. From broad signals several peaks or the middle of the signal and their relative intensity in comparison to the most intensive signal in the spectrum can be shown. For calibrating chemical shift for ¹H spectra, we use tetramethylsilane and/or the chemical shift of the solvent used, especially in the case of spectra measured in DMSO. Therefore, in NMR peak lists, tetramethylsilane peak can occur but not necessarily. The ¹H-NMR peak lists are similar to classical ¹H-NMR prints and contains therefore usually all peaks, which are listed at classical NMR-interpretation. Additionally, they can show like classical ¹H-NMR prints signals of solvents, stereoisomers of the target compounds, which are also object of the invention, and/or peaks of impurities. To show compound signals in the delta-range of solvents and/or water the usual peaks of solvents, for example peaks of DMSO in DMSO-D₆ and the peak of water are shown in our ¹H-NMR peak lists and have usually on average a high intensity. The peaks of stereoisomers of the target compounds and/or peaks of impurities have usually on average a lower intensity than the peaks of target compounds (for example with a purity >90%). Such stereoisomers and/or impurities can be typical for the specific preparation process. Therefore, their peaks can help to recognize the reproduction of our preparation process via "side-products-fingerprints". An expert, who calculates the peaks of the target compounds with known methods (MestreC, ACD-simulation, but also with empirically evaluated expectation values) can isolate the peaks of the target compounds as needed optionally using additional intensity filters. This isolation would be similar to relevant peak picking done in the course of a classical ¹H-NMR interpretation. Further details of NMR-data description with peak lists can be found in the publication "Citation of NMR Peaklist Data within Patent Applications" of the Research Disclosure Database Number 564025.

### I-002: ¹H-NMR (400.6 MHz, CDCl₃)

δ= 7.4132 (0.8); 7.4086 (1.3); 7.4002 (1.0); 7.3972 (1.9); 7.3954 (2.1); 7.3923 (3.8); 7.3872 (0.8); 7.3819 (1.3); 7.3773 (5.1); 7.3745 (4.4); 7.3726 (3.2); 7.3678 (0.5); 7.3598 (2.3); 7.3581 (3.7); 7.2598 (28.6); 7.2360 (3.0); 7.2331 (3.3); 7.2171 (3.7); 7.2154 (3.1); 7.2135 (2.7); 7.1985 (2.4); 7.1955 (2.8); 7.1925 (15.5); 7.1603 (2.5); 7.1578 (1.7); 7.1415 (0.6); 7.1388 (2.3); 7.1358 (3.1); 7.1334 (2.0); 7.1302 (1.0); 7.1161 (1.0); 7.1142 (2.0); 7.1114 (1.2); 6.4422 (1.5); 5.2989 (2.1); 4.6668 (16.0); 1.5824 (0.6); 1.2546 (0.8); 0.0695 (1.8); 0.0080 (0.8); -0.0002 (31.6); -0.0085 (0.9)

### I-003: ¹H-NMR (400.6 MHz, CDCl₃)

δ= 7.4911 (6.3); 7.4585 (0.7); 7.4537 (0.5); 7.4438 (0.8); 7.4404 (1.8); 7.4347 (0.8); 7.4287 (0.5); 7.4209 (1.5); 7.4149 (0.7); 7.4015 (0.5); 7.2608 (10.7); 7.2580 (1.4); 7.2550 (1.2); 7.2494 (0.6); 7.2389 (1.3); 7.2362 (1.6); 7.2202 (0.7); 7.2174 (0.7); 7.1849 (0.8); 7.1828 (0.6); 7.1641 (0.7); 7.1609 (0.9); 7.1388 (0.6); 5.3600 (7.8); 5.2995 (1.3); 4.9765 (0.6); 3.6682 (16.0); 3.3455 (1.5); 3.3297 (2.1); 3.3132 (1.7); 2.6885 (1.9); 2.6719 (2.3); 2.6562 (1.7); 1.5650 (0.9); 1.2548 (1.8); -0.0002 (15.1); -0.0085 (0.5)

### I-005: ¹H-NMR (400.6 MHz, CDCl₃)

δ= 7.2605 (45.4); 7.2523 (1.0); 7.2479 (1.3); 7.2438 (1.2); 7.2376 (4.2); 7.2345 (4.4); 7.2283 (1.5); 7.2197 (0.6); 7.2099 (1.1); 7.2010 (0.9); 7.1335 (1.6); 7.1161 (5.4); 4.7530 (5.5); 2.1264 (14.4); 1.8971 (16.0); 1.5884 (5.0); 0.0079 (2.0); -0.0002 (65.7); -0.0085 (2.0)

### I-008: ¹H-NMR (400.6 MHz, CDCl₃)

δ= 7.4004 (1.0); 7.3957 (1.8); 7.3873 (0.5); 7.3805 (2.1); 7.3771 (2.0); 7.3628 (1.3); 7.3603 (1.0); 7.3438 (3.8); 7.3425 (3.4); 7.2605 (4.7); 7.2491 (1.2); 7.2462 (1.2); 7.2304 (1.6); 7.2287 (1.3); 7.2265 (0.9); 7.2116 (0.8); 7.2088 (0.7); 7.1671 (0.9); 7.1642 (0.7); 7.1455 (0.9); 7.1418 (1.0); 7.1202 (0.7); 5.3275 (9.6); 2.6179 (16.0); 2.1680 (7.0); 2.1629 (6.9); 1.5650 (1.0); -0.0002 (7.5)

### I-009: ¹H-NMR (400.6 MHz, CDCl₃)

δ= 7.6533 (7.8); 7.4338 (0.6); 7.4288 (0.6); 7.4264 (1.0); 7.4250 (0.9); 7.4205 (0.8); 7.4158 (0.7); 7.4136 (0.8); 7.4069 (1.4); 7.4053 (1.1); 7.4024 (0.9); 7.4004 (0.6); 7.3955 (0.7); 7.3875 (0.8); 7.3840 (0.5); 7.2603 (4.9); 7.2503 (1.1); 7.2475 (1.2); 7.2314 (1.4); 7.2293 (1.3); 7.2281 (1.2); 7.2127 (0.8); 7.2099 (0.8); 7.1824 (0.6); 7.1813 (0.7); 7.1798 (0.6); 7.1609 (0.7); 7.1599 (0.6); 7.1577 (1.0); 7.1547 (0.7); 7.1536 (0.6); 7.1361 (0.6); 7.1344 (0.6); 7.1333 (0.5); 5.3562 (9.0); 2.5867 (16.0); 1.5622 (0.7); - 0.0002 (7.1)

### I-010: ¹H-NMR (400.6 MHz, CDCl₃)

δ= 7.4119 (1.2); 7.4070 (0.6); 7.3989 (0.6); 7.3935 (1.3); 7.3885 (1.2); 7.3810 (0.8); 7.3767 (0.8); 7.3743 (0.9); 7.3720 (0.5); 7.3700 (0.8); 7.3635 (0.7); 7.3381 (2.9); 7.3368 (3.0); 7.2608 (8.1); 7.2503 (1.0); 7.2474 (1.1); 7.2323 (0.9); 7.2307 (1.0); 7.2287 (1.6); 7.2128 (0.7); 7.2099 (0.7); 7.1630 (0.8); 7.1605 (0.6); 7.1415 (0.7); 7.1377 (1.1); 7.1352 (0.7); 7.1164 (0.6); 7.1131 (0.5); 5.3188 (7.6); 3.7497 (2.5); 3.7099 (2.6); 3.6628 (16.0); 3.3832 (1.4); 3.3670 (2.2); 3.3503 (1.6); 2.8728 (0.5); 2.6890 (1.9); 2.6721 (2.4); 2.6561 (1.7); 2.1652 (5.0); 2.1643 (5.3); 2.1601 (5.2); 2.1591 (5.2); 1.2550 (1.4); -0.0002 (13.2)

### I-011: ¹H-NMR (400.6 MHz, CDCl₃)

δ= 7.3463 (0.5); 7.3311 (1.2); 7.3278 (1.0); 7.3124 (1.2); 7.3089 (1.1); 7.2661 (0.7); 7.2597 (60.0); 7.2563 (1.4); 7.2555 (1.3); 7.2546 (1.2); 7.2532 (1.6); 7.2515 (1.8); 7.2498 (1.7); 7.2481 (1.3); 7.2419 (1.5); 7.2405 (1.5); 7.2355 (0.9); 7.2334 (1.0); 7.2301 (0.8); 7.2242 (0.6); 7.2228 (0.6); 7.1124 (4.7); 7.0423 (1.3); 7.0381 (1.3); 7.0216 (1.2); 5.1331 (5.2); 4.6256 (0.5); 2.5192 (0.7); 2.1412 (11.9); 1.5342 (16.0); 0.8820 (1.0); 0.0080 (2.3); 0.0065 (0.5); -0.0002 (85.6); -0.0058 (0.7); -0.0085 (2.3)

### I-013: ¹H-NMR (400.6 MHz, CDCl₃)

δ= 7.3401 (2.9); 7.3387 (2.9); 7.3081 (0.8); 7.3046 (0.8); 7.2921 (2.4); 7.2887 (2.4); 7.2858 (1.6); 7.2601 (24.6); 7.2462 (0.7); 7.2398 (0.6); 7.1319 (1.0); 7.1136 (0.8); 5.3284 (1.6); 4.1126 (0.9); 4.0957 (1.2); 4.0788 (0.9); 2.1344 (9.8); 2.0077 (10.4); 2.0066 (10.4); 1.5420 (12.5); 1.1097 (16.0); 1.1038 (0.6); 1.0928 (16.0); 0.0080 (0.9); -0.0002 (34.2); -0.0085 (1.0)

### I-014: ¹H-NMR (400.6 MHz, CDCl₃)

δ= 7.6525 (7.6); 7.3313 (0.9); 7.3171 (1.2); 7.3132 (1.2); 7.2897 (1.4); 7.2881 (1.6); 7.2790 (0.7); 7.2745 (0.7); 7.2729 (0.8); 7.2712 (0.7); 7.2690 (0.7); 7.2598 (13.1); 7.2428 (0.6); 7.2400 (0.5); 7.2063 (1.2); 7.2029 (1.3); 7.1877 (0.7); 7.1840 (0.7); 5.3618 (3.4); 5.2991 (1.0); 2.5491 (16.0); 2.1913 (10.7); 1.5487 (2.6); 1.2551 (0.8); -0.0002 (16.3)

### I-015: ¹H-NMR (400.6 MHz, CDCl₃)

δ= 7.3460 (3.5); 7.3446 (3.5); 7.3033 (1.0); 7.2997 (1.0); 7.2876 (3.0); 7.2841 (3.4); 7.2696 (0.5); 7.2604 (14.8); 7.2538 (0.6); 7.2418 (0.8); 7.2406 (0.8); 7.2350 (0.7); 7.1416 (1.1); 7.1396 (1.2); 7.1213 (0.9); 5.3317 (1.5); 5.2994 (0.8); 2.5693 (16.0); 2.1381 (11.7); 2.0236 (12.6); 2.0223 (12.7); 1.5564 (3.0); 0.0079 (0.6); -0.0002 (19.8); -0.0085 (0.5)

### I-017: ¹H-NMR (400.6 MHz, CDCl₃)

δ= 7.3912 (0.7); 7.3859 (1.6); 7.3779 (0.5); 7.3732 (0.8); 7.3711 (1.0); 7.3659 (3.0); 7.3647 (4.7); 7.3633 (4.5); 7.3533 (1.0); 7.3504 (0.9); 7.3489 (1.1); 7.3457 (0.6); 7.2602 (17.8); 7.2348 (1.3); 7.2319 (1.4); 7.2157 (1.5); 7.2143 (1.4); 7.2122 (1.2); 7.1973 (0.9); 7.1945 (0.9); 7.1584 (0.7); 7.1557 (0.7); 7.1367 (0.9); 7.1332 (0.9); 7.1321 (0.9); 7.1303 (0.8); 7.1287 (0.6); 7.1116 (0.7); 7.1104 (0.6); 7.1088 (0.5); 5.3436 (16.0); 3.5769 (0.7); 3.5687 (0.7); 3.5569 (1.5); 3.5451 (0.8); 3.5369 (0.7); 2.1725 (6.2); 2.1713 (7.0); 2.1674 (6.6); 2.1663 (7.0); 1.5420 (3.1); 1.2550 (0.5); 1.1370 (0.6); 1.1279 (1.9); 1.1201 (2.6); 1.1163 (1.9); 1.1121 (0.9); 1.1107 (1.0); 1.1084 (2.4); 0.0079 (0.8); -0.0002 (28.9); -0.0085 (0.9)

### I-019: ¹H-NMR (400.6 MHz, CDCl₃)

δ= 7.6560 (6.4); 7.4527 (0.6); 7.4387 (0.8); 7.4342 (1.2); 7.4248 (0.6); 7.4191 (0.6); 7.4162 (0.8); 7.4115 (0.7); 7.4046 (0.6); 7.2601 (22.9); 7.2431 (0.9); 7.2403 (0.9); 7.2243 (1.4); 7.2215 (1.5); 7.2055 (0.6); 7.2027 (0.6); 7.1655 (0.6); 7.1632 (0.5); 7.1447 (0.5); 7.1413 (0.7); 7.1382 (0.5); 7.1195 (0.5); 5.3362 (11.5); 3.3948 (16.0); 1.5466 (3.8); 0.0079 (1.0); -0.0002 (32.4); -0.0085 (0.9)

### I-020: ¹H-NMR (400.6 MHz, CDCl₃)

δ= 7.6485 (5.6); 7.4389 (0.5); 7.4336 (0.5); 7.4207 (0.5); 7.4180 (1.0); 7.4132 (0.9); 7.3998 (1.1); 7.3991 (1.2); 7.3944 (0.8); 7.3807 (0.7); 7.3760 (0.5); 7.2605 (5.5); 7.2565 (1.0); 7.2536 (1.1); 7.2378 (1.2); 7.2351 (1.2); 7.2346 (1.2); 7.2189 (0.7); 7.2161 (0.7); 7.1876 (0.6); 7.1854 (0.6); 7.1845 (0.5); 7.1669 (0.5); 7.1635 (0.9); 7.1605 (0.6); 7.1420 (0.5); 5.3526 (10.5); 3.9894 (0.9); 3.9725 (1.2); 3.9556 (0.9); 1.5530 (0.8); 1.2853 (1.8); 1.2684 (1.8); 1.1527 (16.0); 1.1467 (0.5); 1.1358 (15.6); -0.0002 (9.0)

### I-022: ¹H-NMR (400.6 MHz, CDCl₃)

δ= 7.6397 (11.7); 7.4560 (0.5); 7.4514 (0.7); 7.4430 (0.6); 7.4380 (1.0); 7.4354 (0.7); 7.4330 (0.9); 7.4308 (1.0); 7.4246 (0.9); 7.4220 (1.5); 7.4175 (1.9); 7.4125 (1.1); 7.4031 (2.2); 7.3993 (2.1); 7.3840 (1.3); 7.3802 (0.9); 7.2603 (10.5); 7.2568 (2.1); 7.2539 (2.0); 7.2380 (2.3); 7.2354 (2.2); 7.2192 (1.2); 7.2164 (1.3); 7.1868 (1.2); 7.1847 (1.0); 7.1662 (1.1); 7.1628 (1.6); 7.1597 (1.1); 7.1414 (1.0); 7.1393 (0.9); 5.3628 (14.6); 3.0303 (1.6); 3.0119 (5.1); 2.9935 (5.2); 2.9752 (1.7); 1.5534 (6.2); 1.1662 (7.6); 1.1478 (16.0); 1.1294 (7.2); -0.0002 (15.0)

### I-024: ¹H-NMR (400.6 MHz, CDCl₃)

δ= 7.5181 (0.9); 7.3872 (1.0); 7.3712 (2.1); 7.3662 (1.7); 7.3554 (1.1); 7.3502 (4.0); 7.3451 (1.3); 7.3345 (1.6); 7.3292 (2.4); 7.3132 (1.1); 7.2860 (0.6); 7.2677 (1.3); 7.2669 (1.4); 7.2653 (1.6); 7.2597 (173.5); 7.2506 (0.6); 7.2317 (1.0); 7.0021 (0.6); 6.9960 (1.3); 6.9890 (5.7); 6.9808 (0.9); 6.9773 (1.0); 6.9706 (6.0); 6.9680 (5.7); 6.9615 (1.1); 6.9573 (0.8); 6.9497 (4.9); 6.9401 (0.7); 5.2439 (1.3); 4.9718 (16.0); 1.5950 (1.9); 1.2551 (0.6); 0.1457 (0.7); 0.0263 (0.7); 0.0160 (0.6); 0.0144 (0.6); 0.0120 (0.9); 0.0080 (6.7); 0.0057 (1.9); 0.0048 (2.2); -0.0002 (214.1); -0.0085 (5.7); -0.0281 (1.4); -0.1493 (0.7)

### I-025: ¹H-NMR (400.6 MHz, CDCl₃)

δ= 7.5185 (0.6); 7.3882 (2.0); 7.3720 (2.2); 7.3673 (3.9); 7.3512 (3.9); 7.3465 (2.5); 7.3303 (2.4); 7.2872 (0.6); 7.2635 (1.3); 7.2600 (120.2); 7.2355 (17.7); 6.9964 (0.7); 6.9734 (1.6); 6.9711 (1.6); 6.9671 (1.9); 6.9648 (1.9); 6.9535 (1.9); 6.9516 (2.8); 6.9498 (1.9); 6.9472 (2.2); 6.9453 (3.3); 6.9321 (1.5); 6.9299 (1.5); 6.9258 (1.8); 6.9237 (1.7); 6.9100 (2.5); 6.9039 (2.0); 6.8875 (2.8); 6.8856 (2.9); 6.8813 (2.3); 6.8795 (2.5); 6.8631 (2.4); 6.8570 (2.1); 5.2588 (1.9); 4.9396 (16.0); 4.9377 (15.9); 2.1979 (0.6); 1.5982 (1.6); 0.1458 (0.5); 0.0269 (0.8); 0.0079 (4.9); -0.0002 (190.1); -0.0085 (5.9); -0.0131 (0.8); -0.1495 (0.5)

### I-026: ¹H-NMR (400.6 MHz, CDCl₃)

δ= 7.9900 (1.1); 7.9731 (1.6); 7.9673 (1.3); 7.8463 (1.0); 7.8409 (1.6); 7.8236 (1.7); 7.7254 (1.4); 7.7214 (1.2); 7.7143 (1.9); 7.7080 (4.0); 7.7022 (1.0); 7.6959 (1.6); 7.6918 (1.6); 7.6770 (0.5); 7.5287 (2.1); 7.5272 (2.4); 7.5257 (2.3); 7.5090 (2.7); 7.5076 (3.1); 7.5061 (3.0); 7.4712 (1.0); 7.4697 (1.0); 7.4538 (2.2); 7.4524 (2.8); 7.4507 (2.7); 7.4493 (2.2); 7.4336 (2.2); 7.4319 (2.0); 7.3917 (1.3); 7.3884 (1.9); 7.3862 (1.7); 7.3691 (2.5); 7.3511 (1.0); 7.3479 (0.8); 7.3247 (2.8); 7.3231 (3.1); 7.3215 (2.8); 7.3118 (9.4); 7.3103 (9.8); 7.3059 (2.7); 7.3042 (2.8); 7.3026 (2.4); 7.2637 (0.6); 7.2603 (48.9); 7.1105 (1.0); 7.1063 (1.1); 7.0919 (1.1); 7.0899 (1.5); 7.0877 (1.4); 7.0856 (2.1); 7.0811 (1.1); 7.0713 (1.3); 7.0670 (2.1); 7.0648 (1.5); 7.0626 (1.3); 7.0606 (1.4); 7.0462 (1.1); 7.0419 (1.2); 6.9047 (1.1); 6.9009 (1.2); 6.8927 (1.1); 6.8889 (1.2); 6.8848 (1.6); 6.8811 (1.7); 6.8727 (1.6); 6.8691 (1.7); 6.8646 (1.1); 6.8607 (1.1); 6.8525 (0.9); 6.8487 (1.0); 6.2836 (1.0); 6.2682 (1.5); 6.2642 (1.6); 6.2489 (1.0); 5.4948 (14.8); 2.0806 (14.4); 2.0793 (16.0); 2.0742 (15.5); 2.0730 (15.9); 1.5404 (2.1); 1.2551 (2.4); 0.0080 (2.2); 0.0049 (0.6); 0.0040 (0.8); 0.0032 (1.4); 0.0024 (2.2); -0.0002 (80.4); -0.0057 (1.1); -0.0065 (1.0); -0.0085 (2.3)

### I-027: ¹H-NMR (400.6 MHz, CDCl₃)

δ= 7.3543 (3.5); 7.3529 (3.5); 7.2606 (19.1); 7.2325 (0.6); 7.2266 (0.8); 7.2134 (0.6); 7.2102 (0.6); 7.2085 (0.7); 7.2017 (0.6); 7.1897 (0.6); 7.1856 (0.6); 7.1836 (0.9); 7.1734 (0.5); 7.1665 (0.8); 7.1639 (0.9); 7.1536 (1.2); 7.1461 (1.4); 7.1445 (1.3); 7.1375 (1.7); 7.1329 (0.8); 7.1297 (0.7); 7.1269 (0.8); 7.1246 (0.9); 5.3311 (8.7); 2.6077 (16.0); 2.1775 (6.0); 2.1764 (6.1); 2.1725 (6.4); 2.1713 (6.0); 1.5454 (4.4); 0.0080 (0.8); -0.0002 (32.0); -0.0085 (1.0)

### I-031: ¹H-NMR (400.6 MHz, CDCl₃)

δ= 7.4229 (0.7); 7.4197 (1.0); 7.4159 (0.5); 7.4061 (1.2); 7.4017 (3.1); 7.3988 (2.8); 7.3941 (0.7); 7.3888 (4.8); 7.3848 (9.6); 7.3826 (8.4); 7.3758 (0.9); 7.3717 (1.2); 7.3675 (1.7); 7.3586 (1.6); 7.3540 (1.2); 7.3509 (0.8); 7.3449 (1.2); 7.3416 (0.8); 7.3370 (1.4); 7.3351 (0.8); 7.3281 (0.8); 7.3215 (0.7); 7.2593 (7.6); 7.1169 (7.0); 4.4884 (16.0); 2.4389 (1.4); 2.4201 (4.6); 2.4013 (4.7); 2.3826 (1.6); 1.2491 (0.6); 1.1595 (0.5); 1.0676 (6.1); 1.0489 (13.6); 1.0300 (5.9); -0.0002 (12.1)

### I-032: ¹H-NMR (400.6 MHz, CDCl₃)

δ= 7.3238 (10.6); 7.2850 (0.8); 7.2704 (0.9); 7.2653 (1.5); 7.2598 (14.6); 7.2508 (1.5); 7.2452 (1.2); 7.2307 (1.1); 7.0627 (2.0); 7.0437 (1.7); 6.9855 (1.0); 6.9845 (1.0); 6.9632 (1.7); 6.9421 (0.9); 6.7829 (0.6); 5.2990 (1.7); 4.6243 (2.3); 4.6093 (4.3); 4.5795 (4.6); 4.5645 (2.5); 2.1582 (16.0); 1.2531 (0.9); 0.0080 (0.6); -0.0002 (22.5); -0.0085 (0.7)

### I-033: ¹H-NMR (400.6 MHz, CDCl₃)

δ= 7.6448 (12.0); 7.2743 (0.5); 7.2698 (0.6); 7.2663 (0.6); 7.2606 (14.0); 7.2505 (0.8); 7.2473 (0.8); 7.2433 (0.7); 7.2413 (1.2); 7.2346 (0.8); 7.2323 (0.5); 7.2257 (0.6); 7.2164 (0.8); 7.1762 (1.8); 7.1755 (1.7); 7.1690 (2.8); 7.1665 (1.7); 7.1604 (4.6); 7.1585 (2.2); 7.1522 (2.7); 5.3679 (14.8); 3.0153 (1.6); 2.9969 (5.1); 2.9785 (5.3); 2.9602 (1.7); 1.5486 (4.7); 1.1713 (7.5); 1.1529 (16.0); 1.1345 (7.2); -0.0002 (19.9); -0.0084 (0.7)

### I-034: ¹H-NMR (400.6 MHz, CDCl₃)

δ= 9.3389 (1.5); 8.6558 (1.7); 7.6476 (0.5); 7.4196 (1.0); 7.3585 (16.1); 7.2602 (40.9); 7.2464 (0.5); 7.2391 (1.1); 7.2336 (1.2); 7.2197 (1.9); 7.2141 (2.8); 7.2065 (1.1); 7.2008 (1.9); 7.1963 (3.3); 7.1892 (2.0); 7.1769 (1.9); 7.1712 (1.9); 7.1678 (1.6); 7.1651 (1.4); 7.1557 (1.5); 7.1484 (2.6); 7.1457 (3.1); 7.1366 (3.4); 7.1352 (3.5); 7.1282 (2.2); 7.1252 (3.5); 7.1225 (2.9); 7.1172 (8.0); 7.1117 (2.4); 7.1084 (2.5); 7.1060 (2.7); 7.1035 (2.8); 7.0977 (0.8); 7.0928 (0.9); 7.0892 (1.1); 7.0844 (0.9); 5.8508 (1.8); 5.4076 (0.8); 5.0831 (1.9); 4.8221 (16.0); 1.8756 (1.1); 1.5732 (1.8); 1.5319 (0.6); 1.5015 (0.5); 1.2841 (0.7); 1.2552 (1.9); 0.0080 (1.6); -0.0002 (58.8); -0.0085 (1.9)

### I-035: ¹H-NMR (400.6 MHz, CDCl₃)

δ= 7.6607 (6.5); 7.2600 (36.4); 7.2500 (1.1); 7.2314 (0.7); 7.2251 (0.6); 7.2120 (0.5); 7.2066 (0.6); 7.2005 (0.7); 7.1973 (0.8); 7.1863 (0.6); 7.1842 (0.7); 7.1779 (0.5); 7.1759 (0.8); 7.1740 (0.7); 7.1652 (0.6); 7.1625 (0.6); 7.1542 (0.6); 7.1457 (0.5); 7.1427 (0.6); 5.3430 (11.2); 3.3896 (16.0); 1.5396 (1.6); 0.0079 (1.3); -0.0002 (51.3); -0.0085 (1.8)

### I-037: ¹H-NMR (400.6 MHz, CDCl₃)

δ= 7.5185 (0.7); 7.3695 (10.9); 7.3562 (11.7); 7.2601 (117.6); 7.2525 (0.6); 7.2240 (17.5); 6.9964 (0.8); 6.9745 (12.3); 6.9612 (11.2); 5.5919 (0.6); 5.2997 (1.6); 4.9239 (16.0); 4.6246 (0.7); 3.8940 (1.6); 3.4517 (0.5); 3.2830 (1.0); 2.0448 (0.5); 1.6410 (1.0); 1.2838 (0.7); 1.2542 (3.3); 0.8801 (0.7); 0.1456 (0.5); 0.0080 (5.2); 0.0056 (2.7); -0.0002 (167.8); -0.0084 (4.9); -0.0277 (0.6); -0.1492 (0.6)

### I-038: ¹H-NMR (400.6 MHz, CDCl₃)

δ= 7.3148 (1.0); 7.2984 (1.1); 7.2939 (2.0); 7.2775 (2.0); 7.2730 (1.2); 7.2606 (14.0); 7.2567 (1.3); 7.1611 (7.6); 6.9621 (0.7); 6.9598 (0.8); 6.9557 (0.9); 6.9535 (0.9); 6.9398 (1.2); 6.9338 (1.5); 6.9207 (0.7); 6.9184 (0.7); 6.9144 (0.9); 6.9122 (0.9); 6.8956 (1.2); 6.8894 (1.0); 6.8725 (1.6); 6.8659 (1.3); 6.8492 (1.1); 6.8430 (0.9); 5.2993 (3.7); 4.7686 (16.0); 2.3262 (1.0); 2.3074 (3.2); 2.2885 (3.3); 2.2698 (1.2); 2.1553 (0.8); 1.2518 (1.8); 1.0391 (5.3); 1.0300 (0.5); 1.0203 (11.0); 1.0112 (1.0); 1.0015 (5.1); 0.9925 (0.5); -0.0002 (20.7); -0.0085 (0.7)

### I-039: ¹H-NMR (400.6 MHz, CDCl₃)

δ= 7.9160 (0.5); 7.3540 (0.6); 7.2600 (9.5); 7.2499 (0.5); 7.2405 (0.6); 7.2344 (0.8); 7.2320 (0.6); 7.2250 (1.6); 7.2200 (11.0); 7.2151 (1.0); 7.2127 (1.0); 7.2098 (0.8); 7.2070 (1.2); 7.1999 (1.0); 7.1907 (0.7); 7.1821 (0.9); 7.1758 (0.6); 7.1555 (1.7); 7.1483 (3.2); 7.1393 (3.5); 7.1306 (2.3); 5.2989 (1.2); 4.8481 (16.0); 3.2905 (0.8); -0.0002 (15.0)

### I-043: ¹H-NMR (400.6 MHz, CDCl₃)

δ= 7.9895 (0.8); 7.9726 (1.0); 7.9669 (0.8); 7.8461 (0.7); 7.8405 (0.9); 7.8235 (1.1); 7.7246 (1.0); 7.7206 (0.9); 7.7136 (1.4); 7.7073 (2.2); 7.7016 (0.7); 7.6952 (1.0); 7.6911 (1.0); 7.6120 (16.0); 7.5186 (2.2); 7.4990 (2.8); 7.4711 (0.9); 7.4539 (2.4); 7.4350 (1.8); 7.3924 (1.7); 7.3728 (2.2); 7.3539 (0.8); 7.3051 (2.7); 7.2877 (2.0); 7.2862 (2.1); 7.2600 (22.3); 6.7459 (0.9); 6.7413 (2.0); 6.7395 (2.6); 6.7235 (1.7); 6.7183 (7.0); 6.7127 (1.1); 6.6985 (2.9); 6.6971 (3.4); 6.6928 (1.4); 6.6037 (0.8); 6.5842 (1.4); 6.5663 (1.3); 6.5479 (0.5); 5.5208 (7.1); 2.5767 (0.7); 1.5444 (1.0); 1.2758 (0.6); 1.2579 (1.9); 1.2403 (0.6); 0.0079 (1.1); -0.0002 (35.2); -0.0085 (1.0)

### I-045: ¹H-NMR (400.6 MHz, CDCl₃)

δ= 7.6330 (12.0); 7.4124 (1.0); 7.3965 (1.0); 7.3916 (1.8); 7.3756 (1.8); 7.3706 (1.1); 7.3546 (1.1); 7.2606 (11.2); 6.9997 (0.7); 6.9972 (0.8); 6.9934 (0.8); 6.9910 (0.8); 6.9797 (0.9); 6.9774 (1.1); 6.9734 (1.0); 6.9712 (1.3); 6.9583 (0.6); 6.9558 (0.6); 6.9520 (0.8); 6.9496 (0.8); 6.9421 (1.2); 6.9360 (0.8); 6.9198 (1.3); 6.9175 (1.3); 6.9137 (1.0); 6.9114 (1.0); 6.8952 (1.1); 6.8890 (0.9); 5.3609 (14.7); 3.0128 (1.6); 2.9944 (5.1); 2.9760 (5.3); 2.9576 (1.7); 1.5494 (5.6); 1.1703 (7.6); 1.1519 (16.0); 1.1335 (7.3); - 0.0002 (17.2)

### I-046: ¹H-NMR (400.6 MHz, CDCl₃)

δ= 7.6470 (6.8); 7.4178 (0.6); 7.4019 (0.7); 7.3969 (1.2); 7.3810 (1.2); 7.3760 (0.8); 7.3600 (0.7); 7.2609 (4.2); 6.9876 (0.5); 6.9853 (0.5); 6.9719 (0.7); 6.9657 (0.9); 6.9439 (0.5); 6.9376 (0.8); 6.9314 (0.6); 6.9154 (0.9); 6.9129 (0.9); 6.9092 (0.7); 6.9068 (0.7); 6.8907 (0.8); 6.8845 (0.6); 5.3547 (9.6); 2.5766 (16.0); 1.5581 (1.3); -0.0002 (6.3)

### I-047: 1H-NMR (400.6 MHz, CDCl₃):

δ= 7.2596 (15.0); 7.2514 (1.2); 7.2475 (1.5); 7.2414 (1.6); 7.2366 (3.7); 7.2334 (5.0); 7.2229 (0.6); 7.2190 (0.9); 7.2180 (0.9); 7.2113 (0.7); 7.2007 (1.1); 7.1997 (1.1); 7.1921 (1.0); 7.1872 (0.6); 7.1852 (0.6); 7.1776 (0.6); 7.1398 (1.7); 7.1218 (7.8); 5.2988 (0.5); 4.4943 (9.4); 2.1183 (16.0); 0.9798 (5.6); 0.9609 (12.8); 0.9421 (5.6); 0.0079 (0.7); -0.0002 (24.0); -0.0085 (0.7)

### I-048: ¹H-NMR (400.6 MHz, CDCl₃)

δ= 7.4539 (1.5); 7.4498 (2.1); 7.4352 (2.1); 7.4336 (2.3); 7.4307 (2.8); 7.3918 (1.2); 7.3871 (1.4); 7.3733 (2.7); 7.3687 (2.5); 7.3543 (1.9); 7.3493 (3.7); 7.3450 (2.1); 7.3308 (2.9); 7.3269 (2.8); 7.3123 (1.3); 7.3086 (1.1); 7.2596 (50.7); 7.1933 (2.4); 7.1891 (2.0); 7.1876 (1.9); 7.1748 (2.4); 7.1701 (1.8); 7.1050 (6.7); 5.2996 (14.3); 5.1709 (1.5); 5.1492 (4.4); 5.1290 (4.5); 5.1072 (1.5); 1.5353 (16.0); 1.2633 (1.0); 0.8990 (0.5); 0.8822 (1.8); 0.8644 (0.7); 0.0080 (2.0); -0.0002 (71.6); -0.0085 (2.2)

### I-049: ¹H-NMR (400.6 MHz, CDCl₃)

δ= 7.4474 (1.1); 7.4461 (1.1); 7.4428 (1.1); 7.4387 (1.0); 7.4299 (1.4); 7.4243 (1.8); 7.3174 (1.5); 7.3144 (0.8); 7.3121 (2.5); 7.3031 (4.3); 7.2958 (2.7); 7.2940 (1.6); 7.2875 (2.7); 7.2784 (2.2); 7.2771 (2.5); 7.2692 (1.4); 7.2646 (1.3); 7.2600 (40.1); 7.2550 (1.1); 7.1228 (4.0); 7.1218 (4.0); 5.3180 (0.5); 4.8224 (1.5); 1.9481 (16.0); 1.9475 (15.4); 0.0080 (1.6); -0.0002 (64.4); -0.0085 (1.9)

### 1-051: ¹H-NMR (400.6 MHz, CDCl₃)

δ= 7.4032 (0.6); 7.3980 (0.6); 7.3961 (1.1); 7.3902 (0.6); 7.3853 (0.6); 7.3831 (0.7); 7.3774 (1.4); 7.3756 (1.1); 7.3727 (0.8); 7.3651 (0.6); 7.3577 (0.8); 7.3385 (2.9); 7.3373 (3.0); 7.2603 (3.7); 7.2544 (1.0); 7.2516 (1.1); 7.2355 (1.3); 7.2339 (1.1); 7.2322 (0.9); 7.2170 (0.6); 7.2142 (0.6); 7.1695 (0.6); 7.1679 (0.5); 7.1485 (0.6); 7.1452 (0.8); 7.1424 (0.6); 7.1240 (0.6); 7.1224 (0.5); 5.3229 (11.0); 4.1011 (0.9); 4.0842 (1.3); 4.0673 (1.0); 2.1649 (5.6); 2.1597 (5.5); 1.5644 (1.3); 1.1627 (16.0); 1.1458 (15.8); - 0.0002 (5.5)

### I-052: ¹H-NMR (400.6 MHz, CDCl₃)

δ= 7.4166 (0.7); 7.4082 (0.5); 7.4033 (1.1); 7.3975 (2.5); 7.3900 (0.7); 7.3880 (0.6); 7.3853 (1.1); 7.3832 (1.3); 7.3797 (3.0); 7.3654 (1.3); 7.3609 (1.6); 7.3577 (0.8); 7.3312 (5.3); 7.3302 (6.0); 7.2603 (7.4); 7.2555 (1.8); 7.2526 (2.0); 7.2365 (2.4); 7.2354 (2.3); 7.2330 (1.7); 7.2181 (1.2); 7.2152 (1.2); 7.1703 (1.0); 7.1677 (1.2); 7.1649 (0.6); 7.1486 (1.3); 7.1447 (1.5); 7.1424 (1.2); 7.1235 (1.1); 5.3323 (15.6); 3.0804 (1.7); 3.0620 (5.5); 3.0435 (5.6); 3.0251 (1.8); 2.1590 (11.2); 2.1539 (10.9); 1.5622 (3.4); 1.1766 (7.7); 1.1583 (16.0); 1.1397 (7.5); -0.0002 (11.4)

### I-053: ¹H-NMR (400.6 MHz, CDCl₃)

δ= 7.3851 (1.0); 7.3688 (1.1); 7.3641 (2.1); 7.3478 (2.1); 7.3430 (1.2); 7.3269 (6.7); 7.3258 (7.2); 7.2607 (12.2); 7.0042 (0.8); 7.0020 (0.8); 6.9979 (0.8); 6.9957 (0.9); 6.9821 (1.3); 6.9759 (1.5); 6.9628 (0.7); 6.9606 (0.7); 6.9565 (0.7); 6.9543 (0.8); 6.9278 (1.2); 6.9216 (1.0); 6.9055 (1.3); 6.9029 (1.4); 6.8994 (1.1); 6.8969 (1.1); 6.8806 (1.2); 6.8744 (1.0); 5.3296 (16.0); 3.0594 (1.8); 3.0409 (5.7); 3.0225 (5.8); 3.0040 (1.9); 2.1539 (0.8); 2.1467 (11.6); 2.1415 (11.3); 1.5503 (3.5); 1.1789 (7.8); 1.1604 (16.0); 1.1419 (7.6); 0.0080 (0.6); -0.0002 (19.2); -0.0085 (0.5)

### I-054: ¹H-NMR (400.6 MHz, CDCl₃)

δ= 7.3819 (0.6); 7.3656 (0.7); 7.3608 (1.2); 7.3445 (1.3); 7.3397 (0.9); 7.3335 (3.8); 7.3235 (0.8); 7.2625 (1.6); 6.9956 (0.5); 6.9817 (0.8); 6.9755 (1.0); 6.9280 (0.7); 6.9218 (0.6); 6.9056 (0.8); 6.9031 (0.9); 6.8996 (0.7); 6.8970 (0.7); 6.8807 (0.7); 6.8744 (0.6); 5.3200 (10.8); 4.0646 (1.0); 4.0477 (1.4); 4.0307 (1.0); 2.1532 (6.7); 2.1479 (6.6); 1.2464 (1.4); 1.2289 (1.4); 1.1631 (16.0); 1.1462 (15.9); - 0.0002 (2.3)

### I-077: ¹H-NMR (400.6 MHz, CDCl₃)

δ= 7.6425 (6.3); 7.4018 (3.1); 7.3885 (3.3); 7.2602 (33.2); 7.2549 (0.5); 7.0000 (3.4); 6.9867 (3.2); 5.3481 (7.6); 5.2999 (0.8); 3.6816 (16.0); 3.3628 (1.4); 3.3469 (2.0); 3.3306 (1.6); 2.6993 (1.8); 2.6829 (2.2); 2.6672 (1.7); 1.5407 (1.6); 0.0079 (1.4); -0.0002 (48.9); -0.0056 (0.8); -0.0086 (1.3)

### I-078: ¹H-NMR (400.6 MHz, CDCl₃)

δ= 7.6515 (11.1); 7.4035 (5.5); 7.3902 (5.7); 7.2609 (12.7); 7.0020 (5.9); 6.9887 (5.4); 5.3972 (16.0); 5.2997 (4.4); 4.0752 (14.1); 3.9945 (2.0); 3.9766 (6.3); 3.9588 (6.4); 3.9410 (2.0); 1.5506 (0.6); 1.0904 (7.4); 1.0726 (15.4); 1.0547 (7.2); 0.0080 (0.5); -0.0002 (19.5); -0.0085 (0.6)

### I-079: ¹H-NMR (400.6 MHz, CDCl₃)

δ= 7.3582 (15.0); 7.3446 (32.5); 7.2599 (78.6); 6.9708 (15.6); 6.9575 (14.9); 6.0519 (1.2); 5.2997 (2.9); 4.8373 (16.0); 2.9560 (3.1); 2.8843 (2.6); 2.0451 (0.6); 2.0070 (4.1); 1.6521 (1.2); 1.2593 (0.9); 0.8820 (1.0); 0.0277 (0.5); 0.0079 (3.4); -0.0002 (114.2); -0.0086 (3.4); -0.0125 (0.7)

### I-080: ¹H-NMR (400.6 MHz, CDCl₃)

δ= 8.1705 (3.0); 8.1580 (3.0); 7.4223 (0.7); 7.4032 (1.8); 7.3847 (1.4); 7.3708 (0.7); 7.3325 (1.8); 7.3130 (1.3); 7.2899 (4.1); 7.2866 (4.3); 7.2721 (1.3); 7.2595 (27.7); 7.2546 (2.2); 7.2354 (1.0); 7.0151 (1.1); 7.0119 (1.1); 6.9928 (2.0); 6.9735 (0.9); 6.9703 (0.9); 5.3503 (1.8); 5.3301 (3.4); 5.3102 (2.3); 5.2990 (16.0); 5.1911 (2.8); 5.1705 (2.3); 4.8781 (0.7); 4.6253 (0.7); 2.4678 (0.6); 2.4492 (1.5); 2.4304 (2.6); 2.4117 (2.4); 2.3939 (1.4); 2.3757 (1.0); 2.3569 (0.7); 2.0436 (0.8); 1.2585 (0.7); 1.2151 (3.9); 1.1963 (9.4); 1.1783 (9.0); 1.1596 (3.4); 1.1357 (0.8); 1.1168 (1.5); 1.0979 (0.7); 0.8820 (0.8); 0.0080 (1.1); -0.0002 (36.3); -0.0086 (1.1)

### I-081: ¹H-NMR (400.6 MHz, CDCl₃)

δ= 9.3127 (0.6); 8.6284 (0.6); 7.3850 (0.8); 7.3697 (0.8); 7.3664 (0.7); 7.3517 (0.7); 7.3482 (0.7); 7.3321 (1.9); 7.3289 (1.9); 7.3155 (3.0); 7.3120 (3.0); 7.3059 (2.6); 7.3017 (3.8); 7.2831 (9.2); 7.2586 (23.5); 7.2515 (1.4); 7.2372 (2.3); 7.2324 (2.0); 7.2251 (0.7); 7.2202 (1.4); 7.2154 (1.2); 7.1570 (2.5); 7.1543 (2.6); 7.1357 (1.8); 7.1110 (3.8); 7.0166 (0.8); 7.0005 (0.7); 6.9976 (0.7); 5.2982 (3.4); 5.1314 (4.1); 4.3857 (7.9); 2.5074 (1.3); 2.4892 (1.4); 2.4710 (1.4); 2.4528 (1.4); 2.4348 (1.1); 2.4168 (0.6); 1.5433 (1.0); 1.2650 (1.2); 1.2138 (2.7); 1.1949 (5.6); 1.1759 (2.7); 1.1358 (7.4); 1.1169 (16.0); 1.0980 (7.7); 1.0803 (0.6); 0.8987 (0.6); 0.8819 (2.0); 0.8641 (0.9); 0.0080 (0.6); -0.0002 (29.2); -0.0085 (1.1)

### I-082: ¹H-NMR (400.6 MHz, CDCl₃)

δ= 7.6642 (10.4); 7.3412 (0.5); 7.3246 (1.2); 7.3093 (1.5); 7.3048 (1.7); 7.2779 (2.5); 7.2596 (42.5); 7.2554 (2.3); 7.2370 (1.0); 7.2255 (2.0); 7.2216 (2.2); 7.2072 (0.8); 7.2022 (0.7); 5.3767 (5.0); 5.2994 (5.2); 3.4729 (0.9); 3.4646 (1.0); 3.4612 (0.6); 3.4529 (1.9); 3.4446 (0.6); 3.4412 (1.0); 3.4330 (0.9); 2.1923 (16.0); 1.5401 (3.1); 1.2594 (0.6); 1.2229 (2.6); 1.1110 (1.5); 1.1030 (2.0); 1.0922 (1.6); 0.8820 (0.8); 0.8641 (0.8); 0.8568 (2.5); 0.8481 (2.4); 0.8368 (2.5); 0.8282 (2.3); 0.0079 (1.8); -0.0002 (60.2); - 0.0085 (1.8)

### I-086: ¹H-NMR (400.6 MHz, CDCl₃)

δ= 7.4642 (0.9); 7.4579 (0.6); 7.4358 (0.5); 7.3834 (0.7); 7.3789 (0.7); 7.3603 (1.0); 7.3417 (5.6); 7.3295 (2.3); 7.3236 (0.8); 7.2596 (48.8); 7.2129 (1.2); 7.2084 (1.2); 7.1943 (1.5); 7.1899 (1.3); 7.0334 (1.0); 7.0310 (1.0); 7.0148 (1.6); 7.0124 (1.6); 6.9961 (1.0); 6.9938 (0.7); 6.9658 (1.5); 6.9451 (1.3); 6.8385 (0.7); 6.8167 (0.6); 5.7614 (0.5); 5.2994 (2.0); 4.9600 (0.6); 4.9567 (0.6); 4.8006 (1.4); 4.7731 (6.0); 3.8081 (16.0); 3.7890 (4.8); 2.0448 (1.9); 1.5425 (0.6); 1.3038 (0.7); 1.2769 (1.6); 1.2651 (3.0); 1.2593 (2.9); 1.2414 (0.8); 0.8991 (1.6); 0.8821 (5.3); 0.8644 (2.2); 0.0078 (1.9); 0.0054 (1.1); -0.0002 (65.7); -0.0028 (3.4); -0.0062 (0.7); -0.0085 (1.9)

### I-087: ¹H-NMR (400.6 MHz, CDCl₃)

δ= 7.3832 (0.7); 7.3788 (0.7); 7.3623 (1.2); 7.3439 (1.0); 7.3341 (5.8); 7.2598 (23.3); 7.2146 (1.3); 7.2105 (1.2); 7.1960 (1.7); 7.1919 (1.4); 7.0335 (1.1); 7.0149 (1.9); 6.9963 (1.0); 6.9656 (1.9); 6.9448 (1.7); 5.9553 (0.6); 4.7618 (0.7); 4.7347 (9.7); 3.8083 (16.0); 3.7889 (1.2); 1.2546 (1.2); 0.8821 (0.6); 0.0080 (1.1); -0.0002 (34.4)

### I-089: ¹H-NMR (400.6 MHz, CDCl₃)

δ= 9.4965 (2.5); 8.5404 (1.7); 8.1096 (2.1); 7.7223 (0.9); 7.7066 (0.8); 7.4535 (0.8); 7.4377 (0.9); 7.4327 (0.5); 7.4164 (0.5); 7.2610 (22.1); 7.1388 (0.6); 7.0357 (0.6); 7.0286 (1.0); 7.0225 (0.5); 7.0071 (0.8); 7.0035 (0.7); 6.9973 (0.9); 6.9818 (0.6); 6.9753 (0.5); 6.9684 (0.6); 6.9465 (0.5); 6.9430 (0.6); 5.3081 (1.5); 5.2129 (2.2); 5.1807 (2.5); 4.7320 (2.5); 4.6998 (2.2); 3.6769 (0.5); 3.4851 (0.6); 3.4710 (12.7); 3.4340 (1.2); 3.3996 (2.2); 2.8824 (16.0); 2.4806 (4.6); 2.4779 (4.6); 2.3149 (4.1); 2.3095 (4.0); 2.1615 (0.7); 2.1552 (0.8); 0.0079 (3.2); -0.0002 (109.9); -0.0062 (1.6); -0.0085 (3.0)

### I-090: ¹H-NMR (400.6 MHz, CDCl₃)

δ= 7.6493 (6.1); 7.4514 (0.5); 7.4354 (0.6); 7.4304 (1.1); 7.4145 (1.1); 7.4095 (0.6); 7.3936 (0.6); 7.2600 (23.9); 6.9660 (0.7); 6.9601 (0.8); 6.9226 (0.7); 6.9163 (0.5); 6.9000 (0.8); 6.8940 (0.6); 6.8754 (0.6); 6.8692 (0.5); 5.3360 (11.1); 3.3825 (16.0); 1.5375 (2.9); 1.2648 (2.1); 0.8990 (1.1); 0.8821 (3.5); 0.8645 (1.4); 0.0079 (1.0); -0.0002 (35.8); -0.0085 (1.1)

### I-091: ¹H-NMR (400.6 MHz, CDCl₃)

δ= 9.2141 (0.5); 7.3485 (0.8); 7.3440 (0.8); 7.3300 (1.0); 7.3279 (1.0); 7.3256 (1.1); 7.3235 (1.0); 7.3094 (0.9); 7.3048 (1.0); 7.2598 (24.6); 7.2180 (1.1); 7.2136 (1.1); 7.1994 (1.4); 7.1950 (1.2); 7.1008 (3.1); 7.0859 (0.8); 7.0264 (1.0); 7.0240 (1.1); 7.0079 (1.8); 7.0054 (2.0); 6.9893 (0.8); 6.9869 (0.8); 6.9472 (1.6); 6.9266 (1.4); 5.2992 (4.9); 4.7654 (4.8); 3.7760 (16.0); 3.7724 (2.5); 2.3177 (1.1); 2.3160 (1.1); 1.9533 (3.6); 1.9473 (11.5); 1.5538 (1.5); 1.5390 (1.5); 0.0079 (1.2); -0.0002 (37.2); -0.0085 (0.9)

### I-092: ¹H-NMR (400.6 MHz, CDCl₃)

δ= 7.6623 (6.1); 7.3718 (1.0); 7.3675 (1.1); 7.3541 (1.0); 7.3504 (1.1); 7.3264 (1.5); 7.3096 (0.7); 7.2689 (0.6); 7.2651 (0.7); 7.2591 (10.7); 7.2502 (1.2); 7.2466 (1.0); 7.2323 (0.7); 7.2286 (0.6); 7.1877 (1.3); 7.1845 (1.3); 7.1688 (0.8); 7.1658 (0.8); 5.2986 (6.5); 3.3486 (16.0); 1.1231 (0.9); 1.1102 (3.9); 1.1043 (0.6); 1.0913 (8.2); 1.0724 (3.7); 0.0079 (0.6); -0.0002 (15.6)

### I-093: ¹H-NMR (400.6 MHz, CDCl₃)

δ= 7.6505 (6.3); 7.3812 (1.2); 7.3631 (1.2); 7.3597 (1.2); 7.3342 (1.8); 7.3180 (0.9); 7.2816 (0.8); 7.2781 (0.7); 7.2592 (7.9); 7.2448 (0.8); 7.2414 (0.7); 7.1678 (1.6); 7.1648 (1.5); 7.1488 (1.2); 5.3720 (3.2); 5.3577 (3.1); 5.2983 (3.1); 2.5814 (0.5); 2.5635 (0.8); 2.5410 (16.0); 2.4857 (0.7); 2.4668 (0.8); 2.4486 (0.5); 1.5465 (2.5); 1.1188 (4.4); 1.0998 (8.9); 1.0810 (4.1); -0.0002 (9.8)

### I-094: ¹H-NMR (400.6 MHz, CDCl₃)

δ= 7.6362 (11.6); 7.4063 (0.7); 7.4028 (0.7); 7.3871 (1.7); 7.3843 (1.7); 7.3693 (1.8); 7.3658 (1.9); 7.3408 (2.7); 7.3241 (1.2); 7.2869 (1.0); 7.2831 (1.0); 7.2681 (2.1); 7.2644 (2.0); 7.2594 (10.1); 7.2502 (1.1); 7.2463 (1.0); 7.1672 (2.3); 7.1642 (2.3); 7.1483 (1.6); 7.1453 (1.6); 5.4005 (0.6); 5.3774 (4.9); 5.3646 (4.9); 5.3415 (0.6); 5.2979 (4.8); 3.0031 (0.6); 2.9845 (1.6); 2.9721 (0.7); 2.9661 (1.7); 2.9539 (1.7); 2.9478 (0.7); 2.9356 (1.7); 2.9172 (0.6); 2.5821 (0.7); 2.5639 (1.2); 2.5585 (0.6); 2.5451 (1.1); 2.5397 (0.6); 2.4896 (1.1); 2.4708 (1.2); 2.4527 (0.8); 1.8218 (0.8); 1.8183 (0.8); 1.5561 (2.9); 1.2594 (0.6); 1.2407 (0.8); 1.1422 (0.5); 1.1237 (8.4); 1.1199 (8.1); 1.1053 (16.0); 1.1010 (15.9); 1.0869 (7.4); 1.0820 (7.2); -0.0002 (14.5)

### I-095: ¹H-NMR (400.6 MHz, CDCl₃)

δ= 7.6430 (8.8); 7.3877 (1.1); 7.3843 (1.1); 7.3698 (1.3); 7.3662 (1.3); 7.3422 (1.4); 7.3397 (1.7); 7.3230 (0.8); 7.3205 (0.7); 7.2862 (0.8); 7.2825 (0.7); 7.2674 (1.4); 7.2636 (1.4); 7.2595 (11.0); 7.2495 (0.7); 7.2457 (0.7); 7.1580 (1.4); 7.1550 (1.4); 7.1391 (1.0); 7.1361 (1.0); 5.3633 (4.9); 5.3571 (4.8); 5.2985 (1.1); 4.0051 (1.2); 3.9882 (1.6); 3.9713 (1.2); 2.5650 (0.7); 2.5462 (0.7); 2.4968 (0.7); 2.4780 (0.8); 1.5478 (4.6); 1.1178 (5.2); 1.1015 (16.0); 1.0990 (13.8); 1.0846 (14.9); 1.0799 (5.8); 0.0079 (0.5); -0.0002 (16.3)

### I-096: ¹H-NMR (400.6 MHz, CDCl₃)

δ= 7.9986 (0.5); 7.3577 (0.6); 7.3436 (2.2); 7.3408 (2.5); 7.3308 (2.4); 7.3280 (2.7); 7.3107 (2.2); 7.3080 (2.0); 7.3015 (2.5); 7.2987 (2.0); 7.2593 (10.1); 7.0952 (3.0); 7.0913 (4.3); 7.0901 (4.3); 7.0860 (2.6); 7.0824 (2.5); 7.0732 (2.3); 5.2980 (1.3); 4.9035 (16.0); 2.7387 (0.7); 2.7369 (0.7); 2.3468 (15.2); 2.3461 (15.1); 2.3073 (0.6); 1.2539 (3.7); 0.8793 (0.7); -0.0002 (15.0)

### I-097: ¹H-NMR (400.6 MHz, CDCl₃)

δ= 7.3483 (1.3); 7.3450 (1.3); 7.3395 (4.2); 7.3316 (3.1); 7.3291 (2.9); 7.3258 (2.2); 7.3117 (0.5); 7.2598 (36.3); 7.2429 (1.1); 7.2368 (0.8); 7.2259 (0.6); 7.2207 (0.7); 7.1013 (1.5); 7.0838 (1.2); 5.3536 (1.1); 5.3161 (1.1); 5.2997 (1.3); 2.5612 (16.0); 2.0034 (14.1); 1.5406 (4.6); 1.0772 (4.5); 1.0582 (9.6); 1.0394 (4.3); 0.0079 (1.7); 0.0055 (1.6); -0.0002 (49.9); -0.0060 (0.7); -0.0084 (1.7)

### I-098: ¹H-NMR (400.6 MHz, CDCl₃)

δ= 7.3533 (1.4); 7.3500 (1.2); 7.3368 (3.6); 7.3337 (3.0); 7.3267 (4.7); 7.3175 (0.7); 7.2650 (1.0); 7.2596 (6.6); 7.2466 (1.2); 7.2408 (1.0); 7.2303 (0.7); 7.2247 (0.7); 7.1046 (1.6); 7.1019 (1.6); 7.0841 (1.4); 5.3591 (1.3); 5.3221 (1.3); 5.2981 (1.6); 3.0291 (0.7); 3.0109 (0.8); 2.9948 (0.8); 2.9771 (0.7); 2.5173 (0.5); 2.4983 (0.6); 2.4248 (0.5); 1.9926 (16.0); 1.1239 (5.0); 1.1055 (10.3); 1.0870 (5.0); 1.0786 (5.1); 1.0596 (10.3); 1.0408 (4.8); -0.0002 (8.3)

### I-099: ¹H-NMR (400.6 MHz, CDCl₃)

δ= 7.4052 (0.7); 7.4007 (0.7); 7.3867 (0.9); 7.3845 (0.9); 7.3822 (1.0); 7.3801 (0.9); 7.3660 (0.8); 7.3615 (0.9); 7.3128 (3.1); 7.3114 (3.0); 7.2595 (12.7); 7.2550 (1.3); 7.2406 (1.4); 7.2363 (1.2); 7.0607 (1.0); 7.0581 (1.0); 7.0421 (1.7); 7.0395 (1.7); 7.0235 (0.7); 7.0210 (0.7); 6.9879 (1.4); 6.9672 (1.2); 5.3160 (2.2); 3.7986 (16.0); 2.6097 (14.0); 2.0801 (10.8); 2.0789 (10.8); -0.0002 (16.6)

### I-100: ¹H-NMR (400.6 MHz, CDCl₃)

δ= 7.3601 (2.8); 7.3586 (2.9); 7.3384 (0.9); 7.3349 (0.9); 7.3222 (2.4); 7.3189 (2.0); 7.2592 (6.6); 7.2472 (0.5); 7.2415 (0.5); 7.2283 (0.7); 7.2224 (0.7); 7.1175 (1.0); 7.1148 (1.0); 7.0967 (0.8); 5.2980 (4.4); 3.3574 (16.0); 2.0445 (0.7); 2.0151 (9.6); 2.0139 (9.6); 1.0717 (3.7); 1.0590 (0.6); 1.0529 (8.2); 1.0340 (3.7); -0.0002 (9.4)

### I-101: ¹H-NMR (400.6 MHz, CDCl₃)

δ= 7.3928 (0.6); 7.3884 (0.7); 7.3742 (0.8); 7.3720 (0.8); 7.3698 (0.9); 7.3677 (0.8); 7.3536 (0.8); 7.3492 (0.8); 7.3270 (3.2); 7.3258 (2.9); 7.2945 (1.2); 7.2901 (1.1); 7.2758 (1.4); 7.2714 (1.2); 7.2591 (6.4); 7.0504 (0.9); 7.0479 (0.9); 7.0318 (1.5); 7.0293 (1.5); 7.0132 (0.7); 7.0107 (0.6); 6.9697 (1.3); 6.9490 (1.2); 5.2848 (2.3); 3.7856 (14.7); 3.4016 (16.0); 2.0834 (11.1); 2.0446 (1.1); 1.2590 (0.7); - 0.0002 (9.3)

### I-102: ¹H-NMR (400.6 MHz, CDCl₃)

δ= 7.3384 (3.2); 7.2585 (26.2); 7.2198 (1.1); 7.1996 (1.9); 7.1618 (2.4); 7.1525 (1.0); 7.1428 (2.6); 7.1341 (1.1); 7.1241 (1.1); 7.1103 (6.3); 7.0214 (0.5); 6.8866 (1.6); 6.8680 (1.4); 5.1293 (9.3); 4.7194 (2.0); 2.5095 (1.0); 2.3554 (1.0); 2.3121 (16.0); 2.2936 (0.6); 2.2677 (0.8); 2.2612 (0.7); 2.2282 (0.7); 2.2161 (0.7); 2.0624 (5.5); 2.0487 (0.6); 2.0266 (15.2); 2.0052 (7.7); 1.9426 (1.0); 1.9196 (0.6); 1.8562 (0.6); 0.0079 (1.2); -0.0002 (40.9); -0.0085 (1.0)

### I-103: ¹H-NMR (400.6 MHz, CDCl₃)

δ= 7.4591 (2.6); 7.4548 (2.7); 7.4420 (2.8); 7.4379 (4.0); 7.4349 (3.3); 7.4222 (3.0); 7.4178 (3.1); 7.3523 (26.6); 7.2652 (2.9); 7.2599 (41.2); 7.2497 (2.5); 7.2457 (5.4); 7.2416 (3.3); 7.2305 (3.7); 7.2261 (3.2); 7.1678 (3.9); 7.1654 (4.0); 7.1481 (5.6); 7.1459 (5.7); 7.1288 (2.3); 7.1263 (2.4); 5.9653 (1.0); 4.8064 (16.0); 2.0449 (0.6); 1.2591 (0.7); 0.8820 (0.6); 0.0080 (1.7); -0.0002 (55.2); -0.0085 (1.5)

### I-104: ¹H-NMR (400.6 MHz, CDCl₃)

δ= 7.5652 (0.9); 7.5502 (1.8); 7.5464 (1.8); 7.5314 (1.0); 7.4001 (0.9); 7.3955 (0.9); 7.3871 (1.3); 7.3804 (10.1); 7.3793 (9.1); 7.3686 (1.6); 7.3665 (1.4); 7.3638 (1.5); 7.3615 (1.8); 7.3565 (1.3); 7.3480 (1.3); 7.3435 (1.1); 7.2600 (20.4); 7.2364 (2.6); 7.2336 (2.6); 7.2177 (4.1); 7.2148 (4.0); 7.1989 (1.9); 7.1961 (1.8); 7.1435 (2.1); 7.1411 (1.9); 7.1227 (1.9); 7.1191 (2.6); 7.1158 (1.9); 7.0977 (1.7); 7.0952 (1.5); 5.3752 (6.4); 5.3515 (9.0); 5.2652 (0.7); 5.2628 (0.6); 5.2512 (6.2); 5.2276 (4.3); 4.3878 (2.0); 4.3819 (2.0); 4.3655 (2.2); 4.3596 (2.0); 2.9390 (0.6); 2.9194 (0.7); 2.2164 (0.7); 2.1997 (0.9); 2.1826 (16.0); 2.1768 (15.9); 2.1647 (1.2); 2.1606 (1.0); 2.1540 (1.0); 2.1353 (0.9); 2.0328 (0.6); 2.0264 (0.8); 2.0203 (1.2); 2.0168 (1.2); 2.0100 (1.0); 2.0034 (1.2); 1.9998 (1.2); 1.9905 (0.9); 1.9752 (0.9); 1.9683 (0.8); 1.9619 (1.0); 1.9528 (1.0); 1.9465 (1.1); 1.9396 (1.1); 1.9288 (0.6); 1.9240 (1.1); 1.9136 (0.6); 1.9065 (0.5); 1.8912 (0.6); 1.8319 (0.5); 1.8218 (0.6); 1.8158 (0.9); 1.8070 (0.8); 1.8003 (1.2); 1.7930 (0.9); 1.7844 (1.2); 1.7785 (0.7); 1.7688 (0.6); 1.6861 (0.7); 1.6729 (0.9); 1.6568 (1.4); 1.6410 (1.2); 1.6260 (1.1); 1.6118 (0.6); 1.5440 (2.3); 1.3046 (0.9); 1.2865 (1.3); 1.2646 (4.6); 1.2334 (0.7); 0.8987 (2.5); 0.8818 (8.1); 0.8641 (3.3); 0.0080 (1.0); -0.0002 (32.4); -0.0085 (0.9)

### I-145: ¹H-NMR(400.6 MHz, CDCl₃):

δ= 7.3453 (4.6); 7.3441 (4.2); 7.2609 (6.8); 7.2163 (1.1); 7.2061 (0.6); 7.1984 (1.2); 7.1915 (0.7); 7.1866 (0.6); 7.1736 (0.8); 7.1576 (3.2); 7.1477 (3.5); 7.1434 (1.1); 7.1396 (1.7); 7.1366 (0.9); 7.1337 (1.2); 5.3065 (16.0); 3.6295 (2.9); 3.6152 (1.6); 3.6102 (2.8); 3.6045 (1.5); 3.5906 (3.1); 2.1717 (8.2); 2.1712 (8.1); 2.1667 (8.2); 1.9014 (1.4); 1.8871 (0.8); 1.8825 (2.2); 1.8769 (0.7); 1.8686 (0.8); 1.8631 (2.2); 1.8583 (0.7); 1.8441 (1.4); 1.0554 (5.1); 1.0368 (10.6); 1.0182 (4.7); -0.0002 (10.9)

### I-148: ¹H-NMR(400.6 MHz, CDCl₃):

δ= 7.6442 (5.4); 7.2626 (6.0); 7.1810 (0.5); 7.1722 (0.6); 7.1710 (0.6); 7.1640 (0.7); 7.1623 (0.9); 7.1552 (1.0); 7.1519 (0.5); 5.3622 (5.1); 4.7681 (4.4); 3.7638 (0.6); 3.7613 (0.6); 3.7532 (1.4); 3.7509 (0.9); 3.7463 (1.1); 3.7400 (1.3); 3.7377 (1.3); 3.7069 (1.2); 3.7046 (1.3); 3.6983 (1.0); 3.6938 (0.9); 3.6914 (1.4); 3.6834 (0.6); 3.6808 (0.6); 3.6416 (1.1); 3.6327 (1.2); 3.6302 (1.5); 3.6254 (1.2); 3.6179 (2.0); 3.5241 (2.0); 3.5166 (1.2); 3.5119 (1.5); 3.5093 (1.2); 3.5007 (1.1); 3.3425 (16.0); -0.0002 (9.4)

### I-149: ¹H-NMR(400.6 MHz, CDCl₃):

δ= 7.3341 (2.3); 7.3328 (2.4); 7.2614 (15.3); 7.2412 (0.5); 7.1984 (0.6); 7.1762 (0.5); 7.1646 (0.5); 7.1120 (0.6); 7.1083 (0.6); 5.3257 (5.6); 4.8185 (4.9); 3.7751 (0.6); 3.7728 (0.6); 3.7645 (1.6); 3.7577 (1.2); 3.7513 (1.4); 3.7489 (1.4); 3.7163 (1.3); 3.7139 (1.4); 3.7075 (1.2); 3.7007 (1.6); 3.6925 (0.7); 3.6900 (0.6); 3.6499 (1.3); 3.6412 (1.3); 3.6387 (1.8); 3.6339 (1.3); 3.6264 (2.2); 3.5295 (2.2); 3.5219 (1.3); 3.5172 (1.8); 3.5147 (1.4); 3.5059 (1.3); 3.3441 (16.0); 2.1662 (4.0); 2.1617 (4.1); 1.5691 (8.4); 0.0080 (0.6); -0.0002 (21.8); -0.0085 (0.7)

### I-150: ¹H-NMR(400.6 MHz, CDCl₃):

δ= 7.3912 (0.6); 7.3782 (0.6); 7.3735 (0.7); 7.3599 (0.9); 7.3554 (0.8); 7.3414 (0.6); 7.3237 (2.3); 7.3224 (2.2); 7.2617 (11.2); 7.2582 (1.1); 7.2552 (1.0); 7.2396 (1.0); 7.2368 (1.0); 7.2179 (0.5); 7.1710 (0.5); 7.1461 (0.6); 7.1430 (0.5); 5.3213 (5.4); 4.8362 (4.7); 3.7717 (0.6); 3.7692 (0.6); 3.7609 (1.6); 3.7542 (1.2); 3.7476 (1.4); 3.7453 (1.4); 3.7138 (1.4); 3.7115 (1.5); 3.7050 (1.2); 3.7005 (1.2); 3.6983 (1.6); 3.6901 (0.7); 3.6874 (0.6); 3.6467 (1.3); 3.6378 (1.3); 3.6354 (1.8); 3.6306 (1.4); 3.6231 (2.2); 3.5250 (2.1); 3.5174 (1.3); 3.5128 (1.7); 3.5102 (1.3); 3.5015 (1.3); 3.3481 (1.0); 3.3414 (16.0); 2.1584 (3.9); 2.1535 (4.0); 1.5749 (4.2); -0.0002 (18.3); -0.0085 (0.6)

### I-152: ¹H-NMR(400.6 MHz, CDCl₃):

δ= 7.3998 (5.8); 7.3985 (5.4); 7.2610 (13.4); 7.2433 (0.7); 7.2309 (1.3); 7.2224 (0.6); 7.2196 (0.7); 7.2129 (1.0); 7.2060 (0.9); 7.2003 (0.9); 7.1884 (1.3); 7.1811 (3.0); 7.1785 (1.8); 7.1766 (1.8); 7.1748 (1.9); 7.1711 (2.0); 7.1676 (3.1); 7.1626 (1.2); 7.1574 (1.6); 5.3684 (2.0); 5.3445 (8.4); 5.3321 (7.4); 5.3082 (1.8); 5.2704 (0.8); 5.2680 (0.7); 3.9545 (3.8); 3.9105 (4.1); 3.0229 (4.6); 2.9789 (4.2); 2.1687 (9.6); 2.1679 (9.7); 2.1643 (9.9); 2.0453 (2.0); 1.8304 (16.0); 1.5531 (2.4); 1.3038 (0.9); 1.2906 (1.0); 1.2863 (1.1); 1.2772 (1.8); 1.2647 (4.3); 1.2597 (3.7); 1.2417 (1.0); 0.8988 (2.4); 0.8819 (8.4); 0.8642 (3.1); 0.0079 (0.6); -0.0002 (21.3); -0.0026 (0.9); -0.0085 (0.7)

### I-153: ¹H-NMR(400.6 MHz, CDCl₃):

δ= 7.3490 (3.1); 7.3477 (3.0); 7.2606 (20.7); 7.2318 (0.5); 7.2245 (0.6); 7.2119 (0.9); 7.2064 (0.5); 7.1996 (0.6); 7.1677 (0.8); 7.1661 (0.8); 7.1566 (1.9); 7.1480 (1.5); 7.1443 (1.2); 7.1366 (1.0); 5.3228 (7.6); 3.6702 (16.0); 3.3637 (1.4); 3.3475 (2.2); 3.3308 (1.6); 2.6939 (1.8); 2.6771 (2.5); 2.6610 (1.7); 2.1750 (5.2); 2.1707 (5.2); 1.5444 (9.7); 0.0080 (0.8); -0.0002 (32.2); -0.0085 (0.9)

### I-154: ¹H-NMR(400.6 MHz, CDCl₃):

δ= 7.3457 (3.6); 7.3443 (3.5); 7.2611 (8.3); 7.1999 (0.9); 7.1875 (0.6); 7.1817 (0.8); 7.1748 (0.6); 7.1568 (1.0); 7.1366 (1.2); 7.1295 (2.1); 7.1272 (1.2); 7.1204 (2.1); 7.1184 (1.4); 7.1137 (0.8); 7.1114 (1.5); 6.2244 (2.2); 6.2229 (2.2); 5.7531 (0.7); 5.7494 (1.9); 5.7455 (1.9); 5.7418 (0.7); 5.3434 (16.0); 2.1570 (5.9); 2.1558 (6.1); 2.1513 (6.1); 2.1500 (5.9); 2.1082 (5.6); 2.1059 (6.9); 2.1044 (6.9); 2.1022 (5.6); -0.0002 (13.3)

### I-155: ¹H-NMR(400.6 MHz, CDCl₃):

δ= 7.3141 (4.1); 7.3127 (4.1); 7.2618 (8.6); 7.2168 (0.7); 7.2102 (0.9); 7.2046 (0.5); 7.1919 (1.0); 7.1854 (1.1); 7.1828 (0.6); 7.1678 (2.0); 7.1629 (0.7); 7.1597 (1.8); 7.1572 (2.4); 7.1494 (2.2); 7.1419 (0.9); 7.1299 (0.8); 5.2934 (16.0); 4.3234 (0.7); 4.3057 (0.7); 3.8632 (0.6); 3.8592 (0.8); 3.8511 (12.8); 3.8315 (0.5); 3.5496 (0.6); 3.5321 (0.6); 3.4433 (1.8); 3.4258 (5.9); 3.4083 (6.0); 3.3908 (2.0); 3.3738 (0.8); 2.1591 (7.1); 2.1548 (7.1); 2.1541 (6.9); 1.5678 (2.3); 1.4267 (0.7); 1.4090 (1.4); 1.3913 (0.7); 1.2329 (0.7); 1.2154 (1.4); 1.1979 (0.6); 1.1094 (6.1); 1.0919 (12.5); 1.0744 (6.0); -0.0002 (14.2)

### I-156: ¹H-NMR(400.6 MHz, CDCl₃):

δ= 7.3933 (8.9); 7.3919 (9.3); 7.3270 (1.0); 7.3083 (1.8); 7.2979 (0.9); 7.2932 (1.1); 7.2604 (79.7); 7.2342 (0.8); 7.2298 (0.8); 7.2163 (0.7); 7.2136 (1.6); 7.2092 (1.9); 7.2048 (0.9); 7.1959 (1.6); 7.1908 (1.6); 7.1889 (1.8); 7.1841 (1.4); 7.1712 (3.0); 7.1681 (2.1); 7.1663 (1.7); 7.1579 (2.0); 7.1558 (2.0); 7.1522 (1.5); 7.1489 (1.8); 7.1396 (1.4); 7.1367 (1.8); 7.1316 (0.8); 7.1283 (0.8); 7.1190 (0.6); 7.1161 (0.7); 5.3779 (7.8); 5.3543 (11.3); 5.2594 (7.3); 5.2358 (5.1); 4.3960 (1.9); 4.3901 (2.2); 4.3738 (2.1); 4.3678 (2.1); 3.8240 (3.0); 2.9197 (0.7); 2.8975 (0.7); 2.2068 (0.9); 2.1930 (16.0); 2.1882 (15.9); 2.1871 (15.8); 2.1715 (1.1); 2.1613 (1.2); 2.1551 (1.0); 2.1427 (1.0); 2.1259 (1.0); 2.0667 (0.6); 2.0603 (0.5); 2.0500 (0.6); 2.0376 (1.1); 2.0341 (1.2); 2.0276 (1.2); 2.0210 (1.2); 2.0171 (1.2); 2.0045 (0.9); 1.9892 (1.0); 1.9824 (0.8); 1.9760 (1.1); 1.9670 (1.1); 1.9606 (1.2); 1.9539 (1.2); 1.9428 (0.7); 1.9381 (1.2); 1.9278 (0.6); 1.9207 (0.6); 1.9053 (0.7); 1.8460 (0.6); 1.8295 (0.9); 1.8205 (0.8); 1.8136 (1.3); 1.8056 (0.9); 1.7976 (1.3); 1.7917 (0.8); 1.7823 (0.6); 1.6823 (0.8); 1.6662 (0.9); 1.6530 (1.4); 1.6374 (1.2); 1.6220 (1.1); 1.6085 (0.6); 1.5391 (13.0); 0.8818 (0.5); 0.0080 (3.1); 0.0058 (0.8); 0.0051 (0.7); -0.0002 (132.1); -0.0085 (4.2)

### I-157: ¹H-NMR(400.6 MHz, CDCl₃):

δ= 7.3412 (4.2); 7.3398 (4.0); 7.2605 (7.5); 7.2225 (0.9); 7.2043 (1.2); 7.1976 (0.7); 7.1798 (0.9); 7.1673 (3.1); 7.1649 (1.4); 7.1583 (3.3); 7.1511 (0.9); 7.1490 (1.5); 7.1415 (0.8); 5.9147 (0.7); 5.8896 (1.0); 5.8719 (1.1); 5.8468 (0.9); 5.4403 (1.5); 5.4377 (1.5); 5.4150 (1.4); 5.4123 (1.4); 5.3572 (0.6); 5.3543 (1.7); 5.3513 (1.6); 5.3484 (0.6); 5.3146 (0.5); 5.3117 (1.5); 5.3087 (1.4); 5.2721 (16.0); 4.3727 (3.0); 4.3562 (2.1); 4.3544 (2.9); 2.1741 (7.1); 2.1731 (7.0); 2.1692 (7.4); 1.5439 (0.6); 1.2560 (0.7); 0.8816 (0.9); -0.0002 (12.2)

### I-159: ¹H-NMR(400.6 MHz, CDCl₃):

δ= 9.3319 (0.7); 8.6517 (0.8); 7.6538 (6.8); 7.2606 (15.7); 7.1559 (0.6); 7.1513 (0.5); 7.1396 (0.6); 7.1370 (0.7); 7.1211 (0.7); 7.1185 (1.6); 7.1141 (0.9); 7.1042 (1.5); 7.1028 (1.2); 7.1009 (1.3); 7.0975 (1.1); 7.0874 (1.3); 7.0828 (1.1); 5.3401 (11.5); 5.3000 (0.5); 3.3904 (16.0); 3.1406 (2.3); 2.0445 (0.6); 1.5446 (0.8); 1.2590 (0.7); 1.2550 (0.7); 0.0080 (0.6); -0.0002 (21.6); -0.0085 (0.6)

### I-160: ¹H-NMR(400.6 MHz, d₆-DMSO):

δ= 8.1419 (6.4); 7.4046 (0.9); 7.3985 (0.8); 7.3958 (0.6); 7.3926 (0.8); 7.3851 (0.8); 7.3826 (0.6); 7.3811 (0.6); 7.3390 (0.5); 5.3576 (6.5); 3.6099 (1.2); 3.5948 (0.8); 3.5909 (1.2); 3.5867 (0.7); 3.5718 (1.3); 3.3193 (16.0); 2.5193 (0.7); 2.5105 (10.9); 2.5060 (24.3); 2.5014 (34.5); 2.4968 (24.3); 2.4922 (11.0); 1.7667 (0.6); 1.7479 (1.0); 1.7289 (1.1); 1.7102 (0.7); 0.9742 (2.6); 0.9558 (5.7); 0.9371 (2.4); 0.0079 (0.9); -0.0002 (30.3); -0.0085 (0.9)

### I-161: ¹H-NMR(400.6 MHz, CDCl₃):

δ= 7.4221 (0.8); 7.4195 (0.5); 7.4171 (0.6); 7.4148 (0.6); 7.4089 (0.6); 7.4065 (0.5); 7.4041 (0.7); 7.4015 (1.0); 7.3965 (0.7); 7.3883 (0.6); 7.3835 (0.7); 7.3734 (0.7); 7.3689 (0.6); 7.3546 (1.6); 7.3500 (1.4); 7.3459 (4.1); 7.3444 (4.0); 7.3361 (1.1); 7.3315 (0.8); 7.2683 (1.4); 7.2654 (1.8); 7.2614 (20.9); 7.2498 (1.7); 7.2469 (1.7); 7.2310 (0.8); 7.2281 (0.9); 7.1845 (0.9); 7.1820 (0.8); 7.1637 (0.8); 7.1598 (1.0); 7.1566 (0.8); 7.1385 (0.8); 7.1360 (0.7); 5.3292 (9.0); 4.8566 (7.7); 4.4129 (0.9); 4.2062 (0.5); 4.1883 (1.0); 4.1692 (16.0); 4.1560 (0.6); 3.8082 (0.9); 3.8047 (1.0); 3.7998 (2.0); 3.7948 (1.7); 3.7901 (2.2); 3.7863 (3.6); 3.7851 (3.6); 3.7722 (3.4); 3.7706 (3.3); 3.7671 (2.2); 3.7622 (1.4); 3.7573 (1.8); 3.7525 (0.9); 3.7488 (0.7); 2.1661 (6.5); 2.1651 (6.7); 2.1614 (6.9); 2.1604 (6.5); 0.0079 (0.8); -0.0002 (30.4); -0.0085 (0.8)

### I-162: ¹H-NMR(400.6 MHz, CDCl₃):

δ= 7.3325 (3.8); 7.3310 (3.7); 7.3230 (1.6); 7.3216 (1.6); 7.2664 (0.6); 7.2606 (12.0); 7.2500 (0.6); 7.2445 (0.6); 7.2407 (0.6); 7.2280 (0.6); 7.2241 (0.6); 7.2188 (0.6); 7.2022 (0.6); 7.0315 (1.0); 7.0155 (0.9); 7.0067 (0.7); 6.9905 (0.7); 6.9835 (0.6); 6.9668 (0.7); 5.3038 (16.0); 5.2963 (6.6); 3.6389 (1.0); 3.6246 (0.5); 3.6196 (1.0); 3.6102 (2.6); 3.6000 (1.2); 3.5959 (1.5); 3.5908 (2.2); 3.5846 (1.2); 3.5713 (2.6); 2.3172 (2.1); 2.3130 (2.2); 2.1668 (0.6); 2.1624 (3.1); 2.1568 (8.3); 2.1510 (6.3); 2.1499 (6.1); 1.9095 (1.2); 1.9061 (0.6); 1.8953 (0.6); 1.8906 (1.9); 1.8873 (1.1); 1.8768 (0.6); 1.8711 (1.9); 1.8672 (1.1); 1.8521 (1.3); 1.8485 (0.7); 1.5435 (1.4); 1.0648 (4.5); 1.0620 (2.3); 1.0462 (9.5); 1.0433 (4.7); 1.0276 (4.2); 1.0247 (2.1); -0.0002 (20.0); -0.0085 (0.6)

### I-163: ¹H-NMR(400.6 MHz, CDCl₃):

δ= 7.3263 (1.9); 7.3251 (1.9); 7.3180 (0.7); 7.2602 (30.6); 5.3310 (5.2); 5.3264 (2.0); 3.0501 (1.0); 3.0306 (2.1); 3.0120 (1.9); 2.9935 (0.6); 2.3290 (1.0); 2.3248 (1.0); 2.1594 (1.4); 2.1538 (4.4); 2.1480 (3.4); 1.5390 (16.0); 1.1911 (2.7); 1.1876 (1.1); 1.1727 (5.7); 1.1691 (2.2); 1.1543 (2.6); 1.1506 (1.0); 0.0080 (1.2); -0.0002 (45.0); -0.0085 (1.2)

### I-164: ¹H-NMR(400.6 MHz, CDCl₃):

δ= 7.3391 (2.1); 7.3377 (2.2); 7.3306 (0.8); 7.3292 (0.8); 7.2602 (35.3); 7.0207 (0.6); 6.9969 (0.7); 5.3254 (5.4); 5.3212 (2.2); 2.6141 (3.6); 2.6037 (9.6); 2.3241 (1.1); 2.3196 (1.2); 2.1671 (1.6); 2.1615 (4.8); 2.1558 (3.8); 2.1548 (3.8); 1.5395 (16.0); 0.0080 (1.3); -0.0002 (51.2); -0.0085 (1.6)

### I-165: ¹H-NMR(400.6 MHz, CDCl₃):

δ= 7.3490 (3.0); 7.3477 (2.8); 7.3401 (1.2); 7.2666 (0.6); 7.2602 (36.7); 7.2454 (0.6); 7.2415 (0.6); 7.2361 (0.5); 7.1364 (0.6); 7.0314 (0.6); 7.0075 (0.7); 6.9915 (0.7); 6.9832 (0.6); 6.9666 (0.7); 5.3002 (11.6); 5.2923 (4.5); 4.9507 (0.9); 3.4015 (6.6); 3.3872 (16.0); 2.3160 (1.7); 2.3117 (1.8); 2.1683 (2.4); 2.1623 (7.0); 2.1565 (5.5); 2.0351 (1.2); 2.0303 (1.2); 0.0079 (1.6); -0.0002 (54.2); -0.0085 (1.8)

### I-166: ¹H-NMR(400.6 MHz, CDCl₃):

δ= 9.3462 (14.2); 9.3357 (0.7); 8.6589 (16.0); 8.6492 (0.6); 7.6104 (1.1); 7.2906 (0.7); 7.2845 (0.8); 7.2767 (0.8); 7.2671 (1.5); 7.2611 (57.0); 7.2542 (1.8); 7.2486 (1.4); 7.2447 (1.2); 7.2367 (1.0); 7.2306 (1.1); 7.1514 (1.0); 7.1464 (1.0); 7.1343 (1.0); 7.1289 (2.2); 7.1232 (1.4); 7.1111 (1.3); 7.1059 (1.9); 7.1007 (0.9); 7.0887 (0.8); 7.0836 (0.8); 5.3004 (7.7); 5.0835 (1.4); 4.6310 (0.9); 2.0457 (1.1); 1.5754 (3.6); 1.3713 (0.7); 1.2596 (1.1); 1.2550 (0.9); 0.0080 (1.9); -0.0002 (76.0); -0.0085 (2.3)

### I-167: ¹H-NMR(400.6 MHz, CDCl₃):

δ= 7.3311 (5.1); 7.3298 (5.3); 7.2613 (9.1); 7.1175 (0.6); 7.1126 (1.1); 7.1097 (0.7); 7.1072 (0.5); 7.0991 (4.0); 7.0945 (1.1); 7.0917 (1.8); 7.0866 (3.8); 7.0793 (4.6); 7.0754 (0.8); 7.0734 (1.0); 7.0680 (2.0); 7.0659 (1.9); 7.0611 (0.7); 7.0595 (0.8); 5.3333 (14.5); 3.0681 (1.6); 3.0496 (5.1); 3.0312 (5.2); 3.0127 (1.7); 2.1638 (9.7); 2.1584 (9.7); 1.5603 (3.3); 1.1894 (7.8); 1.1710 (16.0); 1.1525 (7.5); -0.0002 (12.7)

### I-168: ¹H-NMR(400.6 MHz, CDCl₃):

δ= 7.3438 (3.4); 7.3424 (3.6); 7.2608 (12.3); 7.1101 (0.8); 7.1017 (0.7); 7.0980 (2.5); 7.0947 (0.9); 7.0894 (1.2); 7.0852 (2.7); 7.0780 (2.5); 7.0735 (0.8); 7.0663 (1.8); 7.0592 (0.8); 5.3279 (8.8); 2.6159 (16.0); 2.1728 (5.9); 2.1717 (6.4); 2.1674 (6.3); 2.1663 (6.4); 1.5504 (2.4); -0.0002 (17.0); -0.0085 (0.5)

### I-169: ¹H-NMR(400.6 MHz, CDCl₃):

δ= 7.3365 (3.8); 7.3352 (3.8); 7.2606 (7.4); 7.1163 (0.5); 7.1146 (0.6); 7.1125 (0.7); 7.1076 (0.6); 7.1048 (0.5); 7.1016 (0.6); 7.0983 (1.3); 7.0966 (1.3); 7.0945 (1.0); 7.0913 (1.0); 7.0891 (1.2); 7.0844 (1.4); 7.0822 (1.5); 7.0796 (0.9); 7.0770 (2.2); 7.0741 (1.0); 7.0710 (1.3); 7.0640 (1.6); 7.0611 (0.9); 7.0595 (1.3); 7.0545 (0.5); 5.3047 (16.0); 3.6351 (2.7); 3.6208 (1.4); 3.6157 (2.5); 3.6095 (1.3); 3.5962 (2.8); 2.1677 (6.6); 2.1669 (6.8); 2.1623 (6.9); 2.1615 (6.7); 1.9088 (1.2); 1.8948 (0.5); 1.8899 (1.9); 1.8842 (0.6); 1.8762 (0.6); 1.8704 (1.9); 1.8656 (0.6); 1.8515 (1.3); 1.5443 (0.9); 1.0629 (4.7); 1.0443 (9.9); 1.0257 (4.4); -0.0002 (11.8)

### I-170: ¹H-NMR(400.6 MHz, CDCl₃):

δ= 7.2645 (0.6); 7.2605 (35.4); 7.2564 (0.6); 7.1201 (8.2); 7.1190 (7.8); 7.0959 (0.7); 7.0842 (0.9); 7.0830 (0.9); 7.0793 (1.2); 7.0729 (4.0); 7.0648 (1.5); 7.0607 (2.9); 7.0581 (2.9); 7.0519 (4.0); 7.0504 (4.2); 7.0441 (1.9); 7.0396 (4.5); 7.0376 (3.0); 7.0318 (2.3); 7.0237 (1.2); 7.0213 (2.2); 7.0170 (0.8); 7.0131 (0.8); 7.0097 (1.0); 7.0016 (0.6); 6.9997 (0.6); 5.3516 (1.1); 4.8366 (14.2); 2.0358 (15.5); 2.0313 (16.0); 1.3331 (0.5); 1.2843 (0.8); 1.2544 (1.3); 0.0080 (1.3); -0.0002 (50.6); -0.0085 (1.4)

### I-171: ¹H-NMR(400.6 MHz, CDCl₃):

δ= 7.3540 (2.7); 7.3526 (2.6); 7.2606 (7.8); 7.1174 (0.6); 7.1156 (0.7); 7.0972 (1.0); 7.0949 (0.8); 7.0915 (0.7); 7.0847 (1.2); 7.0782 (1.4); 7.0738 (1.0); 7.0663 (0.6); 7.0647 (0.8); 7.0619 (1.2); 5.3008 (11.4); 3.4022 (16.0); 2.1733 (4.7); 2.1722 (4.6); 2.1678 (4.8); 2.1667 (4.5); 1.5426 (1.0); -0.0002 (11.0)

### I-172: ¹H-NMR(400.6 MHz, CDCl₃):

δ= 7.5193 (0.6); 7.4294 (0.9); 7.4162 (1.9); 7.3982 (2.0); 7.3778 (2.4); 7.3582 (2.8); 7.3328 (8.6); 7.2609 (74.5); 7.2429 (3.8); 7.2244 (1.8); 7.1787 (2.1); 7.1541 (2.8); 7.1318 (1.7); 5.3270 (12.9); 5.2964 (0.8); 4.8418 (9.5); 4.8289 (3.4); 4.2869 (1.4); 4.1916 (1.0); 4.1693 (3.5); 3.7352 (10.8); 3.7272 (12.1); 3.7113 (10.9); 3.6282 (4.8); 3.6170 (5.1); 3.6067 (3.1); 2.4107 (0.7); 2.1602 (16.0); 2.0667 (0.9); 1.8501 (3.4); 1.6754 (0.6); 1.6596 (0.6); 1.2561 (2.0); 0.1472 (0.6); -0.0002 (114.5)

### I-173: ¹H-NMR(400.6 MHz, CDCl₃):

δ= 9.2342 (3.1); 9.2321 (5.9); 9.2300 (3.2); 7.6738 (16.0); 7.2602 (52.8); 7.2480 (1.2); 7.2319 (1.1); 7.2265 (1.2); 7.2229 (1.2); 7.2102 (1.1); 7.2067 (1.2); 7.2013 (1.1); 7.1851 (1.0); 7.0576 (1.1); 7.0414 (1.1); 7.0352 (1.2); 7.0327 (1.3); 7.0190 (1.2); 7.0164 (1.2); 7.0103 (1.1); 6.9940 (1.1); 5.3029 (14.7); 5.3006 (15.1); 1.5418 (1.9); 0.0079 (2.2); 0.0063 (0.5); -0.0002 (77.6); -0.0051 (1.4); -0.0085 (2.4)

### I-174: ¹H-NMR(400.6 MHz, CDCl₃):

δ= 7.4859 (1.3); 7.4789 (1.0); 7.4756 (1.4); 7.4695 (1.3); 7.4635 (1.9); 7.4629 (1.8); 7.3644 (0.6); 7.3527 (3.2); 7.3460 (2.5); 7.3418 (2.0); 7.3399 (2.1); 7.3359 (6.8); 7.3292 (4.5); 7.3172 (0.8); 7.2865 (0.6); 7.2822 (0.8); 7.2794 (2.3); 7.2788 (2.3); 7.2727 (1.5); 7.2665 (1.5); 7.2632 (1.3); 7.2604 (16.5); 7.2562 (1.5); 5.3577 (1.0); 5.3249 (1.6); 5.3144 (1.1); 5.2993 (0.6); 2.9951 (1.6); 2.9783 (2.2); 2.9754 (2.1); 2.9579 (1.9); 2.3260 (1.7); 2.3247 (1.6); 2.1413 (1.9); 2.0752 (15.0); 2.0739 (16.0); 2.0209 (2.0); 2.0196 (2.1); 1.9587 (0.8); 1.9577 (0.8); 1.7416 (0.6); 1.6773 (1.7); 1.6588 (3.0); 1.6488 (0.6); 1.6400 (2.9); 1.6303 (0.6); 1.6214 (1.8); 1.6032 (0.5); 0.9659 (0.6); 0.9475 (1.3); 0.9288 (0.8); 0.8709 (5.7); 0.8643 (1.1); 0.8526 (12.0); 0.8459 (2.0); 0.8341 (5.2); 0.8275 (0.9); 0.0079 (0.6); -0.0002 (25.2); - 0.0085 (0.8)

### I-175: ¹H-NMR(300.2 MHz, d₆-DMSO):

δ= 8.4948 (0.5); 7.4771 (1.4); 7.4574 (1.5); 7.4507 (1.4); 7.4391 (1.2); 7.4327 (1.7); 7.4280 (1.7); 7.4230 (1.5); 7.4150 (1.4); 7.4060 (2.3); 7.3991 (1.6); 7.3879 (1.4); 7.3812 (1.5); 7.3634 (1.6); 7.3437 (2.6); 7.3377 (2.9); 7.3191 (2.1); 7.3128 (1.9); 7.2709 (5.4); 7.2466 (5.8); 7.2431 (5.8); 7.2317 (7.6); 7.2225 (2.2); 7.2186 (2.0); 7.2127 (1.9); 5.3166 (1.4); 5.2965 (2.2); 5.2783 (1.4); 3.4321 (0.6); 3.3877 (0.7); 3.2757 (0.3); 3.1957 (0.6); 3.1671 (1.7); 2.7840 (0.4); 2.5134 (12.7); 2.5075 (25.3); 2.5016 (34.0); 2.4957 (23.6); 2.2639 (1.2); 2.2053 (0.7); 1.9175 (15.6); 1.9127 (16.0); 1.8648 (0.5); 1.8402 (0.8); 1.8187 (1.6); 1.8021 (1.6); 1.7940 (1.6); 1.7845 (1.8); 1.7776 (1.7); 1.7607 (2.5); 1.7374 (2.2); 1.7145 (1.1); 1.6913 (0.6); 1.4211 (0.7); 1.3961 (1.5); 1.3712 (0.6); 0.9515 (6.7); 0.9273 (14.7); 0.9028 (6.0); 0.0109 (0.6); -0.0001 (21.3); -0.0111 (0.8)

### I-176: ¹H-NMR(300.1 MHz, d₆-DMSO):

δ= 7.5475 (3.9); 7.5168 (0.3); 7.4512 (0.6); 7.4452 (0.7); 7.4220 (1.5); 7.4009 (2.0); 7.3936 (1.4); 7.3824 (1.1); 7.3757 (1.2); 7.3619 (1.4); 7.3430 (2.5); 7.3370 (2.6); 7.3181 (1.9); 7.3114 (1.6); 7.2676 (4.6); 7.2417 (5.5); 7.2046 (9.1); 5.3551 (2.5); 5.3496 (2.5); 5.3329 (2.7); 5.3276 (2.6); 3.3390 (33.0); 2.6463 (0.6); 2.6231 (1.4); 2.5976 (1.4); 2.5733 (1.0); 2.5080 (23.1); 2.5027 (29.4); 2.4973 (21.5); 2.2669 (0.4); 1.9812 (1.2); 1.9574 (1.7); 1.9408 (1.9); 1.9132 (16.0); 1.9089 (16.0); 1.8711 (2.8); 1.8455 (3.5); 1.8268 (3.9); 1.8027 (3.7); 1.7800 (2.1); 1.7007 (0.4); -0.0002 (5.1)

### I-177: ¹H-NMR(400.1 MHz, d₆-DMSO):

δ= 7.4389 (0.5); 7.4343 (0.6); 7.4254 (0.6); 7.4202 (1.2); 7.4163 (1.1); 7.4135 (1.1); 7.4021 (4.5); 7.3869 (0.8); 7.3822 (0.8); 7.3664 (0.7); 7.3616 (0.9); 7.3465 (1.8); 7.3423 (1.8); 7.3282 (1.2); 7.3236 (1.1); 7.2597 (3.7); 7.2398 (4.0); 7.2348 (1.8); 7.2235 (1.1); 7.2208 (1.1); 7.2139 (1.2); 7.1960 (5.6); 5.7566 (0.9); 3.3193 (120.6); 3.2963 (0.5); 2.5100 (16.8); 2.5056 (33.8); 2.5011 (45.3); 2.4966 (31.7); 2.4921 (14.4); 1.9205 (10.2); 1.9170 (9.9); 1.8849 (0.7); 1.8676 (0.9); 1.8501 (1.7); 1.8317 (1.6); 1.8187 (1.6); 1.8006 (1.7); 1.7833 (1.0); 1.7655 (0.6); 1.6208 (16.0); 0.9860 (4.2); 0.9679 (9.3); 0.9495 (3.8); 0.0080 (1.1); -0.0002 (29.2); -0.0084 (1.0)

### I-178: ¹H-NMR(400.6 MHz, CDCl₃):

δ= 7.6450 (7.5); 7.2713 (0.6); 7.2618 (4.5); 7.2550 (0.6); 7.2496 (0.6); 7.2459 (0.6); 7.2333 (0.6); 7.2296 (0.6); 7.2243 (0.6); 7.2079 (0.6); 7.0588 (0.6); 7.0425 (0.6); 7.0360 (0.6); 7.0338 (0.7); 7.0198 (0.6); 7.0176 (0.7); 7.0111 (0.6); 6.9949 (0.6); 5.3571 (9.1); 2.5741 (16.0); -0.0002 (6.7)

### I-179: ¹H-NMR(400.6 MHz, CDCl₃):

δ= 9.2807 (1.1); 8.2563 (0.7); 7.7180 (0.6); 7.5198 (1.0); 7.4357 (4.2); 7.4344 (4.2); 7.2614 (185.7); 7.2174 (2.7); 7.1832 (1.5); 7.1770 (1.5); 7.1627 (2.0); 7.1071 (1.3); 7.0899 (0.9); 7.0844 (1.6); 7.0615 (1.2); 7.0437 (0.9); 6.9977 (1.2); 5.4141 (16.0); 5.3973 (0.8); 5.3006 (5.0); 5.2412 (1.5); 5.1511 (1.3); 5.0812 (1.9); 4.6181 (0.7); 4.5995 (0.9); 3.0061 (0.7); 2.4170 (1.4); 2.4121 (1.5); 2.2121 (7.8); 2.2059 (8.4); 2.0886 (1.2); 2.0662 (12.4); 2.0249 (1.6); 2.0067 (1.4); 1.8051 (1.0); 1.7957 (1.1); 1.3712 (0.8); 1.3532 (0.9); 1.3335 (0.6); 1.2541 (4.2); 0.8802 (1.0); 0.1569 (1.2); 0.1264 (1.4); 0.0690 (2.9); -0.0002 (108.0); -0.0084 (5.8)

### I-180: ¹H-NMR(400.2 MHz, d₆-DMSO):

δ= 7.5082 (4.3); 7.4423 (0.7); 7.4376 (0.8); 7.4288 (0.8); 7.4235 (1.7); 7.4195 (1.4); 7.4102 (1.3); 7.4043 (1.9); 7.3989 (1.2); 7.3903 (1.0); 7.3856 (1.0); 7.3574 (1.0); 7.3526 (1.4); 7.3374 (2.6); 7.3334 (2.7); 7.3194 (1.9); 7.3146 (1.6); 7.2776 (0.4); 7.2630 (5.6); 7.2432 (5.8); 7.2269 (1.6); 7.2244 (1.6); 7.2106 (8.6); 7.1777 (0.4); 5.7590 (2.3); 4.8793 (3.0); 4.8757 (2.9); 4.8598 (3.0); 4.8562 (2.9); 3.3289 (6.1); 2.5069 (7.1); 2.5026 (9.3); 2.4982 (6.6); 2.0758 (0.6); 1.9188 (16.0); 1.9156 (15.5); 1.8819 (0.7); 1.3489 (0.6); 1.3412 (0.9); 1.3292 (1.8); 1.3213 (1.1); 1.3173 (1.2); 1.3094 (1.8); 1.2975 (1.0); 1.2897 (0.7); 1.2776 (0.3); 0.6104 (0.4); 0.5973 (0.5); 0.5867 (1.0); 0.5794 (1.4); 0.5689 (1.6); 0.5598 (0.9); 0.5554 (1.0); 0.5492 (1.0); 0.5433 (1.0); 0.5374 (1.1); 0.5348 (1.1); 0.5273 (1.2); 0.5236 (1.2); 0.5144 (1.5); 0.5074 (1.1); 0.4925 (0.5); 0.4838 (0.5); 0.4445 (0.5); 0.4326 (0.8); 0.4206 (1.2); 0.4090 (2.1); 0.3999 (2.7); 0.3897 (2.5); 0.3818 (1.8); 0.3696 (1.0); 0.3579 (0.6); -0.0001 (4.2)

### I-181: ¹H-NMR(300.1 MHz, d₆-DMSO):

δ= 7.4530 (4.2); 7.4365 (1.0); 7.4294 (1.2); 7.4242 (1.3); 7.4025 (1.6); 7.3956 (1.1); 7.3842 (0.9); 7.3778 (1.1); 7.3606 (0.9); 7.3404 (2.0); 7.3344 (2.1); 7.3159 (1.5); 7.3099 (1.6); 7.2691 (3.7); 7.2447 (4.2); 7.2162 (1.8); 7.2067 (6.9); 3.5256 (1.4); 3.4939 (4.5); 3.4733 (4.7); 3.4417 (1.6); 3.3359 (27.3); 3.3263 (22.2); 3.1945 (0.4); 2.5132 (14.5); 2.5075 (26.7); 2.5017 (34.0); 2.4958 (23.7); 2.4187 (0.4); 2.0722 (0.3); 1.9216 (10.9); 1.9172 (10.3); 1.6189 (16.0); 0.0108 (1.1); -0.0001 (20.7); -0.0111 (0.8)

### I-183: ¹H-NMR(400.6 MHz, CDCl₃):

δ= 7.5499 (1.9); 7.5460 (2.8); 7.5409 (0.9); 7.5329 (1.6); 7.5290 (3.5); 7.5257 (3.0); 7.5203 (0.6); 7.4506 (1.0); 7.4470 (1.6); 7.4424 (0.7); 7.4294 (3.6); 7.4254 (1.6); 7.4144 (1.1); 7.4111 (2.1); 7.4096 (1.7); 7.3942 (1.0); 7.3904 (1.8); 7.3868 (1.0); 7.3790 (0.6); 7.3725 (1.7); 7.3544 (0.5); 7.3342 (4.1); 7.3329 (4.0); 7.2594 (14.5); 5.3100 (16.0); 5.2987 (5.2); 3.7364 (1.4); 3.7179 (4.6); 3.6995 (4.7); 3.6811 (1.4); 2.3160 (14.4); 2.3148 (13.8); 1.5410 (1.9); 1.4105 (4.7); 1.3921 (10.4); 1.3737 (4.7); -0.0002 (15.8); -0.0085 (0.7)

### I-184: ¹H-NMR(400.6 MHz, CDCl₃):

δ= 7.5375 (1.8); 7.5336 (2.6); 7.5284 (0.8); 7.5165 (3.6); 7.5133 (3.1); 7.5080 (0.6); 7.4669 (1.1); 7.4636 (1.8); 7.4590 (0.7); 7.4460 (3.7); 7.4419 (1.7); 7.4307 (1.0); 7.4273 (1.9); 7.4068 (0.9); 7.4031 (1.7); 7.3995 (1.0); 7.3915 (0.6); 7.3851 (1.7); 7.3671 (0.5); 7.3255 (4.1); 7.2599 (50.9); 5.3304 (10.6); 3.1501 (1.1); 3.1317 (3.7); 3.1132 (3.8); 3.0947 (1.3); 2.3168 (14.5); 1.5399 (16.0); 1.2131 (5.2); 1.1947 (10.8); 1.1762 (5.1); 0.0080 (1.7); -0.0002 (55.6); -0.0084 (2.2)

### I-185: ¹H-NMR(400.0 MHz, CDCl₃):

δ= 7.3364 (6.1); 7.2604 (26.2); 7.0894 (4.0); 7.0687 (2.9); 7.0503 (1.4); 5.3328 (13.0); 3.0397 (1.8); 3.0222 (4.9); 3.0041 (4.9); 2.9863 (1.7); 2.1534 (16.0); 1.5408 (14.5); 1.2633 (0.5); 1.1786 (5.2); 1.1606 (9.8); 1.1425 (4.9); -0.0002 (28.7)

### I-187: ¹H-NMR(400.6 MHz, CDCl₃):

δ= 9.1088 (5.6); 8.9099 (2.8); 8.8970 (2.8); 7.6267 (3.4); 7.6138 (3.3); 7.4156 (0.6); 7.4110 (0.7); 7.4025 (0.7); 7.3975 (1.4); 7.3949 (1.0); 7.3926 (1.2); 7.3905 (1.0); 7.3841 (1.0); 7.3796 (1.3); 7.3771 (1.6); 7.3719 (1.4); 7.3693 (1.5); 7.3642 (1.7); 7.3591 (1.2); 7.3504 (2.7); 7.3460 (2.2); 7.3317 (1.8); 7.3272 (1.5); 7.3195 (6.9); 7.2619 (29.4); 7.2411 (2.2); 7.2383 (2.3); 7.2223 (3.1); 7.2196 (2.9); 7.2036 (1.4); 7.2008 (1.3); 7.1612 (1.6); 7.1586 (1.4); 7.1404 (1.6); 7.1364 (2.0); 7.1334 (1.5); 7.1152 (1.4); 7.1129 (1.2); 5.3189 (16.0); 4.8342 (13.9); 4.5037 (4.3); 4.4947 (3.4); 4.4918 (4.9); 4.4883 (3.1); 4.4796 (4.7); 3.8360 (4.6); 3.8273 (3.2); 3.8238 (5.0); 3.8210 (3.2); 3.8119 (4.3); 3.7694 (0.9); 3.7631 (1.6); 3.7570 (2.6); 3.7532 (2.8); 3.7496 (4.5); 3.7447 (7.6); 3.7370 (7.8); 3.7323 (4.9); 3.7288 (3.0); 3.7248 (2.5); 3.7191 (1.8); 3.7127 (1.0); 2.4611 (1.0); 2.1532 (12.6); 2.1487 (12.5); 1.1507 (0.8); 1.1327 (1.6); 1.1147 (0.8); 0.0080 (1.0); -0.0002 (33.5); -0.0084 (1.5)

### I-188: ¹H-NMR(300.1 MHz, d₆-DMSO):

δ= 7.5022 (4.1); 7.4486 (0.5); 7.4428 (0.6); 7.4249 (1.1); 7.4196 (1.2); 7.4058 (1.0); 7.3986 (1.6); 7.3913 (1.1); 7.3792 (1.5); 7.3737 (1.7); 7.3541 (2.0); 7.3483 (2.0); 7.3293 (1.4); 7.3232 (1.2); 7.2661 (3.4); 7.2401 (4.2); 7.2076 (1.6); 7.1905 (6.3); 3.3434 (17.8); 2.5089 (7.0); 2.5035 (8.8); 2.4982 (6.3); 2.0778 (4.2); 2.0601 (0.6); 2.0377 (1.3); 2.0154 (1.8); 1.9929 (1.4); 1.9711 (0.7); 1.9211 (11.2); 1.9169 (10.8); 1.5996 (16.0); 0.9936 (8.8); 0.9705 (15.8); 0.9475 (8.4); -0.0002 (1.1)

### I-189: ¹H-NMR(300.1 MHz, d₆-DMSO):

δ= 7.4482 (0.5); 7.4425 (0.6); 7.4245 (1.2); 7.4190 (1.2); 7.3978 (1.6); 7.3905 (1.1); 7.3793 (1.0); 7.3720 (1.4); 7.3647 (1.1); 7.3453 (2.1); 7.3394 (2.2); 7.3206 (1.7); 7.3141 (1.6); 7.2977 (4.3); 7.2636 (3.8); 7.2383 (4.2); 7.2102 (1.3); 7.2052 (1.4); 7.1796 (6.3); 3.3234 (14.8); 3.1767 (0.9); 3.1593 (0.9); 2.5073 (14.6); 2.5017 (18.9); 2.4962 (13.7); 2.0747 (0.3); 1.9202 (11.4); 1.9159 (11.6); 1.6705 (16.0); 1.3630 (0.4); 1.3452 (0.8); 1.3355 (0.9); 1.3287 (0.7); 1.3178 (1.6); 1.3003 (1.0); 1.2908 (0.9); 1.2738 (0.5); 0.6125 (0.8); 0.5950 (1.1); 0.5863 (1.0); 0.5690 (0.9); 0.5038 (0.3); 0.4671 (2.5); 0.4395 (2.7); 0.4126 (1.3); 0.3969 (1.1); 0.3877 (1.0); 0.3711 (0.7); -0.0002 (10.0)

### I-190: ¹H-NMR(400.2 MHz, d₆-DMSO):

δ= 7.4970 (4.0); 7.4414 (0.5); 7.4367 (0.6); 7.4279 (0.6); 7.4233 (1.2); 7.4188 (1.0); 7.4162 (0.9); 7.4095 (0.9); 7.4033 (1.4); 7.3980 (0.9); 7.3893 (0.7); 7.3845 (0.9); 7.3767 (0.8); 7.3739 (1.0); 7.3588 (1.9); 7.3545 (1.9); 7.3402 (1.3); 7.3357 (1.1); 7.2630 (3.6); 7.2440 (4.2); 7.2263 (1.2); 7.2235 (1.3); 7.2184 (1.3); 7.1806 (6.1); 3.3365 (28.9); 2.6710 (1.1); 2.6500 (1.5); 2.6287 (1.1); 2.5114 (7.6); 2.5071 (14.7); 2.5027 (19.2); 2.4982 (13.5); 2.4939 (6.2); 2.0825 (0.8); 2.0772 (1.0); 2.0563 (1.2); 2.0333 (0.9); 1.9191 (11.0); 1.9157 (10.6); 1.8839 (1.8); 1.8756 (1.3); 1.8637 (2.4); 1.8462 (1.6); 1.8259 (0.9); 1.8199 (0.8); 1.8045 (0.7); 1.7857 (0.7); 1.7626 (1.0); 1.7402 (0.9); 1.7180 (0.5); 1.6700 (0.4); 1.6605 (0.6); 1.6496 (0.6); 1.6415 (0.8); 1.6164 (0.5); 1.5255 (16.0); 0.0080 (0.4); -0.0001 (7.6)

The present invention furthermore provides the use of one or more compounds of the general formula (I) and/or salts thereof, as defined above, preferably in one of the embodiments identified as preferred or particularly preferred, in particular one or more compounds of the formulae (I-001) to (I-192) and/or salts thereof, in each case as defined above, as herbicide and/or plant growth regulator, preferably in crops of useful plants and/or ornamental plants.

The present invention furthermore provides a method for controlling harmful plants and/or for regulating the growth of plants, characterized in that an effective amount
- of one or more compounds of the general formula (I) and/or salts thereof, as defined above, preferably in one of the embodiments identified as preferred or particularly preferred, in particular one or more compounds of the formulae (I-001) to (I-192) and/or salts thereof, in each case as defined above, or
- of a composition according to the invention, as defined below,
   is applied to the (harmful) plants, seeds of (harmful) plants, the soil in which or on which the (harmful) plants grow or the area under cultivation.

The present invention also provides a method for controlling unwanted plants, preferably in crops of useful plants, characterized in that an effective amount
- of one or more compounds of the general formula (I) and/or salts thereof, as defined above, preferably in one of the embodiments identified as preferred or particularly preferred, in particular one or more compounds of the formulae (I-001) to (I-192) and/or salts thereof, in each case as defined above, or
- of a composition according to the invention, as defined below,
   is applied to unwanted plants (for example harmful plants such as mono- or dicotyledonous weeds or unwanted crop plants), the seed of the unwanted plants (i.e. plant seeds, for example grains, seeds or vegetative propagation organs such as tubers or shoot parts with buds), the soil in which or on which the unwanted plants grow (for example the soil of crop land or non-crop land) or the area under cultivation (i.e. the area on which the unwanted plants will grow).

The present invention furthermore also provides methods for regulating the growth of plants, preferably of useful plants, characterized in that an effective amount
- of one or more compounds of the general formula (I) and/or salts thereof, as defined above, preferably in one of the embodiments identified as preferred or particularly preferred, in particular one or more compounds of the formulae (I-001) to (I-192) and/or salts thereof, in each case as defined above, or
- of a composition according to the invention, as defined below,
   is applied to the plant, the seed of the plant (i.e. plant seed, for example grains, seeds or vegetative propagation organs such as tubers or shoot parts with buds), the soil in which or on which the plants grow (for example the soil of crop land or non-crop land) or the area under cultivation (i.e. the area on which the plants will grow).

In this context, the compounds according to the invention or the compositions according to the invention can be applied for example by pre-sowing (if appropriate also by incorporation into the soil), pre-emergence and/or post-emergence processes. Specific examples of some representatives of the monocotyledonous and dicotyledonous weed flora which can be controlled by the compounds according to the invention are as follows, though there is no intention to restrict the enumeration to particular species.

In a method according to the invention for controlling harmful plants or for regulating the growth of plants, one or more compounds of the general formula (I) and/or salts thereof are preferably employed for controlling harmful plants or for regulating growth in crops of useful plants or ornamental plants, where in a preferred embodiment the useful plants or ornamental plants are transgenic plants.

The compounds of the general formula (I) according to the invention and/or their salts are suitable for controlling the following genera of monocotyledonous and dicotyledonous harmful plants: Monocotyledonous harmful plants of the genera: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dicotyledonous harmful plants of the genera: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

When the compounds according to the invention are applied to the soil surface before germination of the harmful plants (weed grasses and/or broad-leaved weeds) (pre-emergence method), either the seedlings of the weed grasses or broad-leaved weeds are prevented completely from emerging or they grow until they have reached the cotyledon stage, but then stop growing and eventually, after three to four weeks have elapsed, die completely.

If the active compounds are applied post-emergence to the green parts of the plants, growth stops after the treatment, and the harmful plants remain at the growth stage at the time of application, or they die completely after a certain time, so that in this manner competition by the weeds, which is harmful to the crop plants, is eliminated very early and in a sustained manner.

Although the compounds according to the invention display an outstanding herbicidal activity against monocotyledonous and dicotyledonous weeds, crop plants of economically important crops, for example dicotyledonous crops of the genera Arachis, Beta, Brassica, Cucumis, Cucurbita, Helianthus, Daucus, Glycine, Gossypium, Ipomoea, Lactuca, Linum, Lycopersicon, Miscanthus, Nicotiana, Phaseolus, Pisum, Solanum, Vicia, or monocotyledonous crops of the genera Allium, Ananas, Asparagus, Avena, Hordeum, Oryza, Panicum, Saccharum, Secale, Sorghum, triticale, triticum, Zea, are damaged only to an insignificant extent, or not at all, depending on the structure of the respective compound according to the invention and its application rate. For these reasons, the present compounds are very suitable for selective control of unwanted plant growth in plant crops such as agriculturally useful plants or ornamental plants.

In addition, the compounds of the invention (depending on their particular structure and the application rate deployed) have outstanding growth-regulating properties in crop plants. They intervene in the plants' own metabolism with regulatory effect and can thus be used for the controlled influencing of plant constituents and to facilitate harvesting, for example by triggering desiccation and stunted growth. Furthermore, they are also suitable for the general control and inhibition of unwanted vegetative growth without killing the plants in the process. Inhibition of vegetative growth plays a major role for many mono- and dicotyledonous crops since, for example, this can reduce or completely prevent lodging.

By virtue of their herbicidal and plant growth regulatory properties, the active compounds can also be used to control harmful plants in crops of genetically modified plants or plants modified by conventional mutagenesis. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material.

It is preferred with a view to transgenic crops to use the compounds according to the invention and/or their salts in economically important transgenic crops of useful plants and ornamentals, for example of cereals such as wheat, barley, rye, oats, millet, rice and corn or else crops of sugar beet, cotton, soybean, oilseed rape, potato, tomato, peas and other vegetables.

It is preferred to employ the compounds according to the invention as herbicides in crops of useful plants which are resistant, or have been made resistant by recombinant means, to the phytotoxic effects of the herbicides.

By virtue of their herbicidal and plant growth regulatory properties, the active compounds can also be used to control harmful plants in crops of genetically modified plants which are known or are yet to be developed. In general, the transgenic plants are characterized by particular advantageous properties, for example by resistances to certain pesticides, in particular certain herbicides, resistances to plant diseases or pathogens of plant diseases, such as certain insects or microorganisms such as fungi, bacteria or viruses. Other specific characteristics relate, for example, to the harvested material with regard to quantity, quality, storability, composition and specific constituents. For instance, there are known transgenic plants with an elevated starch content or altered starch quality, or those with a different fatty acid composition in the harvested material. Further special properties may be tolerance or resistance to abiotic stressors, for example heat, cold, drought, salinity and ultraviolet radiation.

Preference is given to the use of the compounds of the general formula (I) according to the invention or salts thereof in economically important transgenic crops of useful plants and ornamental plants, for example of cereals such as wheat, barley, rye, oats, triticale, millet, rice, cassava and corn, or else crops of sugar beet, cotton, soybean, oilseed rape, potatoes, tomatoes, peas and other vegetables.

The compounds of the general formula (I) can preferably be used as herbicides in crops of useful plants which are resistant, or have been made resistant by recombinant means, to the phytotoxic effects of the herbicides.

Conventional ways of producing novel plants which have modified properties in comparison to existing plants consist, for example, in traditional cultivation methods and the generation of mutants. Alternatively, novel plants with altered properties can be generated with the aid of recombinant methods.

A large number of molecular-biological techniques by means of which novel transgenic plants with modified properties can be generated are known to the person skilled in the art. For such recombinant manipulations, nucleic acid molecules which allow mutagenesis or sequence alteration by recombination of DNA sequences can be introduced into plasmids. With the aid of standard methods, it is possible, for example, to undertake base exchanges, remove parts of sequences or add natural or synthetic sequences. To connect the DNA fragments to each other, adapters or linkers may be added to the fragments.

For example, the generation of plant cells with a reduced activity of a gene product can be achieved by expressing at least one corresponding antisense RNA, a sense RNA for achieving a cosuppression effect, or by expressing at least one suitably constructed ribozyme which specifically cleaves transcripts of the abovementioned gene product.

To this end, it is firstly possible to use DNA molecules which encompass the entire coding sequence of a gene product inclusive of any flanking sequences which may be present, and also DNA molecules which only encompass portions of the coding sequence, in which case it is necessary for these portions to be long enough to have an antisense effect in the cells. It is also possible to use DNA sequences which have a high degree of homology to the coding sequences of a gene product but are not completely identical to them.

When expressing nucleic acid molecules in plants, the protein synthesized may be localized in any desired compartment of the plant cell. However, to achieve localization in a particular compartment, it is possible, for example, to join the coding region to DNA sequences which ensure localization in a particular compartment. Such sequences are known to those skilled in the art (see, for example, Braun et al., EMBO J. 11 (1992), 3219-3227). The nucleic acid molecules can also be expressed in the organelles of the plant cells.

The transgenic plant cells can be regenerated by known techniques to give rise to entire plants. In principle, the transgenic plants may be plants of any desired plant species, i.e. not only monocotyledonous but also dicotyledonous plants.

Thus, transgenic plants can be obtained whose properties are altered by overexpression, suppression or inhibition of homologous (= natural) genes or gene sequences or expression of heterologous (= foreign) genes or gene sequences.

It is preferred to employ the compounds (I) according to the invention in transgenic crops which are resistant to growth regulators such as, for example, dicamba, or to herbicides which inhibit essential plant enzymes, for example acetolactate synthases (ALS), EPSP synthases, glutamine synthases (GS) or hydroxyphenylpyruvate dioxygenases (HPPD), or to herbicides from the group of the sulfonylureas, glyphosate, glufosinate or benzoylisoxazoles and analogous active compounds.

When the active compounds of the invention are employed in transgenic crops, not only do the effects toward harmful plants observed in other crops occur, but frequently also effects which are specific to application in the particular transgenic crop, for example an altered or specifically widened spectrum of weeds which can be controlled, altered application rates which can be used for the application, preferably good combinability with the herbicides to which the transgenic crop is resistant, and influencing of growth and yield of the transgenic crop plants.

The invention therefore also relates to the use of the compounds of the general formula (I) according to the invention and/or their salts as herbicides for controlling harmful plants in crops of useful plants or ornamentals, optionally in transgenic crop plants.

Preference is given to the use in cereals, here preferably corn, wheat, barley, rye, oats, millet or rice, by the pre- or post-emergence method.

Preference is also given to the use in soybeans by the pre- or post-emergence method.

The use according to the invention for the control of harmful plants or for growth regulation of plants also includes the case in which the active compound of the general formula (I) or its salt is not formed from a precursor substance ("prodrug") until after application on the plant, in the plant or in the soil. The invention also provides for the use of one or more compounds of the general formula (I) or salts thereof or of a composition according to the invention (as defined below) (in a method) for controlling harmful plants or for regulating the growth of plants which comprises applying an effective amount of one or more compounds of the general formula (I) or salts thereof onto the plants (harmful plants, if appropriate together with the useful plants), plant seeds, the soil in which or on which the plants grow or the area under cultivation.

The invention also provides a herbicidal and/or plant growth-regulating composition, characterized in that the composition comprises
(a) one or more compounds of the general formula (I) and/or salts thereof, as defined above, preferably in one of the embodiments identified as preferred or particularly preferred, in particular one or more compounds of the formulae (I-001) to (I-192) and/or salts thereof, in each case as defined above,
   and
(b) one or more further substances selected from groups (i) and/or (ii):
   (i) one or more further agrochemically active substances, preferably selected from the group consisting of insecticides, acaricides, nematicides, further herbicides (i.e. those not corresponding to the general formula (I) defined above), fungicides, safeners, fertilizers and/or further growth regulators,
   (ii) one or more formulation auxiliaries customary in crop protection.

Here, the further agrochemically active substances of component (i) of a composition according to the invention are preferably selected from the group of substances mentioned in "The Pesticide Manual", 16th edition, The British Crop Protection Council and the Royal Soc. of Chemistry, 2012.

A herbicidal or plant growth-regulating composition according to the invention comprises preferably one, two, three or more formulation auxiliaries (ii) customary in crop protection selected from the group consisting of surfactants, emulsifiers, dispersants, film-formers, thickeners, inorganic salts, dusting agents, carriers solid at 25 °C and 1013 mbar, preferably adsorbent granulated inert materials, wetting agents, antioxidants, stabilizers, buffer substances, antifoam agents, water, organic solvents, preferably organic solvents miscible with water in any ratio at 25 °C and 1013 mbar.

The compounds of general formula (I) according to the invention can be used in the form of wettable powders, emulsifiable concentrates, sprayable solutions, dusting products or granules in the customary formulations. The invention therefore also provides herbicidal and plant growth-regulating compositions which comprise compounds of the general formula (I) and/or salts thereof.

The compounds of the general formula (I) and/or salts thereof can be formulated in various ways according to which biological and/or physicochemical parameters are required. Possible formulations include, for example: wettable powders (WP), water-soluble powders (SP), water-soluble concentrates, emulsifiable concentrates (EC), emulsions (EW), such as oil-in-water and water-in-oil emulsions, sprayable solutions, suspension concentrates (SC), dispersions based on oil or water, oil-miscible solutions, capsule suspensions (CS), dusting products (DP), dressings, granules for scattering and soil application, granules (GR) in the form of microgranules, spray granules, absorption and adsorption granules, water-dispersible granules (WG), water-soluble granules (SG), ULV formulations, microcapsules and waxes.

These individual formulation types and the formulation assistants, such as inert materials, surfactants, solvents and further additives, are known to the person skilled in the art and are described, for example, in: Watkins, "Handbook of Insecticide Dust Diluents and Carriers", 2nd Ed., Darland Books, Caldwell N.J.; H.v. Olphen, "Introduction to Clay Colloid Chemistry", 2nd ed., J. Wiley & Sons, N.Y.; C. Marsden, "Solvents Guide", 2nd ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflachenaktive Äthylenoxidaddukte" [Interface-active Ethylene Oxide Adducts], Wiss. Verlagsgesellschaft, Stuttgart 1976; Winnacker-Küchler, "Chemische Technologie" [Chemical Technology], volume 7, C. Hanser Verlag Munich, 4th Ed. 1986.

Wettable powders are preparations which can be dispersed uniformly in water and, in addition to the active compound, apart from a diluent or inert substance, also comprise surfactants of the ionic and/or nonionic type (wetting agents, dispersants), for example polyoxyethylated alkylphenols, polyoxyethylated fatty alcohols, polyoxyethylated fatty amines, fatty alcohol polyglycol ether sulfates, alkanesulfonates, alkylbenzenesulfonates, sodium lignosulfonate, sodium 2,2'-dinaphthylmethane-6,6'-disulfonate, sodium dibutylnaphthalenesulfonate or else sodium oleoylmethyltaurate. To produce the wettable powders, the herbicidally active compounds are finely ground, for example in customary apparatuses such as hammer mills, blower mills and air-jet mills, and simultaneously or subsequently mixed with the formulation auxiliaries.

Emulsifiable concentrates are produced by dissolving the active compound in an organic solvent, for example butanol, cyclohexanone, dimethylformamide, xylene, or else relatively high-boiling aromatics or hydrocarbons or mixtures of the organic solvents, with addition of one or more ionic and/or nonionic surfactants (emulsifiers). Examples of emulsifiers which may be used are: calcium alkylarylsulfonates such as calcium dodecylbenzenesulfonate, or nonionic emulsifiers such as fatty acid polyglycol esters, alkylaryl polyglycol ethers, fatty alcohol polyglycol ethers, propylene oxide-ethylene oxide condensation products, alkyl polyethers, sorbitan esters, for example sorbitan fatty acid esters, or polyoxyethylene sorbitan esters, for example polyoxyethylene sorbitan fatty acid esters.

Dusting products are obtained by grinding the active compound with finely distributed solids, for example talc, natural clays, such as kaolin, bentonite and pyrophyllite, or diatomaceous earth. Suspension concentrates may be water- or oil-based. They may be prepared, for example, by wet-grinding by means of commercial bead mills and optional addition of surfactants as have, for example, already been listed above for the other formulation types.

Emulsions, for example oil-in-water emulsions (EW), can be produced, for example, by means of stirrers, colloid mills and/or static mixers using aqueous organic solvents and optionally surfactants as already listed above, for example, for the other formulation types.

Granules can be produced either by spraying the active compound onto adsorptive granular inert material or by applying active compound concentrates to the surface of carriers, such as sand, kaolinites or granular inert material, by means of adhesives, for example polyvinyl alcohol, sodium polyacrylate or else mineral oils. Suitable active compounds can also be granulated in the manner customary for the production of fertilizer granules - if desired as a mixture with fertilizers.

Water-dispersible granules are produced generally by the customary processes such as spray-drying, fluidized-bed granulation, pan granulation, mixing with high-speed mixers and extrusion without solid inert material.

For the production of pan, fluidized-bed, extruder and spray granules, see e.g. processes in "Spray Drying Handbook" 3rd Ed. 1979, G. Goodwin Ltd., London, J.E. Browning, "Agglomeration", Chemical and Engineering 1967, pages 147 ff; "Perry's Chemical Engineer's Handbook", 5th Ed., McGraw Hill, New York 1973, p. 8-57.

For further details regarding the formulation of crop protection compositions, see, for example, G.C. Klingman, "Weed Control as a Science", John Wiley and Sons, Inc., New York, 1961, pages 81-96 and J.D. Freyer, S.A. Evans, "Weed Control Handbook", 5th Ed., Blackwell Scientific Publications, Oxford, 1968, pages 101-103.

The agrochemical preparations, preferably herbicidal or plant growth-regulating compositions, of the present invention preferably comprise a total amount of from 0.1 to 99% by weight, preferably 0.5 to 95% by weight, particularly preferably 1 to 90% by weight, especially preferably 2 to 80% by weight, of active compounds of the general formula (I) and their salts.

In wettable powders, the active compound concentration is, for example, about 10 to 90% by weight, the remainder to 100% by weight consisting of customary formulation constituents. In emulsifiable concentrates, the active compound concentration may be about 1% to 90% and preferably 5% to 80% by weight. Formulations in the form of dusts comprise 1% to 30% by weight of active compound, preferably usually 5% to 20% by weight of active compound; sprayable solutions contain about 0.05% to 80% by weight, preferably 2% to 50% by weight of active compound. In the case of water-dispersible granules, the active compound content depends partially on whether the active compound is in liquid or solid form and on which granulation auxiliaries, fillers, etc., are used. In the water-dispersible granules, the content of active compound is, for example, between 1% and 95% by weight, preferably between 10% and 80% by weight.

In addition, the active compound formulations mentioned optionally comprise the respective customary stickers, wetters, dispersants, emulsifiers, penetrants, preservatives, antifreeze agents and solvents, fillers, carriers and dyes, defoamers, evaporation inhibitors and agents which influence the pH and the viscosity. Examples of formulation auxiliaries are described inter alia in "Chemistry and Technology of Agrochemical Formulations", ed. D.A. Knowles, Kluwer Academic Publishers (1998).

The compounds of the general formula (I) or salts thereof can be used as such or in the form of their preparations (formulations) in a combination with other pesticidally active substances, for example insecticides, acaricides, nematicides, herbicides, fungicides, safeners, fertilizers and/or growth regulators, for example in the form of a finished formulation or of a tank mix. The combination formulations can be prepared on the basis of the abovementioned formulations, while taking account of the physical properties and stabilities of the active compounds to be combined.

Active compounds which can be employed in combination with the compounds of general formula (I) according to the invention in mixture formulations or in a tank mix are, for example, known active compounds based on inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoendesaturase, photosystem I, photosystem II, protoporphyrinogen oxidase, as described, for example, in Weed Research 26 (1986) 441-445 or "The Pesticide Manual", 16th edition, The British Crop Protection Council and the Royal Soc. of Chemistry, 2012 and literature cited therein.

Of particular interest is the selective control of harmful plants in crops of useful plants and ornamentals. Although the compounds of general formula (I) according to the invention have already demonstrated very good to adequate selectivity in a large number of crops, in principle, in some crops and in particular also in the case of mixtures with other, less selective herbicides, phytotoxicities on the crop plants may occur. In this connection, combinations of compounds of general formula (I) according to the invention are of particular interest which comprise the compounds of general formula (I) or their combinations with other herbicides or pesticides and safeners. The safeners, which are used in an antidotically effective amount, reduce the phytotoxic side effects of the herbicides/pesticides employed, for example in economically important crops, such as cereals (wheat, barley, rye, corn, rice, millet), sugarbeet, sugarcane, oilseed rape, cotton and soybeans, preferably cereals.

The weight ratios of herbicide (mixture) to safener depend generally on the herbicide application rate and the efficacy of the safener in question and may vary within wide limits, for example in the range from 200:1 to 1:200, preferably 100:1 to 1:100, in particular 20: 1 to 1:20. Analogously to the compounds (I) or mixtures thereof, the safeners can be formulated with further herbicides/pesticides and be provided and employed as a finished formulation or tank mix with the herbicides.

For application, the herbicide or herbicide/safener formulations present in commercial form are, if appropriate, diluted in a customary manner, for example in the case of wettable powders, emulsifiable concentrates, dispersions and water-dispersible granules with water. Dust-type preparations, granules for soil application or granules for scattering and sprayable solutions are not normally diluted further with other inert substances prior to application.

The application rate of the compounds of the general formula (I) and/or their salts is affected to a certain extent by external conditions such as temperature, humidity, etc. Here, the application rate may vary within wide limits. For the application as a herbicide for controlling harmful plants, the total amount of compounds of the general formula (I) and/or their salts is preferably in the range from 0.001 to 10.0 kg/ha, with preference in the range from 0.005 to 5 kg/ha, more preferably in the range from 0.01 to 1.5 kg/ha, in particular preferably in the range from 0.05 to 1 kg/ha. This applies both to the pre-emergence and the post-emergence application.

When the compounds of the general formula (I) and/or their salts are used as plant growth regulator, for example as culm stabilizer for crop plants like those mentioned above, preferably cereal plants, such as wheat, barley, rye, triticale, millet, rice or corn, the total application rate is preferably in the range of from 0.001 to 2 kg/ha, preferably in the range of from 0.005 to 1 kg/ha, in particular in the range of from 10 to 500 g/ha, very particularly in the range from 20 to 250 g/ha. This applies both to the pre-emergence and the post-emergence application.

The application as culm stabilizer may take place at various stages of the growth of the plants. Preferred is, for example, the application after the tilling phase, at the beginning of the longitudinal growth.

As an alternative, application as plant growth regulator is also possible by treating the seed, which includes various techniques for dressing and coating seed. Here, the application rate depends on the particular techniques and can be determined in preliminary tests.

Active compounds which can be employed in combination with the compounds of the general formula (I) according to the invention in compositions according to the invention (for example in mixed formulations or in the tank mix) are, for example, known active compounds which are based on the inhibition of, for example, acetolactate synthase, acetyl-CoA carboxylase, cellulose synthase, enolpyruvylshikimate-3-phosphate synthase, glutamine synthetase, p-hydroxyphenylpyruvate dioxygenase, phytoene desaturase, photosystem I, photosystem II, protoporphyrinogen oxidase, as are described in, for example, Weed Research 26 (1986) 441-445 or "The Pesticide Manual", 16th edition, The British Crop Protection Council and the Royal Soc. of Chemistry, 2012 and the literature cited therein. Known herbicides or plant growth regulators which can be combined with the compounds according to the invention are, for example, the following active compounds, where the compounds are designated either with the "common name" in accordance with the International Organization for Standardization (ISO) or with the chemical name or with the code number. They always encompass all of the application forms such as, for example, acids, salts, esters and also all isomeric forms such as stereoisomers and optical isomers, even if not explicitly mentioned. Examples of such herbicidal mixing partners are:
acetochlor, acifluorfen, acifluorfen-methyl, acifluorfen-sodium, aclonifen, alachlor, allidochlor, alloxydim, alloxydim-sodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlor-potassium, aminocyclopyrachlor-methyl, aminopyralid, aminopyralid-dimethylammonium, aminopyralid-tripromine, amitrole, ammoniumsulfamate, anilofos, asulam, asulam-potassium, asulam sodium, atrazine, azafenidin, azimsulfuron, beflubutamid, (S)-(-)-beflubutamid, beflubutamid-M, benazolin, benazolin-ethyl, benazolin-dimethylammonium, benazolin-potassium, benfluralin, benfuresate, bensulfuron, bensulfuron-methyl, bensulide, bentazone, bentazone-sodium, benzobicyclon, benzofenap, bicyclopyrone, bifenox, bilanafos, bilanafos-sodium, bipyrazone, bispyribac, bispyribac-sodium, bixlozone, bromacil, bromacil-lithium, bromacil-sodium, bromobutide, bromofenoxim, bromoxynil, bromoxynil-butyrate, -potassium, -heptanoate und -octanoate, busoxinone, butachlor, butafenacil, butamifos, butenachlor, butralin, butroxydim, butylate, cafenstrole, cambendichlor, carbetamide, carfentrazone, carfentrazone-ethyl, chloramben, chloramben-ammonium, chloramben-diolamine, chlroamben-methyl, chloramben-methylammonium, chloramben-sodium, chlorbromuron, chlorfenac, chlorfenac-ammonium, chlorfenac-sodium, chlorfenprop, chlorfenprop-methyl, chlorflurenol, chlorflurenol-methyl, chloridazon, chlorimuron, chlorimuron-ethyl, chlorophthalim, chlorotoluron, chlorsulfuron, chlorthal, chlorthal-dimethyl, chlorthal-monomethyl, cinidon, cinidon-ethyl, cinmethylin, exo-(+)-cinmethylin, i.e. (1R,2S,4S)-4-isopropyl-1-methyl-2-[(2-methylbenzyl)oxy]-7-oxabicyclo[2.2.1]heptane, exo-(-)-cinmethylin, i.e. (1R,2S,4S)-4-isopropyl-1-methyl-2-[(2-methylbenzyl)oxy]-7-oxabicyclo[2.2.1]heptane, cinosulfuron, clacyfos, clethodim, clodinafop, clodinafop-ethyl, clodinafop-propargyl, clomazone, clomeprop, clopyralid, clopyralid-methyl, clopyralid-olamine, clopyralid-potassium, clopyralid-tripomine, cloransulam, cloransulam-methyl, cumyluron, cyanamide, cyanazine, cycloate, cyclopyranil, cyclopyrimorate, cyclosulfamuron, cycloxydim, cyhalofop, cyhalofop-butyl, cyprazine, 2,4-D (including thea mmonium, butotyl, -butyl, choline, diethylammonium, -dimethylammonium, -diolamine, -doboxyl, -dodecylammonium, etexyl, ethyl, 2-ethylhexyl, heptylammonium, isobutyl, isooctyl, isopropyl, isopropylammonium, lithium, meptyl, methyl, potassium, tetradecylammonium, triethylammonium, triisopropanolammonium, tripromine and trolamine salt thereof), 2,4-DB, 2,4-DB-butyl, -dimethylammonium, isooctyl, - potassium und -sodium, daimuron (dymron), dalapon, dalapon-calcium, dalapon-magnesium, dalapon-sodium, dazomet, dazomet-sodium, n-decanol, 7-deoxy-D-sedoheptulose, desmedipham, detosyl-pyrazolate (DTP), dicamba and its salts, e. g. dicamba-biproamine, dicamba-N,N-Bis(3-aminopropyl)methylamine, dicamba-butotyl, dicamba-choline, dicamba-diglycolamine, dicamba-dimethylammonium, dicamba-diethanolamine ammonium, dicamba-diethylammonium, dicamba-isopropylammonium, dicamba-methyl, dicamba-monoethanolamine, dicamba-olamine, dicamba-potassium, dicamba-sodium, dicamba-triethanolamine, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-butotyl, dichlorprop-dimethylammonium, dichhlorprop-etexyl, dichlorprop-ethylammonium, dichlorprop-isoctyl, dichlorprop-methyl, dichlorprop-potassium, dichlorprop-sodium, dichlorprop-P, dichlorprop-P-dimethylammonium, dichlorprop-P-etexyl, dichlorprop-P-potassium, dichlorprop-sodium, diclofop, diclofop-methyl, diclofop-P, diclofop-P-methyl, diclosulam, difenzoquat, difenzoquat-metilsulfate, diflufenican, diflufenzopyr, diflufenzopyr-sodium, dimefuron, dimepiperate, dimesulfazet, dimethachlor, dimethametryn, dimethenamid, dimethenamid-P, dimetrasulfuron, dinitramine, dinoterb, dinoterb-acetate, diphenamid, diquat, diquat-dibromid, diquat-dichloride, dithiopyr, diuron, DNOC, DNOC-ammonium, DNOC-potassium, DNOC-sodium, endothal, endothal-diammonium, endothal-dipotassium, endothal-disodium, Epyrifenacil (S-3100), EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-5231, i.e. N-[2-Chlor-4-fluor-5-[4-(3-fluorpropyl)-4,5-dihydro-5-oxo-1H-tetrazol-1-yl]-phenyl]-ethansulfonamid, F-7967, i.e. 3-[7-Chlor-5-fluor-2-(trifluormethyl)-1H-benzimidazol-4-yl]-1-methyl-6-(trifluormethyl)pyrimidin-2,4(1H,3H)-dione, fenoxaprop, fenoxaprop-P, fenoxaprop-ethyl, fenoxaprop-P-ethyl, fenoxasulfone, fenpyrazone, fenquinotrione, fentrazamide, flamprop, flamprop-isoproyl, flamprop-methyl, flamprop-M-isopropyl, flamprop-M-methyl, flazasulfuron, florasulam, florpyrauxifen, florpyrauxifen-benzyl, fluazifop, fluazifop-butyl, fluazifop-methyl, fluazifop-P, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium und -methyl, fluoroglycofen, fluoroglycofen-ethyl, flupropanate, flupropanate-sdium, flupyrsulfuron, flupyrsulfuron-methyl, flupyrsulfuron-methyl-sodium, fluridone, flurochloridone, fluroxypyr, fluroxypyr-butometyl, fluroxypyr-meptyl, flurtamone, fluthiacet, fluthiacet-methyl, fomesafen, fomesafen-sodium, foramsulfuron, foramsulfuron sodium salt, fosamine, fosamine-ammonium, glufosinate, glufosinate-ammonium, glufosinate-sodium, L-glufosinate-ammonium, L-glufosiante-sodium, glufosinate-P-sodium, glufosinate-P-ammonium, glyphosate, glyphosate-ammonium, -isopropylammonium, -diammonium, -dimethylammonium, -potassium, -sodium, sesquisodium and -trimesium, H-9201, i.e. O-(2,4-Dimethyl-6-nitrophenyl)-O-ethyl-isopropylphosphoramidothioat, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, haloxifop-sodium, hexazinone, HNPC-A8169, i.e. prop-2-yn-1-yl (2S)-2-{3-[(5-tert-butylpyridin-2-yl)oxy]phenoxy}propanoate, HW-02, i.e. 1-(Dimethoxyphosphoryl)-ethyl-(2,4-dichlorphenoxy)acetat, hydantocidin, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazaquin.methyl, imazethapyr, imazethapyr-immonium, imazosulfuron, indanofan, indaziflam, iodosulfuron, iodosulfuron-methyl, iodosulfuron-methyl-sodium, ioxynil, ioxynil-lithium, -octanoate, - potassium und sodium, ipfencarbazone, isoproturon, isouron, isoxaben, isoxaflutole, karbutilate, KUH-043, i.e. 3-({[5-(Difluormethyl)-1-methyl-3-(trifluormethyl)-1H-pyrazol-4-yl]methyl}sulfonyl)-5,5-dimethyl-4,5-dihydro-1,2-oxazol, ketospiradox, ketospiradox-potassium, lactofen, lancotrione, lenacil, linuron, MCPA, MCPA-butotyl, -butyl, -dimethylammonium, -diolamine, -2-ethylhexyl, -ethyl, - isobutyl, isoctyl, -isopropyl, -isopropylammonium, -methyl, olamine, -potassium, -sodium and - trolamine, MCPB, MCPB-methyl, -ethyl und -sodium, mecoprop, mecoprop-butotyl, mecoprop-demethylammonium, mecoprop-diolamine, mecoprop-etexyl, mecoprop-ethadyl, mecoprop-isoctyl, mecoprop-methyl, mecoprop-potassium, mecoprop-sodium, and mecoprop-trolamine, mecoprop-P, mecoprop-P-butotyl, -dimethylammonium, -2-ethylhexyl and -potassium, mefenacet, mefluidide, mefluidide-diolamine, mefluidide-potassium, mesosulfuron, mesosulfuron-methyl, mesosulfuron sodium salt, mesotrione, methabenzthiazuron, metam, metamifop, metamitron, metazachlor, metazo-sulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinate, monolinuron, monosulfuron, monosulfuron-methyl, MT-5950, i.e. N-[3-chlor-4-(1-methylethyl)-phenyl]-2-methylpentanamid, NGGC-011, napropamide, NC-310, i.e. 4-(2,4-Dichlorbenzoyl)-1-methyl-5-benzyloxypyrazol, NC-656, i.e. 3-[(isopropylsulfonyl)methyl]-N-(5-methyl-1,3,4-oxadiazol-2-yl)-5-(trifluoromethyl)[1,2,4]triazolo[4,3-a]pyridine-8-carboxamide, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefone, oxyfluorfen, paraquat, paraquat-dichloride, paraquat-dimethylsulfate, pebulate, pendimethalin, penoxsulam, pentachlorphenol, pentoxazone, pethoxamid, petroleum oils, phenmedipham, phenmedipham-ethyl, picloram, picloram-dimethylammonium, picloram-etexyl, picloram-isoctyl, picloram-methyl, picloram-olamine, picloram-potassium, picloram-triethylammonium, picloram-tripromine, picloram-trolamine, picolinafen, pinoxaden, piperophos, pretilachlor, primisulfuron, primisulfuron-methyl, prodiamine, profoxydim, prometon, prometryn, propachlor, propanil, propaquizafop, propazine, propham, propisochlor, propoxycarbazone, propoxycarbazone-sodium, propyrisulfuron, propyzamide, prosulfocarb, prosulfuron, pyraclonil, pyraflufen, pyraflufen-ethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinclorac-dimethylammonium, quinclorac-methyl, quinmerac, quinoclamine, quizalofop, quizalofop-ethyl, quizalofop-P, quizalofop-P-ethyl, quizalofop-P-tefuryl, QYM201, i.e. 1-{2-chloro-3-[(3-cyclopropyl-5-hydroxy-1-methyl-1H-pyrazol-4-yl)carbonyl]-6-(trifluoromethyl)phenyl}piperidin-2-one, rimsulfuron, saflufenacil, sethoxydim, siduron, simazine, simetryn, SL-261, sulcotrione, sulfentrazone, sulfometuron, sulfometuron-methyl, sulfosulfuron, , SYP-249, i.e. 1-Ethoxy-3-methyl-1-oxobut-3-en-2-yl-5-[2-chlor-4-(trifluormethyl)phenoxy]-2-nitrobenzoat, SYP-300, i.e. 1-[7-Fluor-3-oxo-4-(prop-2-in-1-yl)-3,4-dihydro-2H-1,4-benzoxazin-6-yl]-3-propyl-2-thioxoimidazolidin-4,5-dion, 2,3,6-TBA, TCA (trichloro acetic acid) and its salts, e.g. TCA-ammonium, TCA-calcium, TCA-ethyl, TCA-magnesium, TCA-sodium, tebuthiuron, tefuryltrione, tembotrione, tepraloxydim, terbacil, terbucarb, terbumeton, terbuthylazine, terbutryn, tetflupyrolimet, thaxtomin, thenylchlor, thiazopyr, thiencarbazone, thiencarbazone-methyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, triclopyr-butotyl, triclopyr-choline, triclopyr-ethyl, triclopyr-triethylammonium, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-Dichlor-N-{2-[(4,6-dimethoxypyrimidin-2-yl)oxy]benzyl}anilin, 3-(2-chloro-4-fluoro-5-(3-methyl-2,6-dioxo-4-trifluoromethyl-3,6-dihydropyrimidin-1 (2H)-yl)phenyl)-5-methyl-4,5-dihydroisoxazole-5-carboxylic acid ethyl ester, ethyl-[(3-{2-chlor-4-fluor-5-[3-methyl-2,6-dioxo-4-(trifluormethyl)-3,6-dihydropyrimidin-1(2H)-yl]phenoxy}pyridin-2-yl)oxy]acetate, 3-chloro-2-[3-(difluoromethyl)isoxazolyl-5-yl]phenyl-5-chloropyrimidin-2-yl ether, 2-(3,4-dimethoxyphenyl)-4-[(2-hydroxy-6-oxocyclohex-1-en-1-yl)carbonyl]-6-methylpyridazine-3(2H)-one, 2-({2-[(2-methoxyethoxy)methyl]-6-methylpyridin-3-yl}carbonyl)cyclohexane-1,3-dione, (5-hydroxy-1-methyl-1H-pyrazol-4-yl)(3,3,4-trimethyl-1,1-dioxido-2,3-dihydro-1-benzothiophen-5-yl)methanone, 1-methyl-4-[(3,3,4-trimethyl-1,1-dioxido-2,3-dihydro-1-benzothiophen-5-yl)carbonyl]-1H-pyrazol-5-yl propane-1-sulfonate, 4-{2-chloro-3-[(3,5-dimethyl-1H-pyrazol-1-yl)methyl]-4-(methylsulfonyl)benzoyl}-1-methyl-1H-pyrazol-5-yl-1,3-dimethyl-1H-pyrazole-4-carboxylate; cyanomethyl 4-amino-3-chloro-5-fluoro-6-(7-fluoro-1H-indol-6-yl)pyridine-2-carboxylate, prop-2-yn-1-yl 4-amino-3-chloro-5-fluoro-6-(7-fluoro-1H-indol-6-yl)pyridine-2-carboxylate, methyl 4-amino-3-chloro-5-fluoro-6-(7-fluoro-1H-indol-6-yl)pyridine-2-carboxylate, 4-amino-3-chloro-3-fluoro-6-(7-fluoro-1H-indol-6-yl)pyridine-2-carboxylic acid, benzyl 4-amino-3-chloro-5-fluoro-6-(7-fluoro-1H-indol-6-yl)pyridine-2-carboxylate, ethyl 4-amino-3-chloro-5-fluoro-6-(7-fluoro-1H-indol-6-yl)pyridine-2-carboxylate, methyl 4-amino-3-chloro-5-fluoro-6-(7-fluoro-1-isobutyryl-1H-indol-6-yl)pyridine-2-carboxylate, methyl 6-(1-acetyl-7-fluoro-1H-indol-6-yl)-4-amino-3-chloro-5-fluoropyridine-2-carboxylate, methyl 4-amino-3-chloro-6-[1-(2,2-dimethylpropanoyl)-7-fluoro-1H-indol-6-yl]-5-fluoropyridine-2-carboxylate, methyl 4-amino-3-chloro-5-fluoro-6-[7-fluoro-1-(methoxyacetyl)-1H-indol-6-yl]pyridine-2-carboxylate, potassium 4-amino-3-chloro-5-fluoro-6-(7-fluoro-1H-indol-6-yl)pyridine-2-carboxylate, sodium 4-amino-3-chloro-5-fluoro-6-(7-fluoro-1H-indol-6-yl)pyridine-2-carboxylate, butyl 4-amino-3-chloro-5-fluoro-6-(7-fluoro-1H-indol-6-yl)pyridine-2-carboxylate, 4-hydroxy-1-methyl-3-[4-(trifluoromethyl)pyridin-2-yl]imidazolidin-2-one, 3-(5-tert-butyl-1,2-oxazol-3-yl)-4-hydroxy-1-methylimidazolidin-2-one, 3-[5-chloro-4-(trifluormethyl)pyridin-2-yl]-4-hydroxy-1-methylimidazolidin-2-one, 4-hydroxy-1-methoxy-5-methyl-3-[4-(trifluormethyl)pyridin-2-yl]imidazolidin-2-one, 6-[(2-hydroxy-6-oxocyclohex-1-en-1-yl)carbonyl]-1,5-dimethyl-3-(2-methylphenyl)quinazolin-2,4(1H,3H)-dione, 3-(2,6-dimethylphenyl)-6-[(2-hydroxy-6-oxocyclohex-1-en-1-yl)carbonyl]-1-methylquinazolin-2,4(1H,3H)-dione, 2-[2-chloro-4-(methylsulfonyl)-3-(morpholin-4-ylmethyl)benzoyl]-3-hydroxycyclohex-2-en-1-one, 1-(2-carboxyethyl)-4-(pyrimidin-2-yl)pyridazin-1-ium salt (with anions such as chloride, acetate or trifluoroacetate), 1-(2-carboxyethyl)-4-(pyridazin-3-yl)pyridazin-1-ium salt (with anions such as chloride, acetate or trifluoroacetate), 4-(pyrimidin-2-yl)-1-(2-sulfoethyl)pyridazin-1-ium salt (with anions such as chloride, acetate or trifluoroacetate), 4-(pyridazin-3-yl)-1-(2-sulfoethyl)pyridazin-1-ium salt (with anions such as chloride, acetate or trifluoroacetate), 1-(2-Carboxyethyl)-4-(1,3-thiazol-2-yl)pyridazin-1-ium salt (with anions such as chloride, acetate or trifluoroacetate), 1-(2-Carboxyethyl)-4-(1,3-thiazol-2-yl)pyridazin-1-ium salt (with anions such as chloride, acetate or trifluoroacetate), methyl (2R)-2-{ [(E)-({2-chloro-4-fluoro-5-[3-methyl-2,6-dioxo-4-(trifluoromethyl)-3,6-dihydropyrimidin-1(2H)-yl]phenyl}methylidene)amino]oxy}propanoate, (E)-2-(trifluoromethyl)benzaldehyde O-{2,6-bis[(4,6-dimethoxypyrimidin-2-yl)oxy]benzoyl}oxime, 2-fluoro-N-(5-methyl-1,3,4-oxadiazol-2-yl)-3-[(R)-propylsulfinyl]-4-(trifluoromethyl)benzamide, (2R)-2-[(4-amino-3,5-dichloro-6-fluoro-2-pyridyl)oxy]propanoic acid.

Examples of plant growth regulators as possible mixing partners are: Abscisic acid and related analogs [e.g. (2Z,4E)-5-[6-Ethynyl-1-hydroxy-2,6-dimethyl-4-oxocyclohex-2-en-1-yl]-3-methylpenta-2,4-dienoic acid, methyl-(2Z,4E)-5-[6-ethynyl-1-hydroxy-2,6-dimethyl-4-oxocyclohex-2-en-1-yl]-3-methylpenta-2,4-dienoate, (2Z,4E)-3-ethyl-5-(1-hydroxy-2,6,6-trimethyl-4-oxocyclohex-2-en-1-yl)penta-2,4-dienoic acid, (2E,4E)-5-(1-hydroxy-2,6,6-trimethyl-4-oxocyclohex-2-en-1-yl)-3-(trifluoromethyl)penta-2,4-dienoic acid, methyl (2E,4E)-5-(1-hydroxy-2,6,6-trimethyl-4-oxocyclohex-2-en-1-yl)-3-(trifluoromethyl)penta-2,4-dienoate, (2Z,4E)-5-(2-hydroxy-1,3-dimethyl-5-oxobicyclo[4.1.0]hept-3-en-2-yl)-3-methylpenta-2,4-dienoic acid], acibenzolar, acibenzolar-S-methyl, S-adenosylhomocysteine, allantoin, 2-Aminoethoxyvinylglycine (AVG), aminooxyacetic acid and related esters [e.g. (Isopropylidene)-aminooxyacetic acid-2-(methoxy)-2-oxoethylester, (Isopropylidene)-aminooxyacetic acid-2-(hexyloxy)-2-oxoethylester, (Cyclohexylidene)-aminooxyacetic acid-2-(isopropyloxy)-2-oxoethylester], 1-aminocycloprop-1-yl carboxylic acid and derivatives thereof, e.g. disclosed in DE3335514, EP30287, DE2906507 or US5123951, 5-aminolevulinic acid, ancymidol, 6-benzylaminopurine, bikinin, brassinolide, brassinolide-ethyl, L-canaline, catechin and catechines (e.g. (2S,3R)-2-(3,4-Dihydroxyphenyl)-3,4-dihydro-2H-chromen-3,5,7-triol), chitooligosaccharides (CO; COs differ from LCOs in that they lack the pendant fatty acid chain that is characteristic of LCOs. COs, sometimes referred to as N-acetylchitooligosaccharides, are also composed of GlcNAc residues but have side chain decorations that make them different from chitin molecules [(C₈H₁₃NO₅)ₙ, CAS No. 1398-61-4] and chitosan molecules [(C₅H₁₁NO₄)ₙ, CAS No. 9012-76-4]), chitinous compounds, chlormequat chloride, cloprop, cyclanilide, 3-(Cycloprop-1-enyl)propionic acid, 1-[2-(4-cyano-3,5-dicyclopropylphenyl)acetamido]cyclohexanecarboxylic acid, 1-[2-(4-cyano-3-cyclopropylphenyl)acetamido]cyclohexanecarboxylic acid, daminozide, dazomet, dazomet-sodium, n-decanol, dikegulac, dikegulac-sodium, endothal, endothal-dipotassium, -disodium, and mono(N,N-dimethylalkylammonium), ethephon, flumetralin, flurenol, flurenol-butyl, flurenol-methyl, flurprimidol, forchlorfenuron, gibberellic acid, inabenfide, indol-3-acetic acid (IAA), 4-indol-3-ylbutyric acid, isoprothiolane, probenazole, jasmonic acid, Jasmonic acid or derivatives thereof (e.g. jasmonic acid methyl ester, jasmonic acid ethyl ester), lipo-chitooligosaccharides (LCO, sometimes referred to as symbiotic nodulation (Nod) signals (or Nod factors) or as Myc factors, consist of an oligosaccharide backbone of β-1,4-linked *N*-acetyl-D-glucosamine ("GlcNAc") residues with an N-linked fatty acyl chain condensed at the non-reducing end. As understood in the art, LCOs differ in the number of GlcNAc residues in the backbone, in the length and degree of saturation of the fatty acyl chain and in the substitutions of reducing and non-reducing sugar residues), linoleic acid or derivatives thereof, linolenic acid or derivatives thereof, maleic hydrazide, mepiquat chloride, mepiquat pentaborate, 1-methylcyclopropene, 3-methylcyclopropene, 1-ethylcyclopropene, 1-n-propylcyclopropene, 1-cyclopropenylmethanol, methoxyvinylglycin (MVG), 3'-methyl abscisic acid, 1-(4-methylphenyl)-N-(2-oxo-1-propyl-1,2,3,4-tetrahydroquinolin-6-yl)methanesulfonamide and related substituted tetrahydroquinolin-6-yl)methanesulfonamides, (3E,3aR,8bS)-3-({ [(2R)-4-Methyl-5-oxo-2,5-dihydrofuran-2-yl]oxy}methylen)-3,3a,4,8b-tetrahydro-2H-indeno[1,2-b]furan-2-one and related lactones as outlined in EP2248421, 2-(1-naphthyl)acetamide, 1-naphthylacetic acid, 2-naphthyloxyacetic acid, nitrophenolate-mixture, 4-Oxo-4[(2-phenylethyl)amino]butyric acid, paclobutrazol, 4-phenylbutyric acid and its related salts (e.g. sodium-4-phenylbutanoate, potassium-4-phenylbutanoate), phenylalanine, N-phenylphthalamic acid, prohexadione, prohexadione-calcium, putrescine, prohydrojasmon, rhizobitoxin, salicylic acid, salicylic acid methyl ester, sarcosine, sodium cycloprop-1-en-1-yl acetate, sodium cycloprop-2-en-1-yl acetate, sodium-3-(cycloprop-2-en-1-yl)propanoate, sodium-3-(cycloprop-1-en-1-yl) propanoate, sidefungin, spermidine, spermine, strigolactone, tecnazene, thidiazuron, triacontanol, trinexapac, trinexapac-ethyl, tryptophan, tsitodef, uniconazole, uniconazole-P, 2-fluoro-N-(3-methoxyphenyl)-*9H*-purin-6-amine.

Suitable combination partners for the compounds of the general formula (I) according to the invention also include, for example, the following safeners:
S1) Compounds from the group of heterocyclic carboxylic acid derivatives (formula S1): 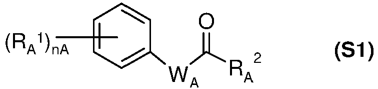 wherein symbols and indices are defined as follows:
   - n_{A}: is an integer value in the range of 0 to 5, preferably 0 to 3;
   - R_{A}¹: is halogen, (C₁-C₄)-alkyl, (C₁-C₄)-alkoxy, nitro or (C₁-C₄)-haloalkyl;

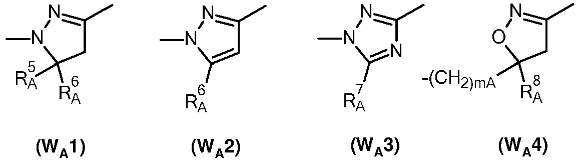

   W_{A} is an unsubstituted or substituted divalent heterocyclic moiety selected from the group of partially unsaturated or aromatic five-membered heterocycles carrying 1 to 3 hetero ring atoms selected from the group of nitrogen (N) und oxygen (O), and carrying at least one N-atom and not more than one O-atom in the ring, preferably a five-membered heterocyclic moiety selected from the group (W_{A}¹) to (W_{A}⁴),
   m_{A} is 0 or 1;
   R_{A}² is OR_{A}³, SR_{A}³ or NR_{A}³R_{A}⁴ or a saturated or unsaturated 3- to 7-membered heterocycle containing at least one N-atom and up to 3 heteroatoms, preferably combined with other heteroatoms from the group of O (oxygen) and S (sulfur), and which is linked to the carbonyl group in (S1) via a nitrogen atom, and which is unsubstituted or substituted by moieties selected from the group of (C₁-C₄)-alkyl, (C₁-C₄)-alkoxy or possibly substituted phenyl, preferably OR_{A}³, NHR_{A}⁴ oder N(CH₃)₂, particularly OR_{A}³
   R_{A}³ is hydrogen or an unsubstituted or substituted aliphatic hydrocarbon moiety, preferably containing 1 to 18 C-atoms;
   R_{A}⁴ is hydrogen, (C₁-C₆)-alkyl, (C₁-C₆)-alkoxy or substituted or unsubstituted phenyl;
   R_{A}⁵ is hydrogen, (C₁-C₈)-alkyl, (C₁-C₈)-haloalkyl, (C₁-C₄)-alkoxy(C₁-C₈)-alkyl, cyano or COOR_{A}⁹, wherein R_{A}⁹ is hydrogen, (C₁-C₈)-alkyl, (C₁-C₈)-haloalkyl, (C₁-C₄)-alkoxy-(C₁-C₄)-alkyl, (C₁-C₆)-hydroxyalkyl, (C₃-C₁₂)-cycloalkyl or tris-(C₁-C₄)-alkylsilyl;
   R_{A}⁶, R_{A}⁷, R_{A}⁸ are independently hydrogen, (C₁-C₈)-alkyl, (C₁-C₈)-haloalkyl, (C₃-C₁₂)-cycloalkyl or substituted or unsubstituted phenyl;
S1^{a}) Compounds of the dichlorophenylpyrazoline-3-carboxylic acid type (S1^{a}), preferably compounds such as
   1-(2,4-dichlorophenyl)-5-(ethoxycarbonyl)-5-methyl-2-pyrazoline-3-carboxylic acid, ethyl 1-(2,4-dichlorophenyl)-5-(ethoxycarbonyl)-5-methyl-2-pyrazoline-3-carboxylate (S1-1) ("mefenpyr-diethyl"), and related compounds as described in WO-A-91/07874;
S1^{b}) Derivatives of dichlorophenylpyrazolecarboxylic acid (S1^{b}), preferably compounds such as ethyl 1-(2,4-dichlorophenyl)-5-methylpyrazole-3-carboxylate (S1-2), ethyl 1-(2,4-dichlorophenyl)-5-isopropylpyrazole-3-carboxylate (S1-3), ethyl 1-(2,4-dichlorophenyl)-5-(1,1-dimethylethyl)pyrazole-3-carboxylate (S1-4) and related compounds as described in EP-A-333131 131 and EP-A-269806;
S1^{c}) Derivatives of 1,5-diphenylpyrazole-3-carboxylic acid (S1^{c}), preferably compounds such as ethyl 1-(2,4-dichlorophenyl)-5-phenylpyrazole-3-carboxylate (S1-5), methyl 1-(2-chlorophenyl)-5-phenylpyrazole-3-carboxylate (S1-6) and related compounds as described, for example, in EP-A-268554;
S1^{d}) Compounds of the triazolecarboxylic acid type (S1^{d}), preferably compounds such as fenchlorazole (ethyl ester), i.e. ethyl 1-(2,4-dichlorophenyl)-5-trichloromethyl-1H-1,2,4-triazole-3-carboxylate (S1-7), and related compounds, as described in EP-A-174562 and EP-A-346620;
S1^{e}) Compounds of the 5-benzyl- or 5-phenyl-2-isoxazoline-3-carboxylic acid or of the 5,5-diphenyl-2-isoxazoline-3-carboxylic acid type (S1^{e}), preferably compounds such as ethyl 5-(2,4-dichlorobenzyl)-2-isoxazoline-3-carboxylate (S1-8) or ethyl 5-phenyl-2-isoxazoline-3-carboxylate (S1-9) and related compounds as described in WO-A-91/08202, or 5,5-diphenyl-2-isoxazolinecarboxylic acid (S1-10) or ethyl 5,5-diphenyl-2-isoxazoline-3-carboxylate (S1-11) ("isoxadifen-ethyl") or n-propyl 5,5-diphenyl-2-isoxazoline-3-carboxylate (S1-12) or ethyl 5-(4-fluorophenyl)-5-phenyl-2-isoxazoline-3-carboxylate (S1-13), as described in patent application WO-A-95/07897.
S2) Compounds from the group of the 8-quinolinoxy derivatives (S2):
S2^{a}) Compounds of the 8-quinolinoxyacetic acid type (S2^{a}), preferably 1-methylhexyl (5-chloro-8-quinolinoxy)acetate ("cloquintocet-mexyl") (S2-1), 1,3-dimethylbut-1-yl (5-chloro-8-quinolinoxy)acetate (S2-2), 4-allyloxybutyl (5-chloro-8-quinolinoxy)acetate (S2-3), 1-allyloxyprop-2-yl (5-chloro-8-quinolinoxy)acetate (S2-4), ethyl (5-chloro-8-quinolinoxy)acetate (S2-5), methyl 5-chloro-8-quinolinoxyacetate (S2-6), allyl (5-chloro-8-quinolinoxy)acetate (S2-7), 2-(2-propylideneiminoxy)-1-ethyl (5-chloro-8-quinolinoxy)acetate (S2-8), 2-oxoprop-1-yl (5-chloro-8-quinolinoxy)acetate (S2-9) and related compounds, as described in EP-A-86750, EP-A-94349 and EP-A-191736 or EP-A-0 492 366, and also (5-chloro-8-quinolinoxy)acetic acid (S2-10), hydrates and salts thereof, for example the lithium, sodium, potassium, calcium, magnesium, aluminum, iron, ammonium, quaternary ammonium, sulfonium or phosphonium salts thereof, as described in WO-A-2002/34048;
S2^{b}) Compounds of the (5-chloro-8-quinolinoxy)malonic acid type (S2^{b}), preferably compounds such as diethyl (5-chloro-8-quinolinoxy)malonate, diallyl (5-chloro-8-quinolinoxy)malonate, methyl ethyl (5-chloro-8-quinolinoxy)malonate and related compounds, as described in EP-A-0 582 198.
S3) Active compounds of the dichloroacetamide type (S3), which are frequently used as pre-emergence safeners (soil-acting safeners), for example
   "dichlormid" (N,N-diallyl-2,2-dichloroacetamide) (S3-1),
   "R-29148" (3-dichloroacetyl-2,2,5-trimethyl-1,3-oxazolidine) from Stauffer (S3-2),
   "R-28725" (3-dichloroacetyl-2,2-dimethyl-1,3-oxazolidine) from Stauffer (S3-3),
   "benoxacor" (4-dichloroacetyl-3,4-dihydro-3-methyl-2H-1,4-benzoxazine) (S3-4),
   "PPG-1292" (N-allyl-N-[(1,3-dioxolan-2-yl)methyl]dichloroacetamide) from PPG Industries (S3-5),
   "DKA-24" (N-allyl-N-[(allylaminocarbonyl)methyl]dichloroacetamide) from Sagro-Chem (S3-6),
   "AD-67" or "MON 4660" (3-dichloroacetyl-1-oxa-3-azaspiro[4.5]decane) from Nitrokemia or Monsanto (S3-7),
   "TI-35" (1-dichloroacetylazepane) from TRI-Chemical RT (S3-8),
   "Diclonon" (Dicyclonon) or "BAS145138" or "LAB145138" (S3-9)
   ((RS)-1-dichloroacetyl-3,3,8a-trimethylperhydropyrrolo[1,2-a]pyrimidin-6-one) from BASF, "furilazole" or "MON 13900" ((RS)-3-dichloroacetyl-5-(2-furyl)-2,2-dimethyloxazolidine) (S3-10), and the (R) isomer thereof (S3-11).
S4) Compounds from the class of the acylsulfonamides (S4):
S4^{a}) N-Acylsulfonamides of the formula (S4^{a}) and salts thereof, as described in WO-A-97/45016, 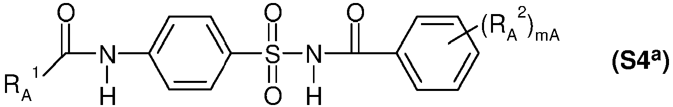 in which
   R_{A}¹ is (C₁-C₆)-alkyl, (C₃-C₆)-cycloalkyl, where the 2 latter radicals are substituted by v_{A} substituents from the group of halogen, (C₁-C₄)-alkoxy, (C₁-C₆)-haloalkoxy and (C₁-C₄)-alkylthio and, in the case of cyclic radicals, also by (C₁-C₄)-alkyl and (C₁-C₄)-haloalkyl;
   R_{A}² is halogen, (C₁-C₄)-alkyl, (C₁-C₄)-alkoxy, CF₃;
   m_{A} is 1 or 2;
   v_{A} is 0, 1, 2 or 3;
S4^{b}) Compounds of the 4-(benzoylsulfamoyl)benzamide type of the formula (S4^{b}) and salts thereof, as described in WO-A-99/16744, 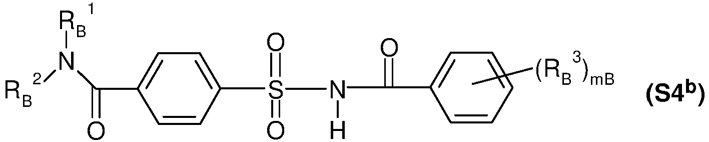 in which
   R_{B}¹, R_{B}² are independently hydrogen, (C₁-C₆)-alkyl, (C₃-C₆)-cycloalkyl, (C₃-C₆)-alkenyl, (C₃-C₆)-alkynyl,
   R_{B}³ is halogen, (C₁-C₄)-alkyl, (C₁-C₄)-haloalkyl or (C₁-C₄)-alkoxy and
   m_{B} is 1 or 2,
   for example those in which
   R_{B}¹ = cyclopropyl, R_{B}² = hydrogen and (R_{B}³) = 2-OMe ("cyprosulfamide", S4-1),
   R_{B}¹ = cyclopropyl, R_{B}² = hydrogen and (R_{B}³) = 5-Cl-2-OMe (S4-2),
   R_{B}¹ = ethyl, R_{B}² = hydrogen and (R_{B}³) = 2-OMe (S4-3),
   R_{B}¹ = isopropyl, R_{B}² = hydrogen and (R_{B}³) = 5-Cl-2-OMe (S4-4) and
   R_{B}¹ = isopropyl, R_{B}² = hydrogen and (R_{B}³) = 2-OMe (S4-5);
S4^{c}) Compounds from the class of the benzoylsulfamoylphenylureas of the formula (S4^{c}), as described in EP-A-365484, 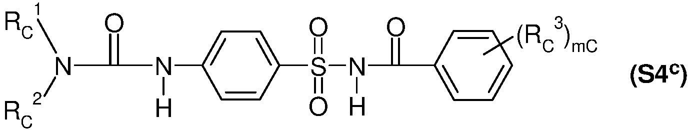 in which
   R_{C}¹, R_{C}² are independently hydrogen, (C₁-C₈)-alkyl, (C₃-C₈)-cycloalkyl, (C₃-C₆)-alkenyl, (C₃-C₆)-alkynyl,
   R_{C}³ is halogen, (C₁-C₄)-alkyl, (C₁-C₄)-alkoxy, CF₃ and
   m_{C} is 1 or 2;
   for example
   1-[4-(N-2-methoxybenzoylsulfamoyl)phenyl]-3-methylurea,
   1-[4-(N-2-methoxybenzoylsulfamoyl)phenyl]-3,3-dimethylurea,
   1-[4-(N-4,5-dimethylbenzoylsulfamoyl)phenyl]-3-methylurea;
S4^{d}) Compounds of the N-phenylsulfonylterephthalamide type of the formula (S4^{d}) and salts thereof, which are known, for example, from CN 101838227, 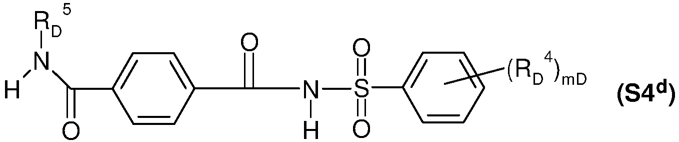 in which
   R_{D}⁴ is halogen, (C₁-C₄)-alkyl, (C₁-C₄)-alkoxy, CF₃;
   m_{D} is 1 or 2;
   R_{D}⁵ is hydrogen, (C₁-C₆)-alkyl, (C₃-C₆)-cycloalkyl, (C₂-C₆)-alkenyl, (C₂-C₆)-alkynyl or (C₅-C₆)-cycloalkenyl.
S5) Active compounds from the class of the hydroxyaromatics and the aromatic-aliphatic carboxylic acid derivatives (S5), for example ethyl 3,4,5-triacetoxybenzoate, 3,5-dimethoxy-4-hydroxybenzoic acid, 3,5-dihydroxybenzoic acid, 4-hydroxysalicylic acid, 4-fluorosalicyclic acid, 2-hydroxycinnamic acid, 2,4-dichlorocinnamic acid, as described in WO-A-2004/084631, WO-A-2005/015994, WO-A-2005/016001.
S6) Active compounds from the class of the 1,2-dihydroquinoxalin-2-ones (S6), for example 1-methyl-3-(2-thienyl)-1,2-dihydroquinoxalin-2-one, 1-methyl-3-(2-thienyl)-1,2-dihydroquinoxaline-2-thione, 1-(2-aminoethyl)-3-(2-thienyl)-1,2-dihydroquinoxalin-2-one hydrochloride, 1-(2-methylsulfonylaminoethyl)-3-(2-thienyl)-1,2-dihydroquinoxalin-2-one, as described in WO-A-2005/112630.
S7) Compounds from the class of the diphenylmethoxyacetic acid derivatives (S7), e.g. methyl diphenylmethoxyacetate (CAS Reg. No. 41858-19-9) (S7-1), ethyl diphenylmethoxyacetate or diphenylmethoxyacetic acid, as described in WO-A-98/38856.
S8) Compounds of the formula (S8), as described in WO-A-98/27049, 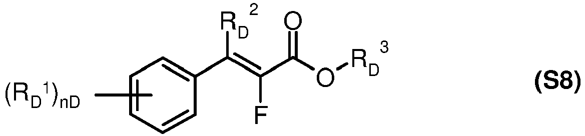 in which the symbols and indices are defined as follows:
   R_{D}¹ is halogen, (C₁-C₄)-alkyl, (C₁-C₄)-haloalkyl, (C₁-C₄)-alkoxy, (C₁-C₄)-haloalkoxy,
   R_{D}² is hydrogen or (C₁-C₄)-alkyl,
   R_{D}³ is hydrogen, (C₁-C₈)-alkyl, (C₂-C₄)-alkenyl, (C₂-C₄)-alkynyl or aryl, where each of the aforementioned carbon-containing radicals is unsubstituted or substituted by one or more, preferably up to three identical or different radicals from the group consisting of halogen and alkoxy; or salts thereof, n_{D} is an integer from 0 to 2.
S9) active compounds from the class of the 3-(5-tetrazolylcarbonyl)-2-quinolones (S9), for example 1,2-dihydro-4-hydroxy-1-ethyl-3-(5-tetrazolylcarbonyl)-2-quinolone (CAS Reg. No. 219479-18-2), 1,2-dihydro-4-hydroxy-1-methyl-3-(5-tetrazolylcarbonyl)-2-quinolone (CAS Reg. No. 95855-00-8), as described in WO-A-199/000020;
S10) Compounds of the formula (S10^{a}) or (S10^{b})
   as described in WO-A-2007/023719 and WO-A-2007/023764 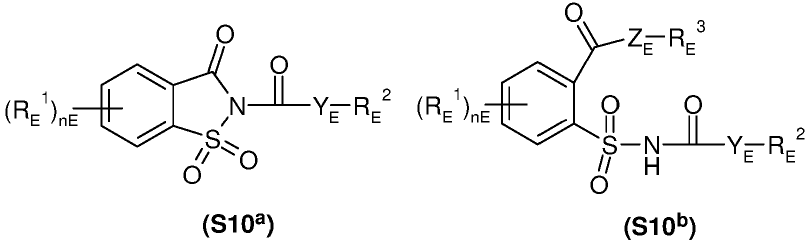
   in which
   R_{E}¹ is halogen, (C₁-C₄)-alkyl, methoxy, nitro, cyano, CF₃, OCF₃
   Y_{E}, Z_{E} are independently O or S,
   n_{E} is an integer from 0 to 4,
   R_{E}² is (C₁-C₁₆)-alkyl, (C₂-C₆)-alkenyl, (C₃-C₆)-cycloalkyl, aryl; benzyl, halobenzyl,
   R_{E}³ is hydrogen or (C₁-C₆)-alkyl.
S11) Active compounds of the oxyimino compound type (S11), which are known as seed-dressing agents, for example
   "oxabetrinil" ((Z)-1,3-dioxolan-2-ylmethoxyimino(phenyl)acetonitrile) (S11-1), which is known as a seed-dressing safener for millet/sorghum against metolachlor damage,
   "fluxofenim" (1-(4-chlorophenyl)-2,2,2-trifluoro-1-ethanone O-(1,3-dioxolan-2-ylmethyl)oxime) (S11-2), which is known as a seed-dressing safener for millet/sorghum against metolachlor damage, and
   "cyometrinil" or "CGA-43089" ((Z)-cyanomethoxyimino(phenyl)acetonitrile) (S11-3), which is known as a seed-dressing safener for millet/sorghum against metolachlor damage.
S12) active compounds from the class of the isothiochromanones (S12), for example methyl [(3-oxo-1H-2-benzothiopyran-4(3H)-ylidene)methoxy]acetate (CAS Reg. No. 205121-04-6) (S12-1) and related compounds from WO-A-1998/13361.
S13) One or more compounds from group (S13):
   "naphthalic anhydride" (1,8-naphthalenedicarboxylic anhydride) (S13-1), which is known as a seed-dressing safener for corn against thiocarbamate herbicide damage,
   "fenclorim" (4,6-dichloro-2-phenylpyrimidine) (S13-2), which is known as a safener for pretilachlor in sown rice,
   "flurazole" (benzyl 2-chloro-4-trifluoromethyl-1,3-thiazole-5-carboxylate) (S13-3), which is known as a seed-dressing safener for millet/sorghum against alachlor and metolachlor damage,
   "CL 304415" (CAS Reg. No. 31541-57-8)
   (4-carboxy-3,4-dihydro-2H-1-benzopyran-4-acetic acid) (S13-4) from American Cyanamid, which is known as a safener for corn against damage by imidazolinones,
   "MG 191" (CAS Reg. No. 96420-72-3) (2-dichloromethyl-2-methyl-1,3-dioxolane) (S13-5) from Nitrokemia, which is known as a safener for corn,
   "MG 838" (CAS Reg. No. 133993-74-5)
   (2-propenyl 1-oxa-4-azaspiro[4.5]decane-4-carbodithioate) (S13-6) from Nitrokemia "disulfoton" (O,O-diethyl S-2-ethylthioethyl phosphorodithioate) (S13-7),
   "dietholate" (O,O-diethyl O-phenyl phosphorothioate) (S13-8),
   "mephenate" (4-chlorophenyl methylcarbamate) (S13-9).
S14) active compounds which, in addition to herbicidal action against weeds, also have safener action on crop plants such as rice, for example
   "dimepiperate" or "MY-93" (S-1-methyl 1-phenylethylpiperidine-1-carbothioate), which is known as a safener for rice against damage by the herbicide molinate,
   "daimuron" or "SK 23" (1-(1-methyl-1-phenylethyl)-3-p-tolylurea), which is known as safener for rice against imazosulfuron herbicide damage,
   "cumyluron" = "JC-940" (3-(2-chlorophenylmethyl)-1-(1-methyl-1-phenylethyl)urea, see JP-A-60087254), which is known as safener for rice against damage by some herbicides,
   "methoxyphenone" or "NK 049" (3,3'-dimethyl-4-methoxybenzophenone), which is known as a safener for rice against damage by some herbicides,
   "CSB" (1-bromo-4-(chloromethylsulfonyl)benzene) from Kumiai, (CAS Reg. No. 54091-06-4), which is known as a safener against damage by some herbicides in rice.
S15) Compounds of the formula (S15) or tautomers thereof 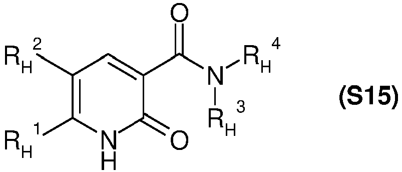 as described in WO-A-2008/131861 and WO-A-2008/131860
   in which
   R_{H}¹ is a (C₁-C₆)-haloalkyl radical and
   R_{H}² is hydrogen or halogen and
   R_{H}³, R_{H}⁴ are each independently hydrogen, (C₁-C₁₆)-alkyl, (C₂-C₁₆)-alkenyl or (C₂-C₁₆)-alkynyl, where each of the 3 latter radicals is unsubstituted or substituted by one or more radicals from the group of halogen, hydroxyl, cyano, (C₁-C₄)-alkoxy, (C₁-C₄)-haloalkoxy, (C₁-C₄)-alkylthio, (C₁-C₄)-alkylamino, di[(C₁-C₄)-alkyl]amino, [(C₁-C₄)-alkoxy]carbonyl, [(C₁-C₄)-haloalkoxy]carbonyl, (C₃-C₆)-cycloalkyl which is unsubstituted or substituted, phenyl which is unsubstituted or substituted, and heterocyclyl which is unsubstituted or substituted,
   or (C₃-C₆)-cycloalkyl, (C₄-C₆)-cycloalkenyl, (C₃-C₆)-cycloalkyl fused on one side of the ring to a 4 to 6-membered saturated or unsaturated carbocyclic ring, or (C₄-C₆)-cycloalkenyl fused on one side of the ring to a 4 to 6-membered saturated or unsaturated carbocyclic ring,
   where each of the 4 latter radicals is unsubstituted or substituted by one or more radicals from the group of halogen, hydroxyl, cyano, (C₁-C₄)-alkyl, (C₁-C₄)-haloalkyl, (C₁-C₄)-alkoxy, (C₁-C₄)-haloalkoxy, (C₁-C₄)-alkylthio, (C₁-C₄)-alkylamino, di[(C₁-C₄)alkyl]amino, [(C₁-C₄)alkoxy]carbonyl, [(C₁-C₄)haloalkoxy]carbonyl, (C₃-C₆)-cycloalkyl which is unsubstituted or substituted, phenyl which is unsubstituted or substituted, and heterocyclyl which is unsubstituted or substituted,
      or
   R_{H}³ is (C₁-C₄)-alkoxy, (C₂-C₄)-alkenyloxy, (C₂-C₆)-alkynyloxy or (C₂-C₄)-haloalkoxy and
   R_{H}⁴ is hydrogen or (C₁-C₄)-alkyl or
   R_{H}³ and R_{H}⁴ together with the directly bonded nitrogen atom are a four- to eight-membered heterocyclic ring which, as well as the nitrogen atom, may also contain further ring heteroatoms, preferably up to two further ring heteroatoms from the group of N, O and S, and which is unsubstituted or substituted by one or more radicals from the group of halogen, cyano, nitro, (C₁-C₄)-alkyl, (C₁-C₄)-haloalkyl, (C₁-C₄)-alkoxy, (C₁-C₄)-haloalkoxy and (C₁-C₄)-alkylthio.
S16) Active compounds which are used primarily as herbicides but also have safener action on crop plants, for example
   (2,4-dichlorophenoxy)acetic acid (2,4-D),
   (4-chlorophenoxy)acetic acid,
   (R,S)-2-(4-chloro-o-tolyloxy)propionic acid (mecoprop),
   4-(2,4-dichlorophenoxy)butyric acid (2,4-DB),
   (4-chloro-o-tolyloxy)acetic acid (MCPA),
   4-(4-chloro-o-tolyloxy)butyric acid,
   4-(4-chlorophenoxy)butyric acid,
   3,6-dichloro-2-methoxybenzoic acid (dicamba),
   1-(ethoxycarbonyl)ethyl 3,6-dichloro-2-methoxybenzoate (lactidichlor-ethyl).

Preferred safeners in combination with the compounds of the general formula (I) according to the invention and/or salts thereof, in particular with the compounds of the formulae (I-001) to (I-192) and and/or salts thereof, are: cloquintocet-mexyl, cyprosulfamide, fenchlorazole-ethyl, isoxadifen-ethyl, mefenpyr-diethyl, fenclorim, cumyluron, S4-1 and S4-5, and particularly preferred safeners are: cloquintocet-mexyl, cyprosulfamide, isoxadifen-ethyl and mefenpyr-diethyl.

### Biological Examples:

The following abbreviations are used in the examples and Tables below:

### Tested harmful plants:

- ABUTH:: Abutilon theophrasti
- AGSTE:: Agrostis tenuis
- ALOMY:: Alopecurus myosuroides
- AMARE: Amaranthus retroflexus
- AVEFA:: Avena Fatua
- DIGSA:: Digitaria sanguinalis
- ECHCG:: Echinochloa crus-galli
- KCHSC:: Kochia scoparia
- LOLRI:: Lolium rigidum
- MATIN:: Matricaria inodora
- PHBPU:: Pharbitis purpurea
- POAAN:: Poa annua
- POLCO:: Polygonum convolvulus
- SETVI:: Setaria viridis
- STEME:: Stellaria media
- VERPE:: Veronica persica
- VIOTR:: Viola tricolor

### Tested crop plants:

- BRSNW:: Brassica napus
- GLXMA:: Glycine max
- ORYSA:: Oryza sativa
- TRZAS:: Triticum aestivum
- ZEAMX:: Zea mays

### A. Herbicidal pre-emergence action

Seeds of mono- and dicotyledonous weed plants were sown in plastic pots (double sowings with one species of mono- and one species of dicotyledonous weed plants per pot), in sandy loam, and covered with soil. The compounds according to the invention, formulated in the form of wettable powders (WP) or as emulsifiable concentrates (EC), were applied to the surface of the covering soil as aqueous suspension or emulsion, with the addition of 0.5% of an additive, at an application rate of 600 l of water per hectare (converted). Following treatment, the pots were placed in a greenhouse and kept under optimum growth conditions for the test plants. The visual grading of the damage to the test plants was carried out after ca. 3 weeks in comparison to untreated controls (herbicidal effect in percent (%): 100% effect = plants have died off, 0% effect = as control plants).

Tables A1 to A12, below, show the effects of selected compounds of the general formula (I) according to table 1 on various harmful plants and an application rate corresponding to 1280 g/ha or below obtained by the experimental procedure mentioned above.

**Table A1: pre-emergence activity at 1280g/ha and 320 g/ha against ABUTH in %**

| Example number | application rate [g/ha] | ABUTH |
|---|---|---|
| I-003 | 1280 | 90 |
| I-005 | 1280 | 90 |
| I-007 | 1280 | 90 |
| I-008 | 1280 | 90 |
| I-009 | 1280 | 90 |
| I-010 | 1280 | 90 |
| I-019 | 1280 | 90 |
| I-024 | 320 | 90 |
| I-027 | 1280 | 90 |
| I-029 | 1280 | 90 |
| I-031 | 1280 | 90 |
| I-035 | 1280 | 90 |
| I-036 | 1280 | 90 |
| I-040 | 1280 | 90 |
| I-041 | 1280 | 90 |
| I-050 | 1280 | 90 |
| I-051 | 320 | 100 |
| I-052 | 1280 | 90 |
| I-055 | 1280 | 90 |
| I-057 | 320 | 90 |
| I-058 | 1280 | 90 |
| I-059 | 320 | 90 |
| I-060 | 320 | 90 |
| I-065 | 1280 | 90 |
| I-066 | 320 | 90 |
| I-069 | 320 | 90 |
| I-071 | 320 | 90 |
| I-072 | 320 | 90 |
| I-073 | 1280 | 90 |
| I-074 | 1280 | 90 |
| I-091 | 1280 | 100 |
| I-100 | 320 | 100 |
| I-104 | 1280 | 90 |
| I-122 | 1280 | 90 |
| I-123 | 1280 | 90 |
| I-120 | 1280 | 90 |
| I-119 | 1280 | 90 |
| I-121 | 1280 | 90 |
| I-124 | 1280 | 90 |
| I-127 | 320 | 90 |
| I-128 | 320 | 90 |
| I-130 | 1280 | 90 |
| I-129 | 1280 | 90 |
| I-137 | 1280 | 100 |
| I-141 | 320 | 100 |
| I-138 | 1280 | 90 |
| I-139 | 320 | 90 |
| I-143 | 1280 | 90 |
| I-144 | 1280 | 90 |
| I-152 | 320 | 90 |
| I-155 | 1280 | 100 |
| I-157 | 320 | 90 |
| I-159 | 1280 | 100 |

**Table A2: pre-emergence activity at 1280g/ha and 320 g/ha against ALOMY in %**

| Example number | application rate [g/ha] | ALOMY |
|---|---|---|
| I-002 | 1280 | 100 |
| I-003 | 1280 | 100 |
| I-005 | 1280 | 90 |
| I-007 | 1280 | 100 |
| I-008 | 1280 | 90 |
| I-009 | 1280 | 90 |
| I-010 | 1280 | 100 |
| I-019 | 1280 | 90 |
| I-024 | 1280 | 100 |
| I-027 | 1280 | 100 |
| I-029 | 1280 | 90 |
| I-031 | 320 | 100 |
| I-033 | 1280 | 90 |
| I-036 | 1280 | 90 |
| I-038 | 1280 | 90 |
| I-041 | 1280 | 90 |
| I-046 | 1280 | 100 |
| I-050 | 1280 | 90 |
| I-051 | 320 | 100 |
| I-052 | 320 | 100 |
| I-055 | 1280 | 90 |
| I-056 | 1280 | 90 |
| I-057 | 1280 | 90 |
| I-058 | 1280 | 100 |
| I-059 | 1280 | 100 |
| I-060 | 1280 | 90 |
| I-064 | 1280 | 90 |
| I-065 | 1280 | 90 |
| I-066 | 1280 | 90 |
| I-069 | 1280 | 100 |
| I-071 | 1280 | 100 |
| I-072 | 1280 | 90 |
| I-074 | 1280 | 90 |
| I-075 | 1280 | 90 |
| I-083 | 320 | 100 |
| I-088 | 1280 | 100 |
| I-091 | 1280 | 100 |
| I-104 | 320 | 90 |
| I-116 | 1280 | 90 |
| I-122 | 1280 | 100 |
| I-123 | 1280 | 90 |
| I-120 | 1280 | 100 |
| I-119 | 1280 | 90 |
| I-121 | 1280 | 90 |
| I-124 | 320 | 100 |
| I-117 | 1280 | 90 |
| I-127 | 320 | 100 |
| I-128 | 320 | 100 |
| I-125 | 1280 | 90 |
| I-130 | 320 | 100 |
| I-129 | 320 | 90 |
| I-137 | 320 | 90 |
| I-141 | 1280 | 90 |
| I-139 | 1280 | 90 |
| I-143 | 1280 | 90 |
| I-144 | 1280 | 90 |
| I-145 | 1280 | 90 |
| I-152 | 1280 | 90 |
| I-154 | 1280 | 90 |
| I-156 | 1280 | 90 |
| I-157 | 1280 | 90 |
| I-168 | 1280 | 90 |
| I-169 | 1280 | 90 |
| I-174 | 1280 | 90 |

**Table A3: pre-emergence activity at 1280g/ha and 320 g/ha against ECHCG in %**

| Example number | application rate [g/ha] | ECHCG |
|---|---|---|
| I-002 | 1280 | 100 |
| I-003 | 1280 | 100 |
| I-004 | 1280 | 100 |
| I-005 | 320 | 100 |
| I-006 | 320 | 100 |
| I-007 | 320 | 100 |
| I-008 | 320 | 100 |
| I-009 | 320 | 100 |
| I-010 | 320 | 100 |
| I-011 | 320 | 100 |
| I-013 | 320 | 100 |
| I-019 | 320 | 100 |
| I-023 | 1280 | 100 |
| I-024 | 320 | 100 |
| I-025 | 1280 | 100 |
| I-027 | 320 | 100 |
| I-029 | 320 | 100 |
| I-030 | 1280 | 100 |
| I-031 | 320 | 100 |
| I-032 | 320 | 100 |
| I-033 | 1280 | 100 |
| I-034 | 1280 | 100 |
| I-035 | 1280 | 100 |
| I-036 | 320 | 100 |
| I-038 | 320 | 100 |
| I-040 | 320 | 100 |
| I-041 | 320 | 100 |
| I-042 | 1280 | 100 |
| I-044 | 1280 | 100 |
| I-045 | 1280 | 90 |
| I-046 | 320 | 100 |
| I-047 | 320 | 100 |
| I-048 | 320 | 100 |
| I-050 | 1280 | 100 |
| I-051 | 1280 | 100 |
| I-052 | 1280 | 100 |
| I-053 | 1280 | 100 |
| I-054 | 1280 | 100 |
| I-055 | 1280 | 100 |
| I-056 | 1280 | 100 |
| I-057 | 1280 | 100 |
| I-058 | 1280 | 100 |
| I-059 | 1280 | 100 |
| I-060 | 1280 | 100 |
| I-062 | 1280 | 100 |
| I-063 | 1280 | 100 |
| I-064 | 1280 | 100 |
| I-065 | 320 | 100 |
| I-066 | 1280 | 100 |
| I-069 | 320 | 100 |
| I-070 | 1280 | 100 |
| I-071 | 320 | 100 |
| I-072 | 320 | 100 |
| I-073 | 320 | 100 |
| I-074 | 320 | 100 |
| I-075 | 320 | 100 |
| I-081 | 1280 | 90 |
| I-083 | 320 | 100 |
| I-084 | 1280 | 100 |
| I-088 | 320 | 100 |
| I-089 | 320 | 100 |
| I-090 | 1280 | 90 |
| I-091 | 1280 | 100 |
| I-098 | 1280 | 90 |
| I-100 | 1280 | 90 |
| I-101 | 1280 | 90 |
| I-103 | 1280 | 90 |
| I-104 | 320 | 100 |
| I-111 | 1280 | 100 |
| I-113 | 1280 | 100 |
| I-122 | 320 | 100 |
| I-123 | 320 | 100 |
| I-120 | 320 | 100 |
| I-119 | 320 | 100 |
| I-121 | 320 | 100 |
| I-124 | 320 | 100 |
| I-117 | 320 | 100 |
| I-127 | 320 | 100 |
| I-128 | 320 | 100 |
| I-125 | 1280 | 100 |
| I-130 | 320 | 100 |
| I-129 | 320 | 100 |
| I-137 | 320 | 100 |
| I-141 | 320 | 100 |
| I-138 | 320 | 90 |
| I-139 | 320 | 100 |
| I-134 | 1280 | 100 |
| I-143 | 320 | 100 |
| I-144 | 1280 | 100 |
| I-142 | 320 | 100 |
| I-145 | 1280 | 100 |
| I-147 | 320 | 100 |
| I-149 | 1280 | 100 |
| I-152 | 1280 | 90 |
| I-153 | 1280 | 90 |
| I-154 | 1280 | 100 |
| I-155 | 1280 | 100 |
| I-156 | 320 | 90 |
| I-157 | 320 | 90 |
| I-159 | 1280 | 100 |
| I-160 | 1280 | 100 |
| I-165 | 1280 | 100 |
| I-166 | 1280 | 90 |
| I-167 | 1280 | 100 |
| I-168 | 1280 | 100 |
| I-171 | 1280 | 100 |
| I-174 | 1280 | 90 |
| I-175 | 1280 | 100 |
| I-181 | 1280 | 90 |

**Table A4: pre-emergence activity at 1280g/ha and 320 g/ha against KCHSC in %**

| Example number | application rate [g/ha] | KCHSC |
|---|---|---|
| I-002 | 1280 | 90 |
| I-003 | 1280 | 100 |
| I-004 | 1280 | 90 |
| I-005 | 1280 | 90 |
| I-006 | 1280 | 90 |
| I-007 | 1280 | 90 |
| I-008 | 1280 | 90 |
| I-009 | 1280 | 90 |
| I-010 | 1280 | 100 |
| I-011 | 1280 | 90 |
| I-013 | 1280 | 90 |
| I-019 | 1280 | 90 |
| I-023 | 1280 | 90 |
| I-024 | 1280 | 90 |
| I-025 | 320 | 90 |
| I-027 | 320 | 90 |
| I-028 | 320 | 90 |
| I-029 | 320 | 100 |
| I-030 | 1280 | 100 |
| I-031 | 1280 | 100 |
| I-032 | 1280 | 90 |
| I-033 | 320 | 90 |
| I-034 | 1280 | 90 |
| I-035 | 1280 | 100 |
| I-036 | 1280 | 100 |
| I-038 | 320 | 90 |
| I-040 | 320 | 90 |
| I-041 | 320 | 90 |
| I-042 | 320 | 90 |
| I-044 | 320 | 90 |
| I-045 | 320 | 90 |
| I-046 | 320 | 90 |
| I-047 | 1280 | 90 |
| I-048 | 1280 | 90 |
| I-050 | 320 | 90 |
| I-051 | 1280 | 100 |
| I-052 | 1280 | 100 |
| I-053 | 320 | 90 |
| I-054 | 1280 | 90 |
| I-055 | 320 | 90 |
| I-056 | 1280 | 100 |
| I-057 | 1280 | 100 |
| I-058 | 1280 | 90 |
| I-059 | 1280 | 90 |
| I-060 | 1280 | 100 |
| I-062 | 1280 | 90 |
| I-063 | 1280 | 90 |
| I-064 | 1280 | 100 |
| I-065 | 1280 | 100 |
| I-066 | 320 | 90 |
| I-069 | 320 | 90 |
| I-070 | 1280 | 90 |
| I-071 | 320 | 90 |
| I-072 | 1280 | 100 |
| I-073 | 1280 | 100 |
| I-074 | 1280 | 100 |
| I-075 | 1280 | 100 |
| I-081 | 1280 | 90 |
| I-083 | 320 | 90 |
| I-084 | 1280 | 90 |
| I-086 | 1280 | 90 |
| I-087 | 1280 | 90 |
| I-088 | 1280 | 90 |
| I-089 | 320 | 90 |
| I-091 | 1280 | 90 |
| I-097 | 1280 | 90 |
| I-099 | 1280 | 90 |
| I-100 | 1280 | 90 |
| I-101 | 1280 | 90 |
| I-104 | 320 | 90 |
| I-115 | 1280 | 90 |
| I-113 | 320 | 90 |
| I-122 | 320 | 90 |
| I-123 | 320 | 90 |
| I-120 | 320 | 90 |
| I-119 | 320 | 90 |
| I-121 | 320 | 90 |
| I-124 | 1280 | 100 |
| I-117 | 1280 | 100 |
| I-127 | 1280 | 100 |
| I-128 | 320 | 90 |
| I-125 | 320 | 90 |
| I-130 | 1280 | 100 |
| I-129 | 320 | 90 |
| I-137 | 320 | 90 |
| I-141 | 1280 | 100 |
| I-138 | 320 | 90 |
| I-139 | 1280 | 90 |
| I-134 | 1280 | 90 |
| I-143 | 320 | 90 |
| I-144 | 320 | 90 |
| I-142 | 320 | 90 |
| I-145 | 320 | 90 |
| I-147 | 320 | 90 |
| I-149 | 1280 | 90 |
| I-152 | 1280 | 100 |
| I-153 | 320 | 90 |
| I-154 | 320 | 90 |
| I-155 | 1280 | 90 |
| I-156 | 320 | 90 |
| I-157 | 1280 | 90 |
| I-159 | 1280 | 90 |
| I-164 | 1280 | 90 |
| I-167 | 1280 | 90 |
| I-168 | 320 | 90 |
| I-169 | 320 | 90 |
| I-171 | 320 | 90 |
| I-174 | 320 | 90 |
| I-175 | 1280 | 90 |
| I-181 | 1280 | 90 |

**Table A5: pre-emergence activity at 1280g/ha and 320 g/ha against LOLRI in %**

| Example number | application rate [g/ha] | LOLRI |
|---|---|---|
| I-002 | 1280 | 100 |
| I-003 | 320 | 100 |
| I-005 | 320 | 100 |
| I-006 | 1280 | 100 |
| I-007 | 320 | 100 |
| I-008 | 320 | 100 |
| I-009 | 320 | 100 |
| I-010 | 320 | 100 |
| I-011 | 320 | 100 |
| I-013 | 320 | 100 |
| I-019 | 1280 | 100 |
| I-023 | 1280 | 100 |
| I-024 | 320 | 100 |
| I-025 | 320 | 100 |
| I-027 | 320 | 100 |
| I-028 | 1280 | 100 |
| I-029 | 320 | 100 |
| I-030 | 320 | 100 |
| I-031 | 320 | 100 |
| I-032 | 1280 | 100 |
| I-033 | 1280 | 90 |
| I-035 | 1280 | 100 |
| I-036 | 1280 | 100 |
| I-038 | 1280 | 100 |
| I-040 | 1280 | 100 |
| I-041 | 1280 | 100 |
| I-042 | 1280 | 90 |
| I-044 | 1280 | 100 |
| I-046 | 1280 | 100 |
| I-047 | 1280 | 100 |
| I-048 | 1280 | 100 |
| I-050 | 320 | 100 |
| I-051 | 320 | 100 |
| I-052 | 320 | 100 |
| I-053 | 1280 | 100 |
| I-054 | 1280 | 100 |
| I-055 | 1280 | 100 |
| I-056 | 320 | 100 |
| I-057 | 320 | 100 |
| 1-058 | 320 | 100 |
| I-059 | 1280 | 100 |
| I-060 | 1280 | 100 |
| I-062 | 1280 | 90 |
| I-063 | 1280 | 100 |
| I-064 | 1280 | 100 |
| I-065 | 320 | 100 |
| I-066 | 1280 | 100 |
| I-069 | 1280 | 100 |
| I-070 | 1280 | 100 |
| I-071 | 320 | 100 |
| I-072 | 320 | 100 |
| I-073 | 320 | 100 |
| I-074 | 320 | 100 |
| I-075 | 320 | 100 |
| I-083 | 320 | 100 |
| I-084 | 1280 | 100 |
| I-086 | 1280 | 100 |
| I-087 | 1280 | 100 |
| I-088 | 320 | 100 |
| I-089 | 1280 | 100 |
| I-091 | 1280 | 100 |
| I-097 | 1280 | 100 |
| I-101 | 1280 | 100 |
| I-104 | 320 | 100 |
| I-113 | 1280 | 100 |
| I-122 | 320 | 100 |
| I-123 | 320 | 100 |
| I-120 | 320 | 100 |
| I-119 | 320 | 100 |
| I-121 | 320 | 100 |
| I-124 | 320 | 100 |
| I-117 | 1280 | 100 |
| I-127 | 320 | 100 |
| I-128 | 320 | 100 |
| I-125 | 320 | 100 |
| I-130 | 320 | 100 |
| I-129 | 320 | 100 |
| I-137 | 1280 | 100 |
| I-141 | 1280 | 100 |
| I-139 | 1280 | 100 |
| I-143 | 320 | 100 |
| I-144 | 320 | 90 |
| I-142 | 320 | 100 |
| I-145 | 1280 | 100 |
| I-147 | 1280 | 90 |
| I-149 | 1280 | 90 |
| I-152 | 1280 | 100 |
| I-153 | 320 | 90 |
| I-154 | 1280 | 100 |
| I-155 | 1280 | 100 |
| I-156 | 320 | 90 |
| I-157 | 1280 | 100 |
| I-166 | 1280 | 90 |
| I-167 | 1280 | 100 |
| I-168 | 320 | 90 |
| I-169 | 1280 | 90 |
| I-171 | 1280 | 100 |
| I-174 | 320 | 100 |

**Table A6: pre-emergence activity at 1280g/ha and 320 g/ha against MATIN in %**

| Example number | application rate [g/ha] | MATIN |
|---|---|---|
| I-002 | 1280 | 100 |
| I-003 | 320 | 90 |
| I-004 | 1280 | 90 |
| I-005 | 320 | 90 |
| I-006 | 1280 | 90 |
| I-007 | 1280 | 90 |
| I-008 | 320 | 90 |
| I-009 | 1280 | 100 |
| I-010 | 320 | 90 |
| I-019 | 1280 | 90 |
| I-023 | 1280 | 90 |
| I-024 | 1280 | 90 |
| I-025 | 1280 | 100 |
| I-027 | 320 | 90 |
| I-028 | 1280 | 90 |
| I-029 | 320 | 90 |
| I-030 | 1280 | 90 |
| I-031 | 320 | 90 |
| I-032 | 1280 | 90 |
| I-035 | 1280 | 90 |
| I-036 | 1280 | 90 |
| I-038 | 1280 | 90 |
| I-040 | 1280 | 90 |
| I-041 | 1280 | 90 |
| I-044 | 320 | 90 |
| I-047 | 1280 | 90 |
| I-048 | 1280 | 90 |
| I-050 | 320 | 90 |
| I-051 | 320 | 90 |
| I-052 | 320 | 90 |
| I-053 | 1280 | 90 |
| I-055 | 1280 | 90 |
| I-056 | 1280 | 90 |
| I-057 | 1280 | 90 |
| I-058 | 320 | 90 |
| I-059 | 320 | 90 |
| I-060 | 320 | 90 |
| I-064 | 320 | 90 |
| I-065 | 1280 | 90 |
| I-066 | 320 | 90 |
| I-069 | 320 | 90 |
| I-070 | 1280 | 90 |
| I-071 | 320 | 90 |
| I-072 | 320 | 90 |
| I-073 | 1280 | 90 |
| I-074 | 320 | 90 |
| I-075 | 1280 | 90 |
| I-083 | 1280 | 90 |
| I-086 | 1280 | 90 |
| I-088 | 1280 | 90 |
| I-089 | 1280 | 90 |
| I-091 | 1280 | 90 |
| I-097 | 1280 | 90 |
| I-104 | 320 | 100 |
| I-122 | 320 | 90 |
| I-123 | 320 | 90 |
| I-120 | 320 | 90 |
| I-119 | 320 | 90 |
| I-121 | 320 | 90 |
| I-124 | 320 | 90 |
| I-117 | 1280 | 90 |
| I-127 | 320 | 100 |
| I-128 | 320 | 100 |
| I-125 | 1280 | 90 |
| I-130 | 320 | 90 |
| I-129 | 1280 | 100 |
| I-137 | 320 | 90 |
| I-141 | 1280 | 90 |
| I-138 | 1280 | 90 |
| I-139 | 1280 | 90 |
| I-143 | 320 | 90 |
| I-144 | 1280 | 100 |
| I-142 | 1280 | 90 |
| I-145 | 1280 | 90 |
| I-147 | 1280 | 90 |
| I-152 | 1280 | 90 |
| I-153 | 1280 | 90 |
| I-154 | 1280 | 90 |
| I-155 | 1280 | 90 |
| I-156 | 1280 | 90 |
| I-157 | 1280 | 90 |
| I-167 | 1280 | 90 |
| I-168 | 320 | 90 |
| I-169 | 1280 | 90 |
| I-171 | 1280 | 90 |
| I-174 | 320 | 90 |

**Table A7: pre-emergence activity at 1280g/ha and 320 g/ha against POAAN in %**

| Example number | application rate [g/ha] | POAAN |
|---|---|---|
| I-002 | 1280 | 100 |
| I-003 | 1280 | 100 |
| I-004 | 1280 | 100 |
| I-005 | 1280 | 100 |
| I-006 | 1280 | 100 |
| I-007 | 320 | 100 |
| I-008 | 320 | 100 |
| I-009 | 320 | 100 |
| I-010 | 320 | 100 |
| I-011 | 320 | 100 |
| I-019 | 1280 | 100 |
| I-023 | 1280 | 100 |
| I-024 | 1280 | 100 |
| I-027 | 1280 | 100 |
| I-028 | 1280 | 100 |
| I-029 | 1280 | 100 |
| I-030 | 1280 | 100 |
| I-031 | 1280 | 100 |
| I-032 | 1280 | 100 |
| I-033 | 1280 | 100 |
| I-034 | 1280 | 100 |
| I-035 | 1280 | 100 |
| I-036 | 1280 | 100 |
| I-038 | 1280 | 100 |
| I-040 | 1280 | 100 |
| I-041 | 1280 | 100 |
| I-042 | 1280 | 100 |
| I-043 | 1280 | 90 |
| I-044 | 1280 | 100 |
| I-045 | 1280 | 100 |
| I-046 | 1280 | 100 |
| I-047 | 1280 | 100 |
| I-048 | 1280 | 100 |
| I-050 | 1280 | 100 |
| I-051 | 320 | 100 |
| I-052 | 320 | 100 |
| I-053 | 320 | 100 |
| I-054 | 320 | 100 |
| I-055 | 320 | 100 |
| I-056 | 1280 | 100 |
| I-057 | 1280 | 100 |
| I-058 | 1280 | 100 |
| I-059 | 320 | 100 |
| I-060 | 320 | 100 |
| I-062 | 320 | 100 |
| I-063 | 320 | 100 |
| I-064 | 320 | 100 |
| I-065 | 320 | 100 |
| I-066 | 320 | 100 |
| I-069 | 320 | 100 |
| I-070 | 320 | 100 |
| I-071 | 320 | 100 |
| I-072 | 320 | 100 |
| I-073 | 320 | 100 |
| I-074 | 320 | 100 |
| I-075 | 320 | 100 |
| I-076 | 1280 | 100 |
| I-081 | 320 | 90 |
| I-083 | 1280 | 100 |
| I-084 | 320 | 100 |
| I-085 | 1280 | 100 |
| I-086 | 320 | 90 |
| I-087 | 320 | 100 |
| I-088 | 320 | 100 |
| I-089 | 320 | 100 |
| I-090 | 320 | 100 |
| I-091 | 320 | 100 |
| I-092 | 1280 | 100 |
| I-097 | 320 | 100 |
| I-098 | 1280 | 100 |
| I-099 | 320 | 100 |
| I-100 | 1280 | 100 |
| I-101 | 1280 | 100 |
| I-103 | 1280 | 100 |
| I-106 | 1280 | 100 |
| I-104 | 320 | 100 |
| I-112 | 1280 | 100 |
| I-113 | 1280 | 100 |
| I-109 | 1280 | 90 |
| I-122 | 320 | 100 |
| I-123 | 320 | 100 |
| I-120 | 320 | 100 |
| I-119 | 320 | 100 |
| I-121 | 320 | 100 |
| I-124 | 320 | 100 |
| I-117 | 320 | 100 |
| I-127 | 320 | 100 |
| I-128 | 320 | 100 |
| I-125 | 320 | 100 |
| I-130 | 320 | 100 |
| I-129 | 320 | 100 |
| I-137 | 320 | 100 |
| I-141 | 320 | 100 |
| I-140 | 1280 | 100 |
| I-138 | 320 | 100 |
| I-139 | 320 | 100 |
| I-134 | 1280 | 100 |
| I-135 | 1280 | 90 |
| I-143 | 320 | 100 |
| I-144 | 320 | 100 |
| I-142 | 320 | 100 |
| I-145 | 320 | 100 |
| I-147 | 320 | 100 |
| I-149 | 1280 | 100 |
| I-152 | 320 | 100 |
| I-153 | 1280 | 100 |
| I-154 | 1280 | 100 |
| I-155 | 1280 | 100 |
| I-156 | 320 | 100 |
| I-157 | 1280 | 100 |
| I-159 | 1280 | 100 |
| I-160 | 1280 | 100 |
| I-162 | 1280 | 100 |
| I-164 | 1280 | 100 |
| I-165 | 1280 | 100 |
| I-166 | 1280 | 100 |
| I-167 | 320 | 100 |
| I-168 | 320 | 100 |
| I-169 | 320 | 100 |
| I-171 | 320 | 100 |
| I-174 | 320 | 100 |
| I-175 | 1280 | 100 |
| I-181 | 1280 | 100 |
| I-178 | 1280 | 90 |
| I-189 | 1280 | 90 |

**Table A8: pre-emergence activity at 1280g/ha and 320 g/ha against SETVI in %**

| Example number | application rate [g/ha] | SETVI |
|---|---|---|
| I-002 | 320 | 100 |
| I-003 | 320 | 100 |
| I-004 | 1280 | 100 |
| I-005 | 320 | 100 |
| I-006 | 1280 | 100 |
| I-007 | 320 | 100 |
| I-008 | 1280 | 100 |
| I-009 | 1280 | 100 |
| I-010 | 1280 | 100 |
| I-011 | 1280 | 90 |
| I-013 | 1280 | 90 |
| I-019 | 1280 | 100 |
| I-023 | 1280 | 100 |
| I-024 | 1280 | 100 |
| I-025 | 1280 | 100 |
| I-027 | 320 | 100 |
| I-028 | 320 | 100 |
| I-029 | 320 | 100 |
| I-030 | 320 | 100 |
| I-031 | 320 | 100 |
| I-032 | 320 | 100 |
| I-033 | 1280 | 100 |
| I-034 | 1280 | 100 |
| I-035 | 320 | 100 |
| I-036 | 320 | 100 |
| I-038 | 320 | 100 |
| I-040 | 320 | 100 |
| I-041 | 320 | 100 |
| I-042 | 1280 | 100 |
| I-044 | 1280 | 100 |
| I-045 | 1280 | 100 |
| I-046 | 1280 | 100 |
| I-047 | 1280 | 100 |
| I-048 | 1280 | 100 |
| I-050 | 320 | 100 |
| I-051 | 320 | 100 |
| I-052 | 320 | 100 |
| I-053 | 320 | 100 |
| I-054 | 1280 | 100 |
| I-055 | 1280 | 100 |
| I-056 | 1280 | 100 |
| I-057 | 1280 | 100 |
| I-058 | 1280 | 100 |
| I-059 | 1280 | 100 |
| I-060 | 1280 | 100 |
| I-062 | 1280 | 100 |
| I-063 | 320 | 100 |
| I-064 | 320 | 100 |
| I-065 | 320 | 100 |
| I-066 | 320 | 100 |
| I-069 | 320 | 100 |
| I-070 | 320 | 100 |
| I-071 | 320 | 100 |
| I-072 | 320 | 100 |
| I-073 | 320 | 100 |
| I-074 | 320 | 100 |
| I-075 | 320 | 100 |
| I-081 | 1280 | 100 |
| I-083 | 320 | 100 |
| I-084 | 320 | 100 |
| I-085 | 1280 | 100 |
| I-086 | 1280 | 100 |
| I-087 | 320 | 100 |
| I-088 | 320 | 100 |
| I-089 | 1280 | 100 |
| I-091 | 320 | 100 |
| I-094 | 1280 | 100 |
| I-097 | 1280 | 100 |
| I-099 | 320 | 100 |
| I-100 | 1280 | 100 |
| I-101 | 320 | 100 |
| I-103 | 1280 | 100 |
| I-106 | 1280 | 100 |
| I-104 | 320 | 100 |
| I-116 | 1280 | 90 |
| I-112 | 1280 | 90 |
| I-113 | 320 | 100 |
| I-109 | 1280 | 100 |
| I-122 | 320 | 100 |
| I-123 | 320 | 100 |
| I-120 | 320 | 100 |
| I-119 | 320 | 100 |
| I-121 | 1280 | 100 |
| I-124 | 320 | 100 |
| I-117 | 320 | 100 |
| I-126 | 1280 | 90 |
| I-127 | 320 | 100 |
| I-128 | 320 | 100 |
| I-125 | 1280 | 100 |
| I-130 | 320 | 100 |
| I-129 | 320 | 100 |
| I-137 | 320 | 100 |
| I-141 | 320 | 100 |
| I-138 | 1280 | 100 |
| I-139 | 320 | 100 |
| I-134 | 1280 | 100 |
| I-143 | 320 | 100 |
| I-144 | 320 | 100 |
| I-142 | 320 | 100 |
| I-145 | 1280 | 100 |
| I-147 | 320 | 100 |
| I-149 | 320 | 100 |
| I-152 | 1280 | 100 |
| I-153 | 1280 | 100 |
| I-154 | 320 | 100 |
| I-155 | 1280 | 100 |
| I-156 | 320 | 100 |
| I-157 | 320 | 100 |
| I-159 | 1280 | 100 |
| I-160 | 1280 | 100 |
| I-164 | 1280 | 100 |
| I-165 | 1280 | 100 |
| I-166 | 1280 | 90 |
| I-167 | 1280 | 90 |
| I-168 | 320 | 100 |
| I-169 | 320 | 100 |
| I-171 | 320 | 100 |
| I-174 | 1280 | 100 |
| I-175 | 1280 | 100 |
| I-176 | 1280 | 90 |

**Table A9: pre-emergence activity at 1280g/ha and 320 g/ha against STEME in %**

| Example number | application rate [g/ha] | STEME |
|---|---|---|
| I-002 | 1280 | 100 |
| I-003 | 1280 | 90 |
| I-005 | 1280 | 90 |
| I-006 | 1280 | 90 |
| I-007 | 1280 | 90 |
| I-008 | 1280 | 90 |
| I-009 | 320 | 100 |
| I-010 | 320 | 100 |
| I-011 | 1280 | 100 |
| I-019 | 320 | 90 |
| I-023 | 320 | 90 |
| I-024 | 320 | 90 |
| I-025 | 1280 | 90 |
| I-027 | 1280 | 90 |
| I-028 | 1280 | 90 |
| I-029 | 320 | 90 |
| I-030 | 320 | 90 |
| I-031 | 320 | 90 |
| I-032 | 1280 | 100 |
| I-034 | 1280 | 90 |
| I-035 | 320 | 90 |
| I-036 | 1280 | 100 |
| I-038 | 1280 | 90 |
| I-040 | 1280 | 100 |
| I-041 | 1280 | 100 |
| I-042 | 1280 | 90 |
| I-044 | 1280 | 90 |
| I-045 | 1280 | 90 |
| I-046 | 1280 | 90 |
| I-047 | 1280 | 90 |
| I-050 | 320 | 90 |
| I-051 | 320 | 90 |
| I-052 | 320 | 90 |
| I-053 | 1280 | 90 |
| I-054 | 1280 | 90 |
| I-055 | 320 | 90 |
| I-056 | 320 | 90 |
| I-057 | 320 | 90 |
| 1-058 | 320 | 90 |
| I-059 | 320 | 90 |
| I-060 | 320 | 90 |
| I-062 | 1280 | 90 |
| I-063 | 1280 | 90 |
| I-064 | 1280 | 90 |
| I-065 | 1280 | 90 |
| I-066 | 1280 | 90 |
| I-069 | 1280 | 90 |
| I-070 | 320 | 90 |
| I-071 | 320 | 90 |
| I-072 | 320 | 90 |
| I-073 | 320 | 90 |
| I-074 | 320 | 90 |
| I-075 | 320 | 90 |
| I-076 | 1280 | 90 |
| I-083 | 320 | 90 |
| I-086 | 1280 | 100 |
| I-087 | 1280 | 90 |
| I-088 | 1280 | 90 |
| I-097 | 1280 | 90 |
| I-104 | 320 | 90 |
| I-115 | 1280 | 90 |
| I-111 | 1280 | 90 |
| I-112 | 1280 | 90 |
| I-113 | 1280 | 90 |
| I-108 | 1280 | 90 |
| I-109 | 1280 | 90 |
| I-122 | 320 | 90 |
| I-123 | 320 | 90 |
| I-120 | 320 | 90 |
| I-119 | 320 | 90 |
| I-121 | 320 | 90 |
| I-124 | 320 | 90 |
| I-117 | 320 | 90 |
| I-126 | 1280 | 90 |
| I-127 | 320 | 90 |
| I-128 | 320 | 90 |
| I-131 | 1280 | 90 |
| I-125 | 320 | 90 |
| I-130 | 320 | 90 |
| I-129 | 320 | 90 |
| I-137 | 1280 | 100 |
| I-141 | 320 | 90 |
| I-140 | 1280 | 90 |
| I-138 | 320 | 90 |
| I-139 | 320 | 90 |
| I-134 | 1280 | 90 |
| I-135 | 320 | 90 |
| I-143 | 320 | 90 |
| I-144 | 320 | 90 |
| I-142 | 320 | 90 |
| I-145 | 320 | 90 |
| I-147 | 1280 | 90 |
| I-149 | 1280 | 90 |
| I-152 | 320 | 90 |
| I-153 | 320 | 90 |
| I-154 | 1280 | 90 |
| I-155 | 320 | 90 |
| I-156 | 320 | 90 |
| I-157 | 320 | 90 |
| I-159 | 1280 | 90 |
| I-160 | 1280 | 90 |
| I-162 | 1280 | 90 |
| I-163 | 1280 | 90 |
| I-164 | 1280 | 90 |
| I-165 | 1280 | 90 |
| I-166 | 1280 | 90 |
| I-167 | 320 | 90 |
| I-168 | 320 | 90 |
| I-169 | 1280 | 90 |
| I-171 | 1280 | 90 |
| I-174 | 1280 | 90 |

**Table A10: pre-emergence activity at 1280g/ha and 320 g/ha against VERPE in %**

| Example number | application rate [g/ha] | VERPE |
|---|---|---|
| I-002 | 1280 | 90 |
| I-003 | 1280 | 90 |
| I-005 | 1280 | 90 |
| I-007 | 1280 | 90 |
| I-008 | 1280 | 90 |
| I-009 | 1280 | 90 |
| I-010 | 1280 | 90 |
| I-011 | 1280 | 90 |
| I-013 | 1280 | 90 |
| I-019 | 1280 | 90 |
| I-023 | 320 | 90 |
| I-024 | 320 | 90 |
| I-025 | 1280 | 90 |
| I-027 | 1280 | 90 |
| I-029 | 1280 | 90 |
| I-030 | 1280 | 90 |
| I-031 | 320 | 90 |
| I-033 | 1280 | 90 |
| I-036 | 1280 | 90 |
| I-038 | 1280 | 90 |
| I-040 | 1280 | 90 |
| I**-**041 | 320 | 90 |
| I-042 | 1280 | 90 |
| I-050 | 1280 | 90 |
| I-051 | 320 | 90 |
| I-052 | 320 | 90 |
| I-053 | 1280 | 90 |
| I-055 | 320 | 90 |
| I-056 | 1280 | 90 |
| I-057 | 1280 | 90 |
| 1-058 | 320 | 100 |
| I-059 | 320 | 90 |
| I-060 | 1280 | 90 |
| I-062 | 1280 | 90 |
| I-063 | 1280 | 90 |
| I-064 | 320 | 90 |
| I-065 | 320 | 90 |
| I-066 | 1280 | 90 |
| I-069 | 320 | 90 |
| I-070 | 1280 | 90 |
| I-071 | 320 | 90 |
| I-072 | 320 | 90 |
| I-073 | 320 | 90 |
| I-074 | 320 | 90 |
| I-075 | 320 | 90 |
| I-083 | 1280 | 90 |
| I-088 | 1280 | 90 |
| I-091 | 320 | 90 |
| I-104 | 1280 | 90 |
| I-116 | 1280 | 90 |
| I-112 | 1280 | 90 |
| I-113 | 1280 | 90 |
| I-108 | 1280 | 90 |
| I-109 | 1280 | 90 |
| I-107 | 320 | 90 |
| I-122 | 1280 | 90 |
| I-123 | 320 | 90 |
| I-120 | 320 | 90 |
| I-119 | 320 | 90 |
| I**-**121 | 1280 | 90 |
| I-124 | 320 | 90 |
| I-117 | 320 | 90 |
| I-127 | 1280 | 90 |
| I-128 | 320 | 90 |
| I-132 | 1280 | 90 |
| I-125 | 1280 | 90 |
| I-130 | 1280 | 90 |
| I-129 | 320 | 90 |
| I-137 | 320 | 90 |
| I-141 | 1280 | 90 |
| I-139 | 1280 | 90 |
| I-143 | 320 | 90 |
| I-144 | 320 | 90 |
| I-142 | 320 | 90 |
| I-145 | 1280 | 90 |
| I-147 | 320 | 90 |
| I-149 | 1280 | 90 |
| I-152 | 1280 | 90 |
| I-153 | 1280 | 90 |
| I-154 | 1280 | 90 |
| I-155 | 320 | 90 |
| I-156 | 1280 | 90 |
| I-157 | 1280 | 90 |
| I-162 | 1280 | 90 |
| I-163 | 1280 | 90 |
| I-164 | 1280 | 90 |
| I-165 | 1280 | 90 |
| I-166 | 1280 | 90 |
| I-167 | 1280 | 90 |
| I-168 | 320 | 90 |
| I-169 | 320 | 90 |
| I-171 | 320 | 90 |
| I-174 | 320 | 90 |

**Table A11: pre-emergence activity at 1280g/ha and 320 g/ha against AMARE in %**

| Example number | application rate [g/ha] | AMARE |
|---|---|---|
| I-002 | 1280 | 90 |
| I-003 | 1280 | 90 |
| I-005 | 320 | 90 |
| I-006 | 320 | 90 |
| I-007 | 1280 | 90 |
| I-008 | 1280 | 90 |
| I-009 | 1280 | 90 |
| I-010 | 1280 | 100 |
| I-011 | 1280 | 90 |
| I-013 | 1280 | 90 |
| I-019 | 320 | 90 |
| I-023 | 1280 | 90 |
| I-024 | 1280 | 90 |
| I-025 | 1280 | 90 |
| I-027 | 320 | 90 |
| I-028 | 320 | 90 |
| I-029 | 320 | 90 |
| I-030 | 320 | 90 |
| I-031 | 320 | 90 |
| I-032 | 320 | 90 |
| I-033 | 320 | 90 |
| I-034 | 1280 | 90 |
| I-035 | 1280 | 90 |
| I-036 | 320 | 90 |
| 1-038 | 320 | 90 |
| I-040 | 320 | 90 |
| I**-**041 | 320 | 90 |
| I-042 | 320 | 90 |
| I-044 | 320 | 90 |
| I-045 | 1280 | 90 |
| I-046 | 320 | 90 |
| I-047 | 1280 | 90 |
| I-048 | 1280 | 90 |
| I-050 | 320 | 90 |
| I-051 | 320 | 90 |
| I-052 | 320 | 90 |
| I-053 | 320 | 90 |
| I-054 | 320 | 90 |
| I-055 | 320 | 90 |
| I-056 | 320 | 90 |
| I-057 | 320 | 90 |
| 1-058 | 1280 | 100 |
| I-059 | 320 | 90 |
| I-060 | 320 | 90 |
| I-062 | 320 | 90 |
| I-063 | 320 | 90 |
| I-064 | 320 | 90 |
| I-065 | 320 | 90 |
| I-066 | 320 | 90 |
| I-069 | 320 | 90 |
| I-070 | 1280 | 90 |
| I-071 | 320 | 90 |
| I-072 | 320 | 90 |
| I-073 | 1280 | 90 |
| I-074 | 320 | 90 |
| I-075 | 320 | 90 |
| I-081 | 1280 | 90 |
| I-083 | 320 | 100 |
| I-084 | 1280 | 90 |
| I-086 | 1280 | 90 |
| I-087 | 1280 | 90 |
| I-088 | 320 | 90 |
| I-089 | 1280 | 90 |
| I-091 | 1280 | 90 |
| I-097 | 1280 | 90 |
| I-100 | 1280 | 90 |
| I-101 | 1280 | 90 |
| I-103 | 1280 | 90 |
| I-106 | 320 | 90 |
| I-104 | 320 | 90 |
| I-115 | 1280 | 90 |
| I-112 | 1280 | 90 |
| I-113 | 320 | 90 |
| I-109 | 1280 | 90 |
| I-122 | 320 | 90 |
| I-123 | 320 | 90 |
| I-120 | 320 | 90 |
| I-119 | 320 | 90 |
| I**-**121 | 320 | 90 |
| I-124 | 320 | 90 |
| I-117 | 320 | 90 |
| I-126 | 320 | 90 |
| I-127 | 320 | 90 |
| I-128 | 320 | 90 |
| I-131 | 1280 | 90 |
| I-125 | 320 | 90 |
| I-130 | 320 | 90 |
| I-129 | 320 | 90 |
| I-137 | 320 | 100 |
| I-141 | 320 | 100 |
| I-140 | 1280 | 90 |
| I-138 | 1280 | 90 |
| I-139 | 320 | 100 |
| I-134 | 1280 | 90 |
| I-143 | 320 | 90 |
| I-144 | 320 | 90 |
| I-142 | 320 | 90 |
| I-145 | 1280 | 90 |
| I-147 | 320 | 90 |
| I-149 | 320 | 90 |
| I-152 | 320 | 100 |
| I-153 | 320 | 90 |
| I-154 | 320 | 90 |
| I-155 | 320 | 90 |
| I-156 | 320 | 90 |
| I-157 | 320 | 90 |
| I-159 | 1280 | 90 |
| I-160 | 1280 | 90 |
| I-162 | 1280 | 90 |
| I-163 | 1280 | 90 |
| I-164 | 320 | 90 |
| I-165 | 1280 | 90 |
| I-166 | 1280 | 90 |
| I-167 | 320 | 90 |
| I-168 | 320 | 90 |
| I-169 | 320 | 90 |
| I-171 | 320 | 90 |
| I-174 | 320 | 90 |
| I-175 | 1280 | 90 |

**Table A12: pre-emergence activity at 1280g/ha and 320 g/ha against DIGSA in %**

| Example number | application rate [g/ha] | DIGSA |
|---|---|---|
| I-002 | 1280 | 100 |
| I-003 | 1280 | 100 |
| I-010 | 320 | 100 |
| I-023 | 1280 | 100 |
| I-027 | 320 | 100 |
| I-028 | 1280 | 100 |
| I-029 | 320 | 100 |
| I-030 | 1280 | 100 |
| I-031 | 320 | 100 |
| I-032 | 1280 | 100 |
| I-033 | 1280 | 100 |
| I-035 | 1280 | 100 |
| I-036 | 1280 | 100 |
| I-038 | 1280 | 100 |
| I-040 | 1280 | 100 |
| I**-**041 | 1280 | 100 |
| I-042 | 1280 | 100 |
| I-044 | 320 | 100 |
| I-045 | 1280 | 100 |
| I-046 | 1280 | 100 |
| I-047 | 1280 | 100 |
| I-048 | 320 | 100 |
| I-050 | 320 | 100 |
| I-051 | 320 | 100 |
| I-052 | 320 | 100 |
| I-053 | 320 | 100 |
| I-054 | 1280 | 100 |
| I-055 | 320 | 100 |
| I-056 | 1280 | 100 |
| I-057 | 320 | 100 |
| I-058 | 320 | 100 |
| I-059 | 320 | 100 |
| I-060 | 320 | 100 |
| I-062 | 1280 | 100 |
| I-063 | 1280 | 100 |
| I-064 | 320 | 100 |
| I-065 | 1280 | 100 |
| I-066 | 320 | 100 |
| I-069 | 320 | 100 |
| I-070 | 1280 | 100 |
| I-071 | 320 | 100 |
| I-072 | 320 | 100 |
| I-073 | 320 | 100 |
| I-074 | 1280 | 100 |
| I-075 | 320 | 100 |
| I-080 | 1280 | 100 |
| I-081 | 1280 | 100 |
| I-083 | 320 | 100 |
| I-084 | 1280 | 100 |
| I-086 | 1280 | 100 |
| I-087 | 1280 | 100 |
| I-088 | 320 | 100 |
| I-089 | 320 | 100 |
| I-091 | 320 | 100 |
| I-097 | 1280 | 100 |
| I-098 | 1280 | 100 |
| I-099 | 1280 | 100 |
| I-100 | 1280 | 90 |
| I-101 | 1280 | 90 |
| I-106 | 1280 | 90 |
| I-104 | 320 | 100 |
| I-115 | 1280 | 100 |
| I-113 | 320 | 100 |
| I-122 | 320 | 100 |
| I-123 | 320 | 100 |
| I-120 | 320 | 100 |
| I-119 | 320 | 100 |
| I**-**121 | 320 | 100 |
| I-124 | 320 | 100 |
| I-117 | 320 | 100 |
| I-127 | 320 | 100 |
| I-128 | 320 | 100 |
| I-125 | 1280 | 90 |
| I-130 | 320 | 100 |
| I-129 | 320 | 100 |
| I-137 | 320 | 100 |
| I-141 | 1280 | 100 |
| I-138 | 1280 | 90 |
| I-139 | 320 | 100 |
| I-134 | 1280 | 100 |
| I-143 | 320 | 100 |
| I-144 | 1280 | 100 |
| I-142 | 1280 | 100 |
| I-145 | 1280 | 100 |
| I-147 | 320 | 90 |
| I-149 | 1280 | 100 |
| I-152 | 320 | 90 |
| I-153 | 320 | 90 |
| I-154 | 320 | 90 |
| I-155 | 1280 | 100 |
| I-156 | 320 | 90 |
| I-157 | 1280 | 100 |
| I-159 | 1280 | 90 |
| I-160 | 1280 | 90 |
| I-164 | 1280 | 100 |
| I-167 | 1280 | 100 |
| I-168 | 320 | 100 |
| I-171 | 1280 | 100 |
| I-174 | 320 | 100 |

As the results show, various compounds of the general formula (I) according the invention have very good herbicidal pre-emergence efficacy against a broad spectrum of mono- and dicotyledonous weeds such as Abutilon theophrasti (ABUTH), Alopecurus myosuroides (ALOMY), Amaranthus retroflexus (AMARE), Digitaria sanguinalis (DIGSA), Echinochloa crus-galli (ECHCG), Kochia scoparia (KCHSC), Lolium rigidum (LOLRI), Matricaria inodora (MATIN), Poa annua (POAAN), Setaria viridis (SETVI), Stellaria media (STEME) and Veronica persica (VERPE) at an application rate of 1280 g or below of active ingredient per hectare.

### B. Herbicidal post-emergence action

Seeds of mono- and dicotyledonous weed plants were placed in plastic pots in sandy loam soil (doubly sown with in each case one species of mono- or dicotyledonous weed plants per pot), covered with soil and cultivated in a greenhouse under controlled growth conditions. 2 to 3 weeks after sowing, the test plants were treated at the one-leaf stage. The compounds of the invention, formulated in the form of wettable powders (WP) or as emulsion concentrates (EC), were applied onto the green parts of the plants as aqueous suspension or emulsion with addition of 0.5% additive at a water application rate of 600 liters per hectare (converted). After the test plants had been kept in the greenhouse under optimum growth conditions for about 3 weeks, the activity of the preparations was rated visually in comparison to untreated controls.

For example, 100% activity = the plants have died, 0% activity = like control plants.

Tables B1 to B12, below, show the effects of selected compounds of the general formula (I) according to Table 1 on various harmful plants and an application rate corresponding to 1280 g/ha or below obtained by the experimental procedure mentioned above.

**Table B1: post-emergence activity at 1280g/ha and 320 g/ha against ABUTH in %**

| Example number | application rate [g/ha] | ABUTH |
|---|---|---|
| I-002 | 1280 | 90 |
| I-010 | 320 | 90 |
| I-019 | 320 | 90 |
| I-024 | 320 | 90 |
| I-025 | 1280 | 90 |
| I-029 | 1280 | 90 |
| I-031 | 320 | 90 |
| I-032 | 1280 | 90 |
| I-040 | 1280 | 90 |
| I**-**041 | 1280 | 90 |
| I-042 | 1280 | 90 |
| I-048 | 1280 | 90 |
| I-056 | 1280 | 90 |
| I-057 | 1280 | 90 |
| I-059 | 1280 | 90 |
| I-060 | 1280 | 90 |
| I-066 | 1280 | 90 |
| I-069 | 1280 | 90 |
| I-071 | 1280 | 90 |
| I-072 | 1280 | 90 |
| I-073 | 1280 | 90 |
| I-083 | 320 | 90 |
| I-085 | 1280 | 90 |
| I-088 | 320 | 90 |
| I-089 | 1280 | 90 |
| I-104 | 320 | 90 |
| I-122 | 1280 | 90 |
| I-123 | 1280 | 90 |
| I-120 | 1280 | 90 |
| I-119 | 1280 | 90 |
| I-124 | 1280 | 90 |
| I-128 | 320 | 90 |
| I-130 | 1280 | 90 |
| I-129 | 1280 | 90 |
| I-137 | 1280 | 90 |
| I-141 | 1280 | 90 |
| I-138 | 1280 | 90 |
| I-139 | 1280 | 90 |
| I-143 | 1280 | 90 |
| I-144 | 1280 | 90 |
| I-142 | 1280 | 90 |
| I-152 | 320 | 90 |
| I-166 | 1280 | 90 |
| I-174 | 1280 | 90 |
| I-178 | 1280 | 90 |

**Table B2: post-emergence activity at 1280g/ha and 320 g/ha against ALOMY in %**

| Example number | application rate [g/ha] | ALOMY |
|---|---|---|
| I-002 | 1280 | 100 |
| I-003 | 320 | 100 |
| I-005 | 1280 | 90 |
| I-007 | 320 | 90 |
| I-008 | 1280 | 90 |
| I-019 | 1280 | 90 |
| I-024 | 320 | 90 |
| I-029 | 1280 | 90 |
| I-031 | 1280 | 90 |
| I**-**041 | 1280 | 90 |
| I-046 | 1280 | 90 |
| I-058 | 1280 | 90 |
| I-059 | 1280 | 90 |
| I-060 | 1280 | 90 |
| I-064 | 1280 | 90 |
| I-066 | 1280 | 90 |
| I-069 | 1280 | 90 |
| I-071 | 320 | 90 |
| I-072 | 1280 | 90 |
| I-083 | 1280 | 90 |
| I-104 | 1280 | 90 |
| I-113 | 1280 | 90 |
| I-122 | 1280 | 90 |
| I-120 | 1280 | 90 |
| I-119 | 1280 | 90 |
| I-124 | 1280 | 90 |
| I-117 | 1280 | 90 |
| I-130 | 1280 | 90 |
| I-144 | 1280 | 90 |

**Table B3: post-emergence activity at 1280g/ha and 320 g/ha against ECHCG in %**

| Example number | application rate [g/ha] | ECHCG |
|---|---|---|
| I-002 | 1280 | 100 |
| I-004 | 1280 | 90 |
| I-005 | 1280 | 90 |
| I-007 | 1280 | 90 |
| I-008 | 1280 | 90 |
| I-009 | 320 | 90 |
| I-010 | 1280 | 90 |
| 1-011 | 1280 | 90 |
| I-019 | 320 | 100 |
| I-023 | 1280 | 90 |
| I-024 | 320 | 90 |
| I-025 | 1280 | 90 |
| I-027 | 1280 | 90 |
| I-029 | 1280 | 90 |
| I-030 | 1280 | 90 |
| I-031 | 320 | 100 |
| I-033 | 1280 | 90 |
| I-036 | 1280 | 90 |
| I-040 | 320 | 90 |
| I**-**041 | 320 | 100 |
| I-042 | 1280 | 90 |
| I-044 | 1280 | 90 |
| I-046 | 1280 | 90 |
| I-047 | 1280 | 90 |
| I-048 | 1280 | 90 |
| I-050 | 1280 | 90 |
| I-051 | 1280 | 90 |
| I-052 | 1280 | 90 |
| I-055 | 1280 | 90 |
| I-057 | 1280 | 90 |
| I-058 | 320 | 90 |
| I-059 | 320 | 90 |
| I-060 | 1280 | 90 |
| I-064 | 1280 | 90 |
| I-065 | 1280 | 90 |
| I-066 | 320 | 90 |
| I-069 | 320 | 90 |
| I-071 | 1280 | 100 |
| I-072 | 320 | 90 |
| I-073 | 1280 | 90 |
| I-075 | 1280 | 90 |
| I-083 | 1280 | 90 |
| I-085 | 1280 | 90 |
| I-088 | 320 | 90 |
| I-089 | 320 | 90 |
| I-090 | 1280 | 90 |
| I-104 | 320 | 90 |
| I-112 | 320 | 100 |
| I-109 | 1280 | 90 |
| I-107 | 1280 | 90 |
| I-122 | 1280 | 100 |
| I-123 | 1280 | 90 |
| I-120 | 1280 | 90 |
| I-119 | 320 | 90 |
| I**-**121 | 1280 | 90 |
| I-124 | 320 | 90 |
| I-117 | 1280 | 90 |
| I-127 | 1280 | 90 |
| I-128 | 320 | 90 |
| I-125 | 1280 | 90 |
| I-130 | 320 | 90 |
| I-129 | 320 | 90 |
| I-137 | 320 | 90 |
| I-141 | 1280 | 90 |
| I-139 | 1280 | 90 |
| I-143 | 320 | 90 |
| I-144 | 320 | 90 |
| I-142 | 320 | 90 |
| I-145 | 1280 | 90 |
| I-155 | 1280 | 90 |
| I-156 | 1280 | 90 |
| I-174 | 1280 | 90 |

**Table B4: post-emergence activity at 1280g/ha and 320 g/ha against KCHSC in %**

| Example number | application rate [g/ha] | KCHSC |
|---|---|---|
| I-002 | 1280 | 90 |
| I-003 | 320 | 90 |
| I-004 | 1280 | 90 |
| I-005 | 1280 | 90 |
| I-006 | 1280 | 90 |
| I-007 | 1280 | 90 |
| I-008 | 1280 | 90 |
| I-009 | 1280 | 90 |
| I-010 | 320 | 90 |
| I-011 | 1280 | 90 |
| I-013 | 1280 | 90 |
| I-019 | 320 | 90 |
| I-023 | 1280 | 90 |
| I-024 | 320 | 90 |
| I-025 | 320 | 90 |
| I-027 | 320 | 90 |
| I-028 | 320 | 90 |
| I-029 | 320 | 90 |
| I-030 | 320 | 90 |
| I-031 | 320 | 90 |
| I-032 | 1280 | 90 |
| I-033 | 1280 | 90 |
| I-035 | 1280 | 90 |
| I-036 | 1280 | 90 |
| I-038 | 1280 | 90 |
| I-040 | 320 | 90 |
| I**-**041 | 320 | 90 |
| I-042 | 320 | 90 |
| I-044 | 320 | 90 |
| I-045 | 1280 | 90 |
| I-046 | 320 | 90 |
| I-047 | 320 | 90 |
| I-048 | 320 | 90 |
| I-050 | 320 | 90 |
| I-051 | 1280 | 90 |
| I-055 | 1280 | 90 |
| I-056 | 320 | 90 |
| I-057 | 320 | 90 |
| I-058 | 320 | 90 |
| I-059 | 320 | 90 |
| I-060 | 320 | 90 |
| I-063 | 1280 | 90 |
| I-064 | 1280 | 90 |
| I-065 | 1280 | 90 |
| I-066 | 320 | 90 |
| I-069 | 1280 | 90 |
| I-070 | 1280 | 90 |
| I-071 | 320 | 90 |
| I-072 | 320 | 90 |
| I-073 | 320 | 90 |
| I-074 | 320 | 90 |
| I-075 | 320 | 90 |
| I-081 | 1280 | 90 |
| I-083 | 320 | 90 |
| I-084 | 1280 | 90 |
| I-086 | 1280 | 90 |
| I-087 | 1280 | 90 |
| I-088 | 320 | 90 |
| I-089 | 320 | 90 |
| I-090 | 320 | 90 |
| I-091 | 320 | 90 |
| I-099 | 1280 | 90 |
| I-100 | 1280 | 90 |
| I-101 | 1280 | 90 |
| I-103 | 1280 | 90 |
| I-106 | 1280 | 90 |
| I-104 | 320 | 90 |
| I-116 | 1280 | 90 |
| I-111 | 1280 | 90 |
| I-113 | 320 | 90 |
| I-122 | 320 | 90 |
| I-123 | 320 | 90 |
| I-120 | 320 | 90 |
| I-119 | 320 | 90 |
| I-124 | 320 | 90 |
| I-117 | 320 | 90 |
| I-127 | 320 | 90 |
| I-128 | 320 | 90 |
| I-125 | 320 | 90 |
| I-130 | 320 | 90 |
| I-129 | 320 | 90 |
| I-137 | 320 | 90 |
| I-141 | 1280 | 90 |
| I-138 | 320 | 90 |
| I-139 | 1280 | 90 |
| I-143 | 1280 | 90 |
| I-144 | 1280 | 90 |
| I-152 | 1280 | 90 |
| I-159 | 1280 | 90 |
| I-164 | 1280 | 90 |
| I-168 | 1280 | 90 |
| I-174 | 320 | 90 |
| I-175 | 320 | 90 |
| I-181 | 1280 | 90 |
| I-176 | 1280 | 90 |

**Table B5: post-emergence activity at 1280g/ha and 320 g/ha against LOLRI in %**

| Example number | application rate [g/ha] | LOLRI |
|---|---|---|
| I-003 | 1280 | 90 |
| I-009 | 1280 | 90 |
| I-010 | 1280 | 90 |
| I-023 | 1280 | 90 |
| I-024 | 320 | 90 |
| I-025 | 1280 | 90 |
| I-029 | 1280 | 90 |
| I-031 | 320 | 100 |
| I**-**041 | 1280 | 90 |
| I-048 | 1280 | 90 |
| I-059 | 1280 | 90 |
| I-060 | 1280 | 90 |
| I-064 | 1280 | 90 |
| I-066 | 1280 | 90 |
| I-069 | 1280 | 100 |
| I-070 | 1280 | 90 |
| I-071 | 320 | 90 |
| I-083 | 1280 | 90 |
| I-088 | 1280 | 90 |
| I-104 | 320 | 90 |
| I-113 | 1280 | 90 |
| I-122 | 1280 | 90 |
| I-120 | 1280 | 90 |
| I-119 | 1280 | 90 |
| I-124 | 1280 | 90 |
| I-127 | 1280 | 90 |
| I-128 | 320 | 90 |
| I-130 | 1280 | 90 |
| I-129 | 1280 | 100 |
| I-143 | 1280 | 90 |
| I-144 | 1280 | 90 |
| I-142 | 1280 | 90 |
| I-152 | 1280 | 90 |
| I-156 | 1280 | 90 |

**Table B6: post-emergence activity at 1280g/ha and 320 g/ha against MATIN in %**

| Example number | application rate [g/ha] | MATIN |
|---|---|---|
| I-007 | 1280 | 90 |
| I-008 | 1280 | 90 |
| I-009 | 1280 | 90 |
| I-010 | 1280 | 90 |
| I-024 | 320 | 90 |
| I-031 | 1280 | 90 |
| I-057 | 1280 | 90 |
| I-059 | 1280 | 90 |
| I-060 | 1280 | 90 |
| I-083 | 320 | 90 |
| I-088 | 1280 | 90 |
| I-104 | 1280 | 90 |
| I-113 | 1280 | 90 |
| I-122 | 1280 | 90 |
| I-123 | 1280 | 90 |
| I-120 | 1280 | 90 |
| I-119 | 1280 | 90 |
| I**-**121 | 1280 | 90 |
| I-127 | 320 | 90 |
| I-128 | 1280 | 90 |
| I-130 | 1280 | 90 |
| I-129 | 1280 | 90 |
| I-137 | 1280 | 90 |

**Table B7: post-emergence activity at 1280g/ha and 320 g/ha against POAAN in %**

| Example number | application rate [g/ha] | POAAN |
|---|---|---|
| I-002 | 1280 | 100 |
| I-003 | 320 | 100 |
| I-005 | 1280 | 90 |
| I-007 | 1280 | 90 |
| I-008 | 1280 | 90 |
| I-010 | 1280 | 90 |
| I-011 | 1280 | 90 |
| I-013 | 1280 | 90 |
| I-019 | 320 | 90 |
| I-023 | 320 | 90 |
| I-024 | 320 | 90 |
| I-025 | 320 | 90 |
| I-027 | 1280 | 90 |
| I-029 | 1280 | 90 |
| I-030 | 1280 | 90 |
| I-031 | 320 | 90 |
| I-040 | 1280 | 90 |
| I**-**041 | 320 | 90 |
| I-042 | 1280 | 90 |
| I-044 | 320 | 100 |
| I-045 | 1280 | 90 |
| I-046 | 1280 | 90 |
| I-047 | 320 | 90 |
| I-048 | 320 | 90 |
| I-051 | 1280 | 90 |
| I-056 | 1280 | 90 |
| I-057 | 320 | 90 |
| I-058 | 320 | 90 |
| I-059 | 320 | 90 |
| I-060 | 1280 | 90 |
| I-064 | 1280 | 90 |
| I-065 | 1280 | 90 |
| I-066 | 320 | 90 |
| I-069 | 1280 | 100 |
| I-070 | 1280 | 90 |
| I-071 | 1280 | 90 |
| I-072 | 320 | 90 |
| I-073 | 1280 | 90 |
| I-074 | 320 | 90 |
| I-083 | 320 | 90 |
| I-085 | 1280 | 90 |
| I-088 | 320 | 90 |
| I-089 | 1280 | 90 |
| I-091 | 1280 | 90 |
| I-106 | 1280 | 90 |
| I-104 | 320 | 90 |
| I-115 | 1280 | 90 |
| I-113 | 320 | 90 |
| I-122 | 320 | 90 |
| I-123 | 1280 | 90 |
| I-120 | 320 | 90 |
| I-119 | 1280 | 90 |
| I**-**121 | 320 | 90 |
| I-124 | 320 | 90 |
| I-117 | 1280 | 90 |
| I-127 | 320 | 90 |
| I-128 | 1280 | 90 |
| I-125 | 320 | 90 |
| I-130 | 320 | 90 |
| I-129 | 320 | 90 |
| I-137 | 320 | 90 |
| I-141 | 1280 | 90 |
| I-138 | 1280 | 90 |
| I-139 | 320 | 90 |
| I-134 | 1280 | 90 |
| I-143 | 1280 | 90 |
| I-144 | 1280 | 90 |
| I-142 | 1280 | 90 |
| I-149 | 1280 | 90 |
| I-152 | 1280 | 90 |

**Table B8: post-emergence activity at 1280g/ha and 320 g/ha against SETVI in %**

| Example number | application rate [g/ha] | SETVI |
|---|---|---|
| I-003 | 1280 | 90 |
| I-005 | 320 | 90 |
| I-007 | 1280 | 90 |
| I-008 | 1280 | 90 |
| I-009 | 1280 | 90 |
| I-010 | 1280 | 90 |
| I-011 | 1280 | 90 |
| I-019 | 1280 | 90 |
| I-024 | 320 | 90 |
| I-025 | 1280 | 90 |
| I-027 | 1280 | 90 |
| I-029 | 1280 | 90 |
| I-031 | 320 | 90 |
| I-040 | 1280 | 90 |
| I**-**041 | 1280 | 90 |
| I-044 | 320 | 90 |
| I-048 | 1280 | 90 |
| I-051 | 1280 | 90 |
| I-052 | 1280 | 90 |
| I-055 | 1280 | 90 |
| I-056 | 1280 | 90 |
| I-057 | 1280 | 90 |
| I-058 | 1280 | 90 |
| I-059 | 320 | 90 |
| I-060 | 320 | 90 |
| I-064 | 1280 | 90 |
| I-065 | 1280 | 90 |
| I-066 | 1280 | 90 |
| I-069 | 320 | 90 |
| I-071 | 320 | 90 |
| I-072 | 1280 | 90 |
| I-074 | 1280 | 90 |
| I-075 | 1280 | 90 |
| I-083 | 1280 | 90 |
| I-088 | 1280 | 90 |
| I-091 | 1280 | 90 |
| I-104 | 1280 | 90 |
| I-122 | 1280 | 90 |
| I-123 | 1280 | 90 |
| I-120 | 1280 | 90 |
| I-119 | 1280 | 90 |
| I**-**121 | 1280 | 90 |
| I-124 | 1280 | 90 |
| I-127 | 1280 | 90 |
| I-128 | 1280 | 90 |
| I-125 | 1280 | 90 |
| I-130 | 1280 | 90 |
| I-129 | 1280 | 90 |
| I-137 | 1280 | 90 |
| I-139 | 1280 | 90 |
| I-143 | 320 | 90 |
| I-144 | 1280 | 90 |
| I-142 | 320 | 90 |
| I-147 | 1280 | 90 |
| I-152 | 1280 | 90 |
| I-153 | 1280 | 90 |
| I-154 | 1280 | 90 |
| I-164 | 1280 | 90 |
| I-174 | 1280 | 90 |

**Table B9: post-emergence activity at 1280g/ha and 320 g/ha against STEME in %**

| Example number | application rate [g/ha] | STEME |
|---|---|---|
| I-003 | 1280 | 90 |
| I-005 | 1280 | 90 |
| I-007 | 1280 | 90 |
| I-009 | 1280 | 90 |
| I-010 | 320 | 90 |
| I-011 | 1280 | 90 |
| I-019 | 320 | 90 |
| I-023 | 1280 | 90 |
| I-024 | 1280 | 90 |
| I-025 | 320 | 90 |
| I-027 | 320 | 90 |
| I-028 | 1280 | 90 |
| I-029 | 320 | 90 |
| I-030 | 320 | 90 |
| I-031 | 320 | 90 |
| I-032 | 1280 | 90 |
| I-033 | 320 | 90 |
| I-034 | 320 | 90 |
| I-035 | 320 | 90 |
| I-036 | 320 | 90 |
| I-040 | 320 | 90 |
| I**-**041 | 320 | 90 |
| I-042 | 1280 | 90 |
| I-044 | 1280 | 90 |
| I-045 | 1280 | 90 |
| I-046 | 1280 | 90 |
| I-047 | 1280 | 90 |
| I-048 | 320 | 90 |
| I-055 | 1280 | 90 |
| I-056 | 1280 | 90 |
| I-057 | 1280 | 90 |
| I-058 | 320 | 90 |
| I-059 | 1280 | 90 |
| I-060 | 320 | 90 |
| I-062 | 320 | 90 |
| I-064 | 320 | 90 |
| I-065 | 1280 | 90 |
| I-066 | 320 | 90 |
| I-069 | 320 | 90 |
| I-070 | 1280 | 90 |
| I-071 | 320 | 90 |
| I-072 | 320 | 90 |
| I-073 | 320 | 90 |
| I-074 | 1280 | 90 |
| I-075 | 1280 | 90 |
| I-081 | 1280 | 90 |
| I-083 | 320 | 90 |
| I-084 | 1280 | 90 |
| I-085 | 1280 | 90 |
| I-088 | 320 | 90 |
| I-089 | 1280 | 90 |
| I-090 | 320 | 90 |
| I-091 | 1280 | 90 |
| I-092 | 1280 | 90 |
| I-100 | 1280 | 90 |
| I-103 | 1280 | 90 |
| I-106 | 1280 | 90 |
| I-104 | 320 | 90 |
| I-113 | 1280 | 90 |
| I-122 | 1280 | 90 |
| I-123 | 1280 | 90 |
| I-120 | 320 | 90 |
| I-119 | 1280 | 90 |
| I-124 | 320 | 90 |
| I-117 | 1280 | 90 |
| I-127 | 1280 | 90 |
| I-128 | 320 | 90 |
| I-125 | 1280 | 90 |
| I-130 | 320 | 90 |
| I-129 | 1280 | 90 |
| I-137 | 1280 | 90 |
| I-141 | 1280 | 90 |
| I-138 | 1280 | 90 |
| I-139 | 1280 | 90 |
| I-135 | 1280 | 90 |
| I-143 | 320 | 90 |
| I-144 | 1280 | 90 |
| I-142 | 1280 | 90 |
| I-145 | 1280 | 90 |
| I-147 | 1280 | 90 |
| I-152 | 320 | 90 |
| I-153 | 1280 | 90 |
| I-154 | 320 | 90 |
| I-155 | 1280 | 90 |
| I-156 | 320 | 90 |
| I-157 | 1280 | 90 |
| I-174 | 320 | 90 |
| I-175 | 1280 | 90 |

**Table B 10: post-emergence activity at 1280g/ha and 320 g/ha against VERPE in %**

| Example number | application rate [g/ha] | VERPE |
|---|---|---|
| I-002 | 1280 | 100 |
| I-003 | 320 | 100 |
| I-004 | 1280 | 90 |
| I-005 | 1280 | 90 |
| I-006 | 1280 | 90 |
| I-007 | 320 | 90 |
| I-008 | 320 | 90 |
| I-009 | 1280 | 90 |
| I-010 | 320 | 90 |
| 1-011 | 1280 | 90 |
| I-019 | 320 | 90 |
| I-023 | 1280 | 90 |
| I-024 | 320 | 90 |
| I-025 | 1280 | 90 |
| I-027 | 1280 | 90 |
| I-029 | 1280 | 90 |
| I-030 | 1280 | 90 |
| I-031 | 320 | 90 |
| I-040 | 320 | 90 |
| I**-**041 | 320 | 90 |
| I-042 | 1280 | 90 |
| I-044 | 1280 | 90 |
| I-045 | 1280 | 90 |
| I-046 | 320 | 90 |
| I-047 | 320 | 90 |
| I-048 | 320 | 90 |
| I-056 | 320 | 90 |
| I-057 | 320 | 90 |
| I-058 | 320 | 90 |
| I-059 | 320 | 90 |
| I-060 | 320 | 90 |
| I-062 | 320 | 90 |
| I-063 | 1280 | 90 |
| I-064 | 320 | 90 |
| I-065 | 320 | 90 |
| I-066 | 320 | 90 |
| I-069 | 320 | 90 |
| I-070 | 1280 | 90 |
| I-071 | 320 | 90 |
| I-072 | 320 | 90 |
| I-073 | 320 | 90 |
| I-074 | 320 | 90 |
| I-075 | 320 | 90 |
| I-083 | 320 | 90 |
| I-084 | 1280 | 90 |
| I-085 | 320 | 100 |
| I-088 | 1280 | 90 |
| I-089 | 1280 | 90 |
| I-090 | 1280 | 90 |
| I-104 | 320 | 90 |
| I-122 | 320 | 90 |
| I-123 | 320 | 90 |
| I-120 | 320 | 90 |
| I-119 | 320 | 90 |
| I-124 | 320 | 90 |
| I-117 | 320 | 90 |
| I-126 | 1280 | 90 |
| I-127 | 1280 | 90 |
| I-128 | 320 | 90 |
| I-131 | 1280 | 90 |
| I-132 | 1280 | 90 |
| I-125 | 320 | 90 |
| I-130 | 320 | 90 |
| I-129 | 320 | 90 |
| I-137 | 1280 | 90 |
| I-139 | 1280 | 90 |
| I-135 | 1280 | 90 |
| I-143 | 1280 | 90 |
| I-144 | 320 | 90 |
| I-142 | 320 | 90 |
| I-154 | 1280 | 90 |
| I-157 | 1280 | 90 |
| I-160 | 1280 | 90 |
| I-168 | 1280 | 90 |
| I-171 | 1280 | 90 |
| I-174 | 320 | 90 |
| I-178 | 320 | 90 |

**Table B11: post-emergence activity at 1280g/ha and 320 g/ha against AMARE in %**

| Example number | application rate [g/ha] | AMARE |
|---|---|---|
| I-002 | 320 | 90 |
| I-003 | 1280 | 90 |
| I-004 | 1280 | 100 |
| I-005 | 1280 | 90 |
| I-006 | 1280 | 90 |
| I-007 | 1280 | 90 |
| I-009 | 1280 | 90 |
| I-010 | 320 | 90 |
| I-011 | 1280 | 90 |
| I-013 | 1280 | 90 |
| I-019 | 1280 | 100 |
| I-024 | 320 | 100 |
| I-025 | 320 | 90 |
| I-027 | 1280 | 90 |
| I-029 | 320 | 90 |
| I-030 | 1280 | 90 |
| I-031 | 1280 | 100 |
| I-032 | 1280 | 90 |
| I-033 | 1280 | 90 |
| I-035 | 1280 | 90 |
| I-040 | 320 | 90 |
| I**-**041 | 320 | 90 |
| I-042 | 320 | 90 |
| I-043 | 1280 | 90 |
| I-044 | 1280 | 90 |
| I-045 | 320 | 90 |
| I-046 | 1280 | 100 |
| I-047 | 320 | 90 |
| I-048 | 320 | 90 |
| I-050 | 1280 | 90 |
| I-051 | 1280 | 90 |
| I-052 | 1280 | 90 |
| I-053 | 1280 | 90 |
| I-055 | 320 | 90 |
| I-056 | 1280 | 90 |
| I-057 | 1280 | 90 |
| I-058 | 1280 | 90 |
| I-059 | 1280 | 90 |
| I-060 | 320 | 90 |
| I-062 | 320 | 90 |
| I-064 | 1280 | 90 |
| I-066 | 320 | 90 |
| I-069 | 320 | 90 |
| I-070 | 1280 | 90 |
| I-071 | 320 | 90 |
| I-072 | 1280 | 90 |
| I-073 | 1280 | 90 |
| I-074 | 1280 | 90 |
| I-075 | 1280 | 90 |
| I-080 | 1280 | 90 |
| I-081 | 1280 | 90 |
| I-083 | 320 | 90 |
| I-084 | 1280 | 90 |
| I-085 | 1280 | 90 |
| I-086 | 1280 | 90 |
| I-088 | 320 | 90 |
| I-089 | 320 | 90 |
| I-090 | 1280 | 90 |
| I-091 | 1280 | 90 |
| I-092 | 1280 | 90 |
| I-105 | 1280 | 90 |
| I-104 | 320 | 90 |
| I-122 | 320 | 90 |
| I-123 | 320 | 90 |
| I-120 | 320 | 90 |
| I-119 | 320 | 90 |
| I-124 | 320 | 90 |
| I-117 | 320 | 90 |
| I-126 | 320 | 90 |
| I-127 | 320 | 90 |
| I-128 | 320 | 90 |
| I-131 | 1280 | 90 |
| I-132 | 1280 | 90 |
| I-125 | 320 | 90 |
| I-130 | 320 | 90 |
| I-129 | 320 | 90 |
| I-137 | 320 | 90 |
| I-141 | 1280 | 90 |
| I-138 | 1280 | 100 |
| I-139 | 320 | 90 |
| I-135 | 1280 | 90 |
| I-143 | 320 | 90 |
| I-144 | 1280 | 100 |
| I-142 | 320 | 90 |
| I-147 | 1280 | 90 |
| I-152 | 1280 | 100 |
| I-156 | 1280 | 90 |
| I-163 | 320 | 90 |
| I-164 | 1280 | 90 |
| I-169 | 1280 | 90 |
| I-171 | 1280 | 90 |
| I-174 | 320 | 90 |
| I-178 | 1280 | 90 |

**Table B12: post-emergence activity at 1280g/ha and 320 g/ha against DIGSA in %**

| Example number | application rate [g/ha] | DIGSA |
|---|---|---|
| I-002 | 320 | 90 |
| I-003 | 1280 | 100 |
| I-010 | 1280 | 90 |
| I-027 | 1280 | 90 |
| I-031 | 320 | 90 |
| I-048 | 1280 | 90 |
| I-051 | 1280 | 90 |
| I-052 | 1280 | 90 |
| I-055 | 1280 | 90 |
| I-056 | 1280 | 90 |
| I-057 | 1280 | 90 |
| I-058 | 1280 | 90 |
| I-059 | 320 | 90 |
| I-060 | 320 | 90 |
| I-065 | 1280 | 90 |
| I-066 | 1280 | 90 |
| I-069 | 1280 | 90 |
| I-071 | 320 | 90 |
| I-072 | 1280 | 90 |
| I-074 | 1280 | 90 |
| I-083 | 1280 | 90 |
| I-088 | 1280 | 100 |
| I-089 | 1280 | 90 |
| I-104 | 1280 | 90 |
| I-115 | 1280 | 90 |
| I-113 | 320 | 90 |
| I-107 | 1280 | 90 |
| I-122 | 1280 | 90 |
| I-120 | 1280 | 90 |
| I-119 | 1280 | 90 |
| I-124 | 1280 | 90 |
| I-127 | 1280 | 90 |
| I-128 | 1280 | 90 |
| I-130 | 1280 | 90 |
| I-129 | 1280 | 90 |
| I-137 | 1280 | 90 |
| I-141 | 1280 | 90 |
| I-139 | 1280 | 90 |
| I-143 | 1280 | 90 |
| I-144 | 1280 | 90 |
| I-142 | 1280 | 90 |

As the results show, various compounds of the general formula (I) according to the invention, in post-emergence applications, have very good herbicidal activity against harmful plants plants such as Abutilon theophrasti (ABUTH), Alopecurus myosuroides (ALOMY), Amaranthus retroflexus (AMARE), Digitaria sanguinalis (DIGSA), Echinochloa crus-galli (ECHCG), Kochia scoparia (KCHSC), Lolium rigidum (LOLRI), Matricaria inodora (MATIN), Poa annua (POAAN), Setaria viridis (SETVI), Stellaria media (STEME) and Veronica persica (VERPE) at an application rate of 1280 g or below of active substance per hectare.

### C. Herbicidal pre-emergence action

Seeds of mono- and dicotyledonous weed plants and crop plants were sown, in plastic or organic planting pots, in sandy loam and covered with soil. The compounds according to the invention, formulated in the form of wettable powders (WP) or as emulsifiable concentrates (EC), were applied to the surface of the covering soil as aqueous suspension or emulsion, with the addition of 0.5% of an additive, at an application rate of 600 l of water/ha (converted).

Following treatment, the pots were placed in a greenhouse and kept under optimum growth conditions for the test plants. The visual grading of the damage to the test plants was carried out after ca. 3 weeks in comparison to untreated controls (herbicidal effect in percent (%): 100% effect = plants have died off, 0% effect = as control plants).

Tables C1 to C13 below show the effects of selected compounds of the general formula (I) according to Table 1 on various harmful plants and an application rate corresponding to 320 g/ha or below obtained by the experimental procedure mentioned above.

**Table C1: pre-emergence activity at 320g/ha and 80 g/ha against ABUTH in %**

| Example number | application rate [g/ha] | ABUTH |
|---|---|---|
| I-005 | 320 | 90 |
| I-007 | 320 | 80 |
| I-010 | 320 | 80 |
| I-013 | 320 | 100 |
| I-024 | 320 | 80 |
| I-049 | 320 | 80 |
| I-051 | 80 | 80 |
| I-052 | 80 | 80 |
| I-055 | 320 | 80 |
| I-057 | 320 | 90 |
| I-058 | 320 | 80 |
| I-059 | 320 | 90 |
| I-060 | 80 | 90 |
| I-064 | 320 | 80 |
| I-066 | 320 | 80 |
| I-069 | 320 | 90 |
| I-072 | 320 | 80 |
| I-083 | 320 | 90 |
| I-088 | 320 | 90 |
| I-104 | 320 | 80 |
| I-124 | 320 | 80 |
| I-127 | 320 | 90 |
| I-128 | 80 | 80 |
| I-137 | 320 | 90 |
| I-141 | 320 | 90 |
| I-139 | 320 | 90 |
| I-143 | 320 | 90 |

**Table C2: pre-emergence activity at 320g/ha and 80 g/ha against ALOMY in %**

| Example number | application rate [g/ha] | ALOMY |
|---|---|---|
| I-001 | 320 | 100 |
| I-003 | 320 | 80 |
| I-005 | 320 | 100 |
| I-007 | 320 | 100 |
| I-008 | 320 | 100 |
| I-009 | 320 | 100 |
| I-010 | 80 | 90 |
| I-011 | 320 | 90 |
| I-014 | 320 | 100 |
| I-015 | 320 | 90 |
| I-018 | 320 | 100 |
| I-020 | 320 | 80 |
| I-021 | 320 | 80 |
| I-022 | 320 | 80 |
| I-024 | 320 | 100 |
| I-027 | 320 | 90 |
| I-029 | 80 | 100 |
| I-030 | 320 | 80 |
| I-031 | 80 | 90 |
| I-036 | 320 | 90 |
| I-037 | 320 | 80 |
| I-039 | 320 | 90 |
| I-041 | 320 | 80 |
| I-044 | 320 | 100 |
| I-047 | 320 | 100 |
| I-049 | 320 | 100 |
| I-051 | 320 | 100 |
| I-052 | 80 | 80 |
| I-055 | 320 | 80 |
| I-057 | 320 | 100 |
| I-058 | 320 | 100 |
| I-059 | 320 | 100 |
| I-060 | 80 | 100 |
| I-065 | 320 | 100 |
| I-066 | 80 | 80 |
| I-067 | 320 | 90 |
| I-068 | 320 | 90 |
| I-069 | 80 | 90 |
| I-071 | 80 | 100 |
| I-072 | 320 | 100 |
| I-074 | 80 | 80 |
| I-083 | 80 | 90 |
| I-088 | 320 | 90 |
| I-091 | 320 | 100 |
| I-104 | 320 | 100 |
| I-122 | 320 | 100 |
| I-123 | 80 | 90 |
| I-120 | 320 | 100 |
| I-119 | 320 | 90 |
| I-124 | 80 | 100 |
| I-117 | 80 | 90 |
| I-127 | 320 | 100 |
| I-128 | 80 | 90 |
| I-130 | 320 | 90 |
| I-129 | 320 | 100 |
| I-137 | 80 | 80 |
| I-141 | 320 | 80 |
| I-139 | 320 | 80 |
| I-143 | 320 | 100 |

**Table C3: pre-emergence activity at 320g/ha and 80 g/ha against ECHCG in %**

| Example number | application rate [g/ha] | ECHCG |
|---|---|---|
| I-001 | 80 | 100 |
| I-003 | 320 | 80 |
| I-005 | 80 | 100 |
| I-007 | 80 | 100 |
| I-008 | 80 | 100 |
| I-009 | 80 | 100 |
| I-010 | 80 | 100 |
| I-011 | 320 | 100 |
| I-013 | 320 | 100 |
| I-014 | 320 | 100 |
| I-015 | 80 | 100 |
| I-018 | 320 | 100 |
| I-019 | 320 | 100 |
| I-021 | 320 | 90 |
| I-022 | 320 | 100 |
| I-024 | 80 | 100 |
| I-027 | 320 | 100 |
| I-029 | 80 | 100 |
| I-030 | 320 | 100 |
| I-031 | 80 | 100 |
| I-036 | 320 | 100 |
| I-037 | 320 | 100 |
| I-039 | 320 | 90 |
| I-040 | 320 | 100 |
| I-041 | 320 | 100 |
| I-044 | 320 | 100 |
| I-047 | 80 | 90 |
| I-049 | 80 | 100 |
| I-051 | 320 | 100 |
| I-052 | 80 | 100 |
| I-055 | 320 | 100 |
| I-057 | 80 | 100 |
| I-058 | 80 | 100 |
| I-059 | 80 | 100 |
| I-060 | 80 | 100 |
| I-064 | 80 | 90 |
| I-065 | 320 | 100 |
| I-066 | 320 | 100 |
| I-067 | 80 | 90 |
| I-068 | 80 | 90 |
| I-069 | 80 | 100 |
| I-071 | 80 | 100 |
| I-072 | 80 | 100 |
| I-074 | 80 | 100 |
| I-077 | 320 | 90 |
| I-082 | 320 | 100 |
| I-083 | 80 | 100 |
| I-087 | 320 | 90 |
| I-088 | 320 | 100 |
| I-089 | 320 | 100 |
| I-091 | 320 | 100 |
| I-104 | 80 | 100 |
| I-113 | 320 | 100 |
| I-122 | 320 | 100 |
| I-123 | 80 | 100 |
| I-120 | 80 | 100 |
| I-119 | 80 | 100 |
| I-124 | 80 | 100 |
| I-117 | 320 | 100 |
| I-127 | 80 | 100 |
| I-128 | 80 | 100 |
| I-130 | 80 | 100 |
| I-129 | 80 | 100 |
| I-137 | 80 | 100 |
| I-141 | 320 | 100 |
| I-139 | 320 | 100 |
| I-133 | 320 | 100 |
| I-143 | 80 | 100 |

**Table C4: pre-emergence activity at 320 g/ha against AVEFA in %**

| Example number | application rate [g/ha] | AVEFA |
|---|---|---|
| I-001 | 320 | 80 |
| I-007 | 320 | 80 |
| I-008 | 320 | 80 |
| I-044 | 320 | 80 |
| I-060 | 320 | 90 |
| I-069 | 320 | 100 |
| I-071 | 320 | 100 |
| I-130 | 320 | 80 |

**Table C5: pre-emergence activity at 320 g/ha and 80 g/ha against LOLRI in %**

| Example number | application rate [g/ha] | LOLRI |
|---|---|---|
| I-001 | 80 | 100 |
| I-005 | 320 | 100 |
| I-007 | 80 | 100 |
| I-008 | 320 | 80 |
| I-009 | 320 | 90 |
| I-010 | 320 | 100 |
| I-011 | 320 | 100 |
| I-013 | 320 | 90 |
| I-014 | 320 | 100 |
| I-015 | 320 | 100 |
| I-018 | 320 | 100 |
| I-019 | 320 | 100 |
| I-024 | 320 | 100 |
| I-027 | 320 | 90 |
| I-029 | 320 | 100 |
| I-030 | 320 | 80 |
| I-036 | 320 | 90 |
| I-044 | 320 | 100 |
| I-047 | 320 | 100 |
| I-049 | 80 | 100 |
| I-051 | 320 | 100 |
| I-052 | 80 | 90 |
| I-055 | 320 | 90 |
| I-058 | 320 | 100 |
| I-059 | 80 | 90 |
| I-060 | 80 | 100 |
| I-066 | 320 | 100 |
| I-067 | 320 | 80 |
| I-068 | 320 | 90 |
| I-069 | 320 | 100 |
| I-071 | 80 | 90 |
| I-072 | 320 | 100 |
| I-083 | 320 | 80 |
| I-089 | 320 | 100 |
| I-091 | 320 | 100 |
| I-104 | 320 | 100 |
| I-113 | 320 | 90 |
| I-122 | 320 | 100 |
| I-123 | 80 | 90 |
| I-120 | 320 | 100 |
| I-119 | 320 | 100 |
| I-124 | 80 | 100 |
| I-117 | 320 | 100 |
| I-127 | 80 | 80 |
| I-128 | 80 | 100 |
| I-130 | 80 | 90 |
| I-129 | 320 | 100 |
| I-137 | 320 | 100 |
| I-139 | 320 | 80 |
| I-143 | 320 | 90 |

**Table C6: pre-emergence activity at 320 g/ha and 80 g/ha against MATIN in %**

| Example number | application rate [g/ha] | MATIN |
|---|---|---|
| I-001 | 320 | 90 |
| I-003 | 320 | 80 |
| I-005 | 320 | 90 |
| I-007 | 80 | 80 |
| I-008 | 320 | 90 |
| I-009 | 320 | 90 |
| I-014 | 320 | 90 |
| I-015 | 320 | 80 |
| I-024 | 320 | 90 |
| I-029 | 320 | 80 |
| I-049 | 320 | 80 |
| I-010 | 320 | 90 |
| I-037 | 320 | 80 |
| I-051 | 320 | 80 |
| I-052 | 320 | 80 |
| I-057 | 320 | 90 |
| I-058 | 320 | 80 |
| I-059 | 320 | 90 |
| I-060 | 320 | 90 |
| I-064 | 320 | 80 |
| I-066 | 320 | 90 |
| I-067 | 320 | 80 |
| I-069 | 320 | 90 |
| I-071 | 320 | 90 |
| I-072 | 320 | 90 |
| I-083 | 320 | 90 |
| I-088 | 320 | 90 |
| I-091 | 320 | 80 |
| I-104 | 800 | 90 |
| I-122 | 320 | 80 |
| I-120 | 320 | 90 |
| I-119 | 320 | 90 |
| I-124 | 320 | 90 |
| I-127 | 320 | 90 |
| I-128 | 80 | 80 |
| I-130 | 320 | 80 |
| I-129 | 320 | 90 |
| I-137 | 320 | 90 |
| I-139 | 320 | 80 |
| I-143 | 320 | 90 |

**Table C7: pre-emergence activity at 320g/ha and 80 g/ha against POLCO in %**

| Example number | application rate [g/ha] | POLCO |
|---|---|---|
| I-001 | 320 | 80 |
| I-005 | 80 | 100 |
| I-007 | 320 | 80 |
| I-009 | 320 | 80 |
| I-010 | 80 | 80 |
| I-013 | 320 | 80 |
| I-014 | 320 | 90 |
| I-015 | 320 | 80 |
| I-018 | 320 | 90 |
| I-019 | 320 | 80 |
| I-022 | 320 | 80 |
| I-024 | 320 | 80 |
| I-027 | 320 | 80 |
| I-029 | 320 | 80 |
| I-030 | 320 | 80 |
| I-031 | 320 | 90 |
| I-037 | 320 | 80 |
| I-039 | 320 | 80 |
| I-040 | 320 | 80 |
| I-041 | 80 | 80 |
| I-044 | 320 | 90 |
| I-047 | 320 | 80 |
| I-049 | 320 | 80 |
| I-051 | 80 | 90 |
| I-052 | 80 | 90 |
| I-055 | 320 | 80 |
| I-057 | 80 | 80 |
| I-058 | 320 | 90 |
| I-059 | 80 | 80 |
| I-060 | 80 | 80 |
| I-064 | 320 | 80 |
| I-065 | 320 | 90 |
| I-066 | 80 | 90 |
| I-067 | 80 | 90 |
| I-068 | 80 | 90 |
| I-069 | 80 | 90 |
| I-071 | 80 | 90 |
| I-072 | 80 | 90 |
| I-074 | 320 | 90 |
| I-083 | 320 | 90 |
| I-088 | 320 | 90 |
| I-089 | 80 | 80 |
| I-091 | 320 | 80 |
| I-104 | 80 | 80 |
| I-113 | 80 | 90 |
| I-122 | 80 | 80 |
| I-120 | 80 | 80 |
| I-119 | 80 | 90 |
| I-124 | 320 | 80 |
| I-127 | 320 | 90 |
| I-128 | 320 | 90 |
| I-130 | 80 | 80 |
| I-137 | 320 | 80 |
| I-141 | 320 | 80 |
| I-139 | 320 | 80 |
| I-143 | 80 | 80 |
| I-148 | 320 | 80 |

**Table C8: pre-emergence activity at 320 g/ha and 80 g/ha against SETVI in %**

| Example number | application rate [g/ha] | SETVI |
|---|---|---|
| I-001 | 80 | 100 |
| I-003 | 320 | 100 |
| I-005 | 80 | 100 |
| I-007 | 80 | 100 |
| I-008 | 80 | 100 |
| I-009 | 320 | 100 |
| I-011 | 80 | 100 |
| I-013 | 320 | 100 |
| I-014 | 320 | 100 |
| I-015 | 320 | 100 |
| I-018 | 320 | 100 |
| I-019 | 320 | 100 |
| I-020 | 320 | 100 |
| I-021 | 320 | 80 |
| I-022 | 320 | 100 |
| I-024 | 80 | 100 |
| I-027 | 320 | 100 |
| I-029 | 80 | 100 |
| I-030 | 320 | 100 |
| I-031 | 80 | 90 |
| I-036 | 320 | 100 |
| I-037 | 320 | 100 |
| I-039 | 80 | 80 |
| I-040 | 320 | 100 |
| I-041 | 80 | 90 |
| I-044 | 320 | 100 |
| I-047 | 320 | 100 |
| I-049 | 80 | 100 |
| I-051 | 80 | 100 |
| I-052 | 80 | 100 |
| I-055 | 320 | 100 |
| I-057 | 80 | 100 |
| I-058 | 320 | 100 |
| I-059 | 80 | 100 |
| I-060 | 80 | 100 |
| I-064 | 320 | 100 |
| I-065 | 80 | 100 |
| I-066 | 80 | 100 |
| I-067 | 320 | 100 |
| I-068 | 320 | 100 |
| I-069 | 80 | 100 |
| I-071 | 80 | 100 |
| I-072 | 320 | 100 |
| I-074 | 320 | 100 |
| I-077 | 320 | 90 |
| I-083 | 80 | 100 |
| I-087 | 320 | 100 |
| I-088 | 80 | 100 |
| I-089 | 320 | 100 |
| I-091 | 80 | 100 |
| I-093 | 320 | 90 |
| I-104 | 80 | 100 |
| I-113 | 320 | 100 |
| I-122 | 320 | 100 |
| I-123 | 80 | 100 |
| I-120 | 80 | 100 |
| I-119 | 80 | 100 |
| I-124 | 80 | 100 |
| I-117 | 320 | 100 |
| I-127 | 80 | 100 |
| I-128 | 80 | 100 |
| I-130 | 320 | 100 |
| I-129 | 320 | 100 |
| I-137 | 80 | 100 |
| I-141 | 320 | 100 |
| I-139 | 320 | 100 |
| I-133 | 320 | 100 |
| I-143 | 80 | 100 |

**Table C9: pre-emergence activity at 320 g/ha and 80 g/ha against VIOTR in %**

| Example number | application rate [g/ha] | VIOTR |
|---|---|---|
| I-005 | 320 | 90 |
| I-031 | 320 | 80 |
| I-047 | 80 | 90 |
| I-057 | 320 | 80 |
| I-058 | 80 | 80 |
| I-059 | 320 | 80 |
| I-060 | 80 | 90 |
| I-065 | 80 | 80 |
| I-066 | 320 | 90 |
| I-067 | 320 | 100 |
| I-069 | 80 | 80 |
| I-071 | 80 | 90 |
| I-072 | 80 | 90 |
| I-074 | 320 | 90 |
| I-083 | 80 | 90 |
| I-088 | 320 | 90 |
| I-104 | 320 | 80 |
| I-122 | 320 | 80 |
| I-123 | 320 | 80 |
| I-120 | 320 | 90 |
| I-119 | 320 | 90 |
| I-124 | 320 | 90 |
| I-129 | 320 | 90 |
| I-143 | 80 | 80 |

**Table C10: pre-emergence activity at 320 g/ha and 80 g/ha against VERPE in %**

| Example number | application rate [g/ha] | VERPE |
|---|---|---|
| I-001 | 320 | 80 |
| I-007 | 320 | 80 |
| I-010 | 320 | 90 |
| I-013 | 320 | 80 |
| I-015 | 80 | 80 |
| I-020 | 320 | 80 |
| I-029 | 320 | 80 |
| I-036 | 320 | 80 |
| I-044 | 320 | 80 |
| I-047 | 80 | 80 |
| I-049 | 320 | 80 |
| I-051 | 320 | 90 |
| I-052 | 320 | 80 |
| I-055 | 320 | 80 |
| I-058 | 80 | 80 |
| I-059 | 80 | 90 |
| I-060 | 80 | 90 |
| I-065 | 80 | 90 |
| I-066 | 80 | 80 |
| I-067 | 320 | 80 |
| I-069 | 80 | 90 |
| I-071 | 80 | 90 |
| I-072 | 80 | 80 |
| I-074 | 80 | 80 |
| I-083 | 80 | 80 |
| I-122 | 320 | 80 |
| I-123 | 320 | 90 |
| I-120 | 320 | 90 |
| I-119 | 320 | 80 |
| I-124 | 320 | 80 |
| I-127 | 320 | 90 |
| I-128 | 80 | 80 |
| I-130 | 320 | 90 |
| I-129 | 320 | 90 |
| I-137 | 80 | 80 |
| I-141 | 320 | 90 |
| I-139 | 320 | 80 |
| I-133 | 320 | 80 |
| I-143 | 320 | 90 |
| I-148 | 320 | 80 |

**Table C11: pre-emergence activity at 320 g/ha and 80 g/ha against AMARE in %**

| Example number | application rate [g/ha] | AMARE |
|---|---|---|
| I-001 | 320 | 90 |
| I-005 | 80 | 80 |
| I-010 | 320 | 90 |
| I-013 | 80 | 90 |
| I-014 | 80 | 90 |
| I-019 | 80 | 80 |
| I-020 | 80 | 90 |
| I-021 | 80 | 100 |
| I-022 | 80 | 80 |
| I-024 | 80 | 80 |
| I-027 | 320 | 80 |
| I-029 | 80 | 80 |
| I-030 | 80 | 80 |
| I-037 | 320 | 90 |
| I-039 | 80 | 80 |
| I-040 | 80 | 90 |
| I-041 | 80 | 90 |
| I-044 | 80 | 90 |
| I-047 | 80 | 90 |
| I-049 | 80 | 80 |
| I-051 | 320 | 90 |
| I-052 | 80 | 90 |
| I-055 | 80 | 90 |
| I-057 | 320 | 90 |
| I-058 | 80 | 90 |
| I-059 | 80 | 90 |
| I-060 | 320 | 90 |
| I-064 | 320 | 90 |
| I-065 | 320 | 90 |
| I-066 | 80 | 90 |
| I-067 | 80 | 80 |
| I-068 | 320 | 90 |
| I-069 | 320 | 90 |
| I-071 | 80 | 90 |
| I-072 | 320 | 90 |
| I-074 | 80 | 90 |
| I-082 | 320 | 80 |
| I-083 | 320 | 90 |
| I-087 | 80 | 90 |
| I-088 | 320 | 90 |
| I-089 | 80 | 90 |
| I-091 | 320 | 90 |
| I-093 | 320 | 90 |
| I-104 | 80 | 80 |
| I-123 | 320 | 90 |
| I-120 | 80 | 80 |
| I-119 | 320 | 80 |
| I-127 | 320 | 90 |
| I-128 | 320 | 90 |
| I-129 | 80 | 80 |
| I-137 | 80 | 90 |
| I-141 | 80 | 100 |
| I-139 | 80 | 90 |
| I-143 | 80 | 90 |
| I-148 | 320 | 90 |
| I-151 | 320 | 100 |

**Table C12: pre-emergence activity at 1280g/ha and 320 g/ha against DIGSA in %**

| Example number | application rate [g/ha] | DIGSA |
|---|---|---|
| I-003 | 320 | 90 |
| I-007 | 80 | 100 |
| I-008 | 320 | 100 |
| I-009 | 80 | 90 |
| I-010 | 80 | 100 |
| I-011 | 320 | 100 |
| I-013 | 320 | 100 |
| I-019 | 320 | 100 |
| I-024 | 80 | 100 |
| I-027 | 320 | 90 |
| I-029 | 80 | 90 |
| I-030 | 320 | 100 |
| I-031 | 80 | 90 |
| I-036 | 320 | 100 |
| I-037 | 320 | 100 |
| I-039 | 320 | 100 |
| I-040 | 320 | 90 |
| I-041 | 320 | 100 |
| I-044 | 80 | 90 |
| I-047 | 80 | 90 |
| I-049 | 80 | 90 |
| I-051 | 80 | 90 |
| I-052 | 80 | 100 |
| I-055 | 80 | 90 |
| I-057 | 320 | 100 |
| I-058 | 80 | 100 |
| I-059 | 80 | 100 |
| I-060 | 80 | 100 |
| I-064 | 320 | 100 |
| I-065 | 320 | 100 |
| I-066 | 80 | 100 |
| I-067 | 320 | 100 |
| I-068 | 320 | 100 |
| I-069 | 80 | 100 |
| I-071 | 80 | 100 |
| I-072 | 80 | 100 |
| I-074 | 320 | 100 |
| I-077 | 320 | 90 |
| I-082 | 320 | 80 |
| I-083 | 320 | 100 |
| I-087 | 320 | 90 |
| I-088 | 320 | 100 |
| I-089 | 320 | 100 |
| I-091 | 320 | 100 |
| I-104 | 80 | 100 |
| I-113 | 320 | 100 |
| I-122 | 320 | 100 |
| I-123 | 80 | 100 |
| I-120 | 320 | 100 |
| I-119 | 80 | 100 |
| I-124 | 80 | 100 |
| I-117 | 320 | 100 |
| I-127 | 80 | 100 |
| I-128 | 80 | 100 |
| I-130 | 320 | 100 |
| I-129 | 320 | 100 |
| I-137 | 320 | 100 |
| I-141 | 320 | 90 |
| I-139 | 320 | 100 |
| I-133 | 320 | 100 |
| I-143 | 80 | 100 |

**Table C13: pre-emergence activity at 320 g/ha and 80 g/ha against KCHSC in %**

| Example number | application rate [g/ha] | KCHSC |
|---|---|---|
| I-036 | 80 | 90 |
| I-037 | 320 | 80 |
| I-057 | 320 | 90 |
| I-064 | 320 | 90 |
| I-088 | 320 | 90 |
| I-089 | 320 | 90 |
| I-091 | 320 | 90 |
| I-104 | 320 | 90 |
| I-113 | 320 | 80 |
| I-122 | 320 | 90 |
| I-123 | 320 | 80 |
| I-120 | 320 | 90 |
| I-119 | 320 | 90 |
| I-124 | 320 | 90 |
| I-117 | 320 | 80 |
| I-127 | 320 | 90 |
| I-128 | 320 | 90 |
| I-130 | 320 | 90 |
| I-129 | 320 | 90 |
| I-137 | 320 | 100 |
| I-141 | 320 | 90 |
| I-139 | 320 | 90 |
| I-133 | 320 | 90 |
| I-143 | 320 | 90 |
| I-148 | 320 | 90 |

### D. Pre-emergence effects on crop plants

Tables D1 to D5 below show the effects of various compounds of the general formula (I) according to Table 1 on various crop plants and an application rate corresponding to 320 g/ha or below obtained by the experimental procedure mentioned above in Biological Examples, Section C.

**Table D1: pre-emergence activity at 320g/ha and 80 g/ha against ZEAMX in %**

| Example number | application rate [g/ha] | ZEAMX |
|---|---|---|
| I-001 | 80 | 10 |
| I-009 | 320 | 10 |
| I-011 | 320 | 10 |
| I-013 | 80 | 10 |
| I-014 | 80 | 0 |
| I-015 | 80 | 10 |
| I-018 | 80 | 20 |
| I-019 | 320 | 0 |
| I-020 | 320 | 10 |
| I-021 | 80 | 0 |
| I-022 | 320 | 0 |
| I-024 | 80 | 10 |
| I-027 | 80 | 20 |
| I-030 | 80 | 20 |
| I-031 | 320 | 10 |
| I-036 | 320 | 0 |
| I-037 | 80 | 0 |
| I-039 | 320 | 20 |
| I-040 | 320 | 20 |
| I-041 | 80 | 0 |
| I-044 | 80 | 20 |
| I-047 | 320 | 20 |
| I-049 | 80 | 10 |
| I-051 | 80 | 20 |
| I-055 | 320 | 0 |
| I-057 | 320 | 20 |
| I-058 | 320 | 0 |
| I-059 | 80 | 0 |
| I-060 | 80 | 20 |
| I-061 | 320 | 0 |
| I-064 | 80 | 0 |
| I-065 | 320 | 0 |
| I-066 | 320 | 0 |
| I-067 | 320 | 20 |
| I-068 | 320 | 20 |
| I-069 | 80 | 0 |
| I-071 | 80 | 0 |
| I-072 | 80 | 0 |
| I-074 | 80 | 0 |
| I-077 | 320 | 10 |
| I-082 | 320 | 20 |
| I-083 | 80 | 10 |
| I-087 | 320 | 10 |
| I-088 | 320 | 0 |
| I-089 | 320 | 20 |
| I-091 | 80 | 0 |
| I-093 | 320 | 10 |
| I-095 | 320 | 0 |
| I-104 | 80 | 0 |
| I-113 | 320 | 0 |
| I-122 | 320 | 0 |
| I-123 | 80 | 10 |
| I-120 | 80 | 10 |
| I-119 | 80 | 20 |
| I-124 | 80 | 0 |
| I-117 | 320 | 20 |
| I-127 | 80 | 20 |
| I-128 | 80 | 0 |
| I-129 | 80 | 10 |
| I-137 | 80 | 20 |
| I-141 | 80 | 0 |
| I-139 | 320 | 20 |
| I-133 | 320 | 20 |
| I-143 | 80 | 0 |
| I-146 | 320 | 0 |
| I-148 | 80 | 0 |

**Table D2: pre-emergence activity at 320g/ha and 80 g/ha against TRZAS in %**

| Example number | application rate [g/ha] | TRZAS |
|---|---|---|
| I-003 | 80 | 20 |
| I-013 | 80 | 0 |
| I-014 | 80 | 10 |
| I-015 | 80 | 10 |
| I-018 | 80 | 10 |
| I-019 | 320 | 10 |
| I-020 | 80 | 0 |
| I-021 | 320 | 0 |
| I-022 | 320 | 20 |
| I-027 | 80 | 0 |
| I-030 | 80 | 0 |
| I-037 | 320 | 0 |
| I-039 | 80 | 20 |
| I-040 | 320 | 0 |
| I-041 | 320 | 20 |
| I-044 | 80 | 20 |
| I-047 | 320 | 10 |
| I-049 | 80 | 20 |
| I-051 | 80 | 20 |
| I-055 | 80 | 0 |
| I-058 | 320 | 0 |
| I-059 | 80 | 20 |
| I-061 | 320 | 0 |
| I-064 | 320 | 0 |
| I-065 | 80 | 0 |
| I-066 | 80 | 0 |
| I-067 | 80 | 10 |
| I-068 | 80 | 10 |
| I-069 | 80 | 0 |
| I-072 | 80 | 0 |
| I-074 | 80 | 0 |
| I-077 | 320 | 0 |
| I-082 | 320 | 0 |
| I-087 | 320 | 10 |
| I-088 | 80 | 0 |
| I-089 | 320 | 0 |
| I-091 | 80 | 0 |
| I-093 | 320 | 0 |
| I-095 | 320 | 0 |
| I-104 | 80 | 0 |
| I-113 | 320 | 10 |
| I-122 | 80 | 10 |
| I-117 | 80 | 0 |
| I-127 | 80 | 20 |
| I-129 | 80 | 10 |
| I-137 | 80 | 0 |
| I-141 | 80 | 0 |
| I-133 | 80 | 0 |
| I-143 | 80 | 0 |
| I-146 | 320 | 0 |
| I-148 | 320 | 0 |
| I-151 | 80 | 0 |

**Table D3: pre-emergence activity at 320g/ha and 80 g/ha against ORYZA in %**

| Example number | application rate [g/ha] | ORYZA |
|---|---|---|
| I-001 | 80 | 20 |
| I-013 | 80 | 10 |
| I-014 | 80 | 10 |
| I-018 | 80 | 10 |
| I-020 | 80 | 20 |
| I-021 | 80 | 10 |
| I-022 | 320 | 20 |
| I-031 | 80 | 0 |
| I-040 | 320 | 10 |
| I-041 | 320 | 20 |
| I-044 | 80 | 10 |
| I-047 | 320 | 10 |
| I-047 | 80 | 0 |
| I-052 | 80 | 10 |
| I-055 | 320 | 20 |
| I-058 | 80 | 0 |
| I-061 | 320 | 20 |
| I-065 | 80 | 0 |
| I-066 | 80 | 0 |
| I-067 | 80 | 20 |
| I-068 | 80 | 10 |
| I-082 | 320 | 0 |
| I-083 | 80 | 20 |
| I-087 | 320 | 20 |
| I-095 | 320 | 10 |

**Table D4: pre-emergence activity at 320g/ha and 80 g/ha against GLXMA in %**

| Example number | application rate [g/ha] | GLXMA |
|---|---|---|
| I-001 | 320 | 0 |
| I-008 | 320 | 0 |
| I-009 | 320 | 10 |
| I-011 | 320 | 0 |
| I-013 | 320 | 20 |
| I-014 | 80 | 0 |
| I-015 | 80 | 0 |
| I-018 | 320 | 10 |
| I-019 | 80 | 10 |
| I-020 | 320 | 20 |
| I-022 | 320 | 0 |
| I-029 | 80 | 10 |
| I-030 | 320 | 0 |
| I-037 | 320 | 0 |
| I-040 | 80 | 20 |
| I-044 | 320 | 20 |
| I-047 | 320 | 20 |
| I-049 | 320 | 0 |
| I-051 | 80 | 20 |
| I-055 | 320 | 20 |
| I-058 | 80 | 0 |
| I-059 | 80 | 0 |
| I-060 | 80 | 0 |
| I-061 | 320 | 0 |
| I-064 | 80 | 0 |
| I-065 | 320 | 0 |
| I-066 | 80 | 0 |
| I-067 | 80 | 20 |
| I-068 | 320 | 20 |
| I-069 | 80 | 0 |
| I-072 | 80 | 0 |
| I-074 | 320 | 0 |
| I-087 | 320 | 20 |
| I-093 | 80 | 0 |
| I-095 | 80 | 0 |
| I-113 | 320 | 10 |
| I-117 | 320 | 10 |
| I-122 | 80 | 10 |
| I-123 | 320 | 0 |
| I-120 | 80 | 10 |
| I-127 | 80 | 20 |
| I-129 | 320 | 0 |
| I-133 | 80 | 20 |
| I-143 | 80 | 0 |
| I-146 | 80 | 0 |
| I-148 | 80 | 0 |
| I-151 | 80 | 0 |

**Table D5: pre-emergence activity at 320g/ha and 80 g/ha against BRSNW in %**

| Example number | application rate [g/ha] | BRSNW |
|---|---|---|
| I-001 | 320 | 20 |
| I-003 | 80 | 20 |
| I-009 | 80 | 10 |
| I-014 | 80 | 10 |
| I-015 | 80 | 20 |
| I-018 | 80 | 10 |
| I-020 | 320 | 10 |
| I-021 | 320 | 0 |
| I-022 | 320 | 10 |
| I-036 | 80 | 0 |
| I-040 | 320 | 10 |
| I-055 | 80 | 20 |
| I-061 | 320 | 20 |
| I-068 | 80 | 20 |
| I-082 | 80 | 20 |
| I-087 | 80 | 20 |
| I-095 | 320 | 20 |
| I-141 | 80 | 20 |
| I-146 | 80 | 0 |
| I-148 | 80 | 0 |

As the results in above given Tables C1 to C13 show, compounds according to the invention have very good herbicidal pre-emergence effectiveness against a broad spectrum of mono- and dicotyledonous weeds such as Abutilon theophrasti (ABUTH), Alopecurus myosuroides (ALOMY), Amaranthus retroflexus (AMARE), Avena fatua (AVEFA), Digitaria sanguinalis (DIGSA), Echinochloa crus-galli (ECHCG), Kochia scoparia (KCHSC), Lolium rigidum (LOLRI), Matricaria inodora (MATIN), Polygonum convolvulus (POLCO), Setaria viridis (SETVI), Veronica persica (VERPE) and Viola tricolor (VIOTR) at an application rate of 320 g or below of active substance per hectare.

As the results in above given Tables D1 to D5 show further, compounds according to the invention show only low or no damaging effects in crop plants, such as Triticum aestivum (TRZAS), Zea mays (ZEAMX), Oryza sativa (ORYZA), Glycine max (GLXMA) and Brassica napus (BRSNW).

### E. Herbicidal post-emergence action

Seeds of mono- and dicotyledonous weed plants and crop plants were sown, in plastic or organic planting pots, in sandy loam, covered with soil and grown in a greenhouse under controlled growth conditions. 2 to 3 weeks after sowing, the test plants were sprayed in the single-leaf stage. The compounds according to the invention, formulated in form of wettable powders (WP) or as emulsifiable concentrates (EC), were sprayed onto the green plant parts as aqueous suspension or emulsion, with the addition of 0.5% of an additive, at an application rate of 600 l of water/ha (converted). The test plants were placed in the greenhouse for ca. 3 weeks under optimum growth conditions, and then the effect of the preparations was assessed visually in comparison with untreated controls (herbicidal effect in percent (%): 100% effect = plants have died off, 0% effect = as control plants).

Tables E1 to E9 below show the effects of selected compounds of the general formula (I) according to Table 1 on various harmful plants and an application rate corresponding to 320 g/ha or below obtained by the experimental procedure mentioned above.

**Table E1: post-emergence activity at 320g/ha and 80 g/ha against ABUTH in %**

| Example number | application rate [g/ha] | ABUTH |
|---|---|---|
| I-005 | 320 | 80 |
| I-007 | 320 | 80 |
| I-010 | 320 | 80 |
| I-031 | 320 | 90 |
| I-057 | 320 | 80 |
| I-059 | 320 | 90 |
| I-060 | 320 | 80 |
| I-069 | 320 | 80 |
| I-071 | 320 | 90 |
| I-072 | 320 | 80 |
| I-083 | 80 | 90 |
| I-088 | 320 | 90 |
| I-104 | 80 | 80 |
| I-122 | 320 | 80 |
| I-123 | 320 | 80 |
| I-120 | 80 | 90 |
| I-119 | 320 | 80 |
| I-124 | 320 | 80 |
| I-127 | 80 | 80 |
| I-128 | 80 | 80 |
| I-130 | 320 | 80 |
| I-137 | 320 | 80 |
| I-141 | 320 | 80 |
| I-139 | 320 | 90 |

**Table E2: post-emergence activity at 320g/ha and 80 g/ha against ALOMY in %**

| Example number | application rate [g/ha] | ALOMY |
|---|---|---|
| I-001 | 320 | 90 |
| I-003 | 320 | 80 |
| I-005 | 320 | 80 |
| I-007 | 320 | 90 |
| I-008 | 320 | 80 |
| I-009 | 320 | 80 |
| I-015 | 320 | 90 |
| I-010 | 320 | 80 |
| I-018 | 320 | 90 |
| I-024 | 320 | 80 |
| I-029 | 320 | 80 |
| I-036 | 320 | 80 |
| I-049 | 320 | 80 |
| I-051 | 320 | 90 |
| I-052 | 320 | 90 |
| I-057 | 320 | 90 |
| I-058 | 320 | 90 |
| I-059 | 320 | 90 |
| I-060 | 320 | 90 |
| I-069 | 320 | 90 |
| I-071 | 320 | 80 |
| I-072 | 320 | 90 |
| I-074 | 320 | 90 |
| I-083 | 320 | 90 |
| I-104 | 320 | 80 |
| I-122 | 320 | 90 |
| I-123 | 320 | 90 |
| I-120 | 320 | 90 |
| I-119 | 320 | 90 |
| I-124 | 80 | 80 |
| I-127 | 320 | 90 |
| I-128 | 320 | 90 |
| I-130 | 320 | 90 |
| I-129 | 320 | 90 |
| I-137 | 320 | 80 |
| I-139 | 320 | 80 |
| I-143 | 320 | 90 |

**Table E3: post-emergence activity at 320 g/ha and 80 g/ha against AMARE in %**

| Example number | application rate [g/ha] | AMARE |
|---|---|---|
| I-001 | 320 | 80 |
| I-007 | 80 | 80 |
| I-009 | 320 | 80 |
| I-024 | 80 | 80 |
| I-010 | 320 | 80 |
| I-013 | 320 | 80 |
| I-027 | 320 | 80 |
| I-029 | 320 | 80 |
| I-030 | 320 | 80 |
| I-039 | 320 | 80 |
| I-044 | 320 | 80 |
| I-049 | 320 | 90 |
| I-051 | 320 | 80 |
| I-055 | 320 | 80 |
| I-059 | 320 | 90 |
| I-060 | 320 | 90 |
| I-067 | 320 | 80 |
| I-068 | 320 | 80 |
| I-069 | 320 | 90 |
| I-071 | 320 | 80 |
| I-072 | 320 | 80 |
| I-083 | 320 | 80 |
| I-089 | 320 | 80 |
| I-120 | 320 | 90 |
| I-119 | 320 | 80 |
| I-124 | 320 | 80 |
| I-127 | 320 | 90 |
| I-128 | 320 | 90 |
| I-137 | 320 | 80 |
| I-141 | 320 | 80 |
| I-139 | 320 | 90 |

**Table E4: post-emergence activity at 1280g/ha and 320 g/ha against DIGSA in %**

| Example number | application rate [g/ha] | DIGSA |
|---|---|---|
| I-007 | 80 | 90 |
| I-024 | 320 | 80 |

**Table E5: post-emergence activity at 320g/ha and 80 g/ha against ECHCG in %**

| Example number | application rate [g/ha] | ECHCG |
|---|---|---|
| I-001 | 320 | 90 |
| I-003 | 320 | 80 |
| I-005 | 80 | 80 |
| I-007 | 80 | 90 |
| I-008 | 320 | 80 |
| I-009 | 320 | 80 |
| I-010 | 320 | 90 |
| I-015 | 320 | 80 |
| I-019 | 320 | 80 |
| I-024 | 320 | 80 |
| I-029 | 320 | 80 |
| I-039 | 320 | 80 |
| I-049 | 320 | 80 |
| I-057 | 320 | 90 |
| I-058 | 320 | 90 |
| I-059 | 320 | 90 |
| I-060 | 320 | ## |
| I-064 | 320 | 80 |
| I-065 | 320 | 80 |
| I-066 | 320 | 90 |
| I-068 | 320 | 80 |
| I-069 | 320 | 90 |
| I-071 | 320 | 100 |
| I-072 | 320 | 90 |
| I-083 | 80 | 80 |
| I-091 | 320 | 80 |
| I-104 | 320 | 80 |
| I-113 | 320 | 80 |
| I-122 | 320 | 90 |
| I-123 | 320 | 80 |
| I-120 | 320 | 90 |
| I-119 | 320 | 90 |
| I-124 | 320 | 90 |
| I-117 | 320 | 80 |
| I-127 | 320 | 90 |
| I-128 | 320 | 90 |
| I-130 | 320 | 90 |
| I-129 | 320 | 90 |
| I-137 | 320 | 90 |
| I-139 | 320 | 90 |
| I-143 | 80 | 90 |

**Table E6: post-emergence activity at 320 g/ha against LOLRI in %**

| Example number | application rate [g/ha] | LOLRI |
|---|---|---|
| I-001 | 320 | 90 |
| I-005 | 320 | 90 |
| I-051 | 320 | 80 |
| I-071 | 320 | 90 |
| I-072 | 320 | 80 |
| I-120 | 320 | 90 |
| I-124 | 320 | 90 |
| I-127 | 320 | 80 |
| I-128 | 320 | 80 |
| I-130 | 320 | 90 |

**Table E7: post-emergence activity at 320 g/ha and 80 g/ha against POLCO in %**

| Example number | application rate [g/ha] | POLCO |
|---|---|---|
| I-001 | 320 | 90 |
| I-005 | 320 | 90 |
| I-007 | 80 | 90 |
| I-008 | 320 | 90 |
| I-009 | 80 | 80 |
| I-010 | 320 | 90 |
| I-011 | 320 | 80 |
| I-018 | 320 | 80 |
| I-019 | 320 | 90 |
| I-022 | 320 | 80 |
| I-024 | 320 | 80 |
| I-029 | 320 | 90 |
| I-030 | 320 | 80 |
| I-031 | 80 | 80 |
| I-036 | 320 | 90 |
| I-049 | 80 | 80 |
| I-051 | 320 | 90 |
| I-052 | 320 | 90 |
| I-057 | 320 | 90 |
| I-059 | 320 | 90 |
| I-060 | 80 | 90 |
| I-064 | 320 | 80 |
| I-065 | 320 | 80 |
| I-066 | 320 | 90 |
| I-068 | 320 | 90 |
| I-069 | 80 | 100 |
| I-071 | 80 | 90 |
| I-072 | 80 | 90 |
| I-083 | 320 | 90 |
| I-088 | 320 | 90 |
| I-089 | 320 | 90 |
| I-104 | 320 | 90 |
| I-122 | 320 | 90 |
| I-123 | 320 | 90 |
| I-120 | 80 | 90 |
| I-119 | 80 | 90 |
| I-124 | 80 | 90 |
| I-117 | 320 | 80 |
| I-127 | 80 | 90 |
| I-128 | 80 | 90 |
| I-130 | 320 | 90 |
| I-129 | 320 | 80 |
| I-137 | 320 | 80 |
| I-139 | 320 | 90 |
| I-133 | 320 | 80 |
| I-143 | 320 | 90 |

**Table E8: post-emergence activity at 320 g/ha and 80 g/ha against SETVI in %**

| Example number | application rate [g/ha] | SETVI |
|---|---|---|
| I-001 | 80 | 90 |
| I-003 | 320 | 80 |
| I-005 | 80 | 90 |
| I-007 | 80 | 90 |
| I-008 | 320 | 80 |
| I-009 | 320 | 80 |
| I-015 | 320 | 80 |
| I-024 | 320 | 80 |
| I-031 | 320 | 80 |
| I-049 | 320 | 80 |
| I-051 | 320 | 90 |
| I-052 | 320 | 90 |
| I-057 | 320 | 90 |
| I-059 | 320 | 90 |
| I-060 | 320 | 90 |
| I-066 | 320 | 90 |
| I-067 | 320 | 80 |
| I-069 | 320 | 90 |
| I-071 | 320 | 90 |
| I-072 | 320 | 80 |
| I-083 | 320 | 80 |
| I-104 | 320 | 90 |
| I-122 | 320 | 80 |
| I-123 | 320 | 90 |
| I-120 | 320 | 90 |
| I-119 | 320 | 80 |
| I-124 | 320 | 80 |
| I-127 | 320 | 80 |
| I-128 | 320 | 80 |
| I-130 | 320 | 80 |
| I-129 | 320 | 80 |
| I-143 | 320 | 80 |

**Table E9: post-emergence activity at 320 g/ha and 80 g/ha against VERPE in %**

| Example number | application rate [g/ha] | VERPE |
|---|---|---|
| I-001 | 80 | 80 |
| I-005 | 320 | 90 |
| I-007 | 80 | 80 |
| I-010 | 320 | 80 |
| I-015 | 320 | 80 |
| I-018 | 320 | 90 |
| I-020 | 320 | 80 |
| I-021 | 320 | 80 |
| I-031 | 80 | 80 |
| I-037 | 320 | 90 |
| I-047 | 320 | 80 |
| I-049 | 320 | 80 |
| I-052 | 320 | 80 |
| I-057 | 80 | 80 |
| I-058 | 320 | 90 |
| I-059 | 320 | 90 |
| I-060 | 80 | 80 |
| I-064 | 80 | 80 |
| I-066 | 320 | 90 |
| I-067 | 320 | 90 |
| I-068 | 320 | 90 |
| I-069 | 80 | 90 |
| I-071 | 320 | 90 |
| I-072 | 320 | 80 |
| I-074 | 320 | 90 |
| I-083 | 80 | 90 |
| I-088 | 320 | 80 |
| I-089 | 320 | 90 |
| I-091 | 320 | 90 |
| I-104 | 80 | 80 |
| I-122 | 320 | 90 |
| I-123 | 80 | 90 |
| I-120 | 80 | 80 |
| I-119 | 80 | 80 |
| I-124 | 320 | 90 |
| I-127 | 320 | 90 |
| I-128 | 80 | 80 |
| I-130 | 320 | 80 |
| I-129 | 320 | 90 |
| I-137 | 320 | 80 |
| I-141 | 320 | 80 |
| I-139 | 320 | 80 |
| I-133 | 320 | 80 |
| I-143 | 320 | 80 |

**Table E10: post-emergence activity at 320 g/ha against VIOTR in %**

| Example number | application rate [g/ha] | VIOTR |
|---|---|---|
| I-088 | 320 | 80 |
| I-104 | 320 | 80 |
| I-123 | 320 | 80 |
| I-129 | 320 | 80 |

**Table E11: post-emergence activity at 320 g/ha and 80 g/ha against KCHSC in %**

| Example number | application rate [g/ha] | KCHSC |
|---|---|---|
| I-036 | 320 | 80 |
| I-057 | 80 | 80 |
| I-088 | 320 | 80 |
| I-089 | 320 | 80 |
| I-104 | 320 | 90 |
| I-122 | 80 | 80 |
| I-123 | 80 | 80 |
| I-120 | 320 | 90 |
| I-119 | 80 | 80 |
| I-124 | 320 | 80 |
| I-117 | 320 | 80 |
| I-127 | 80 | 90 |
| I-128 | 80 | 90 |
| I-130 | 320 | 80 |
| I-129 | 320 | 80 |
| I-137 | 80 | 90 |
| I-141 | 80 | 80 |
| I-139 | 320 | 90 |
| I-133 | 80 | 90 |
| I-143 | 80 | 80 |
| I-148 | 320 | 80 |

### F. Post-emergence effects on crop plants

Tables F1 to F4 below show the effects of selected compounds of the general formula (I) according to Table 1 on various crop plants and an application rate corresponding to 320 g/ha or below obtained by the experimental procedure mentioned above.

**Table F1: post-emergence activity at 320g/ha and 80 g/ha against ZEAMX in %**

| Example number | application rate [g/ha] | ZEAMX |
|---|---|---|
| I-001 | 320 | 10 |
| I-003 | 320 | 0 |
| I-005 | 80 | 0 |
| I-007 | 80 | 0 |
| I-008 | 320 | 0 |
| I-009 | 320 | 0 |
| I-011 | 320 | 0 |
| I-013 | 80 | 0 |
| I-014 | 80 | 10 |
| I-018 | 320 | 20 |
| I-019 | 80 | 0 |
| I-020 | 80 | 10 |
| I-021 | 80 | 10 |
| I-022 | 320 | 20 |
| I-024 | 320 | 0 |
| I-027 | 320 | 0 |
| I-029 | 80 | 0 |
| I-030 | 320 | 0 |
| I-031 | 320 | 20 |
| I-036 | 320 | 0 |
| I-037 | 80 | 20 |
| I-039 | 80 | 20 |
| I-040 | 320 | 0 |
| I-041 | 320 | 20 |
| I-044 | 320 | 20 |
| I-047 | 320 | 0 |
| I-051 | 80 | 10 |
| I-052 | 80 | 0 |
| I-055 | 320 | 0 |
| I-057 | 80 | 0 |
| I-058 | 80 | 0 |
| I-059 | 320 | 0 |
| I-060 | 320 | 0 |
| I-061 | 320 | 20 |
| I-065 | 320 | 0 |
| I-066 | 320 | 0 |
| I-067 | 320 | 0 |
| I-068 | 320 | 10 |
| I-069 | 320 | 0 |
| I-071 | 80 | 10 |
| I-072 | 320 | 20 |
| I-077 | 80 | 0 |
| I-082 | 320 | 0 |
| I-083 | 80 | 10 |
| I-087 | 320 | 0 |
| I-088 | 320 | 0 |
| I-089 | 320 | 0 |
| I-091 | 80 | 20 |
| I-093 | 320 | 0 |
| I-095 | 320 | 0 |
| I-104 | 80 | 20 |
| I-113 | 320 | 0 |
| I-122 | 320 | 0 |
| I-123 | 320 | 0 |
| I-120 | 320 | 0 |
| I-119 | 320 | 0 |
| I-124 | 320 | 0 |
| I-117 | 320 | 20 |
| I-127 | 80 | 20 |
| I-128 | 320 | 0 |
| I-130 | 320 | 0 |
| I-129 | 320 | 0 |
| I-137 | 80 | 0 |
| I-141 | 320 | 0 |
| I-139 | 320 | 0 |
| I-133 | 320 | 0 |
| I-143 | 320 | 0 |
| I-146 | 320 | 0 |
| I-148 | 320 | 0 |
| I-151 | 320 | 0 |

**Table F2: post-emergence activity at 320g/ha and 80 g/ha against TRZAS in %**

| Example number | application rate [g/ha] | TRZAS |
|---|---|---|
| I-001 | 80 | 0 |
| I-003 | 320 | 0 |
| I-005 | 80 | 0 |
| I-008 | 80 | 0 |
| I-009 | 320 | 0 |
| I-011 | 80 | 0 |
| I-013 | 320 | 0 |
| I-014 | 80 | 0 |
| I-015 | 320 | 10 |
| I-018 | 320 | 10 |
| I-019 | 80 | 10 |
| I-020 | 320 | 10 |
| I-021 | 80 | 0 |
| I-022 | 80 | 0 |
| I-024 | 80 | 0 |
| I-027 | 320 | 10 |
| I-029 | 80 | 10 |
| I-030 | 80 | 0 |
| I-031 | 320 | 20 |
| I-036 | 320 | 0 |
| I-037 | 320 | 0 |
| I-039 | 320 | 20 |
| I-040 | 320 | 0 |
| I-041 | 320 | 0 |
| I-044 | 80 | 0 |
| I-047 | 320 | 0 |
| I-049 | 80 | 10 |
| I-051 | 80 | 0 |
| I-052 | 80 | 0 |
| I-055 | 320 | 0 |
| I-057 | 320 | 0 |
| I-058 | 80 | 0 |
| I-059 | 80 | 0 |
| I-060 | 80 | 0 |
| I-061 | 320 | 0 |
| I-064 | 320 | 0 |
| I-065 | 320 | 0 |
| I-066 | 320 | 0 |
| I-067 | 320 | 0 |
| I-068 | 320 | 0 |
| I-069 | 80 | 0 |
| I-071 | 80 | 0 |
| I-072 | 80 | 0 |
| I-074 | 80 | 0 |
| I-077 | 320 | 20 |
| I-082 | 320 | 0 |
| I-087 | 320 | 0 |
| I-088 | 320 | 20 |
| I-089 | 320 | 0 |
| I-091 | 320 | 0 |
| I-093 | 320 | 0 |
| I-095 | 320 | 0 |
| I-104 | 80 | 0 |
| I-113 | 320 | 20 |
| I-122 | 80 | 20 |
| I-123 | 320 | 10 |
| I-120 | 80 | 20 |
| I-119 | 80 | 20 |
| I-124 | 80 | 20 |
| I-117 | 320 | 10 |
| I-127 | 80 | 0 |
| I-130 | 80 | 10 |
| I-129 | 80 | 20 |
| I-137 | 80 | 0 |
| I-141 | 320 | 0 |
| I-139 | 80 | 0 |
| I-133 | 320 | 0 |
| I-143 | 80 | 0 |
| I-146 | 320 | 0 |
| I-148 | 320 | 0 |
| I-151 | 320 | 0 |

**Table F3: post-emergence activity at 320g/ha and 80 g/ha against ORYZA in %**

| Example number | application rate [g/ha] | ORYZA |
|---|---|---|
| I-001 | 320 | 20 |
| I-003 | 320 | 0 |
| I-005 | 80 | 0 |
| I-007 | 80 | 10 |
| I-008 | 320 | 0 |
| I-009 | 320 | 10 |
| I-011 | 320 | 0 |
| I-013 | 80 | 0 |
| I-014 | 80 | 10 |
| I-015 | 80 | 10 |
| I-018 | 80 | 0 |
| I-019 | 320 | 0 |
| I-020 | 320 | 0 |
| I-021 | 80 | 10 |
| I-022 | 320 | 10 |
| I-024 | 80 | 0 |
| I-027 | 80 | 0 |
| I-029 | 80 | 0 |
| I-030 | 320 | 20 |
| I-031 | 320 | 10 |
| I-040 | 320 | 0 |
| I-041 | 320 | 0 |
| I-044 | 320 | 0 |
| I-047 | 320 | 0 |
| I-049 | 80 | 20 |
| I-051 | 320 | 0 |
| I-052 | 320 | 0 |
| I-055 | 320 | 0 |
| I-058 | 320 | 0 |
| I-059 | 80 | 0 |
| I-060 | 80 | 0 |
| I-061 | 320 | 0 |
| I-065 | 80 | 0 |
| I-066 | 320 | 0 |
| I-067 | 320 | 0 |
| I-068 | 320 | 0 |
| I-069 | 80 | 0 |
| I-071 | 80 | 0 |
| I-072 | 320 | 20 |
| I-074 | 80 | 0 |
| I-082 | 320 | 10 |
| I-083 | 320 | 10 |
| I-087 | 320 | 0 |
| I-093 | 320 | 0 |
| I-095 | 320 | 0 |

**Table F4: post-emergence activity at 320g/ha and 80 g/ha against GLXMA in %**

| Example number | application rate [g/ha] | GLXMA |
|---|---|---|
| I-001 | 80 | 20 |
| I-003 | 80 | 0 |
| I-008 | 80 | 0 |
| I-009 | 80 | 0 |
| I-010 | 80 | 0 |
| I-013 | 80 | 10 |
| I-014 | 320 | 0 |
| I-015 | 80 | 0 |
| I-018 | 320 | 0 |
| I-020 | 320 | 0 |
| I-021 | 320 | 20 |
| I-022 | 320 | 10 |
| I-024 | 80 | 10 |
| I-027 | 80 | 20 |
| I-031 | 80 | 10 |
| I-036 | 320 | 0 |
| I-037 | 320 | 0 |
| I-039 | 320 | 20 |
| I-040 | 320 | 10 |
| I-041 | 80 | 0 |
| I-044 | 80 | 0 |
| I-047 | 80 | 0 |
| I-051 | 80 | 10 |
| I-052 | 80 | 10 |
| I-055 | 320 | 20 |
| I-058 | 80 | 20 |
| I-061 | 320 | 0 |
| I-064 | 80 | 0 |
| I-065 | 80 | 0 |
| I-066 | 80 | 0 |
| I-067 | 320 | 10 |
| I-068 | 80 | 10 |
| I-069 | 80 | 0 |
| I-072 | 80 | 0 |
| I-074 | 80 | 0 |
| I-077 | 320 | 10 |
| I-082 | 320 | 0 |
| I-087 | 80 | 0 |
| I-088 | 80 | 0 |
| I-089 | 80 | 0 |
| I-093 | 320 | 0 |
| I-095 | 320 | 0 |
| I-113 | 80 | 0 |
| I-122 | 80 | 10 |
| I-120 | 80 | 0 |
| I-119 | 80 | 20 |
| I-117 | 320 | 0 |
| I-130 | 80 | 20 |
| I-129 | 80 | 10 |
| I-141 | 80 | 20 |
| I-139 | 80 | 20 |
| I-133 | 80 | 0 |
| I-146 | 320 | 0 |
| I-148 | 320 | 0 |
| I-151 | 80 | 0 |

**Table F5: post-emergence activity at 80 g/ha against BRSNW in %**

| Example number | application rate [g/ha] | BRSNW |
|---|---|---|
| I-001 | 80 | 20 |
| I-001 | 80 | 20 |
| I-008 | 80 | 0 |
| I-013 | 80 | 0 |
| I-014 | 80 | 20 |
| I-015 | 80 | 10 |
| I-018 | 80 | 10 |
| I-020 | 80 | 20 |
| I-021 | 80 | 10 |
| I-022 | 80 | 0 |
| I-024 | 80 | 10 |
| I-027 | 80 | 20 |
| I-030 | 80 | 20 |
| I-036 | 80 | 0 |
| I-037 | 80 | 0 |
| I-040 | 80 | 0 |
| I-044 | 320 | 20 |
| I-047 | 320 | 20 |
| I-051 | 80 | 10 |
| I-055 | 80 | 10 |
| I-061 | 80 | 0 |
| I-064 | 80 | 0 |
| I-067 | 80 | 0 |
| I-082 | 320 | 0 |
| I-087 | 80 | 0 |
| I-088 | 80 | 0 |
| I-089 | 320 | 0 |
| I-091 | 80 | 0 |
| I-093 | 320 | 10 |
| I-095 | 320 | 20 |
| I-113 | 320 | 20 |
| I-122 | 80 | 0 |
| I-130 | 80 | 20 |
| I-129 | 80 | 0 |
| I-141 | 80 | 20 |
| I-133 | 80 | 0 |
| I-143 | 80 | 0 |
| I-146 | 320 | 0 |
| I-148 | 80 | 20 |

As the results in Tables E1 to E9 show, compounds according to the invention have good herbicidal post-emergence effectiveness against a broad spectrum of mono- and dicotyledonous weeds such as Abutilon theophrasti (ABUTH), Alopecurus myosuroides (ALOMY), Amaranthus retroflexus (AMARE), Avena fatua (AVEFA), Digitaria sanguinalis (DIGSA), Echinochloa crus-galli (ECHCG), Kochia scoparia (KCHSC), Lolium rigidum (LOLRI), Matricaria inodora (MATIN), Polygonum convolvulus (POLCO), Setaria viridis (SETVI), Stellaria media (STEME), Veronica persica (VERPE) and Viola tricolor (VIOTR) at an application rate of 320 g and less of active ingredient per hectare.

As the results in Tables F1 to F5 show further, compounds according to the invention show only low or no damaging effects in crop plants such as Triticum aestivum (TRZAS), Zea Mays (ZEAMX), Oryza sativa (ORYSA), Glycine max (GLXMA) and Brassica napus (BRSNW).

## Claims

1. Substituted 2,3-dihydro[1,3]thiazolo[4,5-b]pyridines of the general formula (I) or salts thereof 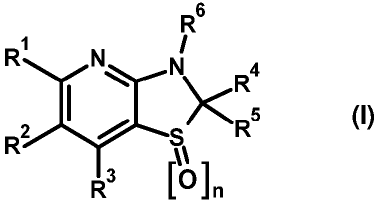 in which
R¹ represents (C₃-C₈)-cycloalkyl, (C₃-C₈)-cycloalkenyl, (C₃-C₈)-cycloalkoxy, aryl, heteroaryl, heterocyclyl, a bicyclic or a heterobicyclic residue, wherein each of the previously mentioned eight residues is unsubstituted or is independently substituted by one or more residues selected from the group R⁷,
R² represents hydrogen, halogen, formyl, hydroxy, hydrothio, hydroxycarbonyl, aminocarbonyl, aminothiocarbonyl, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈) haloalkoxy, (C₁-C₈)-alkoxy-(C₁-C₈)-alkyl, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl, (C₁-C₈)-alkylcarbonyl, (C₂-C₈)-alkenylcarbonyl, (C₂-C₈)-alkynylcarbonyl, (C₁-C₈)-haloalkylcarbonyl, (C₂-C₈)-haloalkenylcarbonyl, (C₂-C₈)-haloalkynylcarbonyl, (C₁-C₈)-alkoxycarbonyl, (C₃-C₈)-cycloalkyl, (C₃-C₈)-cycloalkoxy, (C₃-C₈)-cycloalkylthio, (C₃-C₈)-cycloalkylsulfinyl, (C₃-C₈)-cycloalkylsulfonyl, (C₃-C₈)-halocycloalkyl, (C₃-C₈)-halocycloalkoxy, (C₃-C₈)-halocycloalkylthio, (C₃-C₈)-halocycloalkylsulfinyl, (C₃-C₈)-halocycloalkylsulfonyl, (C₃-C₈)-cycloalkyl-(C₁-C₈)-alkyl, (C₁-C₈)-alkyl-(C₃-C₈)-cycloalkyl, (C₃-C₈)-cycloalkylcarbonyl, (C₃-C₈)-cycloalkyl-(C₁-C₈)-alkylcarbonyl, (C₁-C₈)-alkylaminocarbonyl, (C₂-C₁₂)-dialkylaminocarbonyl, (C₁-C₈)-alkylaminosulfonyl, (C₂-C₁₂)-dialkylaminosulfonyl, tris[(C₁-C₈)alkyl]silyl, or aryl,
R³ represents hydrogen, halogen, cyano, (C₁-C₈)-alkyl, (C₁-C₈)-haloalkyl, (C₂-C₈)-alkenyl, (C₂-C₈)-haloalkenyl, (C₁-C₈)-alkoxy, (C₁-C₈) haloalkoxy, (C₁-C₈)-alkylthio, (C₁-C₈)- haloalkylthio, (C₃-C₈)-cycloalkyl, (C₃-C₈)-halocycloalkyl, (C₃-C₈)-cycloalkoxy or (C₃-C₈)-halocycloalkoxy,
-R⁴ and R⁵ independently of one another represent hydrogen, (C₁-C₈)-alkyl, (C₁-C₈)-haloalkyl, (C₃-C₈)-cycloalkyl, (C₃-C₈)-halocycloalkyl, or R¹⁰O-(C₁-C₈)-alkyl, or
R⁴ and R⁵ together with the carbon atom to which they are bonded form a fully saturated, or partly saturated 3- to 10-membered monocyclic or bicyclic ring optionally interrupted by heteroatoms and optionally having further substitution,
R⁶ represents hydrogen, (C₁-C₈)-alkyl, (C₂-C₈)-alkenyl, (C₂-C₈)-alkynyl, cyano-(C₁-C₈)-alkyl, R¹⁰O-(C₁-C₈)-alkyl, R⁸R⁹N-(C₁-C₈)-alkyl, NR⁸R⁹, (C₃-C₁₀)-cycloalkyl-(C₁-C₈)-alkyl, C(=O)R¹⁰, C(=O)OR¹⁰, C(=O)NR⁸R⁹, R¹⁰O(O)C-(C₁-C₈)-alkyl, R¹⁰(O=)C-(C₁-C₈)-Alkyl, R⁸R⁹N(O)C-(C₁-C₈)-alkyl, SO₂R¹¹, R¹¹S(O)ₘ-(C₁-C₈)-alkyl, BR¹²R¹³, heterocyclyl, heteroaryl, aryl-(C₁-C₈)-alkyl, heteroaryl-(C₁-C₈)-alkyl, heterocyclyl-(C₁-Cs)-alkyl, tris[(C₁-C₈)alkyl]silyl, bis-[(C₁-C₈)-alkyl](aryl)silyl, (C₁-C₈)-alkyl-[bis-(aryl)]silyl, tris-[(C₁-C₈)-alkyl]silyl-(C₁-C₈)-alkyl, bis-[(C₁-C₈)-alkyl](aryl)silyl(C₁-C₈)-alkyl, or (C₁-C₈)-alkyl-[bis-(aryl)]silyl-(C₁-C₈)-alkyl,
m is 0, 1, 2,
n is 0, 1, 2,
R⁷ represents hydrogen, halogen, formyl, hydroxy, hydroxycarbonyl, nitro, cyano, amino, aminocarbonyl, aminothiocarbonyl, (C₁-C₈)-alkyl, (C₁-C₈)-haloalkyl, (C₂-C₈)-alkenyl, (C₂-C₈)-haloalkenyl, (C₂-C₈)-alkynyl, (C₂-C₈)-haloalkynyl, (C₁-C₈)-alkoxy, (C₁-C₈)-haloalkoxy, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl, (C₁-C₈)-alkylcarbonyl, (C₁-C₈)-haloalkylcarbonyl, (C₂-C₈)-alkenylcarbonyl, (C₂-C₈)-haloalkenylcarbonyl, (C₂-C₈)-alkynylcarbonyl, (C₂-C₈)-haloalkynylcarbonyl, (C₁-C₈)-alkoxycarbonyl, (C₁-C₈)-haloalkoxycarbonyl, (C₃-C₈)-cycloalkyl, (C₃-C₈)-halocycloalkyl, (C₃-C₈)-cycloalkoxy, (C₃-C₈)-halocycloalkoxy, (C₃-C₈)-cycloalkylthio, (C₃-C₈)-halocycloalkylthio, (C₃-C₈)-cycloalkylsulfinyl, (C₃-C₈)-halocycloalkylsulfinyl, (C₃-C₈)-cycloalkylsulfonyl, (C₃-C₈)-halocycloalkylsulfonyl, (C₃-C₈)-cycloalkyl-(C₁-C₈)-alkyl, (C₁-C₈)-alkyl-(C₃-C₈)-cycloalkyl, (C₁-C₈)-alkoxycarbonyl-(C₁-C₈)-alkyl, hydroxycarbonyl-(C₁-C₈)-alkyl, (C₃-C₈)-cycloalkylcarbonyl, (C₃-C₈)-cycloalkyl-(C₁-C₈)-alkylcarbonyl, (C₁-C₈)-alkylamino, (C₂-C₁₂)-dialkylamino, (C₁-C₈)- alkylaminocarbonyl, (C₂-C₁₂)-dialkylaminocarbonyl, (C₁-C₈)-alkylaminosulfonyl, (C₂-C₁₂)-dialkylaminosulfonyl or tris[(C₁-C₈)alkyl]silyl.
-R⁸ and R⁹ independently of one another represent hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-haloalkoxy-(C₁-C₈)-alkyl, (C₁-C₈)-alkylthio-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, COR¹⁰, SO₂R¹¹, heterocyclyl, (C₁-C₈)-alkoxycarbonyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkyl-aminocarbonyl-(C₁-C₈)-alkyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl, aryl-(C₁-C₈)-alkoxycarbonyl, heteroaryl-(C₁-C₈)-alkoxycarbonyl, (C₂-C₈)-alkenyloxycarbonyl, (C₂-C₈)-alkynyloxycarbonyl, heterocyclyl-(C₁-C₈)-alkyl, or
R⁸ and R⁹ together with the carbon atom to which they are bonded form a fully saturated or partly saturated 3- to 10-membered monocyclic or bicyclic ring optionally interrupted by heteroatoms and optionally having further substitution,
R¹⁰ represents hydrogen, (C₁-C₈)-alkyl, (C₂-C₈)-alkenyl, (C₂-C₈)-alkinyl, (C₁-C₈)-cyanoalkyl, (C₁-C₁₀)-haloalkyl, (C₂-C₈)-haloalkenyl, (C₃-C₈)-haloalkinyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-haloalkoxy-(C₁-C₈)-alkyl, (C₁-C₈)-alkoxy-(C₁-C₈)-haloalkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkoxy-(C₁-C₈)-alkyl, aryl, aryl-(C₁-C₈)-alkyl, aryl-(C₁-C₈)-alkoxy-(C₁-C₈)-alkyl, aryloxy-(C₁-C₈)-alkyl, heteroaryl-(C₁-C₈)-alkoxy-(C₁-C₈)-alkyl, heteroaryloxy-(C₁-C₈)-alkyl, heteroaryl, heteroaryl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]aminocarbonyl-(C₁-C₈)-alkyl, (C₁-C₈)-alkyl-aminocarbonyl-(C₁-C₈)-alkyl, aryl-(C₁-C₈)-alkylaminocarbonyl-(C₁-C₈)-alkyl, bis-[(C₁-C₈)-alkyl]amino-(C₂-C₆)-alkyl, (C₁-C₈)-alkylamino-(C₂-C₆)-alkyl, aryl-(C₁-C₆)-alkylamino-(C₂-C₆)-alkyl, R¹¹(O)ₘS-(C₁-C₈)-alkyl, hydroxycarbonyl-(C₁-C₈)-alkyl, heterocyclyl, heterocyclyl-(C₁-C₈)-alkyl, heterocyclyl-(C₁-C₈)-alkoxy-(C₁-C₈)-alkyl, heterocyclyloxy-(C₁-C₈)-alkyl, tris-[(C₁-C₈)-alkyl]silyl-(C₁-C₈)-alkyl, bis-[(C₁-C₈)-alkyl](aryl)silyl(C₁-C₈)-alkyl, [(C₁-C₈)-alkyl]-bis-(aryl)silyl-(C₁-C₈)-alkyl, (C₁-C₈)-alkylcarbonyloxy-(C₁-C₈)-alkyl, (C₃-C₈)-cycloalkylcarbonyloxy-(C₁-C₈)-alkyl, arylcarbonyloxy-(C₁-C₈)-alkyl, heteroarylcarbonyloxy-(C₁-C₈)-alkyl, Heterocyclylcarbonyloxy-(C₁-C₈)-alkyl, (C₁-C₈)-alkoxycarbonyl, (C₁-C₈)-alkoxycarbonyl-(C₁-C₈)-alkyl, (C₂-C₈)-alkenyloxycarbonyl-(C₁-C₈)-alkyl, (C₂-C₈)-alkynyloxycarbonyl-(C₁-C₈)-alkyl, aryl-(C₁-C₈)-alkoxycarbonyl-(C₁-C₈)-alkyl, heteroaryl-(C₁-C₈)-alkoxycarbonyl-(C₁-C₈)-alkyl, heterocyclyl-(C₁-C₈)-alkoxycarbonyl-(C₁-C₈)-alkyl, hydroxy-(C₁-C₈)-alkyl, hydroxy-(C₁-C₈)-alkoxy-(C₁-C₈)-alkyl, hydroxy-(C₁-C₈)-alkoxy-(C₁-C₈)-alkoxy-(C₁-C₈)-alkyl, hydroxy-(C₁-C₈)-alkoxy-(C₁-C₈)-alkoxy-(C₁-C₈)-alkoxy-(C₁-C₈)-alkyl, (C₁-C₈)-alkylcarbonyloxy-(C₁-C₈)-alkoxy-(C₁-C₈)-alkyl, (C₁-C₈)-alkylcarbonyloxy-(C₁-C₈)-alkoxy-(C₁-C₈)-alkoxy-(C₁-C₈)-alkyl, (C₁-C₈)-alkylcarbonyloxy-(C₁-C₈)-alkoxy-(C₁-C₈)-alkoxy-(C₁-C₈)-alkoxy-(C₁-C₈)-alkyl, (C₁-C₈)-alkoxycarbonyl-(C₁-C₈)-alkoxy-(C₁-C₈)-alkyl, (C₁-C₈)-alkoxycarbonyl-(C₁-C₈)-alkoxy-(C₁-C₈)-alkoxy-(C₁-C₈)-alkyl, (C₁-C₈)-alkoxycarbonyl-(C₁-C₈)-alkoxy-(C₁-C₈)-alkoxy-(C₁-C₈)-alkoxy-(C₁-C₈)-alkyl, heteroarylcarbonyloxy-(C₁-C₈)-alkoxy-(C₁-C₈)-alkyl, heteroarylcarbonyloxy-(C₁-C₈)-alkoxy-(C₁-C₈)-alkoxy-(C₁-C₈)-alkyl, heteroarylcarbonyloxy-(C₁-C₈)-alkoxy-(C₁-C₈)-alkoxy-(C₁-C₈)-alkoxy-(C₁-C₈)-alkyl, heterocyclylcarbonyloxy-(C₁-C₈)-alkoxy-(C₁-C₈)-alkyl, heterocyclylcarbonyloxy-(C₁-C₈)-alkoxy-(C₁-C₈)-alkoxy-(C₁-C₈)-alkyl, heterocyclylcarbonyloxy-(C₁-C₈)-alkoxy-(C₁-C₈)-alkoxy-(C₁-C₈)-alkoxy-(C₁-C₈)-alkyl, hydroxycarbonyl-(C₁-C₈)-alkoxy-(C₁-C₈)-alkyl, hydroxycarbonyl-(C₁-C₈)-alkoxy-(C₁-C₈)-alkoxy-(C₁-C₈)-alkyl, or hydroxycarbonyl-(C₁-C₈)-alkoxy-(C₁-C₈)-alkoxy-(C₁-C₈)-alkoxy-(C₁-C₈)-alkyl,
R¹¹ represents hydrogen, (C₁-C₈)-alkyl, (C₂-C₈)-alkenyl, (C₂-C₈)-alkynyl, (C₁-C₈)-cyanoalkyl, (C₁-C₁₀)-haloalkyl, (C₂-C₈)-haloalkenyl, (C₃-C₈)-haloalkynyl, (C₃-C₁₀)-cycloalkyl, (C₃-C₁₀)-halocycloalkyl, (C₄-C₁₀)-cycloalkenyl, (C₄-C₁₀)-halocycloalkenyl, (C₁-C₈)-alkoxy-(C₁-C₈)-alkyl, (C₁-C₈)-alkoxy-(C₁-C₈)-haloalkyl, aryl, aryl-(C₁-C₈)-alkyl, heteroaryl, heteroaryl-(C₁-C₈)-alkyl, heterocyclyl-(C₁-C₈)-alkyl, (C₃-C₈)-cycloalkyl-(C₁-C₈)-alkyl, (C₄-C₁₀)-cycloalkenyl-(C₁-C₈)-alkyl, bis-[(C₁-C₈)-alkyl]amino, (C₁-C₈)-alkyl-amino, aryl-(C₁-C₈)-amino, aryl-(C₁-C₆)-alkyl-amino, aryl-[(C₁-C₈)-alkyl]amino, heteroaryl-(C₁-C₈)-amino, heteroaryl-(C₁-C₆)-alkyl-amino, heteroaryl-[(C₁-C₈)-alkyl]amino; Hetercyclyl-(C₁-C₈)-amino, heterocyclyl-(C₁-C₆)-alkyl-amino, heterocyclyl-[(C₁-C₈)-alkyl]amino; (C₃-C₈)-cycloalkyl-amino, (C₃-C₈)-cycloalkyl-[(C₁-Cs)-alkyl]amino, or N-azetidinyl, N-pyrrolidinyl, N-piperidinyl, N-morpholinyl, and
R¹² and R¹³ independently of one another represent hydrogen, NR⁸R⁹, OR¹⁰, or
R¹² and R¹³ together with the boron atom to which they are bonded form a fully saturated or partly saturated 3- to 10-membered monocyclic or bicyclic ring optionally interrupted by heteroatoms and optionally having further substitution.

2. The compound of the general formula (I) as claimed in claim 1 and/or the salt thereof, **characterized in that**
R¹ represents (C₃-C₇)-cycloalkyl, (C₃-C₇)-cycloalkenyl, (C₃-C₇)-cycloalkoxy, aryl, heteroaryl, heterocyclyl, a bicyclic or a heterobicyclic residue, wherein each of the previously mentioned eight residues is unsubstituted or is independently substituted by one or more residues selected from the group R⁷,
R² represents hydrogen, halogen, formyl, hydroxy, hydrothio, hydroxycarbonyl, aminocarbonyl, aminothiocarbonyl, (C₁-C₇)-alkyl, (C₁-C₇)-haloalkyl, (C₂-C₇)-alkenyl, (C₂-C₇)-haloalkenyl, (C₂-C₇)-alkynyl, (C₂-C₇)-haloalkynyl, (C₁-C₇)-alkoxy, (C₁-C₇) haloalkoxy, (C₁-C₇)-alkoxy-(C₁-C₇)-alkyl, (C₁-C₇)-alkylthio, (C₁-C₇)-haloalkylthio, (C₁-C₇)-alkylsulfinyl, (C₁-C₇)-haloalkylsulfinyl, (C₁-C₇)-alkylsulfonyl, (C₁-C₇)-haloalkylsulfonyl, (C₁-C₇)-alkylcarbonyl, (C₂-C₇)-alkenylcarbonyl, (C₂-C₇)-alkynylcarbonyl, (C₁-C₇)-haloalkylcarbonyl, (C₂-C₇)-haloalkenylcarbonyl, (C₂-C₇)-haloalkynylcarbonyl, (C₁-C₇)-alkoxycarbonyl, (C₃-C₇)-cycloalkyl, (C₃-C₇)-cycloalkoxy, (C₃-C₇)-cycloalkylthio, (C₃-C₇)-cycloalkylsulfinyl, (C₃-C₇)-cycloalkylsulfonyl, (C₃-C₇)-halocycloalkyl, (C₃-C₇)-halocycloalkoxy, (C₃-C₇)-halocycloalkylthio, (C₃-C₇)-halocycloalkylsulfinyl, (C₃-C₇)-halocycloalkylsulfonyl, (C₃-C₇)-cycloalkyl-(C₁-C₇)-alkyl, (C₁-C₇)-alkyl-(C₃-C₇)-cycloalkyl, (C₃-C₇)-cycloalkylcarbonyl, (C₃-C₇)-cycloalkyl-(C₁-C₇)-alkylcarbonyl, (C₁-C₇)-alkylaminocarbonyl, (C₂-C₁₂)-dialkylaminocarbonyl, (C₁-C₇)-alkylaminosulfonyl, (C₂-C₁₂)-dialkylaminosulfonyl or tris[(C₁-C₇)alkyl]silyl, or aryl,
R³ represents hydrogen, halogen, cyano, (C₁-C₇)-alkyl, (C₁-C₇)-haloalkyl, (C₂-C₇)-alkenyl, (C₂-C₇)-haloalkenyl, (C₁-C₇)-alkoxy, (C₁-C₇) haloalkoxy, (C₁-C₇)-alkylthio, (C₁-C₇)-haloalkylthio, (C₃-C₇)-cycloalkyl, (C₃-C₇)-halocycloalkyl, (C₃-C₇)-cycloalkoxy or (C₃-C₇)-halocycloalkoxy,
R⁴ and R⁵ independently of one another represent hydrogen, (C₁-C₇)-alkyl, (C₁-C₇)-haloalkyl, (C₃-C₇)-cycloalkyl, (C₃-C₇)-halocycloalkyl, or R¹⁰O-(C₁-C₇)-alkyl, or
R⁴ and R⁵ together with the carbon atom to which they are bonded form a fully saturated, or partly saturated 3- to 10-membered monocyclic or bicyclic ring optionally interrupted by heteroatoms and optionally having further substitution,
R⁶ represents hydrogen, (C₁-C₇)-alkyl, (C₂-C₇)-alkenyl, (C₂-C₇)-alkynyl, cyano-(C₁-C₇)-alkyl, R¹⁰O-(C₁-C₇)-alkyl, R⁸R⁹N-(C₁-C₇)-alkyl, NR⁸R⁹, (C₃-C₁₀)-cycloalkyl-(C₁-C₇)-alkyl, C(=O)R¹⁰, C(=O)OR¹⁰, C(=O)NR⁸R⁹, R¹⁰O(O)C-(C₁-C₇)-alkyl, R¹⁰(O=)C-(C₁-C₇)-Alkyl, R⁸R⁹N(O)C-(C₁-C₇)-alkyl, SO₂R¹¹, R¹¹S(O)ₘ-(C₁-C₇)-alkyl, BR¹²R¹³, heterocyclyl, heteroaryl, aryl-(C₁-C₇)-alkyl, heteroaryl-(C₁-C₇)-alkyl, heterocyclyl-(C₁-C₇)-alkyl, tris[(C₁-C₇)alkyl]silyl, bis-[(C₁-C₇)-alkyl](aryl)silyl, (C₁-C₇)-alkyl-[bis-(aryl)]silyl, tris-[(C₁-C₇)-alkyl]silyl-(C₁-C₇)-alkyl, bis-[(C₁-C₇)-alkyl](aryl)silyl(C₁-C₇)-alkyl, or (C₁-C₇)-alkyl-[bis-(aryl)]silyl-(C₁-C₇)-alkyl,
m is 0, 1, 2,
n is 0, 1, 2,
R⁷ represents hydrogen, halogen, formyl, hydroxy, hydroxycarbonyl, nitro, cyano, amino, aminocarbonyl, aminothiocarbonyl, (C₁-C₇)-alkyl, (C₁-C₇)-haloalkyl, (C₂-C₇)-alkenyl, (C₂-C₇)-haloalkenyl, (C₂-C₇)-alkynyl, (C₂-C₇)-haloalkynyl, (C₁-C₇)-alkoxy, (C₁-C₇)-haloalkoxy, (C₁-C₇)-alkylthio, (C₁-C₇)-haloalkylthio, (C₁-C₇)-alkylsulfinyl, (C₁-C₇)-haloalkylsulfinyl, (C₁-C₇)-alkylsulfonyl, (C₁-C₇)-haloalkylsulfonyl, (C₁-C₇)-alkylcarbonyl, (C₁-C₇)-haloalkylcarbonyl, (C₂-C₇)-alkenylcarbonyl, (C₂-C₇)-haloalkenylcarbonyl, (C₂-C₇)-alkynylcarbonyl, (C₂-C₇)-haloalkynylcarbonyl, (C₁-C₇)-alkoxycarbonyl, (C₁-C₇)-haloalkoxycarbonyl, (C₃-C₇)-cycloalkyl, (C₃-C₇)-halocycloalkyl, (C₃-C₇)-cycloalkoxy, (C₃-C₇)-halocycloalkoxy, (C₃-C₇)-cycloalkylthio, (C₃-C₇)-halocycloalkylthio, (C₃-C₇)-cycloalkylsulfinyl, (C₃-C₇)-halocycloalkylsulfinyl, (C₃-C₇)-cycloalkylsulfonyl, (C₃-C₇)-halocycloalkylsulfonyl, (C₃-C₇)-cycloalkyl-(C₁-C₇)-alkyl, (C₁-C₇)-alkyl-(C₃-C₇)-cycloalkyl, (C₁-C₇)-alkoxycarbonyl-(C₁-C₇)-alkyl, hydroxycarbonyl-(C₁-C₇)-alkyl, (C₃-C₇)-cycloalkylcarbonyl, (C₃-C₇)-cycloalkyl-(C₁-C₇)-alkylcarbonyl, (C₁-C₇)-alkylamino, (C₂-C₁₂)-dialkylamino, (C₁-C₇)-alkylaminocarbonyl, (C₂-C₁₂)-dialkylaminocarbonyl, (C₁-C₇)-alkylaminosulfonyl, (C₂-C₁₂)-dialkylaminosulfonyl or tris[(C₁-C₇)alkyl]silyl,
R⁸ and R⁹ independently of one another represent hydrogen, (C₁-C₇)-alkyl, (C₂-C₇)-alkenyl, (C₂-C₇)-alkynyl, (C₁-C₇)-cyanoalkyl, (C₁-C₇)-haloalkyl, (C₂-C₇)-haloalkenyl, (C₃-C₇)-haloalkynyl, (C₃-C₇)-cycloalkyl, (C₃-C₇)-halocycloalkyl, (C₄-C₇)-cycloalkenyl, (C₄-C₁₀)-halocycloalkenyl, (C₁-C₇)-alkoxy-(C₁-C₇)-alkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-haloalkoxy-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, (C₃-C₇)-cycloalkyl-(C₁-C₇)-alkyl, (C₄-C₇)-cycloalkenyl-(C₁-C₇)-alkyl, COR¹⁰, SO₂R¹¹, heterocyclyl, (C₁-C₇)-alkoxycarbonyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkyl-aminocarbonyl-(C₁-C₇)-alkyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl, aryl-(C₁-C₇)-alkoxycarbonyl, heteroaryl-(C₁-C₇)-alkoxycarbonyl, (C₂-C₇)-alkenyloxycarbonyl, (C₂-C₇)-alkynyloxycarbonyl, heterocyclyl-(C₁-C₇)-alkyl, or
R⁸ and R⁹ together with the carbon atom to which they are bonded form a fully saturated or partly saturated 3- to 10-membered monocyclic or bicyclic ring optionally interrupted by heteroatoms and optionally having further substitution,
R¹⁰ represents hydrogen, (C₁-C₇)-alkyl, (C₂-C₇)-alkenyl, (C₂-C₇)-alkinyl, (C₁-C₇)-cyanoalkyl, (C₁-C₇)-haloalkyl, (C₂-C₇)-haloalkenyl, (C₃-C₇)-haloalkinyl, (C₃-C₇)-cycloalkyl, (C₃-C₇)-halocycloalkyl, (C₄-C₇)-cycloalkenyl, (C₄-C₇)-halocycloalkenyl, (C₁-C₇)-alkoxy-(C₁-C₇)-alkyl, (C₁-C₇)-haloalkoxy-(C₁-C₇)-alkyl, (C₁-C₇)-alkoxy-(C₁-C₇)-haloalkyl, (C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkyl, (C₁-C₇)-alkoxy-(C₁-C₇)-alkoxy-(C₁-C₇)-alkoxy-(C₁-C₇)-alkoxy-(C₁-C₇)-alkyl, aryl, aryl-(C₁-C₇)-alkyl, aryl-(C₁-C₇)-alkoxy-(C₁-C₇)-alkyl, aryloxy-(C₁-C₇)-alkyl, heteroaryl-(C₁-C₇)-alkoxy-(C₁-C₇)-alkyl, heteroaryloxy-(C₁-C₇)-alkyl, heteroaryl, heteroaryl-(C₁-C₇)-alkyl, (C₃-C₇)-cycloalkyl-(C₁-C₇)-alkyl, (C₄-C₇)-cycloalkenyl-(C₁-C₇)-alkyl, bis-[(C₁-C₇)-alkyl]aminocarbonyl-(C₁-C₇)-alkyl, (C₁-C₇)-alkyl-aminocarbonyl-(C₁-C₇)-alkyl, aryl-(C₁-C₇)-alkylaminocarbonyl-(C₁-C₇)-alkyl, bis-[(C₁-C₇)-alkyl]amino-(C₂-C₆)-alkyl, (C₁-C₇)-alkylamino-(C₂-C₆)-alkyl, aryl-(C₁-C₇)-alkylamino-(C₂-C₆)-alkyl, R¹¹(O)ₘS-(C₁-C₇)-alkyl, hydroxycarbonyl-(C₁-C₇)-alkyl, heterocyclyl, heterocyclyl-(C₁-C₇)-alkyl, heterocyclyl-(C₁-C₇)-alkoxy-(C₁-C₇)-alkyl, heterocyclyloxy-(C₁-C₇)-alkyl, tris-[(C₁-C₇)-alkyl]silyl-(C₁-C₇)-alkyl, bis-[(C₁-C₇)-alkyl](aryl)silyl(C₁-C₇)-alkyl, [(C₁-C₇)-alkyl]-bis-(aryl)silyl- (C₁-C₇)-alkyl, (C₁-C₇)-alkylcarbonyloxy-(C₁-C₇)-alkyl, (C₃-C₇)-cycloalkylcarbonyloxy-(C₁-C₇)-alkyl, arylcarbonyloxy-(C₁-C₇)-alkyl, heteroarylcarbonyloxy-(C₁-C₇)-alkyl, Heterocyclylcarbonyloxy-(C₁-C₇)-alkyl, (C₁-C₇)-alkoxycarbonyl, (C₁-C₇)-alkoxycarbonyl-(C₁-C₇)-alkyl, (C₂-C₇)-alkenyloxycarbonyl-(C₁-C₇)-alkyl, (C₂-C₇)-alkynyloxycarbonyl-(C₁-C₇)-alkyl, aryl-(C₁-C₇)-alkoxycarbonyl-(C₁-C₇)-alkyl, heteroaryl-(C₁-C₇)-alkoxycarbonyl-(C₁-C₇)-alkyl, heterocyclyl-(C₁-C₇)-alkoxycarbonyl-(C₁-C₇)-alkyl, hydroxy-(C₁-C₇)-alkyl, hydroxy-(C₁-C₇)-alkoxy-(C₁-C₇)-alkyl, hydroxy-(C₁-C₇)-alkoxy-(C₁-C₇)-alkoxy-(C₁-C₇)-alkyl, hydroxy-(C₁-C₇)-alkoxy-(C₁-C₇)-alkoxy-(C₁-C₇)-alkoxy-(C₁-C₇)-alkyl, (C₁-C₇)-alkylcarbonyloxy-(C₁-C₇)-alkoxy-(C₁-C₇)-alkyl, (C₁-C₇)-alkylcarbonyloxy-(C₁-C₇)-alkoxy-(C₁-C₇)-alkoxy-(C₁-C₇)-alkyl, (C₁-C₇)-alkylcarbonyloxy-(C₁-C₇)-alkoxy-(C₁-C₇)-alkoxy-(C₁-C₇)-alkoxy-(C₁-C₇)-alkyl, (C₁-C₇)-alkoxycarbonyl-(C₁-C₇)-alkoxy-(C₁-C₇)-alkyl, (C₁-C₇)-alkoxycarbonyl-(C₁-C₇)-alkoxy-(C₁-C₇)-alkoxy-(C₁-C₇)-alkyl, (C₁-C₇)-alkoxycarbonyl-(C₁-C₇)-alkoxy-(C₁-C₇)-alkoxy-(C₁-C₇)-alkoxy-(C₁-C₇)-alkyl, heteroarylcarbonyloxy-(C₁-C₇)-alkoxy-(C₁-C₇)-alkyl, heteroarylcarbonyloxy-(C₁-C₇)-alkoxy-(C₁-C₇)-alkoxy-(C₁-C₇)-alkyl, heteroarylcarbonyloxy-(C₁-C₇)-alkoxy-(C₁-C₇)-alkoxy-(C₁-C₇)-alkoxy-(C₁-C₇)-alkyl, heterocyclylcarbonyloxy-(C₁-C₇)-alkoxy-(C₁-C₇)-alkyl, heterocyclylcarbonyloxy-(C₁-C₇)-alkoxy-(C₁-C₇)-alkoxy-(C₁-C₇)-alkyl, heterocyclylcarbonyloxy-(C₁-C₇)-alkoxy-(C₁-C₇)-alkoxy-(C₁-C₇)-alkoxy-(C₁-C₇)-alkyl, hydroxycarbonyl-(C₁-C₇)-alkoxy-(C₁-C₇)-alkyl, hydroxycarbonyl-(C₁-C₇)-alkoxy-(C₁-C₇)-alkoxy-(C₁-C₇)-alkyl, or hydroxycarbonyl-(C₁-C₇)-alkoxy-(C₁-C₇)-alkoxy-(C₁-C₇)-alkoxy-(C₁-C₇)-alkyl,
-R¹¹ represents hydrogen, (C₁-C₇)-alkyl, (C₂-C₇)-alkenyl, (C₂-C₇)-alkynyl, (C₁-C₇)-cyanoalkyl, (C₁-C₇)-haloalkyl, (C₂-C₇)-haloalkenyl, (C₃-C₇)-haloalkynyl, (C₃-C₇)-cycloalkyl, (C₃-C₇)-halocycloalkyl, (C₄-C₇)-cycloalkenyl, (C₄-C₇)-halocycloalkenyl, (C₁-C₇)-alkoxy-(C₁-C₇)-alkyl, (C₁-C₇)-alkoxy-(C₁-C₇)-haloalkyl, aryl, aryl-(C₁-C₇)-alkyl, heteroaryl, heteroaryl-(C₁-C₇)-alkyl, heterocyclyl-(C₁-C₇)-alkyl, (C₃-C₇)-cycloalkyl-(C₁-C₇)-alkyl, (C₄-C₁₀)-cycloalkenyl-(C₁-C₇)-alkyl, bis-[(C₁-C₇)-alkyl]amino, (C₁-C₇)-alkyl-amino, aryl-(C₁-C₇)-amino, aryl-(C₁-C₆)-alkyl-amino, aryl-[(C₁-C₇)-alkyl]amino; heteroaryl-(C₁-C₇)-amino, heteroaryl-(C₁-C₆)-alkyl-amino, heteroaryl-[(C₁-C₇)-alkyl]amino; Hetercyclyl-(C₁-C₇)-amino, heterocyclyl-(C₁-C₆)-alkyl-amino, heterocyclyl-[(C₁-C₇)-alkyl]amino; (C₃-C₇)-cycloalkyl-amino, (C₃-C₇)-cycloalkyl-[(C₁-C₇)-alkyl]amino; N-azetidinyl, N-pyrrolidinyl, N-piperidinyl, or N-morpholinyl,
R¹² and R¹³ independently of one another represent hydrogen,NR⁸R⁹, OR¹⁰, or
R¹² and R¹³ together with the boron atom to which they are bonded form a fully saturated or partly saturated 3- to 10-membered monocyclic or bicyclic ring optionally interrupted by heteroatoms and optionally having further substitution.

3. The compound of the general formula (I) as claimed in claim 1 and/or the salt thereof, **characterized in that**
R¹ represents (C₃-C₆)-cycloalkyl, (C₃-C₆)-cycloalkenyl, (C₃-C₆)-cycloalkoxy, aryl, heteroaryl, heterocyclyl, a bicyclic or a heterobicyclic residue, wherein each of the previously mentioned eight residues is unsubstituted or is independently substituted by one or more residues selected from the group R⁷,
R² represents hydrogen, halogen, formyl, hydroxy, hydrothio, hydroxycarbonyl, aminocarbonyl, aminothiocarbonyl, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₁-C₆) haloalkoxy, (C₁-C₆)-alkoxy-(C₁-C₆)-alkyl, (C₁-C₆)-alkylthio, (C₁-C₆)-haloalkylthio, (C₁-C₆)-alkylsulfinyl, (C₁-C₆)-haloalkylsulfinyl, (C₁-C₆)-alkylsulfonyl, (C₁-C₆)-haloalkylsulfonyl, (C₁-C₆)-alkylcarbonyl, (C₂-C₆)-alkenylcarbonyl, (C₂-C₆)-alkynylcarbonyl, (C₁-C₆)-haloalkylcarbonyl, (C₂-C₆)-haloalkenylcarbonyl, (C₂-C₆)-haloalkynylcarbonyl, (C₁-C₆)-alkoxycarbonyl, (C₃-C₆)-cycloalkyl, (C₃-C₆)-cycloalkoxy, (C₃-C₆)-cycloalkylthio, (C₃-C₆)-cycloalkylsulfinyl, (C₃-C₆)-cycloalkylsulfonyl, (C₃-C₆)-halocycloalkyl, (C₃-C₆)-halocycloalkoxy, (C₃-C₆)-halocycloalkylthio, (C₃-C₆)-halocycloalkylsulfinyl, (C₃-C₆)-halocycloalkylsulfonyl, (C₃-C₆)-cycloalkyl-(C₁-C₆)-alkyl, (C₁-C₆)-alkyl-(C₃-C₆)-cycloalkyl, (C₃-C₆)-cycloalkylcarbonyl, (C₃-C₆)-cycloalkyl-(C₁-C₆)-alkylcarbonyl, (C₁-C₆)-alkylaminocarbonyl, (C₂-C₁₂)-dialkylaminocarbonyl, (C₁-C₆)-alkylaminosulfonyl, (C₂-C₁₂)-dialkylaminosulfonyl or tris[(C₁-C₆)alkyl]silyl, or 2,3,4,5,6-pentafluorophenyl,
R³ represents hydrogen, halogen, cyano, (C₁-C₆)-alkyl, (C₁-C₆)-haloalkyl, (C₂-C₆)-alkenyl, (C₂-C₆)-haloalkenyl, (C₁-C₆)-alkoxy, (C₁-C₆) haloalkoxy, (C₁-C₆)-alkylthio, (C₁-C₆)-haloalkylthio, (C₃-C₆)-cycloalkyl, (C₃-C₆)-halocycloalkyl, (C₃-C₆)-cycloalkoxy or (C₃-C₆)-halocycloalkoxy,
R⁴ and R⁵ independently of one another represent hydrogen, (C₁-C₆)-alkyl, (C₁-C₆)-haloalkyl, (C₃-C₆)-cycloalkyl, (C₃-C₆)-halocycloalkyl, or R¹⁰O-(C₁-C₆)-alkyl, or
R⁴ and R⁵ together with the carbon atom to which they are bonded form a fully saturated, or partly saturated 3- to 10-membered monocyclic or bicyclic ring optionally interrupted by heteroatoms and optionally having further substitution,
R⁶ represents hydrogen, (C₁-C₆)-alkyl, (C₂-C₆)-alkenyl, (C₂-C₆)-alkynyl, cyano-(C₁-C₆)-alkyl, R¹⁰O-(C₁-C₆)-alkyl, R⁸R⁹N-(C₁-C₆)-alkyl, NR⁸R⁹, (C₃-C₁₀)-cycloalkyl-(C₁-C₆)-alkyl, C(=O)R¹⁰, C(=O)OR¹⁰, C(=O)NR⁸R⁹, R¹⁰O(O)C-(C₁-C₆)-alkyl, R¹⁰(O=)C-(C₁-C₆)-Alkyl, R⁸R⁹N(O)C-(C₁-C₆)-alkyl, SO₂R¹¹, R¹¹S(O)ₘ-(C₁-C₆)-alkyl, BR¹²R¹³, heterocyclyl, heteroaryl, aryl-(C₁-C₆)-alkyl, heteroaryl-(C₁-C₆)-alkyl, heterocyclyl-(C₁-C₆)-alkyl, tris[(C₁-C₆)alkyl]silyl, bis-[(C₁-C₆)-alkyl](aryl)silyl, (C₁-C₆)-alkyl-[bis-(aryl)]silyl, tris-[(C₁-C₆)-alkyl]silyl-(C₁-C₆)-alkyl, bis-[(C₁-C₆)-alkyl](aryl)silyl(C₁-C₆)-alkyl, or (C₁-C₆)-alkyl-[bis-(aryl)]silyl-(C₁-C₆)-alkyl,
m is 0, 1, 2,
n is 0, 1, 2,
R⁷ represents hydrogen, halogen, formyl, hydroxy, hydroxycarbonyl, nitro, cyano, amino, aminocarbonyl, aminothiocarbonyl, (C₁-C₆)-alkyl, (C₁-C₆)-haloalkyl, (C₂-C₆)-alkenyl, (C₂-C₆)-haloalkenyl, (C₂-C₆)-alkynyl, (C₂-C₆)-haloalkynyl, (C₁-C₆)-alkoxy, (C₁-C₆)-haloalkoxy, (C₁-C₆)-alkylthio, (C₁-C₆)-haloalkylthio, (C₁-C₆)-alkylsulfinyl, (C₁-C₆)-haloalkylsulfinyl, (C₁-C₆)-alkylsulfonyl, (C₁-C₆)-haloalkylsulfonyl, (C₁-C₆)-alkylcarbonyl, (C₁-C₆)-haloalkylcarbonyl, (C₂-C₆)-alkenylcarbonyl, (C₂-C₆)-haloalkenylcarbonyl, (C₂-C₆)-alkynylcarbonyl, (C₂-C₆)-haloalkynylcarbonyl, (C₁-C₆)-alkoxycarbonyl, (C₁-C₆)-haloalkoxycarbonyl, (C₃-C₆)-cycloalkyl, (C₃-C₆)-halocycloalkyl, (C₃-C₆)-cycloalkoxy, (C₃-C₆)-halocycloalkoxy, (C₃-C₆)-cycloalkylthio, (C₃-C₆)-halocycloalkylthio, (C₃-C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyl-(C₁-C₆)-alkyl, (C₁-C₆)-alkyl-(C₃-C₆)-cycloalkyl, (C₁-C₆)-alkoxycarbonyl-(C₁-C₆)-alkyl, hydroxycarbonyl-(C₁-C₆)-alkyl, (C₃-C₆)-cycloalkylcarbonyl, (C₃-C₆)-cycloalkyl-(C₁-C₆)-alkylcarbonyl, (C₁-C₆)-alkylamino, (C₂-C₁₂)-dialkylamino, (C₁-C₆)-alkylaminocarbonyl, (C₂-C₁₂)-dialkylaminocarbonyl, (C₁-C₆)-alkylaminosulfonyl, (C₂-C₁₂)-dialkylaminosulfonyl or tris[(C₁-C₆)alkyl]silyl,
R⁸ and R⁹ independently of one another represent hydrogen, (C₁-C₆)-alkyl, (C₂-C₆)-alkenyl, (C₂-C₆)-alkynyl, (C₁-C₆)-cyanoalkyl, (C₁-C₆)-haloalkyl, (C₂-C₆)-haloalkenyl, (C₃-C₆)-haloalkynyl, (C₃-C₆)-cycloalkyl, (C₃-C₆)-halocycloalkyl, (C₄-C₆)-cycloalkenyl, (C₄-C₆)- halocycloalkenyl, (C₁-C₆)-alkoxy-(C₁-C₆)-alkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-haloalkoxy-(C₁-C₆)-alkyl, (C₁-C₆)-alkylthio-(C₁-C₆)-alkyl, (C₁-C₆)-haloalkylthio-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, (C₃-C₆)-cycloalkyl-(C₁-C₆)-alkyl, (C₄-C₆)-cycloalkenyl-(C₁-C₆)-alkyl, COR¹⁰, SO₂R¹¹, heterocyclyl, (C₁-C₆)-alkoxycarbonyl, bis-[(C₁-C₆)-alkyl]aminocarbonyl-(C₁-C₆)-alkyl, (C₁-C₆)-alkyl-aminocarbonyl-(C₁-C₆)-alkyl, aryl-(C₁-C₆)-alkyl-aminocarbonyl-(C₁-C₆)-alkyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl, aryl-(C₁-C₆)-alkoxycarbonyl, heteroaryl-(C₁-C₆)-alkoxycarbonyl, (C₂-C₆)-alkenyloxycarbonyl, (C₂-C₆)-alkynyloxycarbonyl, heterocyclyl-(C₁-C₆)-alkyl, or
-R⁸ and R⁹ together with the carbon atom to which they are bonded form a fully saturated or partly saturated 3- to 10-membered monocyclic or bicyclic ring optionally interrupted by heteroatoms and optionally having further substitution,
R¹⁰ represents hydrogen, (C₁-C₆)-alkyl, (C₂-C₆)-alkenyl, (C₂-C₆)-alkinyl, (C₁-C₆)-cyanoalkyl, (C₁-C₆)-haloalkyl, (C₂-C₆)-haloalkenyl, (C₃-C₆)-haloalkinyl, (C₃-C₆)-cycloalkyl, (C₃-C₆)-halocycloalkyl, (C₄-C₆)-cycloalkenyl, (C₄-C₆)-halocycloalkenyl, (C₁-C₆)-alkoxy-(C₁-C₆)-alkyl, (C₁-C₆)-haloalkoxy-(C₁-C₆)-alkyl, (C₁-C₆)-alkoxy-(C₁-C₆)-haloalkyl, (C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkyl, (C₁-C₆)-alkoxy-(C₁-C₆)-alkoxy-(C₁-C₆)-alkoxy-(C₁-C₆)-alkoxy-(C₁-C₆)-alkyl, aryl, aryl-(C₁-C₆)-alkyl, aryl-(C₁-C₆)-alkoxy-(C₁-C₆)-alkyl, aryloxy-(C₁-C₆)-alkyl, heteroaryl-(C₁-C₆)-alkoxy-(C₁-C₆)-alkyl, heteroaryloxy-(C₁-C₆)-alkyl, heteroaryl, heteroaryl-(C₁-C₆)-alkyl, (C₃-C₆)-cycloalkyl-(C₁-C₆)-alkyl, (C₄-C₆)-cycloalkenyl-(C₁-C₆)-alkyl, bis-[(C₁-C₆)-alkyl]aminocarbonyl-(C₁-C₆)-alkyl, (C₁-C₆)-alkyl-aminocarbonyl-(C₁-C₆)-alkyl, aryl-(C₁-C₆)-alkylaminocarbonyl-(C₁-C₆)-alkyl, bis-[(C₁-C₆)-alkyl]amino-(C₂-C₆)-alkyl, (C₁-C₆)-alkylamino-(C₂-C₆)-alkyl, aryl-(C₁-C₆)-alkylamino-(C₂-C₆)-alkyl, R¹¹(O)ₘS-(C₁-C₆)-alkyl, hydroxycarbonyl-(C₁-C₆)-alkyl, heterocyclyl, heterocyclyl-(C₁-C₆)-alkyl, heterocyclyl-(C₁-C₆)-alkoxy-(C₁-C₆)-alkyl, heterocyclyloxy-(C₁-C₆)-alkyl, tris-[(C₁-C₆)-alkyl]silyl-(C₁-C₆)-alkyl, bis-[(C₁-C₆)-alkyl](aryl)silyl(C₁-C₆)-alkyl, [(C₁-C₆)-alkyl]-bis-(aryl)silyl-(C₁-C₆)-alkyl, (C₁-C₆)-alkylcarbonyloxy-(C₁-C₆)-alkyl, (C₃-C₆)-cycloalkylcarbonyloxy-(C₁-C₆)-alkyl, arylcarbonyloxy-(C₁-C₆)-alkyl, heteroarylcarbonyloxy-(C₁-C₆)-alkyl, Heterocyclylcarbonyloxy-(C₁-C₆)-alkyl, (C₁-C₆)-alkoxycarbonyl, (C₁-C₆)-alkoxycarbonyl-(C₁-C₆)-alkyl, (C₂-C₆)-alkenyloxycarbonyl-(C₁-C₆)-alkyl, (C₂-C₆)-alkynyloxycarbonyl-(C₁-C₆)-alkyl, aryl-(C₁-C₆)-alkoxycarbonyl-(C₁-C₆)-alkyl, heteroaryl-(C₁-C₆)-alkoxycarbonyl-(C₁-C₆)-alkyl, heterocyclyl-(C₁-C₆)-alkoxycarbonyl-(C₁-C₆)-alkyl, hydroxy-(C₁-C₆)-alkyl, hydroxy-(C₁-C₆)-alkoxy-(C₁-C₆)-alkyl, hydroxy-(C₁-C₆)-alkoxy-(C₁-C₆)-alkoxy-(C₁-C₆)-alkyl, hydroxy-(C₁-C₆)-alkoxy-(C₁-C₆)-alkoxy-(C₁-C₆)-alkoxy-(C₁-C₆)-alkyl, (C₁-C₆)-alkylcarbonyloxy-(C₁-C₆)-alkoxy-(C₁-C₆)-alkyl, (C₁-C₆)-alkylcarbonyloxy-(C₁-C₆)-alkoxy-(C₁-C₆)-alkoxy-(C₁-C₆)-alkyl, (C₁-C₆)-alkylcarbonyloxy-(C₁-C₆)-alkoxy-(C₁-C₆)-alkoxy-(C₁-C₆)-alkoxy-(C₁-C₆)-alkyl, (C₁-C₆)-alkoxycarbonyl-(C₁-C₆)-alkoxy-(C₁-C₆)-alkyl, (C₁-C₆)-alkoxycarbonyl-(C₁-C₆)-alkoxy-(C₁-C₆)-alkoxy-(C₁-C₆)-alkyl, (C₁-C₆)-alkoxycarbonyl-(C₁-C₆)-alkoxy-(C₁-C₆)-alkoxy-(C₁-C₆)-alkoxy-(C₁-C₆)-alkyl, heteroarylcarbonyloxy-(C₁-C₆)-alkoxy-(C₁-C₆)-alkyl, heteroarylcarbonyloxy-(C₁-C₆)-alkoxy-(C₁-C₆)-alkoxy-(C₁-C₆)-alkyl, heteroarylcarbonyloxy-(C₁-C₆)-alkoxy-(C₁-C₆)-alkoxy-(C₁-C₆)-alkoxy-(C₁-C₆)-alkyl, heterocyclylcarbonyloxy-(C₁-C₆)-alkoxy-(C₁-C₆)-alkyl, heterocyclylcarbonyloxy-(C₁-C₆)-alkoxy-(C₁-C₆)-alkoxy-(C₁-C₆)-alkyl, heterocyclylcarbonyloxy-(C₁-C₆)-alkoxy-(C₁-C₆)-alkoxy-(C₁-C₆)-alkoxy-(C₁-C₆)-alkyl, hydroxycarbonyl-(C₁-C₆)-alkoxy-(C₁-C₆)-alkyl, or hydroxycarbonyl-(C₁-C₆)-alkoxy-(C₁-C₆)-alkoxy-(C₁-C₆)-alkyl, hydroxycarbonyl-(C₁-C₆)-alkoxy-(C₁-C₆)-alkoxy-(C₁-C₆)-alkoxy-(C₁-C₆)-alkyl,
R¹¹ represents hydrogen, (C₁-C₆)-alkyl, (C₂-C₆)-alkenyl, (C₂-C₆)-alkynyl, (C₁-C₆)-cyanoalkyl, (C₁-C₆)-haloalkyl, (C₂-C₆)-haloalkenyl, (C₃-C₆)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₆)-alkoxy-(C₁-C₆)-alkyl, (C₁-C₆)-alkoxy-(C₁-C₆)-haloalkyl, aryl, aryl-(C₁-C₆)-alkyl, heteroaryl, heteroaryl-(C₁-C₆)-alkyl, heterocyclyl-(C₁-C₆)-alkyl, (C₃-C₆)-cycloalkyl-(C₁-C₆)-alkyl, (C₄-C₁₀)-cycloalkenyl-(C₁-C₆)-alkyl, bis-[(C₁-C₆)-alkyl]amino, (C₁-C₆)-alkyl-amino, aryl-(C₁-C₆)-amino, aryl-(C₁-C₆)-alkyl-amino, aryl-[(C₁-C₆)-alkyl]amino; heteroaryl-(C₁-C₆)-amino, heteroaryl-(C₁-C₆)-alkyl-amino, heteroaryl-[(C₁-C₆)-alkyl]amino; Hetercyclyl-(C₁-C₆)-amino, heterocyclyl-(C₁-C₆)-alkyl-amino, heterocyclyl-[(C₁-C₆)-alkyl]amino; (C₃-C₆)-cycloalkyl-amino, (C₃-C₆)-cycloalkyl-[(C₁-C₆)-alkyl]amino; N-azetidinyl, N-pyrrolidinyl, N-piperidinyl, or N-morpholinyl,
R¹² and R¹³ independently of one another represent hydrogen, NR⁸R⁹, OR¹⁰, or
R¹² and R¹³ together with the boron atom to which they are bonded form a fully saturated or partly saturated 3- to 10-membered monocyclic or bicyclic ring optionally interrupted by heteroatoms and optionally having further substitution.

4. The compound of the general formula (I) as claimed in claim 1 and/or the salt thereof, **characterized in that**
R¹ represents phenyl, furyl, pyrrolyl, thienyl, pyridyl, pyrimidinyl, pyridazinyl, pyrazinyl, thiazolyl, isothiazolyl, thiadiazolyl, oxazolyl, isoxazolyl, pyrazolyl, imidazolyl, triazolyl, tetrazolyl, cyclopentenyl, cyclohexenyl or an oxabicycloheptanyl residue, wherein each of the previously mentioned 20 residues is unsubstituted or is independently substituted by one or more residues selected from the group R⁷,
R² represents hydrogen, fluoro, chloro, bromo, iodo, formyl, hydroxy, hydrothio, hydroxycarbonyl, aminocarbonyl, aminothiocarbonyl, (C₁-C₅)-alkyl, (C₁-C₅)-haloalkyl, (C₂-C₅)-alkenyl, (C₂-C₅)-haloalkenyl, (C₂-C₅)-alkynyl, (C₂-C₅)-haloalkynyl, (C₁-C₅)-alkoxy, (C₁-C₅)-haloalkoxy, (C₁-C₅)-alkoxy-(C₁-C₅)-alkyl, (C₁-C₅)-alkylthio, (C₁-C₅)-haloalkylthio, (C₁-C₅)-alkylsulfinyl, (C₁-C₅)-haloalkylsulfinyl, (C₁-C₅)-alkylsulfonyl, (C₁-C₅)-haloalkylsulfonyl, (C₁-C₅)-alkylcarbonyl, (C₂-C₅)-alkenylcarbonyl, (C₂-C₅)-alkynylcarbonyl, (C₁-C₅)-haloalkylcarbonyl, (C₂-C₅)-haloalkenylcarbonyl, (C₂-C₅)-haloalkynylcarbonyl, (C₁-C₅)-alkoxycarbonyl, (C₃-C₆)-cycloalkyl, (C₃-C₆)-cycloalkoxy, (C₃-C₆)-cycloalkylthio, (C₃-C₆)-cycloalkylsulfinyl, (C₃-C₆)-cycloalkylsulfonyl, (C₃-C₅)-halocycloalkyl, (C₃-C₅)-halocycloalkoxy, (C₃-C₅)-halocycloalkylthio, (C₃-C₅)-halocycloalkylsulfinyl, (C₃-C₅)-halocycloalkylsulfonyl, (C₃-C₆)-cycloalkyl-(C₁-C₅)-alkyl, (C₁-C₅)-alkyl-(C₃-C₆)-cycloalkyl, (C₃-C₆)-cycloalkylcarbonyl, (C₃-C₆)-cycloalkyl-(C₁-C₅)-alkylcarbonyl, (C₁-C₅)-alkylaminocarbonyl, (C₂-C₁₀)-dialkylaminocarbonyl, (C₁-C₅)-alkylaminosulfonyl, (C₂-C₁₀)-dialkylaminosulfonyl or tris[(C₁-C₅)alkyl]silyl, 2,3,4,5,6-pentafluorophenyl,
R³ represents hydrogen, fluoro, chloro, bromo, iodo, cyano, (C₁-C₅)-alkyl, (C₁-C₅)-haloalkyl, (C₂-C₅)-alkenyl, (C₂-C₅)-haloalkenyl, (C₁-C₅)-alkoxy, (C₁-C₅) haloalkoxy, (C₁-C₅)-alkylthio, (C₁-C₅)-haloalkylthio, (C₃-C₆)-cycloalkyl, (C₃-C₅)-halocycloalkyl, (C₃-C₆)-cycloalkoxy or (C₃-C₆)-halocycloalkoxy,
R⁴ and R⁵ independently of one another represent hydrogen, (C₁-C₅)-alkyl, (C₁-C₅)-haloalkyl, (C₃-C₆)-cycloalkyl, (C₃-C₆)-halocycloalkyl, or R¹⁰O-(C₁-C₅)-alkyl, or
R⁴ and R⁵ together with the carbon atom to which they are bonded form a fully saturated, or partly saturated 3- to 10-membered monocyclic or bicyclic ring optionally interrupted by heteroatoms and optionally having further substitution,
R⁶ represents hydrogen, (C₁-C₅)-alkyl, (C₂-C₅)-alkenyl, (C₂-C₅)-alkynyl, cyano-(C₁-C₅)-alkyl, R¹⁰O-(C₁-C₅)-alkyl, R⁸R⁹N-(C₁-C₅)-alkyl, NR⁸R⁹, (C₃-C₆)-cycloalkyl-(C₁-C₅)-alkyl, C(=O)R¹⁰, C(=O)OR¹⁰, C(=O)NR⁸R⁹, R¹⁰O(O)C-(C₁-C₅)-alkyl, R¹⁰(O=)C-(C₁-C₅)-Alkyl, R⁸R⁹N(O)C-(C₁-C₅)-alkyl, SO₂R¹¹, R¹¹S(O)ₘ-(C₁-C₅)-alkyl, BR¹²R¹³, heterocyclyl, heteroaryl, aryl-(C₁-C₅)-alkyl, heteroaryl-(C₁-C₅)-alkyl, heterocyclyl-(C₁-C₅)-alkyl, tris[(C₁-C₅)alkyl]silyl, bis-[(C₁-C₅)-alkyl](aryl)silyl, (C₁-C₅)-alkyl-[bis-(aryl)]silyl, tris-[(C₁-C₅)-alkyl]silyl-(C₁-C₅)-alkyl, bis-[(C₁-C₅)-alkyl](aryl)silyl(C₁-C₅)-alkyl, or (C₁-C₅)-alkyl-[bis-(aryl)]silyl-(C₁-C₅)-alkyl,
m is 0, 1, 2,
n is 0, 1, 2,
R⁷ represents hydrogen, fluoro, chloro, bromo, iodo, formyl, hydroxy, hydroxycarbonyl, nitro, cyano, amino, aminocarbonyl, aminothiocarbonyl, (C₁-C₅)-alkyl, (C₁-C₅)-haloalkyl, (C₂-C₅)-alkenyl, (C₂-C₅)-haloalkenyl, (C₂-C₅)-alkynyl, (C₂-C₅)-haloalkynyl, (C₁-C₅)-alkoxy, (C₁-C₅)-haloalkoxy, (C₁-C₅)-alkylthio, (C₁-C₅)-haloalkylthio, (C₁-C₅)-alkylsulfinyl, (C₁-C₅)-haloalkylsulfinyl, (C₁-C₅)-alkylsulfonyl, (C₁-C₅)-haloalkylsulfonyl, (C₁-C₅)-alkylcarbonyl, (C₁-C₅)-haloalkylcarbonyl, (C₂-C₅)-alkenylcarbonyl, (C₂-C₅)-haloalkenylcarbonyl, (C₂-C₅)-alkynylcarbonyl, (C₂-C₅)-haloalkynylcarbonyl, (C₁-C₅)-alkoxycarbonyl, (C₁-C₅)-haloalkoxycarbonyl, (C₃-C₆)-cycloalkyl, (C₃-C₆)-halocycloalkyl, (C₃-C₆)-cycloalkoxy, (C₃-C₆)-halocycloalkoxy, (C₃-C₆)-cycloalkylthio, (C₃-C₆)-halocycloalkylthio, (C₃-C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyl-(C₁-C₅)-alkyl, (C₁-C₅)-alkyl-(C₃-C₆)-cycloalkyl, (C₁-C₅)-alkoxycarbonyl-(C₁-C₅)-alkyl, hydroxycarbonyl-(C₁-C₅)-alkyl, (C₃-C₆)-cycloalkylcarbonyl, (C₃-C₆)-cycloalkyl-(C₁-C₅)-alkylcarbonyl, (C₁-C₅)-alkylamino, (C₂-C₁₀)-dialkylamino, (C₁-C₅)-alkylaminocarbonyl, (C₂-C₁₀)-dialkylaminocarbonyl, (C₁-C₅)-alkylaminosulfonyl, (C₂-C₁₀)-dialkylaminosulfonyl or tris[(C₁-C₅)alkyl]silyl,
R⁸ and R⁹ independently of one another represent hydrogen, (C₁-C₅)-alkyl, (C₂-C₅)-alkenyl, (C₂-C₅)-alkynyl, (C₁-C₅)-cyanoalkyl, (C₁-C₅)-haloalkyl, (C₂-C₅)-haloalkenyl, (C₃-C₅)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₅)-alkoxy-(C₁-C₅)-alkyl, (C₁-C₅)-alkoxy-(C₁-C₅)-alkoxy-(C₁-C₅)-alkyl, (C₁-C₅)-alkoxy-(C₁-C₅)-alkoxy-(C₁-C₅)-alkoxy-(C₁-C₅)-alkyl, (C₁-C₅)-haloalkoxy-(C₁-C₅)-alkyl, (C₁-C₅)-alkylthio-(C₁-C₅)-alkyl, (C₁-C₅)-haloalkylthio- (C₁-C₅)-alkyl, (C₁-C₅)-alkoxy-(C₁-C₅)-haloalkyl, aryl, aryl-(C₁-C₅)-alkyl, heteroaryl, heteroaryl-(C₁-C₅)-alkyl, (C₃-C₆)-cycloalkyl-(C₁-C₅)-alkyl, (C₄-C₆)-cycloalkenyl-(C₁-C₅)-alkyl, COR¹⁰, SO₂R¹¹, heterocyclyl, (C₁-C₅)-alkoxycarbonyl, bis-[(C₁-C₅)-alkyl]aminocarbonyl-(C₁-C₅)-alkyl, (C₁-C₅)-alkyl-aminocarbonyl-(C₁-C₅)-alkyl, aryl-(C₁-C₅)-alkyl-aminocarbonyl-(C₁-C₅)-alkyl, (C₁-C₅)-alkoxycarbonyl-(C₁-C₅)-alkyl, (C₂-C₅)-alkenyloxycarbonyl-(C₁-C₅)-alkyl, (C₂-C₅)-alkynyloxycarbonyl-(C₁-C₅)-alkyl, aryl-(C₁-C₅)-alkoxycarbonyl-(C₁-C₅)-alkyl, heteroaryl-(C₁-C₅)-alkoxycarbonyl-(C₁-C₅)-alkyl, heterocyclyl-(C₁-C₅)-alkoxycarbonyl-(C₁-C₅)-alkyl, aryl-(C₁-C₅)-alkoxycarbonyl, heteroaryl-(C₁-C₅)-alkoxycarbonyl, (C₂-C₅)-alkenyloxycarbonyl, (C₂-C₅)-alkynyloxycarbonyl, heterocyclyl-(C₁-C₅)-alkyl, or
-R⁸ and R⁹ together with the carbon atom to which they are bonded form a fully saturated or partly saturated 3- to 10-membered monocyclic or bicyclic ring optionally interrupted by heteroatoms and optionally having further substitution,
R¹⁰ represents hydrogen, (C₁-C₅)-alkyl, (C₂-C₅)-alkenyl, (C₂-C₅)-alkinyl, (C₁-C₅)-cyanoalkyl, (C₁-C₅)-haloalkyl, (C₂-C₅)-haloalkenyl, (C₃-C₅)-haloalkinyl, (C₃-C₆)-cycloalkyl, (C₃-C₆)-halocycloalkyl, (C₄-C₆)-cycloalkenyl, (C₄-C₆)-halocycloalkenyl, (C₁-C₅)-alkoxy-(C₁-C₅)-alkyl, (C₁-C₅)-haloalkoxy-(C₁-C₅)-alkyl, (C₁-C₅)-alkoxy-(C₁-C₅)-haloalkyl, (C₁-C₅)-alkoxy-(C₁-C₅)-alkoxy-(C₁-C₅)-alkyl, (C₁-C₅)-alkoxy-(C₁-C₅)-alkoxy-(C₁-C₅)-alkoxy-(C₁-C₅)-alkyl, (C₁-C₅)-alkoxy-(C₁-C₅)-alkoxy-(C₁-C₅)-alkoxy-(C₁-C₅)-alkoxy-(C₁-C₅)-alkyl, aryl, aryl-(C₁-C₅)-alkyl, aryl-(C₁-C₅)-alkoxy-(C₁-C₅)-alkyl, aryloxy-(C₁-C₅)-alkyl, heteroaryl-(C₁-C₅)-alkoxy-(C₁-C₅)-alkyl, heteroaryloxy-(C₁-C₅)-alkyl, heteroaryl, heteroaryl-(C₁-C₅)-alkyl, (C₃-C₆)-cycloalkyl-(C₁-C₅)-alkyl, (C₄-C₆)-cycloalkenyl-(C₁-C₅)-alkyl, bis-[(C₁-C₅)-alkyl]aminocarbonyl-(C₁-C₅)-alkyl, (C₁-C₅)-alkyl-aminocarbonyl-(C₁-C₅)-alkyl, aryl-(C₁-C₅)-alkylaminocarbonyl-(C₁-C₅)-alkyl, bis-[(C₁-C₅)-alkyl]amino-(C₂-C₅)-alkyl, (C₁-C₅)-alkylamino-(C₂-C₅)-alkyl, aryl-(C₁-C₅)-alkylamino-(C₂-C₅)-alkyl, R¹¹(O)ₘS-(C₁-C₅)-alkyl, hydroxycarbonyl-(C₁-C₅)-alkyl, heterocyclyl, heterocyclyl-(C₁-C₅)-alkyl, heterocyclyl-(C₁-C₅)-alkoxy-(C₁-C₅)-alkyl, heterocyclyloxy-(C₁-C₅)-alkyl, tris-[(C₁-C₅)-alkyl]silyl-(C₁-C₅)-alkyl, bis-[(C₁-C₅)-alkyl](aryl)silyl(C₁-C₅)-alkyl, [(C₁-C₅)-alkyl]-bis-(aryl)silyl-(C₁-C₅)-alkyl, (C₁-C₅)-alkylcarbonyloxy-(C₁-C₅)-alkyl, (C₃-C₆)-cycloalkylcarbonyloxy-(C₁-C₅)-alkyl, arylcarbonyloxy-(C₁-C₅)-alkyl, heteroarylcarbonyloxy-(C₁-C₅)-alkyl, Heterocyclylcarbonyloxy-(C₁-C₅)-alkyl, (C₁-C₅)-alkoxycarbonyl, (C₁-C₅)-alkoxycarbonyl-(C₁-C₅)-alkyl, (C₂-C₅)-alkenyloxycarbonyl-(C₁-C₅)-alkyl, (C₂-C₅)-alkynyloxycarbonyl-(C₁-C₅)-alkyl, aryl-(C₁-C₅)-alkoxycarbonyl-(C₁-C₅)-alkyl, heteroaryl-(C₁-C₅)-alkoxycarbonyl-(C₁-C₅)-alkyl, heterocyclyl-(C₁-C₅)-alkoxycarbonyl-(C₁-C₅)-alkyl, hydroxy-(C₁-C₅)-alkyl, hydroxy-(C₁-C₅)-alkoxy-(C₁-C₅)-alkyl, hydroxy-(C₁-C₅)-alkoxy-(C₁-C₅)-alkoxy-(C₁-C₅)-alkyl, hydroxy-(C₁-C₅)-alkoxy-(C₁-C₅)-alkoxy-(C₁-C₅)-alkoxy-(C₁-C₅)-alkyl, (C₁-C₅)-alkylcarbonyloxy-(C₁-C₅)-alkoxy-(C₁-C₅)-alkyl, (C₁-C₅)-alkylcarbonyloxy-(C₁-C₅)-alkoxy-(C₁-C₅)-alkoxy-(C₁-C₅)-alkyl, (C₁-C₅)-alkylcarbonyloxy-(C₁-C₅)-alkoxy-(C₁-C₅)-alkoxy-(C₁-C₅)-alkoxy-(C₁-C₅)-alkyl, (C₁-C₅)-alkoxycarbonyl-(C₁-C₅)-alkoxy-(C₁-C₅)-alkyl, (C₁-C₅)-alkoxycarbonyl-(C₁-C₅)-alkoxy-(C₁-C₅)-alkoxy-(C₁-C₅)-alkyl, (C₁-C₅)-alkoxycarbonyl-(C₁-C₅)-alkoxy-(C₁-C₅)-alkoxy-(C₁-C₅)-alkoxy-(C₁-C₅)-alkyl, heteroarylcarbonyloxy-(C₁-C₅)-alkoxy-(C₁-C₅)-alkyl, heteroarylcarbonyloxy-(C₁-C₅)-alkoxy-(C₁-C₅)-alkoxy-(C₁-C₅)-alkyl, heteroarylcarbonyloxy-(C₁-C₅)-alkoxy-(C₁-C₅)-alkoxy-(C₁-C₅)-alkoxy-(C₁-C₅)-alkyl, heterocyclylcarbonyloxy-(C₁-C₅)-alkoxy-(C₁-C₅)-alkyl, heterocyclylcarbonyloxy-(C₁-C₅)-alkoxy-(C₁-C₅)-alkoxy-(C₁-C₅)-alkyl, heterocyclylcarbonyloxy-(C₁-C₅)-alkoxy-(C₁-C₅)-alkoxy-(C₁-C₅)-alkoxy-(C₁-C₅)-alkyl, hydroxycarbonyl-(C₁-C₅)-alkoxy-(C₁-C₅)-alkyl, hydroxycarbonyl-(C₁-C₅)-alkoxy-(C₁-C₅)-alkoxy-(C₁-C₅)-alkyl, or hydroxycarbonyl-(C₁-C₅)-alkoxy-(C₁-C₅)-alkoxy-(C₁-C₅)-alkoxy-(C₁-C₅)-alkyl,
R¹¹ represents hydrogen, (C₁-C₅)-alkyl, (C₂-C₅)-alkenyl, (C₂-C₅)-alkynyl, (C₁-C₅)-cyanoalkyl, (C₁-C₅)-haloalkyl, (C₂-C₅)-haloalkenyl, (C₃-C₅)-haloalkynyl, (C₃-C₆)-cycloalkyl, (C₃-C₆)-halocycloalkyl, (C₄-C₆)-cycloalkenyl, (C₄-C₆)-halocycloalkenyl, (C₁-C₅)-alkoxy-(C₁-C₅)-alkyl, (C₁-C₅)-alkoxy-(C₁-C₅)-haloalkyl, aryl, aryl-(C₁-C₅)-alkyl, heteroaryl, heteroaryl-(C₁-C₅)-alkyl, heterocyclyl-(C₁-C₅)-alkyl, (C₃-C₆)-cycloalkyl-(C₁-C₅)-alkyl, (C₄-C₁₀)-cycloalkenyl-(C₁-C₅)-alkyl, bis-[(C₁-C₅)-alkyl]amino, (C₁-C₅)-alkyl-amino, aryl-(C₁-C₅)-amino, aryl-(C₁-C₅)-alkyl-amino, aryl-[(C₁-C₅)-alkyl]amino; heteroaryl-(C₁-C₅)-amino, heteroaryl-(C₁-C₅)-alkyl-amino, heteroaryl-[(C₁-C₅)-alkyl]amino; Hetercyclyl-(C₁-C₅)-amino, heterocyclyl-(C₁-C₅)-alkyl-amino, heterocyclyl-[(C₁-C₅)-alkyl]amino; (C₃-C₆)-cycloalkyl-amino, (C₃-C₆)-cycloalkyl-[(C₁-C₅)-alkyl]amino; N-azetidinyl, N-pyrrolidinyl, N-piperidinyl, N-morpholinyl.
R¹² and R¹³ independently of one another represent hydrogen, NR⁸R⁹, OR¹⁰, or
R¹² and R¹³ together with the boron atom to which they are bonded form a fully saturated or partly saturated 3- to 10-membered monocyclic or bicyclic ring optionally interrupted by heteroatoms and optionally having further substitution.

5. The compound of the general formula (I) as claimed in claim 1 and/or the salt thereof, **characterized in that**
R¹ represents phenyl, 2-fluorophenyl, 3-fluorophenyl, 4-fluorophenyl, 2-chlorophenyl, 3-chlorophenyl, 4-chlorophenyl, 2-bromophenyl, 2-methylphenyl, 2-ethylphenyl, 2-methoxyphenyl, 2-trifluoromethylphenyl, 2,3-difluorophenyl, 2,4-difluorophenyl, 2,5-difluorophenyl, 2,6-difluorophenyl, 2,3,4-trifluorophenyl, 2,4,5-trifluorophenyl, 2,4,6-trifluorophenyl, 2-fluoro-6-methylphenyl, 2,4-dichlorophenyl, 2,5-dichlorophenyl, 2,6-dichlorophenyl, 3,5-dichlorophenyl, 2,6-dimethylphenyl, 2-chloro-6-methylphenyl, 2-bromo-6-fluorophenyl, 2-bromo-6-chlorophenyl, 2-bromo-6-methylphenyl, 2-bromo-6-methoxyphenyl, 2-fluoro-3-chlorophenyl, 2-fluoro-3-methylphenyl, 2-chloro-3-fluorophenyl, 2-fluoro-4-chlorophenyl, 2-fluoro-6-chlorophenyl, 2,3-dimethylphenyl, 2,4-dimethylphenyl, 2-methyl-3-fluorophenyl, 2-thienyl, 3-fluoro-2-thienyl, 3-chloro-2-thienyl, 3-bromo-2-thienyl, 3-methyl-2-thienyl, 3-methoxy-2-thienyl, 3-thienyl, 2-fluoro-3-thienyl, 2-chloro-3-thienyl, 2-bromo-3-thienyl, 2-methyl-3-thienyl, 2-methoxy-3-thienyl, 4-fluoro-3-thienyl, 4-chloro-3-thienyl, 4-bromo-3-thienyl, 4-methyl-3-thienyl, 4-methoxy-3-thienyl, 3,5-dimethyl-2-thienyl, 5-bromo-3-methyl-2-thienyl, 2,5-dimethyl-3-thienyl, 4,5-dimethyl-3-thienyl, 5-bromo-2-methyl-3-thienyl, 5-bromo-4-methyl-3-thienyl, 2,4,5-trimethyl-3-thienyl, 2,5-dibromo-4-methyl-3-thienyl, pyridin-2-yl, 3-fluoropyridin-2-yl, 3-chloropyridin-2-yl, 3-bromopyridin-2-yl, 3-methylpyridin-2-yl, 3-methoxypyridin-2-yl, 4-fluoropyridin-2-yl, 5-fluoropyridin-2-yl, 6-fluoropyridin-2-yl, pyridin-3-yl, 2-methylpyridin-3-yl, 2-fluoropyridin-3-yl, 2,4-difluoropyridin-3-yl, pyridin-4-yl, 3-fluoropyridin-4-yl, 2-fluoropyridin-4-yl, 2,3-difluoropyridin-4-yl, 4-methylthiazol-5-yl, 4-methylisothiazol-5-yl, cyclopenten-1-yl, cyclohexen-1-yl or 7-oxabicyclo[4.1.0]heptan-1-yl,
R² represents hydrogen, fluorine, chlorine, bromine, iodine, methyl, ethyl, n-propyl, isopropyl, cyclopropyl, cyclobutyl, vinyl, prop-2-en-2-yl, ethynyl, prop-1-yn-1-yl, methoxy, ethoxy, methylthio, ethoxycarbonyl, 2,3,4,5,6-pentafluorophenyl, difluoromethyl or trifluoromethyl,
n is 0, 1, 2,
R³ represents hydrogen, fluorine, chlorine, bromine or methyl, preferably hydrogen,
R⁴ and R⁵ independently of one another represent hydrogen, methyl, ethyl, prop-1-yl, prop-2-yl, but-1-yl, but-2-yl, 1,1-dimethyleth-1-yl, 2-methylprop-1-yl, hydroxymethyl, hydroxyethyl, hydroxyprop-1-yl, methoxymethyl, methoxyethyl, methoxyprop-1-yl, ethoxymethyl, ethoxyethyl, ethoxyprop-1-yl, difluoromethyl, trifluoromethyl, 2,2-difluoroethyl, 3,3,3-trifluoroethyl, pentafluoroethyl, cyclopropyl, cyclobutyl, cyclopentyl, or cyclohexyl, or
R⁴ and R⁵ together with the carbon atom to which they are bonded form a fully saturated, or partly saturated 3- to 10-membered monocyclic or bicyclic ring optionally interrupted by heteroatoms and optionally having further substitution and
R⁶ represents hydrogen, BH₂, B(NH₂)₂,4-amino-1,3,2,4-diazadiboretidin-2-yl, 4-amino-1,3,2,4-dioxadiboretan-2-yl, amino, tris(methyl)silyl, tris(ethyl)silyl, tris(prop-1-yl)silyl, tris(prop-2-yl)silyl, (1,1-dimethyleth-1-yl)(dimethyl)silyl, cyanomethyl, 2-cyanoeth-1-yl, 3-cyanoprop-1-yl, 2-cyanoprop-1-yl, methoxymethyl, 2-methoxyeth-1-yl, 2-methoxyprop-1-yl, 3-methoxyprop-1-yl, ethoxymethyl, 2-(ethoxy)eth-1-yl, 2-(ethoxy)prop-1-yl, 3-ethoxyprop-1-yl, 4-methoxybut-1-yl, 2-phenoxyeth-1-yl, 2-benzyloxyeth-1-yl, 2-(methylcarbonyloxy)eth-1-yl, 2-(ethylcarbonyloxy)eth-1-yl, 2-(iso-propylcarbonyloxy)eth-1-yl, 2-(tert-butylcarbonyloxy)eth-1-yl, 2-(phenylcarbonyloxy)eth-1-yl, 2-(2-trifluormethylphenylcarbonyl)oxyeth-1-yl, 2-(2-chlorophenylcarbonyl)oxyeth-1-yl, 2-(4-trifluormethylpyridin-3-ylcarbonyl)oxyeth-1-yl, 2-(methylsulfonyloxy)eth-1-yl, 3-(methylcarbonyloxy)prop-1-yl, 3-(ethylcarbonyloxy)prop-1-yl, 3-(iso-propylcarbonyloxy)prop-1-yl, 3-(tert-butylcarbonyloxy)prop-1-yl, 2-trifluoromethoxyeth-1-yl, methyl, ethyl, prop-1-yl, 1-methylethyl, but-1-yl, 1-methylprop-1-yl, 2-methylprop-1-yl, 1,1-dimethylethyl, pent-1-yl, 1-methylbut-1-yl, 2-methylbut-1-yl, 3-methylbut-1-yl, 1,1-dimethylprop-1-yl, 1,2-dimethylprop-1-yl, 2,2-dimethylprop-1-yl, 1-ethylprop-1-yl, prop-2-en-1-yl, but-2-en-1-yl, but-3-en-1-yl, pent-2-en-1-yl, pent-3-en-1-yl, pent-4-en-1-yl, 1-methylprop-2-en-1-yl, 2-methylprop-2-en-1-yl, 1-methylbut-2-en-1-yl, 2-methylbut-2-en-1-yl, 3-methylbut-2-en-1-yl, 1-methylbut-3-en-1-yl, 2-methylbut-3-en-1-yl, 3-methylbut-3-en-1-yl, 1,1-dimethylprop-2-en-1-yl, 1,2-dimethylprop-2-en-1-yl, prop-2-yn-1-yl, but-2-yn-1-yl, but-3-yn-1-yl, 1-methyl-prop-2-yn-1-yl, pent-2-yn-1-yl, pent-3-yn-1-yl, pent-4-yn-1-yl, 1-methylbuty-2-n-1-yl, 1-methylbut-3-yn-1-yl, 2-methylbut-3-yn-1-yl, cyclobutyl, cyclopentyl, cyclohexyl, cyclopropylmethyl, cyclobutylmethyl, cyclopentylmethyl, cyclohexylmethyl, 2-(dimethylamino)eth-1-yl, 2-(diethylamino)eth-1-yl, 3-(dimethylamino)prop-1-yl, 3-(diethylamino)prop-1-yl, 2-(N-pyrrolidin-1-yl)eth-1-yl, 2-(N-piperidin-1-yl)eth-1-yl, tetrahydrofuran-2-ylmethyl, tetrahydrofuran-3-ylmethyl, tetrahydropyran-2-ylmethyl, tetrahydropyran-3-ylmethyl, tetrahydropyran-4-ylmethyl, oxetan-3-ylmethyl, methoxyethoxyeth-1-yl, ethoxyethoxyeth-1-yl, benzyl, 2-phenyleth-1-yl, (2-fluorophenyl)methyl, (3-fluorophenyl)methyl, (4-fluorophenyl)methyl, (2,6-difluorophenyl)methyl, (2,5-difluorophenyl)methyl, (2,4-difluorophenyl)methyl, (2,3-difluorophenyl)methyl, (2-fluoro-6-chlorophenyl)methyl, (2-chlorophenyl)methyl, (3-chlorophenyl)methyl, (4-chlorophenyl)methyl, (2,6-dichlorophenyl)methyl, (2,5-dichlorophenyl)methyl, (2,4-dichlorophenyl)methyl, (2,3-dichlorophenyl)methyl, (2-bromophenyl)methyl, (3-bromophenyl)methyl, (4-bromophenyl)methyl, (2-methylphenyl)methyl, (2-trifluoromethylphenyl)methyl, (2-methoxyphenyl)methyl, (2-nitrophenyl)methyl, (3-methylphenyl)methyl, (3-trifluoromethylphenyl)methyl, (3-methoxyphenyl)methyl, (3-nitrophenyl)methyl, (4-methylphenyl)methyl, (4-trifluoromethylphenyl)methyl, (4-methoxyphenyl)methyl, (4-nitrophenyl)methyl, pyridin-2-ylmethyl, pyridin-3-ylmethyl, pyridin-4-ylmethyl, (6-fluoropyridin-2-yl)methyl, (6-chloro-4-trifluoromethylpyridin-2-yl)methyl, (4,6-dichloropyridin-2-yl)methyl, (4-fluoropyridin-3-yl)methyl, (4-chloropyridin-3-yl)methyl, thiophen-2-ylmethyl, methoxycarbonyl, ethoxycarbonyl, 1,1-dimethyleth-1-yloxycarbonyl, benzyloxycarbonyl, (prop-2-en-1-yl)oxycarbonyl, methylaminocarbonyl, ethylaminocarbonyl, prop-1-ylaminocarbonyl, prop-2-ylaminocarbonyl, cyclopentylaminocarbonyl, cyclohexylaminocarbonyl, phenylaminocarbonyl, dimethylamincarbonyl, diethylaminocarbonyl, ethyl(methyl)aminocarbonyl, (2-fluorophenyl)aminocarbonyl, (3-fluorophenyl)aminocarbonyl, (4-fluorophenyl)aminocarbonyl, (2,6-difluorophenyl)aminocarbonyl, (2,5-difluorophenyl)aminocarbonyl, (2,4-difluorophenyl)aminocarbonyl, (2,3-difluorophenyl)aminocarbonyl, (2-fluoro-6-chlorophenyl)aminocarbonyl, (2-chlorophenyl)aminocarbonyl, (3-chlorophenyl)aminocarbonyl, (4-chlorophenyl)aminocarbonyl, (2,6-dichlorophenyl)aminocarbonyl, (2,5-dichlorophenyl)aminocarbonyl, (2,4-dichlorophenyl)aminocarbonyl, (2,3-dichlorophenyl)aminocarbonyl, (2-bromophenyl)aminocarbonyl, (3-bromophenyl)aminocarbonyl, (4-bromophenyl)aminocarbonyl, methoxycarbonylmethyl, 2-(methoxycarbonyl)eth-1-yl, 1-(methoxycarbonyl)eth-1-yl, 3-(methoxycarbonyl)prop-1-yl, 2-(methoxycarbonyl)prop-1-yl, 4-(methoxycarbonyl)but-1-yl, 5-(methoxycarbonyl)pent-1-yl, ethoxycarbonylmethyl, 2-(ethoxycarbonyl)eth-1-yl, 1-(ethoxycarbonyl)eth-1-yl, 3-(ethoxycarbonyl)prop-1-yl, 2-(ethoxycarbonyl)prop-1-yl, 4-(ethoxycarbonyl)but-1-yl, 5-(ethoxycarbonyl)pent-1-yl, benzyloxycarbonylmethyl, 2-(benzyloxycarbonyl)eth-1-yl, hydroxycarbonylmethyl, 2-(hydroxycarbonyl)eth-1-yl, 1-(hydroxycarbonyl)eth-1-yl, 3-(hydroxycarbonyl)prop-1-yl, 2-(hydroxycarbonyl)prop-1-yl, 4-(hydroxycarbonyl)but-1-yl, 5-(hydroxycarbonyl)pent-1-yl, aminocarbonylmethyl, methylaminocarbonylmethyl, ethylaminocarbonylmethyl, prop-1-ylaminocarbonylmethyl, prop-2-ylaminobarbonylmethyl, dimethylaminocarbonylmethyl, diethylaminocarbonylmethyl, benzylaminocarbonylmethyl, cyclopropylaminocarbonylmethyl, cyclobutylaminocarbonylmethyl, cyclopentylaminocarbonylmethyl, cyclohexylaminocarbonylmethyl, 2-(aminocarbonyl)eth-1-yl, 2-(methylaminocarbonyl)eth-1-yl, 2-(ethylaminocarbonyl)eth-1-yl, 2-(prop-1-ylaminocarbonyl)eth-1-yl, 2-(prop-2-ylaminobarbonyl)eth-1-yl, 2-(dimethylaminocarbonyl)eth-1-yl, 2-(diethylaminocarbonyl)eth-1-yl, 2-(benzylaminocarbonyl)eth-1-yl, 2-(cyclopropylaminocarbonyl)eth-1-yl, 2-(cyclobutylaminocarbonyl)eth-1-yl, 2-(cyclopentylaminocarbonyl)eth-1-yl, 2-(cyclohexylaminocarbonyl)eth-1-yl, 2-(methylsulfanyl)eth-1-yl, 2-(methylsulfinyl)eth-1-yl, 2-(methylsulfonyl)eth-1-yl, 2-(ethylsulfanyl)eth-1-yl, 2-(ethylsulfinyl)eth-1-yl, 2-(ethylsulfonyl)eth-1-yl, 2-(methylsulfanyl)prop-1-yl, 2-(methylsulfinyl)prop-1-yl, 2-(methylsulfonyl)prop-1-yl, methylsulfonyl, ethylsulfonyl, prop-1-ylsulfonyl, 1-methyleth-1-ylsulfonyl, but-1-ylsulfonyl, 1-methylprop-1-ylsulfonyl, 2-methylprop-1-ylsulfonyl, 1,1-dimethylethylsulfonyl, pent-1-ylsulfonyl, 1-methylbut-1-ylsulfonyl, 2-methylbut-1-ylsulfonyl, 3-methylbut-1-ylsulfonyl, 1,1-dimethylprop-1-ylsulfonyl, 1,2-dimethylprop-1-ylsulfonyl, 2,2-dimethylprop-1-ylsulfonyl, 1-ethylprop-1-ylsulfonyl, cyanomethylsulfonyl, 3-cyanoprop-1-ylsulfonyl, 1-methylcycloprop-1-ylsulfonyl, tetrahydrofuran-3-ylsulfonyl, methoxycarbonylmethylsulfonyl, ethoxycarbonylmethylsulfonyl, cyclopropylmethylsulfonyl, cyclobutylmethylsulfonyl, cyclopentylmethylsulfonyl, cyclohexylmethylsulfonyl, cyclopropylsulfonyl, cyclobutylsulfonyl, cyclopentylsulfonyl, cyclohexylsulfonyl, prop-2-en-1-ylsulfonyl, prop-2-en-2-ylsulfonyl, but-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-methylprop-2-en-1-ylsulfonyl, 2-methylprop-2-en-1-ylsulfonyl, trifluormethylsulfonyl, benzylsulfonyl, 2-phenyleth-1-ylsulfonyl, (2-fluorophenyl)methylsulfonyl, (3-fluorophenyl)methylsulfonyl, (4-fluorophenyl)-methylsulfonyl, (2,6-difluorophenyl)methylsulfonyl, (2,5-difluorophenyl)-methylsulfonyl, (2,4-difluorophenyl)methylsulfonyl, (2,3-difluorophenyl)-methylsulfonyl, (2-fluoro-6-chlorophenyl)methylsulfonyl, (2-chlorophenyl)-methylsulfonyl, (3-chlorophenyl)methylsulfonyl, (4-chlorophenyl)methylsulfonyl, (2,6-dichlorophenyl)methylsulfonyl, (2,5-dichlorophenyl)methylsulfonyl, (2,4-dichlorophenyl)methylsulfonyl, (2,3-dichlorophenyl)methylsulfonyl, (2-bromophenyl)methylsulfonyl, (3-bromophenyl)methylsulfonyl, (4-bromophenyl)methylsulfonyl, (2-methylphenyl)methylsulfonyl, (2-trifluoromethylphenyl)methylsulfonyl, (2-methoxyphenyl)methylsulfonyl, (2-nitrophenyl)methylsulfonyl, (3-methylphenyl)methylsulfonyl, (3-trifluoromethylphenyl)methylsulfonyl, (3-methoxyphenyl)methylsulfonyl, (3-nitrophenyl)methylsulfonyl, (4-methylphenyl)methylsulfonyl, (4-trifluoromethylphenyl)methylsulfonyl, (4-methoxyphenyl)methylsulfonyl, (4-nitrophenyl)methylsulfonyl, pyridin-2-ylmethylsulfonyl, pyridin-3-ylmethylsulfonyl, pyridin-4-ylmethylsulfonyl, (6-fluoropyridin-2-yl)methylsulfonyl, (6-chloro-4-trifluoromethylpyridin-2-yl)methylsulfonyl, (4,6-dichloropyridin-2-yl)methylsulfonyl, (4-fluoropyridin-3-yl)methylsulfonyl, (4-chloropyridin-3-yl)methylsulfonyl, thiophen-2-ylmethylsulfonyl, phenylsulfonyl, (2-fluorophenyl)sulfonyl, (3-fluorophenyl)sulfonyl, (4-fluorophenyl)sulfonyl, (2,6-difluorophenyl)sulfonyl, (2,5-difluorophenyl)sulfonyl, (2,4-difluorophenyl)sulfonyl, (2,3-difluorophenyl)sulfonyl, (2-fluoro-6-chlorophenyl)sulfonyl, (2-chlorophenyl)sulfonyl, (3-chlorophenyl)sulfonyl, (4-chlorophenyl)sulfonyl, (2,6-dichlorophenyl)sulfonyl, (2,5-dichlorophenyl)sulfonyl, (2,4-dichlorophenyl)sulfonyl, (2,3-dichlorophenyl)sulfonyl, (2-bromophenyl)sulfonyl, (3-bromophenyl)sulfonyl, (4-bromophenyl)sulfonyl, (2-methylphenyl)sulfonyl, (2-trifluoromethylphenyl)sulfonyl, (2-methoxyphenyl)sulfonyl, (2-nitrophenyl)sulfonyl, (3-methylphenyl)sulfonyl, (3-trifluoromethylphenyl)sulfonyl, (3-methoxyphenyl)-sulfonyl, (3-nitrophenyl)sulfonyl, (4-methylphenyl)sulfonyl, (4-trifluoromethylphenyl)-sulfonyl, (4-methoxyphenyl)sulfonyl, (4-nitrophenyl)sulfonyl, pyridin-2-ylsulfonyl, pyridin-3-ylsulfonyl, pyridin-4-ylsulfonyl, (6-fluoropyridin-2-yl)sulfonyl, (6-chloro-4-trifluorosulfonylpyridin-2-yl)sulfonyl, (4,6-dichloropyridin-2-yl)sulfonyl, (4-fluoro-pyridin-3-yl)sulfonyl, (4-chloropyridin-3-yl)sulfonyl, thiophen-2-ylsulfonyl, methoxymethylsulfonyl, 2-(methoxy)eth-1-ylsulfonyl, 2-(methoxy)eth-1-ylsulfonyl, methylaminosulfonyl, ethylaminosulfonyl, dimethylaminosulfonyl, diethylaminosulfonyl, methyl(ethyl)aminosulfonyl, azetidin-1-ylsulfonyl, pyrrolidin-1-ylsulfonyl, piperidin-1-ylsulfonyl, N-morpholinylsulfonyl, 2-(methoxycarbonyl)eth-1-ylsulfonyl, 2-(ethoxycarbonyl)eth-1-ylsulfonyl, 3-(methoxycarbonyl)prop-1-ylsulfonyl, 3-(ethoxycarbonyl)prop-1-ylsulfonyl, 1-(methoxycarbonyl)eth-1-ylsulfonyl, 2-ethoxyeth-1-ylsulfonyl, 1-(prop-2-en-1-yl)cycloprop-1-ylsulfonyl, 3,3,3-trifluoroethylsulfonyl, tetrahydropyran-4-ylsulfonyl, tetrahydrofuran-3-ylmethylsulfonyl, formyl, methylcarbonyl, ethylcarbonyl, prop-1-ylcarbonyl, 1-methyleth-1-ylcarbonyl, but-1-ylcarbonyl, 1-methylprop-1-ylcarbonyl, 2-methylprop-1-ylcarbonyl, 1,1-dimethylethylcarbonyl, pent-1-ylcarbonyl, 1-methylbut-1-ylcarbonyl, 2-methylbut-1-ylcarbonyl, 3-methylbut-1-ylcarbonyl, 1,1-dimethylprop-1-ylcarbonyl, 1,2-dimethylprop-1-ylcarbonyl, 2,2-dimethylprop-1-ylcarbonyl, 1-ethylprop-1-ylcarbonyl, cyclopropylmethylcarbonyl, cyclobutylmethylcarbonyl, cyclopentylmethylcarbonyl, cyclohexylmethylcarbonyl, cyclopropylcarbonyl, cyclobutylcarbonyl, cyclopentylcarbonyl, cyclohexylcarbonyl, methoxymethylcarbonyl, 2-methoxyeth-1-ylcarbonyl, 2-ethoxyeth-1-ylcarbonyl, 3-methoxyprop-1-ylcarbonyl, 3-ethoxyprop-1-ylcarbonyl, methoxycarbonylmethylcarbonyl, 2-(methoxycarbonyl)eth-1-ylcarbonyl, 3-(methoxycarbonyl)prop-1-ylcarbonyl, 4-(methoxycarbonyl)but-1-ylcarbonyl, ethoxycarbonylmethylcarbonyl, 2-(ethoxycarbonyl)eth-1-ylcarbonyl, 3-(ethoxycarbonyl)prop-1-ylcarbonyl, 4-(ethoxycarbonyl)but-1-ylcarbonyl, benzylcarbonyl, 2-phenyleth-1-ylcarbonyl, (2-fluorophenyl)methylcarbonyl, (3-fluorophenyl)methylcarbonyl, (4-fluorophenyl)methylcarbonyl, (2,6-difluorophenyl)methylcarbonyl, (2,5-difluorophenyl)methylcarbonyl, (2,4-difluorophenyl)methylcarbonyl, (2,3-difluorophenyl)methylcarbonyl, (2-fluoro-6-chlorophenyl)methylcarbonyl, (2-chlorophenyl)methylcarbonyl, (3-chlorophenyl)methylcarbonyl, (4-chlorophenyl)methylcarbonyl, (2,6-dichlorophenyl)methylcarbonyl, (2,5-dichlorophenyl)methylcarbonyl, (2,4-dichlorophenyl)methylcarbonyl, (2,3-dichlorophenyl)methylcarbonyl, (2-bromophenyl)methylcarbonyl, (3-bromophenyl)methylcarbonyl, (4-bromophenyl)methylcarbonyl, (2-methylphenyl)methylcarbonyl, (2-trifluoromethylphenyl)methylcarbonyl, (2-methoxyphenyl)methylcarbonyl, (2-nitrophenyl)methylcarbonyl, (3-methylphenyl)methylcarbonyl, (3-trifluoromethylphenyl)methylcarbonyl, (3-methoxyphenyl)methylcarbonyl, (3-nitrophenyl)methylcarbonyl, (4-methylphenyl)methylcarbonyl, (4-trifluoromethylphenyl)methylcarbonyl, (4-methoxyphenyl)methylcarbonyl, (4-nitrophenyl)methylcarbonyl, pyridin-2-ylmethylcarbonyl, pyridin-3-ylmethylcarbonyl, pyridin-4-ylmethylcarbonyl, (6-fluoropyridin-2-yl)methylcarbonyl, (6-chloro-4-trifluoromethylpyridin-2-yl)methylcarbonyl, (4,6-dichloropyridin-2-yl)methylcarbonyl, (4-fluoropyridin-3-yl)methylcarbonyl, (4-chloropyridin-3-yl)methylcarbonyl, thiophen-2-ylmethylcarbonyl, phenylcarbonyl, (2-fluorophenyl)carbonyl, (3-fluorophenyl)carbonyl, (4-fluorophenyl)carbonyl, (2,6-difluorophenyl)carbonyl, (2,5-difluorophenyl)carbonyl, (2,4-difluorophenyl)carbonyl, (2,3-difluorophenyl)carbonyl, (2-fluoro-6-chlorophenyl)carbonyl, (2-chlorophenyl)carbonyl, (3-chlorophenyl)carbonyl, (4-chlorophenyl)carbonyl, (2,6-dichlorophenyl)carbonyl, (2,5-dichlorophenyl)carbonyl, (2,4-dichlorophenyl)carbonyl, (2,3-dichlorophenyl)carbonyl, (2-bromophenyl)carbonyl, (3-bromophenyl)carbonyl, (4-bromophenyl)carbonyl, (2-methylphenyl)carbonyl, (2-trifluoromethylphenyl)carbonyl, (2-methoxyphenyl)carbonyl, (2-nitrophenyl)-carbonyl, (3-methylphenyl)carbonyl, (3-trifluoromethylphenyl)carbonyl, (3-methoxyphenyl)carbonyl, (3-nitrophenyl)carbonyl, (4-methylphenyl)carbonyl, (4-trifluoromethylphenyl)carbonyl, (4-methoxyphenyl)carbonyl, (4-nitrophenyl)-carbonyl, (2-chloro-4-methylsulfonylphenyl)carbonyl, pyridin-2-ylcarbonyl, pyridin-3-ylcarbonyl, pyridin-4-ylcarbonyl, (6-fluoropyridin-2-yl)carbonyl, (6-chloro-4-trifluorocarbonylpyridin-2-yl)carbonyl, (4,6-dichloropyridin-2-yl)carbonyl, (4-fluoropyridin-3-yl)carbonyl, (4-chloropyridin-3-yl)carbonyl, thiophen-2-ylcarbonyl, 1,3-oxazol-2-ylcarbonyl, 1,2-oxazol-3-ylcarbonyl, 3-chloro-5-methyl-4,5-dihydro-1,2-oxazol-5-ylcarbonyl, 3-bromo-5-methyl-4,5-dihydro-1,2-oxazol-5-ylcarbonyl, 3-chloro-5-methoxymethyl-4,5-dihydro-1,2-oxazol-5-ylcarbonyl, 3-chloro-5-ethyl-4,5-dihydro-1,2-oxazol-5-ylcarbonyl, 3-chloro-3a,4,5,6-tetrahydro-6aH-cyclopenta[d][1,2]oxazol-6a-ylcarbonyl, (4-trifluoromethylpyridin-3-yl)carbonyl, 3,5-dimethyl-4,5-dihydro-1,2-oxazol-5-ylcarbonyl, 5-methyl-3-trifluoromethyl-4,5-dihydro-1,2-oxazol-5-ylcarbonyl, 3-chloro-5-hydroxymethyl-4,5-dihydro-1,2-oxazol-5-ylcarbonyl, 3-chloro-5-tert-butyldimethylsilyloxymethyl-4,5-dihydro-1,2-oxazol-5-ylcarbonyl, 3-chloro-5-(1-hydroxyeth-1-yl)-4,5-dihydro-1,2-oxazol-5-ylcarbonyl, 3-chloro-5-(1-tert-butyldimethylsilyloxyeth-1-yl-4,5-dihydro-1,2-oxazol-5-ylcarbonyl, methoxyethoxymethylcarbonyl, ethoxyethoxymethylcarbonyl, hydroxyethoxymethylcarbonyl, methoxyethoxyethoxymethylcarbonyl, ethoxyethoxyethoxymethylcarbonyl, hydroxyethoxyethoxymethylcarbonyl, 4-trifluoromethylpyridin-3-ylcarbonyloxyethoxyethoxymethylcarbonyl, methoxyethoxyethoxyethoxymethylcarbonyl, ethoxyethoxyethoxyethoxymethylcarbonyl, hydroxyethoxyethoxyethoxymethylcarbonyl, methoxycarbonylmethoxyethoxymethylcarbonyl, hydroxycarbonylmethoxyethoxymethylcarbonyl, methoxycarbonylmethoxyethoxyethoxymethylcarbonyl, hydroxycarbonylmethoxyethoxyethoxymethylcarbonyl, methoxycarbonylmethoxyethoxyethoxyethoxymethylcarbonyl, or hydroxycarbonylmethoxyethoxyethoxyethoxymethylcarbonyl,

6. The compound of the general formula (I) as claimed in claim 1 and/or the salt thereof, **characterized in that**
R¹ represents phenyl, 2-fluorophenyl, 3-fluorophenyl, 4-fluorophenyl, 2-chlorophenyl, 3-chlorophenyl, 4-chlorophenyl, 2-bromophenyl, 2-methylphenyl, 2-ethylphenyl, 2-methoxyphenyl, 2-trifluoromethylphenyl, 2,3-difluorophenyl, 2,4-difluorophenyl, 2,5-difluorophenyl, 2,6-difluorophenyl, 2,3,4-trifluorophenyl, 2,4,5-trifluorophenyl, 2,4,6-trifluorophenyl, 2-fluoro-6-methylphenyl, 2-fluoro-3-methylphenyl 2,4-dichlorophenyl, 2,5-dichlorophenyl, 2,6-dichlorophenyl, 3,5-dichlorophenyl, 2,6-dimethylphenyl, 2-chloro-6-methylphenyl, 2-bromo-6-fluorophenyl, 2-bromo-6-chlorophenyl, 2-bromo-6-methylphenyl, 2-bromo-6-methoxyphenyl, 2-fluoro-3-chlorophenyl, 2-chloro-3-fluorophenyl, 2-fluoro-4-chlorophenyl, 2-fluoro-6-chlorophenyl, 2,3-dimethylphenyl, 2,4-dimethylphenyl, 2-methyl-3-fluorophenyl, 2-thienyl, 3-fluoro-2-thienyl, 3-chloro-2-thienyl, 3-bromo-2-thienyl, 3-methyl-2-thienyl, 3-methoxy-2-thienyl, 3-thienyl, 2-fluoro-3-thienyl, 2-chloro-3-thienyl, 2-bromo-3-thienyl, 2-methyl-3-thienyl, 2-methoxy-3-thienyl, 4-fluoro-3-thienyl, 4-chloro-3-thienyl, 4-bromo-3-thienyl, 4-methyl-3-thienyl, 4-methoxy-3-thienyl, 3,5-dimethyl-2-thienyl, 5-bromo-3-methyl-2-thienyl, 2,5-dimethyl-3-thienyl, 4,5-dimethyl-3-thienyl, 5-bromo-2-methyl-3-thienyl, 5-bromo-4-methyl-3-thienyl, 2,4,5-trimethyl-3-thienyl, 2,5-dibromo-4-methyl-3-thienyl, pyridin-2-yl, 3-fluoropyridin-2-yl, 3-chloropyridin-2-yl, 3-bromopyridin-2-yl, 3-methylpyridin-2-yl, 3-methoxypyridin-2-yl, 4-fluoropyridin-2-yl, 5-fluoropyridin-2-yl, 6-fluoropyridin-2-yl, pyridin-3-yl, 2-methylpyridin-3-yl, 2-fluoropyridin-3-yl, 2,4-difluoropyridin-3-yl, pyridin-4-yl, 3-fluoropyridin-4-yl, 2-fluoropyridin-4-yl, 2,3-difluoropyridin-4-yl, 4-methylthiazol-5-yl, 4-methylisothiazol-5-yl, cyclopenten-1-yl, cyclohexen-1-yl or 7-oxabicyclo[4.1.0]heptan-1-yl,
R² represents hydrogen, fluorine, chlorine, bromine, iodine, methyl, ethyl, n-propyl, isopropyl, cyclopropyl, cyclobutyl, vinyl, prop-2-en-2-yl, ethynyl, prop-1-yn-1-yl, methoxy, ethoxy, methylthio, ethoxycarbonyl, 2,3,4,5,6-pentafluorophenyl, difluoromethyl or trifluoromethyl,
R³ represents hydrogen,
n is 0, 1, 2,
R⁴ and R⁵ independently of one another represent hydrogen, methyl, ethyl, prop-1-yl, prop-2-yl, but-1-yl, but-2-yl, 1,1-dimethyleth-1-yl, methoxymethyl, methoxyethyl, methoxyprop-1-yl, ethoxymethyl, ethoxyethyl, difluoromethyl, trifluoromethyl, cyclopropyl, cyclobutyl, or
R⁴ and R⁵ together with the carbon atom to which they are bonded form a fully saturated, or partly saturated 3- to 10-membered monocyclic or bicyclic ring optionally interrupted by heteroatoms and optionally having further substitution, and
R⁶ represents hydrogen, BH₂, B(NH₂)₂, 4-amino-1,3,2,4-diazadiboretidin-2-yl, 4-amino-1,3,2,4-dioxadiboretan-2-yl, amino, tris(methyl)silyl, tris(ethyl)silyl, tris(prop-1-yl)silyl, tris(prop-2-yl)silyl, (1,1-dimethyleth-1-yl)(dimethyl)silyl, methyl, ethyl, prop-1-yl, methoxycarbonylmethyl, 2-(methoxy-carbonyl)eth-1-yl, 1-(methoxycarbonyl)eth-1-yl, 3-(methoxycarbonyl)prop-1-yl, 2-(methoxycarbonyl)prop-1-yl, 4-(methoxycarbonyl)but-1-yl, 5-(methoxycarbonyl)pent-1-yl, ethoxycarbonylmethyl, 2-(ethoxycarbonyl)eth-1-yl, 1-(ethoxycarbonyl)eth-1-yl, 3-(ethoxycarbonyl)prop-1-yl, 2-(ethoxycarbonyl)prop-1-yl, 4-(ethoxycarbonyl)but-1-yl, 5-(ethoxycarbonyl)pent-1-yl, benzyloxycarbonylmethyl, 2-(benzyloxycarbonyl)eth-1-yl, hydroxycarbonylmethyl, 2-(hydroxycarbonyl)eth-1-yl, 1-(hydroxycarbonyl)eth-1-yl, 3-(hydroxycarbonyl)prop-1-yl, 2-(hydroxycarbonyl)prop-1-yl, 4-(hydroxycarbonyl)but-1-yl, 5-(hydroxycarbonyl)-pent-1-yl, methylsulfonyl, ethylsulfonyl, prop-1-ylsulfonyl, 1-methyleth-1-ylsulfonyl, but-1-ylsulfonyl, 1-methylprop-1-ylsulfonyl, 2-methylprop-1-ylsulfonyl, 1,1-dimethylethylsulfonyl, pent-1-ylsulfonyl, 1-methylbut-1-ylsulfonyl, 2-methylbut-1-ylsulfonyl, 3-methylbut-1-ylsulfonyl, 1,1-dimethylprop-1-ylsulfonyl, 1,2-dimethylprop-1-ylsulfonyl, 2,2-dimethylprop-1-ylsulfonyl, 1-ethylprop-1-ylsulfonyl, cyanomethylsulfonyl, 3-cyanoprop-1-ylsulfonyl, 1-methylcycloprop-1-ylsulfonyl, tetrahydrofuran-3-ylsulfonyl, methoxycarbonyl-methylsulfonyl, ethoxycarbonylmethylsulfonyl, cyclopropylmethylsulfonyl, cyclobutylmethylsulfonyl, cyclopentylmethylsulfonyl, cyclohexylmethylsulfonyl, cyclopropylsulfonyl, cyclobutylsulfonyl, cyclopentylsulfonyl, cyclohexylsulfonyl, prop-2-en-1-ylsulfonyl, prop-2-en-2-ylsulfonyl, but-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-methylprop-2-en-1-ylsulfonyl, 2-methylprop-2-en-1-ylsulfonyl, trifluormethylsulfonyl, benzylsulfonyl, 2-phenyleth-1-ylsulfonyl, (2-fluorophenyl)methylsulfonyl, (3-fluorophenyl)methylsulfonyl, (4-fluorophenyl)methylsulfonyl, (2,6-difluorophenyl)methylsulfonyl, (2,5-difluorophenyl)-methylsulfonyl, (2,4-difluorophenyl)methylsulfonyl, (2,3-difluorophenyl)methylsulfonyl, (2-fluoro-6-chlorophenyl)methylsulfonyl, (2-chlorophenyl)methylsulfonyl, (3-chlorophenyl)methylsulfonyl, (4-chlorophenyl)methylsulfonyl, (2,6-dichlorophenyl)-methylsulfonyl, (2,5-dichlorophenyl)methylsulfonyl, (2,4dichlorophenyl)methylsulfonyl, (2,3-dichlorophenyl)methylsulfonyl, (2-bromophenyl)methylsulfonyl, (3-bromophenyl)-methylsulfonyl, (4-bromophenyl)methylsulfonyl, (2-methylphenyl)methylsulfonyl, (2-trifluoromethylphenyl)methylsulfonyl, (2-methoxyphenyl)methylsulfonyl, (2-nitrophenyl)-methylsulfonyl, (3-methylphenyl)methylsulfonyl, (3-trifluoromethylphenyl)methylsulfonyl, (3-methoxyphenyl)methylsulfonyl, (3-nitrophenyl)methylsulfonyl, (4-methylphenyl)methyl-sulfonyl, (4-trifluoromethylphenyl)methylsulfonyl, (4-methoxyphenyl)methylsulfonyl, (4-nitrophenyl)methylsulfonyl, pyridin-2-ylmethylsulfonyl, pyridin-3-ylmethylsulfonyl, pyridin-4-ylmethylsulfonyl, (6-fluoropyridin-2-yl)methylsulfonyl, (6-chloro-4-trifluoromethylpyridin-2-yl)methylsulfonyl, (4,6-dichloropyridin-2-yl)methylsulfonyl, (4-fluoropyridin-3-yl)methylsulfonyl, (4-chloropyridin-3-yl)methylsulfonyl, thiophen-2-ylmethylsulfonyl, phenylsulfonyl, (2-fluorophenyl)sulfonyl, (3-fluorophenyl)sulfonyl, (4-fluorophenyl)sulfonyl, (2,6-difluorophenyl)sulfonyl, (2,5-difluorophenyl)sulfonyl, (2,4-difluorophenyl)sulfonyl, (2,3-difluorophenyl)sulfonyl, (2-fluoro-6-chlorophenyl)sulfonyl, (2-chlorophenyl)sulfonyl, (3-chlorophenyl)sulfonyl, (4-chlorophenyl)sulfonyl, (2,6-dichlorophenyl)sulfonyl, (2,5-dichlorophenyl)sulfonyl, (2,4-dichlorophenyl)sulfonyl, (2,3-dichlorophenyl)sulfonyl, (2-bromophenyl)sulfonyl, (3-bromophenyl)sulfonyl, (4-bromophenyl)sulfonyl, (2-methylphenyl)sulfonyl, (2-trifluoromethylphenyl)sulfonyl, (2-methoxyphenyl)sulfonyl, (2-nitrophenyl)sulfonyl, (3-methylphenyl)sulfonyl, (3-trifluoromethylphenyl)sulfonyl, (3-methoxyphenyl)sulfonyl, (3-nitrophenyl)sulfonyl, (4-methylphenyl)sulfonyl, (4-trifluoromethylphenyl)sulfonyl, (4-methoxyphenyl)sulfonyl, (4-nitrophenyl)sulfonyl, pyridin-2-ylsulfonyl, pyridin-3-ylsulfonyl, pyridin-4-ylsulfonyl, (6-fluoropyridin-2-yl)sulfonyl, (6-chloro-4-trifluorosulfonylpyridin-2-yl)sulfonyl, (4,6-dichloropyridin-2-yl)sulfonyl, (4-fluoropyridin-3-yl)sulfonyl, (4-chloropyridin-3-yl)sulfonyl, thiophen-2-ylsulfonyl, methoxymethylsulfonyl, 2-(methoxy)eth-1-ylsulfonyl, 2-(methoxy)eth-1-ylsulfonyl, methylaminosulfonyl, ethylaminosulfonyl, dimethylaminosulfonyl, diethylaminosulfonyl, methyl(ethyl)aminosulfonyl, azetidin-1-ylsulfonyl, pyrrolidin-1-ylsulfonyl, piperidin-1-ylsulfonyl, N-morpholinylsulfonyl, 2-(methoxycarbonyl)eth-1-ylsulfonyl, 2-(ethoxycarbonyl)eth-1-ylsulfonyl, 3-(methoxycarbonyl)prop-1-ylsulfonyl, 3-(ethoxycarbonyl)prop-1-ylsulfonyl, 1-(methoxycarbonyl)eth-1-ylsulfonyl, 2-ethoxyeth-1-ylsulfonyl, 1-(prop-2-en-1-yl)cycloprop-1-ylsulfonyl, 3,3,3-trifluoroethylsulfonyl, tetrahydropyran-4-ylsulfonyl, tetrahydrofuran-3-ylmethylsulfonyl, formyl, methylcarbonyl, ethylcarbonyl, prop-1-ylcarbonyl, 1-methyleth-1-ylcarbonyl, but-1-ylcarbonyl, 1-methylprop-1-ylcarbonyl, 2-methylprop-1-ylcarbonyl, 1,1-dimethylethylcarbonyl, pent-1-ylcarbonyl, 1-methylbut-1-ylcarbonyl, 2-methylbut-1-ylcarbonyl, 3-methylbut-1-ylcarbonyl, 1,1-dimethylprop-1-ylcarbonyl, 1,2-dimethylprop-1-ylcarbonyl, 2,2-dimethylprop-1-ylcarbonyl, 1-ethylprop-1-ylcarbonyl, cyclopropylmethylcarbonyl, cyclobutylmethylcarbonyl, cyclopentylmethylcarbonyl, cyclohexylmethylcarbonyl, cyclopropylcarbonyl, cyclobutylcarbonyl, cyclopentylcarbonyl, cyclohexylcarbonyl, methoxymethylcarbonyl, 2-methoxyeth-1-ylcarbonyl, 2-ethoxyeth-1-ylcarbonyl, 3-methoxyprop-1-ylcarbonyl, 3-ethoxyprop-1-ylcarbonyl, methoxycarbonylmethylcarbonyl, 2-(methoxycarbonyl)eth-1-ylcarbonyl, 3-(methoxycarbonyl)prop-1-ylcarbonyl, 4-(methoxycarbonyl)but-1-ylcarbonyl, ethoxycarbonylmethylcarbonyl, 2-(ethoxycarbonyl)eth-1-ylcarbonyl, 3-(ethoxycarbonyl)prop-1-ylcarbonyl, 4-(ethoxycarbonyl)but-1-ylcarbonyl, benzylcarbonyl, 2-phenyleth-1-ylcarbonyl, (2-fluorophenyl)methylcarbonyl, (3-fluorophenyl)methylcarbonyl, (4-fluorophenyl)methylcarbonyl, (2,6-difluorophenyl)-methylcarbonyl, (2,5-difluorophenyl)methylcarbonyl, (2,4-difluorophenyl)-methylcarbonyl, (2,3-difluorophenyl)methylcarbonyl, (2-fluoro-6-chlorophenyl)-methylcarbonyl, (2-chlorophenyl)-methylcarbonyl, (3-chlorophenyl)methylcarbonyl, (4-chlorophenyl)methylcarbonyl, (2,6-dichlorophenyl)methylcarbonyl, (2,5-dichlorophenyl)methylcarbonyl, (2,4-dichlorophenyl)-methylcarbonyl, (2,3-dichlorophenyl)methylcarbonyl, (2-bromophenyl)methylcarbonyl, (3-bromophenyl)methylcarbonyl, (4-bromophenyl)methylcarbonyl, (2-methylphenyl)-methylcarbonyl, (2-trifluoromethylphenyl)methylcarbonyl, (2-methoxyphenyl)methylcarbonyl, (2-nitrophenyl)methylcarbonyl, (3-methylphenyl)methylcarbonyl, (3-trifluoromethylphenyl)-methylcarbonyl, (3-methoxyphenyl)methylcarbonyl, (3-nitrophenyl)methylcarbonyl, (4-methylphenyl)methylcarbonyl, (4-trifluoromethylphenyl)methylcarbonyl, (4-methoxyphenyl)methylcarbonyl, (4-nitrophenyl)methylcarbonyl, pyridin-2-ylmethylcarbonyl, pyridin-3-ylmethylcarbonyl, pyridin-4-ylmethylcarbonyl, (6-fluoropyridin-2-yl)methylcarbonyl, (6-chloro-4-trifluoromethylpyridin-2-yl)methylcarbonyl, (4,6-dichloropyridin-2-yl)methylcarbonyl, (4-fluoropyridin-3-yl)methylcarbonyl, (4-chloropyridin-3-yl)methylcarbonyl, thiophen-2-ylmethylcarbonyl, phenylcarbonyl, (2-fluorophenyl)carbonyl, (3-fluorophenyl)carbonyl, (4-fluorophenyl)carbonyl, (2,6-difluorophenyl)carbonyl, (2,5-difluorophenyl)carbonyl, (2,4-difluorophenyl)-carbonyl, (2,3-difluorophenyl)carbonyl, (2-fluoro-6-chlorophenyl)carbonyl, (2-chlorophenyl)carbonyl, (3-chlorophenyl)carbonyl, (4-chlorophenyl)carbonyl, (2,6-dichlorophenyl)carbonyl, (2,5-dichlorophenyl)carbonyl, (2,4-dichlorophenyl)-carbonyl, (2,3-dichlorophenyl)carbonyl, (2-bromophenyl)carbonyl, (3-bromophenyl)-carbonyl, (4-bromophenyl)carbonyl, (2-methylphenyl)carbonyl, (2-trifluoromethylphenyl)-carbonyl, (2-methoxyphenyl)carbonyl, (2-nitrophenyl)carbonyl, (3-methylphenyl)carbonyl, (3-trifluoromethylphenyl)carbonyl, (3-methoxyphenyl)carbonyl, (3-nitrophenyl)carbonyl, (4-methylphenyl)carbonyl, (4-trifluoromethylphenyl)carbonyl, (4-methoxyphenyl)carbonyl, (4-nitrophenyl)carbonyl, (2-chloro-4-methylsulfonyl-phenyl)carbonyl, pyridin-2-ylcarbonyl, pyridin-3-ylcarbonyl, pyridin-4-ylcarbonyl, (6-fluoropyridin-2-yl)carbonyl, (6-chloro-4-trifluorocarbonylpyridin-2-yl)carbonyl, (4,6-dichloropyridin-2-yl)carbonyl, (4-fluoropyridin-3-yl)carbonyl, (4-chloropyridin-3-yl)carbonyl, thiophen-2-ylcarbonyl, 1,3-oxazol-2-ylcarbonyl, 1,2-oxazol-3-ylcarbonyl, 3-chloro-5-methyl-4,5-dihydro-1,2-oxazol-5-ylcarbonyl, 3-bromo-5-methyl-4,5-dihydro-1,2-oxazol-5-ylcarbonyl, 3-chloro-5-methoxymethyl-4,5-dihydro-1,2-oxazol-5-yl-carbonyl, 3-chloro-5-ethyl-4,5-dihydro-1,2-oxazol-5-ylcarbonyl, 3-chloro-3a,4,5,6-tetrahydro-6aH-cyclopenta[d][1,2]oxazol-6a-ylcarbonyl, (4-trifluoromethylpyridin-3-yl)carbonyl, 3,5-dimethyl-4,5-dihydro-1,2-oxazol-5-ylcarbonyl, 5-methyl-3-trifluoromethyl-4,5-dihydro-1,2-oxazol-5-ylcarbonyl, 3-chloro-5-hydroxymethyl-4,5-dihydro-1,2-oxazol-5-ylcarbonyl, 3-chloro-5-tert-butyldimethylsilyloxymethyl-4,5-dihydro-1,2-oxazol-5-ylcarbonyl, 3-chloro-5-(1-hydroxyeth-1-yl)-4,5-dihydro-1,2-oxazol-5-ylcarbonyl, 3-chloro-5-(1-tert-butyldimethyl-silyloxyeth-1-yl-4,5-dihydro-1,2-oxazol-5-ylcarbonyl, methoxyethoxymethylcarbonyl, ethoxyethoxymethylcarbonyl, hydroxyethoxymethylcarbonyl, methoxyethoxyethoxymethylcarbonyl, ethoxyethoxyethoxymethylcarbonyl, hydroxyethoxyethoxymethylcarbonyl, methoxyethoxyethoxyethoxymethylcarbonyl, ethoxyethoxyethoxyethoxymethylcarbonyl, hydroxyethoxyethoxyethoxymethylcarbonyl, methoxycarbonylmethoxyethoxymethylcarbonyl, 4-trifluoromethylpyridin-3-ylcarbonyloxyethoxyethoxy-methylcarbonyl,hydroxycarbonylmethoxyethoxymethylcarbonyl, methoxycarbonylmethoxyethoxyethoxymethylcarbonyl, hydroxycarbonylmethoxyethoxyethoxymethylcarbonyl, methoxycarbonylmethoxyethoxyethoxyethoxymethylcarbonyl, or hydroxycarbonylmethoxyethoxy-ethoxyethoxymethylcarbonyl.

7. The compound of the general formula (I) as claimed in claim 1 and/or the salt thereof, **characterized in that**
R¹ represents phenyl, 2-fluorophenyl, 3-fluorophenyl, 2-chlorophenyl, 2-methylphenyl, 2-ethyl-phenyl, 2-methoxyphenyl, 2,3-difluorophenyl, 2,4-difluorophenyl, 2,5-difluorophenyl, 2,6-difluorophenyl, 2,4,5-trifluorophenyl, 2,3,4-fluorophenyl, 2-fluoro-6-methylphenyl, 2-fluoro-3-methylphenyl, 2-fluoro-3-chlorophenyl, 2,3-dimethylphenyl, 2-thienyl, 3-chloro-2-thienyl, 3-methyl-2-thienyl, 4-methyl-3-thienyl,
R² represents represents hydrogen, fluorine, chlorine, bromine, iodine, methyl, ethyl, 2,3,4,5,6-pentafluorophenyl, ethoxycarbonyl, cyclopropyl,
R³ represents hydrogen,
n is 0, 2,
R⁴ and R⁵ independently of one another represent hydrogen, methyl, ethyl, prop-2-yl, methoxymethyl, cyclopropyl, cyclobutyl, or
R⁴ and R⁵ together with the carbon atom to which they are bonded form a fully saturated, or partly saturated 3- to 10-membered monocyclic or bicyclic ring optionally interrupted by heteroatoms and optionally having further substitution, and
R⁶ represents hydrogen, BH₂, B(NH₂)₂, 4-amino-1,3,2,4-diazadiboretidin-2-yl, 4-amino-1,3,2,4-dioxadiboretan-2-yl, methylsulfonyl, ethylsulfonyl, prop-1-ylsulfonyl, cyanomethylsulfonyl, methoxycarbonylmethylsulfonyl, 2-ethoxyeth-1-ylsulfonyl, prop-2-en-1-ylsulfonyl, prop-2-en-2-ylsulfonyl, (4-fluorophenyl)methylsulfonyl, formyl, methylcarbonyl, ethylcarbonyl, prop-1-ylcarbonyl, 1-methyleth-1-ylcarbonyl, cyclopropylcarbonyl, cyclopentylcarbonyl, 2-(methoxy-carbonyl)eth-1-ylcarbonyl, ethoxycarbonylmethylcarbonyl, 3-(ethoxycarbonyl)prop-1-ylcarbonyl, (2-trifluoromethylphenyl)carbonyl, 3-chloro-5-methyl-4,5-dihydro-1,2-oxazol-5-ylcarbonyl, 3-chloro-5-methoxymethyl-4,5-dihydro-1,2-oxazol-5-ylcarbonyl, 3-chloro-3a,4,5,6-tetrahydro-6aH-cyclopenta[d][1,2]oxazol-6a-ylcarbonyl, hydroxyethoxyethoxymethylcarbonyl, methoxyethoxyethoxymethylcarbonyl, 4-trifluoromethylpyridin-3-ylcarbonyloxyethoxy-ethoxymethylcarbonyl, hydroxycarbonylmethoxyethoxymethylcarbonyl.

8. The compound of the general formula (I) as claimed in claim 1 and/or the salt thereof, **characterized in that**
R¹ represents phenyl, 2-fluorophenyl, 3-fluorophenyl, 2-chlorophenyl, 2-methylphenyl, 2-ethylphenyl, 2-methoxyphenyl, 2,3-difluorophenyl, 2,4-difluorophenyl, 2,6-difluorophenyl, 2-fluoro-6-methylphenyl, 2-fluoro-3-methylphenyl, 2-fluoro-3-chlorophenyl, 2,3-dimethylphenyl, 2-thienyl, 3-chloro-2-thienyl, 3-methyl-2-thienyl, or 4-methyl-3-thienyl,
R² represents hydrogen, fluorine, chlorine, bromine, methyl, ethyl, 2,3,4,5,6-pentafluorophenyl, or ethoxycarbonyl,
R³ represents hydrogen,
n is 0
R⁴ and R⁵ independently of one another represent hydrogen,
R⁶ represents hydrogen, BH₂, B(NH₂)₂, 4-amino-1,3,2,4-diazadiboretidin-2-yl, 4-amino-1,3,2,4-dioxadiboretan-2-yl, methylsulfonyl, ethylsulfonyl, prop-1-ylsulfonyl, cyanomethylsulfonyl, methoxycarbonylmethylsulfonyl, 2-ethoxyeth-1-ylsulfonyl, prop-2-en-1-ylsulfonyl, prop-2-en-2-ylsulfonyl, (4-fluorophenyl)methylsulfonyl, formyl, methylcarbonyl, ethylcarbonyl, prop-1-ylcarbonyl, 1-methyleth-1-ylcarbonyl, cyclopropylcarbonyl, cyclopentylcarbonyl, 2-(methoxy-carbonyl)eth-1-ylcarbonyl, ethoxycarbonylmethylcarbonyl, 3-(ethoxycarbonyl)prop-1-ylcarbonyl, (2-trifluoromethylphenyl)carbonyl, 3-chloro-5-methyl-4,5-dihydro-1,2-oxazol-5-ylcarbonyl, 3-chloro-5-methoxymethyl-4,5-dihydro-1,2-oxazol-5-ylcarbonyl, or 3-chloro-3a,4,5,6-tetrahydro-6aH-cyclopenta[d][1,2]oxazol-6a-ylcarbonyl.

9. The use of one or more compounds of the general formula (I) and/or salts thereof, as defined in any of claims 1 to 8, as herbicide and/or plant growth regulator.

10. A herbicidal and/or plant growth-regulating composition, **characterized in that** the composition comprises one or more compounds of the general formula (I) and/or salts thereof as defined in any of claims 1 to 8, and one or more further substances selected from groups (i) and/or (ii), with
(i) one or more further agrochemically active substances, selected from the group consisting of insecticides, acaricides, nematicides, further herbicides, fungicides, safeners, fertilizers and/or further growth regulators,
(ii) one or more formulation auxiliaries customary in crop protection.

11. A method for controlling harmful plants or for regulating the growth of plants, **characterized in that** an effective amount
- of one or more compounds of the general formula (I) and/or salts thereof, as defined in any of claims 1 to 8, or
- of a composition as claimed in claim 10,
is applied to the plants, seeds of plants, the soil in which or on which the plants grow or the area under cultivation.

## Patentansprüche

1. Substituierte 2,3-Dihydro[1,3]thiazolo[4,5-b]pyridine der allgemeinen Formel (I) oder deren Salze 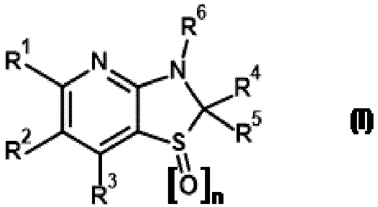 in welcher
R¹ für (C₃-C₈)-Cycloalkyl, (C₃-C₈)-Cycloalkenyl, (C₃-C₈)-Cycloalkoxy, Aryl, Heteroaryl, Heterocyclyl, einen bicyclischen oder einen heterobicyclischen Rest steht, wobei jeder der vorstehend genannten acht Reste unsubstituiert ist oder unabhängig voneinander durch einen oder mehrere Reste, ausgewählt aus der Gruppe R⁷, substituiert ist,
R² Wasserstoff, Halogen, Formyl, Hydroxy, Hydrothio, Hydroxycarbonyl, Aminocarbonyl, Aminothiocarbonyl, (C₁-C₈)-Alkyl, (C₁-C₈)-Haloalkyl, (C₂-C₈)-Alkenyl bedeutet, (C₂-C₈)-Halogenalkenyl, (C₂-C₈)-Alkinyl, (C₂-C₈)-Halogenalkynyl, (C₁-C₈)-Alkoxy, (C₁-C₈)-Haloalkoxy, (C₁-C₈)-Alkoxy-(C₁-C₈)-Alkyl, (C₁-C₈)-Alkylthio, (C₁-C₈)-Haloalkylthio, (C₁-C₈)-Alkylsulfinyl, (C₁-C₈)-Haloalkylsulfinyl, (C₁-C₈)-Alkylsulfonyl, (C₁-C₈)-Haloalkylsulfonyl, (C₁-C₈)-Alkylcarbonyl, (C₂-C₈)-Alkenylcarbonyl, (C₂-C₈)-Alkinylcarbonyl, (C₁-C₈)-Haloalkylcarbonyl, (C₂-C₈)-Haloalkenylcarbonyl, (C₂-C₈)-Haloalkinylcarbonyl, (C₁-C₈)-Alkoxycarbonyl, (C₃-C₈)-Cycloalkyl, (C₃-C₈)-Cycloalkoxy, (C₃-C₈)-Cycloalkylthio, (C₃-C₈)-Cycloalkylsulfinyl, (C₃-C₈)-Cycloalkylsulfonyl, (C₃-C₈)-Halocycloalkyl, (C₃-C₈)-Halocycloalkoxy, (C₃-C₈)-Halocycloalkylthio, (C₃-C₈)-Halocycloalkylsulfinyl, (C₃-C₈)-Halocycloalkylsulfonyl, (C₃-C₈)-Cycloalkyl-(C₁-C₈)-alkyl, (C₁-C₈)-alkyl-(C₃-C₈)-cycloalkyl, (C₃-C₈)-Cycloalkylcarbonyl, (C₃-C₈)-Cycloalkyl-(C₁-C₈)-alkylcarbonyl, (C₁-C₈)-Alkylaminocarbonyl, (C₂-C₁₂)-Dialkylaminocarbonyl, (C₁-C₈)-Alkylaminosulfonyl, (C₂-C₁₂)-Dialkylaminosulfonyl, Tris[(C₁-C₈)alkyl]silyl oder Aryl,
R³ Wasserstoff, Halogen, Cyano, (C₁-C₈)-Alkyl, (C₁-C₈)-Halogenalkyl, (C₂-C₈)-Alkenyl, (C₂-C₈)-Halogenalkenyl, (C₁-C₈)-Alkoxy, (C₁-C₈) Halogenalkoxy, (C₁-C₈)-Alkylthio, (C₁-C₈)-Halogenalkylthio, (C₃-C₈)-Cycloalkyl, (C₃-C₈)-Halogencycloalkyl, (C₃-C₈)-Cycloalkoxy oder (C₃-C₈)-Halogencycloalkoxy,
R⁴ und R⁵ unabhängig voneinander Wasserstoff, (C₁-C₈)-Alkyl, (C₁-C₈)-Halogenalkyl, (C₃-C₈)-Cycloalkyl, (C₃-C₈)-Halogencycloalkyl, oder R¹⁰O-(C₁-C₈)-Alkyl, oder
R⁴ und R⁵ bilden zusammen mit dem Kohlenstoffatom, an das sie gebunden sind, einen vollständig gesättigten oder teilweise gesättigten 3- bis 10-gliedrigen monocyclischen oder bicyclischen Ring, der gegebenenfalls durch Heteroatome unterbrochen ist und gegebenenfalls eine weitere Substitution aufweist,
R⁶ Wasserstoff, (C₁-C₈)-Alkyl, (C₂-C₈)-Alkenyl, (C₂-C₈)-Alkinyl bedeutet, Cyano-(C₁-C₈)-alkyl, R¹⁰O-(C₁-C₈)-alkyl, R⁸R⁹N-(C₁-C₈)-alkyl, NR⁸R⁹, (C₃-C₁₀)-Cycloalkyl-(C₁-C₈)-Alkyl, C(=O)R¹⁰, C(=O)OR¹⁰, C(=O)NR⁸R⁹, R¹⁰O(O)C-(C₁-C₈)-Alkyl, R¹⁰(O=)C-(C₁-C₈)-Alkyl, R⁸R⁹N(O)C-(C₁-C₈)-Alkyl, SO₂R¹¹, R¹¹S(O)ₘ-(C₁-C₈)-alkyl, BR¹²R¹³, Heterocyclyl, Heteroaryl, Aryl-(C₁-C₈)-alkyl, Heteroaryl-(C₁-C₈)-alkyl, Heterocyclyl-(C₁-C₈)-alkyl, Tris[(C₁-C₈)-alkyl]silyl, Bis-[(C₁-C₈)-alkyl](aryl)silyl, (C₁-C₈)-Alkyl-[Bis-(Aryl)]silyl, Tris-[(C₁-C₈)-Alkyl]silyl-(C₁-C₈)-Alkyl, Bis-[(C₁-C₈)-Alkyl](Aryl)silyl-(C₁-C₈)-Alkyl, oder (C₁-C₈)-Alkyl-[Bis-(Aryl)]silyl-(C₁-C₈)-Alkyl,
m 0, 1 oder 2 ist,
n ist 0, 1, 2,
R⁷ Wasserstoff, Halogen, Formyl, Hydroxy, Hydroxycarbonyl, Nitro, Cyano, Amino, Aminocarbonyl, Aminothiocarbonyl, (C₁-C₈)-Alkyl, (C₁-C₈)-Haloalkyl, (C₂-C₈)-Alkenyl, (C₂-C₈)-Haloalkenyl, (C₂-C₈)-Alkinyl, (C₂-C₈)-Haloalkinyl, (C₁-C₈)-Alkoxy, (C₁-C₈)-Haloalkoxy, (C₁-C₈)-Alkylthio, (C₁-C₈)-Halogenalkylthio, (C₁-C₈)-Alkylsulfinyl, (C₁-C₈)-Haloalkylsulfinyl, (C₁-C₈)-Alkylsulfonyl, (C₁-C₈)-Haloalkylsulfonyl, (C₁-C₈)-Alkylcarbonyl, (C₁-C₈)-Haloalkylcarbonyl, (C₂-C₈)- Alkenylcarbonyl, (C₂-C₈)-Halogenalkenylcarbonyl, (C₂-C₈)-Alkinylcarbonyl, (C₂-C₈)-Halogenalkinylcarbonyl, (C₁-C₈)-Alkoxycarbonyl, (C₁-C₈)-Halogenalkoxycarbonyl, (C₃-C₈)-Cycloalkyl, (C₃-C₈)-Halocycloalkyl, (C₃-C₈)-Cycloalkoxy, (C₃-C₈)-Halocycloalkoxy, (C₃-C₈)-Cycloalkylthio, (C₃-C₈)-Halocycloalkylthio, (C₃-C₈)-Cycloalkylsulfinyl, (C₃-C₈)-Halocycloalkylsulfinyl, (C₃-C₈)-Cycloalkylsulfonyl, (C₃-C₈)-Halocycloalkylsulfonyl, (C₃-C₈)-Cycloalkyl-(C₁-C₈)-alkyl, (C₁-C₈)-alkyl-(C₃-C₈)-cycloalkyl, (C₁-C₈)-Alkoxycarbonyl-(C₁-C₈)-Alkyl, Hydroxycarbonyl-(C₁-C₈)-Alkyl, (C₃-C₈)-Cycloalkylcarbonyl, (C₃-C₈)-Cycloalkyl-(C₁-C₈)-Alkylcarbonyl, (C₁-C₈)-Alkylamino, (C₂-C₁₂)-Dialkylamino, (C₁-C₈)-Alkylaminocarbonyl, (C₂-C₁₂)-Dialkylaminocarbonyl, (C₁-C₈)-Alkylaminosulfonyl, (C₂-C₁₂)-Dialkylaminosulfonyl oder Tris[(C₁-C₈)alkyl]silyl.
-R⁸ und R⁹ unabhängig voneinander Wasserstoff, (C₁-C₈)-Alkyl, (C₂-C₈)-Alkenyl, (C₂-C₈)-Alkinyl darstellen, (C₁-C₈)-Cyanalkyl, (C₁-C₁₀)-Halogenalkyl, (C₂-C₈)-Halogenalkenyl, (C₃-C₈)-Halogenalkynyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-alkoxy-(C₁-C₈)-alkyl, (C₁-C₈)-Alkoxy-(C₁-C₈)-Alkoxy-(C₁-C₈)-Alkoxy-(C₁-C₈)-Alkyl, (C₁-C₈)-Haloalkoxy-(C₁-C₈)-Alkyl, (C₁-C₈)-Alkylthio-(C₁-C₈)-Alkyl, (C₁-C₈)-Haloalkylthio-(C₁-C₈)-Alkyl, (C₁-C₈)-Alkoxy-(C₁-C₈)-Haloalkyl, Aryl, Aryl-(C₁-C₈)-alkyl, Heteroaryl, Heteroaryl-(C₁-C₈)-alkyl, (C₃-C₈)-cycloalkyl-(C₁-C₈)-alkyl, (C₄-C₁₀)-Cycloalkenyl-(C₁-C₈)-alkyl, COR¹⁰, SO₂R¹¹, Heterocyclyl, (C₁-C₈)-Alkoxycarbonyl, Bis-[(C₁-C₈)-alkyl]aminocarbonyl-(C₁-C₈)-alkyl, (C₁-C₈)-Alkyl-aminocarbonyl-(C₁-C₈)-Alkyl, Aryl-(C₁-C₈)-Alkyl-aminocarbonyl-(C₁-C₈)-Alkyl, (C₁-C₈)-Alkoxycarbonyl-(C₁-C₈)-Alkyl, (C₂-C₈)-Alkenyloxycarbonyl-(C₁-C₈)-Alkyl, (C₂-C₈)-Alkinyloxycarbonyl-(C₁-C₈)-Alkyl, Aryl-(C₁-C₈)-Alkoxycarbonyl-(C₁-C₈)-Alkyl, Heteroaryl-(C₁-C₈)-Alkoxycarbonyl-(C₁-C₈)-Alkyl, Heterocyclyl-(C₁-C₈)-Alkoxycarbonyl-(C₁-C₈)-Alkyl, Aryl-(C₁-C₈)-Alkoxycarbonyl, Heteroaryl-(C₁-C₈)-Alkoxycarbonyl, (C₂-C₈)-Alkenyloxycarbonyl, (C₂-C₈)-Alkinyloxycarbonyl, Heterocyclyl-(C₁-C₈)-Alkyl, oder
R⁸ und R⁹ zusammen mit dem Kohlenstoffatom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist,
R¹⁰ Wasserstoff, (C₁-C₈)-Alkyl, (C₂-C₈)-Alkenyl, (C₂-C₈)-Alkinyl, (C₁-C₈)-Cyanoalkyl darstellt, (C₁-C₁₀)-Halogenalkyl, (C₂-C₈)-Halogenalkenyl, (C₃-C₈)-Halogenalkinyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-Alkyl, (C₁-C₈)-Haloalkoxy-(C₁-C₈)-Alkyl, (C₁-C₈)-Alkoxy-(C₁-C₈)-Halogenalkyl, (C₁-C₈)-Alkoxy-(C₁-C₈)-Alkoxy-(C₁-C₈)-alkyl, (C₁-C₈)-Alkoxy-(C₁-C₈)-Alkoxy-(C₁-C₈)-Alkoxy-(C₁-C₈)-Alkyl, (C₁-C₈)-Alkoxy-(C₁-C₈)-Alkoxy-(C₁-C₈)-Alkoxy-(C₁-C₈)-Alkoxy-(C₁-C₈)-alkyl, Aryl, Aryl-(C₁-C₈)-alkyl, Aryl-(C₁-C₈)-Alkoxy-(C₁-C₈)-Alkyl, Aryloxy-(C₁-C₈)-Alkyl, Heteroaryl-(C₁-C₈)-Alkoxy-(C₁-C₈)-Alkyl, Heteroaryloxy-(C₁-C₈)-alkyl, Heteroaryl, Heteroaryl-(C₁-C₈)-alkyl, (C₃-C₈)-cycloalkyl-(C₁-C₈)-alkyl, (C₄-C₁₀)-Cycloalkenyl-(C₁-C₈)-Alkyl, Bis-[(C₁-C₈)-Alkyl]Aminocarbonyl-(C₁-C₈)-Alkyl, (C₁-C₈)-Alkyl-aminocarbonyl-(C₁-C₈)-Alkyl, Aryl-(C₁-C₈)-Alkyl-aminocarbonyl-(C₁-C₈)-Alkyl, Bis-[(C₁-C₈)-alkyl]amino-(C₂-C₆)-alkyl, (C₁-C₈)-alkyl-amino-(C₂-C₆)-alkyl, Aryl-(C₁-C₈)-alkylamino-(C₂-C₆)-alkyl, R¹¹(O)ₘS-(C₁-C₈)-Alkyl, Hydroxycarbonyl-(C₁-C₈)-Alkyl, Heterocyclyl, Heterocyclyl-(C₁-C₈)-Alkyl, Heterocyclyl-(C₁-C₈)-alkoxy-(C₁-C₈)-alkyl, Heterocyclyloxy-(C₁-C₈)-alkyl, Tris-[(C₁-C₈)-alkyl]silyl-(C₁-C₈)-alkyl, Bis-[(C₁-C₈)-alkyl](aryl)silyl-(C₁-C₈)-alkyl, [(C₁-C₈)-alkyl]-Bis-(aryl)silyl-(C₁-C₈)-alkyl, (C₁-C₈)-Alkylcarbonyloxy-(C₁-C₈)-Alkyl, (C₃-C₈)-Cycloalkylcarbonyloxy-(C₁-C₈)-Alkyl, Arylcarbonyloxy-(C₁-C₈)-alkyl, Heteroarylcarbonyloxy-(C₁-C₈)-alkyl, Heterocyclylcarbonyloxy-(C₁-C₈)-alkyl, (C₁-C₈)-Alkoxycarbonyl, (C₁-C₈)-Alkoxycarbonyl-(C₁-C₈)-Alkyl, (C₂-C₈)-Alkenyloxycarbonyl-(C₁-C₈)-Alkyl, (C₂-C₈)-Alkinyloxycarbonyl-(C₁-C₈)-Alkyl, Aryl-(C₁-C₈)-Alkoxycarbonyl-(C₁-C₈)-Alkyl, Heteroaryl-(C₁-C₈)-Alkoxycarbonyl-(C₁-C₈)-Alkyl, Heterocyclyl-(C₁-C₈)-Alkoxycarbonyl-(C₁-C₈)-Alkyl, Hydroxy-(C₁-C₈)-Alkyl, Hydroxy-(C₁-C₈)-alkoxy-(C₁-C₈)-alkyl, Hydroxy-(C₁-C₈)-alkoxy-(C₁-C₈)-alkoxy-(C₁-C₈)-alkyl, Hydroxy-(C₁-C₈)-alkoxy-(C₁-C₈)-alkoxy-(C₁-C₈)-alkoxy-(C₁-C₈)-alkyl, (C₁-C₈)-Alkylcarbonyloxy-(C₁-C₈)-alkoxy-(C₁-C₈)-alkyl, (C₁-C₈)-Alkylcarbonyloxy-(C₁-C₈)-Alkoxy-(C₁-C₈)-Alkoxy-(C₁-C₈)-Alkyl, (C₁-C₈)-Alkylcarbonyloxy-(C₁-C₈)-Alkoxy-(C₁-C₈)-Alkoxy-(C₁-C₈)-Alkoxy-(C₁-C₈)-Alkyl, (C₁-C₈)-Alkoxycarbonyl-(C₁-C₈)-Alkoxy-(C₁-C₈)-Alkyl, (C₁-C₈)-Alkoxycarbonyl-(C₁-C₈)-Alkoxy-(C₁-C₈)-Alkoxy-(C₁-C₈)-Alkyl, (C₁-C₈)-Alkoxycarbonyl-(C₁-C₈)-Alkoxy-(C₁-C₈)-Alkoxy-(C₁-C₈)-Alkoxy-(C₁-C₈)-Alkyl, Heteroarylcarbonyloxy-(C₁-C₈)-alkoxy-(C₁-C₈)-alkyl, Heteroarylcarbonyloxy-(C₁-C₈)-alkoxy-(C₁-C₈)-alkoxy-(C₁-C₈)-alkyl, Heteroarylcarbonyloxy-(C₁-C₈)-Alkoxy-(C₁-C₈)-Alkoxy-(C₁-C₈)-Alkoxy-(C₁-C₈)-Alkyl, Heterocyclylcarbonyloxy-(C₁-C₈)-alkoxy-(C₁-C₈)-alkyl, Heterocyclylcarbonyloxy-(C₁-C₈)-alkoxy-(C₁-C₈)-alkoxy-(C₁-C₈)-alkyl, Heterocyclylcarbonyloxy-(C₁-C₈)-Alkoxy-(C₁-C₈)-Alkoxy-(C₁-C₈)-Alkoxy-(C₁-C₈)-Alkyl, Hydroxycarbonyl-(C₁-C₈)-alkoxy-(C₁-C₈)-alkyl, Hydroxycarbonyl-(C₁-C₈)-alkoxy-(C₁-C₈)-alkoxy-(C₁-C₈)-alkyl, oder Hydroxycarbonyl-(C₁-C₈)-alkoxy-(C₁-C₈)-alkoxy-(C₁-C₈)-alkoxy-(C₁-C₈)-alkyl,
R¹¹ Wasserstoff, (C₁-C₈)-Alkyl, (C₂-C₈)-Alkenyl, (C₂-C₈)-Alkinyl, (C₁-C₈)-Cyanoalkyl bedeutet, (C₁-C₁₀)-Halogenalkyl, (C₂-C₈)-Halogenalkenyl, (C₃-C₈)-Halogenalkynyl, (C₃-C₁₀)-Cycloalkyl, (C₃-C₁₀)-Halocycloalkyl, (C₄-C₁₀)-Cycloalkenyl, (C₄-C₁₀)-Halocycloalkenyl, (C₁-C₈)-Alkoxy-(C₁-C₈)-alkyl, (C₁-C₈)-Alkoxy-(C₁-C₈)-halogenalkyl, Aryl, Aryl-(C₁-C₈)-alkyl, Heteroaryl, Heteroaryl-(C₁-C₈)-alkyl, Heterocyclyl-(C₁-C₈)-alkyl, (C₃-C₈)-cycloalkyl-(C₁-C₈)-alkyl, (C₄-C₁₀)-Cycloalkenyl-(C₁-C₈)-alkyl, Bis-[(C₁-C₈)-alkyl]amino, (C₁-C₈)-Alkylamino, Aryl-(C₁-C₈)-amino, Aryl-(C₁-C₆)-alkylamino, Aryl-[(C₁-C₈)-alkyl]amino, Heteroaryl-(C₁-C₈)-amino, Heteroaryl-(C₁-C₆)-alkyl-amino, Heteroaryl-[(C₁-C₈)-alkyl]amino; Heterocyclyl-(C₁-C₈)-amino, Heterocyclyl-(C₁-C₆)-alkyl-amino, Heterocyclyl-[(C₁-C₈)-alkyl]amino; (C₃-C₈)-Cycloalkyl-amino, (C₃-C₈)-Cycloalkyl-[(C₁-C₈)-alkyl]amino, oder N-Azetidinyl, N-Pyrrolidinyl, N-Piperidinyl, N-Morpholinyl, und
R¹² und R¹³ unabhängig voneinander für Wasserstoff, NR⁸R⁹, OR¹⁰, oder
R¹² und R¹³ zusammen mit dem Boratom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist.

2. Die Verbindung der allgemeinen Formel (I), wie in Anspruch 1 beansprucht, und/oder das Salz davon, **dadurch gekennzeichnet, dass**
R¹ für (C₃-C₇)-Cycloalkyl, (C₃-C₇)-Cycloalkenyl, (C₃-C₇)-Cycloalkoxy, Aryl, Heteroaryl, Heterocyclyl, einen bicyclischen oder einen heterobicyclischen Rest steht, wobei jeder der zuvor erwähnten acht Reste unsubstituiert ist oder unabhängig voneinander durch einen oder mehrere Reste, ausgewählt aus der Gruppe R⁷, substituiert ist,
R² Wasserstoff, Halogen, Formyl, Hydroxy, Hydrothio, Hydroxycarbonyl, Aminocarbonyl, Aminothiocarbonyl, (C₁-C₇)-Alkyl, (C₁-C₇)-Haloalkyl, (C₂-C₇)-Alkenyl, (C₂-C₇)-Haloalkenyl, (C₂-C₇)-Alkinyl, (C₂-C₇)-Halogenalkinyl, (C₁-C₇)-Alkoxy, (C₁-C₇)-Haloalkoxy, (C₁-C₇)-Alkoxy-(C₁-C₇)-alkyl, (C₁-C₇)-Alkylthio, (C₁-C₇)-Haloalkylthio, (C₁-C₇)-Alkylsulfinyl, (C₁-C₇)-Haloalkylsulfinyl, (C₁-C₇)-Alkylsulfonyl, (C₁-C₇)-Haloalkylsulfonyl, (C₁-C₇)-Alkylcarbonyl, (C₂-C₇)-Alkenylcarbonyl, (C₂-C₇)-Alkinylcarbonyl, (C₁-C₇)-Haloalkylcarbonyl, (C₂-C₇)-Haloalkenylcarbonyl, (C₂-C₇)-Haloalkinylcarbonyl, (C₁-C₇)-Alkoxycarbonyl, (C₃-C₇)-Cycloalkyl, (C₃-C₇)-Cycloalkoxy, (C₃-C₇)-Cycloalkylthio, (C₃-C₇)-Cycloalkylsulfinyl, (C₃-C₇)-Cycloalkylsulfonyl, (C₃-C₇)-Halocycloalkyl, (C₃-C₇)-Halocycloalkoxy, (C₃-C₇)-Halocycloalkylthio, (C₃-C₇)-Halocycloalkylsulfinyl, (C₃-C₇)-Halocycloalkylsulfonyl, (C₃-C₇)-Cycloalkyl-(C₁-C₇)-Alkyl, (C₁-C₇)-Alkyl-(C₃-C₇)-Cycloalkyl, (C₃-C₇)-Cycloalkylcarbonyl, (C₃-C₇)-Cycloalkyl-(C₁-C₇)-Alkylcarbonyl, (C₁-C₇)-Alkylaminocarbonyl, (C₂-C₁₂)-Dialkylaminocarbonyl, (C₁-C₇)-Alkylaminosulfonyl, (C₂-C₁₂)-Dialkylaminosulfonyl oder Tris[(C₁-C₇)alkyl]silyl, oder Aryl,
R³ Wasserstoff, Halogen, Cyano, (C₁-C₇)-Alkyl, (C₁-C₇)-Halogenalkyl, (C₂-C₇)-Alkenyl, (C₂-C₇)-Halogenalkenyl, (C₁-C₇)-Alkoxy, (C₁-C₇)-Haloalkoxy bedeutet, (C₁-C₇)-Alkylthio, (C₁-C₇)-Halogenalkylthio, (C₃-C₇)-Cycloalkyl, (C₃-C₇)-Halogencycloalkyl, (C₃-C₇)-Cycloalkoxy oder (C₃-C₇)-Halogencycloalkoxy,
R⁴ und R⁵ unabhängig voneinander Wasserstoff, (C₁-C₇)-Alkyl, (C₁-C₇)-Halogenalkyl, (C₃-C₇)-Cycloalkyl, (C₃-C₇)-Halogencycloalkyl, oder R¹⁰O-(C₁-C₇)-Alkyl, oder
R⁴ und R⁵ bilden zusammen mit dem Kohlenstoffatom, an das sie gebunden sind, einen vollständig gesättigten oder teilweise gesättigten 3- bis 10-gliedrigen monocyclischen oder bicyclischen Ring, der gegebenenfalls durch Heteroatome unterbrochen ist und gegebenenfalls eine weitere Substitution aufweist,
R⁶ Wasserstoff, (C₁-C₇)-Alkyl, (C₂-C₇)-Alkenyl, (C₂-C₇)-Alkinyl bedeutet, Cyano-(C₁-C₇)-alkyl, R¹⁰O-(C₁-C₇)-alkyl, R⁸R⁹N-(C₁-C₇)-alkyl, NR⁸R⁹, (C₃-C₁₀)-Cycloalkyl-(C₁-C₇)-Alkyl, C(=O)R¹⁰, C(=O)OR¹⁰, C(=O)NR⁸R⁹, R¹⁰O(O)C-(C₁-C₇)-Alkyl, R¹⁰(O=)C-(C₁-C₇)-Alkyl, R⁸R⁹N(O)C-(C₁-C₇)-Alkyl, SO₂R¹¹, R¹¹S(O)ₘ-(C₁-C₇)-alkyl, BR¹²R¹³, Heterocyclyl, Heteroaryl, Aryl-(C₁-C₇)-alkyl, Heteroaryl-(C₁-C₇)-alkyl, Heterocyclyl-(C₁-C₇)-alkyl, Tris[(C₁-C₇)-alkyl]silyl, Bis-[(C₁-C₇)-alkyl](aryl)silyl, (C₁-C₇)-Alkyl-[Bis-(Aryl)]silyl, Tris-[(C₁-C₇)-Alkyl]silyl-(C₁-C₇)-Alkyl, Bis-[(C₁-C₇)-Alkyl](Aryl)silyl-(C₁-C₇)-Alkyl, oder (C₁-C₇)-Alkyl-[Bis-(Aryl)]silyl-(C₁-C₇)-Alkyl,
m 0, 1 oder 2 ist,
n ist 0, 1, 2,
R⁷ Wasserstoff, Halogen, Formyl, Hydroxy, Hydroxycarbonyl, Nitro, Cyano, Amino, Aminocarbonyl, Aminothiocarbonyl, (C₁-C₇)-Alkyl, (C₁-C₇)-Haloalkyl, (C₂-C₇)-Alkenyl, (C₂-C₇)-Halogenalkenyl, (C₂-C₇)-Alkinyl, (C₂-C₇)-Halogenalkynyl, (C₁-C₇)-Alkoxy, (C₁-C₇)-Haloalkoxy, (C₁-C₇)-Alkylthio, (C₁-C₇)-Haloalkylthio, (C₁-C₇)-Alkylsulfinyl, (C₁-C₇)-Haloalkylsulfinyl, (C₁-C₇)-Alkylsulfonyl, (C₁-C₇)-Haloalkylsulfonyl, (C₁-C₇)-Alkylcarbonyl, (C₁-C₇)-Haloalkylcarbonyl, (C₂-C₇)-Alkenylcarbonyl, (C₂-C₇)-Haloalkenylcarbonyl, (C₂-C₇)-Alkinylcarbonyl, (C₂-C₇)-Haloalkinylcarbonyl, (C₁-C₇)-Alkoxycarbonyl, (C₁-C₇)-Haloalkoxycarbonyl, (C₃-C₇)-Cycloalkyl, (C₃-C₇)-Halocycloalkyl, (C₃-C₇)-Cycloalkoxy, (C₃-C₇)-Halocycloalkoxy, (C₃-C₇)-Cycloalkylthio, (C₃-C₇)-Halocycloalkylthio, (C₃-C₇)-Cycloalkylsulfinyl, (C₃-C₇)-Halocycloalkylsulfinyl, (C₃-C₇)-Cycloalkylsulfonyl, (C₃-C₇)-Halocycloalkylsulfonyl, (C₃-C₇)-Cycloalkyl-(C₁-C₇)-alkyl, (C₁-C₇)-Alkyl-(C₃-C₇)-Cycloalkyl, (C₁-C₇)-Alkoxycarbonyl-(C₁-C₇)-Alkyl, Hydroxycarbonyl-(C₁-C₇)-Alkyl, (C₃-C₇)-Cycloalkylcarbonyl, (C₃-C₇)-Cycloalkyl-(C₁-C₇)-Alkylcarbonyl, (C₁-C₇)-Alkylamino, (C₂-C₁₂)-Dialkylamino, (C₁-C₇)-Alkylaminocarbonyl, (C₂-C₁₂)-Dialkylaminocarbonyl, (C₁-C₇)-Alkylaminosulfonyl, (C₂-C₁₂)-Dialkylaminosulfonyl oder Tris[(C₁-C₇)alkyl]silyl,
R⁸ und R⁹ unabhängig voneinander Wasserstoff, (C₁-C₇)-Alkyl, (C₂-C₇)-Alkenyl, (C₂-C₇)-Alkinyl bedeuten, (C₁-C₇)-Cyanalkyl, (C₁-C₇)-Halogenalkyl, (C₂-C₇)-Halogenalkenyl, (C₃-C₇)-Halogenalkynyl, (C₃-C₇)-Cycloalkyl, (C₃-C₇)-Halocycloalkyl, (C₄-C₇)-Cycloalkenyl, (C₄-C₁₀)-Halocycloalkenyl, (C₁-C₇)-Alkoxy-(C₁-C₇)-alkyl, (C₁-C₇)-Alkoxy-(C₁-C₇)-alkoxy-(C₁-C₇)-alkyl, (C₁-C₇)-Alkoxy-(C₁-C₇)-Alkoxy-(C₁-C₇)-Alkoxy-(C₁-C₇)-Alkyl, (C₁-C₇)-Haloalkoxy-(C₁-C₇)-alkyl, (C₁-C₇)-Alkylthio-(C₁-C₇)-alkyl, (C₁-C₇)-Haloalkylthio-(C₁-C₇)-Alkyl, (C₁-C₇)-Alkoxy-(C₁-C₇)-Haloalkyl, Aryl, Aryl-(C₁-C₇)-alkyl, Heteroaryl, Heteroaryl-(C₁-C₇)-alkyl, (C₃-C₇)-cycloalkyl-(C₁-C₇)-alkyl, (C₄-C₇)-Cycloalkenyl-(C₁-C₇)-alkyl, COR¹⁰, SO₂R¹¹, Heterocyclyl, (C₁-C₇)-Alkoxycarbonyl, Bis-[(C₁-C₇)-alkyl]aminocarbonyl-(C₁-C₇)-alkyl, (C₁-C₇)-Alkyl-aminocarbonyl-(C₁-C₇)-Alkyl, Aryl-(C₁-C₇)-Alkyl-aminocarbonyl-(C₁-C₇)-Alkyl, (C₁-C₇)-Alkoxycarbonyl-(C₁-C₇)-Alkyl, (C₂-C₇)-Alkenyloxycarbonyl-(C₁-C₇)-Alkyl, (C₂-C₇)-Alkinyloxycarbonyl-(C₁-C₇)-Alkyl, Aryl-(C₁-C₇)-Alkoxycarbonyl-(C₁-C₇)-Alkyl, Heteroaryl-(C₁-C₇)-Alkoxycarbonyl-(C₁-C₇)-Alkyl, Heterocyclyl-(C₁-C₇)-Alkoxycarbonyl-(C₁-C₇)-Alkyl, Aryl-(C₁-C₇)-Alkoxycarbonyl, Heteroaryl-(C₁-C₇)-Alkoxycarbonyl, (C₂-C₇)-Alkenyloxycarbonyl, (C₂-C₇)-Alkinyloxycarbonyl, Heterocyclyl-(C₁-C₇)-Alkyl, oder
R⁸ und R⁹ zusammen mit dem Kohlenstoffatom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist,
R¹⁰ Wasserstoff, (C₁-C₇)-Alkyl, (C₂-C₇)-Alkenyl, (C₂-C₇)-Alkinyl, (C₁-C₇)-Cyanoalkyl darstellt, (C₁-C₇)-Halogenalkyl, (C₂-C₇)-Halogenalkenyl, (C₃-C₇)-Halogenalkinyl, (C₃-C₇)-Cycloalkyl, (C₃-C₇)-Halocycloalkyl, (C₄-C₇)-Cycloalkenyl, (C₄-C₇)-Halocycloalkenyl, (C₁-C₇)-Alkoxy-(C₁-C₇)-Alkyl, (C₁-C₇)-Haloalkoxy-(C₁-C₇)-Alkyl, (C₁-C₇)-Alkoxy-(C₁-C₇)-Halogenalkyl, (C₁-C₇)-Alkoxy-(C₁-C₇)-Alkoxy-(C₁-C₇)-alkyl, (C₁-C₇)-Alkoxy-(C₁-C₇)-Alkoxy-(C₁-C₇)-Alkoxy-(C₁-C₇)-Alkyl, (C₁-C₇)-Alkoxy-(C₁-C₇)-Alkoxy-(C₁-C₇)-Alkoxy-(C₁-C₇)-Alkoxy-(C₁-C₇)-alkyl, Aryl, Aryl-(C₁-C₇)-alkyl, Aryl-(C₁-C₇)-alkoxy-(C₁-C₇)-alkyl, Aryloxy-(C₁-C₇)-alkyl, Heteroaryl-(C₁-C₇)-alkoxy-(C₁-C₇)-alkyl, Heteroaryloxy-(C₁-C₇)-alkyl, Heteroaryl, Heteroaryl-(C₁-C₇)-alkyl, (C₃-C₇)-cycloalkyl-(C₁-C₇)-alkyl, (C₄-C₇)-Cycloalkenyl-(C₁-C₇)-Alkyl, Bis-[(C₁-C₇)-Alkyl]Aminocarbonyl-(C₁-C₇)-Alkyl, (C₁-C₇)-Alkyl-aminocarbonyl-(C₁-C₇)-Alkyl, Aryl-(C₁-C₇)-Alkyl-aminocarbonyl-(C₁-C₇)-Alkyl, Bis-[(C₁-C₇)-alkyl]amino-(C₂-C₆)-alkyl, (C₁-C₇)-alkyl-amino-(C₂-C₆)-alkyl, Aryl-(C₁-C₇)-alkylamino-(C₂-C₆)-alkyl, R¹¹(O)ₘS-(C₁-C₇)-Alkyl, Hydroxycarbonyl-(C₁-C₇)-Alkyl, Heterocyclyl, Heterocyclyl-(C₁-C₇)-Alkyl, Heterocyclyl-(C₁-C₇)-alkoxy-(C₁-C₇)-alkyl, Heterocyclyloxy-(C₁-C₇)-alkyl, Tris-[(C₁-C₇)-alkyl]silyl-(C₁-C₇)-alkyl, Bis-[(C₁-C₇)-alkyl](Aryl)silyl-(C₁-C₇)-alkyl, [(C₁-C₇)-alkyl]-Bis-(Aryl)silyl-(C₁-C₇)-alkyl, (C₁-C₇)-Alkylcarbonyloxy-(C₁-C₇)-Alkyl, (C₃-C₇)-Cycloalkylcarbonyloxy-(C₁-C₇)-Alkyl, Arylcarbonyloxy-(C₁-C₇)-alkyl, Heteroarylcarbonyloxy-(C₁-C₇)-alkyl, Heterocyclylcarbonyloxy-(C₁-C₇)-alkyl, (C₁-C₇)-Alkoxycarbonyl, (C₁-C₇)-Alkoxycarbonyl-(C₁-C₇)-Alkyl, (C₂-C₇)-Alkenyloxycarbonyl-(C₁-C₇)-Alkyl, (C₂-C₇)-Alkinyloxycarbonyl-(C₁-C₇)-Alkyl, Aryl-(C₁-C₇)-Alkoxycarbonyl-(C₁-C₇)-Alkyl, Heteroaryl-(C₁-C₇)-Alkoxycarbonyl-(C₁-C₇)-Alkyl, Heterocyclyl-(C₁-C₇)-Alkoxycarbonyl-(C₁-C₇)-Alkyl, Hydroxy-(C₁-C₇)-Alkyl, Hydroxy-(C₁-C₇)-alkoxy-(C₁-C₇)-alkyl, Hydroxy-(C₁-C₇)-alkoxy-(C₁-C₇)-alkoxy-(C₁-C₇)-alkyl, Hydroxy-(C₁-C₇)-alkoxy-(C₁-C₇)-alkoxy-(C₁-C₇)-alkoxy-(C₁-C₇)-alkyl, (C₁-C₇)-Alkylcarbonyloxy-(C₁-C₇)-alkoxy-(C₁-C₇)-alkyl, (C₁-C₇)-Alkylcarbonyloxy-(C₁-C₇)-Alkoxy-(C₁-C₇)-Alkoxy-(C₁-C₇)-Alkyl, (C₁-C₇)-Alkylcarbonyloxy-(C₁-C₇)-Alkoxy-(C₁-C₇)-Alkoxy-(C₁-C₇)-Alkoxy-(C₁-C₇)-Alkyl, (C₁-C₇)-Alkoxycarbonyl-(C₁-C₇)-Alkoxy-(C₁-C₇)-Alkyl, (C₁-C₇)-Alkoxycarbonyl-(C₁-C₇)-Alkoxy-(C₁-C₇)-Alkoxy-(C₁-C₇)-Alkyl, (C₁-C₇)-Alkoxycarbonyl-(C₁-C₇)-Alkoxy-(C₁-C₇)-Alkoxy-(C₁-C₇)-Alkoxy-(C₁-C₇)-Alkyl, Heteroarylcarbonyloxy-(C₁-C₇)-alkoxy-(C₁-C₇)-alkyl, Heteroarylcarbonyloxy-(C₁-C₇)-alkoxy-(C₁-C₇)-alkoxy-(C₁-C₇)-alkyl, Heteroarylcarbonyloxy-(C₁-C₇)-alkoxy-(C₁-C₇)-alkoxy-(C₁-C₇)-alkoxy-(C₁-C₇)-alkyl, Heterocyclylcarbonyloxy-(C₁-C₇)-alkoxy-(C₁-C₇)-alkyl, Heterocyclylcarbonyloxy-(C₁-C₇)-alkoxy-(C₁-C₇)-alkoxy-(C₁-C₇)-alkyl, Heterocyclylcarbonyloxy-(C₁-C₇)-Alkoxy-(C₁-C₇)-Alkoxy-(C₁-C₇)-Alkoxy-(C₁-C₇)-Alkyl, Hydroxycarbonyl-(C₁-C₇)-alkoxy-(C₁-C₇)-alkyl, Hydroxycarbonyl-(C₁-C₇)-alkoxy-(C₁-C₇)-alkoxy-(C₁-C₇)-alkyl, oder Hydroxycarbonyl-(C₁-C₇)-alkoxy-(C₁-C₇)-alkoxy-(C₁-C₇)-alkoxy-(C₁-C₇)-alkyl,
R¹¹ Wasserstoff, (C₁-C₇)-Alkyl, (C₂-C₇)-Alkenyl, (C₂-C₇)-Alkinyl, (C₁-C₇)-Cyanoalkyl, (C₁-C₇)-Halogenalkyl, (C₂-C₇)-Halogenalkenyl bedeutet, (C₃-C₇)-Halogenalkynyl, (C₃-C₇)-Cycloalkyl, (C₃-C₇)-Halogencycloalkyl, (C₄-C₇)-Cycloalkenyl, (C₄-C₇)-Halocycloalkenyl, (C₁-C₇)-Alkoxy-(C₁-C₇)-alkyl, (C₁-C₇)-Alkoxy-(C₁-C₇)-Halogenalkyl, Aryl, Aryl-(C₁-C₇)-Alkyl, Heteroaryl, Heteroaryl-(C₁-C₇)-alkyl, Heterocyclyl-(C₁-C₇)-alkyl, (C₃-C₇)-cycloalkyl-(C₁-C₇)-alkyl, (C₄-C₁₀)-Cycloalkenyl-(C₁-C₇)-Alkyl, Bis-[(C₁-C₇)-Alkyl]amino, (C₁-C₇)-Alkyl-amino, Aryl-(C₁-C₇)-amino, Aryl-(C₁-C₆)-alkyl-amino, Aryl-[(C₁-C₇)-alkyl]amino; Heteroaryl-(C₁-C₇)-amino, Heteroaryl-(C₁-C₆)-alkyl-amino, Heteroaryl-[(C₁-C₇)-alkyl]-amino; Heterocyclyl-(C₁-C₇)-amino, Heterocyclyl-(C₁-C₆)-alkyl-amino, Heterocyclyl-[(C₁-C₇)-alkyl]amino; (C₃-C₇)-Cycloalkyl-amino, (C₃-C₇)-Cycloalkyl-[(C₁-C₇)-alkyl]amino; N-Azetidinyl, N-Pyrrolidinyl, N-Piperidinyl oder N-Morpholinyl,
R¹² und R¹³ unabhängig voneinander Wasserstoff, NR⁸R⁹, OR¹⁰, oder
R¹² und R¹³ zusammen mit dem Boratom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist.

3. Die Verbindung der allgemeinen Formel (I), wie in Anspruch 1 beansprucht, und/oder das Salz davon, **dadurch gekennzeichnet, dass**
R¹ für (C₃-C₆)-Cycloalkyl, (C₃-C₆)-Cycloalkenyl, (C₃-C₆)-Cycloalkoxy, Aryl, Heteroaryl, Heterocyclyl, einen bicyclischen oder einen heterobicyclischen Rest steht, wobei jeder der zuvor erwähnten acht Reste unsubstituiert ist oder unabhängig voneinander durch einen oder mehrere Reste, ausgewählt aus der Gruppe R⁷, substituiert ist,
R² Wasserstoff, Halogen, Formyl, Hydroxy, Hydrothio, Hydroxycarbonyl, Aminocarbonyl, Aminothiocarbonyl, (C₁-C₆)-Alkyl, (C₁-C₆)-Haloalkyl, (C₂-C₆)-Alkenyl, (C₂-C₆)-Haloalkenyl, (C₂-C₆)-Alkinyl, (C₂-C₆)-Halogenalkinyl, (C₁-C₆)-Alkoxy, (C₁-C₆)-Haloalkoxy, (C₁-C₆)-Alkoxy-(C₁-C₆)-alkyl, (C₁-C₆)-Alkylthio, (C₁-C₆)-Haloalkylthio, (C₁-C₆)-Alkylsulfinyl, (C₁-C₆)-Haloalkylsulfinyl, (C₁-C₆)-Alkylsulfonyl, (C₁-C₆)-Haloalkylsulfonyl, (C₁-C₆)-Alkylcarbonyl, (C₂-C₆)-Alkenylcarbonyl, (C₂-C₆)-Alkinylcarbonyl, (C₁-C₆)-Haloalkylcarbonyl, (C₂-C₆)-Haloalkenylcarbonyl, (C₂-C₆)-Haloalkinylcarbonyl, (C₁-C₆)-Alkoxycarbonyl, (C₃-C₆)-Cycloalkyl, (C₃-C₆)-Cycloalkoxy, (C₃-C₆)-Cycloalkylthio, **(C₃-C₆)-**Cycloalkylsulfinyl, (C₃-C₆)-Cycloalkylsulfonyl, (C₃-C₆)-Halocycloalkyl, **(C₃-C₆)-**Halocycloalkoxy, (C₃-C₆)-Halocycloalkylthio, (C₃-C₆)-Halocycloalkylsulfinyl, (C₃-C₆)-Halocycloalkylsulfonyl, (C₃-C₆)-Cycloalkyl-(C₁-C₆)-Alkyl, (C₁-C₆)-Alkyl-(C₃-C₆)-Cycloalkyl, (C₃-C₆)-Cycloalkylcarbonyl, (C₃-C₆)-Cycloalkyl-(C₁-C₆)-Alkylcarbonyl, (C₁-C₆)-Alkylaminocarbonyl, (C₂-C₁₂)-Dialkylaminocarbonyl, (C₁-C₆)-Alkylaminosulfonyl, (C₂-C₁₂)-Dialkylaminosulfonyl oder Tris[(C₁-C₆)alkyl]silyl, oder 2,3,4,5,6-Pentafluorphenyl,
R³ Wasserstoff, Halogen, Cyano, (C₁-C₆)-Alkyl, (C₁-C₆)-Halogenalkyl, (C₂-C₆)-Alkenyl, (C₂-C₆)-Halogenalkenyl, (C₁-C₆)-Alkoxy, (C₁-C₆)-Halogenalkoxy darstellt, (C₁-C₆)-Alkylthio, (C₁-C₆)-Halogenalkylthio, (C₃-C₆)-Cycloalkyl, **(C₃-C₆)-**Halogencycloalkyl, (C₃-C₆)-Cycloalkoxy oder (C₃-C₆)-Halogencycloalkoxy,
R⁴ und R⁵ unabhängig voneinander Wasserstoff, (C₁-C₆)-Alkyl, (C₁-C₆)-Halogenalkyl, (C₃-C₆)-Cycloalkyl, (C₃-C₆)-Halogencycloalkyl, oder R¹⁰O-(C₁-C₆)-Alkyl, oder
R⁴ und R⁵ bilden zusammen mit dem Kohlenstoffatom, an das sie gebunden sind, einen vollständig gesättigten oder teilweise gesättigten 3- bis 10-gliedrigen monocyclischen oder bicyclischen Ring, der gegebenenfalls durch Heteroatome unterbrochen ist und gegebenenfalls eine weitere Substitution aufweist,
R⁶ Wasserstoff, (C₁-C₆)-Alkyl, (C₂-C₆)-Alkenyl, (C₂-C₆)-Alkinyl bedeutet, Cyano-(C₁-C₆)-alkyl, R¹⁰O-(C₁-C₆)-alkyl, R⁸R⁹N-(C₁-C₆)-alkyl, NR⁸R⁹, (C₃-C₁₀)-Cycloalkyl-(C₁-C₆)-Alkyl, C(=O)R¹⁰, C(=O)OR¹⁰, C(=O)NR⁸R⁹, R¹⁰O(O)C-(C₁-C₆)-Alkyl, R¹⁰(O=)C- (C₁-C₆)-Alkyl, R⁸R⁹N(O)C-(C₁-C₆)-Alkyl, SO₂R¹¹, R¹¹S(O)ₘ-(C₁-C₆)-alkyl, BR¹²R¹³, Heterocyclyl, Heteroaryl, Aryl-(C₁-C₆)-alkyl, Heteroaryl-(C₁-C₆)-alkyl, Heterocyclyl-(C₁-C₆)-alkyl, Tris[(C₁-C₆)-alkyl]silyl, Bis-[(C₁-C₆)-alkyl](aryl)silyl, (C₁-C₆)-Alkyl-[Bis-(Aryl)]silyl, Tris-[(C₁-C₆)-Alkyl]silyl-(C₁-C₆)-Alkyl, Bis-[(C₁-C₆)-Alkyl](Aryl)silyl-(C₁-C₆)-Alkyl, oder (C₁-C₆)-Alkyl-[Bis-(Aryl)]silyl-(C₁-C₆)-Alkyl,
-m 0, 1 oder 2 ist,
n ist 0, 1, 2,
R⁷ Wasserstoff, Halogen, Formyl, Hydroxy, Hydroxycarbonyl, Nitro, Cyano, Amino, Aminocarbonyl, Aminothiocarbonyl, (C₁-C₆)-Alkyl, (C₁-C₆)-Haloalkyl, (C₂-C₆)-Alkenyl, (C₂-C₆)-Haloalkenyl, (C₂-C₆)-Alkinyl, (C₂-C₆)-Haloalkinyl, (C₁-C₆)-Alkoxy, (C₁-C₆)-Haloalkoxy, (C₁-C₆)-Alkylthio, (C₁-C₆)-Halogenalkylthio, (C₁-C₆)-Alkylsulfinyl, (C₁-C₆)-Haloalkylsulfinyl, (C₁-C₆)-Alkylsulfonyl, (C₁-C₆)-Haloalkylsulfonyl, (C₁-C₆)-Alkylcarbonyl, (C₁-C₆)-Haloalkylcarbonyl, (C₂-C₆)-Alkenylcarbonyl, (C₂-C₆)-Halogenalkenylcarbonyl, (C₂-C₆)-Alkinylcarbonyl, (C₂-C₆)-Halogenalkinylcarbonyl, (C₁-C₆)-Alkoxycarbonyl, (C₁-C₆)-Halogenalkoxycarbonyl, (C₃-C₆)-Zycloalkyl, (C₃-C₆)-Halocycloalkyl, **(C₃-C₆)-**Cycloalkoxy, (C₃-C₆)-Halocycloalkoxy, (C₃-C₆)-Cycloalkylthio, **(C₃-C₆)-**Halocycloalkylthio, (C₃-C₆)-Cycloalkylsulfinyl, (C₃-C₆)-Halocycloalkylsulfinyl, (C₃-C₆)-Cycloalkylsulfonyl, (C₃-C₆)-Halocycloalkylsulfonyl, (C₃-C₆)-Cycloalkyl-(C₁-C₆)-alkyl, (C₁-C₆)-alkyl-(C₃-C₆)-cycloalkyl, (C₁-C₆)-Alkoxycarbonyl-(C₁-C₆)-Alkyl, Hydroxycarbonyl-(C₁-C₆)-Alkyl, (C₃-C₆)-Cycloalkylcarbonyl, (C₃-C₆)-Cycloalkyl-(C₁-C₆)-Alkylcarbonyl, (C₁-C₆)-Alkylamino, (C₂-C₁₂)-Dialkylamino, (C₁-C₆)-Alkylaminocarbonyl, (C₂-C₁₂)-Dialkylaminocarbonyl, (C₁-C₆)-Alkylaminosulfonyl, (C₂-C₁₂)-Dialkylaminosulfonyl oder Tris[(C₁-C₆)alkyl]silyl,
R⁸ und R⁹ unabhängig voneinander Wasserstoff, (C₁-C₆)-Alkyl, (C₂-C₆)-Alkenyl, (C₂-C₆)-Alkinyl bedeuten, (C₁-C₆)-Cyanalkyl, (C₁-C₆)-Halogenalkyl, (C₂-C₆)-Halogenalkenyl, (C₃-C₆)-Halogenalkynyl, (C₃-C₆)-Cycloalkyl, (C₃-C₆)-Halocycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Alkoxy-(C₁-C₆)-alkoxy-(C₁-C₆)-alkyl, (C₁-C₆)-Alkoxy-(C₁-C₆)-Alkoxy-(C₁-C₆)-Alkoxy-(C₁-C₆)-Alkyl, (C₁-C₆)-Halogenalkoxy-(C₁-C₆)-alkyl, (C₁- C₆)-Alkylthio-(C₁-C₆)-alkyl, (C₁-C₆)-Haloalkylthio-(C₁-C₆)-Alkyl, (C₁-C₆)-Alkoxy-(C₁-C₆)-Haloalkyl, Aryl, Aryl-(C₁-C₆)-alkyl, Heteroaryl, Heteroaryl-(C₁-C₆)-alkyl, (C₃-C₆)-cycloalkyl-(C₁-C₆)-alkyl, (C₄-C₆)-Cycloalkenyl-(C₁-C₆)-alkyl, COR¹⁰, SO₂R¹¹, Heterocyclyl, (C₁-C₆)-Alkoxycarbonyl, Bis-[(C₁-C₆)-alkyl]aminocarbonyl-(C₁-C₆)-alkyl, (C₁-C₆)-Alkyl-aminocarbonyl-(C₁-C₆)-Alkyl, Aryl-(C₁-C₆)-Alkyl-aminocarbonyl-(C₁-C₆)-Alkyl, (C₁-C₆)-Alkoxycarbonyl-(C₁-C₆)-Alkyl, (C₂-C₆)-Alkenyloxycarbonyl-(C₁-C₆)-Alkyl, (C₂-C₆)-Alkinyloxycarbonyl-(C₁-C₆)-Alkyl, Aryl-(C₁-C₆)-Alkoxycarbonyl-(C₁-C₆)-Alkyl, Heteroaryl-(C₁-C₆)-Alkoxycarbonyl-(C₁-C₆)-Alkyl, Heterocyclyl-(C₁-C₆)-Alkoxycarbonyl-(C₁-C₆)-Alkyl, Aryl-(C₁-C₆)-Alkoxycarbonyl, Heteroaryl-(C₁-C₆)-Alkoxycarbonyl, (C₂-C₆)-Alkenyloxycarbonyl, (C₂-C₆)-Alkinyloxycarbonyl, Heterocyclyl-(C₁-C₆)-Alkyl, oder
-R⁸ und R⁹ zusammen mit dem Kohlenstoffatom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist,
R¹⁰ Wasserstoff, (C₁-C₆)-Alkyl, (C₂-C₆)-Alkenyl, (C₂-C₆)-Alkinyl, (C₁-C₆)-Cyanoalkyl darstellt, (C₁-C₆)-Halogenalkyl, (C₂-C₆)-Halogenalkenyl, (C₃-C₆)-Halogenalkinyl, (C₃-C₆)-Cycloalkyl, (C₃-C₆)-Halocycloalkyl, (C₄-C₆)-Cycloalkenyl, (C₄-C₆)-Halocycloalkenyl, (C₁-C₆)-Alkoxy-(C₁-C₆)-Alkyl, (C₁-C₆)-Haloalkoxy-(C₁-C₆)-Alkyl, (C₁-C₆)-Alkoxy-(C₁-C₆)-Halogenalkyl, (C₁-C₆)-Alkoxy-(C₁-C₆)-Alkoxy-(C₁-C₆)-alkyl, (C₁-C₆)-Alkoxy-(C₁-C₆)-Alkoxy-(C₁-C₆)-Alkoxy-(C₁-C₆)-Alkyl, (C₁-C₆)-Alkoxy-(C₁-C₆)-Alkoxy-(C₁-C₆)-Alkoxy-(C₁-C₆)-Alkoxy-(C₁-C₆)-alkyl, Aryl, Aryl-(C₁-C₆)-alkyl, Aryl-(C₁-C₆)-alkoxy-(C₁-C₆)-alkyl, Aryloxy-(C₁-C₆)-alkyl, Heteroaryl-(C₁-C₆)-alkoxy-(C₁-C₆)-alkyl, Heteroaryloxy-(C₁-C₆)-alkyl, Heteroaryl, Heteroaryl-(C₁-C₆)-alkyl, (C₃-C₆)-cycloalkyl-(C₁-C₆)-alkyl, (C₄-C₆)-Cycloalkenyl-(C₁-C₆)-Alkyl, Bis-[(C₁-C₆)-Alkyl]Aminocarbonyl-(C₁-C₆)-Alkyl, (C₁-C₆)-Alkyl-aminocarbonyl-(C₁-C₆)-Alkyl, Aryl-(C₁-C₆)-Alkyl-aminocarbonyl-(C₁-C₆)-Alkyl, Bis-[(C₁-C₆)-alkyl]amino-(C₂-C₆)-alkyl, (C₁-C₆)-alkyl-amino-(C₂-C₆)-alkyl, Aryl-(C₁-C₆)-alkylamino-(C₂-C₆)-alkyl, R¹¹(O)ₘS-(C₁-C₆)-Alkyl, Hydroxycarbonyl-(C₁-C₆)-Alkyl, Heterocyclyl, Heterocyclyl-(C₁-C₆)-Alkyl, Heterocyclyl-(C₁-C₆)-alkoxy-(C₁-C₆)-alkyl, Heterocyclyloxy-(C₁C₆)-alkyl, Tris-[(C₁-C₆)-alkyl]silyl-(C₁-C₆)-alkyl, Bis-[(C₁-C₆)-alkyl](Aryl)silyl-(C₁-C₆)-alkyl, [(C₁-C₆)-alkyl]-Bis-(Aryl)silyl-(C₁-C₆)-alkyl, (C₁-C₆)-Alkylcarbonyloxy-(C₁-C₆)-Alkyl, (C₃-C₆)-Cycloalkylcarbonyloxy-(C₁-C₆)-Alkyl, Arylcarbonyloxy-(C₁-C₆)-alkyl, Heteroarylcarbonyloxy-(C₁-C₆)-alkyl, Heterocyclylcarbonyloxy-(C₁-C₆)-alkyl, (C₁-C₆)-Alkoxycarbonyl, (C₁-C₆)-Alkoxycarbonyl-(C₁-C₆)-Alkyl, (C₂-C₆)-Alkenyloxycarbonyl-(C₁-C₆)-Alkyl, (C₂-C₆)-Alkinyloxycarbonyl-(C₁-C₆)-Alkyl, Aryl-(C₁-C₆)-Alkoxycarbonyl-(C₁-C₆)-Alkyl, Heteroaryl-(C₁-C₆)-Alkoxycarbonyl-(C₁-C₆)-alkyl, Heterocyclyl-(C₁-C₆)-Alkoxycarbonyl-(C₁-C₆)-alkyl, Hydroxy-(C₁-C₆)-alkyl, Hydroxy-(C₁-C₆)-alkoxy-(C₁-C₆)-alkyl, Hydroxy-(C₁-C₆)-alkoxy-(C₁-C₆)-alkoxy-(C₁-C₆)-alkyl, Hydroxy-(C₁-C₆)-alkoxy-(C₁-C₆)-alkoxy-(C₁-C₆)-alkoxy-(C₁-C₆)-alkyl, (C₁-C₆)-Alkylcarbonyloxy-(C₁-C₆)-alkoxy-(C₁-C₆)-alkyl, (C₁-C₆)-Alkylcarbonyloxy-(C₁-C₆)-Alkoxy-(C₁-C₆)-Alkoxy-(C₁-C₆)-Alkyl, (C₁-C₆)-Alkylcarbonyloxy-(C₁-C₆)-Alkoxy-(C₁-C₆)-Alkoxy-(C₁-C₆)-Alkoxy-(C₁-C₆)-Alkyl, (C₁-C₆)-Alkoxycarbonyl-(C₁-C₆)-Alkoxy-(C₁-C₆)-Alkyl, (C₁-C₆)-Alkoxycarbonyl-(C₁-C₆)-Alkoxy-(C₁-C₆)-Alkoxy-(C₁-C₆)-Alkyl, (C₁-C₆)-Alkoxycarbonyl-(C₁-C₆)-alkoxy-(C₁-C₆)-alkoxy-(C₁-C₆)-alkoxy-(C₁-C₆)-alkyl, Heteroarylcarbonyloxy-(C₁-C₆)-alkoxy-(C₁-C₆)-alkyl, Heteroarylcarbonyloxy-(C₁-C₆)-alkoxy-(C₁-C₆)-alkoxy-(C₁-C₆)-alkyl, Heteroarylcarbonyloxy-(C₁-C₆)-alkoxy-(C₁-C₆)-alkoxy-(C₁-C₆)-alkoxy-(C₁-C₆)-alkyl, Heterocyclylcarbonyloxy-(C₁-C₆)-alkoxy-(C₁-C₆)-alkyl, Heterocyclylcarbonyloxy-(C₁-C₆)-alkoxy-(C₁-C₆)-alkoxy-(C₁-C₆)-alkyl, Heterocyclylcarbonyloxy-(C₁-C₆)-alkoxy-(C₁-C₆)-alkoxy-(C₁-C₆)-alkoxy-(C₁-C₆)-alkyl, Hydroxycarbonyl-(C₁-C₆)-alkoxy-(C₁-C₆)-alkyl, oder Hydroxycarbonyl-(C₁-C₆)-alkoxy-(C₁-C₆)-alkoxy-(C₁-C₆)-alkyl, Hydroxycarbonyl-(C₁-C₆)-alkoxy-(C₁-C₆)-alkoxy-(C₁-C₆)-alkoxy-(C₁-C₆)-alkyl,
R¹¹ Wasserstoff, (C₁-C₆)-Alkyl, (C₂-C₆)-Alkenyl, (C₂-C₆)-Alkinyl, (C₁-C₆)-Cyanoalkyl, (C₁-C₆)-Halogenalkyl, (C₂-C₆)-Halogenalkenyl bedeutet, (C₃-C₆)-Halogenalkynyl, (C₃-C₆)-Cycloalkyl, (C₃-C₆)-Halogencycloalkyl, (C₄-C₆)-Cycloalkenyl, (C₄-C₆)-Halocycloalkenyl, (C₁-C₆)-Alkoxy-(C₁-C₆)-alkyl, (C₁-C₆)-Alkoxy-(C₁-C₆)-Halogenalkyl, Aryl, Aryl-(C₁-C₆)-alkyl, Heteroaryl, Heteroaryl-(C₁-C₆)-alkyl, Heterocyclyl-(C₁-C₆)-alkyl, (C₃-C₆)-cycloalkyl-(C₁-C₆)-alkyl, (C₄-C₁₀)-Cycloalkenyl-(C₁-C₆)-alkyl, Bis-[(C₁-C₆)-alkyl]amino, (C₁-C₆)-alkyl-amino, Aryl- (C₁-C₆)-amino, Aryl-(C₁-C₆)-alkyl-amino, Aryl-[(C₁-C₆)-alkyl]amino; Heteroaryl-(C₁-C₆)-amino, Heteroaryl-(C₁-C₆)-alkyl-amino, Heteroaryl-[(C₁-C₆)-alkyl]amino; Heterocyclyl-(C₁-C₆)-amino, Heterocyclyl-(C₁-C₆)-alkyl-amino, Heterocyclyl-[(C₁-C₆)-alkyl]amino; (C₃-C₆)-Cycloalkyl-amino, (C₃-C₆)-Cycloalkyl-[(C₁-C₆)-alkyl]amino; N-Azetidinyl, N-Pyrrolidinyl, N-Piperidinyl oder N-Morpholinyl,
-R¹² und R¹³ unabhängig voneinander für Wasserstoff, NR⁸R⁹, OR¹⁰, oder
R¹² und R¹³ zusammen mit dem Boratom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist.

4. Die Verbindung der allgemeinen Formel (I), wie in Anspruch 1 beansprucht, und/oder das Salz davon, **dadurch gekennzeichnet, dass**
R¹ für Phenyl, Furyl, Pyrrolyl, Thienyl, Pyridyl, Pyrimidinyl, Pyridazinyl, Pyrazinyl, Thiazolyl, Isothiazolyl, Thiadiazolyl, Oxazolyl, Isoxazolyl, Pyrazolyl, Imidazolyl, Triazolyl, Tetrazolyl, Cyclopentenyl, Cyclohexenyl oder ein Oxabicycloheptanylrest, wobei jeder der zuvor erwähnten 20 Reste unsubstituiert ist oder unabhängig voneinander durch einen oder mehrere Reste, ausgewählt aus der Gruppe R⁷, substituiert ist,
R² Wasserstoff, Fluor, Chlor, Brom, lod, Formyl, Hydroxy, Hydrothio, Hydroxycarbonyl, Aminocarbonyl, Aminothiocarbonyl, (C₁-C₅)-Alkyl, (C₁-C₅)-Halogenalkyl, (C₂-C₅)-Alkenyl darstellt, (C₂-C₅)-Halogenalkenyl, (C₂-C₅)-Alkinyl, (C₂-C₅)-Halogenalkinyl, (C₁-C₅)-Alkoxy, (C₁-C₅)-Halogenalkoxy, (C₁-C₅)-Alkoxy-(C₁-C₅)-alkyl, (C₁-C₅)-Alkylthio, (C₁-C₅)-Haloalkylthio, (C₁-C₅)-Alkylsulfinyl, (C₁-C₅)-Haloalkylsulfinyl, (C₁-C₅)-Alkylsulfonyl, (C₁-C₅)-Haloalkylsulfonyl, (C₁-C₅)-Alkylcarbonyl, (C₂-C₆)-Alkenylcarbonyl, (C₂-C₅)-Alkinylcarbonyl, (C₁-C₅)-Haloalkylcarbonyl, (C₂-C₅)-Haloalkenylcarbonyl, (C₂-C₅)-Haloalkinylcarbonyl, (C₁-C₅)-Alkoxycarbonyl, (C₃-C₆)-Cycloalkyl, (C₃-C₆)-Cycloalkoxy, (C₃-C₆)-Cycloalkylthio, (C₃-C₆)-Cycloalkylsulfinyl, (C₃-C₆)-Cycloalkylsulfonyl, (C₃-C₅)-Halocycloalkyl, (C₃-C₆)-Halocycloalkoxy, (C₃-C₆)-Halocycloalkylthio, (C₃-C₅)-Halocycloalkylsulfinyl, (C₃-C₅)-Halocycloalkylsulfonyl, (C₃-C₆)-Cycloalkyl-(C₁-C₅)-alkyl, (C₁-C₅)-alkyl-(C₃-C₆)-cycloalkyl, (C₃-C₆)-Cycloalkylcarbonyl, (C₃-C₆)-Cycloalkyl-(C₁-C₅)-alkylcarbonyl, (C₁-C₅)-Alkylaminocarbonyl, (C₂-C₁₀)-Dialkylaminocarbonyl, (C₁-C₅)-Alkylaminosulfonyl, (C₂-C₁₀)-Dialkylaminosulfonyl oder Tris[(C₁-C₅)alkyl]silyl, 2,3,4,5,6-Pentafluorphenyl,
R³ Wasserstoff, Fluor, Chlor, Brom, lod, Cyano, (C₁-C₅)-Alkyl, (C₁-C₅)-Halogenalkyl, (C₂-C₅)-Alkenyl, (C₂-C₅)-Halogenalkenyl, (C₁-C₅)-Alkoxy darstellt, (C₁-C₅)-Haloalkoxy, (C₁-C₅)-Alkylthio, (C₁-C₅)-Haloalkylthio, (C₃-C₆)-Cycloalkyl, (C₃-C₅)-Halocycloalkyl, (C₃-C₆)-Cycloalkoxy oder (C₃-C₆)-Halocycloalkoxy,
R⁴ und R⁵ unabhängig voneinander Wasserstoff, (C₁-C₅)-Alkyl, (C₁-C₅)-Halogenalkyl, (C₃-C₆)-Cycloalkyl, (C₃-C₆)-Halogencycloalkyl, oder R¹⁰O-(C₁-C₅)-Alkyl, oder
R⁴ und R⁵ bilden zusammen mit dem Kohlenstoffatom, an das sie gebunden sind, einen vollständig gesättigten oder teilweise gesättigten 3- bis 10-gliedrigen monocyclischen oder bicyclischen Ring, der gegebenenfalls durch Heteroatome unterbrochen ist und gegebenenfalls eine weitere Substitution aufweist,
R⁶ Wasserstoff, (C₁-C₅)-Alkyl, (C₂-C₅)-Alkenyl, (C₂-C₆)-Alkinyl bedeutet, Cyano-(C₁-Cs)-alkyl, R¹⁰O-(C₁-C₅)-alkyl, R⁸R⁹N-(C₁-C₅)-alkyl, NR⁸R⁹, (C₃-C₆)-Cycloalkyl-(C₁-C₅)-Alkyl, C(=O)R¹⁰, C(=O)OR¹⁰, C(=O)NR⁸R⁹, R¹⁰O(O)C-(C₁-C₅)-Alkyl, R¹⁰(O=)C-(C₁-C₅)-Alkyl, R⁸R⁹N(O)C-(C₁-C₅)-Alkyl, SO₂R¹¹, R¹¹S(O)ₘ-(C₁-C₅)-alkyl, BR¹²R¹³, Heterocyclyl, Heteroaryl, Aryl-(C₁-C₅)-alkyl, Heteroaryl-(C₁-C₅)-alkyl, Heterocyclyl-(C₁-C₅)-alkyl, Tris[(C₁-C₅)-alkyl]silyl, Bis-[(C₁-C₅)-alkyl](aryl)silyl, (C₁-C₅)-Alkyl-[Bis-(Aryl)]silyl, Tris-[(C₁-C₅)-Alkyl]silyl-(C₁-C₅)-Alkyl, Bis-[(C₁-C₅)-alkyl](Aryl)silyl-(C₁-C₅)-alkyl, oder (C₁-C₅)-alkyl-[Bis-(aryl)]silyl-(C₁-C₅)-alkyl,
m 0, 1 oder 2 ist,
n ist 0, 1, 2,
R⁷ Wasserstoff, Fluor, Chlor, Brom, lod, Formyl, Hydroxy, Hydroxycarbonyl, Nitro, Cyano, Amino, Aminocarbonyl, Aminothiocarbonyl, (C₁-C₅)-Alkyl, (C₁- C₅)-Halogenalkyl, (C₂-C₅)-Alkenyl, (C₂-C₅)-Halogenalkenyl, (C₂-C₆)-Alkinyl, (C₂-C₅)-Halogenalkynyl, (C₁-C₅)-Alkoxy, (C₁-C₅)-Haloalkoxy, (C₁-C₅)-Alkylthio, (C₁-C₅)-Haloalkylthio, (C₁-C₅)-Alkylsulfinyl, (C₁-C₅)-Haloalkylsulfinyl, (C₁-C₅)-Alkylsulfonyl, (C₁-C₅)-Haloalkylsulfonyl, (C₁-C₅)-Alkylcarbonyl, (C₁-C₅)-Haloalkylcarbonyl, (C₂-C₅)-Alkenylcarbonyl, (C₂-C₅)-Halogenalkenylcarbonyl, (C₂-C₅)-Alkinylcarbonyl, (C₂-C₅)-Halogenalkinylcarbonyl, (C₁-C₅)-Alkoxycarbonyl, (C₁-C₅)-Halogenalkoxycarbonyl, (C₃-C₆)-Cycloalkyl, **(C₃-C₆)-**Halocycloalkyl, (C₃-C₆)-Cycloalkoxy, (C₃-C₆)-Halocycloalkoxy, **(C₃-C₆)-**Cycloalkylthio, (C₃-C₆)-Halocycloalkylthio, (C₃-C₆)-Cycloalkylsulfinyl, **(C₃-C₆)-**Halocycloalkylsulfinyl, (C₃-C₆)-Cycloalkylsulfonyl, **(C₃-C₆)-**Halocycloalkylsulfonyl, (C₃-C₆)-Cycloalkyl-(C₁-C₅)-alkyl, (C₁-C₅)-alkyl-(C₃-C₆)-cycloalkyl, (C₁-C₅)-Alkoxycarbonyl-(C₁-C₅)-Alkyl, Hydroxycarbonyl-(C₁-C₅)-Alkyl, (C₃-C₆)-Cycloalkylcarbonyl, (C₃-C₆)-Cycloalkyl-(C₁-C₅)-Alkylcarbonyl, (C₁-C₅)-Alkylamino, (C₂-C₁₀)-Dialkylamino, (C₁-C₅)-Alkylaminocarbonyl, (C₂-C₁₀)-Dialkylaminocarbonyl, (C₁-C₅)-Alkylaminosulfonyl, (C₂-C₁₀)-Dialkylaminosulfonyl oder Tris[(C₁-C₅)alkyl]silyl,
-R⁸ und R⁹ unabhängig voneinander Wasserstoff, (C₁-C₅)-Alkyl, (C₂-C₅)-Alkenyl, (C₂-C₅)-Alkinyl bedeuten, (C₁-C₅)-Cyanalkyl, (C₁-C₅)-Halogenalkyl, (C₂-C₅)-Halogenalkenyl, (C₃-C₅)-Halogenalkynyl, (C₃-C₆)-Cycloalkyl, (C₃-C₆)-Halocycloalkyl, (C₄-C₆)-Cycloalkenyl, (C₄-C₆)-Halocycloalkenyl, (C₁-C₅)-Alkoxy-(C₁-C₅)-alkyl, (C₁-C₅)-Alkoxy-(C₁-C₅)-alkoxy-(C₁-C₅)-alkyl, (C₁-C₅)-Alkoxy-(C₁-C₅)-Alkoxy-(C₁-C₅)-Alkoxy-(C₁-C₅)-Alkyl, (C₁-C₅)-Haloalkoxy-(C₁-C₅)-alkyl, (C₁-C₅)-Alkylthio-(C₁-C₅)-alkyl, (C₁-C₅)-Haloalkylthio-(C₁-C₅)-Alkyl, (C₁-C₅)-Alkoxy-(C₁-C₅)-Haloalkyl, Aryl, Aryl-(C₁-C₅)-alkyl, Heteroaryl, Heteroaryl-(C₁-C₅)-alkyl, (C₃-C₆)-cycloalkyl-(C₁-C₅)-alkyl, (C₄-C₆)-Cycloalkenyl-(C₁-C₅)-alkyl, COR¹⁰, SO₂R¹¹, Heterocyclyl, (C₁-C₅)-Alkoxycarbonyl, Bis-[(C₁-C₅)-alkyl]aminocarbonyl-(C₁-C₅)-alkyl, (C₁-C₅)-Alkyl-aminocarbonyl-(C₁-C₅)-Alkyl, Aryl-(C₁-C₅)-Alkyl-aminocarbonyl-(C₁-C₅)-Alkyl, (C₁-C₅)-Alkoxycarbonyl-(C₁-C₅)-Alkyl, (C₂-C₅)-Alkenyloxycarbonyl-(C₁-C₅)-Alkyl, (C₂-C₅)-Alkinyloxycarbonyl-(C₁-C₅)-Alkyl, Aryl-(C₁-C₅)-Alkoxycarbonyl-(C₁-C₅)-Alkyl, Heteroaryl-(C₁-C₅)-Alkoxycarbonyl-(C₁-C₅)-Alkyl, Heterocyclyl-(C₁-C₅)-Alkoxycarbonyl-(C₁-C₅)-Alkyl, Aryl-(C₁-C₅)-Alkoxycarbonyl, Heteroaryl-(C₁-C₅)-Alkoxycarbonyl, (C₂-C₅)-Alkenyloxycarbonyl, (C₂-C₅)-Alkinyloxycarbonyl, Heterocyclyl-(C₁-C₅)-Alkyl, oder
R⁸ und R⁹ zusammen mit dem Kohlenstoffatom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist,
R¹⁰ Wasserstoff, (C₁-C₅)-Alkyl, (C₂-C₅)-Alkenyl, (C₂-C₆)-Alkinyl, (C₁-C₅)-Cyanoalkyl darstellt, (C₁-C₅)-Halogenalkyl, (C₂-C₅)-Halogenalkenyl, (C₃-C₅)-Halogenalkinyl, (C₃-C₆)-Cycloalkyl, (C₃-C₆)-Halocycloalkyl, (C₄-C₆)-Cycloalkenyl, **(C₄-C₆)-**Halocycloalkenyl, (C₁-C₅)-Alkoxy-(C₁-C₅)-Alkyl, (C₁-C₅)-Haloalkoxy-(C₁-C₅)-Alkyl, (C₁-C₅)-Alkoxy-(C₁-C₅)-Halogenalkyl, (C₁-C₅)-Alkoxy-(C₁-C₅)-Alkoxy-(C₁-C₅)-alkyl, (C₁-C₅)-Alkoxy-(C₁-C₅)-Alkoxy-(C₁-C₅)-Alkoxy-(C₁-C₅)-Alkyl, (C₁₋C₅)-Alkoxy-(C₁₋C₅)-Alkoxy-(C₁-C₅)-Alkoxy-(C₁-C₅)-Alkoxy-(C₁-C₅)-alkyl, Aryl, Aryl-(C₁-C₅)-alkyl, Aryl-(C₁-C₅)-alkoxy-(C₁-C₅)-alkyl, Aryloxy-(C₁-C₅)-alkyl, Heteroaryl-(C₁-C₅)-alkoxy-(C₁-C₅)-alkyl, Heteroaryloxy-(C₁-C₅)-alkyl, Heteroaryl, Heteroaryl-(C₁-C₅)-alkyl, (C₃-C₆)-cycloalkyl-(C₁-C₅)-alkyl, (C₄-C₆)-Cycloalkenyl-(C₁-C₅)-Alkyl, Bis-[(C₁-C₅)-Alkyl]Aminocarbonyl-(C₁-C₅)-Alkyl, (C₁-C₅)-Alkyl-aminocarbonyl-(C₁-C₅)-Alkyl, Aryl-(C₁-C₅)-Alkyl-aminocarbonyl-(C₁-C₅)-Alkyl, Bis-[(C₁-C₅)-alkyl]amino-(C₂-C₆)-alkyl, (C₁-C₅)-alkyl-amino-(C₂-C₅)-alkyl, aryl-(C₁-C₅)-alkylamino-(C₂-C₅)-alkyl, R¹¹(O)ₘS-(C₁-C₅)-Alkyl, Hydroxycarbonyl-(C₁-C₅)-Alkyl, Heterocyclyl, Heterocyclyl-(C₁-C₅)-Alkyl, Heterocyclyl-(C₁-C₅)-alkoxy-(C₁-C₅)-alkyl, Heterocyclyloxy-(C₁-C₅)-alkyl, Tris-[(C₁-C₅)-alkyl]silyl-(C₁-C₅)-alkyl, Bis-[(C₁-C₅)-alkyl](Aryl)silyl-(C₁-C₅)-alkyl, [(C₁-C₅)-alkyl]-Bis-(Aryl)silyl-(C₁-C₅)-alkyl, (C₁-C₅)-Alkylcarbonyloxy-(C₁-C₅)-Alkyl, (C₃-C₆)-Cycloalkylcarbonyloxy-(C₁-C₅)-Alkyl, Arylcarbonyloxy-(C₁-C₅)-alkyl, Heteroarylcarbonyloxy-(C₁-C₅)-alkyl, Heterocyclylcarbonyloxy-(C₁-C₅)-alkyl, (C₁-C₅)-Alkoxycarbonyl, (C₁-C₅)-Alkoxycarbonyl-(C₁-C₅)-Alkyl, (C₂-C₅)-Alkenyloxycarbonyl-(C₁-C₅)-Alkyl, (C₂-C₅)-Alkinyloxycarbonyl-(C₁-C₅)-Alkyl, Aryl-(C₁-C₅)-Alkoxycarbonyl-(C₁-C₅)-Alkyl, Heteroaryl-(C₁-C₅)-Alkoxycarbonyl-(C₁-C₅)-Alkyl, Heterocyclyl-(C₁-C₅)-Alkoxycarbonyl-(C₁-C₅)-Alkyl, Hydroxy-(C₁-C₅)-Alkyl, Hydroxy-(C₁-C₅)-alkoxy-(C₁-C₅)-alkyl, Hydroxy-(C₁-C₅)-alkoxy-(C₁-C₅)-alkoxy-(C₁-C₅)-alkyl, Hydroxy-(C₁-C₅)-alkoxy-(C₁-C₅)-alkoxy-(C₁-C₅)-alkoxy-(C₁-C₅)-alkyl, (C₁-C₅)-Alkylcarbonyloxy-(C₁-C₅)-alkoxy-(C₁-C₅)-alkyl, (C₁-C₅)-Alkylcarbonyloxy-(C₁-C₅)-Alkoxy-(C₁-C₅)-Alkoxy-(C₁-C₅)-Alkyl, (C₁-C₅)-Alkylcarbonyloxy-(C₁-C₅)-Alkoxy-(C₁-C₅)-Alkoxy-(C₁-C₅)-Alkoxy-(C₁-C₅)-Alkyl, (C₁-C₅)-Alkoxycarbonyl-(C₁-C₅)-Alkoxy-(C₁-C₅)-Alkyl, (C₁-C₅)-Alkoxycarbonyl-(C₁-C₅)-Alkoxy-(C₁-C₅)-Alkoxy-(C₁-C₅)-Alkyl, (C₁-C₅)-Alkoxycarbonyl-(C₁-C₅)-alkoxy-(C₁-C₅)-alkoxy-(C₁-C₅)-alkoxy-(C₁-C₅)-alkyl, Heteroarylcarbonyloxy-(C₁-C₅)-alkoxy-(C₁-C₅)-alkyl, Heteroarylcarbonyloxy-(C₁-C₅)-alkoxy-(C₁-C₅)-alkoxy-(C₁-C₅)-alkyl, Heteroarylcarbonyloxy-(C₁-C₅)-alkoxy-(C₁-C₅)-alkoxy-(C₁-C₅)-alkoxy-(C₁-C₅)-alkyl, Heterocyclylcarbonyloxy-(C₁-C₅)-alkoxy-(C₁-C₅)-alkyl, Heterocyclylcarbonyloxy-(C₁-C₅)-alkoxy-(C₁-C₅)-alkoxy-(C₁-C₅)-alkyl, Heterocyclylcarbonyloxy-(C₁-C₅)-Alkoxy-(C₁-C₅)-Alkoxy-(C₁-C₅)-Alkoxy-(C₁-C₅)-Alkyl, Hydroxycarbonyl-(C₁-C₅)-alkoxy-(C₁-C₅)-alkyl, Hydroxycarbonyl-(C₁-C₅)-alkoxy-(C₁-C₅)-alkoxy-(C₁-C₅)-alkyl, oder Hydroxycarbonyl-(C₁-C₅)-alkoxy-(C₁-C₅)-alkoxy-(C₁-C₅)-alkoxy-(C₁-C₅)-alkyl,
R¹¹ Wasserstoff, (C₁-C₅)-Alkyl, (C₂-C₅)-Alkenyl, (C₂-C₆)-Alkinyl, (C₁-C₅)-Cyanoalkyl, (C₁-C₅)-Halogenalkyl, (C₂-C₅)-Halogenalkenyl darstellt, (C₃-C₅)-Halogenalkynyl, (C₃-C₆)-Cycloalkyl, (C₃-C₆)-Halogencycloalkyl, (C₄-C₆)-Cycloalkenyl, (C₄-C₆)-Halocycloalkenyl, (C₁-C₅)-Alkoxy-(C₁-C₅)-alkyl, (C₁-C₅)-Alkoxy-(C₁-C₅)-Halogenalkyl, Aryl, Aryl-(C₁-C₅)-Alkyl, Heteroaryl, Heteroaryl-(C₁-C₅)-alkyl, Heterocyclyl-(C₁-C₅)-alkyl, (C₃-C₆)-cycloalkyl-(C₁-C₅)-alkyl, (C₄-C₁₀)-Cycloalkenyl-(C₁-C₅)-alkyl, Bis-[(C₁-C₅)-alkyl]amino, (C₁-C₅)-alkyl-amino, Aryl-(C₁-C₅)-amino, Aryl-(C₁-C₅)-alkyl-amino, Aryl-[(C₁-C₅)-alkyl]amino; Heteroaryl-(C₁-C₅)-amino, Heteroaryl-(C₁-C₅)-alkyl-amino, Heteroaryl-[(C₁-C₅)-alkyl]amino; Heterocyclyl-(C₁-C₅)-amino, Heterocyclyl-(C₁-C₅)-alkyl-amino, Heterocyclyl-[(C₁-C₅)-alkyl]amino; (C₃-C₆)-Cycloalkyl-amino, (C₃-C₆)-Cycloalkyl-[(C₁-C₅)-alkyl]amino; N-Azetidinyl, N-Pyrrolidinyl, N-Piperidinyl, N-Morpholinyl.
R¹² und R¹³ unabhängig voneinander Wasserstoff, NR⁸R⁹, OR¹⁰, oder
R¹² und R¹³ zusammen mit dem Boratom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist.

5. Die Verbindung der allgemeinen Formel (I), wie in Anspruch 1 beansprucht, und/oder das Salz davon, **dadurch gekennzeichnet, dass**
R¹ für Phenyl, 2-Fluorphenyl, 3-Fluorphenyl, 4-Fluorphenyl, 2-Chlorphenyl, 3-Chlorphenyl, 4-Chlorphenyl, 2-Bromphenyl, 2-Methylphenyl, 2-Ethylphenyl, 2-Methoxyphenyl, 2-Trifluormethylphenyl, 2,3-Difluorphenyl, 2,4-Difluorphenyl, 2,5-Difluorphenyl, 2,6-Difluorphenyl, 2,3,4-Trifluorphenyl, 2,4,5-Trifluorphenyl, 2,4,6-Trifluorphenyl, 2-Fluor-6-methylphenyl, 2,4-Dichlorphenyl, 2,5-Dichlorphenyl, 2,6-Dichlorphenyl, 3,5-Dichlorphenyl, 2,6-Dimethylphenyl, 2-Chlor-6-methylphenyl, 2-Brom-6-fluorphenyl, 2-Brom-6-chlorphenyl, 2-Brom-6-methylphenyl, 2-Brom-6-methoxyphenyl, 2-Fluor-3-chlorphenyl, 2-Fluor-3-methylphenyl, 2-Chlor-3-fluorphenyl, 2-Fluor-4-chlorphenyl, 2-Fluor-6-chlorphenyl, 2,3-Dimethylphenyl, 2,4-Dimethylphenyl, 2-Methyl-3-fluorphenyl, 2-Thienyl, 3-Fluor-2-thienyl, 3-Chlor-2-thienyl, 3-Brom-2-thienyl, 3-Methyl-2-thienyl, 3-Methoxy-2-thienyl, 3-Thienyl,
2-Fluor-3-thienyl, 2-Chlor-3-thienyl, 2-Brom-3-thienyl, 2-Methyl-3-thienyl,
2-Methoxy-3-thienyl, 4-Fluoro-3-thienyl, 4-Chloro-3-thienyl, 4-Bromo-3-thienyl,
4-Methyl-3-thienyl, 4-Methoxy-3-thienyl, 3,5-Dimethyl-2-thienyl, 5-Bromo-3-methyl-2-thienyl, 2,5-Dimethyl-3-thienyl, 4,5-Dimethyl-3-thienyl, 5-Bromo-2-methyl-3-thienyl,
5-Brom-4-methyl-3-thienyl, 2,4,5-Trimethyl-3-thienyl, 2,5-Dibrom-4-methyl-3-thienyl, Pyridin-2-yl, 3-Fluoropyridin-2-yl, 3-Chloropyridin-2-yl, 3-Bromopyridin-2-yl,
3-Methylpyridin-2-yl, 3-Methoxypyridin-2-yl, 4-Fluorpyridin-2-yl, 5-Fluorpyridin-2-yl, 6-Fluorpyridin-2-yl, Pyridin-3-yl, 2-Methylpyridin-3-yl, 2-Fluorpyridin-3-yl,
2,4-Difluoropyridin-3-yl, Pyridin-4-yl, 3-Fluoropyridin-4-yl, 2-Fluoropyridin-4-yl, 2,3-Difluorpyridin-4-yl, 4-Methylthiazol-5-yl, 4-Methylisothiazol-5-yl, Cyclopenten-1-yl, Cyclohexen-1-yl oder 7-Oxabicyclo[4.1.0]heptan-1-yl,
R² Wasserstoff, Fluor, Chlor, Brom, Jod, Methyl, Ethyl, n-Propyl, Isopropyl, Cyclopropyl, Cyclobutyl, Vinyl, Prop-2-en-2-yl, Ethinyl, Prop-1-in-1-yl, Methoxy, Ethoxy, Methylthio, Ethoxycarbonyl, 2,3,4,5,6-Pentafluorphenyl, Difluormethyl oder Trifluormethyl bedeutet,
n 0, 1 oder 2 ist,
R³ Wasserstoff, Fluor, Chlor, Brom oder Methyl, vorzugsweise Wasserstoff, bedeutet,
R⁴ und R⁵ unabhängig voneinander Wasserstoff, Methyl, Ethyl, Prop-1-yl, Prop-2-yl, But-1-yl, But-2-yl, 1,1-Dimethyleth-1-yl, 2-Methylprop-1-yl, Hydroxymethyl, Hydroxyethyl, Hydroxyprop-1-yl, Methoxymethyl, Methoxyethyl, Methoxyprop-1-yl, Ethoxymethyl, Ethoxyethyl, Ethoxyprop-1-yl, Difluoromethyl, Trifluoromethyl,
2,2-Difluorethyl, 3,3,3-Trifluorethyl, Pentafluorethyl, Cyclopropyl, Cyclobutyl, Cyclopentyl, oder Cyclohexyl, oder
R⁴ und R⁵ zusammen mit dem Kohlenstoffatom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist und
R⁶ Wasserstoff, BH₂, B(NH₂)₂, 4-Amino-1,3,2,4-diazadiboretidin-2-yl, 4-Amino-1,3,2,4-dioxadiboretan-2-yl, Amino, Tris(methyl)silyl, Tris(ethyl)silyl darstellt, Tris(prop-1-yl)silyl, Tris(prop-2-yl)silyl, (1,1-Dimethyleth-1-yl)(dimethyl)silyl, Cyanomethyl, 2-Cyanoeth-1-yl, 3-Cyanoprop-1-yl, 2-Cyanoprop-1-yl, Methoxymethyl, 2-Methoxyeth-1-yl,
2-Methoxyprop-1-yl, 3-Methoxyprop-1-yl, Ethoxymethyl, 2-(Ethoxy)eth-1-yl,
2-(Ethoxy)prop-1-yl, 3-Ethoxyprop-1-yl, 4-Methoxybut-1-yl, 2-Phenoxyeth-1-yl,
2-benzyloxyeth-1-yl, 2-(Methylcarbonyloxy)eth-1-yl, 2-(Ethylcarbonyloxy)eth-1-yl,
2-(Iso-Propylcarbonyloxy)eth-1-yl, 2-(tert.-Butylcarbonyloxy)eth-1-yl,
2-(Phenylcarbonyloxy)eth-1-yl, 2-(2-Trifluormethylphenylcarbonyl)oxyeth-1-yl,
2-(2-Chlorphenylcarbonyl)oxyeth-1-yl, 2-(4-Trifluormethylpyridin-3-ylcarbonyl)oxyeth-1-yl, 2-(Methylsulfonyloxy)eth-1-yl, 3-(Methylcarbonyloxy)prop-1-yl, 3-(Ethylcarbonyloxy)prop-1-yl, 3-(Isopropylcarbonyloxy)prop-1-yl, 3-(tert-Butylcarbonyloxy)prop-1-yl, 2-Trifluormethoxyeth-1-yl, Methyl, Ethyl, prop-1-yl,
1-Methylethyl, But-1-yl, 1-Methylprop-1-yl, 2-Methylprop-1-yl, 1,1-Dimethylethyl, Pent-1-yl, 1-Methylbut-1-yl, 2-Methylbut-1-yl, 3-Methylbut-1-yl, 1,1-Dimethylprop-1-yl, 1,2-Dimethylprop-1-yl, 2,2-Dimethylprop-1-yl, 1-Ethylprop-1-yl, Prop-2-en-1-yl, but-2-en-1-yl, but-3-en-1-yl, pent-2-en-1-yl, pent-3-en-1-yl, pent-4-en-1-yl, 1-Methylprop-2-en-1-yl, 2-Methylprop-2-en-1-yl, 1-Methylbut-2-en-1-yl, 2-Methylbut-2-en-1-yl, 3-Methylbut-2-en-1-yl, 1-Methylbut-3-en-1-yl, 2-Methylbut-3-en-1-yl, 3-Methylbut-3-en-1-yl, 1,1-Dimethylprop-2-en-1-yl, 1,2-Dimethylprop-2-en-1-yl, Prop-2-yn-1-yl, But-2-yn-1-yl, But-3-yn-1-yl, 1-Methyl-prop-2-yn-1-yl, Pent-2-yn-1-yl, Pent-3-yn-1-yl, Pent-4-yn-1-yl,
1-Methylbuty-2-n-1-yl, 1-Methylbut-3-yn-1-yl, 2-Methylbut-3-yn-1-yl, Cyclobutyl, Cyclopentyl, Cyclohexyl, Cyclopropylmethyl, Cyclobutylmethyl, Cyclopentylmethyl, Cyclohexylmethyl, 2-(Dimethylamino)eth-1-yl, 2-(Diethylamino)eth-1-yl, 3-(Dimethylamino)prop-1-yl, 3-(Diethylamino)prop-1-yl, 2-(N-Pyrrolidin-1-yl)eth-1-yl,
2-(N-Piperidin-1-yl)eth-1-yl, Tetrahydrofuran-2-ylmethyl, Tetrahydrofuran-3-ylmethyl, Tetrahydropyran-2-ylmethyl, Tetrahydropyran-3-ylmethyl, Tetrahydropyran-4-ylmethyl, Oxetan-3-ylmethyl, Methoxyethoxyeth-1-yl, Ethoxyethoxyeth-1-yl, Benzyl, 2-Phenyleth-1-yl, (2-Fluorphenyl)methyl, (3-Fluorphenyl)methyl, (4-Fluorphenyl)methyl, (2,6-Difluorphenyl)methyl, (2,5- Difluorphenyl)methyl, (2,4-Difluorphenyl)methyl, (2,3-Difluorphenyl)methyl, (2-Fluor-6-chlorphenyl)methyl, (2-Chlorphenyl)methyl,
(3-Chlorphenyl)methyl, (4-Chlorphenyl)methyl, (2,6-Dichlorphenyl)methyl, (2,5-Dichlorphenyl)methyl, (2,4-Dichlorphenyl)methyl, (2,3-Dichlorphenyl)methyl,
(2-Bromphenyl)methyl, (3-Bromphenyl)methyl, (4-Bromphenyl)methyl,
(2-Methylphenyl)methyl, (2-Trifluormethylphenyl)methyl, (2-Methoxyphenyl)methyl, (2-Nitrophenyl)methyl, (3-Methylphenyl)methyl, (3-Trifluormethylphenyl)methyl,
(3-Methoxyphenyl)methyl, (3-Nitrophenyl)methyl, (4-Methylphenyl)methyl,
(4-Trifluormethylphenyl)methyl, (4-Methoxyphenyl)methyl, (4-Nitrophenyl)methyl, Pyridin-2-ylmethyl, Pyridin-3-ylmethyl, Pyridin-4-ylmethyl, (6-Fluoropyridin-2-yl)methyl, (6-Chlor-4-trifluormethylpyridin-2-yl)methyl, (4,6-Dichlorpyridin-2-yl)methyl, (4-Fluorpyridin-3-yl)methyl, (4-Chlorpyridin-3-yl)methyl, Thiophen-2-ylmethyl, Methoxycarbonyl, Ethoxycarbonyl, 1,1-Dimethyleth-1-yloxycarbonyl, Benzyloxycarbonyl, (Prop-2-en-1-yl)oxycarbonyl, Methylaminocarbonyl, Ethylaminocarbonyl, Prop-1-ylaminocarbonyl, Prop-2-ylaminocarbonyl, Cyclopentylaminocarbonyl, Cyclohexylaminocarbonyl, Phenylaminocarbonyl, Dimethylamincarbonyl, Diethylaminocarbonyl, Ethyl(methyl)aminocarbonyl,
(2-Fluorphenyl)aminocarbonyl, (3-Fluorphenyl)aminocarbonyl,
(4-Fluorphenyl)aminocarbonyl, (2,6-Difluorphenyl)aminocarbonyl, (2,5-Difluorphenyl)aminocarbonyl, (2,4-Difluorphenyl)aminocarbonyl, (2,3-Difluorphenyl)aminocarbonyl, (2-Fluoro-6-chlorophenyl)aminocarbonyl,
(2-Chlorphenyl)aminocarbonyl, (3-Chlorphenyl)aminocarbonyl,
(4-Chlorphenyl)-Aminocarbonyl, (2,6-Dichlorphenyl)-Aminocarbonyl, (2,5-Dichlorphenyl)-Aminocarbonyl, (2,4-Dichlorphenyl)-Aminocarbonyl, (2,3-Dichlorphenyl)-Aminocarbonyl, (2-Bromphenyl)-Aminocarbonyl,
(3-Bromphenyl)aminocarbonyl, (4-Bromphenyl)aminocarbonyl, Methoxycarbonylmethyl, 2-(Methoxycarbonyl)eth-1-yl, 1-(Methoxycarbonyl)eth-1-yl, 3-(Methoxycarbonyl)prop-1-yl, 2-(Methoxycarbonyl)prop-1-yl, 4-(Methoxycarbonyl)but-1-yl, 5-(Methoxycarbonyl)pent-1-yl, Ethoxycarbonylmethyl, 2-(Ethoxycarbonyl)eth-1-yl, 1-(Ethoxycarbonyl)eth-1-yl, 3-(Ethoxycarbonyl)prop-1-yl, 2-(Ethoxycarbonyl)prop-1-yl, 4-(Ethoxycarbonyl)but-1-yl, 5-(Ethoxycarbonyl)pent-1-yl, Benzyloxycarbonylmethyl, 2-(Benzyloxycarbonyl)eth-1-yl, Hydroxycarbonylmethyl, 2-(Hydroxycarbonyl)eth-1-yl, 1-(Hydroxycarbonyl)eth-1-yl, 3-(Hydroxycarbonyl)prop-1-yl, 2-(Hydroxycarbonyl)prop-1-yl, 4-(Hydroxycarbonyl)but-1-yl, 5-(Hydroxycarbonyl)pent-1-yl, Aminocarbonylmethyl, Methylaminocarbonylmethyl, Ethylaminocarbonylmethyl, Prop-1-ylaminocarbonylmethyl, Prop-2-ylaminobarbonylmethyl, Dimethylaminocarbonylmethyl, Diethylaminocarbonylmethyl, Benzylaminocarbonylmethyl, Cyclopropylaminocarbonylmethyl, Cyclobutylaminocarbonylmethyl, Cyclopentylaminocarbonylmethyl, Cyclohexylaminocarbonylmethyl, 2-(Aminocarbonyl)eth-1-yl,
2-(Methylaminocarbonyl)eth-1-yl, 2-(Ethylaminocarbonyl)eth-1-yl, 2-(Prop-1-ylaminocarbonyl)eth-1-yl, 2-(Prop-2-ylaminobarbonyl)eth-1-yl,
2-(Dimethylaminocarbonyl)-Eth-1-yl, 2-(Diethylaminocarbonyl)-Eth-1-yl,
2-(Benzylaminocarbonyl)-Eth-1-yl, 2-(Cyclopropylaminocarbonyl)-Eth-1-yl,
2-(Cyclobutylaminocarbonyl)-Eth-1-yl, 2-(Cyclopentylaminocarbonyl)-Eth-1-yl,
2-(Cyclohexylaminocarbonyl)-Eth-1-yl, 2-(Methylsulfanyl)-Eth-1-yl,
2-(Methylsulfinyl)eth-1-yl, 2-(Methylsulfonyl)eth-1-yl, 2-(Ethylsulfanyl)eth-1-yl,
2-(Ethylsulfinyl)eth-1-yl, 2-(Ethylsulfonyl)eth-1-yl, 2-(Methylsulfanyl)prop-1-yl,
2-(Methylsulfinyl)prop-1-yl, 2-(Methylsulfonyl)prop-1-yl, Methylsulfonyl, Ethylsulfonyl, Prop-1-ylsulfonyl, 1-Methyleth-1-ylsulfonyl, But-1-ylsulfonyl,
1-Methylprop-1-ylsulfonyl, 2-Methylprop-1-ylsulfonyl, 1,1-Dimethylethylsulfonyl, Pent-1-ylsulfonyl, 1-Methylbut-1-ylsulfonyl, 2-Methylbut-1-ylsulfonyl, 3-Methylbut-1-ylsulfonyl, 1,1-Dimethylprop-1-ylsulfonyl, 1,2-Dimethylprop-1-ylsulfonyl,
2,2-Dimethylprop-1-ylsulfonyl, 1-Ethylprop-1-ylsulfonyl, Cyanomethylsulfonyl,
3-Cyanoprop-1-ylsulfonyl, 1-Methylcycloprop-1-ylsulfonyl, Tetrahydrofuran-3-ylsulfonyl, Methoxycarbonylmethylsulfonyl, Ethoxycarbonylmethylsulfonyl, Cyclopropylmethylsulfonyl, Cyclobutylmethylsulfonyl, Cyclopentylmethylsulfonyl, Cyclohexylmethylsulfonyl, Cyclopropylsulfonyl, Cyclobutylsulfonyl, Cyclopentylsulfonyl, Cyclohexylsulfonyl, prop-2-en-1-ylsulfonyl, prop-2-en-2-ylsulfonyl, but-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-Methylprop-2-en-1-ylsulfonyl, 2-Methylprop-2-en-1-ylsulfonyl, Trifluormethylsulfonyl, Benzylsulfonyl, 2-Phenyleth-1-ylsulfonyl, (2-Fluorphenyl)-methylsulfonyl, (3-Fluorphenyl)methylsulfonyl, (4-Fluorphenyl)-methylsulfonyl, (2,6-Difluorphenyl)methylsulfonyl, (2,5-Difluorphenyl)-methylsulfonyl, (2,4-Difluorphenyl)methylsulfonyl, (2,3-Difluorphenyl)-methylsulfonyl, (2-Fluor-6-chlorphenyl)methylsulfonyl, (2-Chlorphenyl)-methylsulfonyl, (3-Chlorphenyl)methylsulfonyl, (4-Chlorphenyl)methylsulfonyl, (2,6-Dichlorphenyl)methylsulfonyl, (2,5-Dichlorphenyl)methylsulfonyl, (2,4-Dichlorphenyl)methylsulfonyl, (2,3-Dichlorphenyl)methylsulfonyl, (2-Bromphenyl)methylsulfonyl, (3-Bromphenyl)methylsulfonyl, (4-Bromphenyl)methylsulfonyl, (2-Methylphenyl)methylsulfonyl, (2-TrifluorMethylphenyl)methylsulfonyl, (2-Methoxyphenyl)methylsulfonyl,
(2-Nitrophenyl)methylsulfonyl, (3-Methylphenyl)methylsulfonyl,
(3-Trifluormethylphenyl)-methylsulfonyl, (3-Methoxyphenyl)-methylsulfonyl,
(3-Nitrophenyl)methylsulfonyl, (4-Methylphenyl)methylsulfonyl,
(4-Trifluormethylphenyl)-methylsulfonyl, (4-Methoxyphenyl)-methylsulfonyl,
(4-Nitrophenyl)methylsulfonyl, Pyridin-2-ylmethylsulfonyl, Pyridin-3-ylmethylsulfonyl, Pyridin-4-ylmethylsulfonyl, (6-Fluoropyridin-2-yl)methylsulfonyl, (6-Chlor-4-trifluormethylpyridin-2-yl)methylsulfonyl, (4,6-Dichlorpyridin-2-yl)methylsulfonyl, (4-Fluorpyridin-3-yl)methylsulfonyl, (4-Chlorpyridin-3-yl)methylsulfonyl, Thiophen-2-ylmethylsulfonyl, Phenylsulfonyl, (2-Fluorphenyl)sulfonyl, (3-Fluorphenyl)sulfonyl, (4-Fluorphenyl)sulfonyl, (2,6-Difluorphenyl)sulfonyl, (2,5-Difluorphenyl)sulfonyl, (2,4-Difluorphenyl)sulfonyl, (2,3-Difluorphenyl)sulfonyl, (2-Fluor-6-chlorphenyl)sulfonyl, (2-Chlorphenyl)sulfonyl, (3-Chlorphenyl)sulfonyl,
(4-Chlorphenyl)sulfonyl, (2,6-Dichlorphenyl)sulfonyl, (2,5-Dichlorphenyl)sulfonyl, (2,4-Dichlorphenyl)sulfonyl, (2,3-Dichlorphenyl)sulfonyl, (2-Bromphenyl)sulfonyl, (3-Bromphenyl)sulfonyl, (4-Bromphenyl)sulfonyl, (2-Methylphenyl)sulfonyl,
(2-Trifluormethylphenyl)sulfonyl, (2-Methoxyphenyl)sulfonyl, (2-Nitrophenyl)sulfonyl, (3-Methylphenyl)sulfonyl, (3-Trifluormethylphenyl)sulfonyl, (3-Methoxyphenyl)-sulfonyl, (3-Nitrophenyl)sulfonyl, (4-Methylphenyl)sulfonyl, (4-Trifluormethylphenyl)-sulfonyl, (4-Methoxyphenyl)sulfonyl, (4-Nitrophenyl)sulfonyl, Pyridin-2-ylsulfonyl, Pyridin-3-ylsulfonyl, Pyridin-4-ylsulfonyl, (6-Fluorpyridin-2-yl)sulfonyl, (6-Chlor-4-trifluorsulfonylpyridin-2-yl)sulfonyl, (4,6-Dichlorpyridin-2-yl)sulfonyl, (4-Fluor-pyridin-3-yl)sulfonyl, (4-Chlorpyridin-3-yl)sulfonyl, Thiophen-2-ylsulfonyl, Methoxymethylsulfonyl, 2-(Methoxy)eth-1-ylsulfonyl, 2-(Methoxy)eth-1-ylsulfonyl, Methylaminosulfonyl, Ethylaminosulfonyl, Dimethylaminosulfonyl, Diethylamino-sulfonyl, Methyl(ethyl)aminosulfonyl, Azetidin-1-ylsulfonyl, Pyrrolidin-1-ylsulfonyl, Piperidin-1-ylsulfonyl, N-Morpholinylsulfonyl, 2-(Methoxycarbonyl)eth-1-ylsulfonyl, 2-(Ethoxycarbonyl)eth-1-ylsulfonyl, 3-(Methoxycarbonyl)prop-1-ylsulfonyl,
3-(Ethoxycarbonyl)prop-1-ylsulfonyl, 1-(Methoxycarbonyl)eth-1-ylsulfonyl, 2-Ethoxyeth-1-ylsulfonyl, 1-(Prop-2-en-1-yl)cycloprop-1-ylsulfonyl, 3,3,3-Trifluorethylsulfonyl, Tetrahydropyran-4-ylsulfonyl, Tetrahydrofuran-3-ylmethylsulfonyl, Formyl, Methylcarbonyl, Ethylcarbonyl, Prop-1-ylcarbonyl,
1-Methyleth-1-ylcarbonyl, But-1-ylcarbonyl, 1-Methylprop-1-ylcarbonyl, 2-Methylprop-1-ylcarbonyl, 1,1-Dimethylethylcarbonyl, Pent-1-ylcarbonyl, 1-Methylbut-1-ylcarbonyl, 2-Methylbut-1-ylcarbonyl, 3-Methylbut-1-ylcarbonyl, 1,1-Dimethylprop-1-ylcarbonyl, 1,2-Dimethylprop-1-ylcarbonyl, 2,2-Dimethylprop-1-ylcarbonyl, 1-Ethylprop-1-ylcarbonyl, Cyclopropylmethylcarbonyl, Cyclobutylmethylcarbonyl, Cyclopentylmethylcarbonyl, Cyclohexylmethylcarbonyl, Cyclopropylcarbonyl, Cyclobutylcarbonyl, Cyclopentylcarbonyl, Cyclohexylcarbonyl, Methoxymethylcarbonyl, 2-Methoxyeth-1-ylcarbonyl, 2-Ethoxyeth-1-ylcarbonyl, 3-Methoxyprop-1-ylcarbonyl,
3-Ethoxyprop-1-ylcarbonyl, Methoxycarbonylmethylcarbonyl, 2-(Methoxycarbonyl)eth-1-ylcarbonyl, 3-(Methoxycarbonyl)prop-1-ylcarbonyl, 4-(Methoxycarbonyl)but-1-ylcarbonyl, Ethoxycarbonylmethylcarbonyl, 2-(Ethoxycarbonyl)eth-1-ylcarbonyl,
3-(Ethoxycarbonyl)prop-1-ylcarbonyl, 4-(Ethoxycarbonyl)but-1-ylcarbonyl, Benzylcarbonyl, 2-Phenyleth-1-ylcarbonyl, (2-Fluorophenyl)methylcarbonyl,
(3-Fluorphenyl)methylcarbonyl, (4-Fluorphenyl)methylcarbonyl,
(2,6-Difluorphenyl)-methylcarbonyl, (2,5-Difluorphenyl)-methylcarbonyl,
(2,4-Difluorphenyl)methylcarbonyl, (2,3-Difluorphenyl)methylcarbonyl, (2-Fluor-6-chlorphenyl)methylcarbonyl, (2-Chlorphenyl)methylcarbonyl,
(3-Chlorphenyl)methylcarbonyl, (4-Chlorphenyl)methylcarbonyl,
(2,6-Dichlorphenyl)-methylcarbonyl, (2,5-Dichlorphenyl)-methylcarbonyl,
(2,4-Dichlorphenyl)methylcarbonyl, (2,3-Dichlorphenyl)methylcarbonyl,
(2-Bromphenyl)methylcarbonyl, (3-Bromphenyl)methylcarbonyl,
(4-Bromphenyl)methylcarbonyl, (2-Methylphenyl)methylcarbonyl,
(2-Trifluormethylphenyl)-methylcarbonyl, (2-Methoxyphenyl)-methylcarbonyl,
(2-Nitrophenyl)methylcarbonyl, (3-Methylphenyl)methylcarbonyl,
(3-Trifluormethylphenyl)-methylcarbonyl, (3-Methoxyphenyl)-methylcarbonyl,
(3-Nitrophenyl)methylcarbonyl, (4-Methylphenyl)methylcarbonyl,
(4-Trifluormethylphenyl)-methylcarbonyl, (4-Methoxyphenyl)-methylcarbonyl,
(4-Nitrophenyl)methylcarbonyl, Pyridin-2-ylmethylcarbonyl, Pyridin-3-ylmethylcarbonyl, Pyridin-4-ylmethylcarbonyl, (6-Fluoropyridin-2-yl)methylcarbonyl, (6-Chlor-4-trifluormethylpyridin-2-yl)methylcarbonyl, (4,6-Dichlorpyridin-2-yl)methylcarbonyl, (4-Fluorpyridin-3-yl)methylcarbonyl, (4-Chlorpyridin-3-yl)methylcarbonyl, Thiophen-2-ylmethylcarbonyl, Phenylcarbonyl,
(2-Fluorophenyl)carbonyl, (3-Fluorophenyl)carbonyl, (4-Fluorophenyl)carbonyl,
(2,6-Difluorphenyl)carbonyl, (2,5-Difluorphenyl)carbonyl,
(2,4-Difluorphenyl)carbonyl, (2,3-Difluorphenyl)carbonyl, (2-Fluor-6-chlorphenyl)carbonyl, (2-Chlorphenyl)carbonyl, (3-Chlorphenyl)carbonyl,
(4-Chlorphenyl)carbonyl, (2,6-Dichlorphenyl)carbonyl, (2,5-Dichlorphenyl)carbonyl, (2,4-Dichlorphenyl)carbonyl, (2,3-Dichlorphenyl)carbonyl, (2-Bromphenyl)carbonyl, (3-Bromphenyl)carbonyl, (4-Bromphenyl)carbonyl, (2-Methylphenyl)carbonyl,
(2-Trifluormethylphenyl)-carbonyl, (2-Methoxyphenyl)-carbonyl, (2-Nitrophenyl)-carbonyl, (3-Methylphenyl)-carbonyl, (3-Trifluormethylphenyl)-carbonyl,
(3-Methoxyphenyl)carbonyl, (3-Nitrophenyl)carbonyl, (4-Methylphenyl)carbonyl,
(4-Trifluormethylphenyl)carbonyl, (4-Methoxyphenyl)carbonyl, (4-Nitrophenyl)-carbonyl, (2-Chlor-4-methylsulfonylphenyl)carbonyl, Pyridin-2-ylcarbonyl, Pyridin-3-ylcarbonyl, Pyridin-4-ylcarbonyl, (6-Fluoropyridin-2-yl)carbonyl, (6-Chlor-4-trifluorcarbonylpyridin-2-yl)carbonyl, (4,6-Dichlorpyridin-2-yl)carbonyl,
(4-Fluorpyridin-3-yl)carbonyl, (4-Chlorpyridin-3-yl)carbonyl, Thiophen-2-ylcarbonyl, 1,3-Oxazol-2-ylcarbonyl, 1,2-Oxazol-3-ylcarbonyl, 3-Chlor-5-methyl-4,5-dihydro-1,2-oxazol-5-ylcarbonyl, 3-Brom-5-methyl-4,5-dihydro-1,2-oxazol-5-ylcarbonyl, 3-Chlor-5-methoxymethyl-4,5-Dihydro-1,2-oxazol-5-ylcarbonyl, 3-Chlor-5-ethyl-4,5-dihydro-1,2-oxazol-5-ylcarbonyl, 3-Chlor-3a,4,5,6-tetrahydro-6aH-cyclopenta[d][1,2]oxazol-6a-ylcarbonyl, (4-Trifluormethylpyridin-3-yl)carbonyl, 3,5-Dimethyl-4,5-dihydro-1,2-oxazol-5-ylcarbonyl, 5-Methyl-3-trifluormethyl-4,5-dihydro-1,2-oxazol-5-ylcarbonyl, 3-Chlor-5-hydroxymethyl-4,5-dihydro-1,2-oxazol-5-ylcarbonyl, 3-Chlor-5-tert-butyldimethylsilyloxymethyl-4,5-dihydro-1,2-oxazol-5-ylcarbonyl, 3-Chlor-5-(1-hydroxyeth-1-yl)-4,5-dihydro-1,2-oxazol-5-ylcarbonyl, 3-Chlor-5-(1-tert-butyldimethylsilyloxyeth-1-yl-4,5-dihydro-1,2-oxazol-5-ylcarbonyl, Methoxyethoxymethylcarbonyl, Ethoxyethoxymethylcarbonyl, Hydroxyethoxymethylcarbonyl, Methoxyethoxyethoxymethylcarbonyl, Ethoxyethoxyethoxymethylcarbonyl, Hydroxyethoxyethoxymethylcarbonyl,
4-Trifluormethylpyridin-3-yl-carbonyloxyethoxyethoxymethylcarbonyl, Methoxyethoxyethoxyethoxymethylcarbonyl, Ethoxyethoxyethoxyethoxymethylcarbonyl, Hydroxyethoxyethoxyethoxymethylcarbonyl, Methoxycarbonylmethoxyethoxymethylcarbonyl, Hydroxycarbonylmethoxyethoxymethylcarbonyl, Methoxycarbonylmethoxyethoxyethoxymethylcarbonyl, Hydroxycarbonylmethoxyethoxyethoxymethylcarbonyl, Methoxycarbonylmethoxyethoxyethoxymethylcarbonyl oder Hydroxycarbonylmethoxyethoxyethoxyethoxymethylcarbonyl,

6. Die Verbindung der allgemeinen Formel (I), wie in Anspruch 1 beansprucht, und/oder das Salz davon, **dadurch gekennzeichnet, dass**
R¹ für Phenyl, 2-Fluorphenyl, 3-Fluorphenyl, 4-Fluorphenyl, 2-Chlorphenyl,
3-Chlorphenyl, 4-Chlorphenyl, 2-Bromphenyl, 2-Methylphenyl, 2-Ethylphenyl,
2-Methoxyphenyl, 2-Trifluormethylphenyl, 2,3-Difluorphenyl, 2,4-Difluorphenyl,
2,5-Difluorphenyl, 2,6-Difluorphenyl, 2,3,4-Trifluorphenyl, 2,4,5-Trifluorphenyl, 2,4,6-Trifluorphenyl, 2-Fluor-6-methylphenyl, 2-Fluor-3-methylphenyl 2,4-Dichlorphenyl, 2,5-Dichlorphenyl, 2,6-Dichlorphenyl, 3,5-Dichlorphenyl,
2,6-Dimethylphenyl, 2-Chlor-6-methylphenyl, 2-Brom-6-fluorphenyl, 2-Brom-6-chlorphenyl, 2-Brom-6-methylphenyl, 2-Brom-6-methoxyphenyl, 2-Fluor-3-chlorphenyl, 2-Chlor-3-fluorphenyl, 2-Fluor-4-chlorphenyl, 2-Fluor-6-chlorphenyl, 2,3-Dimethylphenyl, 2,4-Dimethylphenyl, 2-Methyl-3-fluorphenyl,
2-Thienyl, 3-Fluor-2-Thienyl, 3-Chlor-2-Thienyl, 3-Brom-2-Thienyl, 3-Methyl-2-Thienyl, 3-Methoxy-2-Thienyl, 3-Thienyl, 2-Fluor-3-Thienyl, 2-Chlor-3-Thienyl, 2-Brom-3-Thienyl, 2-Methyl-3-thienyl, 2-Methoxy-3-thienyl, 4-Fluoro-3-thienyl, 4-Chloro-3-thienyl, 4-Bromo-3-thienyl, 4-Methyl-3-thienyl, 4-Methoxy-3-thienyl, 3,5-Dimethyl-2-thienyl,
5-Brom-3-methyl-2-thienyl, 2,5-Dimethyl-3-thienyl, 4,5-Dimethyl-3-thienyl, 5-Brom-2-methyl-3-thienyl, 5-Brom-4-methyl-3-thienyl, 2,4,5-Trimethyl-3-thienyl,
2,5-Dibrom-4-methyl-3-thienyl, Pyridin-2-yl, 3-Fluorpyridin-2-yl, 3-Chlorpyridin-2-yl, 3-Brompyridin-2-yl, 3-Methylpyridin-2-yl, 3-Methoxypyridin-2-yl, 4-Fluorpyridin-2-yl, 5-Fluorpyridin-2-yl, 6-Fluorpyridin-2-yl, Pyridin-3-yl, 2-Methylpyridin-3-yl,
2-Fluorpyridin-3-yl, 2,4-Difluorpyridin-3-yl, Pyridin-4-yl, 3-Fluorpyridin-4-yl,
2-Fluorpyridin-4-yl, 2,3-Difluorpyridin-4-yl, 4-Methylthiazol-5-yl, 4-Methylisothiazol-5-yl, Cyclopenten-1-yl, Cyclohexen-1-yl oder 7-Oxabicyclo[4.1.0]heptan-1-yl,
R² Wasserstoff, Fluor, Chlor, Brom, Jod, Methyl, Ethyl, n-Propyl, Isopropyl, Cyclopropyl, Cyclobutyl, Vinyl, Prop-2-en-2-yl, Ethinyl, Prop-1-in-1-yl, Methoxy, Ethoxy, Methylthio, Ethoxycarbonyl, 2,3,4,5,6-Pentafluorphenyl, Difluormethyl oder Trifluormethyl bedeutet,
R³ Wasserstoff bedeutet,
n ist 0, 1, 2,
R⁴ und R⁵ unabhängig voneinander Wasserstoff, Methyl, Ethyl, Prop-1-yl, Prop-2-yl, But-1-yl, But-2-yl, 1,1-Dimethyleth-1-yl, Methoxymethyl, Methoxyethyl, Methoxyprop-1-yl, Ethoxymethyl, Ethoxyethyl, Difluormethyl, Trifluormethyl, Cyclopropyl, Cyclobutyl, oder
R⁴ und R⁵ zusammen mit dem Kohlenstoffatom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist, und
R⁶ Wasserstoff, BH₂, B(NH₂)₂, 4-Amino-1,3,2,4-diazadiboretidin-2-yl, 4-Amino-1,3,2,4-dioxadiboretan-2-yl, Amino, Tris(methyl)silyl, Tris(ethyl)silyl, Tris(prop-1-yl)silyl, Tris(prop-2-yl)silyl, (1,1-Dimethyleth-1-yl)(dimethyl)silyl darstellt, Methyl, Ethyl, Prop-1-yl, Methoxycarbonylmethyl, 2-(Methoxycarbonyl)eth-1-yl, 1-(Methoxycarbonyl)eth-1-yl, 3-(Methoxycarbonyl)prop-1-yl, 2-(Methoxycarbonyl)prop-1-yl, 4-(Methoxycarbonyl)but-1-yl, 5-(Methoxycarbonyl)pent-1-yl, Ethoxycarbonylmethyl,
2-(Ethoxycarbonyl)eth-1-yl, 1-(Ethoxycarbonyl)eth-1-yl, 3-(Ethoxycarbonyl)prop-1-yl, 2-(Ethoxycarbonyl)prop-1-yl, 4-(Ethoxycarbonyl)but-1-yl, 5-(Ethoxycarbonyl)pent-1-yl, Benzyloxycarbonylmethyl, 2-(Benzyloxycarbonyl)eth-1-yl, Hydroxycarbonylmethyl,
2-(Hydroxycarbonyl)eth-1-yl, 1-(Hydroxycarbonyl)eth-1-yl, 3-(Hydroxycarbonyl)prop-1-yl, 2-(Hydroxycarbonyl)prop-1-yl, 4-(Hydroxycarbonyl)but-1-yl, 5-(Hydroxycarbonyl)-pent-1-yl, Methylsulfonyl, Ethylsulfonyl, Prop-1-ylsulfonyl, 1-Methyleth-1-ylsulfonyl, But-1-ylsulfonyl, 1-Methylprop-1-ylsulfonyl, 2-Methylprop-1-ylsulfonyl, 1,1-Dimethyl-ethylsulfonyl, Pent-1-ylsulfonyl, 1-Methylbut-1-ylsulfonyl, 2-Methylbut-1-ylsulfonyl,
3-Methylbut-1-ylsulfonyl, 1,1-Dimethylprop-1-ylsulfonyl, 1,2-Dimethylprop-1-ylsulfonyl, 2,2-Dimethylprop-1-ylsulfonyl, 1-Ethylprop-1-ylsulfonyl, Cyanomethylsulfonyl, 3-Cyanoprop-1-ylsulfonyl, 1-Methylcycloprop-1-ylsulfonyl, Tetrahydrofuran-3-ylsulfonyl, Methoxycarbonyl-Methylsulfonyl, Ethoxycarbonylmethylsulfonyl, Cyclopropylmethylsulfonyl, Cyclobutylmethylsulfonyl, Cyclopentylmethylsulfonyl, Cyclohexylmethylsulfonyl, Cyclopropylsulfonyl, Cyclobutylsulfonyl, Cyclopentylsulfonyl, Cyclohexylsulfonyl, Prop-2-en-1-ylsulfonyl, Prop-2-en-2-ylsulfonyl, But-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-Methylprop-2-en-1-ylsulfonyl, 2-Methylprop-2-en-1-ylsulfonyl, Trifluormethylsulfonyl, Benzylsulfonyl,
2-Phenyleth-1-ylsulfonyl, (2-Fluorphenyl)methylsulfonyl,
(3-Fluorphenyl)-methylsulfonyl, (4-Fluorphenyl)-methylsulfonyl,
(2,6-Difluorphenyl)methylsulfonyl, (2,5-Difluorphenyl)-methylsulfonyl,
(2,4-Difluorphenyl)methylsulfonyl, (2,3-Difluorphenyl)methylsulfonyl,
(2-Fluor-6-chlorphenyl)methylsulfonyl, (2-Chlorphenyl)methylsulfonyl,
(3-Chlorphenyl)methylsulfonyl, (4-Chlorphenyl)methylsulfonyl, (2,6-Dichlorphenyl)-methylsulfonyl, (2,5-Dichlorphenyl)methylsulfonyl, (2,4-Dichlorphenyl)methylsulfonyl, (2,3-Dichlorphenyl)methylsulfonyl,
(2-Bromphenyl)methylsulfonyl, (3-Bromphenyl)-methylsulfonyl,
(4-Bromphenyl)methylsulfonyl, (2-Methylphenyl)methylsulfonyl,
(2-Trifluormethylphenyl)-methylsulfonyl, (2-Methoxyphenyl)-methylsulfonyl,
(2-Nitrophenyl)-methylsulfonyl, (3-Methylphenyl)-methylsulfonyl,
(3-Trifluormethylphenyl)-methylsulfonyl, (3-Methoxyphenyl)-methylsulfonyl,
(3-Nitrophenyl)methylsulfonyl, (4-Methylphenyl)methylsulfonyl,
(4-Trifluormethylphenyl)-methylsulfonyl, (4-Methoxyphenyl)-methylsulfonyl,
(4-Nitrophenyl)methylsulfonyl, Pyridin-2-ylmethylsulfonyl, Pyridin-3-ylmethylsulfonyl, Pyridin-4-ylmethylsulfonyl, (6-Fluoropyridin-2-yl)methylsulfonyl, (6-Chlor-4-trifluormethylpyridin-2-yl)methylsulfonyl, (4,6-Dichlorpyridin-2-yl)methylsulfonyl, (4-Fluorpyridin-3-yl)methylsulfonyl, (4-Chlorpyridin-3-yl)methylsulfonyl, Thiophen-2-ylmethylsulfonyl, Phenylsulfonyl, (2-Fluorphenyl)sulfonyl, (3-Fluorphenyl)sulfonyl, (4-Fluorphenyl)sulfonyl, (2,6-Difluorphenyl)sulfonyl, (2,5-Difluorphenyl)sulfonyl, (2,4-Difluorphenyl)sulfonyl, (2,3-Difluorphenyl)sulfonyl, (2-Fluor-6-chlorphenyl)sulfonyl, (2-Chlorphenyl)sulfonyl, (3-Chlorphenyl)sulfonyl,
(4-Chlorphenyl)sulfonyl, (2,6-Dichlorphenyl)sulfonyl, (2,5-Dichlorphenyl)sulfonyl, (2,4-Dichlorphenyl)sulfonyl, (2,3-Dichlorphenyl)sulfonyl, (2-Bromphenyl)sulfonyl, (3-Bromphenyl)sulfonyl, (4-Bromphenyl)sulfonyl, (2-Methylphenyl)sulfonyl,
(2-Trifluormethylphenyl)sulfonyl, (2-Methoxyphenyl)sulfonyl, (2-Nitrophenyl)sulfonyl, (3-Methylphenyl)sulfonyl, (3-Trifluormethylphenyl)sulfonyl,
(3-Methoxyphenyl)sulfonyl, (3-Nitrophenyl)sulfonyl, (4-Methylphenyl)sulfonyl,
(4-Trifluormethylphenyl)sulfonyl, (4-Methoxyphenyl)sulfonyl, (4-Nitrophenyl)sulfonyl, Pyridin-2-ylsulfonyl, Pyridin-3-ylsulfonyl, Pyridin-4-ylsulfonyl, (6-Fluoropyridin-2-yl)sulfonyl, (6-Chlor-4-trifluorsulfonylpyridin-2-yl)sulfonyl, (4,6-Dichlorpyridin-2-yl)sulfonyl, (4-Fluorpyridin-3-yl)sulfonyl, (4-Chlorpyridin-3-yl)sulfonyl, Thiophen-2-ylsulfonyl, Methoxymethylsulfonyl, 2-(Methoxy)eth-1-ylsulfonyl, 2-(Methoxy)eth-1-ylsulfonyl, Methylaminosulfonyl, Ethylaminosulfonyl, Dimethylaminosulfonyl, Diethylaminosulfonyl, Methyl(ethyl)aminosulfonyl, Azetidin-1-ylsulfonyl, Pyrrolidin-1-ylsulfonyl, Piperidin-1-ylsulfonyl, N-Morpholinylsulfonyl, 2-(Methoxycarbonyl)eth-1-ylsulfonyl, 2-(Ethoxycarbonyl)eth-1-ylsulfonyl, 3-(Methoxycarbonyl)prop-1-ylsulfonyl, 3-(Ethoxycarbonyl)prop-1-ylsulfonyl, 1-(Methoxycarbonyl)eth-1-ylsulfonyl,
2-Ethoxyeth-1-ylsulfonyl, 1-(Prop-2-en-1-yl)cycloprop-1-ylsulfonyl, 3,3,3-Trifluorethylsulfonyl, Tetrahydropyran-4-ylsulfonyl, Tetrahydrofuran-3-ylmethylsulfonyl, Formyl, Methylcarbonyl, Ethylcarbonyl, Prop-1-ylcarbonyl,
1-Methyleth-1-ylcarbonyl, But-1-ylcarbonyl, 1-Methylprop-1-ylcarbonyl, 2-Methylprop-1-ylcarbonyl, 1,1-Dimethylethylcarbonyl, Pent-1-ylcarbonyl, 1-Methylbut-1-ylcarbonyl, 2-Methylbut-1-ylcarbonyl, 3-Methylbut-1-ylcarbonyl, 1,1-Dimethylprop-1-ylcarbonyl, 1,2-Dimethylprop-1-ylcarbonyl, 2,2-Dimethylprop-1-ylcarbonyl, 1-Ethylprop-1-ylcarbonyl, Cyclopropylmethylcarbonyl, Cyclobutylmethylcarbonyl, Cyclopentylmethylcarbonyl, Cyclohexylmethylcarbonyl, Cyclopropylcarbonyl, Cyclobutylcarbonyl, Cyclopentylcarbonyl, Cyclohexylcarbonyl, Methoxymethylcarbonyl, 2-Methoxyeth-1-ylcarbonyl, 2-Ethoxyeth-1-ylcarbonyl, 3-Methoxyprop-1-ylcarbonyl,
3-Ethoxyprop-1-ylcarbonyl, Methoxycarbonylmethylcarbonyl, 2-(Methoxycarbonyl)eth-1-ylcarbonyl, 3-(Methoxycarbonyl)prop-1-ylcarbonyl, 4-(Methoxycarbonyl)but-1-ylcarbonyl, Ethoxycarbonylmethylcarbonyl, 2-(Ethoxycarbonyl)eth-1-ylcarbonyl,
3-(Ethoxycarbonyl)prop-1-ylcarbonyl, 4-(Ethoxycarbonyl)but-1-ylcarbonyl, Benzylcarbonyl, 2-Phenyleth-1-ylcarbonyl, (2-Fluorophenyl)methylcarbonyl,
(3-Fluorphenyl)methylcarbonyl, (4-Fluorphenyl)methylcarbonyl, (2,6-Difluorphenyl)-methylcarbonyl, (2,5-Difluorphenyl)methylcarbonyl, (2,4-Difluorphenyl)-methylcarbonyl, (2,3-Difluorphenyl)methylcarbonyl, (2-Fluor-6-chlorphenyl)-methylcarbonyl, (2-Chlorphenyl)-methylcarbonyl,
(3-Chlorphenyl)methylcarbonyl, (4-Chlorphenyl)methylcarbonyl,
(2,6-Dichlorphenyl)methylcarbonyl, (2,5-Dichlorphenyl)methylcarbonyl,
(2,4-Dichlorphenyl)-methylcarbonyl, (2,3-Dichlorphenyl)-methylcarbonyl,
(2-Bromphenyl)-methylcarbonyl, (3-Bromphenyl)-methylcarbonyl,
(4-Bromphenyl)methylcarbonyl, (2-Methylphenyl)-methylcarbonyl,
(2-Trifluormethylphenyl)methylcarbonyl, (2-Methoxyphenyl)methylcarbonyl,
(2-Nitrophenyl)methylcarbonyl, (3-Methylphenyl)methylcarbonyl,
(3-Trifluormethylphenyl)-methylcarbonyl, (3-Methoxyphenyl)-methylcarbonyl,
(3-Nitrophenyl)methylcarbonyl, (4-Methylphenyl)methylcarbonyl,
(4-Trifluormethylphenyl)-methylcarbonyl, (4-Methoxyphenyl)-methylcarbonyl,
(4-Nitrophenyl)methylcarbonyl, Pyridin-2-ylmethylcarbonyl, Pyridin-3-ylmethylcarbonyl, Pyridin-4-ylmethylcarbonyl, (6-Fluoropyridin-2-yl)methylcarbonyl, (6-Chlor-4-trifluormethylpyridin-2-yl)methylcarbonyl, (4,6-Dichlorpyridin-2-yl)methylcarbonyl, (4-Fluorpyridin-3-yl)methylcarbonyl, (4-Chlorpyridin-3-yl)methylcarbonyl, Thiophen-2-ylmethylcarbonyl, Phenylcarbonyl,
(2-Fluorophenyl)carbonyl, (3-Fluorophenyl)carbonyl, (4-Fluorophenyl)carbonyl,
(2,6-Difluorphenyl)carbonyl, (2,5-Difluorphenyl)carbonyl, (2,4-Difluorphenyl)-carbonyl, (2,3-Difluorphenyl)carbonyl, (2-Fluor-6-chlorphenyl)carbonyl,
(2-Chlorphenyl)carbonyl, (3-Chlorphenyl)carbonyl, (4-Chlorphenyl)carbonyl,
(2,6-Dichlorphenyl)carbonyl, (2,5-Dichlorphenyl)carbonyl, (2,4-Dichlorphenyl)-carbonyl, (2,3-Dichlorphenyl)carbonyl, (2-Bromphenyl)carbonyl, (3-Bromphenyl)-carbonyl, (4-Bromphenyl)carbonyl, (2-Methylphenyl)carbonyl, (2-Trifluormethylphenyl)-carbonyl, (2-Methoxyphenyl)carbonyl, (2-Nitrophenyl)carbonyl, (3-Methylphenyl)carbonyl, (3-Trifluormethylphenyl)carbonyl, (3-Methoxyphenyl)carbonyl,
(3-Nitrophenyl)carbonyl, (4-Methylphenyl)carbonyl, (4-Trifluormethylphenyl)carbonyl, (4-Methoxyphenyl)carbonyl, (4-Nitrophenyl)carbonyl, (2-Chlor-4-methylsulfonyl-phenyl)carbonyl, Pyridin-2-ylcarbonyl, Pyridin-3-ylcarbonyl, Pyridin-4-ylcarbonyl,
(6-Fluorpyridin-2-yl)carbonyl, (6-Chlor-4-trifluorcarbonylpyridin-2-yl)carbonyl, (4,6-Dichlorpyridin-2-yl)carbonyl, (4-Fluorpyridin-3-yl)carbonyl, (4-Chlorpyridin-3-yl)carbonyl, Thiophen-2-ylcarbonyl, 1,3-Oxazol-2-ylcarbonyl, 1,2-Oxazol-3-ylcarbonyl, 3-Chlor-5-methyl-4,5-dihydro-1,2-oxazol-5-ylcarbonyl, 3-Brom-5-methyl-4,5-dihydro-1,2-oxazol-5-ylcarbonyl, 3-Chlor-5-methoxymethyl-4,5-dihydro-1,2-oxazol-5-yl-carbonyl, 3-Chlor-5-ethyl-4,5-dihydro-1,2-oxazol-5-ylcarbonyl, 3-Chlor-3a,4,5,6-tetrahydro-6aH-cyclopenta[d][1,2]oxazol-6a-ylcarbonyl, (4-Trifluormethylpyridin-3-yl)carbonyl, 3,5-Dimethyl-4,5-dihydro-1,2-oxazol-5-ylcarbonyl, 5-Methyl-3-trifluormethyl-4,5-dihydro-1,2-oxazol-5-ylcarbonyl, 3-Chlor-5-hydroxymethyl-4,5-dihydro-1,2-oxazol-5-ylcarbonyl, 3-Chlor-5-tert-butyldimethylsilyloxymethyl-4,5-dihydro-1,2-oxazol-5-ylcarbonyl, 3-Chlor-5-(1-hydroxyeth-1-yl)-4,5-dihydro-1,2-oxazol-5-ylcarbonyl, 3-Chlor-5-(1-tert-butyldimethyl-silyloxyeth-1-yl-4,5-dihydro-1,2-oxazol-5-ylcarbonyl, Methoxyethoxymethylcarbonyl, Ethoxyethoxymethylcarbonyl, Hydroxyethoxymethylcarbonyl, Methoxyethoxyethoxymethylcarbonyl, Ethoxyethoxyethoxymethylcarbonyl, Hydroxyethoxyethoxymethylcarbonyl, Methoxyethoxyethoxymethylcarbonyl, Ethoxyethoxyethoxyethoxymethylcarbonyl, Hydroxyethoxyethoxyethoxymethylcarbonyl, Methoxycarbonylmethoxyethoxymethylcarbonyl, 4-Trifluormethylpyridin-3-ylcarbonyloxyethoxyethoxy-methylcarbonyl,Hydroxycarbonylmethoxyethoxymethylcarbonyl, Methoxycarbonylmethoxyethoxyethoxymethylcarbonyl, Hydroxycarbonylmethoxyethoxyethoxymethylcarbonyl, Methoxycarbonylmethoxyethoxyethoxyethoxymethylcarbonyl oder Hydroxycarbonylmethoxyethoxyethoxyethoxymethylcarbonyl.

7. Die Verbindung der allgemeinen Formel (I), wie in Anspruch 1 beansprucht, und/oder das Salz davon, **dadurch gekennzeichnet, dass**
R¹ für Phenyl, 2-Fluorphenyl, 3-Fluorphenyl, 2-Chlorphenyl, 2-Methylphenyl, 2-Ethyl-Phenyl, 2-Methoxyphenyl, 2,3-Difluorphenyl, 2,4-Difluorphenyl, 2,5-Difluorphenyl, 2,6-Difluorphenyl, 2,4,5-Trifluorphenyl, 2,3,4-Fluorphenyl, 2-Fluor-6-methylphenyl, 2-Fluor-3-methylphenyl, 2-Fluor-3-chlorphenyl, 2,3-Dimethylphenyl, 2-Thienyl, 3-Chlor-2-Thienyl, 3-Methyl-2-Thienyl, 4-Methyl-3-Thienyl,
R² steht für Wasserstoff, Fluor, Chlor, Brom, Jod, Methyl, Ethyl, 2,3,4,5,6-Pentafluorphenyl, Ethoxycarbonyl, Cyclopropyl,
R³ steht für Wasserstoff,
n ist 0, 2,
R⁴ und R⁵ unabhängig voneinander Wasserstoff, Methyl, Ethyl, Prop-2-yl, Methoxymethyl, Cyclopropyl, Cyclobutyl, oder
R⁴ und R⁵ zusammen mit dem Kohlenstoffatom, an das sie gebunden sind, einen vollständig gesättigten oder teilweise gesättigten 3- bis 10-gliedrigen monocyclischen oder bicyclischen Ring bilden, der gegebenenfalls durch Heteroatome unterbrochen ist und gegebenenfalls eine weitere Substitution aufweist, und
R⁶ Wasserstoff, BH₂, B(NH₂)₂, 4-Amino-1,3,2,4-diazadiboretidin-2-yl, 4-Amino-1,3,2,4-dioxadiboretan-2-yl, Methylsulfonyl, Ethylsulfonyl, Prop-1-ylsulfonyl, Cyanomethylsulfonyl, Methoxycarbonylmethylsulfonyl, 2-Ethoxyeth-1-ylsulfonyl, Prop-2-en-1-ylsulfonyl, Prop-2-en-2-ylsulfonyl, (4-Fluorophenyl)methylsulfonyl, Formyl, Methylcarbonyl, Ethylcarbonyl, Prop-1-ylcarbonyl, 1-Methyleth-1-ylcarbonyl, Cyclopropylcarbonyl, Cyclopentylcarbonyl, 2-(Methoxycarbonyl)eth-1-ylcarbonyl, Ethoxycarbonylmethylcarbonyl, 3-(Ethoxycarbonyl)prop-1-ylcarbonyl, (2-Trifluormethylphenyl)carbonyl, 3-Chlor-5-methyl-4,5-dihydro-1,2-oxazol-5-ylcarbonyl, 3-Chlor-5-methoxymethyl-4,5-dihydro-1,2-oxazol-5-ylcarbonyl, 3-Chlor-3a,4,5,6-tetrahydro-6aH-cyclopenta[d][1,2]oxazol-6a-ylcarbonyl, Hydroxyethoxyethoxymethylcarbonyl, Methoxyethoxyethoxymethylcarbonyl, 4-Trifluormethylpyridin-3-ylcarbonyloxyethoxy-ethoxymethylcarbonyl, Hydroxycarbonylmethoxyethoxy-methylcarbonyl.

8. Die Verbindung der allgemeinen Formel (I), wie in Anspruch 1 beansprucht, und/oder das Salz davon, **dadurch gekennzeichnet, dass**
R¹ für Phenyl, 2-Fluorphenyl, 3-Fluorphenyl, 2-Chlorphenyl, 2-Methylphenyl, 2-Ethylphenyl, 2-Methoxyphenyl, 2,3-Difluorophenyl, 2,4-Difluorophenyl, 2,6-Difluorphenyl, 2-Fluor-6-methylphenyl, 2-Fluor-3-methylphenyl, 2-Fluor-3-chlorphenyl, 2,3-Dimethylphenyl, 2-Thienyl, 3-Chlor-2-thienyl, 3-Methyl-2-thienyl oder 4-Methyl-3-thienyl,
R² Wasserstoff, Fluor, Chlor, Brom, Methyl, Ethyl, 2,3,4,5,6-Pentafluorphenyl oder Ethoxycarbonyl bedeutet,
R³ für Wasserstoff steht,
n ist 0
R⁴ und R⁵ unabhängig voneinander für Wasserstoff stehen,
R⁶ Wasserstoff, BH₂, B(NH₂)₂, 4-Amino-1,3,2,4-diazadiboretidin-2-yl, 4-Amino-1,3,2,4-dioxadiboretan-2-yl, Methylsulfonyl, Ethylsulfonyl, Prop-1-ylsulfonyl, Cyanomethylsulfonyl, Methoxycarbonylmethylsulfonyl, 2-Ethoxyeth-1-ylsulfonyl, Prop-2-en-1-ylsulfonyl, Prop-2-en-2-ylsulfonyl, (4-Fluorphenyl)methylsulfonyl, Formyl, Methylcarbonyl, Ethylcarbonyl, Prop-1-ylcarbonyl, 1-Methyleth-1-ylcarbonyl, Cyclopropylcarbonyl, Cyclopentylcarbonyl, 2-(Methoxy-carbonyl)eth-1-ylcarbonyl, Ethoxycarbonylmethylcarbonyl, 3-(Ethoxycarbonyl)prop-1-ylcarbonyl, (2-Trifluormethylphenyl)carbonyl, 3-Chlor-5-methyl-4,5-dihydro-1,2-oxazol-5-ylcarbonyl, 3-Chlor-5-methoxymethyl-4,5-dihydro-1,2-oxazol-5-ylcarbonyl, oder 3-Chlor-3a,4,5,6-tetrahydro-6aH-cyclopenta[d][1,2]oxazol-6a-ylcarbonyl.

9. Die Verwendung einer oder mehrerer Verbindungen der allgemeinen Formel (I) und/oder deren Salze, wie in einem der Ansprüche 1 bis 8 definiert, als Herbizid und/oder Pflanzenwachstumsregulator.

10. Herbizide und/oder das Pflanzenwachstum regulierende Zusammensetzung, **dadurch gekennzeichnet, dass** die Zusammensetzung eine oder mehrere Verbindungen der allgemeinen Formel (I) und/oder deren Salze, wie in einem der Ansprüche 1 bis 8 definiert, und eine oder mehrere weitere Substanzen, ausgewählt aus den Gruppen (i) und/oder (ii), umfasst, wobei
(i) eine oder mehrere weitere agrochemisch aktive Substanzen, ausgewählt aus der Gruppe bestehend aus Insektiziden, Akariziden, Nematiziden, weiteren Herbiziden, Fungiziden, Safenern, Düngemitteln und/oder weiteren Wachstumsregulatoren,
(ii) ein oder mehrere im Pflanzenschutz übliche Formulierungshilfsmittel.

11. Verfahren zur Bekämpfung von Schadpflanzen oder zur Regulierung des Wachstums von Pflanzen, **dadurch gekennzeichnet, dass** eine wirksame Menge
- einer oder mehrerer Verbindungen der allgemeinen Formel (I) und/oder deren Salze, wie in einem der Ansprüche 1 bis 8 definiert, oder
- einer Zusammensetzung wie in Anspruch 10 beansprucht,
auf die Pflanzen, Samen von Pflanzen, den Boden, in oder auf dem die Pflanzen aufziehen, oder die Anbaufläche aufgebracht wird.

## Revendications

1. 2,3-dihydro[1,3]thiazolo[4,5-b]pyridines substituées de formule générale (I) ou leurs sels 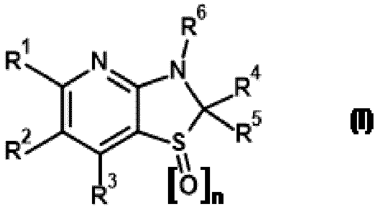 dans laquelle
R¹ représente un (C₃-C₈)-cycloalkyle, (C₃-C₈)-cycloalkényle, (C₃-C₈)-cycloalkoxy, un aryle, un hétéroaryle, un hétérocyclyle, un résidu bicyclique ou hétérobicyclique, dans lequel chacun des huit résidus susmentionnés est non substitué ou est substitué indépendamment par un ou plusieurs résidus choisis dans le groupe R⁷,
R² représente l'hydrogène, l'halogène, le formyle, l'hydroxy, l'hydrothio, l'hydroxycarbonyle, l'aminocarbonyle, l'aminothiocarbonyle, (C₁-C₈)-alkyl, (C₁-C₈)-haloalkyl, (C₂-C₈)-alkenyl, (C₂-C₈)-haloalcényle, (C₂-C₈)-alcynyle, (C₂-C₈)-haloalcynyle, (C₁-C₈)-alkoxy, (C₁-C₈) haloalkoxy, (C₁-C₈)-alkoxy-(C₁-C₈)-alkyl, (C₁-C₈)-alkylthio, (C₁-C₈)-haloalkylthio, (C₁-C₈)-alkylsulfinyle, (C₁-C₈)-haloalkylsulfinyle, (C₁-C₈)-alkylsulfonyle, (C₁-C₈)-haloalkylsulfonyle, (C₁-C₈)-alkylcarbonyle, (C₂-C₈)-alcénylcarbonyle, (C₂-C₈)-alcynylcarbonyle, (C₁-C₈)-haloalkylcarbonyle, (C₂-C₈)-haloalcénylcarbonyle, (C₂-C₈)-haloalcynylcarbonyle, (C₁-C₈)-alkoxycarbonyle, (C₃-C₈)-cycloalkyle, (C₃-C₈)-cycloalkoxy, (C₃-C₈)-cycloalkylthio, (C₃-C₈)-cycloalkylsulfinyle, (C₃-C₈)-cycloalkylsulfonyle, (C₃-C₈)-halocycloalkyle, (C₃-C₈)-halocycloalkoxy, (C₃-C₈)-halocycloalkylthio, (C₃-C₈)-halocycloalkylsulfinyle, (C₃-C₈)-halocycloalkylsulfonyle, (C₃-C₈)-cycloalkyle-(C₁-C₈)-alkyle, (C₁-C₈)-alkyle-(C₃-C₈)-cycloalkyle, (C₃-C₈)-cycloalkylcarbonyle, (C₃-C₈)-cycloalkyle-(C₁-C₈)-alkylcarbonyle, (C₁-C₈)-alkylaminocarbonyle, (C₂-C₁₂)-dialkylaminocarbonyle, (C₁-C₈)-alkylaminosulfonyle, (C₂-C₁₂)-dialkylaminosulfonyle, tris[(C₁-C₈)alkyl]silyle, ou aryle,
R³ représente l'hydrogène, l'halogène, le cyano, (C₁-C₈)-alkyl, (C₁-C₈)-haloalkyl, (C₂-C₈)-alcényle, (C₂-C₈)-haloalcényle, (C₁-C₈)-alkoxy, (C₁-C₈) haloalkoxy, (C₁-C₈)-alkylthio, (C₁-C₈)-haloalkylthio, (C₃-C₈)-cycloalkyle, (C₃-C₈)-halocycloalkyle, (C₃-C₈)-cycloalkoxy ou (C₃-C₈)-halocycloalkoxy,
R⁴ et R⁵ représentent indépendamment l'un de l'autre l'hydrogène, (C₁-C₈)-alkyl, (C₁-C₈)-haloalkyl, (C₃-C₈)-cycloalkyl, (C₃-C₈)-halocycloalkyl, ou R¹⁰O-(C₁-C₈)-alkyl, ou
R⁴ et R⁵ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R⁶ représente l'hydrogène, (C₁-C₈)-alkyl, (C₂-C₈)-alkenyl, (C₂-C₈)-alkynyl, cyano-(C₁-Cs)-alkyl, R¹⁰O-(C₁-C₈)-alkyl, R⁸R⁹N-(C₁-C₈)-alkyl, NR⁸R⁹, (C₃-C₁₀)-cycloalkyle-(C₁-C₈)-alkyle, C(=O)R¹⁰, C(=O)OR¹⁰, C(=O)NR⁸R⁹, R¹⁰O(O)C-(C₁-C₈)-alkyl, R¹⁰(O=)C-(C₁-C₈)-Alkyl, R⁸R⁹N(O)C-(C₁-C₈)-alkyl, SO₂R¹¹, R¹¹S(O)ₘ-(C₁-C₈)-alkyl, BR¹²R¹³, hétérocyclyle, hétéroaryle, aryle-(C₁-C₈)-alkyl, hétéroaryle-(C₁-C₈)-alkyl, hétérocyclyle-(C₁-C₈)-alkyl, tris[(C₁-C₈)alkyl]silyl, bis-[(C₁-C₈)-alkyl](aryl)silyl, (C₁-C₈)-alkyl-[bis-(aryl)]silyl, tris-[(C₁-C₈)-alkyl]silyl-(C₁-C₈)-alkyl, bis-[(C₁-C₈)-alkyl](aryl)silyl(C₁-C₈)-alkyl, ou (C₁-C₈)-alkyl-[bis-(aryl)]silyl-(C₁-C₈)-alkyl,
m est 0, 1, 2,
n est 0, 1, 2,
R⁷ représente l'hydrogène, l'halogène, le formyle, l'hydroxy, l'hydroxycarbonyle, le nitro, le cyano, l'amino, l'aminocarbonyle, l'aminothiocarbonyle, (C₁-C₈)-alkyl, (C₁-C₈)-haloalkyl, (C₂-C₈)-alcényle, (C₂-C₈)-haloalcényle, (C₂-C₈)-alcynyle, (C₂-C₈)-haloalcynyle, (C₁-C₈)-alcoxy, (C₁-C₈)-haloalcoxy, (C₁-C₈)-alkylthio, (C₁-C₈)-haloalkylthio, (C₁-C₈)-alkylsulfinyl, (C₁-C₈)-haloalkylsulfinyl, (C₁-C₈)-alkylsulfonyl, (C₁-C₈)-haloalkylsulfonyl, (C₁-C₈)-alkylcarbonyl, (C₁-C₈)-haloalkylcarbonyl, (C₂-C₈)-alcénylcarbonyl, (C₂-C₈)-haloalcénylcarbonyle, (C₂-C₈)-alcynylcarbonyle, (C₂-C₈)-haloalcynylcarbonyle, (C₁-C₈)-alcoxycarbonyle, (C₁-C₈)-haloalcoxycarbonyle, (C₃-C₈)-cycloalkyle, (C₃-C₈)-halocycloalkyle, (C₃-C₈)-cycloalkoxy, (C₃-C₈)-halocycloalkoxy, (C₃-C₈)-cycloalkylthio, (C₃-C₈)-halocycloalkylthio, (C₃-C₈)- cycloalkylsulfinyl, (C₃-C₈)-halocycloalkylsulfinyl, (C₃-C₈)-cycloalkylsulfonyl, (C₃-C₈)-halocycloalkylsulfonyl, (C₃-C₈)-cycloalkyle-(C₁-C₈)-alkyle, (C₁-C₈)-alkyle-(C₃-C₈)-cycloalkyle, (C₁-C₈)-alkoxycarbonyle-(C₁-C₈)-alkyl, hydroxycarbonyle-(C₁-C₈)-alkyl, (C₃-C₈)-cycloalkylcarbonyle, (C₃-C₈)-cycloalkyl-(C₁-C₈)-alkylcarbonyle, (C₁-C₈)-alkylamino, (C₂-C₁₂)-dialkylamino, (C₁-C₈)-alkylaminocarbonyle, (C₂-C₁₂)-dialkylaminocarbonyle, (C₁-C₈)-alkylaminosulfonyle, (C₂-C₁₂)-dialkylaminosulfonyle ou tris[(C₁-C₈)alkyl]silyle.
-R⁸ et R⁹ représentent indépendamment des autres l'hydrogène, (C₁-C₈)-alkyl, (C₂-C₈)-alkenyl, (C₂-C₈)-alkynyl, (C₁-C₈)-cyanoalkyl, (C₁-C₈)-haloalkyl, (C₂-C₈)-haloalcényle, (C₃-C₈)-haloalkynyl, (C₃-C₈)-cycloalkyl, (C₃-C₈)-halocycloalkyl, (C₄-C₈)-cycloalkenyl, (C₄-C₈)-halocycloalkenyl, (C₁-C₈)-alkoxy-(C₁-C₈)-alkyl, (C₁-C₈)-alkoxy-(C₁-C₈)-alkoxy-(C₁-C₈)-alkyl, (C₁-C₈)-alcoxy-(C₁-C₈)-alcoxy-(C₁-C₈)-alcoxy-(C₁-C₈)-alkyl, (C₁-C₈)-haloalcoxy-(C₁-C₈)-alkyl, (C₁-C₈)-alkylthio-(C₁-C₈)-alkyl, (C₁-C₈)-haloalkylthio-(C₁-C₈)-alkyl, (C₁-C₈)-alkoxy-(C₁-C₈)-haloalkyl, aryl, aryl-(C₁-C₈)-alkyl, hétéroaryl, hétéroaryl-(C₁-C₈)-alkyl, (C₃-C₈)-cycloalkyl-(C₁-C₈)-alkyl, (C₄-C₈)-cycloalkenyl-(C₁-C₈)-alkyl, COR¹⁰, SO₂R¹¹, hétérocycle, (C₁-C₈)-alkoxycarbonyle, bis-[(C₁-C₈)-alkyl]aminocarbonyle-(C₁-C₈)-alkyl, (C₁-C₈)-alkyl-aminocarbonyle-(C₁-C₈)-alkyl, aryl-(C₁-C₈)-alkyl-aminocarbonyle-(C₁-C₈)-alkyl, (C₁-C₈)-alkoxycarbonyle-(C₁-C₈)-alkyl, (C₂-C₈)-alkényloxycarbonyle-(C₁-C₈)-alkyl, (C₂-C₈)-alkynyloxycarbonyle-(C₁-C₈)-alkyl, hétérocyclyle-(C₁-C₈)-alkyl, ou
R⁸ et R⁹ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R¹⁰ représente l'hydrogène, (C₁-C₈)-alkyl, (C₂-C₈)-alkenyl, (C₂-C₈)-alkinyl, (C₁-C₈)-cyanoalkyl, (C₁-C₈)-haloalkyl, (C₂-C₈)-haloalcényle, (C₃-C₈)-haloalkinyle, (C₃-C₈)-cycloalkyl, (C₃-C₈)-halocycloalkyl, (C₄-C₈)-cycloalkenyl, (C₄-C₈)-halocycloalkenyl, (C₁-C₈)-alkoxy-(C₁-C₈)-alkyl, (C₁-C₈)-haloalkoxy-(C₁-C₈)-alkyl, (C₁-C₈)-alkoxy-(C₁-C₈)-haloalkyl, (C₁-C₈)-alkoxy-(C₁-C₈)-alkoxy-(C₁-C₈)-alkyl, (C₁-C₈)-alkoxy-(C₁-C₈)-alkoxy-(C₁-C₈)-alkoxy-(C₁-C₈)-alkyl, aryl, aryl-(C₁-C₈)-alkyl, aryl-(C₁-C₈)-alkoxy-(C₁-C₈)-alkyl, aryloxy-(C₁-C₈)-alkyl, hétéroaryl-(C₁-C₈)-alkoxy-(C₁-C₈)-alkyl, hétéroaryloxy-(C₁-C₈)-alkyl, hétéroaryl, hétéroaryl-(C₁-C₈)-alkyl, (C₃-C₈)-cycloalkyl-(C₁-C₈)-alkyl, (C₄-C₈)-cycloalkenyl-(C₁-C₈)-alkyl, bis-[(C₁-C₈)-alkyl]aminocarbonyle-(C₁-C₈)-alkyl, (C₁-C₈)-alkyl-aminocarbonyle-(C₁-C₈)-alkyl, aryl-(C₁-C₈)-alkyl-aminocarbonyle-(C₁-C₈)-alkyl, (C₁-C₈)-alkoxycarbonyle-(C₁-C₈)-alkyl, (C₂-C₈)-alkényloxycarbonyle-(C₁-C₈)-alkyl, (C₂-C₈)-alkynyloxycarbonyle-(C₁-Cs)-alkyl, (C₁-C₈)-alkylcarbonyloxy-(C₁-C₈)-alkyl, (C₃-C₈)-cycloalkylcarbonyloxy-(C₁-C₈)-alkyl, arylcarbonyloxy-(C₁-C₈)-alkyl, heteroarylcarbonyloxy-(C₁-C₈)-alkyl, Heterocyclylcarbonyloxy-(C₁-C₈)-alkyl, (C₁-C₈)-alkoxycarbonyle-(C₁-C₈)-alkyl, (C₁-C₈)-alkoxycarbonyle-(C₁-C₈)-alkyl, (C₂-C₈)-alkényloxycarbonyle-(C₁-C₈)-alkyl, (C₂-C₈)-alkynyloxycarbonyle-(C₁-C₈)-alkyl, (C₁-C₈)-alkylcarbonyloxy-(C₁-C₈)-alkoxy-(C₁-C₈)-alkyl, (C₁-C₈)-alkylcarbonyloxy-(C₁-C₈)-alkoxy-(C₁-C₈)-alkoxy-(C₁-C₈)-alkyl, (C₁-C₈)-alkoxycarbonyle-(C₁-C₈)-alkoxy-(C₁-C₈)-alkyl, (C₁-C₈)-alkoxycarbonyle-(C₁-C₈)-alkoxy-(C₁-C₈)-alkoxy-(C₁-C₈)-alkyl, hétéroarylcarbonyloxy-(C₁-C₈)-alkoxy-(C₁-C₈)-alkyl, hétéroarylcarbonyloxy-(C₁-C₈)-alkoxy-(C₁-C₈)-alkoxy-(C₁-C₈)-alkyl, hétéroarylcarbonyloxy-(C₁-C₈)-alkoxy-(C₁-C₈)-alkoxy-(C₁-C₈)-alkoxy-(C₁-C₈)-alkyl, hétérocyclylcarbonyloxy-(C₁-C₈)-alkoxy-(C₁-C₈)-alkyl, hétérocyclylcarbonyloxy-(C₁-Cₐ)-alkoxy-(C₁-C₈)-alkoxy-(C₁-C₈)-alkyl, hydroxycarbonyle-(C₁-C₈)-alkoxy-(C₁-C₈)-alkyl, ou hydroxycarbonyle-(C₁-C₈)-alkoxy-(C₁-C₈)-alkoxy-(C₁-C₈)-alkoxy-(C₁-C₈)-alkyl,
R¹¹ représente l'hydrogène, (C₁-C₈)-alkyl, (C₂-C₈)-alkenyl, (C₂-C₄)-alkynyl, (C₁-C₈)-cyanoalkyl, (C₁-C₈)-haloalkyl, (C₂-C₈)-haloalkenyl, (C₃-C₈)-halogényle, (C₃-C₈)-cycloalkyle, (C₃-C₈)-halocycloalkyle, (C₄-C₈)-cycloalkényle, (C₄-C₈)-halocycloalkényle, (C₁-C₈)-alcoxy-(C₁-C₈)-alkyle, (C₁-C₈)-alkoxy-(C₁-C₈)-haloalkyle, aryle, aryle-(C₁-C₈)-alkyle, hétéroaryle, hétéroaryle-(C₁-C₈)-alkyle, hétérocyclyle-(C₁-C₈)-alkyle, (C₃-C₈)-cycloalkyle-(C₁-C₈)-alkyle, (C₄-C₈)-cycloalkenyl-(C₁-C₈)-alkyle, bis-[(C₁-C₈)-alkyl]amino, (C₁-C₈)-alkyl-amino, aryl-(C₁-C₈)-amino, aryl-(C₁-C₈)-alkyl-amino, aryl-[(C₁-C₈)-alkyl]amino ; hétéroaryle-(C₁-C₈)-amino, hétéroaryle-(C₁-C₈)-alkyl-amino, hétéroaryle-[(C₁-C₈)-alkyl]amino ; Hétérocyclyle-(C₁-C₈)-amino, hétérocyclyle-(C₁-C₈)-alkyl-amino, hétérocyclyle-[(C₁-C₈)-alkyl]amino ; (C₃-C₈)-cycloalkyl-amino, (C₃-C₈)-cycloalkyl-[(C₁-C₈)-alkyl]amino ; N-azétidinyl, N-pyrrolidinyl, N-piperidinyl, ou N-morpholinyl,
R¹² et R¹³ représentent indépendamment les uns des autres l'hydrogène, NR⁸R⁹, OR¹⁰, ou
R¹² et R¹³ forment, avec l'atome de bore auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire.

2. Composé de formule générale (I) selon la revendication 1 et/ou son sel, **caractérisé en ce que**
R¹ représente un (C₃-C₇)-cycloalkyle, (C₃-C₇)-cycloalkényle, (C₃-C₇)-cycloalkoxy, un aryle, un hétéroaryle, un hétérocyclyle, un résidu bicyclique ou hétérobicyclique, dans lequel chacun des huit résidus mentionnés précédemment est non substitué ou est indépendamment substitué par un ou plusieurs résidus choisis dans le groupe R⁷,
R² représente l'hydrogène, l'halogène, le formyle, l'hydroxy, l'hydrothio, l'hydroxycarbonyle, l'aminocarbonyle, l'aminothiocarbonyle, (C₁-C₇)-alkyl, (C₁-C₇)-haloalkyl, (C₂-C₇)-alcényle, (C₂-C₇)-haloalcényle, (C₂-C₇)-alcynyle, (C₂-C₇)-haloalcynyle, (C₁-C₇)-alcoxy, (C₁-C₇) haloalkoxy, (C₁-C₇)-alcoxy-(C₁-C₇)-alkyl, (C₁-C₇)-alkylthio, (C₁-C₇)-haloalkylthio, (C₁-C₇)-alkylsulfinyle, (C₁-C₇)-haloalkylsulfinyle, (C₁-C₇)-alkylsulfonyle, (C₁-C₇)-haloalkylsulfonyle, (C₁-C₇)-alkylcarbonyle, (C₂-C₇)-alcénylcarbonyle, (C₂-C₇)-alcynylcarbonyle, (C₁-C₇)-haloalkylcarbonyle, (C₂-C₇)-haloalcénylcarbonyle, (C₂-C₇)-haloalcynylcarbonyle, (C₁-C₇)-alkoxycarbonyle, (C₃-C₇)-cycloalkyle, (C₃-C₇)-cycloalkoxy, (C₃-C₇)-cycloalkylthio, (C₃-C₇)-cycloalkylsulfinyle, (C₃-C₇)-cycloalkylsulfonyle, (C₃-C₇)-halocycloalkyle, (C₃-C₇)-halocycloalkoxy, (C₃-C₇)-halocycloalkylthio, (C₃-C₇)-halocycloalkylsulfinyle, (C₃-C₇)-halocycloalkylsulfonyle, (C₃-C₇)-cycloalkyle-(C₁-C₇)-alkyle, (C₁-C₇)-alkyle-(C₃-C₇)-cycloalkyle, (C₃-C₇)-cycloalkylcarbonyle, (C₃-C₇)-cycloalkyle-(C₁-C₇)-alkylcarbonyle, (C₁-C₇)-alkylaminocarbonyle, (C₂-C₁₂)-dialkylaminocarbonyle, (C₁-C₇)-alkylaminosulfonyle, (C₂-C₁₂)-dialkylaminosulfonyle ou tris[(C₁-C₇)alkyl]silyle, ou aryle,
R³ représente l'hydrogène, l'halogène, le cyano, (C₁-C₇)-alkyl, (C₁-C₇)-haloalkyl, (C₂-C₇)-alcényle, (C₂-C₇)-haloalcényle, (C₁-C₇)-alkoxy, (C₁-C₇) haloalkoxy, (C₁-C₇)- alkylthio, (C₁-C₇)-haloalkylthio, (C₃-C₇)-cycloalkyle, (C₃-C₇)-halocycloalkyle, (C₃-C₇)-cycloalkoxy ou (C₃-C₇)-halocycloalkoxy,
-R⁴ et R⁵ représentent indépendamment l'un de l'autre l'hydrogène, (C₁-C₇)-alkyl, (C₁-C₇)-haloalkyl, (C₃-C₇)-cycloalkyl, (C₃-C₇)-halocycloalkyl, ou R¹⁰O-(C₁-C₇)-alkyl, ou
R⁴ et R⁵ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R⁶ représente l'hydrogène, (C₁-C₇)-alkyl, (C₂-C₇)-alkenyl, (C₂-C₇)-alkynyl, cyano-(C₁-C₇)-alkyl, R¹⁰O-(C₁-C₇)-alkyl, R⁸R⁹N-(C₁-C₇)-alkyl, NR⁸R⁹, (C₃-C₁₀)-cycloalkyle-(C₁-C₇)-alkyle, C(=O)R¹⁰, C(=O)OR¹⁰, C(=O)NR⁸R⁹, R¹⁰O(O)C-(C₁-C₇)-alkyl, R¹⁰(O=)C-(C₁-C₇)-Alkyl, R⁸R⁹N(O)C-(C₁-C₇)-alkyl, SO₂R¹¹, R¹¹S(O)ₘ-(C₁-C₇)-alkyl, BR¹²R¹³, hétérocyclyle, hétéroaryle, aryle-(C₁-C₇)-alkyl, hétéroaryle-(C₁-C₇)-alkyl, hétérocyclyle-(C₁-C₇)-alkyl, tris[(C₁-C₇)alkyl]silyl, bis-[(C₁-C₇)-alkyl](aryl)silyl, (C₁-C₇)-alkyl-[bis-(aryl)]silyl, tris-[(C₁-C₇)-alkyl]silyl-(C₁-C₇)-alkyl, bis-[(C₁-C₇)-alkyl](aryl)silyl(C₁-C₇)-alkyl, ou (C₁-C₇)-alkyl-[bis-(aryl)]silyl-(C₁-C₇)-alkyl,
m est 0, 1, 2,
n est 0, 1, 2,
R⁷ représente l'hydrogène, l'halogène, le formyle, l'hydroxy, l'hydroxycarbonyle, le nitro, le cyano, l'amino, l'aminocarbonyle, l'aminothiocarbonyle, (C₁-C₇)-alkyl, (C₁-C₇)-haloalkyl, (C₂-C₇)-alcényle, (C₂-C₇)-haloalcényle, (C₂-C₇)-alcynyle, (C₂-C₇)-haloalcynyle, (C₁-C₇)-alkoxy, (C₁-C₇)-haloalcoxy, (C₁-C₇)-alkylthio, (C₁-C₇)-haloalkylthio, (C₁-C₇)-alkylsulfinyle, (C₁-C₇)-haloalkylsulfinyle, (C₁-C₇)-alkylsulfonyle, (C₁-C₇)-haloalkylsulfonyle, (C₁-C₇)-alkylcarbonyle, (C₁-C₇)-haloalkylcarbonyle, (C₂-C₇)-alcénylcarbonyle, (C₂-C₇)-halogénylcarbonyle, (C₂-C₇)-alcynylcarbonyle, (C₂-C₇)-halogénylcarbonyle, (C₁-C₇)-alcoxycarbonyle, (C₁-C₇)-halogénoalcoxycarbonyle, (C₃-C₇)-cycloalkyle, (C₃-C₇)-halocycloalkyle, (C₃-C₇)-cycloalkoxy, (C₃-C₇)-halocycloalkoxy, (C₃-C₇)-cycloalkylthio, (C₃-C₇)-halocycloalkylthio, (C₃-C₇)-cycloalkylsulfinyle, (C₃-C₇)-halocycloalkylsulfinyle, (C₃-C₇)-cycloalkylsulfonyle, (C₃-C₇)-halocycloalkylsulfonyle, (C₃-C₇)-cycloalkyle-(C₁- C₇)-alkyle, (C₁-C₇)-alkyl-(C₃-C₇)-cycloalkyl, (C₁-C₇)-alkoxycarbonyle-(C₁-C₇)-alkyl, hydroxycarbonyle-(C₁-C₇)-alkyl, (C₃-C₇)-cycloalkylcarbonyle, (C₃-C₇)-cycloalkyle-(C₁-C₇)-alkylcarbonyle, (C₁-C₇)-alkylamino, (C₂-C₁₂)-dialkylamino, (C₁-C₇)-alkylaminocarbonyle, (C₂-C₁₂)-dialkylaminocarbonyle, (C₁-C₇)-alkylaminosulfonyle, (C₂-C₁₂)-dialkylaminosulfonyl ou tris[(C₁-C₇)alkyl]silyl,
-R⁸ et R⁹ représentent indépendamment des autres l'hydrogène, (C₁-C₇)-alkyl , (C₂-C₇)-alkenyl, (C₂-C₇)-alkynyl, (C₁-C₇)-cyanoalkyle, (C₁-C₇)-haloalkyle, (C₂-C₇)-haloalcényle, (C₃-C₇)-haloalcynyle, (C₃-C₇)-cycloalkyle, (C₃-C₇)-halocycloalkyle, (C₄-C₇)-cycloalcényle, (Cₐ-C₁₀)-halocycloalcényle, (C₁-C₇)-alkoxy-(C₁-C₇)-alkyl, (C₁-C₇)-alkoxy-(C₁-C₇)-alkoxy-(C₁-C₇)-alkyl, (C₁-C₇)-alcoxy-(C₁-C₇)-alcoxy-(C₁-C₇)-alcoxy-(C₁-C₇)-alkyl, (C₁-C₇)-haloalcoxy-(C₁-C₇)-alkyl, (C₁-C₇)-alkylthio-(C₁-C₇)-alkyl, (C₁-C₇)-haloalkylthio-(C₁-C₇)-alkyl, (C₁-C₇)-alkoxy-(C₁-C₇)-haloalkyl, aryl, aryl-(C₁-C₇)-alkyl, hétéroaryl, hétéroaryl-(C₁-C₇)-alkyl, (C₃-C₇)-cycloalkyl-(C₁-C₇)-alkyl, (C₄-C₇)-cycloalcényle-(C₁-C₇)-alkyle, COR¹⁰, SO₂R¹¹, hétérocycle, (C₁-C₇)-alkoxycarbonyle, bis-[(C₁-C₇)-alkyle]aminocarbonyle-(C₁-C₇)-alkyle, (C₁-C₇)-alkyl-aminocarbonyle-(C₁-C₇)-alkyl, aryl-(C₁-C₇)-alkyl-aminocarbonyle-(C₁-C₇)-alkyl, (C₁-C₇)-alkoxycarbonyle-(C₁-C₇)-alkyle, (C₂-C₇)-alkényloxycarbonyle-(C₁-C₇)-alkyle, (C₂-C₇)-alkynyloxycarbonyle-(C₁-C₇)-alkyle, aryle-(C₁-C₇)-alkoxycarbonyle-(C₁-C₇)-alkyle, hétéroaryle-(C₁-C₇)-alkoxycarbonyle-(C₁-C₇)-alkyle, hétérocyclyle-(C₁-C₇)-alkoxycarbonyle-(C₁-C₇)-alkyle, aryl-(C₁-C₇)-alkoxycarbonyle, hétéroaryle-(C₁-C₇)-alkoxycarbonyle, (C₂-C₇)-alcenyloxycarbonyle, (C₂-C₇)-alkynyloxycarbonyle, hétérocyclyle-(C₁-C₇)-alkyl, ou
R⁸ et R⁹ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R¹⁰ représente l'hydrogène, (C₁-C₇)-alkyl , (C₂-C₇)-alkenyl, (C₂-C₇)-alkinyl, (C₁-C₇)-cyanoalkyl, (C₁-C₇)-haloalkyle, (C₂-C₇)-haloalcényle, (C₃-C₇)-haloalkinyle, (C₃-C₇)-cycloalkyle, (C₃-C₇)-halocycloalkyle, (C₄-C₇)-cycloalkényle, (C₄-C₇)- halocycloalkényle, (C₁-C₇)-alcoxy-(C₁-C₇)-alkyle, (C₁-C₇)-haloalcoxy-(C₁-C₇)-alkyle, (C₁-C₇)-alcoxy-(C₁-C₇)-haloalkyle, (C₁-C₇)-alcoxy-(C₁-C₇)-alcoxy-(C₁-C₇)-alkyle, (C₁-C₇)-alkoxy-(C₁-C₇)-alkoxy-(C₁-C₇)-alkoxy-(C₁-C₇)-alkyl, (C₁-C₇)-alcoxy-(C₁-C₇)-alcoxy-(C₁-C₇)-alcoxy-(C₁-C₇)-alcoxy-(C₁-C₇)-alkyl, aryl, aryl-(C₁-C₇)-alkyl, aryl-(C1-C₇)-alkoxy-(C₁-C₇)-alkyl, aryloxy-(C₁-C₇)-alkyl, hétéroaryl-(C₁-C₇)-alkoxy-(C₁-C₇)-alkyl, hétéroaryloxy-(C₁-C₇)-alkyle, hétéroaryle, hétéroaryle-(C₁-C₇)-alkyle, (C₃-C₇)-cycloalkyle-(C₁-C₇)-alkyle, (C₄-C₇)-cycloalcényle-(C₁-C₇)-alkyle, bis-[(C₁-C₇)-alkyle]aminocarbonyle-(C₁-C₇)-alkyle, (C₁-C₇)-alkyl-aminocarbonyle-(C₁-C₇)-alkyl, aryl-(C₁-C₇)-alkyl-aminocarbonyle-(C₁-C₇)-alkyl, bis-[(C₁-C₇)-alkyl]amino-(C₂C₆)-alkyl, (C₁-C₇)-alkyl-amino-(C₂-C₆)-alkyl, aryl-(C₁-C₇)-alkylamino-(C₂-C₆)-alkyl, R¹¹(O)ₘS-(C₁-C₇)-alkyl, hydroxycarbonyle-(C₁-C₇)-alkyl, hétérocycle, hétérocycle-(C₁-C₇)-alkyl , hétérocycle-(C₁-C₇)-alkyl, hétérocyclyle-(C₁-C₇)-alcoxy-(C₁-C₇)-alkyle, hétérocyclyloxy-(C₁-C₇)-alkyle, tris-[(C₁-C₇)-alkyl]silyl-(C₁-C₇)-alkyle, bis-[(C₁-C₇)-alkyle, etc, bis-[(C₁-C₇)-alkyl](aryl)silyl(C₁-C₇)-alkyl, [(C₁-C₇)-alkyl]-bis-(aryl)silyl-(C₁-C₇)-alkyl, (C₁-C₇)-alkylcarbonyloxy-(C₁-C₇)-alkyl, (C₃-C₇)-cycloalkylcarbonyloxy-(C₁-C₇)-alkyl, arylcarbonyloxy-(C₁-C₇)-alkyl, heteroarylcarbonyloxy-(C₁-C₇)-alkyl, Heterocyclylcarbonyloxy-(C₁-C₇)-alkyl, (C₁-C₇)-alkoxycarbonyle, (C₁-C₇)-alkoxycarbonyle-(C₁-C₇)-alkyle, (C₂-C₇)-alkényloxycarbonyle-(C₁-C₇)-alkyle, (C₂ -C₇)-alkynyloxycarbonyle-(C₁-C₇)-alkyle, aryl-(C₁-C₇)-alkoxycarbonyle-(C₁-C₇)-alkyle, hétéroaryle-(C₁-C₇)-alco-carbonyle-(C₁-C₇)-alkyle, hétérocyclyle-(C₁-C₇)-alco-carbonyle-(C₁-C₇)-alkyle, hydroxy-(C₁-C₇)-alkyle, hydroxy-(C₁-C₇)-alkyle, hydroxy-(C₁-C₇)-alkyle, hydroxy-(C₁-C₇)-alkyle, hydroxy-(C₇)-alkyl, hydroxy-(C₁-C₇)-alkoxy-(C₁-C₇)-alkyl, hydroxy-(C₁-C₇)-alkoxy-(C₁-C₇)-alkoxy-(C₁-C₇)-alkyl, hydroxy-(C₁-C₇)-alkoxy-(C₁-C₇)-alkoxy-(C₁-C₇)-alkoxy-(C₁-C₇)-alkyl , (C₁-C₇)-alkylcarbonyloxy-(C₁-C₇)-alkoxy-(C₁-C₇)-alkyl, (C₁-C₇)-alkylcarbonyloxy-(C₁-C₇)-alkoxy-(C₁-C₇)-alkoxy-(C₁-C₇)-alkyl, (C₁-C₇)-alkylcarbonyloxy-(C₁-C₇)-alkoxy-(C₁-C₇)-alkoxy-(C₁-C₇)-alkoxy-(C₁-C₇)-alkyl, (C₁-C₇)-alkoxycarbonyle-(C₁-C₇)-alkoxy-(C₁-C₇)-alkyle, (C₁-C₇)-alkoxycarbonyle-(C₁-C₇)-alcoxy-(C₁-C₇)-alcoxy-(C₁-C₇)-alkyle, (C₁-C₇)-alkoxycarbonyle-(C₁-C₇)-alkoxy-(C₁-C₇)-alkoxy-(C₁-C₇)-alkoxy-(C₁-C₇)-alkyl, hétéroarylcarbonyloxy-(C₁-C₇)-alkoxy-(C₁-C₇)-alkyl , hétéroarylcarbonyloxy-(C₁-C₇)-alkoxy-(C₁-C₇)-alkoxy-(C₁-C₇)-alkyl, hétéroarylcarbonyloxy-(C₁-C₇)-alkoxy-(C₁-C₇)-alkoxy-(C₁-C₇)-alkoxy-(C₁-C₇)-alkyl, hétérocyclylcarbonyloxy-(C₁-C₇)-alkoxy-(C₁-C₇)-alkyl, hétérocyclylcarbonyloxy-(C₁-C₇)-alkoxy-(C₁-C₇)-alkoxy-(C₁-C₇)-alkyl, hétérocyclylcarbonyloxy-(C₁-C₇)-alkoxy-(C₁-C₇)-alkoxy-(C₁-C₇)-alkoxy-(C₁-C₇)-alkyl, hydroxycarbonyle-(C₁-C₇)-alcoxy-(C₁-C₇)-alkyle, hydroxycarbonyle-(C₁-C₇)-alcoxy-(C₁-C₇)-alcoxy-(C₁-C₇)-alkyle, ou hydroxycarbonyle-(C₁-C₇)-alcoxy-(C₁-C₇)-alcoxy-(C₁-C₇)-alcoxy-(C₁-C₇)-alkyle,
-R¹¹ représente l'hydrogène, (C₁-C₇)-alkyl, (C₂-C₇)-alkenyl, (C₂-C₇)-alkynyl, (C₁-C₇)-cyanoalkyl, (C₁-C₇)-haloalkyl, (C₂-C₇)-haloalkenyl, (C₃-C₇)-halogényle, (C₃-C₇)-cycloalkyle, (C₃-C₇)-halocycloalkyle, (C₄-C₇)-cycloalkényle, (C₄-C₇)-halocycloalkényle, (C₁-C₇)-alcoxy-(C₁-C₇)-alkyle, (C₁-C₇)-alkoxy-(C₁-C₇)-haloalkyle, aryle, aryle-(C₁-C₇)-alkyle, hétéroaryle, hétéroaryle-(C₁-C₇)-alkyle, hétérocyclyle-(C₁-C₇)-alkyle, (C₃-C₇)-cycloalkyle-(C₁-C₇)-alkyle, (C₄-C₁₀)-cycloalcényle-(C₁-C₇)-alkyle, bis-[(C₁-C₇)-alkyl]amino, (C₁-C₇)-alkyl-amino, aryl-(C₁-C₇)-amino, aryl-(C₁-C₆)-alkyl-amino, aryl-[(C₁-C₇)-alkyl]amino ; hétéroaryle-(C₁-C₇)-amino, hétéroaryle-(C₁-C₈)-alkyl-amino, hétéroaryle-[(C₁-C₇)-alkyl]amino ; Hétérocyclyle-(C₁-C₇)-amino, hétérocyclyle-(C₁-C₆)-alkyl-amino, hétérocyclyle-[(C₁-C₇)-alkyl]amino; (C₃-C₇)-cycloalkyl-amino, (C₃-C₇)-cycloalkyl-[(C₁-C₇)-alkyl]amino ; N-azétidinyl, N-pyrrolidinyl, N-piperidinyl, ou N-morpholinyl,
R¹² et R¹³ représentent indépendamment les uns des autres l'hydrogène, NR⁸R⁹, OR¹⁰, ou
R¹² et R¹³ forment, avec l'atome de bore auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire.

3. Composé de formule générale (I) selon la revendication 1 et/ou son sel, **caractérisé en ce que**
R¹ représente un (C₃-C₆)-cycloalkyle, (C₃-C₆)-cycloalkényle, (C₃-C₆)-cycloalkoxy, un aryle, un hétéroaryle, un hétérocyclyle, un résidu bicyclique ou hétérobicyclique, dans lequel chacun des huit résidus mentionnés précédemment est non substitué ou est indépendamment substitué par un ou plusieurs résidus choisis dans le groupe R⁷,
R² représente l'hydrogène, l'halogène, le formyle, l'hydroxy, l'hydrothio, l'hydroxycarbonyle, l'aminocarbonyle, l'aminothiocarbonyle, (C₁-C₆)-alkyl, (C₁-C₆)-haloalkyl, (C₂-C₆)-alcényle, (C₂-C₆)-haloalcényle, (C₂-C₆)-alcynyle, (C₂-C₆)-haloalcynyle, (C₁-C₆)-alcoxy, (C₁-C₆) haloalcoxy, (C₁-C₆)-alcoxy-(C₁-C₆)-alkyle, (C₁-C₆)-alkylthio, (C₁-C₆)-haloalkylthio, (C₁-C₆)-alkylsulfinyle, (C₁-C₆)-haloalkylsulfinyle, (C₁-C₆)-alkylsulfonyle, (C₁-C₆)-haloalkylsulfonyle, (C₁-C₆)-alkylcarbonyle, (C₂-C₆)-alcénylcarbonyle, (C₂-C₆)-alcynylcarbonyle, (C₁-C₆)-haloalkylcarbonyle, (C₂-C₆)-haloalcénylcarbonyle, (C₂-C₆)-haloalcynylcarbonyle, (C₁-C₆)-alkoxycarbonyle, (C₃-C₆)-cycloalkyle, (C₃-C₆)-cycloalcoxy, (C₃-C₆)-cycloalkylthio, (C₃-C₆)-cycloalkylsulfinyle, (C₃-C₆)-cycloalkylsulfonyle, (C₃-C₆)-halocycloalkyle, (C₃-C₆)-halocycloalkoxy, (C₃-C₆)-halocycloalkylthio, (C₃-C₆)-halocycloalkylsulfinyle, (C₃-C₆)-halocycloalkylsulfonyle, (C₃-C₆)-cycloalkyle-(C₁-C₆)-alkyle, (C₁-C₆)-alkyle-(C₃-C₆)-cycloalkyle, (C₃-C₆)-cycloalkylcarbonyle, (C₃-C₆)-cycloalkyle-(C₁-C₆)-alkylcarbonyle, (C₁-C₆)-alkylaminocarbonyle, (C₂-C₁₂)-dialkylaminocarbonyle, (C₁-C₈)-alkylaminosulfonyle, (C₂-C₁₂)-dialkylaminosulfonyle ou tris[(C₁-C₆)alkyl]silyle, ou 2,3,4,5,6-pentafluorophényle,
R³ représente l'hydrogène, l'halogène, le cyano, (C₁-C₆)-alkyl, (C₁-C₆)-haloalkyl, (C₂-C₆)-alkenyl, (C₂-C₆)-haloalkenyl, (C₁-C₆)-alkoxy, (C₁-C₆) haloalkoxy, (C₁-C₆)-alkylthio, (C₁-C₆)-haloalkylthio, (C₃-C₆)-cycloalkyle, (C₃-C₆)-halocycloalkyle, (C₃-C₆)-cycloalkoxy ou (C₃-C₆)-halocycloalkoxy,
R⁴ et R⁵ représentent indépendamment l'un de l'autre l'hydrogène, (C₁-C₆)-alkyl, (C₁-C₆)-haloalkyl, (C₃-C₆)-cycloalkyl, (C₃-C₆)-halocycloalkyl, ou R¹⁰O-(C₁-C₆)-alkyl, ou
R⁴ et R⁵ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R⁶ représente l'hydrogène, (C₁-C₆)-alkyl, (C₂-C₆)-alkenyl, (C₂-C₆)-alkynyl, cyano-(C₁-C₆)-alkyl, R¹⁰O-(C₁-C₆)-alkyl, R⁸R⁹N-(C₁-C₆)-alkyl, NR⁸R⁹, (C₃-C₁₀)-cycloalkyle-(C₁-C₆)-alkyle, C(=O)R¹⁰, C(=O)OR¹⁰, C(=O)NR⁸R⁹, R¹⁰O(O)C-(C₁-C₆)-alkyl, R¹⁰(O=)C- (C₁-C₆)-Alkyl, R⁸R⁹N(O)C-(C₁-C₆)-alkyl, SO₂R¹¹, R¹¹S(O)ₘ-(C₁-C₆)-alkyl, BR¹²R¹³, hétérocyclyle, hétéroaryle, aryle-(C₁-C₆)-alkyl, hétéroaryle-(C₁-C₆)-alkyl, hétérocyclyle-(C₁-C₆)-alkyl, tris[(C₁-C₆)alkyl]silyl, bis-[(C₁-C₆)-alkyl](aryl)silyl, (C₁-C₆)-alkyl-[bis-(aryl)]silyl, tris-[(C₁-C₆)-alkyl]silyl-(C₁-C₆)-alkyl, bis-[(C₁-C₆)-alkyl](aryl)silyl(C₁-C₆)-alkyl, ou (C₁-C₆)-alkyl-[bis-(aryl)]silyl-(C₁-C₆)-alkyl,
-m est 0, 1, 2,
n est 0, 1, 2,
R⁷ représente l'hydrogène, l'halogène, le formyle, l'hydroxy, l'hydroxycarbonyle, le nitro, le cyano, l'amino, l'aminocarbonyle, l'aminothiocarbonyle, (C₁-C₆)-alkyl, (C₁-C₆)-haloalkyl, (C₂-C₆)-alcényle, (C₂-C₆)-haloalcényle, (C₂-C₆)-alcynyle, (C₂-C₆)-haloalcynyle, (C₁-C₆)-alcoxy, (C₁-C₆)-haloalcoxy, (C₁-C₆)-alkylthio, (C₁-C₆)-haloalkylthio, (C₁-C₆)-alkylsulfinyl, (C₁-C₆)-haloalkylsulfinyle, (C₁-C₆)-alkylsulfonyle, (C₁-C₆)-haloalkylsulfonyle, (C₁-C₆)-alkylcarbonyle, (C₁-C₆)-haloalkylcarbonyle, (C₂-C₆)-alcénylcarbonyle, (C₂-C₆)-haloalcénylcarbonyle, (C₂-C₆)-alcynylcarbonyle, (C₂-C₆)-haloalcynylcarbonyle, (C₁-C₆)-alcoxycarbonyle, (C₁-C₆)-haloalcoxycarbonyle, (C₃-C₆)-cycloalkyle, (C₃-C₆)-halocycloalkyle, (C₃-C₆)-cycloalkoxy, (C₃-C₆)-halocycloalkoxy, (C₃-C₆)-cycloalkylthio, (C₃-C₆)-halocycloalkylthio, (C₃-C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyle-(C₁-C₆)-alkyle, (C₁-C₆)-alkyle-(C₃-C₆)-cycloalkyle, (C₁-C₆)-alkoxycarbonyle-(C₁-C₆)-al kyl, hydroxycarbonyle-(C₁-C₆)-alkyl, (C₃-C₆)-cycloalkylcarbonyle, (C₃-C₆)-cycloalkyl-(C₁-C₆)-alkylcarbonyle, (C₁-C₆)-alkylamino, (C₂-C₁₂)-dialkylamino, (C₁-C₆)-alkylaminocarbonyle, (C₂-C₁₂)-dialkylaminocarbonyle, (C₁-C₆)-alkylaminosulfonyle, (C₂-C₁₂)-dialkylaminosulfonyle ou tris[(C₁-C₆)alkyl]silyle,
R⁸ et R⁹ représentent indépendamment des autres l'hydrogène, (C₁-C₆)-alkyl, (C₂-C₆)-alkenyl, (C₂-C₆)-alkynyl, (C₁-C₆)-cyanoalkyle, (C₁-C₆)-haloalkyle, (C₂-C₆)-haloalcényle, (C₃-C₆)-haloalcynyle, (C₃-C₆)-cycloalkyle, (C₃-C₆)-halocycloalkyle, (C₄-C₆)-cycloalcényle, (C₄-C₆)-halocycloalcényle, (C₁-C₆)-alkoxy-(C₁-C₆)-alkyl, (C₁-C₆)-alkoxy-(C₁-C₆)-alkoxy-(C₁-C₆)-alkyl, (C₁-C₆)-alcoxy-(C₁-C₆)-alcoxy-(C₁-C₆)-alcoxy-(C₁-C₆)-alkyl, (C₁-C₆)-haloalkoxy-( C₁-C₆)-alkyl, (C₁-C₆)-alkylthio-(C₁-C₆)- alkyl, (C₁-C₆)-haloalkylthio-(C₁-C₆)-alkyl, (C₁-C₆)-alkoxy-(C₁-C₆)-haloalkyl, aryl, aryl-(C₁-C₆)-alkyl, hétéroaryl, hétéroaryl-(C₁-C₆)-alkyl, (C₃-C₆)-cycloalkyl-(C₁-C₆)-alkyl, (C₄-C₆)-cycloalcényle-(C₁-C₆)-alkyle, COR¹⁰, SO₂R¹¹, hétérocycle, (C₁-C₆)-alkoxycarbonyle, bis-[(C₁-C₆)-alkyle]aminocarbonyle-(C₁-C₆)-alkyle, (C₁-C₆)-alkyl-aminocarbonyle-(C₁-C₆)-alkyl, aryl-(C₁-C₆)-alkyl-aminocarbonyle-(C₁-C₆)-alkyl, (C₁-C₆)-alkoxycarbonyle-(C₁-C₆)-alkyle, (C₂-C₆)-alkényloxycarbonyle-(C₁-C₆)-alkyle, (C₂-C₆)-alkynyloxycarbonyle-(C₁-C₆)-alkyle, aryle-(C₁-C₆)-alkoxycarbonyle-(C₁-C₆)-alkyle, hétéroaryle-(C₁-C₆)-alkoxycarbonyle-(C₁-C₆)-alkyle, hétérocyclyle-(C₁-C₆)-alkoxycarbonyle-(C₁-C₆)-alkyle, aryl-(C₁-C₆)-alkoxycarbonyle, hétéroaryle-(C₁-C₆)-alkoxycarbonyle, (C₂-C₆)-alcenyloxycarbonyle, (C₂-C₆)-alkynyloxycarbonyle, hétérocyclyle-(C₁-C₆)-alkyl, ou
-R⁸ et R⁹ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R¹⁰ représente l'hydrogène, (C₁-C₆)-alkyl, (C₂-C₆)-alkenyl, (C₂-C₆)-alkinyl, (C₁-C₆)-cyanoalkyl, (C₁-C₆)-haloalkyle, (C₂-C₆)-haloalcényle, (C₃-C₆)-haloalkinyle, (C₃-C₆)-cycloalkyle, (C₃-C₆)-halocycloalkyle, (C₄-C₆)-cycloalkényle, (C₄-C₆)-halocycloalkényle, (C₁-C₆)-alcoxy-(C₁-C₆)-alkyle, (C₁-C₆)-haloalcoxy-(C₁-C₆)-alkyle, (C₁-C₆)-alcoxy-(C₁-C₆)-haloalkyle, (C₁-C₆)-alcoxy-(C₁-C₆)-alcoxy-(C₁-C₆)-alkyle, (C₁-C₆)-alcoxy-(C₁-C₆)-alcoxy-(C₁-C₆)-alcoxy-(C₁-C₆)-alkyl, (C₁-C₆)-alkoxy-(C₁-C₆)-alkoxy-(C₁-C₆)-alkoxy-(C₁-C₆)-alkoxy-(C₁-C₆)-alkyl, aryl, aryl-(C₁-C₆)-alkyl, aryl-(C₁-C₆)-alkoxy-(C₁-C₆)-alkyl, aryloxy-(C₁-C₆)-alkyl, hétéroaryl-(C₁-C₆)-alkoxy-(C₁-C₆)-alkyl, hétéroaryloxy-(C₁-C₆)-alkyle, hétéroaryle, hétéroaryle-(C₁-C₆)-alkyle, (C₃-C₆)-cycloalkyle-(C₁-C₆)-alkyle, (C₄-C₆)-cycloalcényle-(C₁-C₆)-alkyle, bis-[(C₁-C₆)-alkyle]aminocarbonyle-(C₁-C₆)-alkyle, (C₁-C₆)-alkyl-aminocarbonyle-(C₁-C₆)-alkyl, aryl-(C₁-C₆)-alkyl-aminocarbonyle-(C₁-C₆)-alkyl, bis-[(C₁-C₆)-alkyl]amino-(C₂-C₆)-alkyl, (C₁-C₆)-alkyl-amino-(C₂-C₆)-alkyl, aryl-(C₁-C₆)-alkylamino-(C₂-C₆)-alkyl, R¹¹(O)ₘS-(C₁-C₆)-alkyl, hydroxycarbonyle-(C₁-C₆)-alkyl, hétérocycle, hétérocycle-(C₁-C₆)-alkyl, hétérocycle-(C₁-C₆)-alkyl, hétérocyclyle-(C₁-C₆)-alcoxy-(C₁-C₆)-alkyle, hétérocyclyloxy-(C₁-C₆)-alkyle, tris-[(C₁-C₆)-alkyl]silyl-(C₁-C₆)-alkyle, bis-[(C₁-C₆)-alcoyl-(C₁-C₆)-alcoyl-(C₁-C₆)-Alcoyl) bis-[(C₁-C₆)-alkyl](aryl)silyl(C₁-C₆)-alkyl, [(C₁-C₆)-alkyl]-bis-(aryl)silyl-(C₁-C₆)-alkyl, (C₁-C₆)-alkylcarbonyloxy-(C₁-C₆)-alkyl, (C₃-C₆)-cycloalkylcarbonyloxy-(C₁-C₆)-alkyl, arylcarbonyloxy-(C₁-C₆)-alkyl, heteroarylcarbonyloxy-(C₁-C₆)-alkyl, Heterocyclylcarbonyloxy-(C₁-C₆)-alkyl, (C₁-C₆)-alkoxycarbonyle, (C₁-C₆)-alkoxycarbonyle-(C₁-C₆)-alkyle, (C₂-C₆)-alkényloxycarbonyle-(C₁-C₆)-alkyle, (C₂-C₆)-alkynyloxycarbonyle-(C₁-C₆)-alkyle, aryle-(C₁-C₆)-alkoxycarbonyle-(C₁-C₆)-alkyle, hétéroaryle-(C₁-C₆)-alco-carbonyle-(C₁-C₆)-alkyle, hétérocyclyle-(C₁-C₆)-alco-carbonyle-(C₁-C₆)-alkyle, hydroxy-(C₁-C₆)-alkyle, hydroxy-(C₁-C₆)-alkyle, hydroxy-(C₁-C₆)-alkyle, hydroxy-(C₁-C₆)-alkyle, hydroxy-(C₁-C₆)-alkoxy-(C₁-C₆)-alkyl, hydroxy-(C₁-C₆)-alkoxy-(C₁-C₆)-alkoxy-(C₁-C₆)-alkyl, hydroxy-(C₁-C₆)-alkoxy-(C₁-C₆)-alkoxy-(C₁-C₆)-alkoxy-(C₁-C₆)-alkyl, (C₁-C₆)-alkylcarbonyloxy-(C₁-C₆)-alkoxy-(C₁-C₆)-alkyl, (C₁-C₆)-alkylcarbonyloxy-(C₁-C₆)-alkoxy-(C₁-C₆)-alkoxy-(C₁-C₆)-alkyl, (C₁-C₆)-alkylcarbonyloxy-(C₁-C₆)-alkoxy-(C₁-C₆)-alkoxy-(C₁-C₆)-alkoxy-(C₁-C₆)-alkyl, (C₁-C₆)-alkoxycarbonyle-(C₁-C₆)-alkoxy-(C₁-C₆)-alkyle, (C₁-C₆)-alkoxycarbonyle-(C₁-C₆)-alcoxy-(C₁-C₆)-alcoxy-(C₁-C₆)-alkyle, (C₁-C₆)-alkoxycarbonyle-(C₁-C₆)-alkoxy-(C₁-C₆)-alkoxy-(C₁-C₆)-alkoxy-(C₁-C₆)-alkyl, hétéroarylcarbonyloxy-(C₁-C₆)-alkoxy-(C₁-C₆)-alkyl, hétéroarylcarbonyloxy-(C₁-C₆)-alkoxy-(C₁-C₆)-alkoxy-(C₁-C₆)-alkyl, hétéroarylcarbonyloxy-(C₁-C₆)-alkoxy-(C₁-C₆)-alkoxy-(C₁-C₆)-alkoxy-(C₁-C₆)-alkyl, hétérocyclylcarbonyloxy-(C₁-C₆)-alkoxy-(C₁-C₆)-alkyl, hétérocyclylcarbonyloxy-(C₁-C₆)-alkoxy-(C₁-C₆)-alkoxy-(C₁-C₆)-alkyl, hétérocyclylcarbonyloxy-(C₁-C₆)-alkoxy-(C₁-C₆)-alkoxy-(C₁-C₆)-alkoxy-(C₁-C₆)-alkyl, hydroxycarbonyle-(C₁-C₆)-alcoxy-(C₁-C₆)-alkyle, ou hydroxycarbonyle-(C₁-C₆)-alcoxy-(C₁-C₆)-alcoxy-(C₁-C₆)-alkyle, hydroxycarbonyle-(C₁-C₆)-alcoxy-(C₁-C₆)-alcoxy-(C₁-C₆)-alcoxy-(C₁-C₆)-alkyle,
R¹¹ représente l'hydrogène, (C₁-C₆)-alkyl, (C₂-C₆)-alkenyl, (C₂-C₆)-alkynyl, (C₁-C₆)-cyanoalkyl, (C₁-C₆)-haloalkyl, (C₂-C₆)-haloalkenyl, (C₃-C₆)-halogényle, (C₃-C₆)-cycloalkyle, (C₃-C₆)-halocycloalkyle, (C₄-C₆)-cycloalkényle, (C₄-C₆)-halocycloalkényle, (C₁-C₆)-alcoxy-(C₁-C₆)-alkyle, (C₁-C₆)-alkoxy-(C₁-C₆)-haloalkyle, aryle, aryle-(C₁-C₆)-alkyle, hétéroaryle, hétéroaryle-(C₁-C₆)-alkyle, hétérocyclyle-(C₁-C₆)-alkyle, (C₃-C₆)-cycloalkyle-(C₁-C₆)-alkyle, (C₄-C₁₀)-cycloalcényle-(C₁-C₆)-alkyle, bis-[(C₁-C₆)-alkyl]amino, (C₁-C₆)-alkyl-amino, aryl-(C₁-C₆)-amino, aryl-(C₁- C₆)-alkyl-amino, aryl-[(C₁-C₆)-alkyl]amino ; hétéroaryle-(C₁-C₆)-amino, hétéroaryle-(C₁-C₆)-alkyl-amino, hétéroaryle-[(C₁-C₆)-alkyl]amino ; Hétérocyclyle-(C₁-C₆)-amino, hétérocyclyle-(C₁-C₆)-alkyl-amino, hétérocyclyle-[(C₁-C₆)-alkyl]amino ; (C₃-C₆)-cycloalkyl-amino, (C₃-C₆)-cycloalkyl-[(C₁-C₆)-alkyl]amino ; N-azétidinyl, N-pyrrolidinyl, N-piperidinyl, ou N-morpholinyl,
-R¹² et R¹³ représentent indépendamment les uns des autres l'hydrogène, NR⁸R⁹, OR¹⁰, ou
R¹² et R¹³ forment, avec l'atome de bore auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire.

4. Composé de formule générale (I) selon la revendication 1 et/ou son sel, **caractérisé en ce que**
R¹ représente un phényle, un furyle, un pyrrolyle, un thiényle, un pyridyle, un pyrimidinyle, un pyridazinyle, un pyrazinyle, un thiazolyle, un isothiazolyle, un thiadiazolyle, un oxazolyle, un isoxazolyle, un pyrazolyle, un imidazolyle, un triazolyle, un tétrazolyle, un cyclopentényle, un cyclohexényle ou un résidu oxabicycloheptanyle, cyclohexényle ou un résidu oxabicycloheptanyl, caratérisée **en ce que** chacun des 20 résidus mentionnés précédemment est non substitué ou est indépendamment substitué par un ou plusieurs résidus choisis dans le groupe R⁷,
R² représente l'hydrogène, le fluor, le chlore, le brome, l'iode, le formyle, l'hydroxy, l'hydrothio, l'hydroxycarbonyle, l'aminocarbonyle, l'aminothiocarbonyle, (C₁-C₅)-alkyl, (C₁-C₅)-haloalkyl, (C₂-C₆)-alcényle, (C₂-C₅)-haloalcényle, (C₂-C₅)-alcynyle, (C₂-C₅)-haloalcynyle, (C₁-C₅)-alcoxy, (C₁-C₅)-haloalcoxy, (C₁-C₅)-alcoxy-(C₁-C₅)-alkyle, (C₁-C₆)-alkylthio, (C₁-C₅)-haloalkylthio, (C₁-C₅)-alkylsulfinyl, (C₁-C₅)-haloalkylsulfinyl, (C₁-C₅)-alkylsulfonyl, (C₁-C₅)-haloalkylsulfonyl, (C₁-C₅)-alkylcarbonyle, (C₂-C₅)-alcénylcarbonyle, (C₂-C₅)-alcynylcarbonyle, (C₁-C₅)-haloalkylcarbonyle, (C₂-C₅)-haloalcénylcarbonyle, (C₂-C₅)-haloalcynylcarbonyle, (C₁-C₆)-alkoxycarbonyle, (C₃-C₆)-cycloalkyle, (C₃-C₆)-cycloalkoxy, (C₃-C₆)-cycloalkylthio, (C₃-C₆)-cycloalkylsulfinyle, (C₃-C₆)-cycloalkylsulfonyle, (C₃-C₆)-halocycloalkyle, (C₃-C₅)-halocycloalkoxy, (C₃-C₅)-halocycloalkylthio, (C₃-C₅)-halocycloalkylsulfinyle, (C₃-C₅)-halocycloalkylsulfonyle, (C₃-C₆)-cycloalkyle-(C₁-C₅)-alkyle, (C₁-C₅)-alkyle-(C₃-C₆)-cycloalkyle, (C₃-C₆)-cycloalkylcarbonyle, (C₃-C₆)-cycloalkyle-(C₁-C₅)-alkylcarbonyle, (C₁-C₅)-alkylaminocarbonyle, (C₂-C₁₀)-dialkylaminocarbonyle, (C₁-C₅)-alkylaminosulfonyle, (C₂-C₁₀)-dialkylaminosulfonyle ou tris[(C₁-C₅)alkyl]silyl, 2,3,4,5,6-pentafluorophényle,
R³ représente l'hydrogène, le fluoro, le chloro, le bromo, l'iodo, le cyano, (C₁-C₅)-alkyl, (C₁-C₅)-haloalkyl, (C₂-C₅)-alcényle, (C₂-C₅)-haloalcényle, (C₁-C₅)-alkoxy, (C₁-Cs) haloalkoxy, (C₁-C₆)-alkylthio, (C₁-C₅)-haloalkylthio, (C₃-C₆)-cycloalkyle, (C₃-C₅)-halocycloalkyle, (C₃-C₆)-cycloalkoxy ou (C₃-C₆)-halocycloalkoxy,
R⁴ et R⁵ représentent indépendamment l'un de l'autre l'hydrogène, (C₁-C₅)-alkyl, (C₁-C₅)-haloalkyl, (C₃-C₆)-cycloalkyl, (C₃-C₆)-halocycloalkyl, ou R¹⁰O-(C₁-C₅)-alkyl, ou
R⁴ et R⁵ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R⁶ représente l'hydrogène, (C₁-C₅)-alkyl, (C₂-C₅)-alkenyl, (C₂-C₅)-alkynyl, cyano-(C₁-Cs)-alkyl, R¹⁰O-(C₁-C₅)-alkyl, R⁸R⁹N-(C₁-C₅)-alkyl, NR⁸R⁹, (C₃-C₆)-cycloalkyle-(C₁-C₅)-alkyle, C(=O)R¹⁰, C(=O)OR¹⁰, C(=O)NR⁸R⁹, R¹⁰O(O)C-(C₁-C₅)-alkyl, R¹⁰(O=)C-(C₁-C₅)-Alkyl, R⁸R⁹N(O)C-(C₁-C₅)-alkyl, SO₂R¹¹, R¹¹S(O)ₘ-(C₁-C₅)-alkyl, BR¹²R¹³, hétérocyclyle, hétéroaryle, aryle-(C₁-C₅)-alkyl, hétéroaryle-(C₁-C₅)-alkyl, hétérocyclyle-(C₁-C₅)-alkyl, tris[(C₁-C₅)alkyl]silyl, bis-[(C₁-C₅)-alkyl](aryl)silyl, (C₁-Cs)-alkyl-[bis-(aryl)]silyl, tris-[(C₁-C₅)-alkyl]silyl-(C₁-C₅)-alkyl, bis-[(C₁-C₅)-alkyl](aryl)silyl(C₁-C₅)-alkyl, ou (C₁-C₅)-alkyl-[bis-(aryl)]silyl-(C₁-C₅)-alkyl,
m est 0, 1, 2,
n est 0, 1, 2,
R⁷ représente l'hydrogène, le fluoro, le chloro, le bromo, l'iodo, le formyle, l'hydroxy, l'hydroxycarbonyle, le nitro, le cyano, l'amino, l'aminocarbonyle, l'aminothiocarbonyle, (C₁-C₅)-alkyl, (C₁-C₅)-haloalkyl, (C₂-C₅)-alcényle, (C₂-C₅)-haloalcényle, (C₂-C₅)-alcynyle, (C₂-C₅)-haloalcynyle, (C₁-C₅)-alkoxy, (C₁-C₅)-haloalcoxy, (C₁-C₆)-alkylthio, (C₁-C₅)-haloalkylthio, (C₁-C₅)-alkylsulfinyle, (C₁-C₅)-haloalkylsulfinyle, (C₁-C₅)-alkylsulfonyle, (C₁-C₅)-haloalkylsulfonyle, (C₁-C₅)-alkylcarbonyle, (C₁-C₅)-haloalkylcarbonyle, (C₂-C₅)-alcénylcarbonyle, (C₂-C₅)-haloalcénylcarbonyle, (C₂-C₅)-alcynylcarbonyle, (C₂-C₅)-haloalcynylcarbonyle, (C₁-C₅)-alcoxycarbonyle, (C₁-C₅)-haloalcoxycarbonyle, (C₃-C₆)-cycloalkyle, (C₃-C₆)-halocycloalkyle, (C₃-C₆)-cycloalkoxy, (C₃-C₆)-halocycloalkoxy, (C₃-C₆)-cycloalkylthio, (C₃-C₆)-halocycloalkylthio, (C₃-C₆)-cycloalkylsulfinyl, (C₃-C₆)-halocycloalkylsulfinyl, (C₃-C₆)-cycloalkylsulfonyl, (C₃-C₆)-halocycloalkylsulfonyl, (C₃-C₆)-cycloalkyle-(C₁-C₅)-alkyle, (C₁-C₅)-alkyle-(C₃-C₆)-cycloalkyle, (C₁-C₅)-alkoxycarbonyle-(C₁-C₅)-alkyl, hydroxycarbonyle-(C₁-C₅)-alkyl, (C₃-C₆)-cycloalkylcarbonyle, (C₃-C₆)-cycloalkyl-(C₁-C₅)-alkylcarbonyle, (C₁-C₅)-alkylamino, (C₂-C₁₀)-dialkylamino, (C₁-C₅)-alkylaminocarbonyle, (C₂-C₁₀)-dialkylaminocarbonyle, (C₁-C₅)-alkylaminosulfonyle, (C₂-C₁₀)-dialkylaminosulfonyle ou tris[(C₁-C₅)alkyl]silyle,
R⁸ et R⁹ représentent indépendamment des autres l'hydrogène, (C₁-C₅)-alkyl, (C₂-C₅)-alkenyl, (C₂-C₅)-alkynyl, (C₁-C₅)-cyanoalkyle, (C₁-C₅)-haloalkyle, (C₂-C₅)-haloalcényle, (C₃-C₅)-haloalcynyle, (C₃-C₆)-cycloalkyle, (C₃-C₆)-halocycloalkyle, (C₄-C₆)-cycloalcényle, (C₄-C₆)-halocycloalcényle, (C₁-C₅)-alkoxy-(C₁-C₅)-alkyl, (C₁-C₅)-alkoxy-(C₁-C₅)-alkoxy-(C₁-C₅)-alkyl, (C₁-C₅)-alcoxy-(C₁-C₅)-alcoxy-(C₁-C₅)-alcoxy-(C₁-C₅)-alkyl, (C₁-C₅)-haloalcoxy-(C₁-C₅)-alkyl, (C₁-C₅)-alkylthio-(C₁-C₅)-alkyl, (C₁-C₅)-haloalkylthio-(C₁-C₅)-alkyl, (C₁-C₅)-alkoxy-(C₁-C₅)-haloalkyl, aryl, aryl-(C₁-C₆)-alkyl, hétéroaryl, hétéroaryl-(C₁-C₅)-alkyl, (C₃-C₆)-cycloalkyl-(C₁-C₅)-alkyl, (C₄-C₆)-cycloalcényle-(C₁-C₅)-alkyle, COR¹⁰, SO₂R¹¹, hétérocycle, (C₁-C₅)-alkoxycarbonyle, bis-[(C₁-C₅)-alkyle]aminocarbonyle-(C₁-C₅)-alkyle, (C₁-C₅)-alkyl-aminocarbonyle-(C₁-C₅)-alkyl, aryl-(C₁-C₅)-alkyl-aminocarbonyle-(C₁-C₅)-alkyl, (C₁-C₅)-alkoxycarbonyle-(C₁-C₅)-alkyle, (C₂-C₅)-alkényloxycarbonyle-(C₁-C₅)-alkyle, (C₂-C₅)-alkynyloxycarbonyle-(C₁-C₅)-alkyle, aryl-(C₁-C₅)-alkoxycarbonyle-(C₁-C₅)-alkyle, hétéroaryle-(C₁-C₅)-alkoxycarbonyle-(C₁-C₅)-alkyle, hétérocyclyle-(C₁-C₅)-alkoxycarbonyle-(C₁-C₅)-alkyle, aryl-(C₁-C₅)-alkoxycarbonyle, hétéroaryle-(C₁-C₆)-alkoxycarbonyle, (C₂-C₅)-alcenyloxycarbonyle, (C₂-C₅)-alkynyloxycarbonyle, hétérocyclyle-(C₁-C₅)-alkyl, ou
R⁸ et R⁹ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire,
R¹⁰ représente l'hydrogène, (C₁-C₅)-alkyl, (C₂-C₅)-alkenyl, (C₂-C₅)-alkinyl, (C₁-C₅)-cyanoalkyl, (C₁-C₅)-haloalkyle, (C₂-C₅)-haloalcényle, (C₃-C₅)-haloalkinyle, (C₃-C₆)-cycloalkyle, (C₃-C₆)-halocycloalkyle, (C₄-C₆)-cycloalkényle, (C₄-C₆)-halocycloalkényle, (C₁-C₅)-alcoxy-(C₁-C₅)-alkyle, (C₁-C₅)-haloalcoxy-(C₁-C₅)-alkyle, (C₁-C₅)-alcoxy-(C₁-C₅)-haloalkyle, (C₁-C₅)-alcoxy-(C₁-C₅)-alcoxy-(C₁-C₅)-alkyle, (C₁-C₅)-alcoxy-(C₁-C₅)-alcoxy-(C₁-C₅)-alcoxy-(C₁-C₅)-alkyl, (C₁-C₅)-alcoxy-(C₁-C₅)-alcoxy-(C₁-C₅)-alcoxy-(C₁-C₅)-alcoxy-(C₁-C₅)-alkyl, aryl, aryl-(C₁-C₆)-alkyl, aryl-(C₁-C₅)-alkoxy-(C₁-C₅)-alkyl, aryloxy-(C₁-C₅)-alkyl, hétéroaryl-(C₁-C₅)-alkoxy-(C₁-C₅)-alkyl, hétéroaryloxy-(C₁-C₅)-alkyle, hétéroaryle, hétéroaryle-(C₁-C₅)-alkyle, (C₃-C₆)-cycloalkyle-(C₁-C₅)-alkyle, (C₄-C₆)-cycloalcényle-(C₁-C₅)-alkyle, bis-[(C₁-C₅)-alkyle]aminocarbonyle-(C₁-C₅)-alkyle, (C₁-C₅)-alkyl-aminocarbonyle-(C₁-C₅)-alkyl, aryl-(C₁-C₅)-alkyl-aminocarbonyle-(C₁-C₅)-alkyl, bis-[(C₁-C₅)-alkyl]amino-(C₂-C₅)-alkyl, (C₁-C₅)-alkyl-amino-(C₂-C₅)-alkyl, aryl-(C₁-C₅)-alkylamino-(C₂-C₅)-alkyl, R¹¹(O)ₘS-(C₁-C₅)-alkyl, hydroxycarbonyle-(C₁-C₅)-alkyl, hétérocycle, hétérocycle-(C₁-C₅)-alkyl, hétérocycle-(C₁-C₅)-alkyl, hétérocyclyle-(C₁-C₅)-alcoxy-(C₁-C₅)-alkyle, hétérocyclyloxy-(C₁-C₅)-alkyle, tris-[(C₁-C₅)-alkyl]silyl-(C₁-C₅)-alkyle, bis-[(C₁-C₅)-alcoyl-(C₁-C₅)-alcoyl-(C₁-C₅)-alcoyl-), bis-[(C₁-C₅)-alkyl](aryl)silyl(C₁-C₅)-alkyl, [(C₁-C₅)-alkyl]-bis-(aryl)silyl-(C₁-C₅)-alkyl, (C₁-C₅)-alkylcarbonyloxy-(C₁-C₅)-alkyl, (C₃-C₆)-cycloalkylcarbonyloxy-(C₁-C₅)-alkyl, arylcarbonyloxy-(C₁-C₅)-alkyl, heteroarylcarbonyloxy-(C₁-C₅)-alkyl, Heterocyclylcarbonyloxy-(C₁-C₅)-alkyl, (C₁-C₅)-alkoxycarbonyle, (C₁-C₅)-alkoxycarbonyle-(C₁-C₅)-alkyle, (C₂-C₅)-alkényloxycarbonyle-(C₁-C₅)-alkyle, (C₂-C₅)-alkynyloxycarbonyle-(C₁-C₅)-alkyle, aryl-(C₁-C₅)-alkoxycarbonyle-(C₁-C₅)-alkyle, hétéroaryle-(C₁-C₅)-alco-carbonyle-(C₁-C₅)-alkyle, hétérocyclyle-(C₁-C₅)-alco-carbonyle-(C₁-C₅)-alkyle, hydroxy-(C₁-C₅)-alkyle, hydroxy-(C₁-C₅)-alkyle, hydroxy-(C₁-C₅)-alkyle, hydroxy-(C₁-C₅)-alkyle, hydroxy-(Cs)-alkyl, hydroxy-(C₁-C₅)-alkoxy-(C₁-C₅)-alkyl, hydroxy-(C₁-C₅)-alkoxy-(C₁-C₅)-alkoxy-(C₁-C₅)-alkyl, hydroxy-(C₁-C₅)-alkoxy-(C₁-C₅)-alkoxy-(C₁-C₅)-alkoxy-(C₁-C₅)-alkyl, (C₁-C₅)-alkylcarbonyloxy-(C₁-C₅)-alkoxy-(C₁-C₅)-alkyl, (C₁-C₅)-alkylcarbonyloxy-(C₁-C₅)-alkoxy-(C₁-C₅)-alkoxy-(C₁-C₅)-alkyl, (C₁-C₅)-alkylcarbonyloxy-(C₁-C)-alkoxy-(C₁-C₅)-alkoxy-(C₁-C₅)-alkoxy-(C₁-C₅)-alkyl, (C₁-C₅)-alkoxycarbonyle-(C₁-C₅)-alkoxy-(C₁-C₅)-alkyle, (C₁-C₅)-alkoxycarbonyle-(C₁-C₅)-alcoxy-(C₁-C₅)-alcoxy-(C₁-C₅)-alkyle, (C₁-C₅)-alkoxycarbonyle-(C₁-C₅)-alkoxy-(C₁-C₅)-alkoxy-(C₁-C₅)-alkoxy-(C₁-C₅)-alkyl, hétéroarylcarbonyloxy-(C₁-C₅)-alkoxy-(C₁-C₅)-alkyl, hétéroarylcarbonyloxy-(C₁-C₅)-alkoxy-(C₁-C₅)-alkoxy-(C₁-C₅)-alkyl, hétéroarylcarbonyloxy-(C₁-C₅)-alkoxy-(C₁-C₅)-alkoxy-(C₁-C₅)-alkoxy-(C₁-C₅)-alkyl, hétérocyclylcarbonyloxy-(C₁-C₅)-alkoxy-(C₁-C₅)-alkyl, hétérocyclylcarbonyloxy-(C₁-C₅)-alkoxy-(C₁-C₅)-alkoxy-(C₁-C₅)-alkyl, hétérocyclylcarbonyloxy-(C₁-C₅)-alkoxy-(C₁-C₅)-alkoxy-(C₁-C₅)-alkoxy-(C₁-C₅)-alkyl, hydroxycarbonyle-(C₁-C₅)-alcoxy-(C₁-C₅)-alkyle, hydroxycarbonyle-(C₁-C₅)-alcoxy-(C₁-C₅)-alcoxy-(C₁-C₅)-alkyle, ou hydroxycarbonyle-(C₁-C₅)-alcoxy-(C₁-C₅)-alcoxy-(C₁-C₅)-alcoxy-(C₁-C₅)-alkyle,
R¹¹ représente l'hydrogène, (C₁-C₅)-alkyl, (C₂-C₅)-alkenyl, (C₂-C₅)-alkynyl, (C₁-C₅)-cyanoalkyl, (C₁-C₅)-haloalkyl, (C₂-C₅)-haloalkenyl, (C₃-C₅)-halogényle, (C₃-C₆)-cycloalkyle, (C₃-C₆)-halocycloalkyle, (C₄-C₆)-cycloalkényle, (C₄-C₆)-halocycloalkényle, (C₁-C₅)-alcoxy-(C₁-C₅)-alkyle, (C₁-C₅)-alcoxy-(C₁-C₅)-haloalkyle, aryle, aryle-(C₁-C₅)-alkyle, hétéroaryle, hétéroaryle-(C₁-C₅)-alkyle, hétérocyclyle-(C₁-C₅)-alkyle, (C₃-C₆)-cycloalkyle-(C₁-C₅)-alkyle, (C₄-C₁₀)-cycloalcényle-(C₁-C₅)-alkyle, bis-[(C₁-C₅)-alkyl]amino, (C₁-C₅)-alkyl-amino, aryl-(C₁-C₅)-amino, aryl-(C₁-Cs)-alkyl-amino, aryl-[(C₁-C₅)-alkyl]amino ; hétéroaryle-(C₁-C₅)-amino, hétéroaryle-(C₁-C₅)-alkyl-amino, hétéroaryle-[(C₁-C₅)-alkyl]amino ; Hétérocyclyle-(C₁-C₅)-amino, hétérocyclyle-(C₁-C₅)-alkyl-amino, hétérocyclyle-[(C₁-C₅)-alkyl]amino ; (C₃-C₆)-cycloalkyl-amino, (C₃-C₆)-cycloalkyl-[(C₁-C₅)-alkyl]amino ; N-azétidinyl, N-pyrrolidinyl, N-piperidinyl, N-morpholinyl.
R¹² et R¹³ représentent indépendamment les uns des autres l'hydrogène, NR⁸R⁹, OR¹⁰, ou
R¹² et R¹³ forment, avec l'atome de bore auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire.

5. Le composé de formule générale (I) selon la revendication 1 et/ou son sel, **caractérisé en ce que**
R¹ représente le phényle, le 2-fluorophényle, le 3-fluorophényle, le 4-fluorophényle, le 2-chlorophényle, le 3-chlorophényle, le 4-chlorophényle, le 2-bromophényle, le 2-méthylphényle, le 2-éthylphényle, le 2-méthoxyphényle, le 2-trifluorométhylphényle, le 2,3-difluorophényle, le 2,4-difluorophényle, le 2,5-difluorophényle, le 2,6-difluorophényle, le 2,3,4-trifluorophényle, le 2,4,5-trifluorophényle, le 2,4,6-trifluorophényle, le 2-fluoro-6-méthylphényle, le 2,4-dichlorophényle, le 2,5-dichlorophényle, le 2,6-dichlorophényle, le 3,5-dichlorophényle, le 2,6-diméthylphényle, le 2-chloro-6-méthylphényle, le 2-bromo-6-fluorophényle, le 2-bromo-6-chlorophényle, le 2-bromo-6-méthylphényle, le 2-bromo-6-méthoxyphényle, le 2-fluoro-3-chlorophényle, le 2-fluoro-3-méthylphényle, le 2-chloro-3-fluorophényle, le 2-fluoro-4-chlorophényle, le 2-fluoro-6-chlorophényle, le 2,3-diméthylphényle, le 2,4-diméthylphényle, le 2-méthyl-3-fluorophényle, le 2-thiényle, le 3-fluoro-2-thiényle, le 3-chloro-2-thiényle, le 3-bromo-2-thiényle, le 3-méthyl-2-thiényle, le 3-méthoxy-2-thiényle, le 3-thiényle,
le 2-fluoro-3-thiényle, le 2-chloro-3-thiényle, le 2-bromo-3-thiényle, le 2-méthyl-3-thiényle,
le 2-méthoxy-3-thiényle, le 4-fluoro-3-thiényle, le 4-chloro-3-thiényle, le 4-bromo-3-thiényle,
le 4-méthyl-3-thiényle, le 4-méthoxy-3-thiényle, le 3,5-diméthyl-2-thiényle, le 5-bromo-3-méthyl-2-thiényle, le 2,5-diméthyl-3-thiényle, le 4,5-diméthyl-3-thiényle, le 5-bromo-2-méthyl-3-thiényle, le 5-bromo-4-méthyl-3-thiényle, le 2,4,5-triméthyl-3-thiényle,
le 2,5-dibromo-4-méthyl-3-thiényle, le pyridin-2-yl, le 3-fluoropyridin-2-yl, le 3-chloropyridin-2-yl, le 3-bromopyridin-2-yl,
le 3-méthylpyridin-2-yl, le 3-méthoxypyridin-2-yl, le 4-fluoropyridin-2-yl, le 5-fluoropyridin-2-yl, le 6-fluoropyridin-2-yl, le pyridin-3-yl, le 2-méthylpyridin-3-yl,
le 2-fluoropyridin-3-yl, le 2,4-difluoropyridin-3-yl, le pyridin-4-yl, le 3-fluoropyridin-4-yl,
le 2-fluoropyridin-4-yl, le 2,3-difluoropyridin-4-yl, le 4-méthylthiazol-5-yl, le 4-méthylisothiazol-5-yl, le cyclopentényle, le cyclohexényle ou le résidu 7-oxabicyclo[4.1.0]heptan-1-yl,
R² représente l'hydrogène, le fluor, le chlore, le brome, l'iode, le formyle, l'hydroxy, l'hydrothio, l'hydroxycarbonyle, l'aminocarbonyle, l'aminothiocarbonyle, (C₁-C₅)-alkyl, (C₁-C₅)-haloalkyl, (C₂-C₅)-alcényle, (C₂-C₅)-haloalcényle, (C₂-C₅)-alcynyle, (C₂-C₅)-haloalcynyle, (C₁-C₅)-alkoxy, (C₁-C₅) haloalkoxy, (C₁-C₅)-alkoxy-(C₁-C₅)-alkyl, (C₁-C₅)-alkylthio, (C₁-C₅)-haloalkylthio, (C₁-C₅)-alkylsulfinyl, (C₁-C₅)-haloalkylsulfinyl, (C₁-C₅)-alkylsulfonyl, (C₁-C₅)-haloalkylsulfonyl, (C₁-C₅)-alkylcarbonyl, (C₂-C₅)-alcénylecarbonyl, (C₂-C₅)-alcynylcarbonyl, (C₁-C₅)-haloalkylcarbonyl, (C₂-C₅)-haloalcénylecarbonyl, (C₂-C₅)-haloalcynylcarbonyl, (C₁-C₅)-alkoxycarbonyl, (C₃-C₅)-cycloalkyl, (C₃-C₅)-cycloalkoxy, (C₃-C₅)-cycloalkylthio, (C₃-C₅)-cycloalkylsulfinyl, (C₃-C₅)-cycloalkylsulfonyl, (C₃-C₅)-halocycloalkyl, (C₃-C₅)-halocycloalkoxy, (C₃-C₅)-halocycloalkylthio, (C₃-C₅)-halocycloalkylsulfinyl, (C₃-C₅)-halocycloalkylsulfonyl, (C₃-C₅)-cycloalkyl-(C₁-C₅)-alkyl, (C₁-C₅)-alkyl-(C₃-C₅)-cycloalkyl, (C₃-C₅)-cycloalkylcarbonyl, (C₃-C₅)-cycloalkyl-(C₁-C₅)-alkylcarbonyl, (C₁-C₅)-alkylaminocarbonyl, (C₂-C₁₀)-dialkylaminocarbonyl, (C₁-C₅)-alkylaminosulfonyl, (C₂-C₁₀)-dialkylaminocarbonyl ou tris[(C₁-C₅)alkyl]silyl,
n représente l'hydrogène, le fluor, le chlore, le brome, l'iode, le méthyle, l'éthyle, le n-propyle, l'iso-propyle, le cyclopropyle, le cyclobutyle, le vinyle, le prop-2-én-2-yl, l'éthynyle, le prop-1-yn-1-yl, le méthoxy, l'éthoxy, le méthylthio, l'éthoxycarbonyle, 2,3,4,5,6-pentafluorophényle, le difluorométhyle ou le trifluorométhyle,
R³ est 0, 1, 2,
R⁴ et R⁵ représentent indépendamment des autres l'hydrogène, le méthyle, l'éthyle, le prop-1-yl, le prop-2-yl, le but-1-yl, le but-2-yl, 1,1-diméthyléth-1-yl, le méthoxyméthyle, le méthoxyéthyle, le méthoxyprop-1-yl, l'éthoxyméthyle, l'éthoxyéthyle, l'éthoxyprop-1-yl, le difluorométhyle, le trifluorométhyle, 2,2-difluoroéthyle, 3,3,3-trifluoroéthyle, pentafluoroéthyle, cyclopropyle, cyclobutyle, cyclopentyle, ou cyclohexyle, ou
R⁴ et R⁵ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire, et
R⁶ représente l'hydrogène, BH₂, B(NH₂)₂, 4-amino-1,3,2,4-diazadiboretidin-2-yl, 4-amino-1,3,2,4-dioxadiboretan-2-yl, méthylsulfonyl, éthylsulfonyl, prop-1-ylsulfonyl, cyanométhylsulfonyl, méthoxycarbonylmethylsulfonyl, 2-(méthoxycarbonyl)éth-1-ylsulfonyl, prop-2-en-1-ylsulfonyl, prop-2-en-2-ylsulfonyl, (4-fluorophényl)methylsulfonyl, formyl, méthylcarbonyl, éthylcarbonyl, prop-1-ylcarbonyl, 1-méthyléth-1-ylcarbonyl, cyclopropylcarbonyl, cyclopentylcarbonyl, 2-(méthoxy-carbonyl)éth-1-ylcarbonyl, éthoxycarbonylméthylcarbonyl, 3-(éthoxycarbonyl)prop-1-ylcarbonyl,
(2-trifluorométhylphényl)carbonyle, 3-chloro-5-méthyl-4,5-dihydro-1,2-oxazol-5-ylcarbonyle, 3-chloro-5-méthoxyméthyl-4,5-dihydro-1,2-oxazol-5-ylcarbonyle, ou 3-chloro-3a,4,5,6-tétrahydro-6aH-cyclopenta[d][1,2]oxazol-6a-ylcarbonyle.
2-(éthoxy)prop-1-yl, 3-éthoxyprop-1-yl, 4-méthoxybut-1-yl, 2-phénoxyéth-1-yl,
2-benzyloxyéth-1-yl, 2-(méthylcarbonyloxy)éth-1-yl, 2-(éthylcarbonyloxy)éth-1-yl,
2-(iso-propylcarbonyloxy)éth-1-yl, 2-(tert-butylcarbonyloxy)éth-1-yl,
2-(phénylcarbonyloxy)éth-1-yl, 2-(2-trifluorméthylphénylcarbonyl)oxyéth-1-yl,
2-(2-chlorophenylcarbonyl)oxyeth-1-yl, 2-(4-trifluormethylpyridin-3-ylcarbonyl)oxyeth-1-yl, 2-(methylsulfonyloxy)eth-1-yl, 3-(methylcarbonyloxy)prop-1-yl, 3-(éthylcarbonyloxy)prop-1-yl, 3-(iso-propylcarbonyloxy)prop-1-yl, 3-(tert-butylcarbonyloxy)prop-1-yl, 2-trifluorométhoxyéth-1-yl, méthyl, éthyl, prop-1-yl,
1-méthyléthyle, but-1-yle, 1-méthylprop-1-yle, 2-méthylprop-1-yle, 1,1-diméthyléthyle, pent-1-yle, 1-méthylbut-1-yle, 2-méthylbut-1-yle, 3-méthylbut-1-yle, 1,1-diméthylprop-1-yle, 1,2-diméthylprop-1-yl, 2,2-diméthylprop-1-yl, 1-éthylprop-1-yl, prop-2-én-1-yl, but-2-én-1-yl, but-3-én-1-yl, pent-2-én-1-yl, pent-3-én-1-yl, pent-4-én-1-yl, 1-méthylprop-2-én-1-yl, 2-méthylprop-2-én-1-yl, 1-méthylbut-2-én-1-yl, 2-méthylbut-2-én-1-yl, 3-méthylbut-2-én-1-yl, 1-méthylbut-3-én-1-yl, 2-méthylbut-3-én-1-yl, 3-méthylbut-3-én-1-yl, 1,1-diméthylprop-2-én-1-yl, 1,2-diméthylprop-2-én-1-yl, prop-2-yn-1-yl, but-2-yn-1-yl, but-3-yn-1-yl, 1-méthyl-prop-2-yn-1-yl, pent-2-yn-1-yl, pent-3-yn-1-yl, pent-4-yn-1-yl,
1-méthylbuty-2-n-1-yl, 1-méthylbut-3-yn-1-yl, 2-méthylbut-3-yn-1-yl, cyclobutyl, cyclopentyl, cyclohexyl, cyclopropylméthyl, cyclobutylméthyl, cyclopentylméthyl, cyclohexylméthyle, 2-(diméthylamino)éth-1-yl, 2-(diéthylamino)éth-1-yl, 3-(diméthylamino)prop-1-yl, 3-(diéthylamino)prop-1-yl, 2-(N-pyrrolidin-1-yl)éth-1-yl,
2-(N-pipéridin-1-yl)éth-1-yl, tétrahydrofurane-2-ylméthyle, tétrahydrofurane-3-ylméthyle, tétrahydropyran-2-ylméthyle, tétrahydropyran-3-ylméthyle, tétrahydropyran-4-ylméthyle, oxétan-3-ylméthyle, méthoxyéthoxyéthyle-1-yl, éthoxyéthoxyéthyle-1-yl, benzyle, 2-phényléth-1-yl, (2-fluorophényl)méthyl, (3-fluorophényl)méthyl, (4-fluorophényl)méthyl, (2,6-difluorophényl)méthyl, (2,5-difluorophényl)méthyl, (2,4-difluorophényl)méthyl, (2,3-difluorophényl)méthyl, (2-fluoro-6-chlorophényl)méthyl, (2-chlorophényl)méthyl,
(3-chlorophényl)méthyle, (4-chlorophényl)méthyle, (2,6-dichlorophényl)méthyle, (2,5-dichlorophényl)méthyle, (2,4-dichlorophényl)méthyle, (2,3-dichlorophényl)méthyle,
(2-bromophényl)méthyle, (3-bromophényl)méthyle, (4-bromophényl)méthyle,
(2-méthylphényl)méthyle, (2-trifluorométhylphényl)méthyle, (2-méthoxyphényl)méthyle, (2-nitrophényl)méthyle, (3-méthylphényl)méthyle, (3-trifluorométhylphényl)méthyle,
(3-méthoxyphényl)méthyle, (3-nitrophényl)méthyle, (4-méthylphényl)méthyle,
(4-trifluorométhylphényl)méthyle, (4-méthoxyphényl)méthyle, (4-nitrophényl)méthyle, pyridine-2-ylméthyle, pyridine-3-ylméthyle, pyridine-4-ylméthyle, (6-fluoropyridin-2-yl)méthyle, (6-chloro-4-trifluorométhylpyridin-2-yl)méthyle, (4,6-dichloropyridin-2-yl)méthyle, (4-fluoropyridin-3-yl)méthyle, (4-chloropyridin-3-yl)méthyle, thiophène-2-ylméthyle, méthoxycarbonyle, éthoxycarbonyle, 1,1-diméthyléth-1-yloxycarbonyle, benzyloxycarbonyle, (prop-2-én-1-yl)oxycarbonyle, méthylaminocarbonyle, éthylaminocarbonyle, prop-1-ylaminocarbonyle, prop-2-ylaminocarbonyle, cyclopentylaminocarbonyle, cyclohexylaminocarbonyle, phénylaminocarbonyle, diméthylamincarbonyle, diéthylaminocarbonyle, éthyl(méthyl)aminocarbonyle,
(2-fluorophényl)aminocarbonyle, (3-fluorophényl)aminocarbonyle,
(4-fluorophényl)aminocarbonyle, (2,6-difluorophényl)aminocarbonyle, (2,5-difluorophényl)aminocarbonyle, (2,4-difluorophényl)aminocarbonyle, (2,3-difluorophényl)aminocarbonyle, (2-fluoro-6-chlorophényl)aminocarbonyle,
(2-chlorophényl)aminocarbonyle, (3-chlorophényl)aminocarbonyle,
(4-chlorophényl)aminocarbonyle, (2,6-dichlorophényl)aminocarbonyle, (2,5-dichlorophényl)aminocarbonyle, (2,4-dichlorophényl)aminocarbonyle, (2,3-dichlorophényl)aminocarbonyle, (2-bromophényl)aminocarbonyle,
(3-bromophényl)aminocarbonyle, (4-bromophényl)aminocarbonyle, méthoxycarbonylméthyle, 2-(méthoxycarbonyle)éth-1-yl, 1-(méthoxycarbonyle)éth-1-yl, 3-(méthoxycarbonyle)prop-1-yl, 2-(méthoxycarbonyle)prop-1-yl, 4-(méthoxycarbonyle)but-1-yl, 5-(méthoxycarbonyle)pent-1-yl, éthoxycarbonylméthyle, 2-(éthoxycarbonyl)éth-1-yl, 1-(éthoxycarbonyl)éth-1-yl, 3-(éthoxycarbonyl)prop-1-yl, 2-(éthoxycarbonyl)prop-1-yl, 4-(éthoxycarbonyl)but-1-yl, 5-(éthoxycarbonyl)pent-1-yl, benzyloxycarbonylméthyle, 2-(benzyloxycarbonyl)éth-1-yl, hydroxycarbonylméthyle, 2-(hydroxycarbonyl)éth-1-yl, 1-(hydroxycarbonyl)éth-1-yl, 3-(hydroxycarbonyl)prop-1-yl, 2-(hydroxycarbonyl)prop-1-yl, 4-(hydroxycarbonyl)but-1-yl, 5-(hydroxycarbonyl)pent-1-yl, aminocarbonylméthyl, méthylaminocarbonylméthyl, éthylaminocarbonylméthyl, prop-1- ylaminocarbonylméthyl, prop-2-ylaminobarbonylméthyle, diméthylaminocarbonylméthyle, diéthylaminocarbonylméthyle, benzylaminocarbonylméthyle, cyclopropylaminocarbonylméthyle, cyclobutylaminocarbonylméthyle, cyclopentylaminocarbonylméthyle, cyclohexylaminocarbonylméthyle, 2-(aminocarbonyl)éth-1-yl,
2-(méthylaminocarbonyl)éth-1-yl, 2-(éthylaminocarbonyl)éth-1-yl, 2-(prop-1-ylaminocarbonyl)éth-1-yl, 2-(prop-2-ylaminobarbonyl)éth-1-yl,
2-(diméthylaminocarbonyle)éth-1-yl, 2-(diéthylaminocarbonyle)éth-1-yl,
2-(benzylaminocarbonyl)éth-1-yl, 2-(cyclopropylaminocarbonyl)éth-1-yl,
2-(cyclobutylaminocarbonyl)éth-1-yl, 2-(cyclopentylaminocarbonyl)éth-1-yl,
2-(cyclohexylaminocarbonyl)éth-1-yl, 2-(méthylsulfanyl)éth-1-yl,
2-(méthylsulfinyl)éth-1-yl, 2-(méthylsulfonyl)éth-1-yl, 2-(éthylsulfanyl)éth-1-yl,
2-(éthylsulfinyl)éth-1-yl, 2-(éthylsulfonyl)éth-1-yl, 2-(méthylsulfanyl)prop-1-yl,
2-(méthylsulfinyl)prop-1-yl, 2-(méthylsulfonyl)prop-1-yl, méthylsulfonyl, éthylsulfonyl, prop-1-ylsulfonyl, 1-méthyléth-1-ylsulfonyl, but-1-ylsulfonyl,
1-méthylprop-1-ylsulfonyl, 2-méthylprop-1-ylsulfonyl, 1,1-diméthyléthylsulfonyl, pent-1-ylsulfonyl, 1-méthylbut-1-ylsulfonyl, 2-méthylbut-1-ylsulfonyl, 3-méthylbut-1-ylsulfonyl, 1,1-diméthylprop-1-ylsulfonyl, 1,2-diméthylprop-1-ylsulfonyl,
2,2-diméthylprop-1-ylsulfonyl, 1-éthylprop-1-ylsulfonyl, cyanométhylsulfonyl,
3-cyanoprop-1-ylsulfonyl, 1-méthylcycloprop-1-ylsulfonyl, tétrahydrofurane-3-ylsulfonyl, méthoxycarbonylméthylsulfonyl, éthoxycarbonylméthylsulfonyl, cyclopropylméthylsulfonyl, cyclobutylméthylsulfonyle, cyclopentylméthylsulfonyle, cyclohexylméthylsulfonyle, cyclopropylsulfonyle, cyclobutylsulfonyle, cyclopentylsulfonyle, cyclohexylsulfonyle, prop-2-en-1-ylsulfonyl, prop-2-en-2-ylsulfonyl, but-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-méthylprop-2-en-1-ylsulfonyl, 2-méthylprop-2-en-1-ylsulfonyl, trifluorméthylsulfonyl, benzylsulfonyl, 2-phényléth-1-ylsulfonyl, (2-fluorophényl)méthylsulfonyl, (3-fluorophényl)méthylsulfonyle, (4- fluorophényl)-méthylsulfonyle, (2,6-difluorophényl)méthylsulfonyle, (2,5-difluorophényl)-méthylsulfonyle, (2,4-difluorophényl)méthylsulfonyle, (2,3-difluorophényl)-méthylsulfonyle, (2-fluoro-6-chlorophényl)méthylsulfonyle, (2-chlorophényl)-méthylsulfonyle, (3-chlorophényl)méthylsulfonyle, (4-chlorophényl)méthylsulfonyle, (2,6-dichlorophényl)méthylsulfonyle, (2,5-dichlorophényl)méthylsulfonyle, (2,4-dichloro-phényl)méthylsulfonyle, (2,3-dichlorophényl)méthylsulfonyle, (2-bromo-phényl)méthylsulfonyle, (3-bromophényl)méthylsulfonyle, (4-bromo-phényl)méthylsulfonyle, (2-méthylphényl)méthylsulfonyle, (2-trifluoro-méthylphényl)méthylsulfonyle, (2-méthoxyphényl)méthylsulfonyle,
(2-nitrophényl)méthylsulfonyl, (3-méthylphényl)méthylsulfonyl,
(3-trifluorométhylphényl)méthylsulfonyle, (3-méthoxyphényl)méthylsulfonyle,
(3-nitrophényl)méthylsulfonyl, (4-méthylphényl)méthylsulfonyl,
(4-trifluorométhylphényl)méthylsulfonyle, (4-méthoxyphényl)méthylsulfonyle,
(4-nitrophényl)méthylsulfonyl, pyridine-2-ylméthylsulfonyl, pyridine-3-ylméthylsulfonyl, pyridine-4-ylméthylsulfonyl, (6-fluoropyridin-2-yl)méthylsulfonyl, (6-chloro-4-trifluorométhylpyridin-2-yl)méthylsulfonyle, (4,6-dichloropyridin-2-yl)méthylsulfonyle, (4-fluoropyridin-3-yl)méthylsulfonyle, (4-chloropyridin-3-yl)méthylsulfonyle, thiophène-2-ylméthylsulfonyle, phénylsulfonyle, (2-fluorophényl)sulfonyle, (3-fluorophényl)sulfonyle, (4-fluorophényl)sulfonyle, (2,6-difluorophényl)sulfonyle, (2,5-difluorophényl)sulfonyle, (2,4-difluorophényl)sulfonyle, (2,3-difluorophényl)sulfonyle, (2-fluoro-6-chlorophényl)sulfonyle, (2-chlorophényl)sulfonyle, (3-chlorophényl)sulfonyle,
(4-chlorophényl)sulfonyle, (2,6-dichlorophényl)sulfonyle, (2,5-dichlorophényl)sulfonyle, (2,4-dichlorophényl)sulfonyle, (2,3-dichlorophényl)sulfonyle, (2-bromophényl)sulfonyle, (3-bromophényl)sulfonyle, (4-bromophényl)sulfonyle, (2-méthylphényl)sulfonyle,
(2-trifluorométhylphényl)sulfonyle, (2-méthoxyphényl)sulfonyle, (2-nitrophényl)sulfonyle, (3-méthylphényl)sulfonyle, (3- trifluorométhylphényl)sulfonyle, (3-méthoxyphényl)-sulfonyle, (3-nitrophényl)sulfonyle, (4-méthylphényl)sulfonyle, (4-trifluorométhylphényl)-sulfonyle, (4-méthoxyphényl)sulfonyle, (4-nitrophényl)sulfonyle, pyridine-2-ylsulfonyle, pyridine-3-ylsulfonyle, pyridine-4-ylsulfonyle, (6-fluoropyridin-2-yl)sulfonyle, (6-chloro-4-trifluorosulfonylpyridin-2-yl)sulfonyle, (4,6-dichloropyridin-2-yl)sulfonyle, (4-fluoro-pyridin-3-yl)sulfonyl, (4-chloropyridin-3-yl)sulfonyl, thiophen-2-ylsulfonyl, méthoxyméthylsulfonyl, 2-(méthoxy)éth-1-ylsulfonyl, 2-(méthoxy)éth-1-ylsulfonyl, méthylaminosulfonyl, éthylaminosulfonyl, diméthylaminosulfonyl, diéthylamino-sulfonyl, méthyl(éthyl)aminosulfonyl, azetidin-1-ylsulfonyl, pyrrolidin-1-ylsulfonyl, piperidin-1-ylsulfonyl, N-morpholinylsulfonyl, 2-(méthoxycarbonyl)éth-1-ylsulfonyl, 2-(éthoxycarbonyl)éth-1-ylsulfonyl, 3-(méthoxycarbonyl)prop-1-ylsulfonyl,
3-(éthoxycarbonyle)prop-1-ylsulfonyle, 1-(méthoxycarbonyle)éth-1-ylsulfonyle,
2-éthoxyéth-1-ylsulfonyl, 1-(prop-2-en-1-yl)cycloprop-1-ylsulfonyl, 3,3,3-trifluoroéthylsulfonyl, tétrahydropyran-4-ylsulfonyl, tétrahydrofuran-3-ylméthylsulfonyl, formyl, méthylcarbonyl, éthylcarbonyl, prop-1-ylcarbonyl,
1-méthyléthyl-1-ylcarbonyl, but-1-ylcarbonyl, 1-méthylprop-1-ylcarbonyl, 2-méthylprop-1-ylcarbonyl, 1,1-diméthyléthylcarbonyl, pent-1-ylcarbonyl, 1-méthylbut-1-ylcarbonyl, 2-méthylbut-1-ylcarbonyl, 3-méthylbut-1-ylcarbonyl, 1,1-diméthylprop-1-ylcarbonyl, 1,2-diméthylprop-1-ylcarbonyl, 2,2-diméthylprop-1-ylcarbonyl, 1-éthylprop-1-ylcarbonyle, cyclopropylméthylcarbonyle, cyclobutylméthylcarbonyle, cyclopentylméthylcarbonyle, cyclohexylméthylcarbonyle, cyclopropylcarbonyle, cyclobutylcarbonyle, cyclopentylcarbonyle, cyclohexylcarbonyle, méthoxyméthylcarbonyle, 2-méthoxyéth-1-ylcarbonyle, 2-éthoxyéth-1-ylcarbonyle, 3-méthoxyprop-1-ylcarbonyle,
3-éthoxyprop-1-ylcarbonyl, méthoxycarbonylméthylcarbonyl, 2-(méthoxycarbonyl)éth-1-ylcarbonyl, 3-(méthoxycarbonyl)prop-1-ylcarbonyl, 4-(méthoxycarbonyl)but-1-ylcarbonyl, éthoxycarbonylméthylcarbonyl, 2-(éthoxycarbonyl)éth-1-ylcarbonyl,
3-(éthoxycarbonyle)prop-1-ylcarbonyle, 4-(éthoxycarbonyle)but-1-ylcarbonyle, benzylcarbonyle, 2-phényléth-1-ylcarbonyle, (2-fluorophényl)méthylcarbonyle,
(3-fluorophényl)méthylcarbonyle, (4-fluorophényl)méthylcarbonyle,
(2,6-difluorophényl)méthylcarbonyle, (2,5-difluorophényl)méthylcarbonyle,
(2,4-difluorophényl)méthylcarbonyle, (2,3-difluorophényl)méthylcarbonyle, (2-fluoro-6-chlorophényl)méthylcarbonyle, (2-chlorophényl)méthylcarbonyle,
(3-chlorophényl)méthylcarbonyle, (4-chlorophényl)méthylcarbonyle,
(2,6-dichlorophényl)méthylcarbonyle, (2,5-dichlorophényl)méthylcarbonyle,
(2,4-dichlorophényl)méthylcarbonyle, (2,3-dichlorophényl)méthylcarbonyle,
(2-bromophényl)méthylcarbonyle, (3-bromophényl)méthylcarbonyle,
(4-bromophényl)méthylcarbonyle, (2-méthylphényl)méthylcarbonyle,
(2-trifluorométhylphényl)méthylcarbonyle, (2-méthoxyphényl)méthylcarbonyle,
(2-nitrophényl)méthylcarbonyle, (3-méthylphényl)méthylcarbonyle,
(3-trifluorométhylphényl)méthylcarbonyle, (3-méthoxyphényl)méthylcarbonyle,
(3-nitrophényl)méthylcarbonyle, (4-méthylphényl)méthylcarbonyle,
(4-trifluorométhylphényl)méthylcarbonyle, (4-méthoxyphényl)méthylcarbonyle,
(4-nitrophényl)méthylcarbonyle, pyridine-2-ylméthylcarbonyle, pyridine-3-ylméthylcarbonyle, pyridine-4-ylméthylcarbonyle, (6-fluoropyridin-2-yl)méthylcarbonyle, (6-chloro-4-trifluorométhylpyridin-2-yl)méthylcarbonyle, (4,6-dichloropyridin-2-yl)méthylcarbonyle, (4-fluoropyridin-3-yl)méthylcarbonyle, (4-chloropyridin-3-yl)méthylcarbonyle, thiophène-2-ylméthylcarbonyle, phénylcarbonyle,
(2-fluorophényl)carbonyle, (3-fluorophényl)carbonyle, (4-fluorophényl)carbonyle,
(2,6-difluorophényl)carbonyle, (2,5-difluorophényl)carbonyle,
(2,4-difluorophényl)carbonyle, (2,3-difluorophényl)carbonyle, (2-fluoro-6-chlorophényl)carbonyle, (2-chlorophényl)carbonyle, (3-chlorophényl)carbonyle,
(4-chlorophényl)carbonyle, (2,6-dichlorophényl)carbonyle, (2,5-dichlorophényl)carbonyle, (2,4-dichlorophényl)carbonyle, (2,3-dichlorophényl)carbonyle, (2-bromophényl)carbonyle, (3-bromophényl)carbonyle, (4-bromophényl)carbonyle, (2-méthylphényl)carbonyle,
(2-trifluorométhylphényl)carbonyle, (2-méthoxyphényl)carbonyle, (2-nitrophényl)-carbonyle, (3-méthylphényl)carbonyle, (3-trifluorométhylphényl)carbonyle,
(3-méthoxyphényl)-carbonyle, (3-nitrophényl)-carbonyle, (4-méthylphényl)-carbonyle,
(4-trifluorométhylphényl)carbonyle, (4-méthoxyphényl)carbonyle, (4-nitrophényl)-carbonyle, (2-chloro-4-méthylsulfonylphényl)carbonyle, pyridine-2-ylcarbonyle, pyridine-3-ylcarbonyl, pyridine-4-ylcarbonyl, (6-fluoropyridin-2-yl)carbonyl, (6-chloro-4-trifluorocarbonylpyridin-2-yl)carbonyl, (4,6-dichloropyridin-2-yl)carbonyl,
(4-fluoropyridin-3-yl)carbonyl, (4-chloropyridin-3-yl)carbonyl, thiophen-2-ylcarbonyle, 1,3-oxazol-2-ylcarbonyle, 1,2-oxazol-3-ylcarbonyle, 3-chloro-5-méthyl-4,5-dihydro-1,2-oxazol-5-ylcarbonyle, 3-bromo-5-méthyl-4,5-dihydro-1,2-oxazol-5-ylcarbonyle, 3-chloro-5-méthoxyméthyl-4,5-dihydro-1,2-oxazol-5-ylcarbonyle, 3-chloro-5-éthyl-4,5-dihydro-1,2-oxazol-5-ylcarbonyle, 3-chloro-3a,4,5,6-tétrahydro-6aH-cyclopenta[d][1,2]oxazol-6a-ylcarbonyle, (4-trifluorométhylpyridine-3-yl)carbonyl, 3,5-diméthyl-4,5-dihydro-1,2-oxazol-5-ylcarbonyle, 5-méthyl-3-trifluorométhyl-4,5-dihydro-1,2-oxazol-5-ylcarbonyl, 3-chloro-5-hydroxyméthyl-4,5-dihydro-1,2-oxazol-5-ylcarbonyl, 3-chloro-5-tert-butyldiméthylsilyloxyméthyl-4,5-dihydro-1,2-oxazol-5-ylcarbonyl, 3-chloro-5-(1-hydroxyéth-1-yl)-4,5-dihydro-1,2-oxazol-5-ylcarbonyl, 3-chloro-5-(1-tert-butyldiméthylsilyloxyeth-1-yl-4,5-dihydro-1,2-oxazol-5-ylcarbonyl, méthoxyéthoxyméthylcarbonyl, éthoxyéthoxyméthylcarbonyl, hydroxyéthoxyméthylcarbonyl, méthoxyéthoxyéthoxyméthylcarbonyl, éthoxyéthoxyéthoxyméthylcarbonyl, hydroxyéthoxyéthoxyméthylcarbonyl, 4-trifluorométhylpyridine-3-ylcarbonyloxyéthoxyéthoxyméthylcarbonyle, méthoxyéthoxyéthoxyéthoxyméthylcarbonyle, éthoxyéthoxyéthoxyéthoxyméthylcarbonyle, hydroxyéthoxyéthoxyéthoxyméthylcarbonyle, méthoxycarbonylméthoxyéthoxyéthoxyméthylcarbonyle, hydroxycarbonylméthoxyéthoxyméthylcarbonyl, méthoxycarbonylméthoxyéthoxyéthoxyméthylcarbonyl, hydroxycarbonylméthoxyéthoxyéthoxyméthylcarbonyl, méthoxycarbonylméthoxyéthoxyéthoxyéthoxyméthylcarbonyl, ou hydroxycarbonylméthoxyéthoxyéthoxyéthoxyéthoxyméthylcarbonyl,

6. Le composé de formule générale (I) selon la revendication 1 et/ou son sel, **caractérisé en ce que**
R¹ représente le phényle, le 2-fluorophényle, le 3-fluorophényle, le 4-fluorophényle, le 2-chlorophényle,
3-chlorophényle, 4-chlorophényle, 2-bromophényle, 2-méthylphényle, 2-éthylphényle,
2-méthoxyphényle, 2-trifluorométhylphényle, 2,3-difluorophényle, 2,4-difluorophényle,
2,5-difluorophényle, 2,6-difluorophényle, 2,4,5-trifluorophényle, 2,3,4-fluorophényle,
2-fluoro-6-méthylphényle, 2-fluoro-3-méthylphényle, 2-fluoro-3-chlorophényle,
2,3-diméthylphényle, 2-thiényle, 3-chloro-2-thiényle, 3-méthyl-2-thiényle, 4-méthyl-3-thiényle,
représente l'hydrogène, le fluor, le chlore, le brome, l'iode, le méthyle, l'éthyle, le 2,3,4,5,6-pentafluorophényle, l'éthoxycarbonyle, le cyclopropyle,
représente l'hydrogène,
est 0, 1, 2,
représentent indépendamment des autres l'hydrogène, le méthyle, l'éthyle, le prop-1-yl, le prop-2-yl, le but-1-yl, le but-2-yl, 1,1-diméthyléth-1-yl,
R² méthoxyméthyle, méthoxyéthyle, méthoxyprop-1-yl, éthoxyméthyle, éthoxyéthyle,
R³ difluorométhyle, trifluorométhyle,
n 2,2-difluoroéthyle, 3,3,3-trifluoroéthyle, pentafluoroéthyle, cyclopropyle, cyclobutyle, cyclopentyle, ou cyclohexyle, ou
R⁴ et R⁵ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire, et
R⁴ et R⁵ représente l'hydrogène, BH₂, B(NH₂)₂, 4-amino-1,3,2,4-diazadiboretidin-2-yl, 4-amino-1,3,2,4-dioxadiboretan-2-yl, méthylsulfonyl, éthylsulfonyl, prop-1-ylsulfonyl, cyanométhylsulfonyl, méthoxycarbonylmethylsulfonyl, 2-(méthoxycarbonyl)éth-1-ylsulfonyl, prop-2-en-1-ylsulfonyl, prop-2-en-2-ylsulfonyl, (4-fluorophényl)methylsulfonyl, formyl, méthylcarbonyl, éthylcarbonyl, prop-1-ylcarbonyl, 1-méthyléth-1-ylcarbonyl, cyclopropylcarbonyl, cyclopentylcarbonyl, 2-(méthoxy-carbonyl)éth-1-ylcarbonyl, éthoxycarbonylméthylcarbonyl, 3-(éthoxycarbonyl)prop-1-ylcarbonyl,
R⁶ (2-trifluorométhylphényl)carbonyle, 3-chloro-5-méthyl-4,5-dihydro-1,2-oxazol-5-ylcarbonyle, 3-chloro-5-méthoxyméthyl-4,5-dihydro-1,2-oxazol-5-ylcarbonyle, ou 3-chloro-3a,4,5,6-tétrahydro-6aH-cyclopenta[d][1,2]oxazol-6a-ylcarbonyle.
2-(éthoxycarbonyl)éth-1-yl, 1-(éthoxycarbonyl)éth-1-yl, 3-(éthoxycarbonyl)prop-1-yl, 2-(éthoxycarbonyl)prop-1-yl, 4-(éthoxycarbonyl)but-1-yl, 5-(éthoxycarbonyl)pent-1-yl, benzyloxycarbonylméthyl, 2-(benzyloxycarbonyl)éth-1-yl, hydroxycarbonylméthyl,
2-(hydroxycarbonyl)éth-1-yl, 1-(hydroxycarbonyl)éth-1-yl, 3-(hydroxycarbonyl)prop-1-yl, 2-(hydroxycarbonyl)prop-1-yl, 4-(hydroxycarbonyl)but-1-yl, 5-(hydroxycarbonyl)-pent-1-yl, méthylsulfonyl, éthylsulfonyl, prop-1-ylsulfonyl, 1-méthyleth-1-ylsulfonyl, but-1-ylsulfonyl, 1-méthylprop-1-ylsulfonyl, 2-méthylprop-1-ylsulfonyl, 1,1-diméthyl-éthylsulfonyl, pent-1-ylsulfonyl, 1-méthylbut-1-ylsulfonyl, 2-méthylbut-1-ylsulfonyl,
3-méthylbut-1-ylsulfonyl, 1,1-diméthylprop-1-ylsulfonyl, 1,2-diméthylprop-1-ylsulfonyl, 2,2-diméthylprop-1-ylsulfonyl, 1-éthylprop-1-ylsulfonyl, cyanométhylsulfonyl, 3-cyanoprop-1-ylsulfonyl, 1-méthylcycloprop-1-ylsulfonyl, tétrahydrofurane-3-ylsulfonyl, méthoxycarbonyl-méthylsulfonyl, éthoxycarbonylméthylsulfonyl, cyclopropylméthylsulfonyl, cyclobutylméthylsulfonyl, cyclopentylméthylsulfonyl, cyclohexylméthylsulfonyl, cyclopropylsulfonyl, cyclobutylsulfonyl, cyclopentylsulfonyl, cyclohexylsulfonyl, prop-2-en-1-ylsulfonyl, prop-2-en-2-ylsulfonyl, but-2-en-1-ylsulfonyl, but-3-en-1-ylsulfonyl, pent-2-en-1-ylsulfonyl, pent-3-en-1-ylsulfonyl, pent-4-en-1-ylsulfonyl, 1-méthylprop-2-en-1-ylsulfonyl, 2-méthylprop-2-en-1-ylsulfonyl, trifluorméthylsulfonyl, benzylsulfonyl,
2-phényléth-1-ylsulfonyl, (2-fluorophényl)méthylsulfonyl,
(3-fluorophényl)méthylsulfonyle, (4-fluorophényl)méthylsulfonyle,
(2,6-difluorophényl)méthylsulfonyl, (2,5-difluorophényl)-méthylsulfonyl,
(2,4-difluorophényl)méthylsulfonyle, (2,3-difluorophényl)méthylsulfonyle,
(2-fluoro-6-chlorophényl)méthylsulfonyle, (2-chlorophényl)méthylsulfonyle,
(3-chlorophényl)méthylsulfonyle, (4-chlorophényl)méthylsulfonyle, (2,6-dichlorophényl)méthylsulfonyle, (2,5-dichlorophényl)méthylsulfonyle,
(2,4dichlorophényl)méthylsulfonyle, (2,3-dichlorophényl)méthylsulfonyle,
(2-bromophényl)méthylsulfonyle, (3-bromophényl)-méthylsulfonyle,
(4-bromophényl)méthylsulfonyl, (2-méthylphényl)méthylsulfonyl,
(2-trifluorométhylphényl)méthylsulfonyle, (2-méthoxyphényl)méthylsulfonyle,
(2-nitrophényl)-méthylsulfonyl, (3-méthylphényl)méthylsulfonyl,
(3-trifluorométhylphényl)méthylsulfonyle, (3-méthoxyphényl)méthylsulfonyle,
(3-nitrophényl)méthylsulfonyl, (4-méthylphényl)méthylsulfonyl,
(4-trifluorométhylphényl)méthylsulfonyle, (4-méthoxyphényl)méthylsulfonyle,
(4-nitrophényl)méthylsulfonyle, pyridine-2-ylméthylsulfonyle, pyridine-3-ylméthylsulfonyle, pyridine-4-ylméthylsulfonyle, (6-fluoropyridin-2-yl)méthylsulfonyle, (6-chloro-4-trifluorométhylpyridin-2-yl)méthylsulfonyle, (4,6-dichloropyridin-2-yl)méthylsulfonyle, (4-fluoropyridin-3-yl)méthylsulfonyle, (4-chloropyridin-3-yl)méthylsulfonyle, thiophène-2-ylméthylsulfonyle, phénylsulfonyle, (2-fluorophényl)sulfonyle, (3-fluorophényl)sulfonyle, (4-fluorophényl)sulfonyle, (2,6-difluorophényl)sulfonyle, (2,5-difluorophényl)sulfonyle, (2,4-difluorophényl)sulfonyle, (2,3-difluorophényl)sulfonyle, (2-fluoro-6-chlorophényl)sulfonyle, (2-chlorophényl)sulfonyle, (3-chlorophényl)sulfonyle,
(4-chlorophényl)sulfonyle, (2,6-dichlorophényl)sulfonyle, (2,5-dichlorophényl)sulfonyle, (2,4-dichlorophényl)sulfonyle, (2,3-dichlorophényl)sulfonyle, (2-bromophényl)sulfonyle, (3-bromophényl)sulfonyle, (4-bromophényl)sulfonyle, (2-méthylphényl)sulfonyle,
(2-trifluorométhylphényl)sulfonyle, (2-méthoxyphényl)sulfonyle, (2-nitrophényl)sulfonyle, (3-méthylphényl)sulfonyle, (3-trifluorométhylphényl)sulfonyle,
(3-méthoxyphényl)sulfonyle, (3-nitrophényl)sulfonyle, (4-méthylphényl)sulfonyle,
(4-trifluorométhylphényl)sulfonyle, (4-méthoxyphényl)sulfonyle, (4-nitrophényl)sulfonyle, pyridine-2-ylsulfonyle, pyridine-3-ylsulfonyle, pyridine-4-ylsulfonyle, (6-fluoropyridin-2-yl)sulfonyle, (6-chloro-4-trifluorosulfonylpyridin-2-yl)sulfonyl, (4,6-dichloropyridin-2-yl)sulfonyl, (4-fluoropyridin-3-yl)sulfonyl, (4-chloropyridin-3-yl)sulfonyl, thiophen-2-ylsulfonyl, méthoxyméthylsulfonyl, 2-(méthoxy)éth-1-ylsulfonyl, 2-(méthoxy)éth-1-ylsulfonyl, méthylaminosulfonyl, éthylaminosulfonyl, diméthylaminosulfonyl, diéthylaminosulfonyl, méthyl(éthyl)aminosulfonyl, azetidin-1-ylsulfonyl, pyrrolidin-1-ylsulfonyl, piperidin-1-ylsulfonyl, N-morpholinylsulfonyl, 2-(méthoxycarbonyl)éth-1-ylsulfonyl, 2-(éthoxycarbonyl)éth-1-ylsulfonyl, 3-(méthoxycarbonyl)prop-1-ylsulfonyl, 3-(éthoxycarbonyl)prop-1-ylsulfonyl, 1-(méthoxycarbonyl)éth-1-ylsulfonyl,
2-éthoxyéth-1-ylsulfonyl, 1-(prop-2-en-1-yl)cycloprop-1-ylsulfonyl, 3,3,3-trifluoroéthylsulfonyl, tétrahydropyran-4-ylsulfonyl, tétrahydrofuran-3-ylméthylsulfonyl, formyl, méthylcarbonyl, éthylcarbonyl, prop-1-ylcarbonyl,
1-méthyléthyl-1-ylcarbonyl, but-1-ylcarbonyl, 1-méthylprop-1-ylcarbonyl, 2-méthylprop-1-ylcarbonyl, 1,1-diméthyléthylcarbonyl, pent-1-ylcarbonyl, 1-méthylbut-1-ylcarbonyl, 2-méthylbut-1-ylcarbonyl, 3-méthylbut-1-ylcarbonyl, 1,1-diméthylprop-1-ylcarbonyl, 1,2-diméthylprop-1-ylcarbonyl, 2,2-diméthylprop-1-ylcarbonyl, 1-éthylprop-1-ylcarbonyle, cyclopropylméthylcarbonyle, cyclobutylméthylcarbonyle, cyclopentylméthylcarbonyle, cyclohexylméthylcarbonyle, cyclopropylcarbonyle, cyclobutylcarbonyle, cyclopentylcarbonyle, cyclohexylcarbonyle, méthoxyméthylcarbonyle, 2-méthoxyéth-1-ylcarbonyle, 2-éthoxyéth-1-ylcarbonyle, 3-méthoxyprop-1-ylcarbonyle,
3-éthoxyprop-1-ylcarbonyl, méthoxycarbonylméthylcarbonyl, 2-(méthoxycarbonyl)éth-1-ylcarbonyl, 3-(méthoxycarbonyl)prop-1-ylcarbonyl, 4-(méthoxycarbonyl)but-1-ylcarbonyl, éthoxycarbonylméthylcarbonyl, 2-(éthoxycarbonyl)éth-1-ylcarbonyl,
3-(éthoxycarbonyle)prop-1-ylcarbonyle, 4-(éthoxycarbonyle)but-1-ylcarbonyle, benzylcarbonyle, 2-phényléth-1-ylcarbonyle, (2-fluorophényl)méthylcarbonyle,
(3-fluorophényl)méthylcarbonyle, (4-fluorophényl)méthylcarbonyle, (2,6-difluorophényl)-méthylcarbonyle, (2,5-difluorophényl)méthylcarbonyle, (2,4-difluorophényl)-méthylcarbonyle, (2,3-difluorophényl)méthylcarbonyle, (2-fluoro-6-chlorophényl)-méthylcarbonyle, (2-chlorophényl)-méthylcarbonyle,
(3-chlorophényl)-méthylcarbonyle, (4-chlorophényl)-méthylcarbonyle,
(2,6-dichlorophényl)méthylcarbonyle, (2,5-dichlorophényl)méthylcarbonyle,
(2,4-dichlorophényl)-méthylcarbonyle, (2,3-dichlorophényl)méthylcarbonyle,
(2-bromophényl)méthylcarbonyle, (3-bromophényl)méthylcarbonyle,
(4-bromophényl)méthylcarbonyle, (2-méthylphényl)-méthylcarbonyle,
(2-trifluorométhylphényl)méthylcarbonyle, (2-méthoxyphényl)méthylcarbonyle,
(2-nitrophényl)méthylcarbonyle, (3-méthylphényl)méthylcarbonyle,
(3-trifluorométhylphényl)-méthylcarbonyle, (3-méthoxyphényl)méthylcarbonyle,
(3-nitrophényl)méthylcarbonyle, (4-méthylphényl)méthylcarbonyle,
(4-trifluorométhylphényl)méthylcarbonyle, (4-méthoxyphényl)méthylcarbonyle,
(4-nitrophényl)méthylcarbonyle, pyridine-2-ylméthylcarbonyle, pyridine-3-ylméthylcarbonyle, pyridine-4-ylméthylcarbonyle, (6-fluoropyridin-2-yl)méthylcarbonyle, (6-chloro-4-trifluorométhylpyridin-2-yl)méthylcarbonyle, (4,6-dichloropyridin-2-yl)méthylcarbonyle, (4-fluoropyridin-3-yl)méthylcarbonyle, (4-chloropyridin-3-yl)méthylcarbonyle, thiophène-2-ylméthylcarbonyle, phénylcarbonyle,
(2-fluorophényl)carbonyle, (3-fluorophényl)carbonyle, (4-fluorophényl)carbonyle,
(2,6-difluorophényl)carbonyl, (2,5-difluorophényl)carbonyl, (2,4-difluorophényl)-carbonyle, (2,3-difluorophényl)carbonyl, (2-fluoro-6-chlorophényl)carbonyl,
(2-chlorophényl)-carbonyle, (3-chlorophényl)-carbonyle, (4-chlorophényl)-carbonyle,
(2,6-dichlorophényl)carbonyle, (2,5-dichlorophényl)carbonyle, (2,4-dichlorophényl)-carbonyle, (2,3-dichlorophényl)carbonyle, (2-bromophényl)carbonyle, (3-bromophényl)-carbonyle, (4-bromophényl)carbonyle, (2-méthylphényl)-carbonyle, (2-trifluorométhyl-phényl)-carbonyle, (2-méthoxyphényl)-carbonyle, (2-nitrophényl)-carbonyle, (3-méthyl-phényl)-carbonyle, (3-trifluorométhylphényl)-carbonyle, (3-méthoxyphényl)-carbonyle,
(3-nitrophényl)carbonyl, (4-méthylphényl)carbonyl, (4-trifluorométhylphényl)carbonyl, (4-méthoxyphényl)carbonyl, (4-nitrophényl)carbonyl, (2-chloro-4-méthylsulfonyl-phényl)carbonyl, pyridine-2-ylcarbonyl, pyridine-3-ylcarbonyl, pyridine-4-ylcarbonyl,
(6-fluoropyridin-2-yl)carbonyl, (6-chloro-4-trifluorocarbonylpyridin-2-yl)carbonyl, (4,6-dichloropyridin-2-yl)carbonyl, (4-fluoropyridin-3-yl)carbonyl, (4-chloropyridin-3-yl)carbonyl, thiophen-2-ylcarbonyl, 1,3-oxazol-2-ylcarbonyle, 1,2-oxazol-3-ylcarbonyle, 3-chloro-5-méthyl-4,5-dihydro-1,2-oxazol-5-ylcarbonyle, 3-bromo-5-méthyl-4,5-dihydro-1,2-oxazol-5-ylcarbonyle, 3-chloro-5-méthoxyméthyl-4,5-dihydro-1,2-oxazol-5-ylcarbonyle, 3-chloro-5-éthyl-4,5-dihydro-1,2-oxazol-5-ylcarbonyle, 3-chloro-3a,4,5,6-tétrahydro-6aH-cyclopenta[d][1,2]oxazol-6a-ylcarbonyle, (4-trifluorométhylpyridine-3-yl)carbonyl, 3,5-diméthyl-4,5-dihydro-1,2-oxazol-5-ylcarbonyle, 5-méthyl-3-trifluorométhyl-4,5-dihydro-1,2-oxazol-5-ylcarbonyl, 3-chloro-5-hydroxyméthyl-4,5-dihydro-1,2-oxazol-5-ylcarbonyl, 3-chloro-5-tert-butyldiméthylsilyloxyméthyl-4,5-dihydro-1,2-oxazol-5-ylcarbonyl, 3-chloro-5-(1-hydroxyeth-1-yl)-4,5-dihydro-1,2-oxazol-5-ylcarbonyl, 3-chloro-5-(1-tert-butyldimethyl-silyloxyeth-1-yl-4,5-dihydro-1,2-oxazol-5-ylcarbonyl, methoxyethoxymethylcarbonyl, ethoxyethoxymethylcarbonyl, hydroxyéthoxyméthylcarbonyle, méthoxyéthoxyéthoxyméthylcarbonyle, éthoxyéthoxyéthoxyméthylcarbonyle, hydroxyéthoxyéthoxyméthylcarbonyle, méthoxyéthoxyéthoxyéthoxyméthylcarbonyle, éthoxyéthoxyéthoxyéthoxyméthylcarbonyle, hydroxyéthoxyéthoxyéthoxyméthylcarbonyle, méthoxycarbonylméthoxyéthoxyméthylcarbonyle, 4-trifluorométhylpyridine-3-ylcarbonyloxyéthoxyéthoxy-méthylcarbonyle, hydroxycarbonylméthoxyéthoxyméthylcarbonyle, méthoxycarbonylméthoxyéthoxyéthoxyméthylcarbonyle, hydroxycarbonylméthoxyéthoxyéthoxyméthylcarbonyle, méthoxycarbonylméthoxyéthoxyéthoxyéthoxy-méthylcarbonyle ou hydroxycarbonylméthoxyéthoxyéthoxyéthoxyméthylcarbonyle.

7. Le composé de formule générale (I) selon la revendication 1 et/ou son sel, **caractérisé en ce que**
R¹ représente le phényle, le 2-fluorophényle, le 3-fluorophényle, le 2-chlorophényle, le 2-méthylphényle, 2-éthylphényle, 2-méthoxyphényle, 2,3-difluorophényle, 2,4-difluorophényle, 2,5-difluorophényle, 2,6-difluorophényle, 2,4,5-trifluorophényle, 2,3,4-fluorophényle, 2-fluoro-6-méthylphényle, 2-fluoro-3-méthylphényle, 2-fluoro-3-chlorophényle, 2,3-diméthylphényle, 2-thiényle, 3-chloro-2-thiényle, 3-méthyl-2-thiényle, 4-méthyl-3-thiényle,
R² représente l'hydrogène, le fluor, le chlore, le brome, l'iode, le méthyle, l'éthyle, le 2,3,4,5,6-pentafluorophényle, l'éthoxycarbonyle, le cyclopropyle,
R³ représente l'hydrogène,
n est 0, 2,
R⁴ et R⁵ représentent indépendamment des autres l'hydrogène, le méthyle, l'éthyle, le prop-1-yl, le prop-2-yl, le but-1-yl, le but-2-yl, 1,1-diméthyléth-1-yl, 2-méthylprop-1-yl, hydroxyméthyle, hydroxyéthyle, hydroxyprop-1-yl, méthoxyméthyle, méthoxyéthyle, méthoxyprop-1-yl, éthoxyméthyle, éthoxyéthyle, éthoxyprop-1-yl, difluorométhyle, trifluorométhyle,
R⁴ et R⁵ 2,2-difluoroéthyle, 3,3,3-trifluoroéthyle, pentafluoroéthyle, cyclopropyle, cyclobutyle, cyclopentyle, ou cyclohexyle, ou
R⁶ forment, avec l'atome de carbone auquel ils sont liés, un anneau monocyclique ou bicyclique entièrement saturé ou partiellement saturé, de 3 à 10 chaînons, éventuellement interrompu par des hétéroatomes et présentant éventuellement une substitution supplémentaire, et
représente l'hydrogène, BH₂, B(NH₂)₂, 4-amino-1,3,2,4-diazadiboretidin-2-yl, 4-amino-1,3,2,4-dioxadiboretan-2-yl, méthylsulfonyl, éthylsulfonyl, prop-1-ylsulfonyl, cyanométhylsulfonyl, méthoxycarbonylmethylsulfonyl, 2-(méthoxycarbonyl)éth-1-ylsulfonyl, prop-2-en-1-ylsulfonyl, prop-2-en-2-ylsulfonyl, (4-fluorophényl)methylsulfonyl, formyl, méthylcarbonyl, éthylcarbonyl, prop-1- ylcarbonyl, 1-méthyléth-1-ylcarbonyl, cyclopropylcarbonyl, cyclopentylcarbonyl, 2-(méthoxy-carbonyl)éth-1-ylcarbonyl, éthoxycarbonylméthylcarbonyl, 3-(éthoxycarbonyl)prop-1-ylcarbonyl,

8. Le composé de formule générale (I) selon la revendication 1 et/ou son sel, **caractérisé en ce que**
R¹ représente le phényle, le 2-fluorophényle, le 3-fluorophényle, le 2-chlorophényle, le 2-méthylphényle, 2-éthylphényle, 2-méthoxyphényle, 2,3-difluorophényle, 2,4-difluorophényle, 2,6-difluorophényle, 2-fluoro-6-méthylphényle, 2-fluoro-3-méthylphényle, 2-fluoro-3-chlorophényle, 2,3-diméthylphényle, 2-thiényle, 3-chloro-2-thiényle, 3-méthyl-2-thiényle, ou 4-méthyl-3-thiényle,
R² représente l'hydrogène, le fluor, le chlore, le brome, le méthyle, l'éthyle, le 2,3,4,5,6-pentafluorophényle, ou l'éthoxycarbonyle,
R³ représente l'hydrogène,
n est 0, 2,
R⁴ et R⁵ représentent indépendamment des autres l'hydrogène, le méthyle, l'éthyle, le prop-1-yl, le prop-2-yl, le but-1-yl, le but-2-yl, 1,1-diméthyléth-1-yl, 2-méthylprop-1-yl, hydroxyméthyle, hydroxyéthyle, hydroxyprop-1-yl, méthoxyméthyle, méthoxyéthyle, méthoxyprop-1-yl, éthoxyméthyle, éthoxyéthyle,
R⁶ difluorométhyle, trifluorométhyle, 2,2-difluoroéthyle, 3,3,3-trifluoroéthyle, pentafluoroéthyle, cyclopropyle, cyclobutyle, cyclopentyle, ou cyclohexyle, ou

9. L'utilisation d'un ou de plusieurs composés de la formule générale (I) et/ou de leurs sels, tels que définis dans l'une quelconque des revendications 1 à 8, comme herbicide et/ou régulateur de croissance végétale.

10. Composition herbicide et/ou régulatrice de la croissance des végétaux, **caractérisée en ce que** la composition comprend un ou plusieurs composés de la formule générale (I) et/ou leurs sels tels que définis dans l'une quelconque des revendications 1 à 8, et une ou plusieurs autres substances choisies dans les groupes (i) et/ou (ii), avec
(i) une ou plusieurs autres substances agrochimiquement actives, constituées du groupe constitué par les insecticides, les acaricides, les nématicides, d'autres herbicides, les fongicides, les phytoprotecteurs, les engrais et/ou d'autres régulateurs de croissance,
(ii) une ou plusieurs auxiliaires de formulation habituels dans le domaine de la protection des cultures.

11. Méthode de lutte contre les végétaux nuisibles ou de régulation de la croissance des végétaux, **caractérisée en ce qu'**une quantité efficace
- d'un ou de plusieurs composés de la formule générale (I) et/ou de leurs sels, tels que définis dans l'une quelconque des revendications 1 à 8, ou
- d'une composition telle que revendiquée dans la revendication 10,
est appliquée aux végétaux, aux graines de végétaux, au sol dans lequel ou sur lequel les végétaux poussent ou à la zone cultivée.
